# EUROPEAN PATENT SPECIFICATION

(11) **EP 4 228 630 B1**
(45) Date of publication and mention of the grant of the patent: **05.03.2025**
(21) Application number: 21794528.6
(22) Date of filing: 15.10.2021
(51) Int. Cl.: A61K 31/4245, A61K 31/00, A61K 31/17, A61K 31/341, A61K 31/47, A61K 31/4709, A61P 31/00, A61P 35/00, A61P 37/04

(54) **NLRP3 ACTIVATORS FOR USE IN THE TREATMENT OF INFECTIOUS DISEASES OR CANCER BY ACTIVATING NLRP3 INFLAMMASOME**
NLRP3-AKTIVATOREN ZUR BEHANDLUNG VON INFEKTIONSKRANKHEITEN ODER KREBS DURCH AKTIVIERUNG DES NLRP3-INFLAMMASOMS
ACTIVATEURS DE NLRP3 À UTILISER DANS LE TRAITEMENT DES MALADIES INFECTIEUSES OU DU CANCER EN ACTIVANT L'INFLAMMASOME NLRP3

(30) Priority: 16.10.2020 EP 20202297
(43) Date of publication of application: 23.08.2023
(73) Proprietor: Albert-Ludwigs-Universität Freiburg, 79098 Freiburg (DE)
(72) Inventor: GROSS, Olaf, 79341 Kenzingen (DE); NEUWIRT, Emilia, 79114 Freiburg (DE); JUNG, Manfred, 79194 Gundelfingen (DE)
(74) Representative: Michalski Hüttermann & Partner Patentanwälte mbB
(86) International application number: PCT/EP2021/078623
(87) International publication number: WO 2022/079246

(56) References cited:
- WO-A1-03/080578
- WO-A1-2018/150302
- WO-A1-2018/167468
- WO-A1-2018/215818
- WO-A1-2019/010293
- WO-A1-2019/209896
- WO-A1-2020/102576
- WO-A1-2020/104657
- WO-A1-2020/123675
- WO-A2-2006/049941
- KR-A- 20190 026 154
- DATABASE caplus [online] 1 January 2019 (2019-01-01), DE ITURRATE MARTINEZ ET AL: "Toward discovery of new leishmanicidal scaffolds able to inhibit Leishmania GSK-3", XP55961546, Database accession no. 2019:2225572
- GÓMEZ DAVID ESTEBAN ET AL: "Urea vs. thiourea in anion recognition", ORGANIC & BIOMOLECULAR CHEMISTRY, vol. 3, no. 8, 1 January 2005 (2005-01-01), pages 1495 - 1500, XP55961197, ISSN: 1477-0520, DOI: 10.1039/B500123D
- MUSUMECI DOMENICA ET AL: "Supplementary Material: Tandem application of ligand-based virtual screening and G4-OAS assay to identify novel G-quadruplex-targeting chemotypes", 1 January 2017 (2017-01-01), XP55880690, Retrieved from the Internet <URL:https://ars.els-cdn.com/content/image/1-s2.0-S0304416517300326-mmc2.pdf> [retrieved on 20220118]
- GÓMEZ DAVID ESTEBAN ET AL: "Urea vs. thiourea in anion recognition", ORGANIC & BIOMOLECULAR CHEMISTRY, vol. 3, no. 8, 1 January 2005 (2005-01-01), pages 1495 - 1500, XP055961197, ISSN: 1477-0520, DOI: 10.1039/B500123D
- JULIEN P. N. PAPILLON ET AL: "Discovery of Orally Active Inhibitors of Brahma Homolog (BRM)/SMARCA2 ATPase Activity for the Treatment of Brahma Related Gene 1 (BRG1)/SMARCA4-Mutant Cancers", JOURNAL OF MEDICINAL CHEMISTRY, vol. 61, no. 22, 19 October 2018 (2018-10-19), US, pages 10155 - 10172, XP055619669, ISSN: 0022-2623, DOI: 10.1021/acs.jmedchem.8b01318
- DATABASE HCAPLUS [online] 1 January 1962 (1962-01-01), MALATESTA ET AL: "Synthesis of some new anilides with local anesthetic activity", XP55880467, Database accession no. 1963:8592
- ANTONIO LAVECCHIA ET AL: "Discovery of New Inhibitors of Cdc25B Dual Specificity Phosphatases by Structure-Based Virtual Screening", JOURNAL OF MEDICINAL CHEMISTRY, vol. 55, no. 9, 10 May 2012 (2012-05-10), pages 4142 - 4158, XP055156044, ISSN: 0022-2623, DOI: 10.1021/jm201624h
- KHAN K M ET AL: "Unsymmetrically disubstituted urea derivatives: A potent class of antiglycating agents", BIOORGANIC & MEDICINAL CHEMISTRY, ELSEVIER, AMSTERDAM, NL, vol. 17, no. 6, 15 March 2009 (2009-03-15), pages 2447 - 2451, XP025981943, ISSN: 0968-0896, [retrieved on 20090209], DOI: 10.1016/J.BMC.2009.01.075
- DATABASE HCAPLUS [online] 1 January 1987 (1987-01-01), MURATA AU ET AL: "Chemical Abstract: AN 1987:636465", XP55880463, Database accession no. 1987:636465
- DATABASE Registry [online] 1 January 2011 (2011-01-01), ANONYMOUS: "2,4-Imidazolidinedione, 1-(4-fluorophenyl)-5-methyl-3-[[3-(4-methylphenyl)- 1,2,4-oxadiazol-5-yl]methyl]-", XP055790630, Database accession no. 1277156-39-4
- DATABASE Registry [online] 1 January 2011 (2011-01-01), ANONYMOUS: "Urea, N-(3-chloro-2,6-diethylphenyl)-N'-[2-methyl-1-[3-(3-pyridinyl)-1,2,4-oxadiazol-5-yl]propyl]-", XP055790631, Database accession no. 1311951-62-8
- DATABASE Registry [online] 1 January 2004 (2004-01-01), ANONYMOUS: "683206-01-1", XP055790629, Database accession no. 683206-01-1
- ZHANG YAN-FENG ET AL: "A COX-2/sEH dual inhibitor PTUPB ameliorates cecal ligation and puncture-induced sepsis in mice via anti-inflammation and anti-oxidative stress", BIOMEDICINE & PHARMACOTHERAPY, ELSEVIER, FR, vol. 126, 27 February 2020 (2020-02-27), XP086138328, ISSN: 0753-3322, [retrieved on 20200227], DOI: 10.1016/J.BIOPHA.2020.109907
- KUMAR K SANTOSH ET AL: "Synthesis and antimicrobial evaluation of novel urea derivatives from chromene based oxadiazole amines", MEDICINAL CHEMISTRY RESEARCH, BIRKHAEUSER, BOSTON, US, vol. 25, no. 10, 26 July 2016 (2016-07-26), pages 2179 - 2186, XP036081690, ISSN: 1054-2523, [retrieved on 20160726], DOI: 10.1007/S00044-016-1651-6
- DATABASE Registry [online] 1 January 2004 (2004-01-01), ANONYMOUS: "683206-01-1", XP055790629, Database accession no. 683206-01-1
- DATABASE Registry [online] 1 January 2011 (2011-01-01), ANONYMOUS: "2,4-Imidazolidinedione, 1-(4-fluorophenyl)-5-methyl-3-[[3-(4-methylphenyl)- 1,2,4-oxadiazol-5-yl]methyl]-", XP055790630, Database accession no. 1277156-39-4
- DATABASE Registry [online] 1 January 2011 (2011-01-01), ANONYMOUS: "Urea, N-(3-chloro-2,6-diethylphenyl)-N'-[2-methyl-1-[3-(3-pyridinyl)-1,2,4-oxadiazol-5-yl]propyl]-", XP055790631, Database accession no. 1311951-62-8
- MUSUMECI DOMENICA ET AL: "Tandem application of ligand-based virtual screening and G4-OAS assay to identify novel G-quadruplex-targeting chemotypes", BIOCHIMICA ET BIOPHYSICA ACTA, ELSEVIER, AMSTERDAM, NL, vol. 1861, no. 5, 24 January 2017 (2017-01-24), pages 1341 - 1352, XP085056372, ISSN: 0304-4165, DOI: 10.1016/J.BBAGEN.2017.01.024
- MUSUMECI DOMENICA ET AL: "Supplementary Material: Tandem application of ligand-based virtual screening and G4-OAS assay to identify novel G-quadruplex-targeting chemotypes", 1 January 2017 (2017-01-01), XP055880690, Retrieved from the Internet <URL:https://ars.els-cdn.com/content/image/1-s2.0-S0304416517300326-mmc2.pdf> [retrieved on 20220118]
- KLINGLER FRANCA-MARIA ET AL: "SAR by Space: Enriching Hit Sets from the Chemical Space", MOLECULES, vol. 24, no. 17, 26 August 2019 (2019-08-26), pages 3096, XP055880754, DOI: 10.3390/molecules24173096
- RUAN BANFENG ET AL: "Synthesis and structure-activity relationship study of pyrrolidine-oxadiazoles as anthelmintics against Haemonchus contortus", EUROPEAN JOURNAL OF MEDICINAL CHEMISTRY, ELSEVIER, AMSTERDAM, NL, vol. 190, 25 January 2020 (2020-01-25), XP086065708, ISSN: 0223-5234, [retrieved on 20200125], DOI: 10.1016/J.EJMECH.2020.112100
- BRUCE M I ET AL: "Chemical structure and plant kinin activity- the activity of urea and thiourea derivatives", LIFE SCIENCE, PERGAMON PRESS, OXFORD, GB, vol. 4, no. 4, 1 February 1965 (1965-02-01), pages 461 - 466, XP025517974, ISSN: 0024-3205, [retrieved on 19650201], DOI: 10.1016/0024-3205(65)90094-9
- DATABASE HCAPLUS [online] 1 January 1962 (1962-01-01), MALATESTA ET AL: "Synthesis of some new anilides with local anesthetic activity", XP055880467, Database accession no. 1963:8592
- GOTTLIEB L ET AL: "Electrophilic formamidines. Organometallic addition to 2-methoxy pyrrolidine or piperidine N-t-butyl formamidines. Formation of 2-substituted pyrrolidines and piperidines", TETRAHEDRON LETTERS, ELSEVIER, AMSTERDAM , NL, vol. 31, no. 33, 1 January 1990 (1990-01-01), pages 4723 - 4726, XP026024991, ISSN: 0040-4039, [retrieved on 19900101], DOI: 10.1016/S0040-4039(00)97716-5
- DATABASE HCAPLUS [online] 1 January 1987 (1987-01-01), MURATA AU ET AL: "Chemical Abstract: AN 1987:636465", XP055880463, Database accession no. 1987:636465
- CHEN HAO ET AL: "Discovery of dronedarone and its analogues as NLRP3 inflammasome inhibitors with potent anti-inflammation activity", BIOORGANIC & MEDICINAL CHEMISTRY LETTERS, ELSEVIER, AMSTERDAM, NL, vol. 46, 29 May 2021 (2021-05-29), XP086644747, ISSN: 0960-894X, [retrieved on 20210529], DOI: 10.1016/J.BMCL.2021.128160

## Description

### Technical Field

The present invention relates to compounds or pharmaceutically acceceptable salts thereof, that activate the NLRP3 inflammasome. Moreover, TLR7 and/or TLR8 is not activated by these compounds. Moreover, the mitochondrial respiratory chain is not inhibited by these compounds. This invention further relates to the compounds and/or compositions for use in the prevention and treatment of a condition, a disease or a disorder by activating the NLRP3 inflammasome, wherein the disease is selected from a group comprising infectious diseases or cancer. Moreover, the invention relates to the use of the present compounds as an adjuvant for vaccination. Moreover, the invention relates to the use of the present compounds for analyzing the mechanisms and consequences of NLRP3 activation.

### Background of the Invention

The present invention provides compounds useful as modulators of the NLRP3 inflammasome. NLRP (Nucleotide-binding oligomerization domain, Leucine rich Repeat and Pyrin domain containing) proteins, are a subfamily of NLRs (NOD-like receptor), which play a role in the formation of inflammasomes.

Inflammasomes are intracellular multiprotein oligomers of the innate immune system responsible for the activation of inflammatory responses. Moreover, inflammasomes control the bioactivity of potent pro-inflammatory cytokines of the interleukin-1 (IL-1) family members such as IL-1β and induce a form of lytic, proinflammatory cell death known as pyroptosis. These effects of inflammasome activity are mediated by one of its components, the protease caspase-1. It directly mediates maturation of inactive pro-IL-1β and controls IL-1 release and subsequent pyroptosis by its pore-forming substrate Gasdermin-D (GSDMD). The inflammasome/IL-1 axis promotes protective inflammatory responses in the context of infection, but its dysregulation can lead to pathological inflammation. The different inflammasomes respond to a variety of endogenous and exogenous danger signals and thus contribute significantly to the defense against pathogens. Most inflammasomes are triggered by the presence of specific pathogen components within the cytoplasm, either by direct binding or by detecting their activity. Instead, the NLRP3 inflammasome appears to respond to cellular stress signals triggered by structurally diverse pathogens, endogenous danger signals, and environmental irritants.

NLRP3 is expressed predominantly in certain immune cells such as in macrophages as well as in epithelial cells. NLRP3 is a cytoplasmic sensor protein responding to sterile endogenous danger signals that are typically products of damaged cells such as extracellular **ATP** and crystalline uric acid. Upon activation, it nucleates an inflammasome by connecting to the protease caspase-1 via the adaptor protein ASC (encoded by *PYCARD),* thereby triggering an immune response. While other inflammasome nucleating receptors mainly react to pathogen-derived signals, NLRP3 is the main contributor to sterile inflammation.

A targeted activation of NLRP3 can induce an inflammasome-dependent anti-cancer immune response, e.g. by increasing the activity of cytotoxic cells against tumor cells. Thus, NLRP3 activators may be used for the treatment of malignancies.

WO 2019/209896 A1 discloses compounds that modulate, i.e. either agonize or partially agonize NLRP3 that are useful, e.g. for treating a condition, disease or disorder in which an increase in NLRP3 signaling may correct a deficiency in innate immune activity that contributes to the pathology and/or symptoms and/or progression and/or treatment refractory state of the condition, disease or disorder in a subject.

Moreover, many of the currently known NLRP3 activators have additional unspecific or unrelated activities that are unfavorable. For example, bacterial ionophore toxins such as nigericin that allow potassium efflux thought to be involved in NLRP3 activation integrate into cellular membranes of any cell type. Furthermore, known small molecule NLRP3 activators such as the imidazoquinoline compounds imiquimod (also known as R837) or CL097 also activate toll-like receptor-7 (TLR7) and/or TLR8. TLR7/8 activation mechanistically explains the production of interferons and other proinflammatory cytokines like TNF and IL-6 and the antiviral activity triggered by this class of molecules, and it is classically thought to account for their anti-tumor effect. It was discovered that imiquimod and the similar molecule CL097 are not only NLRP3 activators and TLR7/8 ligands, but that they also inhibit cellular respiration by inhibiting the mitochondrial electron transport chain (Christina J. Groß, Ritu Mishra, Katharina S. Schneider, Guillaume Médard, Jennifer Wettmarshausen, Daniela C. Dittlein, Hexin Shi, Oliver Gorka, Paul-Albert Koenig, Stephan Fromm, Giovanni Magnani, Tamara Ćiković, Lara Hartjes, Joachim Smollich, Avril A.B. Robertson, Matthew A. Cooper, Marc Schmidt-Supprian, Michael Schuster, Kate Schroder, Petr Broz, Claudia Traidl-Hoffmann, Bruce Beutler, Bernhard Kuster, Jürgen Ruland, Sabine Schneider, Fabiana Perocchi and Olaf Groß, "K+ Efflux-Independent NLRP3 Inflammasome Activation by Small Molecules Targeting Mitochondria", Immunity, volume 45, issue 4, October 18, 2016, pp. 761-773). Both TLR7/8 activation and electron transport chain inhibition by imidazoquinolines might contribute to their antiviral and anti-tumor activity, but are also known to have adverse effects. Thus, there is still a need to provide selective activators for the NLRP3 inflammasome.

In summary, there is a need for NLRP3 activators, which are more selective than previous NLRP3 activators and can boost immune responses and trigger tumoricidal activity of the immune system.

In addition, new small molecule NLRP3 activators are useful to study the biology of the NLRP3 inflammasome and thus make an important contribution to the fundamental understanding of the activation mechanisms of the NLRP3 inflammasome and its function in health and disease and enable the targeted development of specific drugs.

The present invention addresses these needs. In particular, the present invention provides compounds and compositions comprising these compounds, which can activate NLRP3 without activating TLR7 and/or TLR8 and/or the mitochondrial electron transport chain. Therefore, these compounds are useful in the prevention or treatment of diseases such as cancer as well as infectious diseases or in the context of vaccination to boost specific immune responses as an adjuvant by activating NLRP3. Moreover, these compounds are useful in methods for analyzing the biology of NLRP3 activation and signaling mechanisms involved.

### Summary of the Invention

According to the first aspect, the present invention relates to a compound for use in the prevention or treatment of a disease by activating the NLRP3 inflammasome, wherein the disease is selected from a group comprising infectious diseases or cancer, and
wherein the compound is a compound of Formula (I), Formula (I') or Formula (I") 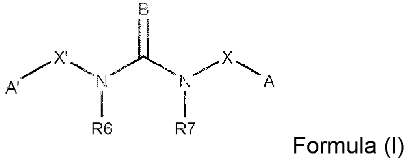 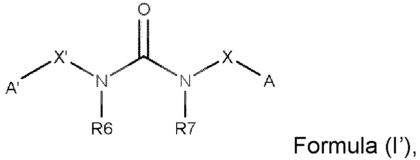 or 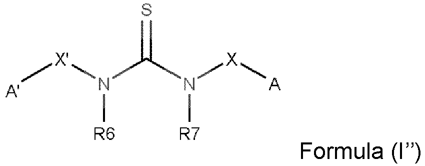 or a pharmaceutically acceceptable salt thereof, wherein:
A and A' are independently selected from each other a a a a a a a and are as defined herein below
B is O or S, preferably O.
X and X' are each independently present or not, wherein X and X' are each independently a C₁-C₅ alkyl group; more preferably selected from -CH₂-, -C₂H₄-, -C₃H₆-, -CH(CH₃)₂-, - CHCH(CH₃)₂-, -CHC(CH₃)₃-, -CH(CH(CH₃)₂)-, -CH(CH₂CH(CH₃)₂)-, or -CH(CH₂OH)-.
R⁶ and R⁷ are each independently hydrogen, a C₁-C₃ alkyl, preferably -H, -CH₃, -C₂H₅, -C₃H₇, or -C₃H₉, or optionally
R⁶ and R⁷ when taken together form a five- or six-membered heterocyclic ring.
preferably selected from 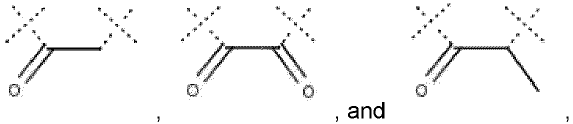 or optionally,
R⁷, N and X when taken together form a five- or six-membered heterocyclic ring, preferably selected from

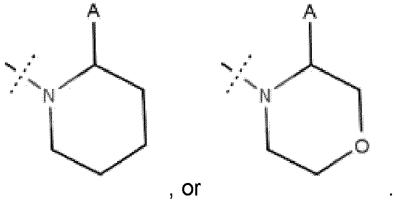

In one embodiment, the compound of Formula (I), (I') or (I") of the invention comprises X and X', wherein X and X' are each independently -(CH₂)ₘ-, wherein each m is independently an integer from 0 to 1, 0 to 2, 0 to 3 or 0 to 4, or C₁-C₃ alkyl, a C₁-C₂ alkyl, preferably, -CH₂-, - C₂H₄-, -C₃H₆-, -CH(CH₃)₂-, -CH(CH(CH₃)₂)-, -CH(CH₂CH(CH₃)₂)-, or -CH(CH₂OH)-, wherein preferably only one of X and X' is present and is selected from a C₁-C₃ alkyl, a C₁-C₂ alkyl, preferably, -CH₂-, -C₂H₄-, -C₃H₆-, -CH(CH₃)₂-, -CH(CH(CH₃)₂)-, -CH(CH₂CH(CH₃)₂)-, or - CH(CH₂OH)-; and wherein
R⁶ and R⁷ are each independently hydrogen, a C₁-C₃ alkyl, C₁-C₂ alkyl, more preferably hydrogen or CH₃, even more preferably preferably wherein R⁶ and R⁷ are each hydrogen, and wherein A and A' are preferably as defined herein below.

In one further embodiment, the compound of Formula (I), (I') or (I") of the invention comprises X and X', wherein X and X' are each independently -(CH₂)ₘ-, wherein each m is independently an integer from 0 to 1, 0 to 2, 0 to 3 or 0 to 4, or C₁-C₃ alkyl, a C₁-C₂ alkyl, preferably, -CH₂-, - C₂H₄-, -C₃H₆-, -CH(CH₃)₂-, -CH(CH(CH₃)₂)-, -CH(CH₂CH(CH₃)₂)-, or -CH(CH₂OH)-, preferably only one of X and X' is present and is a C₁-C₃ alkyl, a C₁-C₂ alkyl, preferably, -CH₂-, -C₂H₄-, - C₃H₆-, -CH(CH₃)₂-, -CH(CH(CH₃)₂)-, -CH(CH₂CH(CH₃)₂)-, or -CH(CH₂OH)-; and wherein
R⁶ and R⁷ when taken together form a five- or six-membered heterocyclic ring.
preferably selected from 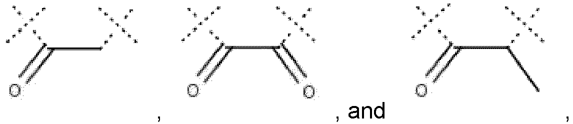 and wherein A and A' are preferably as defined herein below.

In one further embodiment, the compound of Formula (I), (I') or (I") of the invention comprises X and X', wherein X and X' are each independently -(CH₂)ₘ-, wherein each m is independently an integer from 0 to 1, 0 to 2, 0 to 3 or 0 to 4, or C₁-C₃ alkyl, a C₁-C₂ alkyl, preferably, -CH₂-, - C₂H₄-, -C₃H₆-, -CH(CH₃)₂-, -CH(CH(CH₃)₂)-, -CH(CH₂CH(CH₃)₂)-, or -CH(CH₂OH)-, preferably only one of X and X' is present and is a C₁-C₃ alkyl, a C₁-C₂ alkyl, preferably, -CH₂-, -C₂H₄-, - C₃H₆-, -CH(CH₃)₂-, -CH(CH(CH₃)₂)-, -CH(CH₂CH(CH₃)₂)-, or -CH(CH₂OH)-; and wherein R⁷, N and X when taken together form a five- or six-membered heterocyclic ring, preferably selected from 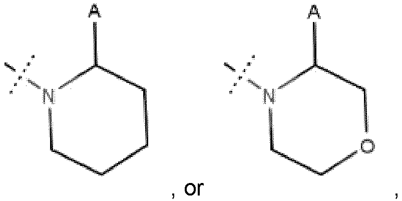 and wherein A and A' are preferably as herein below.

In one embodiment, the compound of Formula (I), (I') or (I") of the invention comprises X and X', wherein X and X' are each independently -(CH₂)ₘ-, wherein each m is independently an integer from 0 to 2, preferably 0 or 1, more preferably 0, wherein A and A' are preferably as defined herein below.

In one embodiment, the compound of Formula (I), (I') or (I") of the invention comprises X and X', wherein X and X' are each independently -(CH₂)ₘ-, wherein each m is independently an integer from 0 to 2, preferably 0 or 1, more preferably 0, and R⁶ and R⁷ are each independently hydrogen, wherein A and A' are preferably as defined herein below.

In one further embodiment, the compound of Formula (I), (I') or (I") of the invention comprises none of X and X'; and R⁶ and R⁷ when taken together form a five- or six-membered heterocyclic ring. preferably selected from 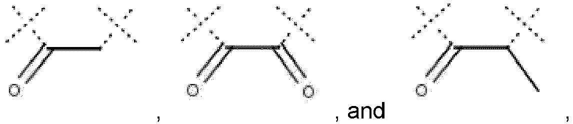 and wherein A is preferably as defined herein below.

In one further embodiment, in the compound of Formula (I), (I') or (I") of the invention R⁷, N and X when taken together form a five- or six-membered heterocyclic ring, preferably selected from 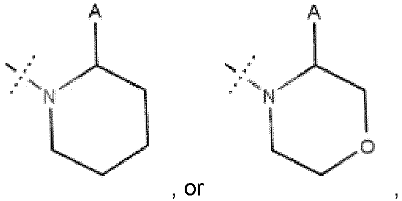 and wherein and A' are as herein below.

In one preferred embodiment, the compound of Formula (I), (I') or (I") of the invention comprises X and X', wherein X and X' are each independently a C₁-C₃ alkyl, a C₁-C₂ alkyl, preferably, -CH₂-, -C₂H₄-, -C₃H₆-, -CH(CH₃)₂-, -CH(CH(CH₃)₂)-, -CH(CH₂CH(CH₃)₂)-, or - CH(CH₂OH)-, and
preferably only one of X and X' is present and is a C₁-C₃ alkyl, a C₁-C₂ alkyl, preferably selected from the group consisting of, -CH₂-, -C₂H₄-, -C₃H₆-, -CH(CH₃)₂-, -CH(CH(CH₃)₂)-, - CH(CH₂CH(CH₃)₂)-, or -CH(CH₂OH)-, and
A and A' are each independently isoquinoline, quinoline, benzene, pyridine, 1,5-naphthyridine, isothiazole, or oxadiazole as defined herein. A and A' are different to each other. A and A' are preferably as defined herein below.

A and A' are each independently quinoline, isoquinoline, benzene, pyridine, naphthyridine, 1,5-naphthyridine, 1,6-naphthyridine, 1,8-naphthyridine, isothiazole, oxadiazole, or thiophene, preferably selected from groups as defined in the following in any of sections a) to i):
a) a benzene group of the following structure: 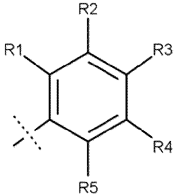 wherein R¹-R⁵ are each independently selected from the group consisting of:
   hydrogen; -OH, -OCH₃, -OCF₃, -OCHF₂, -OCH₂OH; -OCH₂CH₃; -OCH(CH₃)₂; -F; -Cl; -Br; - CN; NO₂; NH₂; C₁-C₅ alkyl, C₁-C₅ alkoxy, C₂-C₅ alkenyl, -CH₃, -CH₂F, -CHF₂, -CF₃, -CH₂Cl, - CHCl₂, -CCl₃, CH(CH₃)₂; CHCH(CH₃)₂; -CH₂CH₃; -CH₂-CH₂-CH(CH₃)₂; -CH₂-N(CH₃)₂; - CH(CH₃)-NHC(O)CH₃; - -NH-CH(CH₃)₂, -NH-cyclopropyl, -CHF₂, -C(O)-C₂H₅; -C(O)-CH₃; - C(O)-aziridine; -C(O)-azetidine; -C(O)-pyrrolidine; -C(CH₃)=NOH; -S(O)₂-cyclopentyl; -S(O)₂-CH₃; -S(O)₂-cyclopentyl; pyrrolidinyl, C₁-C₁₀ cycloalkyl, C₁-C₁₀ cycloheteroalkyl, 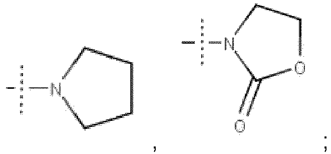 a C₆-C₁₀ aryl; a 5-10 membered heteroaryl; or
   NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-CH(CH₃)₂, -NH-cyclopropyl, C₁-C₅ alkyl, -CH₂F, -CHF₂, -CF₃, -CH₂Cl; -CHCl₂; -CCl₃; -CH₃, CH₂CH₃, CHCH(CH₃)₂; or
   -C(O)OR⁴², wherein R⁴² is selected from C₁-C₅ alkyl, including -CH₃; -CH₂CH₃, -CH₂CH₂CH₃; -CH(CH₃)₂; -CH₂CH₂CH₂CH₃; -CH₂NHS(O)₂CH₃; or
   -NHR⁴³, wherein R⁴³ is selected from 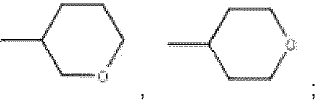 or 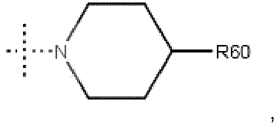
   wherein R⁶⁰ is selected from hydrogen or -CH₃; or 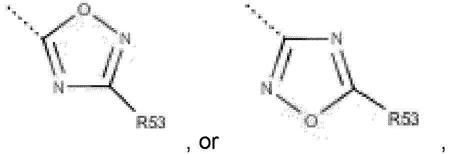
   wherein
   R⁵³ is preferably selected from the group consisting of hydrogen, OH, -OCH₃, -OCF₃, - OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -CH₂F, -CHF₂, -CF₃; -CH₂Cl; -CHCl₂; -CCl₃; -O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, -NH(C=O)NH₂, -NH(C=S)NH₂, -(C=O)NH₂,-CHO,-CHS, -OCHO, -NCHO, 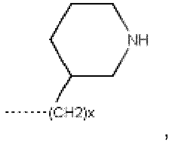 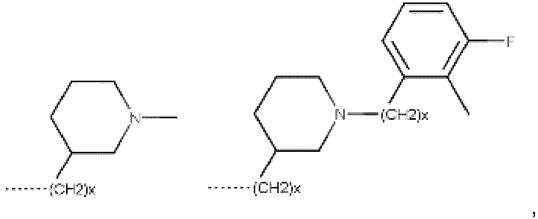 with X = 0 or 1, preferably 1; 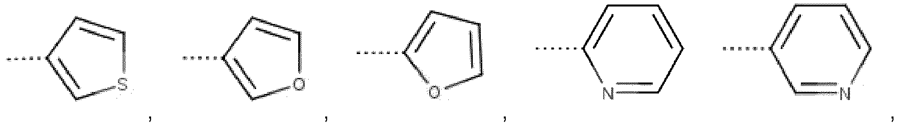 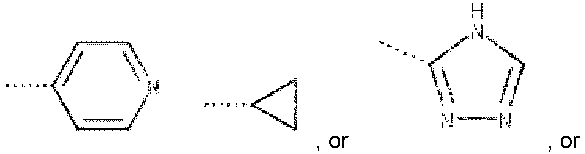
   R⁵³ is 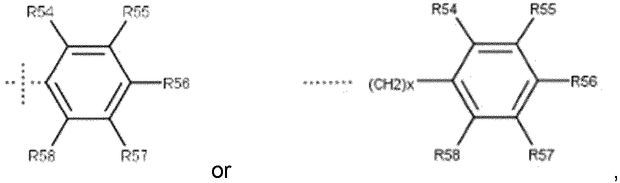 **wherein** R⁵⁴, R⁵⁵, R⁵⁶, R⁵⁷ or R⁵⁸ are selected each independently from hydrogen, -CH₃, -C₂H₅, -CF₃, --CH₂Cl; - CHCl₂; -CCl₃; OCH₃, -F, -Cl, -Br, NH₂, NO₂, and x is 0, 1 or 2; or
   R⁵³ is 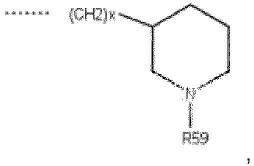 wherein R⁵⁹ is 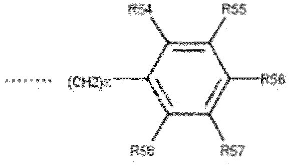 and wherein
   R⁵⁴, R⁵⁵, R⁵⁶, R⁵⁷ or R⁵⁸ are selected separately from the above from hydrogen, -CH₃, -C₂H₅, - CH₂CF, -CF₃, -CH₂Cl; -CHCl₂; -CCl₃; -OCH₃, -F, -Cl, -Br, NO₂, NH₂, and x is 0, 1 or 2; as defined above;
   and optionally, wherein R¹ and R², R² and R³, R³ and R⁴, or R⁴ and R⁵ together, preferably R² and R³ together, form a five- or six-membered cyclic or heterocyclic ring, preferably selected from 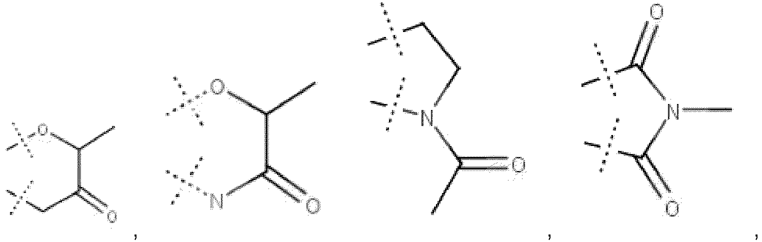 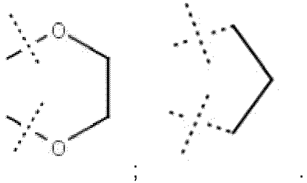
   Preferably X is not present in Formulae (I), (I') or (I") in case A or A' is a benzene group as defined under a). Likewise, one of X or X'is present or both of X and X' are present, and X and X' or are each preferably selected from -CH₂-, -C₂H₄-, -C₃H₆-, -CHCH(CH₃)₂-, - CHC(CH₃)₃-, -CH(CH(CH₃)₂)-, -CH(CH₂CH(CH₃)₂)-, or -CH(CH₂OH)-.
   Likewise preferably either all of R¹ to R⁵ are hydrogen or 1, 2 or 3, preferably 1 or 2 of R¹ to R⁵ are different from hydrogen and selected from substitutions defined or presented herein above under a) for R¹ to R⁵, and remaining substituents of R¹ to R⁵ are hydrogen.
b) a pyridine group of one of the following structures: 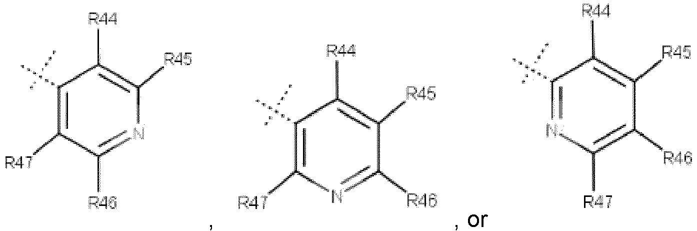 wherein R ⁴⁴ -R ⁴⁷ are each independently selected from the group consisting of:
   hydrogen, -OH, -OCH₃, CH₃, F, Cl, Br, -CH₂F; -CHF₂; -CF₃; -CH₂Cl; -CHCl₂; -CCl₃; -OCF₃, - OCHF₂, -OCH₂OH; -OCH₂CH₃; -CN; NO₂; NH₂; -CH₃, CH(CH₃)₂; -CH₂CH₃; -CH₂OH; -CH₂-CH₂-CH(CH₃)₂; -CH₂-N(CH₃)₂; -CH(CH₃)-NHC(O)CH₃; 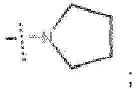
   -NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-cyclopropyl, -CHF₂, -CH₂F; -CHF₂; -CF₃, -CH₂Cl; -CHCl₂; -CCl₃; -CH₃, CH₂CH₃, CHCH(CH₃)₂; -C(O)-aziridine; -C(O)-azetidine; - C(O)-pyrrolidine; a C₆-C₁₀ aryl; a 5-10 membered heteroaryl; C₁-C₁₀ cycloalkyl, C₁-C₁₀ cycloheteroalkyl,
   -C(O)OR⁴², wherein R⁴² is selected from C₁-C₅ alkyl, including -CH₃; -CH₂CH₃, -CH₂CH₂CH₃; -CH(CH₃)₂; -CH₂CH₂CH₂CH₃; -CH₂NHS(O)₂CH₃; -C(CH₃)=NOH; -S(O)₂-cyclopentyl; -S(O)₂-CH₃; -S(O)₂-cyclopentyl; pyrrolidinyl, 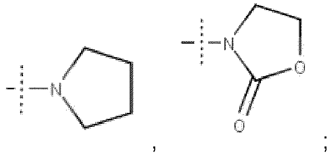 or
   -NHR⁴³, wherein R⁴³ is selected from 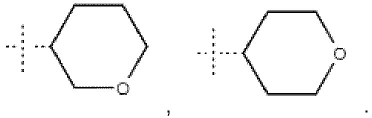
   Preferably in Formula (I), (I') or (I") in case A and/or A' is a pyridine group as defined under b) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
   Likewise preferably either all of R⁴⁴ to R⁴⁷ are hydrogen or 1, 2 or 3, preferably 1 or 2 of R⁴⁵ to R⁴⁷ are different from hydrogen and selected from substitutions defined or presented herein above under b) for R⁴⁴ to R⁴⁷, and remaining substituents of R⁴⁴ to R⁴⁷ are hydrogen.
c) a quinoline or an isoquinoline group, preferably of one of the the following structures: 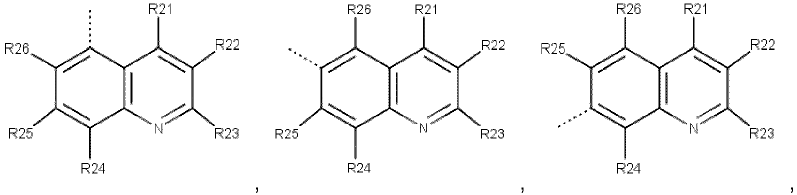 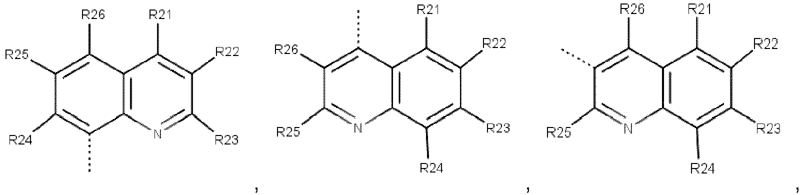 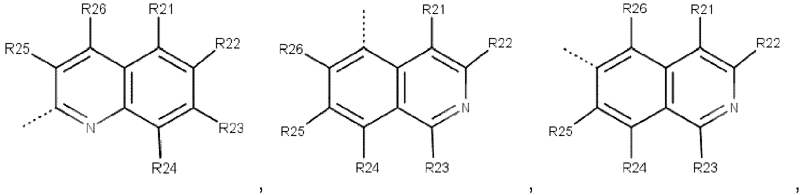 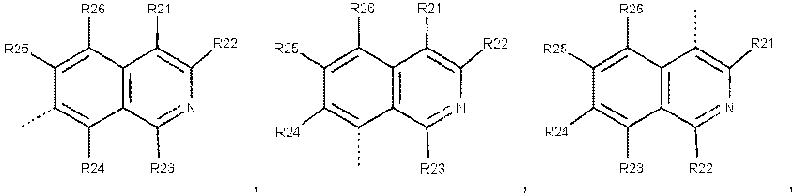 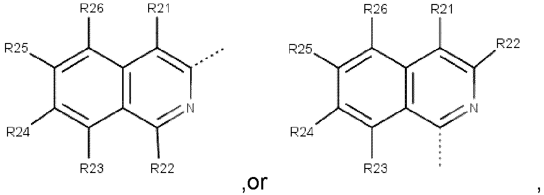 wherein
   R²¹ to R²⁶ are each independently selected from the group consisting of:
   hydrogen, OH, -OCH₃, -OCF₃, -OCHF₂, -OCH₂OH; -OCH₂CH₃; -F; -Cl; -Br; -CH₂F; -CHF₂; - CF₃; -CH₂Cl; -CHCl₂; -CCl₃; -CN; NO₂; NH₂; C₁-C₅ alkyl, C₁-C₅ alkoxy, C₂-C₅ alkenyl, preferably -CH₃, CH(CH₃)₂; -CH₂CH₃; -C₂H-CH₂-CH(CH₃)₂; -CH₂-N(CH₃)₂; -CH(CH₃)-NHC(O)CH₃; -O-N=O, -N=O, -CH=NH, -N₃, -SH, -SO₃H, -SO₂H, -S(O)₂CH₃ -CN, -NC,-OCN, - NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -C(O)-aziridine; -C(O)-azetidine; - C(O)-pyrrolidine; -C(O)OR⁴², wherein R⁴² is selected from C1-C5 alkyl, including -CH₃; - CH₂CH₃, -CH₂CH₂CH₃; -CH(CH₃)₂; -CH₂CH₂CH₂CH₃; -CH₂NHS(O)₂CH₃; -C(CH₃)=NOH; - S(O)₂-cyclopentyl; -S(O)₂-CH₃; -S(O)₂-cyclopentyl; pyrrolidinyl, 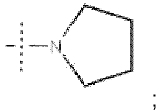 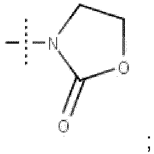 a C₆-C₁₀ aryl; a 5-10 membered heteroaryl; C₁-C₁₀ cycloalkyl, C₁-C₁₀ cycloheteroalkyl; or
   -NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-cyclopropyl, -CH₂F, -CHF₂, -CF₃, - CH₂Cl; -CHCl₂; -CCl₃; -CH₃, CH₂CH₃, CHCH(CH₃)₂; or
   -NHR⁴³, wherein R⁴³ is selected from 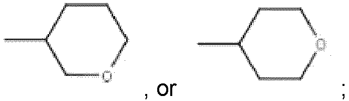
   or more preferably wherein R²¹ to R²⁶ are each hydrogen, OH, -OCH₃, -OCF₃, -OCHF₂, - OCH₂OH; -OCH₂CH₃; -F; -Cl; -Br; -CH₂F; -CHF₂; -CF₃; -CH₂Cl; -CHCl₂; -CCl₃;-CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃;
   or even more preferably wherein R²¹ to R²⁶ are each hydrogen, or -F; Cl, Br.
   Preferably in case A and/or A' is a quinoline or an isoquinoline group in Formula (I), (I') or (I") as defined in c) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
   Likewise preferably either all of R²¹ to R²⁶ are hydrogen or 1, 2 or 3, preferably 1 or 2 of R²¹ to R²⁶ are different from hydrogen and selected from substitutions defined or presented herein above under c) for R²¹ to R²⁶ and remaining substituents of R²¹ to R²⁶ are hydrogen;
d) a naphthyridine, preferably a 1,5 naphthyridine group, a 1,6 naphthyridin group or a 1,8 naphthyridin group of any of the following structures: 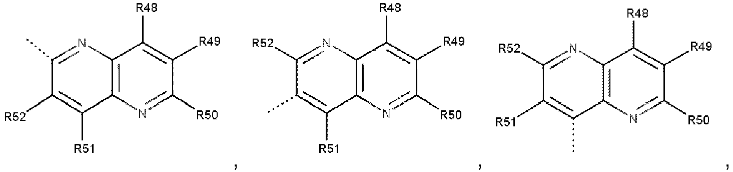 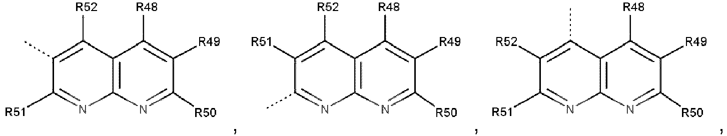 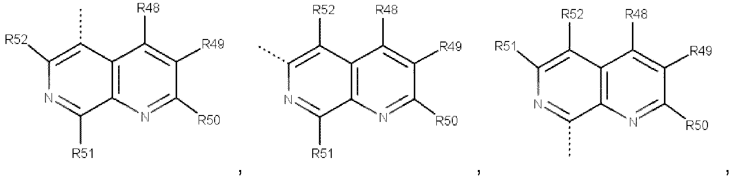 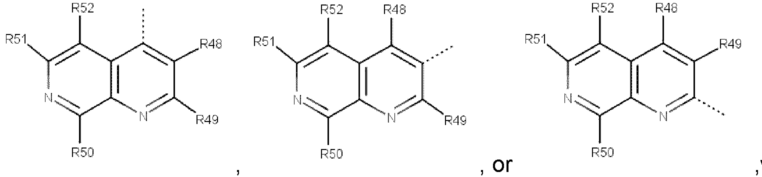 wherein
   R⁴⁸ to R⁵² are each independently selected from the group consisting of:
   hydrogen, OH, -OCH₃, -OCF₃, -OCHF₂, -OCH₂OH; -OCH₂CH₃; -F; -Cl; -Br; -CH₂F; -CHF₂; - CF₃; -CH₂Cl; -CHCl₂; -CCl₃; -CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -O-N=O, -N=O, -CH=NH, -N₃, -SH, -SO₃H, -SO₂H, -S(O)₂CH₃ -CN, -NC,-OCN, - NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO,
   -NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-cyclopropyl, -CHF₂, -CH₂F; -CHF₂; -CF₃; -CH₂Cl; -CHCl₂; -CCl₃;-CH₃, CH₂CH₃, CHCH(CH₃)₂; -C(O)-aziridine; -C(O)-azetidine; - C(O)-pyrrolidine;
   -C(O)OR⁴², wherein R⁴² is selected from C₁-C₅ alkyl, including -CH₃; -CH₂CH₃, -CH₂CH₂CH₃; -CH(CH₃)₂; -CH₂CH₂CH₂CH₃; -CH₂NHS(O)₂CH₃; -C(CH₃)=NOH; -S(O)₂-cyclopentyl; -S(O)₂-CH₃; -S(O)₂-cyclopentyl; pyrrolidinyl, 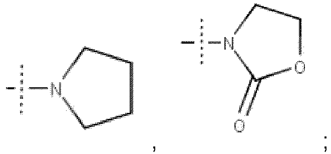 a C₆-C₁₀ aryl; a 5-10 membered heteroaryl; C₁-C₁₀ cycloalkyl, C₁-C₁₀ cycloheteroalkyl;
   -NHR⁴³, wherein R⁴³ is selected from 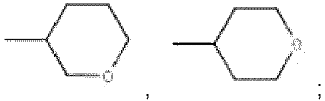
   or more preferably wherein R⁴⁸ to R⁵² are each hydrogen, OH, -OCH₃, -OCF₃, -OCHF₂, - OCH₂OH; -OCH₂CH₃; -F; -Cl; -Br; -CH₂F; -CHF₂; -CF₃; -CH₂Cl; -CHCl₂; -CCl₃;-CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃;
   or even more preferably wherein R⁴⁸ to R⁵² are each hydrogen, or -F; Cl, Br.
   Preferably in Formula (I), (I') or (I") in case A and/or A' is a 1,5-naphthyridine group, 1,6 naphthyridine group or a 1,8 naphthyridine group as defined under d) X and/or X' are either not present, only one of X or X'is present or both of X and X' are present, and X and X' or are each preferably selected from -CH₂-, -C₂H₄-, -C₃H₆-, -CHCH(CH₃)₂-, -CHC(CH₃)₃-, - CH(CH(CH₃)₂)-, -CH(CH₂CH(CH₃)₂)-, or -CH(CH₂OH)-.
   Likewise preferably either all of R⁴⁸ to R⁵² are hydrogen or 1, 2 or 3, preferably 1 or 2 of R⁴⁸ to R⁵² are different from hydrogen and selected from substitutions defined or presented herein above under d) for R⁴⁸ to R⁵² and remaining substituents of R⁴⁸ to R⁵² are hydrogen;
e) an oxadiazole group, preferably selected from one of the following structures: 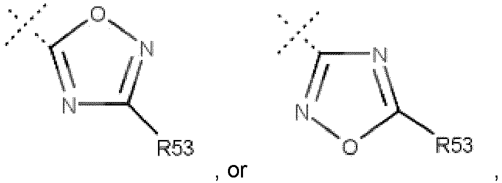 wherein
   R⁵³ is preferably selected from the group consisting of:
   hydrogen, OH, -OCH₃, -OCF₃, -OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -CHF₂, -CH₂F; -CHF₂; -CF₃; -CH₂Cl; - CHCl₂; -CCl₃;, -O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, -NH(C=O)NH₂, -NH(C=S)NH₂, -(C=O)NH₂,-CHO,-CHS, - OCHO, -NCHO, a C₆-C₁₀ aryl; a 5-10 membered heteroaryl; a C₁-C₁₀ cycloalkyl, a C₁-C₁₀ cycloheteroalkyl, 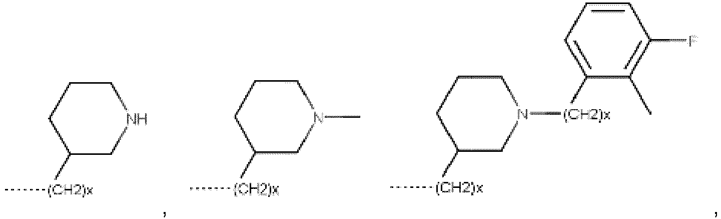 with X = 0 or 1, preferably 1; 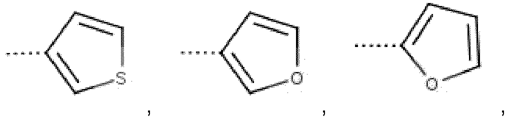 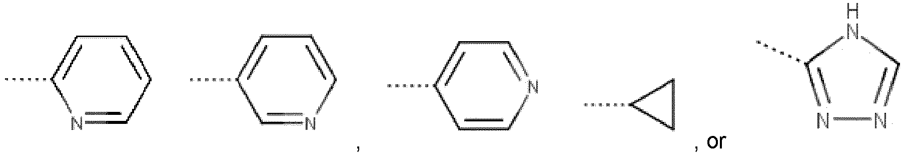 , or
   R⁵³ is 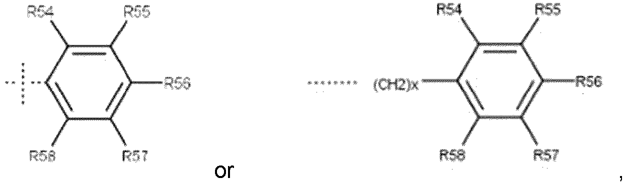 **wherein** R⁵⁴, R⁵⁵, R⁵⁶, R⁵⁷ or R⁵⁸ are selected each independently from hydrogen, -CH₃, -C₂H₅, -CH₂F; -CHF₂; -CF₃; -CH₂Cl; -CHCl₂; -CCl₃;, -OCH₃, -F, -Cl, -Br, NH₂, NO₂, and x is 0, 1 or 2;
   or R⁵³ is 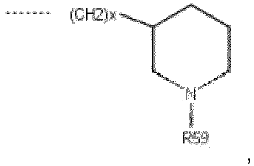 wherein R⁵⁹ is 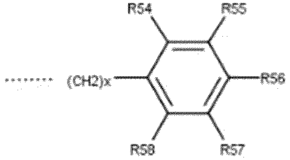 and wherein R⁵⁴, R⁵⁵, R⁵⁶, R⁵⁷ or R⁵⁸ are selected separately from the above from hydrogen, -CH₃, -C₂H₅, - CH₂F; -CHF₂; -CF₃; -CH₂Cl; -CHCl₂; -CCl₃;-OCH₃, -F, OCF₃; -Cl, -Br, NO₂, NH₂, and x is 0, 1 or 2; as defined above, and x is 0, 1 or 2;
   and wherein preferably either all of R⁵⁴ to R⁵⁸ are hydrogen or 1, 2 or 3, preferably 1 or 2 of R⁵⁴ to R⁵⁸ are different from hydrogen and selected from substitutions defined or presented herein above under e) for R⁵⁴ to R⁵⁸ and remaining positions of R⁵⁴ to R⁵⁸ are hydrogen;
   Preferably in Formula (I), (I') or (I") in case A and/or A' is a oxadiazole group as defined under e) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
f) a thiadiazole group, preferably selected from one of the following structures: 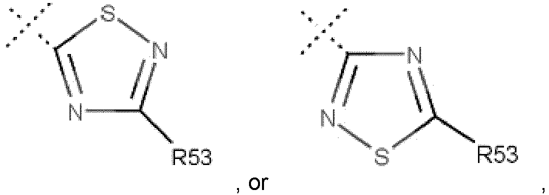 wherein R⁵³ is preferably as defined above in section e);
   Preferably in Formula (I), (I') or (I") in case A and/or A' is a thiadiazole group as defined under f) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
g) an isothiazole group, preferably selected from one of the following structures: 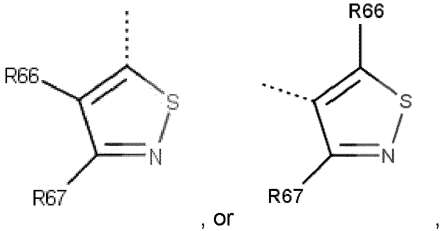 wherein R⁶⁶ and R⁶⁷ are each selected independently from hydrogen, -CH₃, -C₂H₅, -CH₂F; -CHF₂; -CF₃; -CH₂Cl; -CHCl₂; -CCl₃;-CHF₂, -F, -Cl, -Br, preferably wherein either of R⁶⁶ or R⁶⁷ is hydrogen and the other residue is selected from -CH₃, -C₂H₅, -CF₃, -CHF₂, -F, -Cl, -Br, or both of R⁶⁶ and R⁶⁷ is hydrogen;
   Preferably in Formula (I), (I') or (I") in case A and/or A' is a isothiazole group as defined under g) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
h) a thiophene group, preferably selected from one of the following structures: 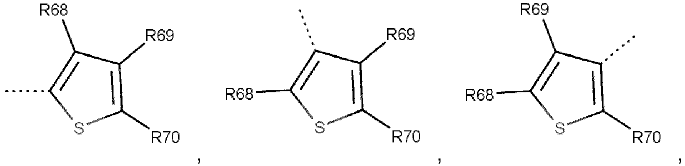 or 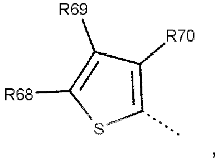 wherein R⁶⁸, R⁶⁹ and R⁷⁰ are each selected independently from hydrogen, -CH₃, -C₂H₅, - CH₂F; -CHF₂; -CF₃; -CH₂Cl; -CHCl₂; -CCl₃;-F, -Cl, -Br, preferably wherein either of R⁶⁸, R⁶⁹ or R⁷⁰ is hydrogen and the other residue is selected from -CH₃, -C₂H₅, -CF₃, -CHF₂, -F, -Cl, -Br, or each of R⁶⁸, R⁶⁹ and R⁷⁰ is hydrogen.
   Preferably in Formula (I), (I') or (I") in case A and/or A' is a thiophene group as defined under g) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
i) A is NH₂ in Formula (I), (I') or (I"), wherein A' is different to A and is selected from any of groups a) to h) as defined above.

In one embodiment, the compound of Formula (I), (I') or (I") of the invention is a compound of any one of Formulae (Ic), (Ic1), (Ic2), (Ic3), or (Ic4), or a pharmaceutically acceptable salt thereof: 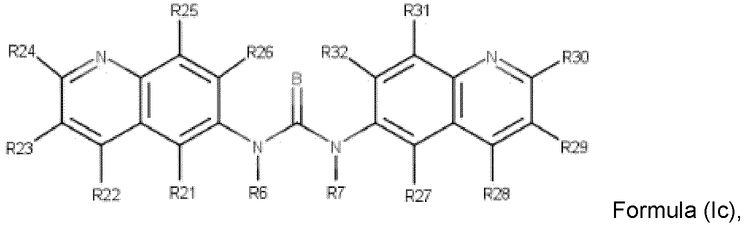 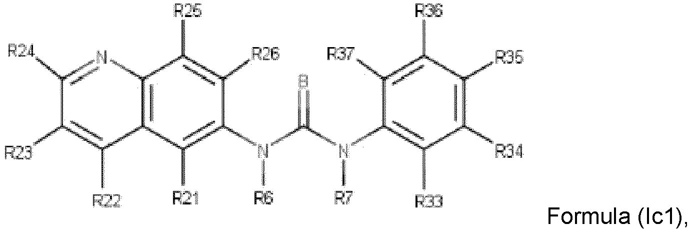 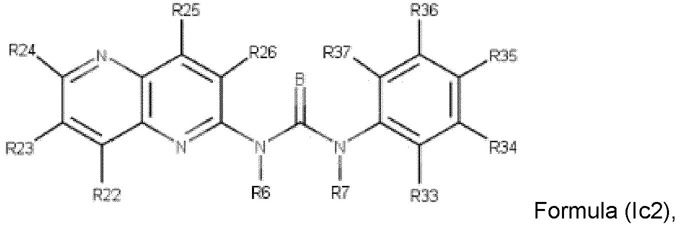 or 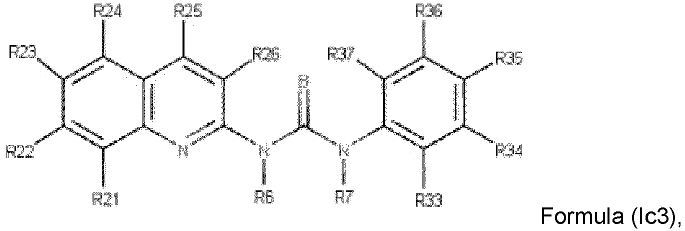 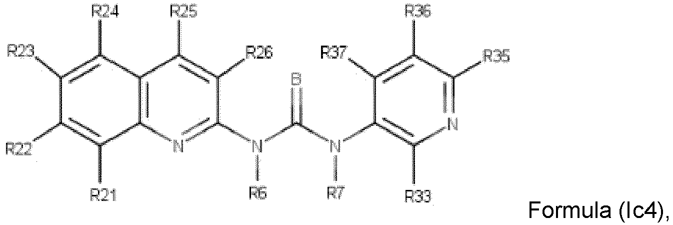
wherein B = O or S, preferably O;
wherein R⁶ and R⁷ are independently each hydrogen, C₁₋₃ alkyl, preferably hydrogen, CH₃, more preferably, either R⁶ is hydrogen and R⁷ is CH₃, or R6 and R7 are both hydrogen; and
R²¹ to R³⁷ are each independently selected from the group consisting of hydrogen, OH, - OCH₃, -OCF₃, -OCHF₂, -OCH₂OH; -OCH₂CH₃; -CH₂F; -CHF₂; -CF₃; -CH₂Cl; -CHCl₂; -CCl₃; - F; -Cl; -Br; -CN; NO₂; NH₂; C₁-C₅ alkyl, C₁-C₆ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -O-N=O, - N=O, -OH, -NH₂, -CH=NH, -N₃, -SH, -SO₃H, -SO₂H, -S(O)₂CH₃ -CN, -NC,-OCN, -NCO, - SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂ (C=O)R¹¹, (C=O)OR¹¹, (C=O)SR¹¹, (C=O)NR¹¹R¹², (SO₂)R¹¹, or C(NR¹¹)R¹², -NHC(O)R^{II}, and -NHC(O)NHR¹¹, and wherein R¹¹ and R¹² are independently selected from the group consisting of C₁-C₅ alkyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; C₂-C₁₀ alkenyl, C₃-C₂₀ cycloalkyl, C₆-C₁₈ aryl, 5-20 membered heteroaryl, 2-20 membered heteroalkyl, -CH₂F; -CHF₂, -CF₃, -CH₂Cl; -CHCl₂, and -CCl₃; or
preferably wherein R²¹ to R³⁷ are each independently selected from the group consisting of hydrogen, C₁-C₆ alkoxy, C₁-C₅ alkyl, C₁-C₅ alkyl(SO₂)C₁-C₅ alkyl, preferably -CH₂(SO₂)CH₃, - CH₂(SO₂)CH₂CH₃, -CH₂CH₂(SO₂)CH₂CH₃; -(SO₂)C₁-C₅ alkyl, preferably -(SO₂)CH₃, - (SO₂)CH₂CH₃, -CF₃, -F, -Cl, and -Br; or
wherein preferably either all of R²¹ to R³⁷ are hydrogen or 1, 2 or 3, preferably 1 or 2 of R²¹ to R³⁷ are different from hydrogen and selected from substitutions presented above for R²¹ to R³⁷ and remaining substituents of R²¹ to R³⁷ are hydrogen; or
more preferably wherein R²¹ to R³⁷ are each hydrogen.

In one more preferred embodiment, the compound of Formula (I), (I') or (I") of the invention is a compound any one of Formulae (Ic5), (Ic6), (Ic7), (Ic8), (Ic9) (Ic10), (Ic11), (Ic12), (Ic13), or (Ic14) or a pharmaceutically acceptable salt thereof: 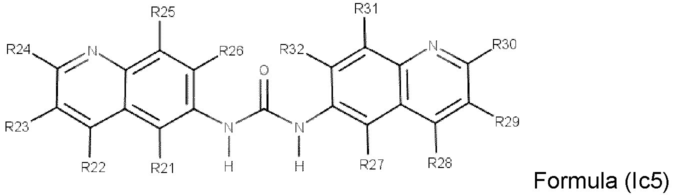 or 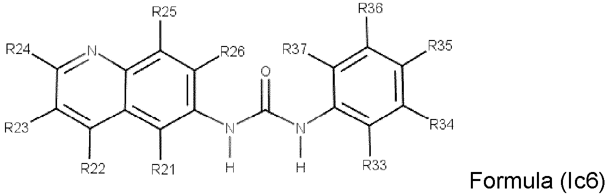 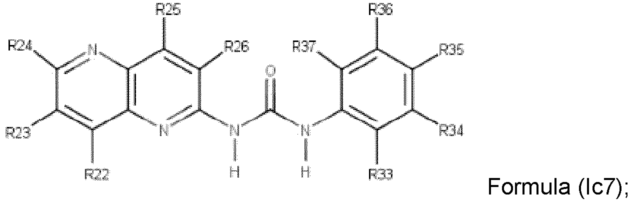 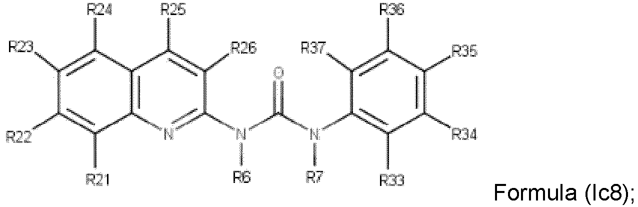 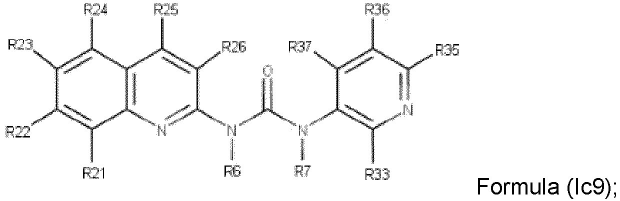 or 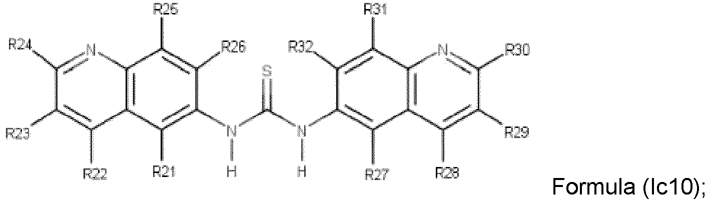 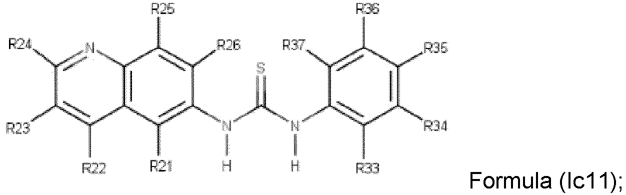 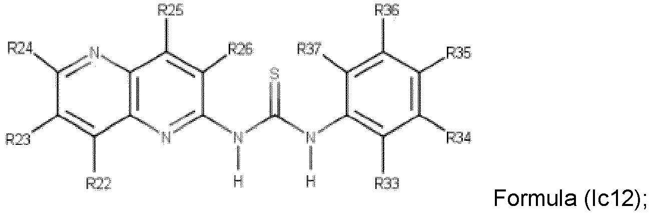 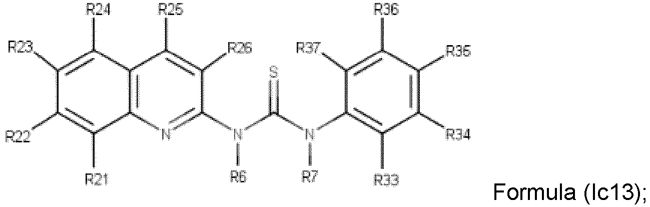 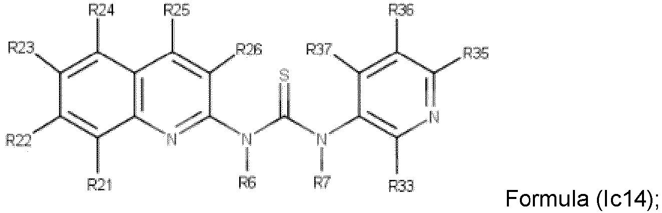
wherein R²¹ to R³⁷ are each independently selected from the group consisting of hydrogen, OH, -OCH₃, -OCF₃, -OCHF₂, -OCH₂OH; -OCH₂CH₃; -CH₂F; -CHF₂; -CF₃; -CH₂Cl; -CHCl₂; - CCl₃;-F; -Cl; -Br; -CN; NO₂; NH₂; C₁-C₆ alkoxy, C₁-C₅ alkyl, preferably -CH₃, CH(CH₃)₂; - CH₂CH₃; or C₁-C₅ alkyl(SO₂)C₁-C₅ alkyl, preferably -CH₂(SO₂)CH₃, -CH₂(SO₂)CH₂CH₃, - CH₂CH₂(SO₂)CH₂CH₃; -(SO₂)C₁-C₅ alkyl, preferably -(SO₂)CH₃, -(SO₂)CH₂CH₃, or
even more preferably R²¹ to R³⁷ are each hydrogen, or 1, 2 or 3, preferably 1 or 2 of R²¹ to R³⁷ are different from hydrogen and selected from substitutions presented above for R²¹ to R³⁷ and remaining substituents of R²¹ to R³⁷ are hydrogen.

In a second aspect the compound of Formula (I) of the invention is a compound of Formula (la) or (la1), 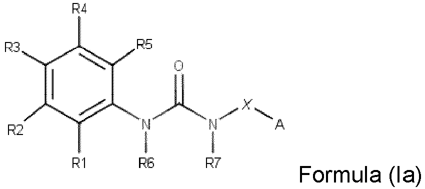 or 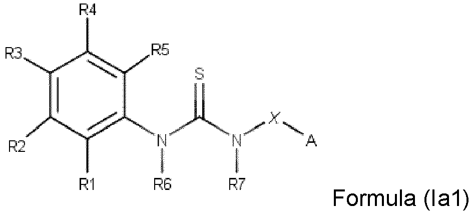 or a pharmaceutically acceceptable salt thereof, wherein:
X is either present or not, and X, if present, is preferably selected from -CH₂-, -C₂H₄-, -C₃H₆-, -CHCH(CH₃)₂-, -CHC(CH₃)₃-, -CH(CH(CH₃)₂)-, -CH(CH₂CH(CH₃)₂)-, or -CH(CH₂OH)-; and
R⁶ and R⁷ are each independently hydrogen, -CH₃, -C₂H₅, -C₃H₇, or -C₃H₉, or optionally,
R⁶ and R⁷ when taken together form a five- or six-membered heterocyclic ring, preferably selected from 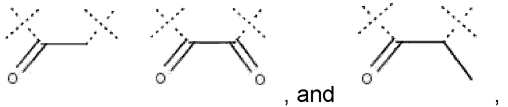 or optionally,
R⁷, N and X when taken together form a five- or six-membered heterocyclic ring, preferably selected from 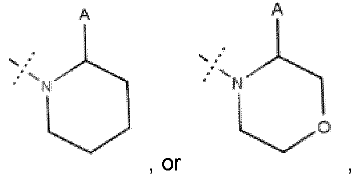 and
wherein R¹-R⁵ are each independently preferably selected as defined above, more preferably selected from the group consisting of hydrogen; -OH, -OCH₃, -OCF₃, -OCHF₂, -OCH₂OH; - OCH₂CH₃; -OCH(CH₃)₂; -F; -Cl; -Br; -CN; NO₂; NH₂; -CH₃, -CH₂F; -CHF₂; -CF₃; -CH₂Cl; - CHCl₂; -CCl₃;CH(CH₃)₂; -CH₂CH₃;
-C(O)OR⁴², wherein R⁴² is selected from -CH₃; -CH₂CH₃, -CH₂CH₂CH₃; -CH(CH₃)₂; - CH₂CH₂CH₂CH₃; 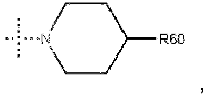 wherein R⁶⁰ is selected from hydrogen or -CH₃;
optionally, wherein R¹ and R², R² and R³, R³ and R⁴, or R⁴ and R⁵ together, preferably R¹ and R² or R² and R³ together, form a five- or six-membered cyclic or heterocyclic ring, preferably selected from

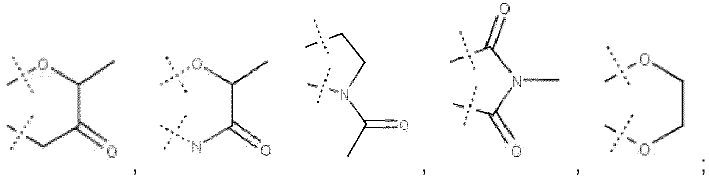

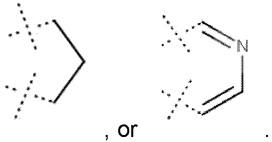

A in any of Formulae (la) or (Ia1) is preferably selected from one of the following substituents as defined above for A or A', more preferably from A as defined below in sections a) to e):
A may be preferably selected for any of Formulae (la) or (la1) from the following groups a) to e):
a) a benzene group of the following structure: 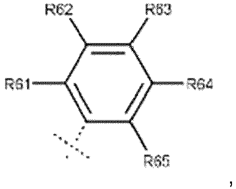 wherein R⁶¹-R⁶⁶ are each independently selected from the group consisting of:
   hydrogen; -OH, -OCH₃, -OCF₃, -OCHF₂, -OCH₂OH; -OCH₂CH₃; -OCH(CH₃)₂; -F; -Cl; -Br; - CN; NO₂; NH₂; -CH₃, -CH₂F; -CHF₂; -CF₃; -CH₂Cl; -CHCl₂; -CCl₃;CH(CH₃)₂; -CH₂CH₃; -CH₂-CH₂-CH(CH₃)₂; -CH₂-N(CH₃)₂; -CH(CH₃)-NHC(O)CH₃; -C(O)-C₂H₅; -C(O)-CH₃; -C(O)-aziridine; -C(O)-azetidine; -C(O)-pyrrolidine; -CH₂NHS(O)₂CH₃; -C(CH₃)=NOH; -S(O)₂-cyclopentyl; -S(O)₂-CH₃; -S(O)₂-cyclopentyl; pyrrolidinyl, 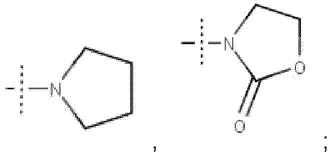 or -NHC(O)-R⁴¹, wherein R₄₁ is selected from C₁-C₁₀ cycloalkyl, C₁-C₁₀ cycloheteroalkyl, furan, tetrahydrofuran, cyclopentyl, cyclobutyl, cyclopropyl, -NH-CH(CH₃)₂, -NH-cyclopropyl, -CHF₂, -CH₂F; -CHF₂; -CF₃; -CH₂Cl; -CHCl₂; -CCl₃;, -CH₃, CH₂CH₃, CHCH(CH₃)₂; or
   -C(O)OR⁴², wherein R₄₂ is selected from C₁-C₅ alkyl, including -CH₃; -CH₂CH₃, -CH₂CH₂CH₃; -CH(CH₃)₂; -CH₂CH₂CH₂CH₃; or
   -NHR⁴³, wherein R₄₃ is selected from 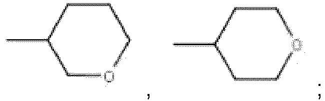 or 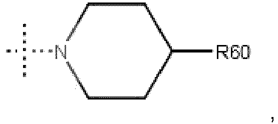
   wherein R⁶⁰ is selected from hydrogen or -CH₃; or 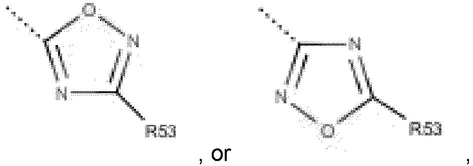
   wherein R⁵³ is preferably as defined above selected from the group consisting of hydrogen, OH, -OCH₃, -OCF₃, -OCHF₂, -OCH₂OH; - OCH₂CH₃; -F; -Cl; -Br; -CN; NO₂; NH₂; C₁-C₅ alkyl, C₁-C₅ alkoxy, C₂-C₅ alkenyl, preferably - CH₃, CH(CH₃)₂; -CH₂CH₃; -CH₂-CH₂-CH(CH₃)₂; -CH₂-N(CH₃)₂; -CH(CH₃)-NHC(O)CH₃; -CHF₂, -CH₂F; -CHF₂; -CF₃; -CH₂Cl; -CHCl₂; -CCl₃; -O-N=O, -N=O, -CH=NH, -N₃, -SH, -SO₃H, - SO₂H, -S(O)₂CH₃ -CN, -NC,-OCN, -NCO, -(C=O)OH, -(C=O)NHOH, -NH(C=O)NH₂, - 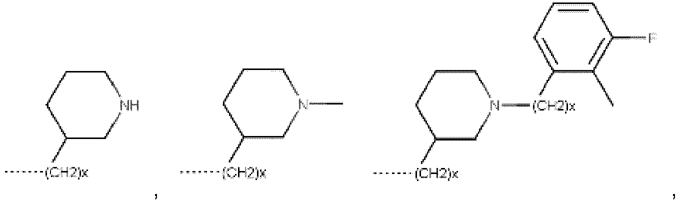 with X = 0 or 1, preferably 1; , 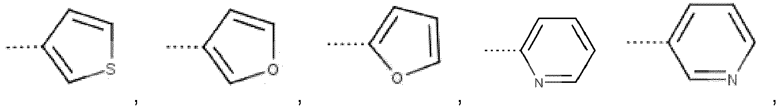 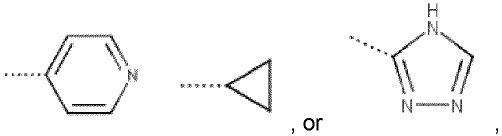
   or R⁵³ is 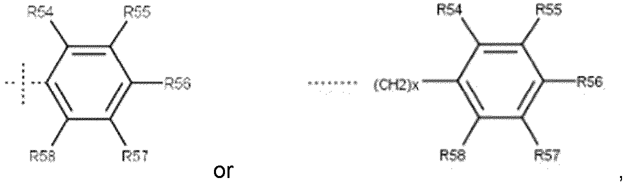 wherein R⁵⁴, R⁵⁵, R⁵⁶, R⁵⁷ or R⁵⁸ are selected each independently from hydrogen, -CH₃, -C₂H₅, -CH₂F; -CHF₂; - CF₃; -CH₂Cl; -CHCl₂; -CCl₃;- -OCH₃, -F, -Cl, -Br, NO₂, NH₂, and x is 0, 1 or 2; or
   or R⁵³ is 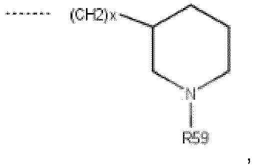 wherein R⁵⁹ is 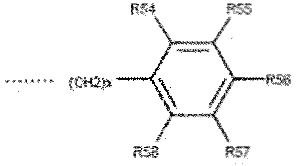 and wherein R⁵⁴, R⁵⁵, R⁵⁶, R⁵⁷ or R⁵⁸ are selected separately from the above from hydrogen, - CH₃, -C₂H₅, -CH₂F; -CHF₂; -CF₃; -CH₂Cl; -CHCl₂; -CCl₃;-OCH₃, -F, -Cl, -Br, NO₂, NH₂, and x is 0, 1 or 2; as defined above, and x is 0, 1 or 2;
      and
   optionally, wherein R⁶¹ and R⁶², R⁶² and R⁶³, R⁶³ and R⁶⁴, or R⁶⁴ and R⁶⁵ together, preferably R⁶² and R⁶³ together, form a five- or six-membered cyclic or heterocyclic ring, preferably selected from 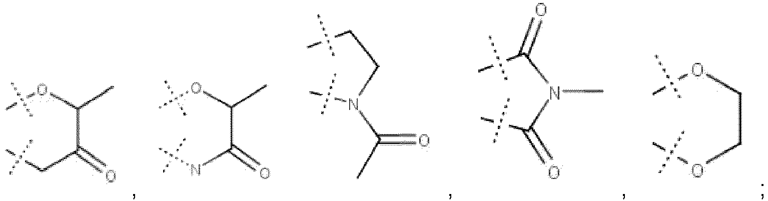 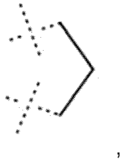
   wherein preferably either all of R¹ to R⁵ are hydrogen or 1, 2 or 3, preferably 1 or 2 of R¹ to R⁵ are different from hydrogen and selected from substitutions defined above for R¹ to R⁵ and remaining substituents of R¹ to R⁵ are hydrogen;
   Likewise preferably, X is then not present in Formula (la) and (la1).
b) an oxadiazole group of one of the following structures: 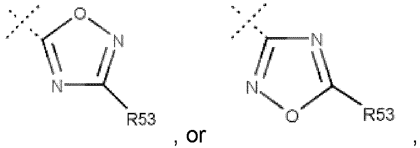 wherein
   R⁵³ is preferably selected as above from the group consisting of hydrogen, OH, -OCH₃, - OCF₃, -OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; - CH(CH₃)-NHC(O)CH₃; -CH₂F; -CHF₂; -CF₃; -CH₂Cl; -CHCl₂; -CCl₃; -O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, - NH(C=O)NH₂, -NH(C=S)NH₂, -(C=O)NH₂,-CHO,-CHS, -OCHO, -NCHO, - 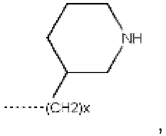 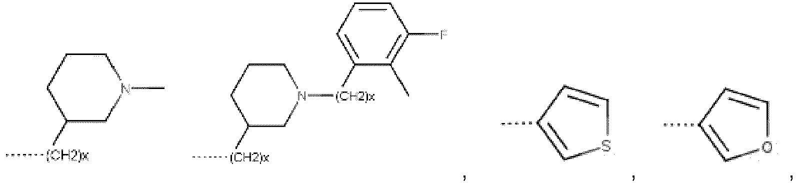 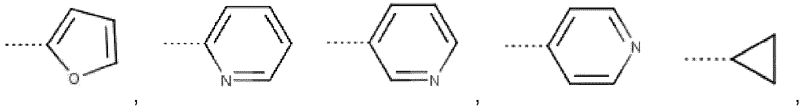 or 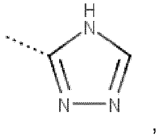 or
   R⁵³ is 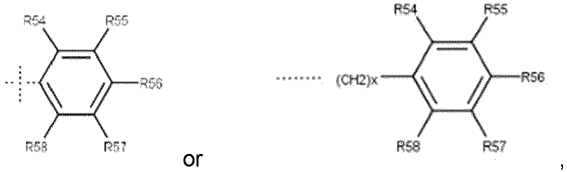 wherein R⁵⁴, R⁵⁵, R⁵⁶, R⁵⁷ or R⁵⁸ are selected each independently from hydrogen, -CH₃, -C₂H₅, -CH₂F; -CHF₂; -CF₃; - CH₂Cl; -CHCl₂; -CCl₃;, -OCH₃, -F, -Cl, -Br, NO₂, NH₂, and x is 0, 1 or 2;
   or R⁵³ is 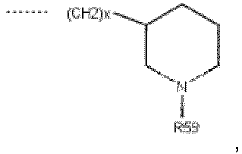 wherein R⁵⁹ is 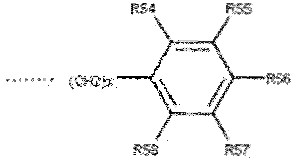 and wherein R⁵⁴, R⁵⁵, R⁵⁶, R⁵⁷ or R⁵⁸ are selected separately from the above from hydrogen, -CH₃, -C₂H₅, -CH₂F; - CHF₂; -CF₃; -CH₂Cl; -CHCl₂; -CCl₃; -OCH₃, -F, -Cl, -Br, NO₂, NH₂, and x is 0, 1 or 2; as defined above, and x is 0, 1 or 2;
   and wherein preferably either all of R⁵⁴ to R⁵⁸ are hydrogen or 1, 2 or 3, preferably 1 or 2 of R⁵⁴ to R⁵⁸ are different from hydrogen and selected from substitutions presented above and remaining positions of R⁵⁴ to R⁵⁸ are selected from hydrogen;
   Preferably, X is then selected in Formula (la) or (la1), from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
c) a thiadiazole group of one of the following structures: 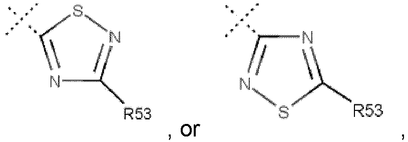
   wherein R⁵³ is preferably as defined herein above.
   Preferably, X is then selected in formula (Ia) or (la1), from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
d) an isothiazole group of the following structure: 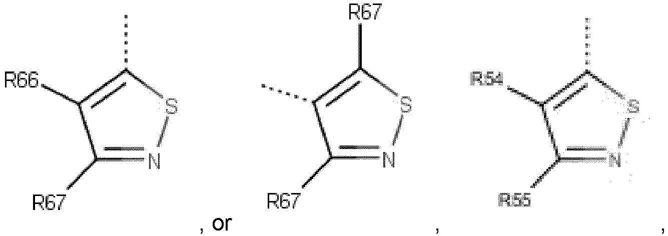 wherein R⁶⁶ and R⁶⁷ are each selected independently from hydrogen, -CH₃, -C₂H₅, -CH₂F; -CHF₂; -CF₃; -CH₂Cl; - CHCl₂; -CCl₃;-F, -Cl, -Br.
   Preferably, X is then selected in Formula (la) or (la1), from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
e) a thiophene group, preferablyselected from one of 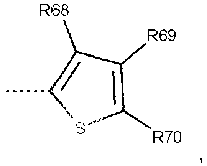 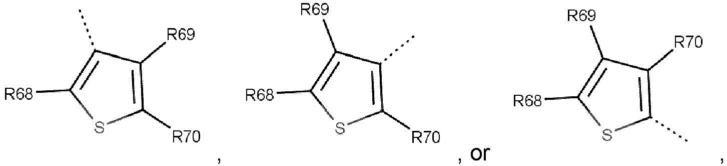 wherein R⁶⁸, R⁶⁹ and R⁷⁰ are each selected independently from hydrogen, -CH₃, -C₂H₅, -CH₂F; -CHF₂; -CF₃; - CH₂Cl; -CHCl₂; -CCl₃;-F, -Cl, -Br, preferably wherein either of R⁶⁸, R⁶⁹ or R⁷⁰ is hydrogen and the other residue is selected from -CH₃, -C₂H₅, -CF₃, -CHF₂, -F, -Cl, -Br, and preferably X is then selected in Formula (la) or (la1), from -CH₂-, -C₂H₄-, -C₃H₆-, -CHCH(CH₃)₂-, - CHC(CH₃)₃-, -CH(CH(CH₃)₂)-, -CH(CH₂CH(CH₃)₂)-, or -CH(CH₂OH)-.

According to one particular aspect, in Formula (la) or Formula (la1) A is preferably a C₆-C₁₈ aryl as defined above, and more preferably the following aryl/benzene group: 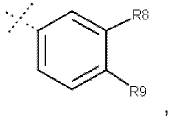
and X is present or not present, preferably not present;
or A is a five- or six-membered heterocyclic ring as defined above, and more preferably A is an oxadiazole group of onne of the foillowing structures: 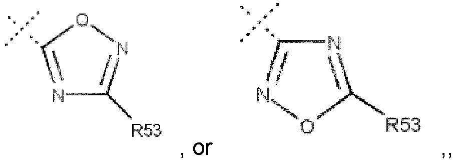
   wherein R⁵³ is preferably as defined herein,
   wherein X is preferably selected in formula (Ia) or (la1), from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-,
   and, in either case R¹, R², R³, R⁴, R⁵, R⁸ and R⁹ are each independently selected from hydrogen, -F, -Cl, -Br, -NO₂,-O-N=O, -N=O, -OH, -NH₂, -CH=NH, -N₃, -SH, -SO₃H, -SO₂H, - S(O)₂CH₃ -CN, -NC,-OCN, -NCO, -SCN, -NCS, -(P=O)(OH)₂, -O(P=O)(OH)₂, -(C=O)OH, - (C=O)NHOH, -NH(C=O)NH₂, -NH(C=S)NH₂, -(C=O)NH₂,-CHO,-CHS, -OCHO, -NCHO, - B(OH)₂, C₁-C₁₀ alkyl, C₁-C₁₀ alkoxy, C₂-C₁₀ alkenyl, C₃-C₂₀ cycloalkyl, C₃-C₂₀ cycloalkyl C₁-C₁₀ alkyl, 3-20 membered cycloheteroalkyl or 3-20 membered cycloheteroalkyl C₁-C₁₀ alkyl, C₆-C₁₈ aryl, C₆-C₁₈ aryl C₁-C₁₀ alkyl, 5-20 membered heteroaryl, 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹, wherein R¹¹ and R¹² are independently selected from the group consisting of C₁-C₁₀ alkyl, C₂-C₁₀ alkenyl, C₃-C₂₀ cycloalkyl, C₆-C₁₈ aryl, 5-20 membered heteroaryl, 2-20 membered heteroalkyl, - CH₂F; -CHF₂, -CF₃, -CH₂Cl; -CHCl₂, and -CCl₃; or
   R¹ and R², R² and R³, R³ and R⁴, R⁴ and R⁵, or R⁸ and R⁹, when taken together form a five- or six-membered cycloalkyl, five- or six-membered cycloheteroalkyl, five- or six-membered aryl, or five- or six-membered heteroaryl, and
   R⁶ and R⁷ are each independently hydrogen, a C₁-C₁₀ alkyl or R⁶ and R⁷ when taken together form a five- or six-membered heterocyclic ring.

According to one further aspect, in Formula (la) or Formula (la1) R¹, R², R³, R⁴ and R⁵ are each hydrogen and/or A is 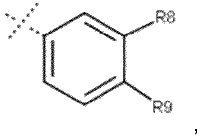 and R⁸ and R⁹ are each independently hydrogen, substituted C₁-C₁₀ alkyl, substituted 2-10 membered heteroalkyl, substituted C₂-C₁₄ aryl, substituted 5-20 membered heteroaryl, substituted five- or six-membered cycloheteroalkyl, -S(O)₂CH₃, -F, -Cl, -Br, -NHC(O)R¹¹, or -NHC(O)NHR¹¹, wherein R¹¹ is as defined above selected from C₁-C₅ alkyl, C₃-C₆ cycloalkyl, -CH₂F; -CHF₂; -CF₃; -CH₂Cl; -CHCl₂; -CCl₃; or five-membered heterocyclic ring; -OH, -OCH₃, -OCF₃, -OCHF₂, -OCH₂OH; -OCH₂CH₃; -OCH(CH₃)₂; -CN; NO₂; NH₂; -CH₃, CH(CH₃)₂; -CH₂CH₃; -CH₂-CH₂-CH(CH₃)₂; -CH₂-N(CH₃)₂; - CH(CH₃)-NHC(O)CH₃; - -NH-CH(CH₃)₂, -NH-cyclopropyl, CH₂CH₃, CHCH(CH₃)₂; -C(O)-C₂H₅; -C(O)-CH₃; -C(O)-aziridine; -C(O)-azetidine; -C(O)-pyrrolidine; -C(CH₃)=NOH; - CH₂NHS(O)₂CH₃; -S(O)₂-cyclopentyl; -S(O)₂-CH₃; -S(O)₂-cyclopentyl; pyrrolidinyl, 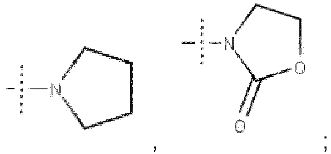 or
NHC(O)-R⁴¹, wherein R⁴¹ is selected from furan, tetrahydrofuran, cyclopentyl, cyclobutyl, cyclopropyl, -NH-CH(CH₃)₂, -NH-cyclopropyl, C₁-C₅ alkyl, -CHF₂, -CH₂F; -CHF₂; -CF₃; -CH₂Cl; -CHCl₂; -CCl₃; -CH₃, CH₂CH₃, CHCH(CH₃)₂; or
-C(O)OR⁴², wherein R⁴² is selected from -CH₃; -CH₂CH₃, -CH₂CH₂CH₃; -CH(CH₃)₂; - CH₂CH₂CH₂CH₃; or
-NHR⁴³, wherein R⁴³ is selected from 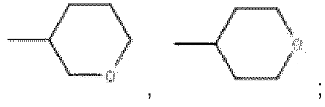 or 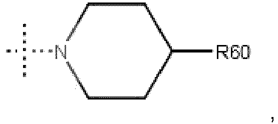
wherein R⁶⁰ is selected from hydrogen or -CH₃; or 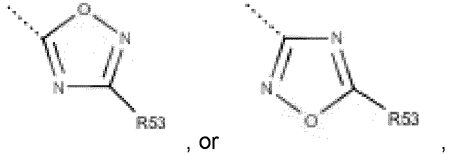
wherein
R⁵³ is selected from the group consisting of hydrogen, OH, -OCH₃, -OCF₃, -OCHF₂, - OCH₂OH; -OCH₂CH₃; -F; -Cl; -Br; -CN; NO₂; NH₂; -CH₃, CH(CH₃)₂; -CH₂CH₃; -CH₂-CH₂-CH(CH₃)₂; -CH₂-N(CH₃)₂; -CH(CH₃)-NHC(O)CH₃; -CH₂F; -CHF₂; -CF₃; -CH₂Cl; -CHCl₂; - CCl₃;-O-N=O, -N=O, -CH=NH, -N₃, -SH, -SO₃H, -SO₂H, -S(O)₂CH₃ -CN, -NC,-OCN, -NCO, - (C=O)OH, -(C=O)NHOH, -NH(C=O)NH₂, -NH(C=S)NH₂, -(C=O)NH₂,-CHO,-CHS, -OCHO, - NCHO, 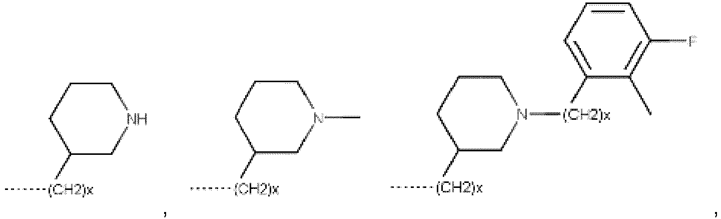 with X = 0 or 1, preferably 1; 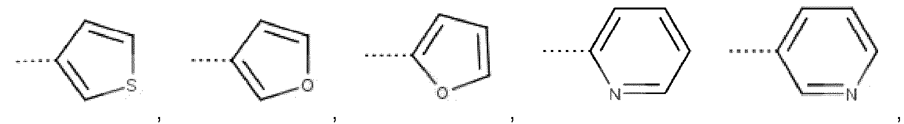 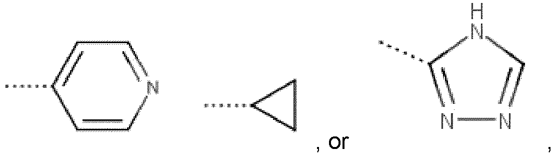 or
R⁵³ is 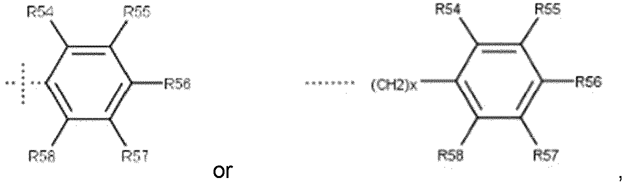 wherein R⁵⁴, R⁵⁵, R⁵⁶, R⁵⁷ or R⁵⁸ are selected each independently from hydrogen, -CH₃, -C₂H₅, -CH₂F; -CHF₂; -CF₃; -CH₂Cl; -CHCl₂; -CCl₃;-OCH₃, -F, -Cl, -Br, NH₂, NO₂, and x is 0, 1 or 2;
or R⁵³ is 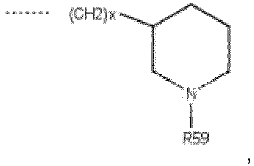 wherein R⁵⁹ is 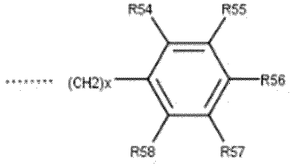 and wherein R⁵⁴, R⁵⁵, R⁵⁶, R⁵⁷ or R⁵⁸ are selected separately from the above from hydrogen, -CH₃, -C₂H₅, - CH₂F; -CHF₂; -CF₃; -CH₂Cl; -CHCl₂; -CCl₃; -OCH₃, -F, -Cl, -Br, NO₂, NH₂, and x is 0, 1 or 2; as defined above;
with the optional proviso that R⁸ is not identical to R⁹, or optionally
R⁸ and R⁹, when taken together form a five- or six-membered cycloalkyl, five- or six-membered cycloheteroalkyl or five or six-membered aryl, five- or six-membered heteroaryl, preferably the heterocyclic ring is selected from:

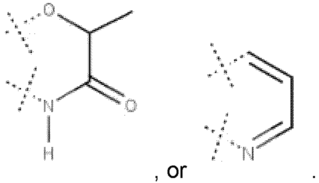

According to one further aspect, in the compound according to Formulae (la) and (la1) R⁸ and R⁹ are each independently defined as above, with the proviso that if one of R⁸ or R⁹ is -F, -Cl, or -Br, the other R⁸ or R⁹ is neither -F, -Cl, -Br nor hydrogen.

According to one further aspect, in the compound according to Formulae (la) and (la1) R⁸ and R⁹ are each independently defined as above, with the proviso that R⁸ or R⁹ are not identical to each other.

Preferably in another aspect according to Formulae (la) and (la1), in the compound according to Formulae (Ia) and (Ia1), R⁹ is selected from a C₁-C₃ alkyl, a five-membered heterocyclic ring, -OCH₃, -S(O)₂CH₃, -NHC(O)R¹¹, or -NHC(O)NHR¹¹, wherein R¹¹ is selected from C₂-C₅ alkyl, C₃-C₆ cycloalkyl, -CH₂F; -CHF₂; -CF₃; -CH₂Cl; -CHCl₂; -CCl₃;-CHCl₂, -CCl₃; or five-membered heterocyclic ring; and/or
R⁸ is -S(O)₂CH₃, -NHC(O)R¹¹, or -NHC(O)NHR¹¹, and wherein R¹¹ is selected from C₂-C₅ alkyl, C₃-C₅ cycloalkyl, -CHF₂; or five-membered heterocyclic ring;
or R⁸ and R⁹, when taken together form a heterocyclic ring selected from:

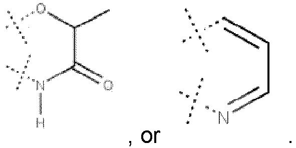

According one specific aspect the -X-A group in any of Formulae (la) or (la1) is 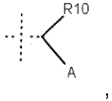 wherein R¹⁰ is hydrogen or X is a C₁-C₃ alkyl, preferably -CH (CH₃)₂ -CH₂-, -C₂H₄-, -C₃H₆-, - CHCH(CH₃)₂-, -CHC(CH₃)₃-, -CH(CH(CH₃)₂)-, or -CH(CH₂CH(CH₃)₂)-, or -CH(CH₂OH)-, or - (CH₂)ₙ-A, wherein n is an integer from 1-3, preferably 1, and/or A is 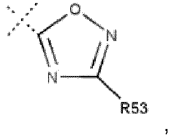 preferably forming Formula (Ib) or (Ib1) as shown below):
According one specific aspect of the invention the compound according to Formulae (la) and (la1) is a compond according to Formula (Ib) or (Ib1): 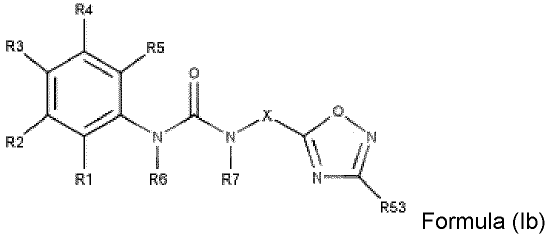 or 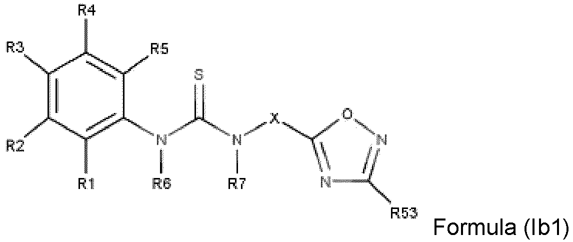
wherein X is preferably a C₁-C₃ alkyl, preferably -CH (CH₃)₂ -CH₂-, -C₂H₄-, -C₃H₆-, - CHCH(CH₃)₂-, -CHC(CH₃)₃-, -CH(CH(CH₃)₂)-, or -CH(CH₂CH(CH₃)₂)-, or -CH(CH₂OH)-,
wherein R⁵³ is preferably hydrogen, OH, -OCH₃, -OCF₃, -OCHF₂, -OCH₂OH; -OCH₂CH₃; -F; - Cl; -Br; -CN; NO₂; NH₂; C₁-C₅ alkyl, C₁-C₅ alkoxy, C₂-C₅ alkenyl, preferably -CH₃, CH(CH₃)₂ - CH₂CH₃; -CH₂-CH₂-CH(CH₃)₂; -CH₂-N(CH₃)₂; -CH(CH₃)-NHC(O)CH₃; -CH₂F; -CHF₂; -CF₃; - CH₂Cl; -CHCl₂; -CCl₃; -O-N=O, -N=O, -CH=NH, -N₃, -SH, -SO₃H, -SO₂H, -S(O)₂CH₃ -CN, - NC,-OCN, -NCO, -(C=O)OH, -(C=O)NHOH, -NH(C=O)NH₂, -NH(C=S)NH₂, -(C=O)NH₂,-CHO,-CHS, -OCHO, -NCHO, 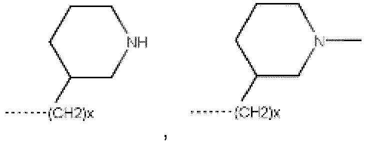 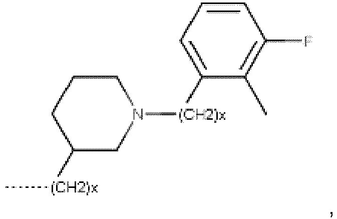 with X = 0 or 1, preferably 1; 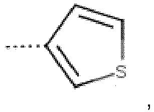 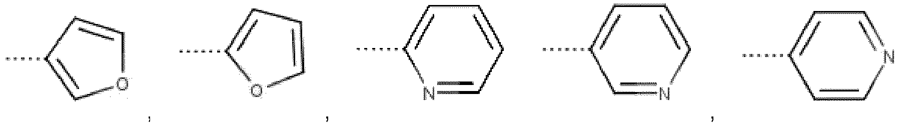 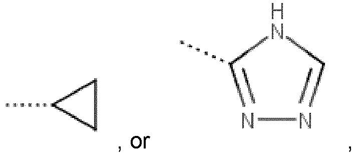 or
R⁵³ is 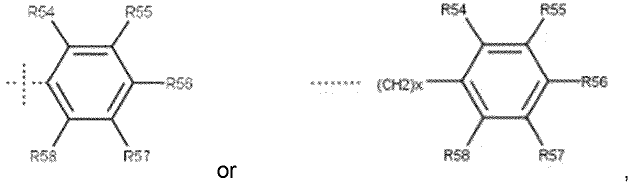 wherein R⁵⁴, R⁵⁵, R⁵⁶, R⁵⁷ or R⁵⁸ are selected each independently from hydrogen, -CH₃, -C₂H₅, -CH₂F; -CHF₂; -CF₃; -CH₂Cl; -CHCl₂; -CCl₃;-OCH₃, -F, -Cl, -Br, NH₂, NO₂, and x is 0, 1 or 2;
or R⁵³ is 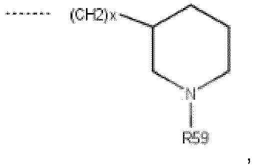 wherein R⁵⁹ is 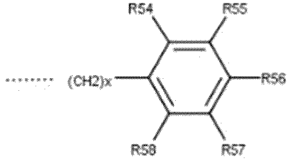 and wherein R⁵⁴, R⁵⁵, R⁵⁶, R⁵⁷ or R⁵⁸ are selected separately from the above from hydrogen, - CH₃, -C₂H₅, -CH₂F; -CHF₂; -CF₃; -CH₂Cl; -CHCl₂; -CCl₃;-OCH₃, -F, -Cl, -Br, NO₂, NH₂, and x is 0, 1 or 2; as defined above, and x is 0, 1 or 2;
and wherein preferably either all of R⁵⁴ to R⁵⁸ are hydrogen or 1, 2 or 3, preferably 1 or 2 of R⁵⁴ to R⁵⁸ are different from hydrogen and selected from substitutions presented above and remaining positions of R⁵⁴ to R⁵⁸ are selected from hydrogen.

According to a further aspect of the present invention the compound according to Formulae (la) and (la1) is a compond according Formula (Id): 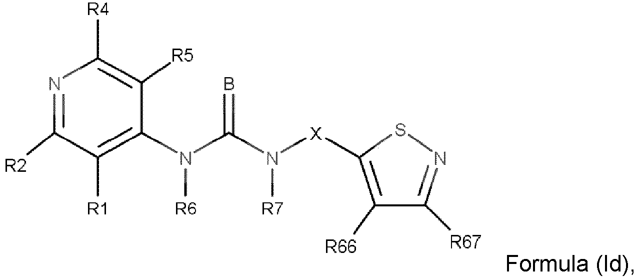 wherein preferably
B is O or S, preferably O,
R¹, R², R⁴ and R⁵ are each selected separately from hydrogen, -CH₃, -C₂H₅, -CH₂CH₃, - CH₂F; -CHF₂; -CF₃; -CH₂Cl; -CHCl₂; -CCl₃;-OCH₃, -F, -Cl, -Br, NO₂, NH₂, preferably wherein two or three of R¹, R², R⁴ and R⁵ are hydrogen and the remaining of R¹, R², R⁴ and R⁵ are not hydrogen; and
R⁶⁶ and R⁶⁷ are each selected separately from hydrogen, -CH₃, -C₂H₅, -CH₂F; -CHF₂; -CF₃; - CH₂Cl; -CHCl₂; -CCl₃;-OCH₃, -F, -Cl, -Br, NO₂, NH₂, preferably wherein one of R⁶⁶ and R⁶⁷ is hydrogen and the other is one of -CH₃, -C₂H₅, -CF₃, -CHF₂, -OCH₃, -F, -Cl, -Br, NO₂, NH₂.

Described herein is also a compound of Formula (II),

R²¹-D-CO-R²² Formula (II)

or a pharmaceutically acceceptable salt thereof, wherein:
D is an optionanlly substituted heteroaromatic bicycle selected from the group comprising 2-benzofuran, 2-benzothiophene, isoindol, indol, indolizine, and
wherein D is preferably indolizine 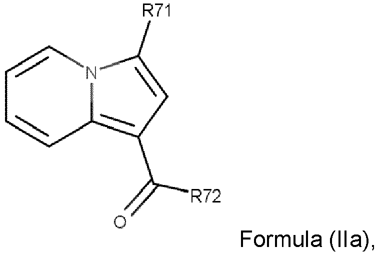
R⁷¹ is an optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, and
R⁷² is optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, -OR⁷³, -NR⁷³, and wherein
R⁷³ is hydrogen, optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl.

The present invention relates to the present compounds for use in the prevention or treatment of a disease by activating the NLRP3 inflammasome, wherein the disease is selected from a group comprising infectious diseases or cancer.

Described herein is also a pharmaceutical composition comprising a compound of the present invention and one or more pharmaceutically acceptable excipients.

Moreover, herein described is a method for modulating NLRP3 activity, the method comprising engaging NLRP3 with a compound of the present invention.

The method of treating cancer may comprise administering to a subject in need of such treatment an effective amount of a compound of the present invention, or a pharmaceutical composition of the present invention

Additionally, herein described is a method for analyzing the mechanisms of NLRP3 activation.

According to one aspect, the present invention relates to the use of the compounds of the present invention for boosting immune responses or breaking immune evasion in cancer diseases in a subject.

According to one aspect, the present invention relates to the use of the compounds of the present invention for boosting immune responses as an adjuvant in a vaccine in a subject. Preferably, the compounds of the present invention may be used as an adjuvant in the context of vaccination to boost specific immune responses by activating the NLRP3 inflammasome.

### Brief Description of the Drawings

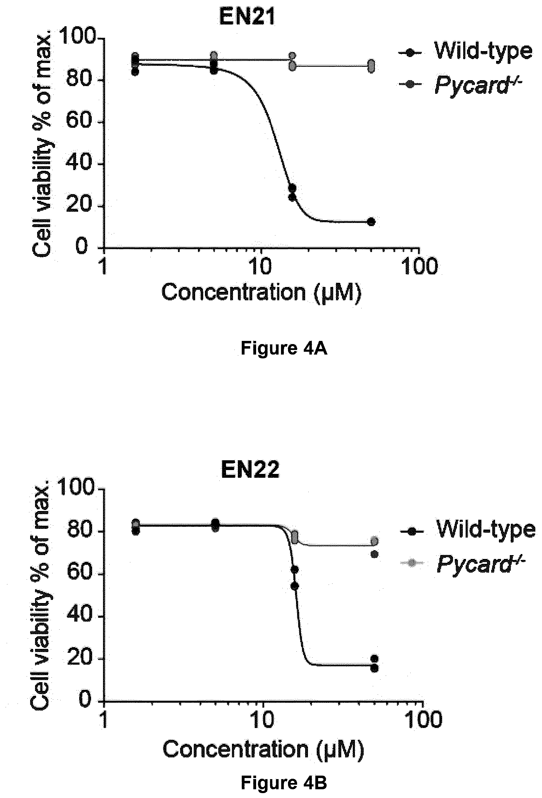

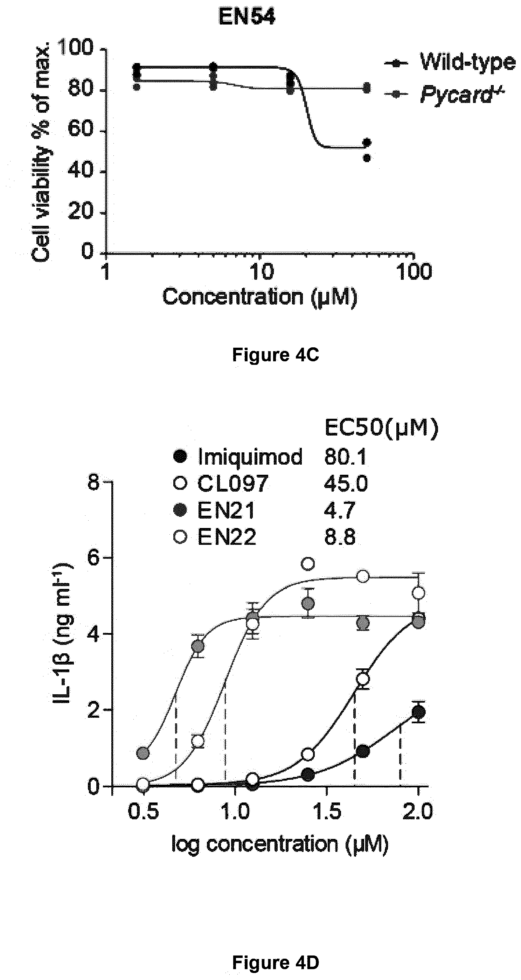

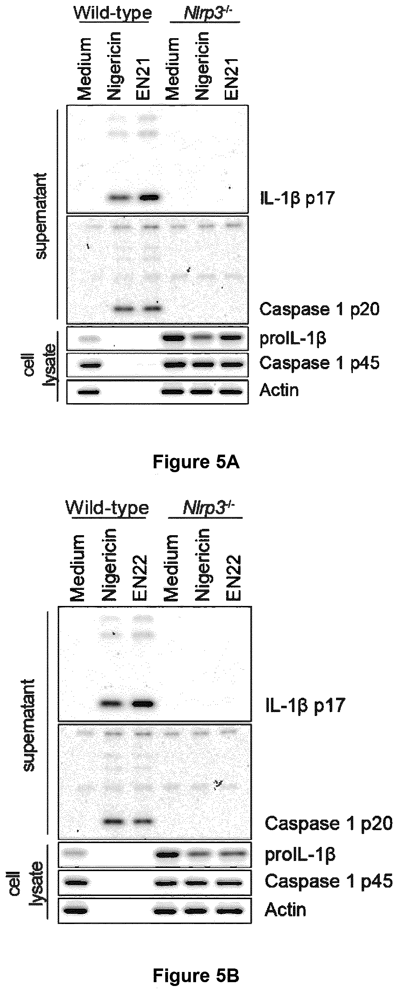

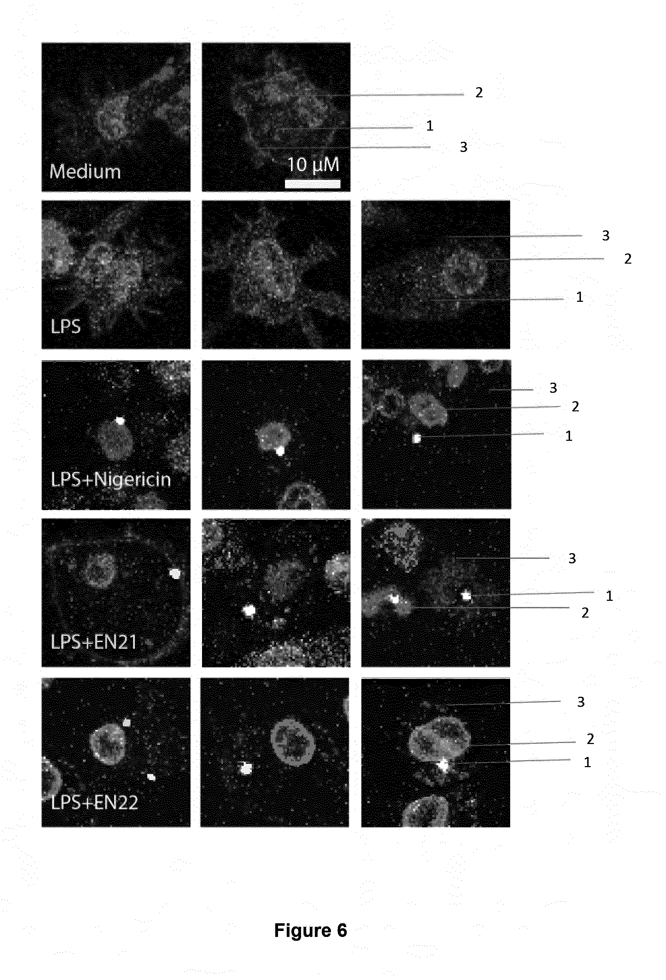

**Figure 1****:** Line diagram of the high-throughput screening of about 50 000 low molecular weight compounds. The diagram shows a partial data set for 39 440 compounds tested at 25 µM of the compounds after about 20 hours of incubation in LPS-primed murine bone marrow-derived dendritic cells (BMDCs). Primary identification of cell death inducing compounds.
**Figure 2****:** Dot diagram of the selection of NLRP3 activators for which cell death is dependent on the inflammasome adapter ASC (*Pycard*^{-/-}) in LPS-primed BMDCs after 16 hours incubation at 50 µM of the compounds, the framed dots represent the respective replicates of the tested compounds EN21, EN22 and EN54.
**Figure 3****:** Dot diagram of the selection of activators, which lead to the release of IL-1β as an indicator of inflammasome activation (from BMDCs after 20 hours incubation at 50 µM of the compounds). The cells were each pretreated for 3 hours with 50 ng ml⁻¹ LPS to prime for inflammasome activation.
**Figure 4A****:** Line diagram of the dose dependent cytotoxicity of compound EN21 in wild-type and inflammasome knockout (*Pycard*^{-/-}) BMDCs. Cytotoxicity was assessed by quantification of cellular ATP levels.
**Figure 4B****:** Line diagram of the dose dependent cytotoxicity of compound EN22 in wild-type and inflammasome knockout (*Pycard*^{-/-}) BMDCs. Cytotoxicity was assessed by quantification of cellular ATP levels.
**Figure 4C****:** Line diagram of the dose dependent cytotoxicity of compound EN54 in wild-type and inflammasome knockout (*Pycard*^{-/-}) BMDCs. Cytotoxicity was assessed by quantification of cellular ATP levels.
**Figure 4D****:** Line diagram of the EC₅₀ for induction of IL-1β release from BMDCs for compounds EN21 and EN22 in comparison to the imidazoquinoline NLRP3 activators imiquimod and CL097. The presence of IL-1β in the cell culture supernatant was determined by enzyme-linked immunosorbent assay (ELISA) using a kit selective for the mature form of the cytokine.
**Figure 5A****:** Immunoblot analysis of the effects of compound EN21. Caspase-1 activation and release of the biologically active form of IL-1β in BMDCs after treatment with the present compounds at 50 µM for 3 hours. The cells were pretreated with 50 ng ml⁻¹ LPS.
**Figure 5B****:** Immunoblot analysis of the effects of compound EN22. Caspase-1 activation and release of the biologically active form of IL-1β in BMDCs after treatment with the present compounds at 50 µM for 3 hours. The cells were pretreated with 50 ng ml⁻¹ LPS.
**Figure 6****:** Images of ASC speck formation in BMDCs of ASC^{citrine} mice expressing a fluorescent fusion protein of the inflammasome adapter ASC. ASC speck formation can be used as a readout for inflammasome activation. ASC 1 (green/bright grey, ASC specks green/bright grey spots), nuclei **2** (blue/dark grey, bigger round structure), CD45 3 (red/dark grey, small spots). The cells were first treated with 50 ng ml⁻¹ LPS for 3 hours, then with 25 µM of the activators for 2 hours.
   The present compounds induce typical characteristics of inflammasome activation, including the activation of caspase-1 and the release of the biologically active forms of leaderless proinflammatory cytokines of the IL-1 family such as IL-1β (Figures 5A and B). Moreover, inflammasome-dependent lytic cell death (pyroptosis, Figure 4) and ASC speck formation are induced by the present compounds (Figure 6). For the induction a concentration dependence can be observed. An advantage of the present compounds is that they are active *in vitro* at approximately 10-fold more lower concentrations than established imidazoquinolines.

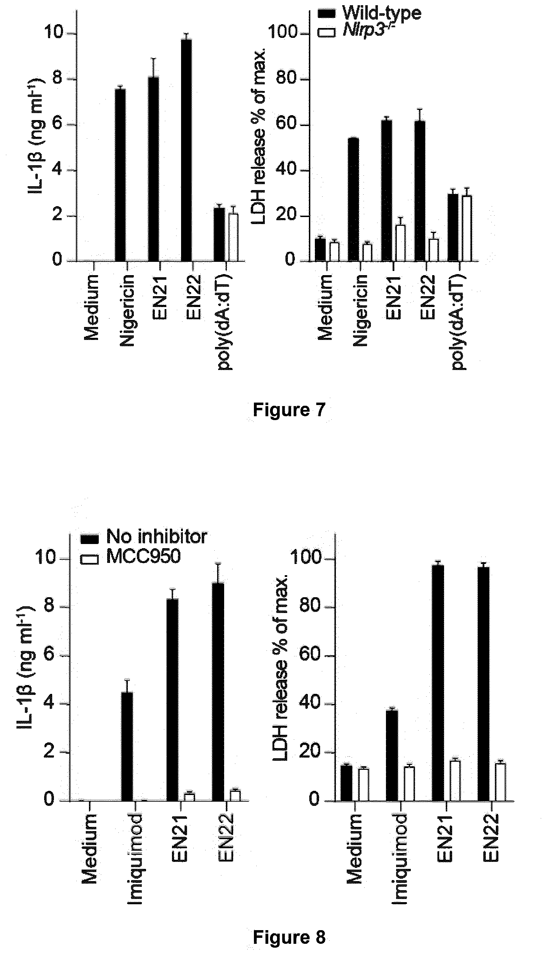

**Figure 7****:** Bar diagram of IL-1β and lactate dehydrogenase release (as marker for lytic cell death and pyroptosis) from BMDCs from wild-type or NLRP3 knockout mice. The cells were each pretreated with 50 ng ml⁻¹ LPS and stimulated with 50 µM EN21, EN22 or control activators.
**Figure 8****:** Bar diagram of IL-1β and lactate dehydrogenase release from BMDCs from wild-type mice with or without pre-incubation with the NLRP3-specific inhibitor MCC950. The cells were each pretreated with 50 ng ml⁻¹ LPS and stimulated with 50 µM EN21, EN22 or control activators.
   Without wishing to be bound to a theory, it is assumed that the compounds EN21 and EN22 activate the NLRP3 inflammasome, since the observed characteristics of inflammasome activation do not occur in cells isolated from NLRP3 knockout mice (Figure 7) and, in addition, the NLRP3-specific inhibitor MCC950 completely suppresses inflammasome activation (Figure 8).
   A further advantage of the present compounds is that the present compounds seem to activate NLRP3 rapidly. Especially with EN22, the inflammasome activation in BMDCs from wild-type mice without inhibitor is almost maximum after only 30 minutes.

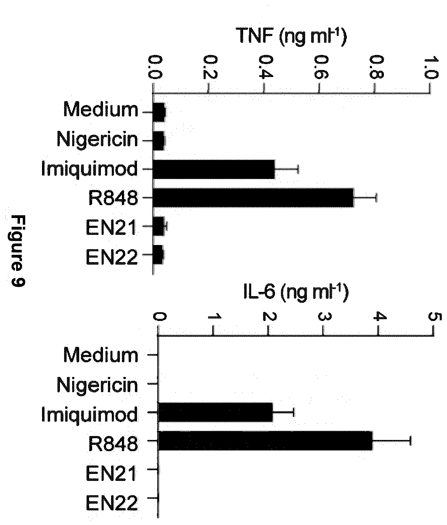

**Figure 9****:** Bar diagram of TNF and IL-6 release of BMDCs as readout for toll-like receptor activation. The cells were stimulated with 50 µM EN21, EN22 or control activators.
   In contrast to the small molecule the NLRP3 inflammasome activators imiquimod (patent US 2019/0127368 A1), the present compounds are more specific, since no simultaneous activation of Toll-like receptors (TLR7/ TLR8) occurs and thus the present compounds do not induce the release of further proinflammatory cytokines such as TNF or IL-6.

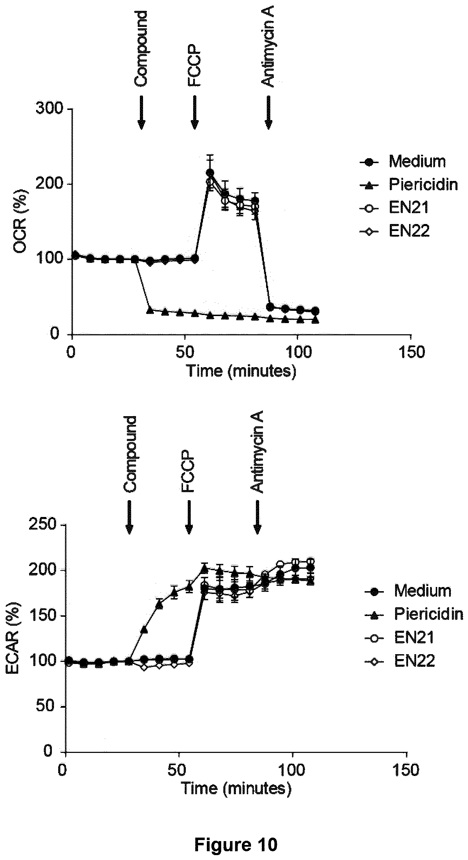

**Figure 10****:** Line diagram of extracellular flux analysis in BMDCs of inflammasome knockout mice. Upper panel: Oxygen consumption rate (OCR) from the medium, which shows the mitochondrial respiration over time. Lower panel: Acidification of the medium, which shows glycolytic activity of the cells over time (extracellular acidification rate, ECAR). The cells were treated with 50 µM EN21, EN22 or 10 µM Piericidin as a control. The mitochondrial uncoupler FCCP was used to prove responsiveness of the cells and therefore functionality of the assay.
   In contrast to other known small molecule NLRP3 activators, the present compounds do not inhibit mitochondrial respiration and are therefore less susceptible to unspecific undesired effects.

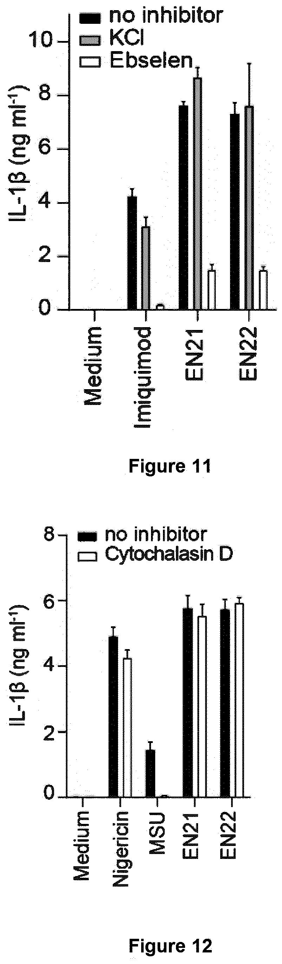

**Figure 11****:** Bar diagram of IL-1β release from BMDCs of wild-type mice either left without inhibitor or pretreated with 50 mM extracellular KCI to inhibit potassium efflux from the cells, or with 30 µM ebselen used as a ROS scavenger. The cells were pretreated with 50 ng ml⁻¹ LPS and stimulated with 50 µM EN21, EN22 or control activators.
   Most of the known NLRP3 activators lead to substantial potassium efflux from the cell via different mechanisms, which precedes inflammasome activation. Without wishing to be bound to a theory, it is assumed that the present compounds such as EN21 and EN22 activate the NLRP3 inflammasome independently of potassium efflux from the cell. It is assumed that reactive oxygen species (ROS) are involved in the mechanism of action for NLRP3 activation of the present compounds, since the ROS scavenger ebselen can inhibit inflammasome activation. It might be also possible that ROS influences a further mechanism that is required for NLRP3 activation in parallel to the mechanism activated by EN21 and/or EN22.
**Figure 12****:** Bar diagram of IL-1β release from LPS-primed BMDCs from wild-type mice either left without inhibitor or pretreated with the endocytosis inhibitor cytochalasin D. The cells were stimulated with either 50 µM EN21, EN22 or control activators.
   The endocytosis inhibitor cytochalasin D prevents MSU-induced NLRP3 activation, but has no effect on NLRP3 activation by EN21 and EN22. Without wishing to be bound to a theory, it is assumed that contrary to particulate NLRP3 activators such as uric acid crystals (mono sodium urate, MSU) or aluminum hydroxide, the present compounds are not endocyted by immune cells as a prerequisite for the NLRP3 inflammasome activation.

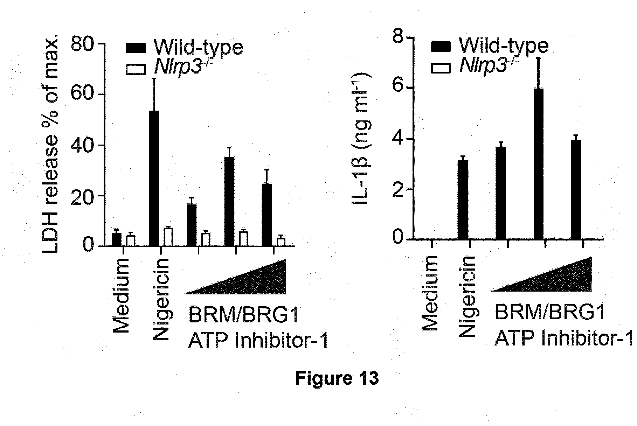

**Figure 13****:** Bar diagram of IL-1β and lactate dehydrogenase release (as marker for lytic cell death and pyroptosis) from BMDCs from wild-type or NLRP3 knockout mice The cells were each pretreated with 50 ng ml⁻¹ LPS and stimulated with 5 µM, 50 µM, 100 µM BRM/BRG1 ATP Inhibitor-1 (concentrations increasing from left to right, indicated by the wedge below). or control activators.

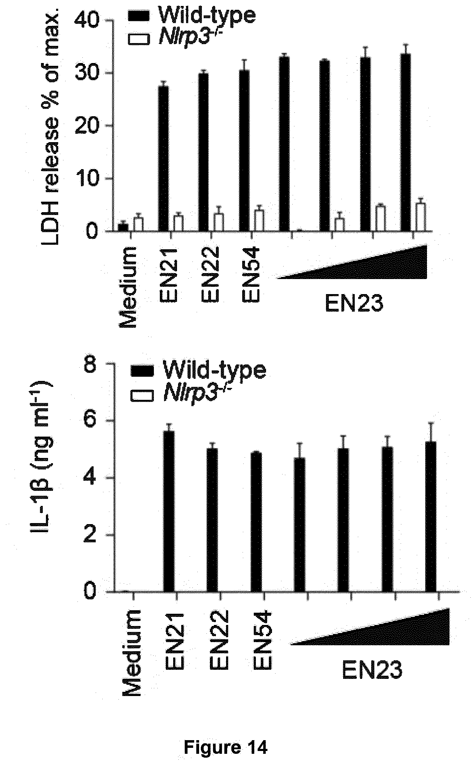

**Figure 14****:** Bar diagram of IL-1β (lower figure) and lactate dehydrogenase (upper figure) release of BMDCs from wild-type and NLRP3 knockout mice. The cells were primed with 50 ng ml⁻¹ LPS for 3 hours and then stimulated with the indicated compounds for 3 hours as follows: EN21 12.5 µM, EN22 25 µM, EN54 50 µM, EN23 25 µM, 50 µM, 100 µM, 200 µM (concentrations increasing from left to right, indicated by the wedge below).

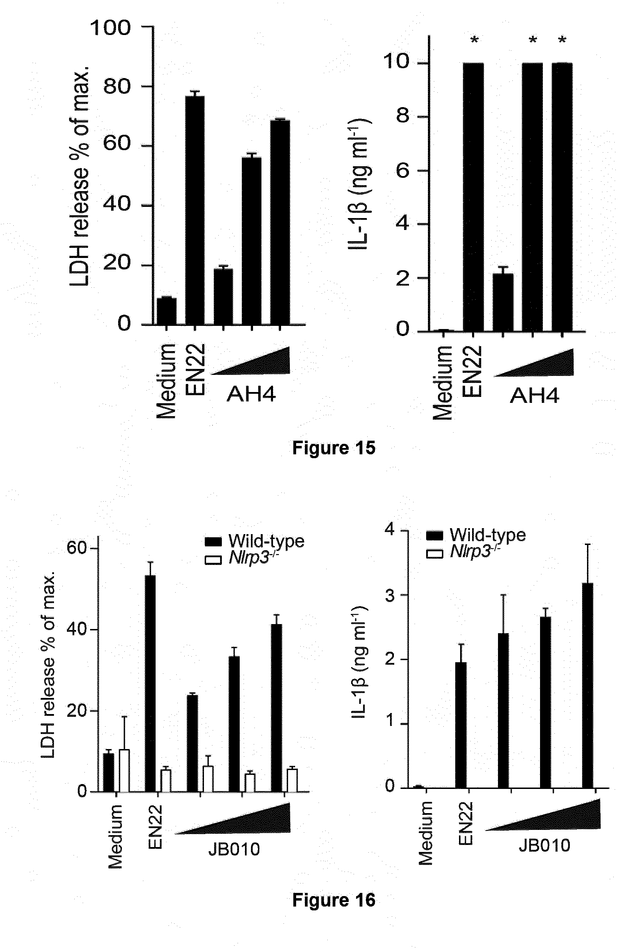

**Figure 15****:** Bar diagram of IL-1β and lactate dehydrogenase release (as marker for lytic cell death and pyroptosis) from BMDCs from wild-type mice. The cells were each pretreated with 50 ng ml⁻¹ LPS and stimulated with 25 µM, 50 µM, 75 µM AH4 (concentrations increasing from left to right, indicated by the wedge below) or 10 µM EN22.
**Figure 16****:** Bar diagram of IL-1β (lower figure) and lactate dehydrogenase (upper figure) release of BMDCs from wild-type and NLRP3 knockout mice. The cells were primed with 50 ng ml⁻¹ LPS for 3 hours and then stimulated with the indicated compounds for 3 hours as follows: EN22 20 µM, JB010 20 µM, 40 µM, 80 µM (concentrations increasing from left to right, indicated by the wedge below).
   Without wishing to be bound to a theory, it is assumed that the compounds BRM/BRG1 ATP Inhibitor-1,EN23, AH4 and JB010 act similarly as EN21 and EN22 activates the NLRP3 inflammasome, since the observed characteristics of inflammasome activation do not occur in cells isolated from NLRP3 knockout mice (Figures 7, 13, 14 and 16).

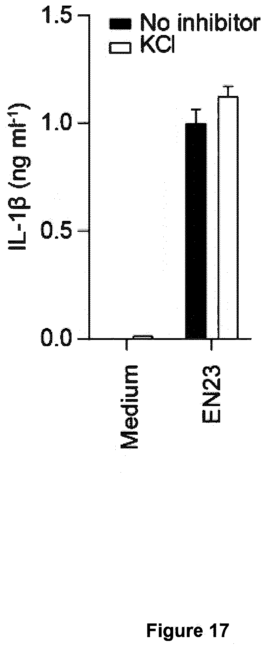

**Figure 17****:** Bar diagram of IL-1β release of wild-type BMDCs. The cells were primed with 50 ng ml⁻¹ LPS for 3 hours, incubated with 50 mM extracellular KCI to prevent potassium efflux from the cells for 30 minutes and then stimulated with 12.5 µM EN23 for 3 hours.
   Without wishing to be bound to a theory, it is assumed that the present compounds such as EN23 activate the NLRP3 inflammasome independently of potassium efflux from the cell. (Figure 17)

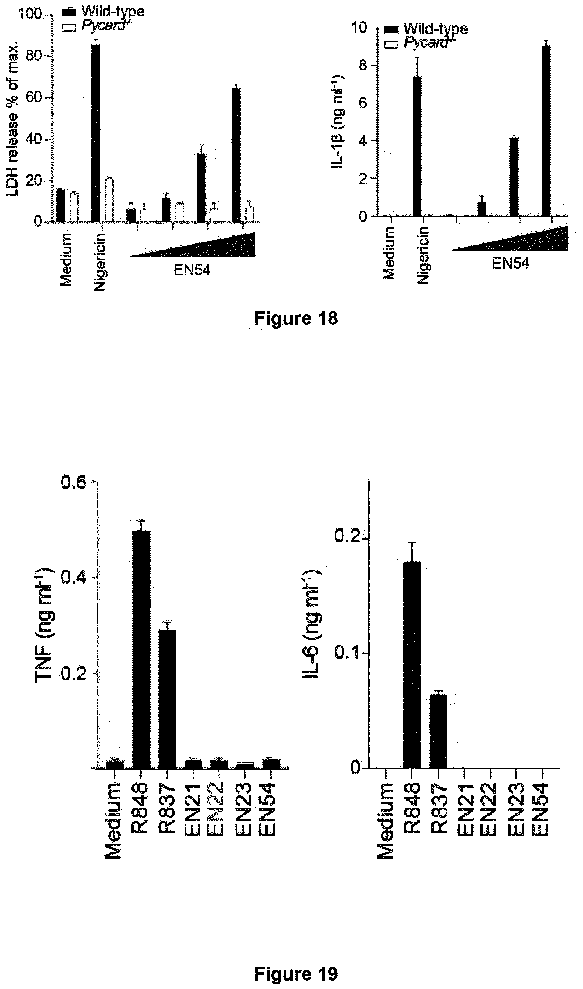

**Figure 18****:** Bar diagram of IL-1β and lactate dehydrogenase release of BMDCs from wild-type and ASC knockout (*Pycard*^{-/-}) mice. The cells were primed with 50 ng ml⁻¹ LPS for 3 hours and then stimulated with the indicated compounds for 3 hours as follows: nigericin 5 µM, EN54 10 µM, 20 µM, 40 µM, 80 µM (concentrations increasing from left to right, indicated by the wedge below).
   Without wishing to be bound to a theory, it is assumed that the compound EN54 acts similarly as EN21 and EN22 activates the NLRP3 inflammasome, since the observed characteristics of inflammasome activation do not occur in cells isolated from NLRP3 knockout mice (Figure 18).
**Figure 19****:** Bar diagram of TNF and IL-6 release of BMDCs as readout for toll-like receptor activation.

### Detailed description of the invention

The terms "a" and "an" do not denote a limitation of quantity, but rather denote the presence of at least one of the referenced item. The term "or" or "and/or" is used as a function word to indicate that two words or expressions are to be taken together or individually. The terms "comprising", "having", "including", and "containing" are to be construed as open-ended terms (i.e., meaning "including, but not limited to"). The endpoints of all ranges directed to the same component or property are inclusive and independently combinable.

The term "present compound(s)" or "compound(s) of the present invention" refers to compounds encompassed by a structural formula disclosed herein, such as Formulae (I), (I'), (I"), (Ia), (Ia1), (Ib), (Ib1), (1c), (1c1), (1c2), (1c3), (1c4), (1c5), (1c6), (1c7), (1c8), (1c9), (1c10), (1c11), (1c12), (1c13), (1c14), (Id), (II), (IIa), and (IIb) and includes any subgenus and specific compounds within the formula whose structure is disclosed herein. Compounds may be identified either by their chemical structure and/or chemical name. When the chemical structure and chemical name conflict, the chemical structure is determinative of the identity of the compound. The compounds described herein may contain one or more chiral centers and/or double bonds and therefore, may exist as stereoisomers, such as double-bond isomers (*i.e.,* geometric isomers), enantiomers or diastereomers. Accordingly, the chemical structures depicted herein encompass all possible enantiomers and stereoisomers of the illustrated compounds including the stereoisomerically pure form (*e.g*., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the skilled artisan. The compounds may also exist in several tautomeric forms including the enol form, the keto form and mixtures thereof. Accordingly, the chemical structures depicted herein encompass all possible tautomeric forms of the illustrated compounds. The compounds described also include isotopically labeled compounds where one or more atoms have an atomic mass different from the atomic mass conventionally found in nature. Examples of isotopes that may be incorporated into the compounds of the invention include, but are not limited to, ²H, ³H, ¹³C, ¹⁴C, ¹⁵N, ¹⁸O, ¹⁷O, *etc.* Compounds may exist in unsolvated forms as well as solvated forms, including hydrated forms and as N-oxides. In general, compounds may be hydrated, solvated or N-oxides. Certain compounds may exist in multiple crystalline or amorphous forms. In general, all physical forms are equivalent for the uses contemplated herein and are intended to be within the scope of the present invention. Further, it should be understood, when partial structures of the compounds are illustrated, that brackets indicate the point of attachment of the partial structure to the rest of the molecule. The term "tautomer" as used herein refers to isomers that change into one another with great ease so that they can exist together in equilibrium.

"Alkyl," by itself or as part of another substituent, refers to a saturated branched, or straight-chain hydrocarbon radical derived by the removal of one hydrogen atom from a single carbon atom of a parent alkane. Typical alkyl groups include, but are not limited to, methyl; ethyl; propyls such as propan-1-yl, propan-2-yl (isopropyl), *etc.;* butanyls such as butan-1-yl, butan-2-yl (*sec*-butyl), 2-methyl-propan-1-yl (isobutyl), 2-methyl-propan-2-yl (*t*-butyl); and the like. In some embodiments, an alkyl group comprises from 1 to 20 carbon atoms (C₁-C₂₀ alkyl). In other embodiments, an alkyl group comprises from 1 to 10 carbon atoms (C₁-C₁₀ alkyl). In still other embodiments, an alkyl group comprises from 1 to 6 carbon atoms (C₁-C₆ alkyl) or 1 to 4 carbon atoms (C₁-C₄ alkyl). In still other embodiments, an alkyl group comprises from 1 to 3 carbon atoms (C₁-C₃ alkyl). C₁-C₆ alkyl is also known as "lower alkyl".

"Cycloalkyl," by itself or as part of another substituent, refers to a saturated cycloalkyl hydrocarbon radical derived by the removal of one hydrogen atom from a single carbon atom of a parent cycloalkane. Cycloalkyl further includes cycloalkenyls as defined herein. Typical cycloalkyl groups include, but are not limited to, cyclopropyl, cyclobutyl, cyclopentyl, cyclohexyl; cyclopropenyl, cyclobutenyl, cyclopentenyl, cyclohexenyl; cyclobutadienyl, cyclohexadienyl; and the like. In some embodiments, a cycloalkyl group comprises from 3 to 20 carbon atoms (C₃-C₂₀ cycloalkyl). In other embodiments, a cycloalkyl group comprises from 3 to 10 carbon atoms (C₃-C₁₀ cycloalkyl). In a preferred embodiment, a cycloalkyl group comprises from 3 to 6 carbon atoms (C₃-C₆ cycloalkyl). In a more preferred embodiment, a cycloalkyl group comprises from 3 to 5 carbon atoms (C₃-C₅ cycloalkyl).

"Cycloalkyl alkyl," by itself or as part of another substituent, refers to an acyclic alkyl group in which one of the hydrogen atoms bonded to a carbon atom, typically a terminal or *sp*³ carbon atom, is replaced with a cycloalkyl group as defined herein. That is, cycloalkyl alkyl can also be considered as an alkyl substituted by cycloalkyl. Typical cycloalkyl alkyl groups include, but are not limited to, cyclopropylmethyl, 2-cyclopropylethan-1-yl, 2-cyclopropylethen-1-yl, pentylmethyl, 2-pentylethan-1-yl, 2-pentylethen-1-yl, and the like. Where specific alkyl moieties are intended, the nomenclature cycloalkyl alkanyl, cycloalkyl alkenyl and/or cycloalkyl alkynyl is used. In some embodiments, a cycloalkyl alkyl group is (C₃-C₃₀) cycloalkyl alkyl, *e.g.,* the alkanyl, alkenyl or alkynyl moiety of the cycloalkyl alkyl group is (C₁-C₁₀) alkyl and the cycloalkyl moiety is (C₃-C₂₀) cycloalkyl. In other embodiments, a cycloalkyl alkyl group is (C₄-C₂₀) cycloalkyl alkyl, *e.g.,* the alkanyl, alkenyl or alkynyl moiety of the cycloalkyl alkyl group is (C₁-C₈) alkyl and the cycloalkyl moiety is (C₃-C₁₂) cycloalkyl. In still other embodiments, a cycloalkyl alkyl group is (C₄-C₁₀) cycloalkyl alkyl, *e.g.,* the alkanyl, alkenyl or alkynyl moiety of the cycloalkyl alkyl group is (C₁-C₄) alkyl and the cycloalkyl moiety is (C₃-C₆) cycloalkyl.

It is noted that when an alkyl group is further connected to another atom, it becomes an "alkylene" group. In other words, the term "alkylene" refers to a divalent alkyl. For example, - CH₂CH₃ is an ethyl, while -CH₂CH₂- is an ethylene. That is, "Alkylene," by itself or as part of another substituent, refers to a saturated or unsaturated, branched, straight-chain or cyclic divalent hydrocarbon radical derived by the removal of two hydrogen atoms from a single carbon atom or two different carbon atoms of a parent alkane, alkene or alkyne. The term "alkylene" is specifically intended to include groups having any degree or level of saturation, *i.e.,* groups having exclusively single carbon-carbon bonds, groups having one or more double carbon-carbon bonds, groups having one or more triple carbon-carbon bonds and groups having mixtures of single, double and triple carbon-carbon bonds. In some embodiments, an alkylene group comprises from 1 to 20 carbon atoms (C₁-C₂₀ alkylene). In other embodiments, an alkylene group comprises from 1 to 10 carbon atoms (C₁-C₁₀ alkylene). In still other embodiments, an alkylene group comprises from 1 to 6 carbon atoms (C₁-C₆ alkylene).

"Alkenyl," by itself or as part of another substituent, refers to an unsaturated branched or straight-chain monovalent hydrocarbon radical having at least one carbon-carbon double bond derived by the removal of one hydrogen atom from a single carbon atom of a parent alkene. The group may be in either the *cis* or *trans* conformation about the double bond(s). Typical alkenyl groups include, but are not limited to, ethenyl; propenyls such as prop-1-en-1-yl, prop-1-en-2-yl, prop-2-en-1-yl (allyl), prop-2-en-2-yl, butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, buta-1,3-dien-2-yl, *etc.;* and the like. In some embodiments, an alkenyl group comprises from 2 to 20 carbon atoms (C₂-C₂₀ alkenyl). In other embodiments, an alkenyl group comprises from 2 to 10 carbon atoms (C₂-C₁₀ alkenyl). In still other embodiments, an alkenyl group comprises from 2 to 6 carbon atoms (C₂-C₆ alkenyl) or 1 to 4 carbon atoms (C₂-C₄ alkenyl). C₂-C₆ alkenyl is also known as "lower alkenyl".

"Cycloalkenyl," by itself or as part of another substituent, refers to an unsaturated cycloalkenyl hydrocarbon radical derived by the removal of one hydrogen atom from a single carbon atom of a parent cycloalkene. Typical cycloalkenyl groups include, but are not limited to, cyclopropenyls such as clycloprop-1-en-1-yl, cycloprop-2-en-1-yl, cyclobutenyls such as cyclobut-1-en-1-yl, cyclobut-1-en-3-yl, cyclobuta-1,3-dien-1-yl, *etc.;* and the like. In some embodiments, a cycloalkenyl group comprises from 3 to 20 carbon atoms (C₃-C₂₀ cycloalkenyl). In other embodiments, a cycloalkenyl group comprises from 3 to 10 carbon atoms (C₃-C₁₀ cycloalkenyl). In a preferred embodiment, a cycloalkenyl group comprises from 3 to 6 carbon atoms (C₃-C₆ cycloalkenyl). In a more preferred embodiment, a cycloalkenyl group comprises from 3 to 5 carbon atoms (C₃-C₅ cycloalkenyl).

"Cycloalkenyl alkyl," by itself or as part of another substituent, refers to an acyclic alkyl group in which one of the hydrogen atoms bonded to a carbon atom, typically a terminal or *sp*³ carbon atom, is replaced with a cycloalkenyl group as defined herein. That is, cycloalkenyl alkyl can also be considered as an alkyl substituted by cycloalkenyl. Typical cycloalkenyl alkyl groups include, but are not limited to, cyclopropenylmethyl, 2-cyclopropenylethan-1-yl, 2-cyclopropenylethen-1-yl, pentylmethyl, 2-pentenylethan-1-yl, 2-pentenylethen-1-yl, and the like. Where specific alkyl moieties are intended, the nomenclature cycloalkenyl alkanyl, cycloalkenyl alkenyl and/or cycloalkenyl alkynyl is used. In some embodiments, a cycloalkenyl alkyl group is (C₃-C₃₀) cycloalkenyl alkyl, *e.g.,* the alkanyl, alkenyl or alkynyl moiety of the cycloalkenyl alkyl group is (C₁-C₁₀) alkyl and the cycloalkenyl moiety is (C₃-C₂₀) cycloalkenyl. In other embodiments, a cycloalkenyl alkyl group is (C₄-C₂₀) cycloalkenyl alkyl, *e.g.,* the alkanyl, alkenyl or alkynyl moiety of the cycloalkenyl alkyl group is (C₁-C₈) alkyl and the cycloalkenyl moiety is (C₃-C₁₂) cycloalkenyl. In still other embodiments, a cycloalkenyl alkyl group is (C₄-C₁₀) cycloalkenyl alkyl, *e.g.,* the alkanyl, alkenyl or alkynyl moiety of the cycloalkenyl alkyl group is (C₁-C₄) alkyl and the cycloalkenyl moiety is (C₃-C₆) cycloalkenyl.

"Alkynyl," by itself or as part of another substituent refers to an unsaturated branched, or straight-chain monovalent hydrocarbon radical having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl, prop-2-yn-1-yl, *etc.;* butynyls such as but-1-yn-1-yl, but-1-yn-3-yl, but-3-yn-1-yl, *etc.;* and the like. In some embodiments, an alkynyl group comprises from 1 to 20 carbon atoms (C₂-C₂₀ alkynyl). In other embodiments, an alkynyl group comprises from 1 to 10 carbon atoms (C₂-C₁₀ alkynyl). In still other embodiments, an alkynyl group comprises from 1 to 6 carbon atoms (C₂-C₆ alkynyl) or 1 to 4 carbon atoms (C₂-C₄ alkynyl). C₂-C₆ alkynyl is also known as "lower alkynyl".

"Alkoxy," by itself or as part of another substituent, refers to a radical of the formula -O-R¹⁹⁹, where R¹⁹⁹ is alkyl or substituted alkyl as defined herein. In some embodiments, R¹⁹⁹ is a C₁-C₃ alkyl substituent. In some embodiments, R¹⁹⁹ is a C₁-C₂ alkyl substituent. In some embodiments, R¹⁹⁹ is a C₁ alkyl substituent.

"Acyl" by itself or as part of another substituent refers to a radical -C(O0)R²⁰⁰, where R²⁰⁰ is hydrogen, alkyl, substituted alkyl, aryl, substituted aryl, arylalkyl, substituted arylalkyl, heteroalkyl, substituted heteroalkyl, heteroarylalkyl or substituted heteroarylalkyl as defined herein. Representative examples include, but are not limited to formyl, acetyl, cyclohexylcarbonyl, cyclohexylmethylcarbonyl, benzoyl, benzylcarbonyl and the like.

"Aryl," by itself or as part of another substituent, refers to a monovalent aromatic hydrocarbon group derived by the removal of one hydrogen atom from a single carbon atom of a parent aromatic ring system, as defined herein. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene and the like. In some embodiments, an aryl group comprises from 6 to 18 carbon atoms (C₆-C₁₈ aryl). In other embodiments, an aryl group comprises from 6 to 15 carbon atoms (C₆-C₁₅ aryl). In still other embodiments, an aryl group comprises from 6 to 10 carbon atoms (C₆-C₁₀ aryl).

"Arylalkyl," by itself or as part of another substituent, refers to an acyclic alkyl group in which one of the hydrogen atoms bonded to a carbon atom, typically a terminal or *sp*³ carbon atom, is replaced with an aryl group, as defined herein. That is, arylalkyl can also be considered as an alkyl substituted by aryl. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. Where specific alkyl moieties are intended, the nomenclature arylalkanyl, arylalkenyl and/or arylalkynyl is used. In some embodiments, an arylalkyl group is (C₆-C₃₀) arylalkyl, *e.g.,* the alkanyl, alkenyl or alkynyl moiety of the arylalkyl group is (C₁-C₁₀) alkyl and the aryl moiety is (C₆-C₁₈) aryl. In other embodiments, an arylalkyl group is (C₆-C₁₈) arylalkyl, *e.g.,* the alkanyl, alkenyl or alkynyl moiety of the arylalkyl group is (C₁-C₈) alkyl and the aryl moiety is (C₆-C₁₂) aryl. In still other embodiments, an arylalkyl group is (C₆-C₁₄) arylalkyl, *e.g.,* the alkanyl, alkenyl or alkynyl moiety of the arylalkyl group is (C₁-C₅) alkyl and the aryl moiety is (C₆-C₁₀) aryl.

"Carbocyclic," or "Carbocyclyl," by itself or as part of another substituent, refers to a saturated or partially saturated, aromatic, cyclic monovalent hydrocarbon radical, including cycloalkyl, cycloalkenyl, and cycloalkynyl as defined herein. Typical carbocyclyl groups include, but are not limited to, groups derived from cyclopropane, cyclobutane, cyclopentane, cyclohexane, and the like. In some embodiments, the cycloalkyl group comprises from 3 to 10 ring atoms (C₃-C₁₀ cycloalkyl). In other embodiments, the cycloalkyl group comprises from 3 to 7 ring atoms (C₃-C₇ cycloalkyl). The carbocyclyl may be further substituted by one or more heteroatoms including, but not limited to, N, P, O, S, and Si, which attach to the carbon atoms of the cycloalkyl via monovalent or multivalent bond.

"Heteroalkyl," by themselves or as part of other substituents, refer to alkyl groups, in which one or more of the carbon atoms, are each, independently of one another, replaced with the same or different heteroatoms or heteroatomic groups. Each atom in the linear heteroalkyl forms a member of the heteroalkyl. For example -CH₂-O-CH₂-CH₂-O-CH₃ is a six-membered heteroalkyl. In some embodiments, the heteroalkyl group comprises from 2 to 20 members. In other embodiments, the heteroalkyl group comprises from 2 to 15 members. In some embodiments, the heteroalkyl group comprises from 2 to 10 members. In other embodiments, the heteroalkyl group comprises from 2 to 5 members. Typical heteroatoms or heteroatomic groups, which can replace the carbon atoms include, but are not limited to, -O-, -S-, -N-, -Si-, -NH-, -S(O)-, -S(O)₂-, -S(O)NH-, -S(O)₂NH- and the like and combinations thereof. The heteroatoms or heteroatomic groups may be placed at any interior position of the alkyl group. Typical heteroatomic groups which can be included in these groups include, but are not limited to, -O-, -S-, -O-O-, -S-S-, -O-S-, -NR²⁰¹R²⁰²-, =N-N=, -N=N-, -N=N-NR²⁰³R²⁰⁴, -PR²⁰⁵-, -P(O)₂-, -POR²⁰⁰-, -O-P(O)₂-, -SO-, -SO₂-, -SnR²⁰⁷R^{2 08}- and the like, where R²⁰¹, R²⁰², R²⁰³, R²⁰⁴, R²⁰⁵, R²⁰⁰, R²⁰⁷ and R²⁰⁸ are independently hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl. Preferably the heteroatoms, which can replace the carbon atoms include -O-, -S-, -N-, or -NH-.

"Heterocyclic ring" or "Heterocyclyl" by itself or as part of another substituent, refers to a carbocyclic radical in which one or more carbon atoms are independently replaced with the same or different heteroatom. The heterocyclyl may be a cycloheteroaryl or a cycloheteroalkyl. The heterocyclyl may be further substituted by one or more heteroatoms including, but not limited to, N, P, O, S, and Si, which attach to the carbon atoms of the heterocyclyl via monovalent or multivalent bond. Typical heteroatoms to replace the carbon atom(s) include, but are not limited to, N, P, O, S, Si, *etc.* Typical heterocyclyl groups include, but are not limited to, groups derived from epoxides, azirines, thiiranes, imidazolidine, morpholine, piperazine, piperidine, pyrazolidine, pyrrolidone, quinuclidine, and the like. In preferrred embodiments, heterocyclyl groups include, but are not limited to tetrahydrofuran, furan, pyrrolidine, pyridine, pyrazol, indolizine, quinoline, thiadiazol, oxadiazol and 1,2,4-oxadiazol. In some embodiments, the heterocyclyl group comprises from 3 to 20 ring atoms (3-20 membered heterocyclyl). In preferred embodiments, the heterocyclyl group comprises from 3 to 14 ring atoms (3-14 membered heterocyclyl). In more preferred embodiments, the heterocyclyl group comprises from 3 to 10 ring atoms (3-10 membered heterocyclyl). In still more preferred embodiments, the heterocyclyl group comprise from 5 to 7 ring atoms (5-7 membered heterocyclyl). A cycloheteroalkyl group may be substituted at a heteroatom, for example, a nitrogen atom, with a (C₁-C₆) alkyl group. As specific examples, N-methyl-imidazolidinyl, N-methyl-morpholinyl, N-methyl-piperazinyl, N-methyl-piperidinyl, N-methyl-pyrazolidinyl and N-methyl-pyrrolidinyl are included within the definition of "heterocyclyl." A heterocyclyl group may be attached to the remainder of the molecule *via* a ring carbon atom or a ring heteroatom.

"Halo," by itself or as part of another substituent refers to a radical -F, -Cl, -Br or -I.

"Heteroaryl," by itself or as part of another substituent, refers to a monovalent heteroaromatic radical derived by the removal of one hydrogen atom from a single atom of a parent heteroaromatic ring systems, as defined herein. Typical heteroaryl groups include, but are not limited to, groups derived from acridine, β-carboline, chromane, chromene, cinnoline, furan, imidazole, indazole, indole, indoline, indolizine, isobenzofuran, isochromene, isoindole, isoindoline, dihydroindoline, isoquinoline, isothiazole, isoxazole, naphthyridine, 1,5-naphthyridine, 1,6-naphthyridine, 1,8-naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, furan, 2-benzofuran, 1,3-dihydro-2-benzofuran, quinazoline, quinoline, quinolizine, quinoxaline, tetrazole, thiadiazole, thiazole, thiophene, triazole, xanthene, and the like. In some embodiments, the heteroaryl group comprises from 5 to 20 ring atoms (5-20 membered heteroaryl). In other embodiments, the heteroaryl group comprises from 5 to 10 ring atoms (5-10 membered heteroaryl). Exemplary heteroaryl groups include those derived from furan, thiophene, pyrrole, benzothiophene, benzofuran, benzimidazole, indole, pyridine, pyrazole, quinoline, imidazole, oxazole, isoxazole and pyrazine.

"Heteroarylalkyl" by itself or as part of another substituent refers to an acyclic alkyl group in which one of the hydrogen atoms bonded to a carbon atom, typically a terminal or *sp³* carbon atom, is replaced with a heteroaryl group. Where specific alkyl moieties are intended, the nomenclature heteroarylalkanyl, heteroarylakenyl and/or heteroarylalkynyl is used. In some embodiments, the heteroarylalkyl group is a 4-30 membered heteroarylalkyl, *e.g.,* the alkanyl, alkenyl or alkynyl moiety of the heteroarylalkyl is (C₁-C₁₀) alkyl and the heteroaryl moiety is a 5-20-membered heteroaryl. In other embodiments, the heteroarylalkyl is a 5-15 membered heteroarylalkyl, *e.g.,* the alkanyl, alkenyl or alkynyl moiety is (C₁-C₅) alkyl and the heteroaryl moiety is a 5-10 membered heteroaryl.

"Salt" refers to a salt of a compound, which possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound is replaced by a metal ion, *e.g.,* an alkali metal ion, an alkaline earth ion, or an aluminum ion; or coordinates with an organic base such as ethanolamine, diethanolamine, triethanolamine, N-methylglucamine and the like.

"Solvate" means a compound formed by solvation (the combination of solvent molecules with molecules or ions of the solute), or an aggregate that consists of a solute ion or molecule, i.e., a compound of the present invention, with one or more solvent molecules. When water is the solvent, the corresponding solvate is "hydrate". In one embodiment, each compound of the present invention is a solvate.

"Substituted," when used to modify a specified group or radical, means that one or more hydrogen atoms of the specified group or radical are each, independently of one another, replaced with the same or different substituent(s). Substituent groups useful for substituting saturated carbon atoms in the specified group or radical include, but are not limited to -R^{a}, halo, -O, =O, -OR^{b}, -SR^{b}, -S⁻, =S, -NR^{c}R^{c}, =NR^{b}, =N-OR^{b}, trihalomethyl, -CH₂F; -CHF₂; -CF₃; -CH₂Cl; -CHCl₂; -CCl₃; -CN, -OCN, -SCN, -NO, -NO₂, =N₂, -N₃, -S(O)₂R^{b}, -S(O)₂NR^{b}, -S(O)₂O⁻, -S(O)₂OR^{b}, -OS(O)₂R^{b}, -OS(O)₂O⁻, -OS(O)₂OR^{b}, -P( O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O⁻, -C(O)OR^{b}, -C(S)OR^{b}, -C(O)NR⁻R⁻, -C(NR^{b})NR^{c}R^{c}, -OC(O)R^{b}, -OC(S)R^{b}, -OC(O)O⁻, -OC(O)OR^{b}, -OC(S) OR^{b}, -NR^{b}C(O)R^{b}, -NR^{b}C(S)R^{b}, -NR^{b}C(O)O-, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a} is selected from the group consisting of alkyl, cycloalkyl, heteroalkyl, cycloheteroalkyl, aryl, arylalkyl, heteroaryl and heteroarylalkyl; each R^{b} is independently hydrogen or R^{a}; and each R^{c} is independently R^{b} or alternatively, the two R^{c}s may be taken together with the nitrogen atom to which they are bonded to form a 4-, 5-, 6- or 7-membered cycloheteroalkyl which may optionally include from 1 to 4 of the same or different additional heteroatoms selected from the group consisting of O, N and S. As specific examples, -NR^{c}R^{c} is meant to include -NH₂, -NH-alkyl, N-pyrrolidinyl and N-morpholinyl. As another specific example, a substituted alkyl is meant to include -alkylene-O-alkyl, -alkylene-heteroaryl, -alkylene-cycloheteroalkyl, -alkylene-C(O)OR^{b}, -alkylene-C(O)NR^{b}R^{b}, and -CH₂-CH₂-C(O)-CH₃. The one or more substituent groups, taken together with the atoms to which they are bonded, may form a cyclic ring including cycloalkyl and cycloheteroalkyl.

Similarly, substituent groups useful for substituting unsaturated carbon atoms in the specified group or radical include, but are not limited to, -R^{a}, halo, -O⁻, -OR^{b}, -SR^{b}, -S-, -NR^{c}R^{c}, trihalomethyl, -CH₂F; -CHF₂; -CF₃; -CH₂Cl; -CHCl₂; - CCl₃;-CN, -OCN, -SCN, -NO, -NO₂, -N₃, -S(O)₂R^{b}, -S(O)₂O⁻, -S(O)₂OR^{b}, -OS(O)₂R^{b}, -OS(O)₂ O⁻, -OS(O)₂OR^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)O⁻, -OC(O)OR^{b}, -OC(S)OR^{b}, -NR^{b}C(O)R^{b}, -NR^{b}C(S)R^{b}, -NR^{b}C(O)O-, -NR^{b}C(O)OR^{b}, -NR^{b}C(S)O R^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups useful for substituting nitrogen atoms in heteroalkyl and cycloheteroalkyl groups include, but are not limited to, -R^{a}, -O⁻, -OR^{b}, -SR^{b}, -S⁻, -NR^{c}R^{c}, trihalomethyl, -CH₂F; - CHF₂; -CF₃; -CH₂Cl; -CHCl₂; - CCl₃;-CN, -NO, -NO₂, -S(O)₂R^{b}, -S(O)₂O⁻, -S(O)₂OR^{b}, -OS(O)₂R^{b}, -OS(O)₂O⁻, -OS(O)₂OR^{b}, -P (O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)OR^{b}, -C(S)O R^{b}, -C(O)NR^{c}R^{c}, -C(NR^{b})NR^{c}R^{c}, -OC(O)R^{b}, -OC(S)R^{b}, -OC(O)OR^{b}, -OC(S)OR^{b}, -NR^{b}C(O)R^{b}, -NR^{b}C(S)R^{b}, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups from the above lists useful for substituting other specified groups or atoms will be apparent to those of skill in the art.

The term "substituted" specifically envisions and allows for one or more substitutions that are common in the art. However, it is generally understood by those skilled in the art that the substituents should be selected so as to not adversely affect the useful characteristics of the compound or adversely interfere with its function. Suitable substituents may include, for example, halogen groups, perfluoroalkyl groups, perfluoroalkoxy groups, alkyl groups, alkenyl groups, alkynyl groups, hydroxy groups, oxo groups, mercapto groups, alkylthio groups, alkoxy groups, aryl or heteroaryl groups, aryloxy or heteroaryloxy groups, arylalkyl or heteroarylalkyl groups, arylalkoxy or heteroarylalkoxy groups, amino groups, alkyl- and dialkylamino groups, carbamoyl groups, alkylcarbonyl groups, carboxyl groups, alkoxycarbonyl groups, alkylaminocarbonyl groups, dialkylamino carbonyl groups, arylcarbonyl groups, aryloxycarbonyl groups, alkylsulfonyl groups, arylsulfonyl groups, cycloalkyl groups, cyano groups, C₁-C₆ alkylthio groups, arylthio groups, nitro groups, keto groups, acyl groups, boronate or boronyl groups, phosphate or phosphonyl groups, sulfamyl groups, sulfonyl groups, sulfinyl groups, and combinations thereof. In the case of substituted combinations, such as "substituted arylalkyl," either the aryl or the alkyl group may be substituted, or both the aryl and the alkyl groups may be substituted with one or more substituents. Additionally, in some cases, suitable substituents may combine to form one or more rings as known to those of skill in the art.

A "substituted" cycloalkyl, heterocyclic ring or aryl group may include the substitution with one or more substituent each substituent independently selected from the group comprising -F, - Cl, -Br, -NO₂,-O-N=O, -N=O, -OH, -NH₂, -CH=NH, -N₃, -SH, -SO₃H, -SO₂H, -S(O)₂CH₃ -CN, - NC,-OCN, -NCO, -SCN, -NCS, -(P=O)(OH)₂, -O(P=O)(OH)₂, -(C=O)OH, -(C=O)NHOH, - NH(C=O)NH₂, -NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or - NHC(O)NHR¹¹, wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, --CH₂F; -CHF₂; -CF₃; -CH₂Cl; - CHCl₂; and -CCl₃;.

More preferably, a "substituted" alkyl, heteroalkyl, cycloalkyl, heterocyclic ring or aryl group includes the substitution with one or more substituent each substituent independently selected from the group comprising -F, -Cl, -Br, -NO₂,-O-N=O, -N=O, -OH, -NH₂, -CH=NH, -N₃, -SH, - SO₃H, -SO₂H, -S(O)₂CH₃ -CN, -NC,-OCN, -NCO, -SCN, -NCS, -(P=O)(OH)₂, -O(P=O)(OH)₂, - (C=O)OH, -(C=O)NHOH, -NH(C=O)NH₂, -NH(C=S)NH₂, -(C=O)NH₂,-CHO,-CHS, -OCHO, - NCHO, -B(OH)₂, C₁-C₄ alkyl, C₁-C₄ alkoxy, C₂-C₄ alkenyl, C₃-C₁₀ cycloalkyl, C₃-C₁₀ cycloalkyl C₁-C₂ alkyl, 3-10 membered cycloheteroalkyl or 3-10 membered cycloheteroalkyl C₁-C₂ alkyl, C₆-C₁₀ aryl, C₆-C₁₀ aryl C₁-C₂ alkyl, 5-10 membered heteroaryl, 5-10 membered heteroaryl C₁-C₂ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹, wherein R¹¹ and R¹² are independently selected from the group consisting of C₁-C₄ alkyl, C₂-C₄ alkenyl, C₃-C₁₀ cycloalkyl, C₆-C₁₀ aryl, 5-10 membered heteroaryl, 2-10 membered heteroalkyl, -CH₂F; -CHF₂; -CF₃; -CH₂Cl; -CHCl₂; and -CCl₃;.

Preferably, a "substituted" cycloalkyl, heterocyclic ring or aryl group includes the substitution with one, two or three substituents, still more preferably with one or two substituents.

The term "optionally substituted" denotes the presence or absence of the substituent group(s). That is, it means "substituted or unsubstituted". For example, optionally substituted alkyl includes both unsubstituted alkyl and substituted alkyl. The substituents used to substitute a specified group can be further substituted, typically with one or more of the same or different groups selected from the various groups specified above.

"Carrier" refers to a diluent, excipient or vehicle with which a compound is administered.

"NLRP3" is meant to include, without limitation, nucleic acids, polynucleotides, oligonucleotides, sense and antisense polynucleotide strands, complementary sequences, peptides, polypeptides, proteins, homologous and/or orthologous NLRP3 molecules, isoforms, precursors, mutants, variants, derivatives, splice variants, alleles, different species, and active fragments thereof.

A "modulator" herein refers to a compound that can regulate the activity of NLRP3. Such regulation includes activating NLRP3, blocking NLRP3, or potentiating/reducing the activation of NLRP3. That is, the modulators include agonists, antagonists, enhancers, etc.

An "activator" or an "agonist" of NLRP3 herein refers to a compound that can (positively) modulate the activity of NLRP3. Such modulation includes activating NLRP3, or increasing the activation of NLRP3. An "activator" or an "agonist" includes compounds that, at the protein level, directly bind or modify NLRP3 such that an activity of NLRP3 is increased, e.g., by activation, stabilization, altered distribution, or otherwise.

"Treating" or "treatment" of any condition, disease or disorder refers to ameliorating the condition, disease or disorder (*i.e.,* arresting or reducing the development of the condition, disease or disorder or at least one of the clinical symptoms thereof). In other embodiments "treating" or "treatment" refers to ameliorating at least one physical parameter, which may not be discernible by the patient. In yet other embodiments, "treating" or "treatment" refers to inhibiting the condition, disease or disorder, either physically, (*e*.*g*., stabilization of a discernible symptom), physiologically, (*e*.*g*., stabilization of a physical parameter) or both. In yet other embodiments, "preventing," "prevention," "treating" or "treatment" refers to delaying the onset of the condition, disease or disorder.

"Therapeutically effective amount" means the amount of the present compound, a pharmaceutically acceptable salt and/or hydrate and/or cocrystal thereof; e.g., a compound, such as niclosamide or a pharmaceutically acceptable salt and/or hydrate and/or cocrystal thereof that, when administered to a subject for treating a condition, disease or disorder, is sufficient to effect such treatment for the condition, disease or disorder. The "therapeutically effective amount" will vary depending on the compound, the condition, disease or disorder and its severity and the age, weight, *etc.,* of the subject to be treated. The result includes reduction and/or alleviation of the signs, symptoms, or causes of a disease, or any other desired alteration of a biological system.

"Subject" refers to an animal, including, but not limited to, a primate (e.g., human), monkey, cow, pig, sheep, goat, horse, dog, cat, rabbit, rat, or mouse. The terms "subject" and "patient" are used interchangeably herein in reference, for example, to a mammalian subject, such as a human.

"Pharmaceutical composition" refers to a mixture of a compound described herein with other chemical components (referred to collectively herein as "excipients"), such as carriers, stabilizers, diluents, dispersing agents, suspending agents, and/or thickening agents. The pharmaceutical composition facilitates administration of the compound to an organism. Multiple techniques of administering a compound exist in the art including, but not limited to: rectal, oral, intravenous, aerosol, parenteral, ophthalmic, pulmonary, and topical administration. A further technique of administering is the intratumoral injection.

"Excipient" or "pharmaceutically acceptable excipient" means a pharmaceutically-acceptable material, composition, or vehicle, such as a liquid or solid filler, diluent, carrier, solvent, or encapsulating material. In one embodiment, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. See, e.g., Remington: The Science and Practice of Pharmacy, 21st ed.; Lippincott Williams & Wilkins: Philadelphia, PA, 2005; Handbook of Pharmaceutical Excipients, 6th ed. ; Rowe et al, Eds.; The Pharmaceutical Press and the American Pharmaceutical Association: 2009; Handbook of Pharmaceutical Additives, 3rd ed. ; Ash and Ash Eds.; Gower Publishing Company: 2007; Pharmaceutical Preformulation and Formulation, 2nd ed. ; Gibson Ed.; CRC Press LLC: Boca Raton, FL, 2009.

### Embodiments of the Compounds

According to the first aspect, the present invention relates to a compound for use in the prevention or treatment of a disease by activating the NLRP3 inflammasome, wherein the disease is selected from a group comprising infectious diseases or cancer, and
wherein the compound is a compound of Formula (I), Formula (I') or Formula (I") 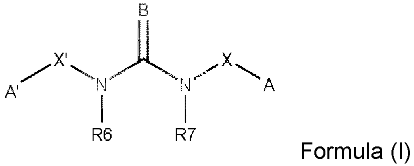 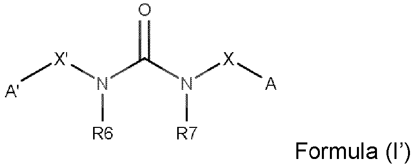 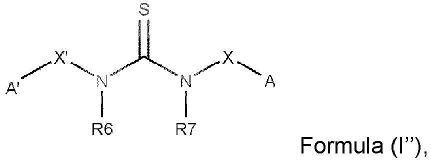
or a pharmaceutically acceceptable salt thereof, wherein:
   A and A' are independently selected from each other from A and A' as defined herein below.
   B is O or S, preferably O;
   and X and X' are each independently present or not, wherein X and X' are each independently a C₁-C₁₀ alkyl group; preferably an C₁-C₅ alkyl group, or an C₁-C₅ alkanol group; more preferably selected from -CH₂-, -C₂H₄-, -C₃H₆-, -CH(CH₃)₂-, -CHCH(CH₃)₂-, -CHC(CH₃)₃-, - CH(CH(CH₃)₂)-, -CH(CH₂CH(CH₃)₂)-, or -CH(CH₂OH)-.
   R⁶ and R⁷ are each independently hydrogen, a C₁-C₁₀ alkyl or R⁶ and R⁷ when taken together form a five- or six-membered heterocyclic ring,
   preferably selected from 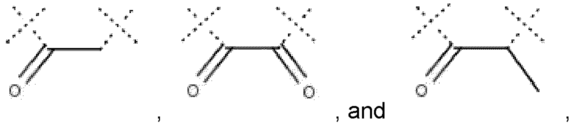 or optionally,
   R⁷, N and X when taken together form a five- or six-membered heterocyclic ring, preferably selected from 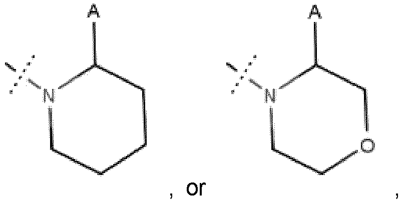 wherein A is as herein above and below.

X and X' are each independently a linker group which bonds the nitrogen of the urea to the A or A' respectively. If X or X' is not present, the nitrogen of the urea is directly bound to the A or A', respectively.

In one embodiment, the compound of Formula (I), (I') or I") of the invention comprises X and X', wherein X and X' are each independently -(CH₂)ₘ-, wherein each m is independently an integer from 0 to 1, 0 to 2, 0 to 3 or 0 to 4, or C₁-C₃ alkyl, a C₁-C₂ alkyl, preferably, -CH₂-, - C₂H₄-, -C₃H₆-, -CH(CH₃)₂-, -CH(CH(CH₃)₂)-, -CH(CH₂CH(CH₃)₂)-, or -CH(CH₂OH)-, preferably only one of X and X' is present and is a C₁-C₃ alkyl, a C₁-C₂ alkyl, preferably, -CH₂-, -C₂H₄-, -C₃H₆-, -CH(CH₃)₂-, -CH(CH(CH₃)₂)-, -CH(CH₂CH(CH₃)₂)-, or -CH(CH₂OH)-; and wherein
R⁶ and R⁷ are each independently hydrogen, a C₁-C₁₀ alkyl, preferably a C₁-C₅ alkyl, C₁-C₄ alkyl, C₁-C₃ alkyl, C₁-C₂ alkyl, more preferably hydrogen or CH₃, even more preferably wherein R⁶ and R⁷ are each hydrogen.

In one further embodiment, the compound of Formula (I), (I') or (I") of the invention comprises X and X', wherein X and X' are each independently -(CH₂)ₘ-, wherein each m is independently an integer from 0 to 1, 0 to 2, 0 to 3 or 0 to 4, or C₁-C₃ alkyl, a C₁-C₂ alkyl, preferably, -CH₂-, - C₂H₄-, -C₃H₆-, -CH(CH₃)₂-, -CH(CH(CH₃)₂)-, -CH(CH₂CH(CH₃)₂)-, or -CH(CH₂OH)-, preferably only one of X and X' is present and is a C₁-C₃ alkyl, a C₁-C₂ alkyl, preferably, -CH₂-, -C₂H₄-, -C₃H₆-, -CH(CH₃)₂-, -CH(CH(CH₃)₂)-, -CH(CH₂CH(CH₃)₂)-, or -CH(CH₂OH)-; and wherein
R⁶ and R⁷ when taken together form a five- or six-membered heterocyclic ring.
preferably selected from 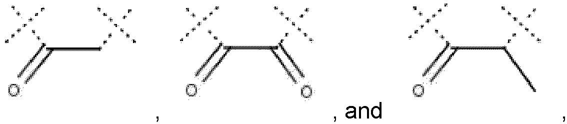 and
wherein A and A' are preferably as defined herein below.

In one further embodiment, the compound of Formula (I), (I') or (I") of the invention comprises X and X', wherein X and X' are each independently -(CH₂)ₘ-, wherein each m is independently an integer from 0 to 1, 0 to 2, 0 to 3 or 0 to 4, or C₁-C₃ alkyl, a C₁-C₂ alkyl, preferably, -CH₂-, - C₂H₄-, -C₃H₆-, -CH(CH₃)₂-, -CH(CH(CH₃)₂)-, -CH(CH₂CH(CH₃)₂)-, or -CH(CH₂OH)-, preferably only one of X and X' is present and is a C₁-C₃ alkyl, a C₁-C₂ alkyl, preferably, -CH₂-, -C₂H₄-, -C₃H₆-, -CH(CH₃)₂-, -CH(CH(CH₃)₂)-, -CH(CH₂CH(CH₃)₂)-, or -CH(CH₂OH)-; and wherein R⁷, N and X when taken together form a five- or six-membered heterocyclic ring, preferably selected from 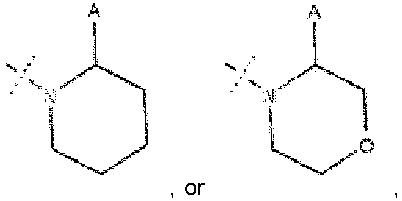 and wherein A and A' are preferably as herein below.

In one embodiment, the compound of Formula (I), (I') or (I") of the invention comprises X and X', wherein X and X' are each independently -(CH₂)ₘ-, wherein each m is independently an integer from 0 to 2, preferably 0 or 1, more preferably 0.

In one embodiment, the compound of Formula (I), (I') or (I") of the invention comprises X and X', wherein X and X' are each independently -(CH₂)ₘ-, wherein each m is independently an integer from 0 to 2, preferably 0 or 1, more preferably 0,and R⁶ and R⁷ are each independently hydrogen, wherein A and A' are preferably as defined herein below.

In one further embodiment, the compound of Formula (I), (I') or (I") of the invention comprises none of X and X'; and wherein R⁶ and R⁷ when taken together form a five- or six-membered heterocyclic ring.
preferably selected from 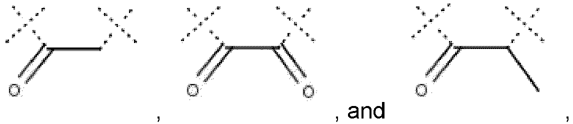 and wherein A is preferably as defined herein below.

In one further embodiment, the compound of Formula (I), (I') or (I") of the invention comprises none of X and X', and wherein R⁷, N and X when taken together form a five- or six-membered heterocyclic ring, preferably selected from 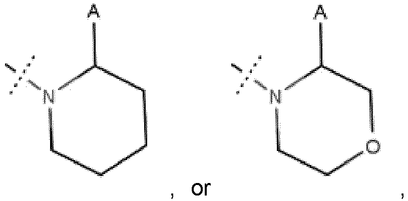 and wherein A is as herein above and below.

In one preferred embodiment, the compound of Formula (I), (I') or (I") of the invention comprises X and X', wherein X and X' are each independently a C₁-C₃ alkyl, a C₁-C₂ alkyl, preferably, -CH₂-, -C₂H₄-, -C₃H₆-, -CH(CH₃)₂-, -CH(CH(CH₃)₂)-, -CH(CH₂CH(CH₃)₂)-, or - CH(CH₂OH)-,
preferably only one of X and X' is present and is a C₁-C₃ alkyl, a C₁-C₂ alkyl, preferably selected from the group consisting of, -CH₂-, -C₂H₄-, -C₃H₆-, -CH(CH₃)₂-, -CH(CH(CH₃)₂)-, - CH(CH₂CH(CH₃)₂)-, or -CH(CH₂OH)-, and A and A' are each independently isoquinoline, quinoline, benzene, pyridine, 1,5-naphthyridine, isothiazole, or oxadiazole. A and A' may be identical to each other or different to each other. In one preferred embodiment, A and A' are different to each other. A and A' are preferably as defined herein below.

In one preferred embodiment, the compound of Formula (I), (I') or (I") of the invention comprises A and A', wherein A and A' are each independently benzene, imidazolidine, morpholine, piperazine, piperidine, pyrazolidine, pyrrolidone, quinuclidine, tetrahydrofuran, furan, pyrrolidine, pyridine, pyrazol, indolizine, quinoline, thiadiazol, oxadiazol, 1,2,4-oxadiazol, acridine, β-carboline, chromane, chromene, cinnoline, furan, imidazole, indazole, indole, indoline, indolizine, isobenzofuran, isochromene, isoindole, isoindoline, dihydroindoline, isoquinoline, isothiazole, isoxazole, naphthyridine, 1,5-naphthyridine, 1,6-naphthyridine, 1,8-naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, furan, 2-benzofuran, 1,3-dihydro-2-benzofuran, quinazoline, quinoline, quinolizine, quinoxaline, tetrazole, thiadiazole, thiazole, thiophene, triazole, xanthene, cyclopropyl, cyclobutyl, cyclopentyl, cyclohexyl; cyclopropenyl, cyclobutenyl, cyclopentenyl, cyclohexenyl; cyclobutadienyl, cyclohexadienyl, carbazole, benzimidazole, imidazopyridine, benzoisoxazole, tetrahydrothiophene, benzofuran, benzoxazole, or benzthiozole,
more preferably wherein A and A' are each independently benzene, pyrrolidine, pyridine, pyrazole, pyridazine, , pyrimidine, pyrrole, pyrrolizine, furan, 2-benzofuran, 1,3-dihydro-2-benzofuran, diazole, triazole, oxazole, oxadiazole, 1,2,4-oxadiazole, thiazole, thiophene, thiadiazole, imidazole, indazole, indole, indoline, isoindole, isoindoline, dihydroindoline, quinoline, isoquinoline, isothiazole, isoxazole, naphthyridine,1,5-naphthyridine, 1,6-naphthyridine, 1,8-naphthyridine, phthalazine, purine, quinazoline, quinoxaline, benzimidazole, benzoisoxazole, tetrahydrothiophene, benzoxazole or benzthiazole,
even more preferably wherein A and A' are each independently quinoline, isoquinoline, indole, benzene, pyridine, pyrazole, pyrrol, furan, thiophene; naphthyridine,1,5-naphthyridine, 1,6-naphthyridine, 1,8-naphthyridine, isothiazole, or oxadiazole,
likewise more preferably wherein A and A' are each independently quinoline, benzene, pyridine, naphthyridine, 1,5-naphthyridine, isothiazole, oxadiazole, or thiophene,
most preferably wherein A and A' are each independently isoquinoline, quinoline, benzene, pyridine, 1,5-naphthyridine, isothiazole, or oxadiazole.

A and A' may be identical to each other or different to each other. In one preferred embodiment, A and A' are different to each other.

In one embodiment, the compound of Formula (I), (I') or (I") of the invention comprises X and X', wherein X and X' are each independently -(CH₂)ₘ-, wherein each m is independently an integer from 0 to 2, preferably 0 or 1, more preferably 0.

In one preferred embodiment, the compound of Formula (I), (I') or (I") of the invention comprises X and X', wherein X and X' are each independently a C₁-C₃ alkyl, a C₁-C₂ alkyl, preferably, -CH₂-, -C₂H₄-, -C₃H₆-, -CH(CH₃)₂-, -CH(CH(CH₃)₂)-, -CH(CH₂CH(CH₃)₂)-, or - CH(CH₂OH)-,
preferably only one of X and X' is present and is a C₁-C₃ alkyl, a C₁-C₂ alkyl, preferably selected from the group consisting of, -CH₂-, -C₂H₄-, -C₃H₆-, -CH(CH₃)₂-, -CH(CH(CH₃)₂)-, - CH(CH₂CH(CH₃)₂)-, or -CH(CH₂OH)-, and A and A' are each independently isoquinoline, quinoline, benzene, pyridine, 1,5-naphthyridine, 1,6-naphthyridine, 1,8-naphthyridine, isothiazole, or oxadiazole. A and A' may be identical to each other or different to each other. In one preferred embodiment, A and A' are different to each other.

Preferably, according to the preferred embodiment, A and A' are each independently quinoline, isoquinoline, benzene, pyridine, naphthyridine, 1,5-naphthyridine, 1,6-naphthyridine, 1,8-naphthyridine, isothiazole, oxadiazole, or thiophene, preferably selected from groups as defined in the following in any of sections a) to h):
a) a benzene group of the following structure: 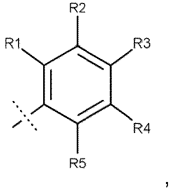 wherein R¹-R⁵ are each independently selected from the group consisting of:
   hydrogen; -OH, -OCH₃, -OCF₃, -OCHF₂, -OCH₂OH; -OCH₂CH₃; -OCH(CH₃)₂; -F; -Cl; -Br; - CN; NO₂; NH₂; C₁-C₅ alkyl, C₁-C₅ alkoxy, C₂-C₅ alkenyl, -CH₃, -CH₂F; -CHF₂; -CF₃; -CH₂Cl; - CHCl₂; -CCl₃;CH(CH₃)₂; -CH₂CH₃; -CH₂-CH₂-CH(CH₃)₂; -CH₂-N(CH₃)₂; -CH(CH₃)-NHC(O)CH₃; - -NH-CH(CH₃)₂, -NH-cyclopropyl, -CH₃, CH₂CH₃, CHCH(CH₃)₂; -C(O)-C₂H₅; - C(O)-CH₃; -C(O)-aziridine; -C(O)-azetidine; -C(O)-pyrrolidine; -C(CH₃)=NOH; -S(O)₂-cyclopentyl; -S(O)₂-CH₃; -S(O)₂-cyclopentyl; pyrrolidinyl, C₁-C₁₀ cycloalkyl, C₁-C₁₀ cycloheteroalkyl, 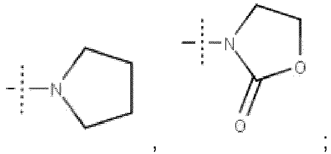 a C₆-C₁₀ aryl; a 5-10 membered heteroaryl;
   NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-CH(CH₃)₂, -NH-cyclopropyl, C₁-C₅ alkyl, -CH₂F; -CHF₂; -CF₃; -CH₂Cl; -CHCl₂; -CCl₃;-CH₃, CH₂CH₃, CHCH(CH₃)₂;
   -C(O)OR⁴², wherein R⁴² is selected from C₁-C₅ alkyl, including -CH₃; -CH₂CH₃, -CH₂CH₂CH_{3;} -CH(CH₃)₂; -CH₂CH₂CH₂CH₃; -CH₂NHS(O)₂CH₃;
   -NHR⁴³, wherein R⁴³ is selected from 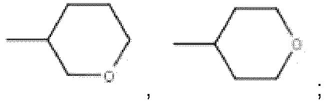 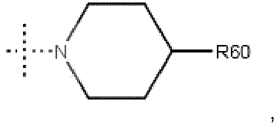
   wherein R⁶⁰ is selected from hydrogen or -CH₃; 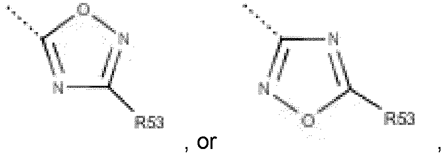
   wherein
   R⁵³ is preferably selected from the group consisting of hydrogen, a C₁-C₁₀ alkyl, 2-20 membered heteroalkyl, C₃-C₂₀ cycloalkyl, 3-20 membered cycloheteroalkyl, C₆-C₁₈ aryl or 5-20 membered heteroaryl, preferably a C₆-C₁₄ aryl or 5-20 membered heteroaryl; more preferably from hydrogen, OH, -OCH₃, -OCF₃, -OCHF₂, -OCH₂OH; -OCH₂CH₃; -F; -Cl; -Br; - CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -CHF₂, -CH₂F; -CHF₂; -CF₃; - CH₂Cl; -CHCl₂; -CCl₃;-O-N=O, -N=O, -CH=NH, -N₃, -SH, -SO₃H, -SO₂H, -S(O)₂CH₃ -CN, - NC,-OCN, -NCO, -(C=O)OH, -(C=O)NHOH, -NH(C=O)NH₂, -NH(C=S)NH₂, -(C=O)NH₂,-CHO,-CHS, -OCHO, -NCHO, 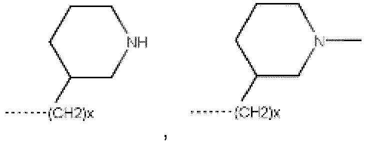 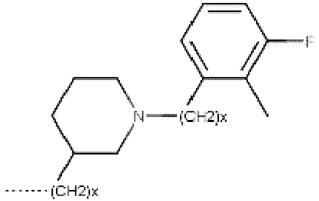 , with X = 0 or 1, preferably 1; 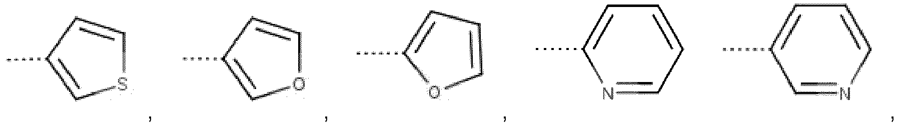 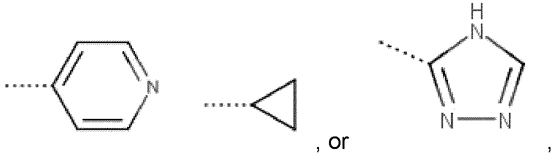 or
   R⁵³ is 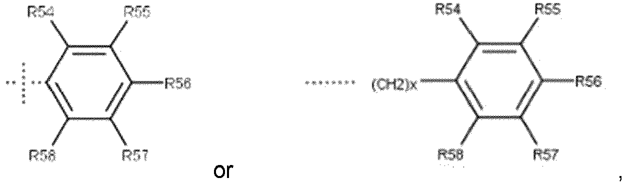 wherein R⁵⁴, R⁵⁵, R⁵⁶, R⁵⁷ or R⁵⁸ are selected each independently from hydrogen, -CH₃, -C₂H₅, -CH₂F; -CHF₂; -CF₃; -CH₂Cl; -CHCl₂; -CCl₃; -OCH₃, -F, -Cl, -Br, NH₂, NO₂, and x is 0, 1 or 2;
   or R⁵³ is 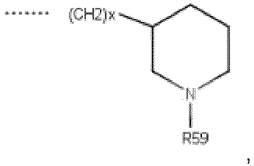 wherein R⁵⁹ is 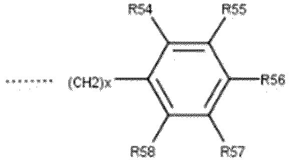 and wherein R⁵⁴, R⁵⁵, R⁵⁶, R⁵⁷ or R⁵⁸ are selected separately from the above from hydrogen, -CH₃, -C₂H₅, - CH₂F; -CHF₂; -CF₃; -CH₂Cl; -CHCl₂; -CCl₃; -OCH₃, -F, -Cl, -Br, NO₂, NH₂, and x is 0, 1 or 2; as defined above;
      and
   optionally, wherein R¹ and R², R² and R³, R³ and R⁴, or R⁴ and R⁵ together, preferably R² and R³ together, form a five- or six-membered cyclic or heterocyclic ring, preferably selected from 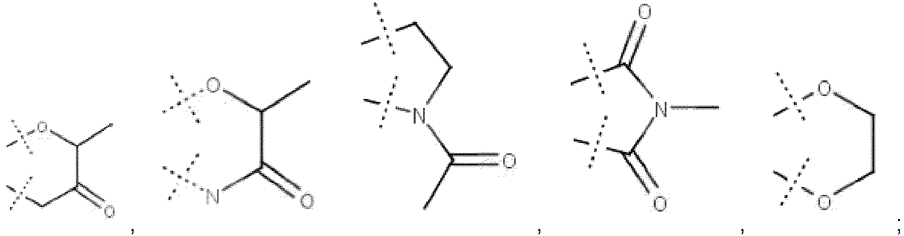 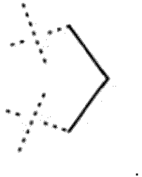
   Preferably X is not present in Formulae (I), (I') or (I") in case A or A' is a benzene group as defined under a). Likewise preferably, one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
   Preferably either all of R¹ to R⁵ are hydrogen or 1, 2 or 3, preferably 1 or 2 of R¹ to R⁵ are different from hydrogen and selected from substitutions defined or presented herein above under a) for R¹ to R⁵, and remaining substituents of R¹ to R⁵ are hydrogen.
b) a pyridine group of one of the following structures: 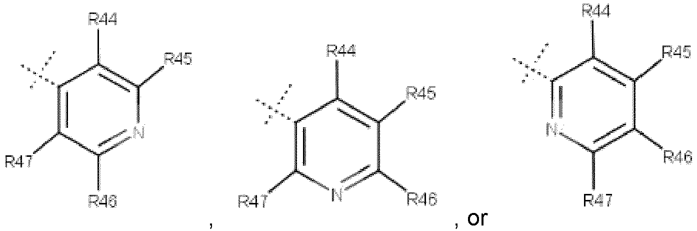 wherein R⁴⁴-R ⁴⁷ are each independently selected from the group consisting of:
   hydrogen, -OH, -OCH₃, CH₃, F, Cl, Br, -CH₂F; -CHF₂; -CF₃; -CH₂Cl; -CHCl₂; -CCl₃;-OCF₃, - OCHF₂, -OCH₂OH; -OCH₂CH₃; -CN; NO₂; NH₂; -CH₃, CH(CH₃)₂; -CH₂CH₃; -CH₂OH; -CH₂-CH₂-CH(CH₃)₂; -CH₂-N(CH₃)₂; -CH(CH₃)-NHC(O)CH₃; 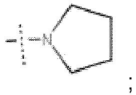
   -NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-cyclopropyl, -CH₂F; -CHF₂; -CF₃; - CH₂Cl; -CHCl₂; -CCl₃;-CH₃, CH₂CH₃, CHCH(CH₃)₂; -C(O)-aziridine; -C(O)-azetidine; -C(O)-pyrrolidine; a C₆-C₁₀ aryl; a 5-10 membered heteroaryl; C₁-C₁₀ cycloalkyl, C₁-C₁₀ cycloheteroalkyl,
   -C(O)OR⁴², wherein R⁴² is selected from C₁-C₅ alkyl, including -CH₃; -CH₂CH₃, -CH₂CH₂CH_{3;} -CH(CH₃)₂; -CH₂CH₂CH₂CH₃; -CH₂NHS(O)₂CH₃; -C(CH₃)=NOH; -S(O)₂-cyclopentyl; -S(O)₂-CH₃; -S(O)₂-cyclopentyl; pyrrolidinyl, 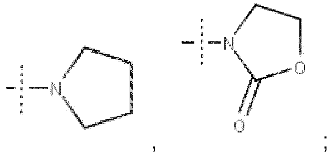 or
   -NHR⁴³, wherein R⁴³ is selected from 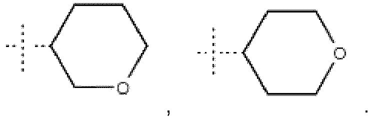
   Preferably in Formula (I), (I') or (I") in case A and/or A' is a pyridine group as defined under b) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
   Preferably either all of R⁴⁴ to R⁴⁷ are hydrogen or 1, 2 or 3, preferably 1 or 2 of R⁴⁵ to R⁴⁷ are different from hydrogen and selected from substitutions defined or presented herein above under b) for R⁴⁴ to R⁴⁷, and remaining substituents of R⁴⁴ to R⁴⁷ are hydrogen.
c) a quinoline or an isoquinoline group, preferably of one of the the following structures: 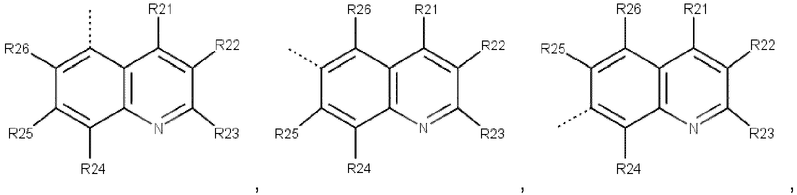 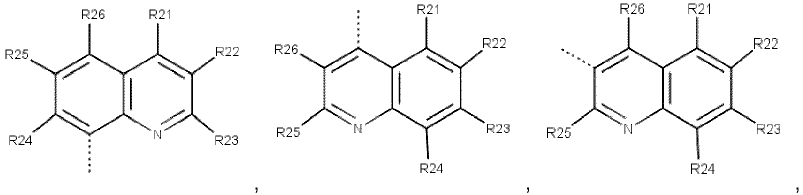 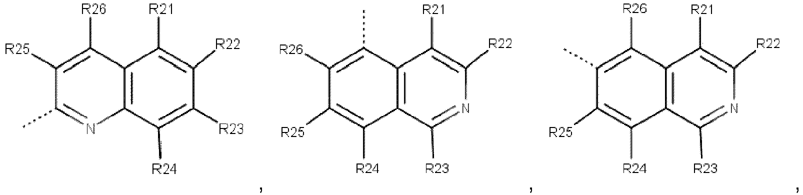 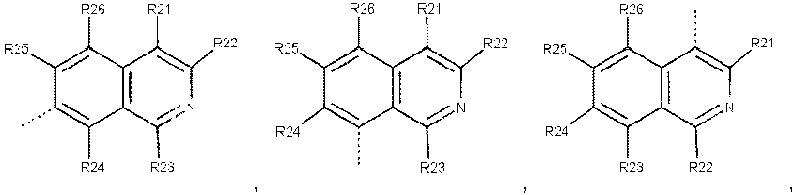 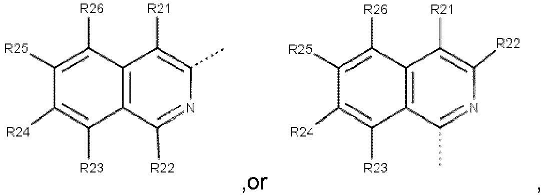 wherein
   R²¹ to R²⁶ are each independently selected from the group consisting of:
   hydrogen, OH, -OCH₃, -OCF₃, -OCHF₂, -OCH₂OH; -OCH₂CH₃; -F; -Cl; -Br; -CH₂F; -CHF₂; - CF₃; -CH₂Cl; -CHCl₂; -CCl₃;-CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -O-N=O, -N=O, -CH=NH, -N₃, -SH, -SO₃H, -SO₂H, -S(O)₂CH₃ -CN, -NC,-OCN, - NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -C(O)-aziridine; -C(O)-azetidine; - C(O)-pyrrolidine; -C(O)OR₄₂, wherein R⁴² is selected from C1-C5 alkyl, including -CH₃; - CH₂CH₃, -CH₂CH₂CH_{3;} -CH(CH₃)₂; -CH₂CH₂CH₂CH₃; -CH₂NHS(O)₂CH₃; -C(CH₃)=NOH; - S(O)₂-cyclopentyl; -S(O)₂-CH₃; -S(O)₂-cyclopentyl; pyrrolidinyl, 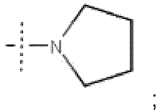 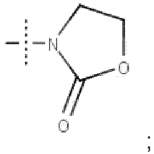 a C₆-C₁₀ aryl; a 5-10 membered heteroaryl; C₁-C₁₀ cycloalkyl, C₁-C₁₀ cycloheteroalkyl;
   -NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-cyclopropyl, -CH₂F; -CHF₂; -CF₃; - CH₂Cl; -CHCl₂; -CCl₃;-CH₃, CH₂CH₃, CHCH(CH₃)₂;
   -NHR⁴³, wherein R⁴³ is selected from 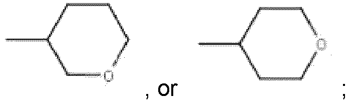
   or more preferably wherein R²¹ to R²⁶ are each hydrogen, OH, -OCH₃, -OCF₃, -OCHF₂, - OCH₂OH; -OCH₂CH₃; -F; -Cl; -Br; -CH₂F; -CHF₂; -CF₃; -CH₂Cl; -CHCl₂; -CCl₃;-CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃;
   or even more preferably wherein R²¹ to R²⁶ are each hydrogen, or -F; Cl, Br.
   Preferably in case A and/or A' is a quinoline or an isoquinoline group quinoline or an isoquinoline group in Formula (I), (I') or (I") as defined in c) X and/or X' are either not present, only one of X or X'is present or both of X and X' are present, and X and X' or are each preferably selected from -CH₂-, -C₂H₄-, -C₃H₆-, -CHCH(CH₃)₂-, -CHC(CH₃)₃-, - CH(CH(CH₃)₂)-, -CH(CH₂CH(CH₃)₂)-, or -CH(CH₂OH)-.
   Preferably either all of R²¹ to R²⁶ are hydrogen or 1, 2 or 3, preferably 1 or 2 of R²¹ to R²⁶ are different from hydrogen and selected from substitutions defined or presented herein above under c) for R²¹ to R²⁶ and remaining substituents of R²¹ to R²⁶ are hydrogen;
d) a naphthyridine, preferably a 1,5 naphthyridine group, a 1,6 naphthyridin group or a 1,8 naphthyridin group of any of the following structures: 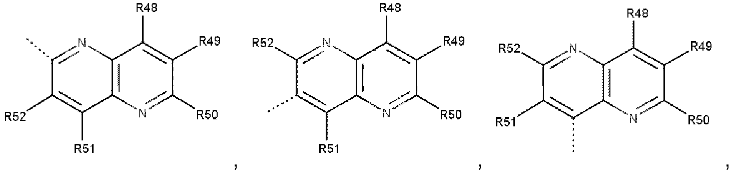 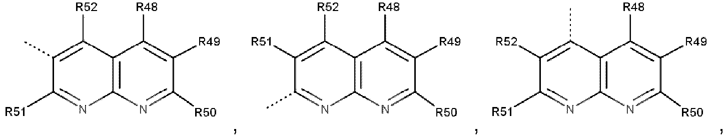 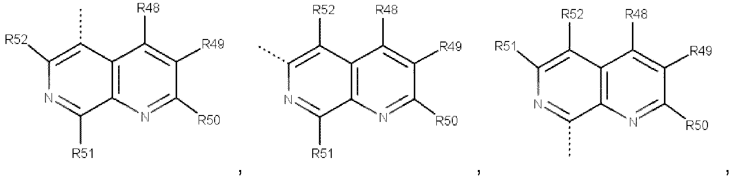 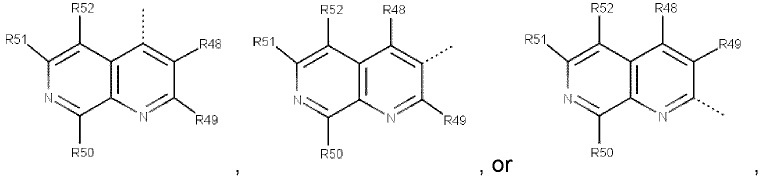 wherein
   R⁴⁸ to R⁵² are each independently selected from the group consisting of:
   hydrogen, OH, -OCH₃, -OCF₃, -OCHF₂, -OCH₂OH; -OCH₂CH₃; -F; -Cl; -Br; -CH₂F; -CHF₂; - CF₃; -CH₂Cl; -CHCl₂; -CCl₃; -CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -O-N=O, -N=O, -CH=NH, -N₃, -SH, -SO₃H, -SO₂H, -S(O)₂CH₃ -CN, -NC,-OCN, - NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO,
   -NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-cyclopropyl, -CH₂F; -CHF₂; -CF₃; - CH₂Cl; -CHCl₂; -CCl₃;-CH₃, CH₂CH₃, CHCH(CH₃)₂; -C(O)-aziridine; -C(O)-azetidine; -C(O)-pyrrolidine;
   -C(O)OR ⁴², wherein R⁴² is selected from C₁-C₅ alkyl, including -CH₃; -CH₂CH₃, -CH₂CH₂CH_{3;} -CH(CH₃)₂; -CH₂CH₂CH₂CH₃; -CH₂NHS(O)₂CH₃; -C(CH₃)=NOH; -S(O)₂-cyclopentyl; -S(O)₂-CH₃; -S(O)₂-cyclopentyl; pyrrolidinyl, 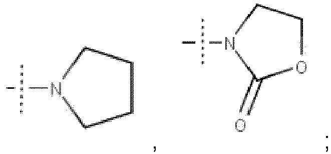 a C₆-C₁₀ aryl; a 5-10 membered heteroaryl; C₁-C₁₀ cycloalkyl, C₁-C₁₀ cycloheteroalkyl;
   -NHR₄₃, wherein R₄₃ is selected from 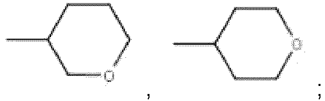
   or more preferably wherein R⁴⁸ to R⁵² are each hydrogen, OH, -OCH₃, -OCF₃, -OCHF₂, - OCH₂OH; -OCH₂CH₃; -F; -Cl; -Br; -CH₂F; -CHF₂; -CF₃; -CH₂Cl; -CHCl₂; -CCl₃;-CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃;
   or even more preferably wherein R⁴⁸ to R⁵² are each hydrogen, or -F; Cl, Br.
   Preferably in Formula (I), (I') or (I") in case A and/or A' is a 1,5-naphthyridine group as defined under d) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
   Preferably either all of R⁴⁸ to R⁵² are hydrogen or 1, 2 or 3, preferably 1 or 2 of R⁴⁸ to R⁵² are different from hydrogen and selected from substitutions defined or presented herein above under d) for R⁴⁸ to R⁵² and remaining substituents R⁴⁸ to R⁵² are hydrogen;
e) an oxadiazole group of one of the following formulae: 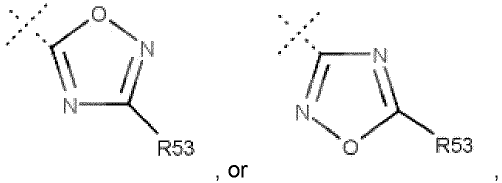 wherein
   R⁵³ is preferably selected from the group consisting of:
   hydrogen, a C₁-C₁₀ alkyl, 2-20 membered heteroalkyl, C₃-C₂₀ cycloalkyl, 3-20 membered cycloheteroalkyl, C₆-C₁₈ aryl or 5-20 membered heteroaryl, preferably a C₆-C₁₄ aryl or 5-20 membered heteroaryl; more preferably from
   hydrogen, OH, -OCH₃, -OCF₃, -OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -CH₂F; -CHF₂; -CF₃; -CH₂Cl; -CHCl₂; -CCl₃; -O-N=O, -N=O, -CH=NH, -N₃, -SH, -SO₃H, -SO₂H, -S(O)₂CH₃ -CN, -NC,-OCN, -NCO, - (C=O)OH, -(C=O)NHOH, -NH(C=O)NH₂, -NH(C=S)NH₂, -(C=O)NH₂,-CHO,-CHS, -OCHO, - NCHO, a C₆-C₁₀ aryl; a 5-10 membered heteroaryl; a C₁-C₁₀ cycloalkyl, a C₁-C₁₀ cycloheteroalkyl, 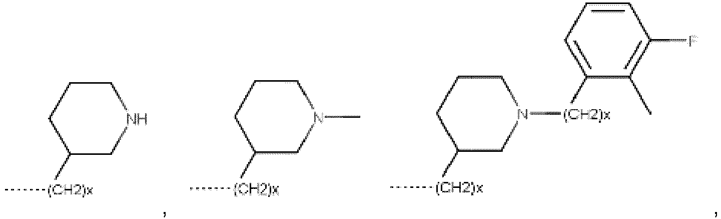 with X = 0 or 1, preferably 1; 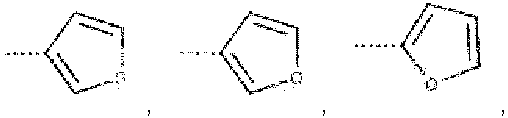 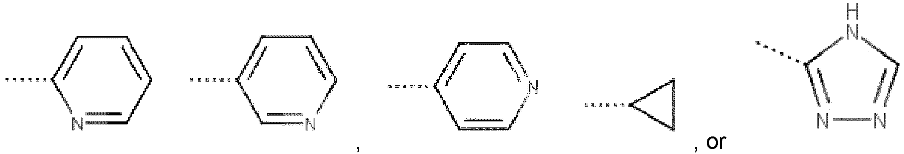 , or
   R⁵³ is 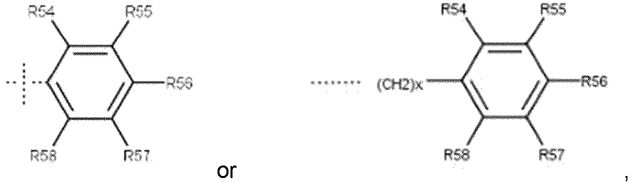 wherein R⁵⁴, R⁵⁵, R⁵⁶, R⁵⁷ or R⁵⁸ are selected each independently from hydrogen, -CH₃, -C₂H₅, -CH₂F; -CHF₂; -CF₃; -CH₂Cl; -CHCl₂; -CCl₃; -OCH₃, -F, -Cl, -Br, NH₂, NO₂, and x is 0, 1 or 2;
   or R⁵³ is 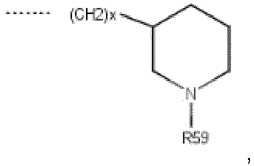 wherein R⁵⁹ is 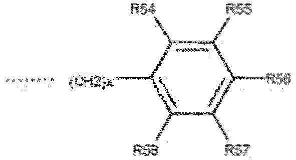 and wherein R⁵⁴, R⁵⁵, R⁵⁶, R⁵⁷ or R⁵⁸ are selected separately from the above from hydrogen, -CH₃, -C₂H₅, - CH₂F; -CHF₂; -CF₃; -CH₂Cl; -CHCl₂; -CCl₃; -OCH₃, -F, -Cl, -Br, NO₂, NH₂, and x is 0, 1 or 2; as defined above, and x is 0, 1 or 2;
   and wherein preferably either all of R⁵⁴ to R⁵⁸ are hydrogen or 1, 2 or 3, preferably 1 or 2 of R⁵⁴ to R⁵⁸ are different from hydrogen and selected from substitutions defined or presented herein above under e) for R⁵⁴ to R⁵⁸ and remaining positions of R⁵⁴ to R⁵⁸ are hydrogen;
f) a thiadiazole group, preferbly selected of one of the following formulae: 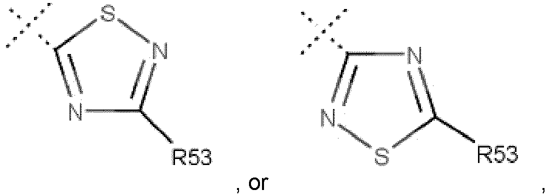
   wherein R⁵³ is preferably as defined above in section e);
   Preferably in Formula (I), (I') or (I") in case A and/or A' is a thiadiazole group as defined under f) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
g) an isothiazole group of the following formula: 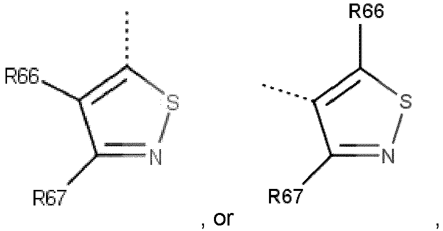 wherein R⁶⁶ and R⁶⁷ are each selected independently from hydrogen, -CH₃, -C₂H₅, -CH₂F; -CHF₂; -CF₃; -CH₂Cl; -CHCl₂; -CCl₃;-F, - Cl, -Br, preferably wherein either of R⁶⁶ or R⁶⁷ is hydrogen and the other residue is selected from -CH₃, -C₂H₅, -CF₃, -CHF₂, -F, -Cl, -Br;
   Preferably in Formula (I), (I') or (I") in case A and/or A' is a isothiazole group as defined under g) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
h) a thiophene group, preferably selected of any of the following formulae: 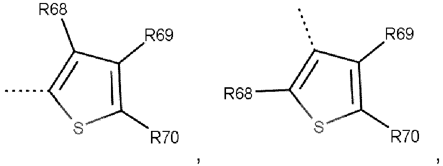 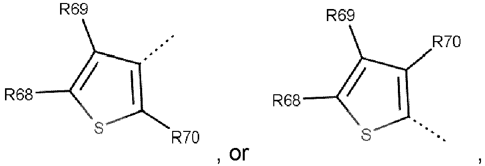 wherein R⁶⁸, R⁶⁹ and R⁷⁰ are each preferably selected independently from hydrogen, -CH₃, - C₂H₅, -CH₂F; -CHF₂; -CF₃; -CH₂Cl; -CHCl₂; -CCl₃;-F, -Cl, -Br, preferably wherein either of R⁶⁸, R⁶⁹ or R⁷⁰ is hydrogen and the other residue is selected from -CH₃, -C₂H₅, -CF₃, -CHF₂, -F, - Cl, -Br

Preferably in Formula (I), (I') or (I") in case A and/or A' is a thiophene group as defined under g) X and/or X' are either not present, only one of X or X'is present or both of X and X' are present, and X and X' or are each preferably selected from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.

In one alternatively preferred embodiment, the compound of Formula (I), (I') or (I") of the invention is a compound of any one of Formulae (Ic), (Ic1), (Ic2), (Ic3), or (Ic4), or a pharmaceutically acceptable salt thereof: 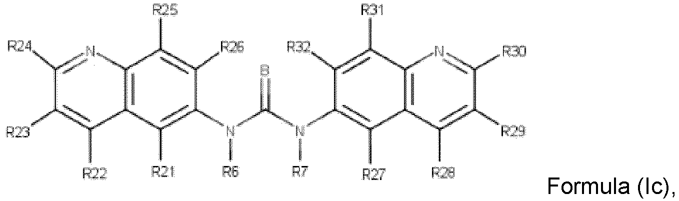 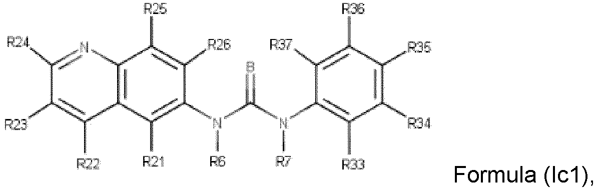 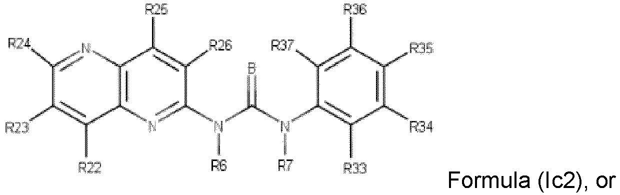 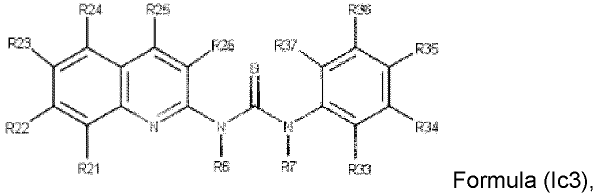 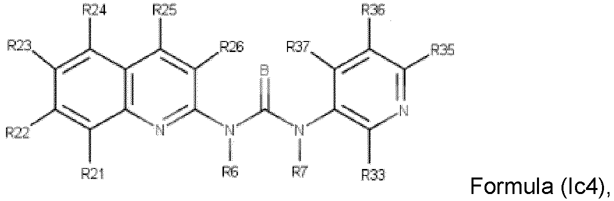
wherein B = O or S, preferably O;
wherein R⁶ and R⁷ are independently each hydrogen, a C₁-C₁₀ alkyl, preferably a C₁-C₅ alkyl, or C₁₋₃ alkyl, more preferably -H, -CH₃, -C₂H₅, -C₃H₇, or -C₃H₉, even more preferably hydrogen or CH₃, more preferably, either R⁶ is hydrogen and R⁷ is CH₃, -C₂H₅, -C₃H₇, or - C₃H₉, or more preferably, either R⁷ is hydrogen and R⁶ is CH₃, -C₂H₅, -C₃H₇, or -C₃H₉, or R6 and R7 are independently each hydrogen, or optionally
R⁶ and R⁷ when taken together form a five- or six-membered heterocyclic ring. preferably selected from

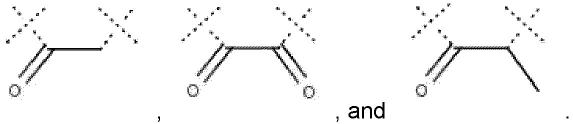

In the compound of any one of Formulae (Ic), (Ic1), (Ic2), (Ic3), or (Ic4), R²¹ to R³⁷ are preferably each independently selected from the group consisting of hydrogen, -OH, -F, -Cl, -Br, -NO₂,-O-N=O, -N=O, -OH, -NH₂, -CH=NH, -N₃, -SH, -SO₃H, -SO₂H, -S(O)₂CH₃ -CN, -NC,-OCN, - NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, OCH₃, -OCF₃, -OCHF₂, - OCH₂OH; -OCH₂CH₃; -CH₂F; -CHF₂; -CF₃; -CH₂Cl; -CHCl₂; -CCl₃; -F; -Cl; -Br; -CN; NO₂; NH₂; -O-N=O, -N=O, -N₃, -SH, -SO₃H, -SO₂H, -S(O)₂CH₃ CH=NH, C₁-C₁₀ alkyl, C₁-C₁₀ alkoxy, C₂-C₁₀ alkenyl, C₃-C₂₀ cycloalkyl, C₃-C₂₀ cycloalkyl C₁-C₁₀ alkyl, 3-20 membered cycloheteroalkyl or 3-20 membered cycloheteroalkyl C₁-C₁₀ alkyl, C₆-C₁₈ aryl, C₆-C₁₈ aryl C₁-C₁₀ alkyl, 5-20 membered heteroaryl, 5-20 membered heteroaryl C₁-C₁₀ alkyl, e.g. -CH₃, CH(CH₃)₂; -CH₂CH₃; -CH₂-CH₂-CH(CH₃)₂; -CH₂-N(CH₃)₂; -CH(CH₃)-NHC(O)CH₃; (C=O)R¹¹, (C=O)OR¹¹, (C-O)SR¹¹, (C=O)NR¹¹R¹², (SO₂)R¹¹, (SNO)R¹¹, (P=O)R¹¹R¹² (P=O)OR¹¹OR¹² or C(NR¹¹)R¹², -NHC(O)R¹¹, and -NHC(O)NHR¹¹, wherein R¹¹ and R¹² are independently selected from the group consisting of C₁-C₁₀ alkyl, C₂-C₁₀ alkenyl, C₃-C₂₀ cycloalkyl, C₆-C₁₈ aryl, 5-20 membered heteroaryl, and 2-20 membered heteroalkyl, or
more preferably wherein R²¹ to R³⁷ are each independently selected from the group consisting of hydrogen, C₁-C₆ alkoxy, C₁-C₅ alkyl, -(C=O)CH₃, -CN, C₁-C₅ alkyl(SO₂)C₁-C₅ alkyl, preferably -CH₂(SO₂)CH₃, -CH₂(SO₂)CH₂CH₃, -CH₂CH₂(SO₂)CH₂CH₃; -(SO₂)C₁-C₅ alkyl, preferably -(SO₂)CH₃, -(SO₂)CH₂CH₃, -CH₂F; -CHF₂; -CF₃; -CH₂Cl; -CHCl₂; -CCl₃;-F, -Cl, and -Br; or even
more preferably wherein R²¹ to R³⁷ are each hydrogen.

Even more preferably, in a compound Formula (Ic), (Ic1), (Ic2), (Ic3), or (Ic4), R⁶ and R⁷ are independently each hydrogen, C₁₋₃ alkyl, preferably hydrogen, CH₃, more preferably, either R⁶ is hydrogen and R⁷ is CH₃, or R6 and R7 are both hydrogen; and
R²¹ to R³⁷ are each independently selected from the group consisting of hydrogen, OH, - OCH₃, -OCF₃, -OCHF₂, -OCH₂OH; -OCH₂CH₃; -CH₂F; -CHF₂; -CF₃; -CH₂Cl; -CHCl₂; -CCl₃;-F; -Cl; -Br; -CN; NO₂; NH₂; C₁-C₅ alkyl, C₁-C₆ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -O-N=O, -N=O, -OH, - NH₂, -CH=NH, -N₃, -SH, -SO₃H, -SO₂H, -S(O)₂CH₃ -CN, -NC,-OCN, -NCO, -SCN, -NCS, - (P=O)(OH)₂, -O(P=O)(OH)₂, -(C=O)OH, -(C=O)NHOH, -NH(C=O)NH₂, -NH(C=S)NH₂, - (C=O)NH₂,-CHO,-CHS, -OCHO, -NCHO, -B(OH)₂ (C=O)R¹¹, (C=O)OR¹¹, (C=O)SR¹¹, (C=O)NR¹¹R¹², (SO₂)R¹¹, or C(NR¹¹)R¹², -NHC(O)R¹¹, and -NHC(O)NHR¹¹, and wherein R¹¹ and R¹² are independently selected from the group consisting of C₁-C₅ alkyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; C₂-C₁₀ alkenyl, C₃-C₂₀ cycloalkyl, C₆-C₁₈ aryl, 5-20 membered heteroaryl, and 2-20 membered heteroalkyl; or
preferably wherein R²¹ to R³⁷ are each independently selected from the group consisting of hydrogen, C₁-C₆ alkoxy, C₁-C₅ alkyl, C₁-C₅ alkyl(SO₂)C₁-C₅ alkyl, preferably -CH₂(SO₂)CH₃, - CH₂(SO₂)CH₂CH₃, -CH₂CH₂(SO₂)CH₂CH₃; -(SO₂)C₁-C₆ alkyl, preferably -(SO₂)CH₃, - (SO₂)CH₂CH₃, -CH₂F; -CHF₂; -CF₃; -CH₂Cl; -CHCl₂; -CCl₃;, -F, -Cl, and -Br; or
wherein preferably either all of R²¹ to R³⁷ are hydrogen or 1, 2 or 3, preferably 1 or 2 of R²¹ to R³⁷ are different from hydrogen and selected from substitutions presented above for R²¹ to R³⁷ and remaining substituents of R²¹ to R³⁷ are hydrogen; or
more preferably wherein R²¹ to R³⁷ are each hydrogen.

In one further alternatively preferred embodiment, the compound of Formula (I), (I') or (I") of the invention is a compound of any one of Formulae (Ic5), (Ic6), (Ic7), (Ic8), (Ic9) (Ic10), (Ic11), (Ic12), (Ic13), or (Ic14) or a pharmaceutically acceptable salt thereof: 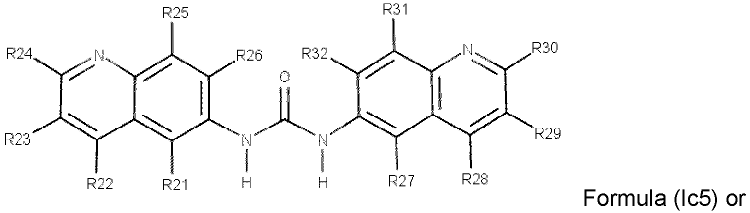 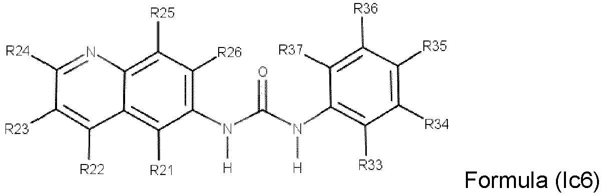 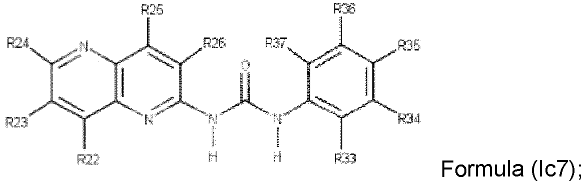 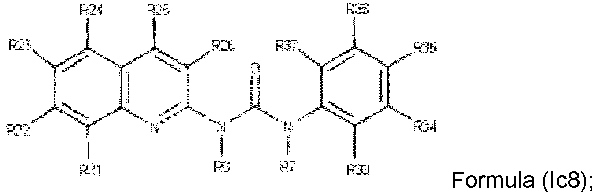 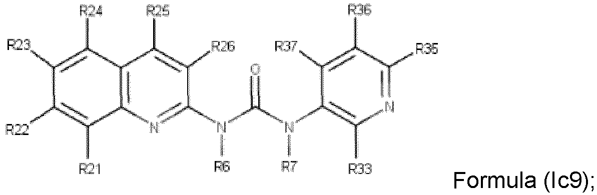 or 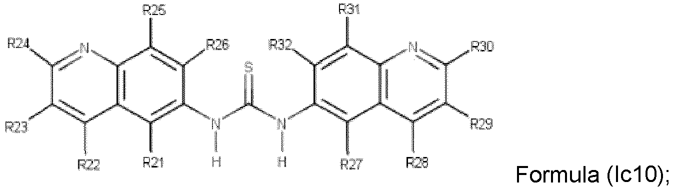 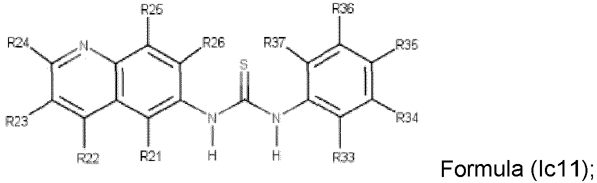 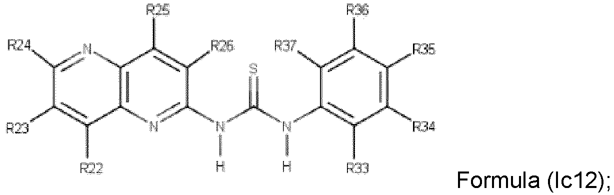 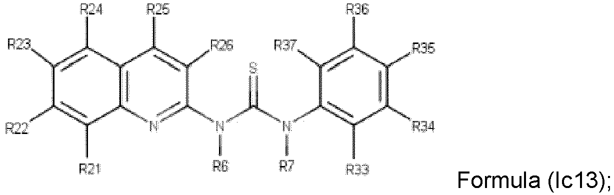 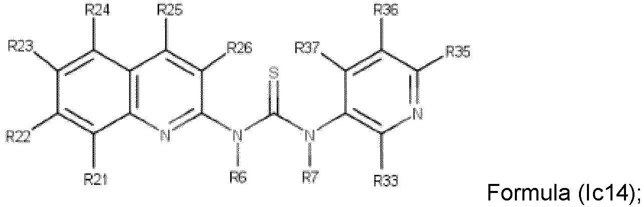
wherein R⁶ and R⁷ are independently each hydrogen, a C₁-C₁₀ alkyl, preferably a C₁-C₅ alkyl, or C₁₋₃ alkyl, more preferably -H, -CH₃, -C₂H₅, -C₃H₇, or -C₃H₉, even more preferably hydrogen or CH₃, more preferably, either R⁶ is hydrogen and R⁷ is CH₃, -C₂H₅, -C₃H₇, or - C₃H₉, or more preferably, either R⁷ is hydrogen and R⁶ is CH₃, -C₂H₅, -C₃H₇, or -C₃H₉, or R6 and R7 are independently each hydrogen, or optionally
R⁶ and R⁷ when taken together form a five- or six-membered heterocyclic ring.
preferably selected from

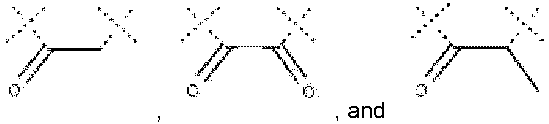

In the compound of any one of Formulae (Ic5), (Ic6), (Ic7), (Ic8), (Ic9) (Ic10), (Ic11), (Ic12), (Ic13),or (Ic14) R²¹ to R³⁷ are preferably each independently selected from the group consisting of hydrogen, -OH, -F, -Cl, -Br, -NO₂,-O-N=O, -N=O, -OH, -NH₂, -CH=NH, -N₃, -SH, -SO₃H, - SO₂H, -S(O)₂CH₃ -CN, -NC,-OCN, -NCO, -SCN, -NCS, -(P=O)(OH)₂, -O(P=O)(OH)₂, - (C=O)OH, -(C=O)NHOH, -NH(C=O)NH₂, -NH(C=S)NH₂, -(C=O)NH₂,-CHO,-CHS, -OCHO, - NCHO, -B(OH)₂, OCH₃, -OCF₃, -OCHF₂, -OCH₂OH; -OCH₂CH₃; -CH₂F; -CHF₂; -CF₃; -CH₂Cl; -CHCl₂; -CCl₃; CH=NH, C₁-C₁₀ alkyl, C₁-C₁₀ alkoxy, C₂-C₁₀ alkenyl, C₃-C₂₀ cycloalkyl, C₃-C₂₀ cycloalkyl C₁-C₁₀ alkyl, 3-20 membered cycloheteroalkyl or 3-20 membered cycloheteroalkyl C₁-C₁₀ alkyl, C₆-C₁₈ aryl, C₆-C₁₈ aryl C₁-C₁₀ alkyl, 5-20 membered heteroaryl, 5-20 membered heteroaryl C₁-C₁₀ alkyl, e.g. -CH₃, CH(CH₃)₂; -CH₂CH₃; -CH₂-CH₂-CH(CH₃)₂; -CH₂-N(CH₃)₂; - CH(CH₃)-NHC(O)CH₃; (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, and -NHC(O)NHR¹¹, wherein R¹¹ and R¹² are independently selected from the group consisting of C₁-C₁₀ alkyl, C₂-C₁₀ alkenyl, C₃-C₂₀ cycloalkyl, C₆-C₁₈ aryl, 5-20 membered heteroaryl, and 2-20 membered heteroalkyl; or
more preferably wherein R²¹ to R³⁷ are each independently selected from the group consisting of hydrogen, C₁-C₆ alkoxy, C₁-C₅ alkyl, -(C=O)CH₃, -CN, C₁-C₅ alkyl(SO₂)C₁-C₅ alkyl, preferably -CH₂(SO₂)CH₃, -CH₂(SO₂)CH₂CH₃, -CH₂CH₂(SO₂)CH₂CH₃; -(SO₂)C₁-C_{S} alkyl, preferably -(SO₂)CH₃, -(SO₂)CH₂CH₃, -CH₂F; -CHF₂; -CF₃; -CH₂Cl; -CHCl₂; -CCl₃; -F, -Cl, and -Br; or
even more preferably wherein R²¹ to R³⁷ are each hydrogen. In an even more preferred embodiment, in the compounds of any of Formulae (Ic), (Ic1), (Ic2), (Ic3), (Ic4), (Ic5), (Ic6), (Ic7), (Ic8), (Ic9) (Ic10), (Ic11), (Ic12), (Ic13) or (Ic14), R²¹ to R³⁷ are each independently selected from the group consisting of hydrogen, OH, -OCH₃, -OCF₃, -OCHF₂, -OCH₂OH; - OCH₂CH₃; -CH₂F; -CHF₂; -CF₃; -CH₂Cl; -CHCl₂; -CCl₃; -F; -Cl; -Br; -CN; NO₂; NH₂; C₁-C₆ alkoxy, C₁-C₅ alkyl, preferably -CH₃, CH(CH₃)₂; -CH₂CH₃; or C₁-C₅ alkyl(SO₂)C₁-C₅ alkyl, preferably -CH₂(SO₂)CH₃, -CH₂(SO₂)CH₂CH₃, -CH₂CH₂(SO₂)CH₂CH₃; -(SO₂)C₁-C₆ alkyl, preferably -(SO₂)CH₃, -(SO₂)CH₂CH₃.

In a preferred embodiment, the compound of any of Formulae (Ic), (Ic5) and (Ic10) is as defined above, wherein R²¹, R²⁶, R²⁷ and R³² is preferably hydrogen.

In a preferred embodiment, the compound of any of Formulae (Ic), (Ic1), (Ic2), (Ic3), (Ic4), (Ic6), (Ic7), (Ic8), (Ic9) (Ic10), (Ic11), (Ic12), (Ic13) or (Ic14), is as defined above, wherein R²¹, R²⁶, R³³ and R³⁷ is preferably hydrogen.

In a preferred embodiment, the compound of any of Formulae (Ic), (Ic1), (Ic2), (Ic3), (Ic4), (Ic5), (Ic6), (Ic7), (Ic8), (Ic9) (Ic10), (Ic11), (Ic12), (Ic13) or (Ic14),is as defined above, wherein R²⁴ is C₁-C₆ alkoxy, -OCH₃, -OCF₃, -OCHF₂, -OCH₂OH; -OCH₂CH₃; -CH₂F; -CHF₂; -CF₃; -CH₂Cl; - CHCl₂; -CCl₃; C₁-C₅ alkyl, preferably -CH₃, CH(CH₃)₂; -CH₂CH₃; -(C=O)CH₃, -CN, C₁-C₅ alkyl(SO₂)C₁-C₅ alkyl, preferably -CH₂(SO₂)CH₃, -CH₂(SO₂)CH₂CH₃, -CH₂CH₂(SO₂)CH₂CH₃; - (SO₂)C₁-C₅ alkyl, preferably -(SO₂)CH₃, -(SO₂)CH₂CH₃, -CH₂F; -CHF₂; -CF₃; -CH₂Cl; -CHCl₂; - CCl₃; -F, -Cl, or -Br, and wherein R²¹ to R²³ and R²⁵ to R³⁷ are each hydrogen.

In a preferred embodiment, the compound of any of Formulae (Ic), (Ic1), (Ic2), (Ic3), (Ic4), (Ic5), (Ic6), (Ic7), (Ic8), (Ic9) (Ic10), (Ic11), (Ic12), (Ic13) or (Ic14),is as defined above, wherein R²³ is C₁-C₆ alkoxy, -OCH₃, -OCF₃, -OCHF₂, -OCH₂OH; -OCH₂CH₃; -CH₂F; -CHF₂; -CF₃; -CH₂Cl; - CHCl₂; -CCl₃; C₁-C₅ alkyl, preferably -CH₃, CH(CH₃)₂; -CH₂CH₃; -(C=O)CH₃, -CN, C₁-C₅ alkyl(SO₂)C₁-C₅ alkyl, preferably -CH₂(SO₂)CH₃, -CH₂(SO₂)CH₂CH₃, -CH₂CH₂(SO₂)CH₂CH₃; - (SO₂)C₁-C₅ alkyl, preferably -(SO₂)CH₃, -(SO₂)CH₂CH₃, -CF₃, -F, -Cl, or -Br, and wherein R²¹, R²² and R²⁴ to R³⁷ are each hydrogen.

In a preferred embodiment, the compound of Formula (Ic), (Ic1), (Ic2), (Ic3), (Ic4), (Ic6), (Ic7), (Ic8), (Ic9) (Ic10), (Ic11), (Ic12), (Ic13) or (Ic14), is as defined above, wherein R³⁵ is C₁-C₆ alkoxy, -OCH₃, -OCF₃, -OCHF₂, -OCH₂OH; -OCH₂CH₃; -CH₂F; -CHF₂; -CF₃; -CH₂Cl; -CHCl₂; - CCl₃; C₁-C₅ alkyl, preferably -CH₃, CH(CH₃)₂; -CH₂CH₃; -(C=O)CH₃, -CN, C₁-C₅ alkyl(SO₂)C₁-Cs alkyl, preferably -CH₂(SO₂)CH₃, -CH₂(SO₂)CH₂CH₃, -CH₂CH₂(SO₂)CH₂CH₃; -(SO₂)C₁-C₆ alkyl, preferably -(SO₂)CH₃, -(SO₂)CH₂CH₃, -CF₃, -F, -Cl, or -Br, and wherein R²¹ to R²⁶, R³³, R³⁴ and R³⁶, R³⁷ are each hydrogen.

In a preferred embodiment, the compound of any of Formula (Ic), (Ic1), (Ic2), (Ic3), (Ic4), (Ic6), (Ic7), (Ic8), (Ic9) (Ic10), (Ic11), (Ic12), (Ic13) or (Ic14),is as defined above, wherein R³⁶ is C₁-C₆ alkoxy, -OCH₃, -OCF₃, -OCHF₂, -OCH₂OH; -OCH₂CH₃; -CH₂F; -CHF₂; -CF₃; -CH₂Cl; - CHCl₂; -CCl₃; C₁-C₅ alkyl, preferably -CH₃, CH(CH₃)₂; -CH₂CH₃; -(C=O)CH₃, -CN, C₁-C₅ alkyl(SO₂)C₁-C₅ alkyl, preferably -CH₂(SO₂)CH₃, -CH₂(SO₂)CH₂CH₃, -CH₂CH₂(SO₂)CH₂CH₃; - (SO₂)C₁-C₅ alkyl, preferably -(SO₂)CH₃, -(SO₂)CH₂CH₃, -CF₃, -F, -Cl, or -Br, and wherein R²¹ to R²⁶, R³³, R³⁴ and R³⁶, R³⁷ are each hydrogen.

In a preferred embodiment, the compound of any of Formula (Ic), (Ic1), (Ic2), (Ic3), (Ic4), (Ic5), (Ic6), (Ic7), (Ic8), (Ic9) (Ic10), (Ic11), (Ic12), (Ic13) or (Ic14),is as defined above, wherein R²² is C₁-C₆ alkoxy, -OCH₃, -OCF₃, -OCHF₂, -OCH₂OH; -OCH₂CH₃; -CH₂F; -CHF₂; -CF₃; -CH₂Cl; - CHCl₂; -CCl₃; C₁-C₅ alkyl, preferably -CH₃, CH(CH₃)₂; -CH₂CH₃; -(C=O)CH₃, -CN, C₁-C₅ alkyl(SO₂)C₁-C₅ alkyl, preferably -CH₂(SO₂)CH₃, -CH₂(SO₂)CH₂CH₃, -CH₂CH₂(SO₂)CH₂CH₃; - (SO₂)C₁-C₅ alkyl, preferably -(SO₂)CH₃, -(SO₂)CH₂CH₃, -CF₃, -F, -Cl, or -Br, and wherein R²¹ and R²³ to R³⁷ are each hydrogen.

In a preferred embodiment, the compound of any of Formulae (Ic), (Ic1), (Ic2), (Ic3), (Ic4), (Ic5), (Ic6), (Ic7), (Ic8), (Ic9) (Ic10), (Ic11), (Ic12), (Ic13) or (Ic14)is as defined above, wherein R²¹ to R³⁷ are each hydrogen.

In a preferred embodiment, the compound of any of Formulae (Ic), (Ic1), (Ic2), (Ic3), (Ic4), (Ic5), (Ic6), (Ic7), (Ic8), (Ic9) (Ic10), (Ic11), (Ic12), (Ic13) or (Ic14) is one of the following structures:

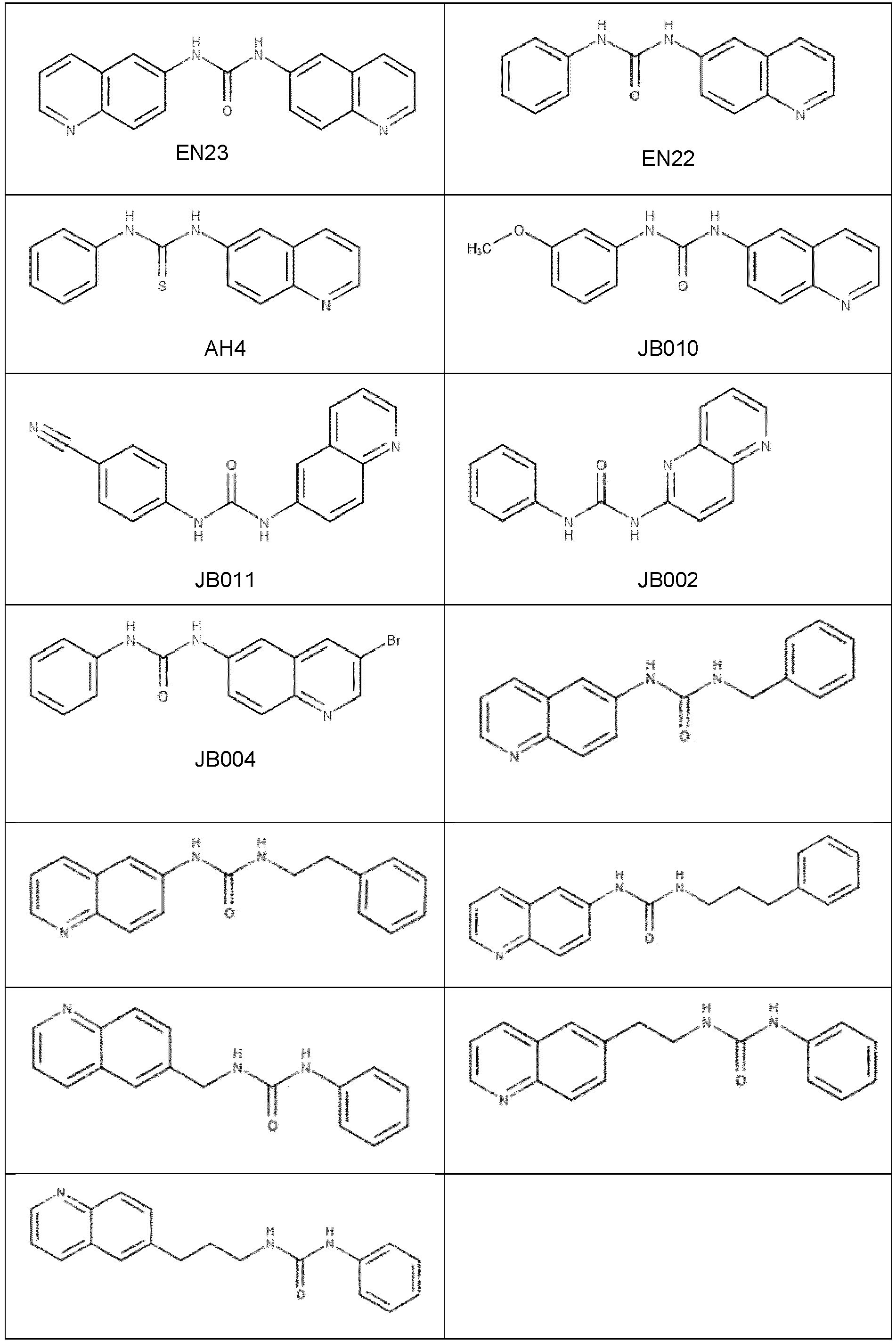

In a second aspect, the compound of Formula (I) of the invention is a compound of Formula (Ia) or (Ia1), 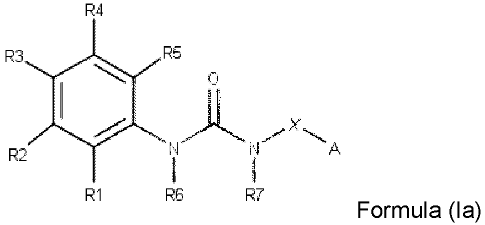 or 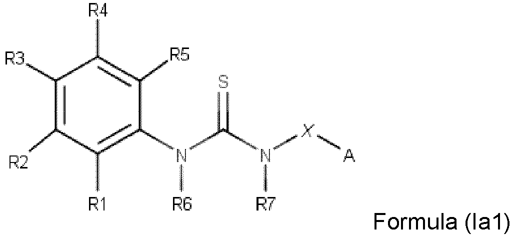 or a pharmaceutically acceceptable salt thereof, wherein:
X either present or not, and wherein X, if present, is selected from a C₁-C₁₀ alkyl group, a C₁-C₅ alkyl group, e.g. -CH₂-, -C₂H₄-, -C₃H₆-, -CHCH(CH₃)₂-, -CHC(CH₃)₃-, -CH(CH(CH₃)₂)-, - CH(CH₂CH(CH₃)₂)-, or a C₁-C₅ alkanol group, e.g. -CH(CH₂OH)-; and
R¹ and R², R² and R³, R³ and R⁴, R⁴ and R⁵, when taken together form a five- or six-membered cycloalkyl, five- or six-membered cycloheteroalkyl, five- or six-membered aryl, or five- or six-membered heteroaryl, and
R⁶ and R⁷ are each independently hydrogen, a C₁-C₁₀ alkyl, preferably R⁶ and R⁷ are each independently hydrogen, -CH₃, -C₂H₅, -C₃H₇, or -C₃H₉, or R⁶ and R⁷ when taken together form a five- or six-membered heterocyclic ring.

Alternatively, R⁶ and R⁷ when taken together form a five- or six-membered heterocyclic ring, preferably selected from 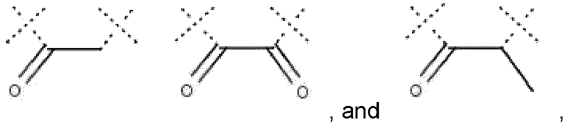 or optionally,
R⁷, N and X when taken together form a five- or six-membered heterocyclic ring, preferably selected from

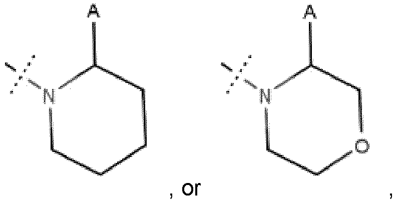

According to one embodiment of formulae (la) or (la1), R¹, R², R³, R⁴, and R⁵ are each independently selected from the group consisting hydrogen, -F, -Cl, -Br, -NO₂,-O-N=O, -N=O, -OH, -NH₂, -CH=NH, -N₃, -SH, -SO₃H, -SO₂H, -S(O)₂CH₃ -CN, -NC,-OCN, -NCO, -SCN, - NCS, -(P=O)(OH)₂, -O(P=O)(OH)₂, -(C=O)OH, -(C=O)NHOH, -NH(C=O)NH₂, -NH(C=S)NH₂, -(C=O)NH₂,-CHO,-CHS, -OCHO, -NCHO, -B(OH)₂, -OCH₃, -OCF₃, -OCHF₂, -OCH₂OH; - OCH₂CH₃; -OCH(CH₃)₂; -CH₃, -CH₂F; -CHF₂; -CF₃; -CH₂Cl; -CHCl₂; -CCl₃;CH(CH₃)₂ - CH₂CH₃, C₁-C₁₀ alkyl, C₁-C₁₀ alkoxy, C₂-C₁₀ alkenyl, C₃-C₂₀ cycloalkyl, C₃-C₂₀ cycloalkyl C₁-C₁₀ alkyl, 3-20 membered cycloheteroalkyl or 3-20 membered cycloheteroalkyl C₁-C₁₀ alkyl, C₆-C₁₈ aryl, C₆-C₁₈ aryl C₁-C₁₀ alkyl, 5-20 membered heteroaryl, 5-20 membered heteroaryl, C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹, wherein R¹¹ and R¹² are independently selected from the group consisting of C₁-C₁₀ alkyl, C₂-C₁₀ alkenyl, C₃-C₂₀ cycloalkyl, C₆-C₁₈ aryl, 5-20 membered heteroaryl, and 2-20 membered heteroalkyl;
Preferably, R¹-R⁵ are each independently selected from the group consisting of hydrogen; - OH, -OCH₃, -OCF₃, -OCH₂OH; -OCH₂CH₃; -OCH(CH₃)₂; -F; -Cl; -Br; -CN; NO₂; NH₂; -CH₃, - CH₂F; -CHF₂; -CF₃; -CH₂Cl; -CHCl₂; -CCl₃;CH(CH₃)₂; -CH₂CH₃;
-C(O)OR₄₂, wherein R⁴² is selected from -CH₃; -CH₂CH₃, -CH₂CH₂CH₃; -CH(CH₃)₂; - CH₂CH₂CH₂CH₃; 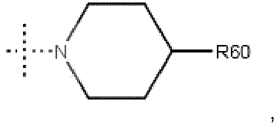
wherein R⁶⁰ is selected from hydrogen or -CH₃;
optionally, wherein R¹ and R², R² and R³, R³ and R⁴, or R⁴ and R⁵ together, preferably R¹ and R² or R² and R³ together, form a five- or six-membered cyclic or heterocyclic ring, preferably selected from

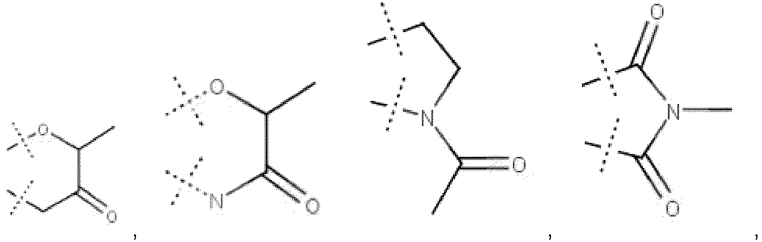

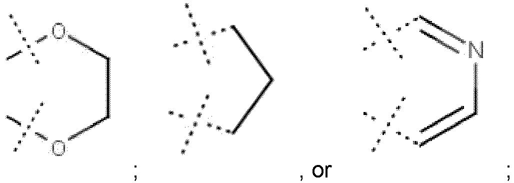

In a compound of Formula (Ia) or (la1), A is preferably a C₆-C₁₈ aryl, a 5-10 membered heteroaryl, 5-10 membered heteroaryl, preferably a benzene, a oxadiazole, a thiadiazole, a isothiazole, or a thiophene.

Preferably, A in a compound of Formula (la) or (la1) is selected from a benzene, a oxadiazole, a thiadiazole, a isothiazole, or a thiophene as defined in the following under sections a) to e):
a) a benzene group of the following structure: 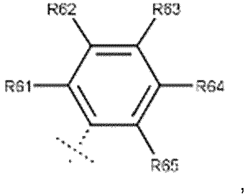 wherein R⁶¹-R⁶⁵ are each independently selected from the group consisting of:
   hydrogen; -OH, -OCH₃, -OCF₃, -OCHF₂, -OCH₂OH; -OCH₂CH₃; -OCH(CH₃)₂; -F; -Cl; -Br; - CN; NO₂; NH₂; -CH₃, -CH₂F; -CHF₂; -CF₃; -CH₂Cl; -CHCl₂; -CCl₃;CH(CH₃)₂; -CH₂CH₃; -CH₂-CH₂-CH(CH₃)₂; -CH₂-N(CH₃)₂; -CH(CH₃)-NHC(O)CH₃; -C(O)-C₂H₅; -C(O)-CH₃; -C(O)-aziridine; -C(O)-azetidine; -C(O)-pyrrolidine; -CH₂NHS(O)₂CH₃; -C(CH₃)=NOH; -S(O)₂-cyclopentyl; -S(O)₂-CH₃; -S(O)₂-cyclopentyl; pyrrolidinyl, 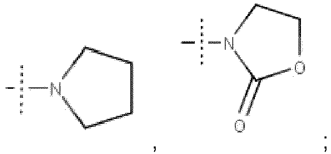 or
   -NHC(O)-R₄₁, wherein R₄₁ is selected from C₁-C₁₀ cycloalkyl, C₁-C₁₀ cycloheteroalkyl, furan, tetrahydrofuran, cyclopentyl, cyclobutyl, cyclopropyl, -NH-CH(CH₃)₂, -NH-cyclopropyl, -CH₂F; -CHF₂; -CF₃; -CH₂Cl; -CHCl₂; -CCl₃; -CH₃, CH₂CH₃, CHCH(CH₃)₂; or
   -C(O)OR₄₂, wherein R⁴² is selected from C₁-C₅ alkyl, including -CH₃; -CH₂CH₃, -CH₂CH₂CH₃; -CH(CH₃)₂; -CH₂CH₂CH₂CH₃; or
   -NHR₄₃, wherein R₄₃ is selected from 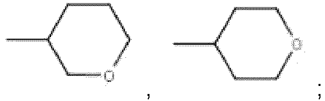 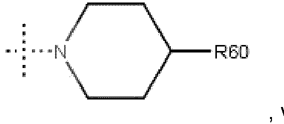 wherein R⁶⁰ is selected from hydrogen or -CH₃; or 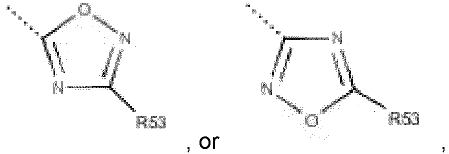 wherein R⁵³ is preferably selected from the group consisting of hydrogen, OH, -OCH₃, -OCF₃, -OCHF₂, -OCH₂OH; -OCH₂CH₃; -F; -Cl; - Br; -CN; NO₂; NH₂; C₁-C₅ alkyl, C₁-C₅ alkoxy, C₂-C₅ alkenyl, preferably -CH₃, CH(CH₃)₂ - CH₂CH₃; -CH₂-CH₂-CH(CH₃)₂; -CH₂-N(CH₃)₂; -CH(CH₃)-NHC(O)CH₃; -CH₂F; -CHF₂; -CF₃; - CH₂Cl; -CHCl₂; -CCl₃; -O-N=O, -N=O, -CH=NH, -N₃, -SH, -SO₃H, -SO₂H, -S(O)₂CH₃ -CN, - NC,-OCN, -NCO, -(C=O)OH, -(C=O)NHOH, -NH(C=O)NH₂, - 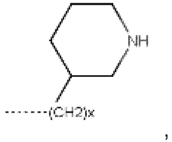 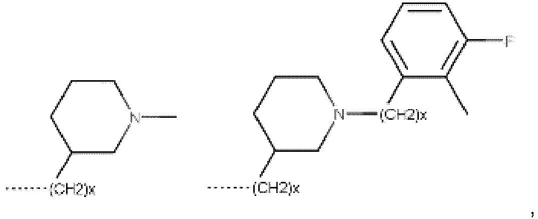 with X = 0 or 1, preferably 1; , 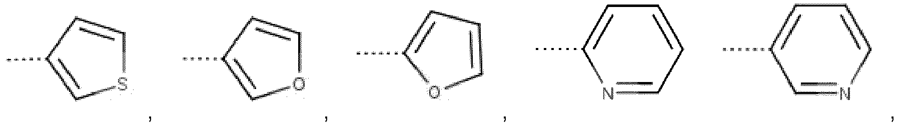 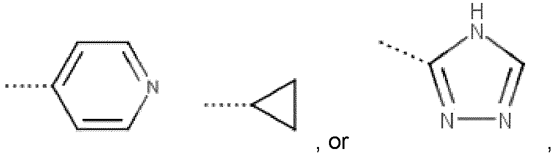
   or R⁵³ is 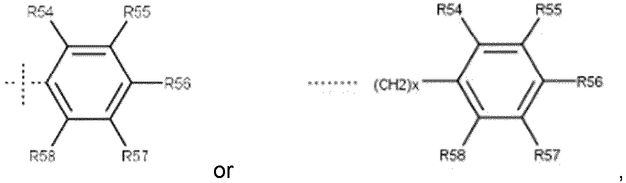 **wherein** R⁵⁴, R⁵⁵, R⁵⁶, R⁵⁷ or R⁵⁸ are selected each independently from hydrogen, -CH₃, -C₂H₅, -CH₂F; -CHF₂; - CF₃; -CH₂Cl; -CHCl₂; -CCl₃; --OCH₃, -F, -Cl, -Br, NO₂, NH₂, and x is 0, 1 or 2;
   or R⁵³ is 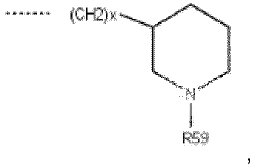 wherein R⁵⁹ is 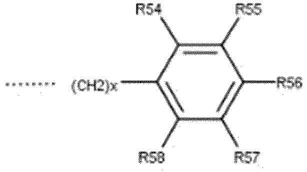 and wherein R⁵⁴, R⁵⁵, R⁵⁶, R⁵⁷ or R⁵⁸ are selected separately from the above from hydrogen, - CH₃, -C₂H₅, -CH₂F; -CHF₂; -CF₃; -CH₂Cl; -CHCl₂; -CCl₃; -OCH₃, -F, -Cl, -Br, NO₂, NH₂, and x is 0, 1 or 2; as defined above, and x is 0, 1 or 2;
      and
   optionally, wherein R⁶¹ and R⁶², R⁶² and R⁶³, R⁶³ and R⁶⁴, or R⁶⁴ and R⁶⁵ together, preferably R⁶² and R⁶³ together, form a five- or six-membered cyclic or heterocyclic ring, preferably selected from 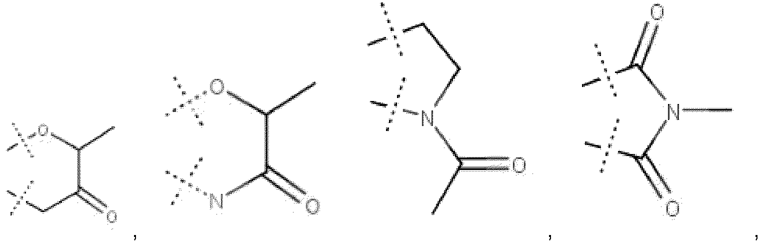 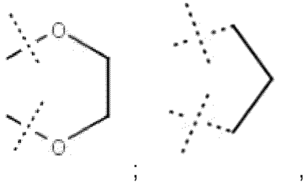
   wherein preferably either all of R¹ to R⁵ are hydrogen or 1, 2 or 3, preferably 1 or 2 of R¹ to R⁵ are different from hydrogen and selected from substitutions defined above for R¹ to R⁵ and remaining substituents of R¹ to R⁵ are hydrogen;
   Likewise, preferably either all of R⁶¹ to R⁶⁵ are hydrogen or 1, 2 or 3, preferably 1 or 2 of R¹ to R⁵ are different from hydrogen and selected from substitutions defined above for R⁶¹ to R⁶⁵ and remaining substituents of R⁶¹ to R⁶⁵ are hydrogen;
   Likewise preferably, X is then not present in Formula (la) and (la1), or may be selected from -CH₂-, -C₂H₄-, -C₃H₆-, -CHCH(CH₃)₂-, -CHC(CH₃)₃-, -CH(CH(CH₃)₂)-, -CH(CH₂CH(CH₃)₂)-, or -CH(CH₂OH)-. More preferably, X is not present.
b) an oxadiazole group, preferably selected from one of the following structures: 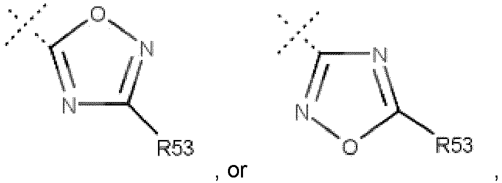 wherein
   R⁵³ is preferably selected from the group consisting of is hydrogen, a C₁-C₁₀ alkyl, 2-20 membered heteroalkyl, C₃-C₂₀ cycloalkyl, 3-20 membered cycloheteroalkyl, C₆-C₁₈ aryl or 5-20 membered heteroaryl, preferably a C₆-C₁₄ aryl or 5-20 membered heteroaryl;, more preferably from hydrogen, OH, -OCH₃, -OCF₃, -OCHF₂, -OCH₂OH; -OCH₂CH₃; -F; -CI; -Br; - CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -CH₂F; -CHF₂; -CF₃; -CH₂Cl; - CHCl₂; -CCl₃; -O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, -NH(C=O)NH₂, -NH(C=S)NH₂, -(C=O)NH₂,-CHO,-CHS, - OCHO, -NCHO, - 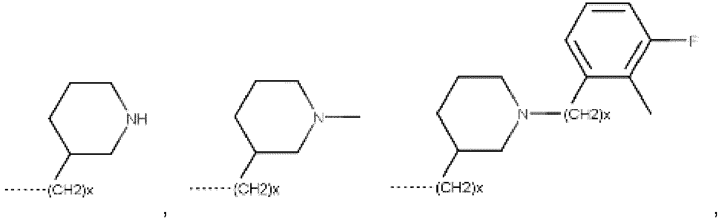 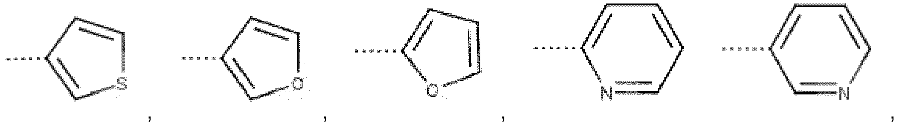 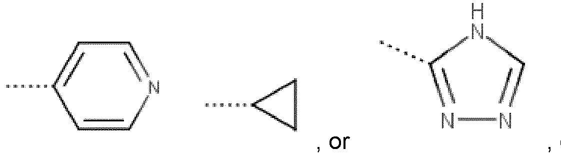 or
   R⁵³ is 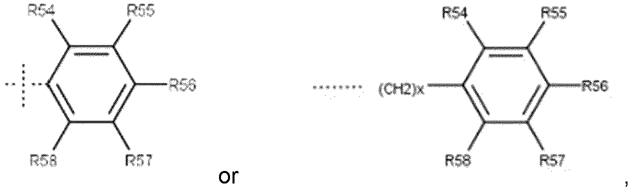 **wherein** R⁵⁴, R⁵⁵, R⁵⁶, R⁵⁷ or R⁵⁸ are selected each independently from hydrogen, -CH₃, -C₂H₅, -CH₂F; -CHF₂; -CF₃; -CH₂Cl; -CHCl₂; -CCl₃; -OCH₃, -F, -Cl, -Br, NO₂, NH₂, and x is 0, 1 or 2;
   or R⁵³ is 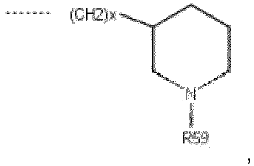 wherein R⁵⁹ is 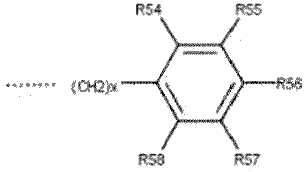 and wherein R⁵⁴, R⁵⁵, R⁵⁶, R⁵⁷ or R⁵⁸ are selected separately from the above from hydrogen, -CH₃, -C₂H₅, - CH₂F; -CHF₂; -CF₃; -CH₂Cl; -CHCl₂; -CCl₃; -OCH₃, -F, -Cl, -Br, NO₂, NH₂, and x is 0, 1 or 2; as defined above, and x is 0, 1 or 2;
   and wherein preferably either all of R⁵⁴ to R⁵⁸ are hydrogen or 1, 2 or 3, preferably 1 or 2 of R⁵⁴ to R⁵⁸ are different from hydrogen and selected from substitutions presented above and remaining positions of R⁵⁴ to R⁵⁸ are selected from hydrogen;
   Preferably, X is then selected in Formula (Ia) or (la1), from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
   Alternatively, X may not be present.
c) a thiadiazole group, preferably selected from one of the following structures: 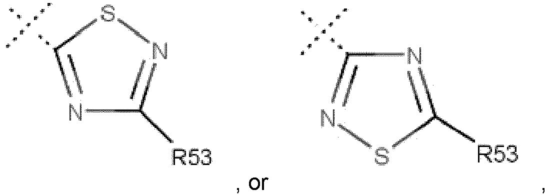
   wherein R⁵³ is preferably as defined herein above.
   Preferably, X is then selected in formula (la) or (la1), from -CH2-, -C2H4-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
   Alternatively, X may not be present.
d) an isothiazole group, preferably selected from of the following structures: 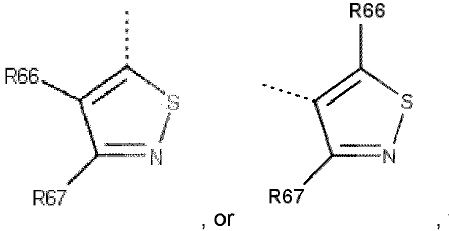 wherein R⁶⁶ and R⁶⁷ are each selected independently from hydrogen, -CH₃, -C₂H₅, -CH₂F; -CHF₂; -CF₃; -CH₂Cl; -CHCl₂; -CCl₃; -F, - Cl, -Br, preferably wherein either of R⁶⁶ or R⁶⁷ is hydrogen and the other residue is selected from -CH₃, -C₂H₅, -CF₃, -CHF₂, -F, -Cl, -Br.
   Preferably, X is then selected in Formula (la) or (la1), from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.
e) a thiophene group, preferably selected from of the following structures 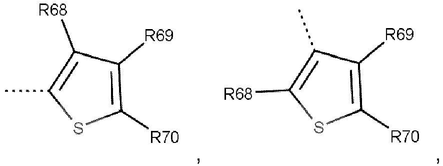 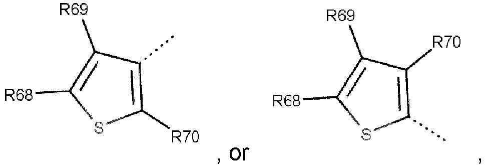
   wherein R⁶⁸, R⁶⁹ and R⁷⁰ are each selected independently from hydrogen, -CH₃, -C₂H₅, - CH₂F; -CHF₂; -CF₃; -CH₂Cl; -CHCl₂; -CCl₃;-F, -Cl, -Br, preferably wherein either of R⁶⁸, R⁶⁹ or R⁷⁰ is hydrogen and the other residue is selected from -CH₃, -C₂H₅, -CF₃, -CHF₂, -F, -Cl, -Br.

Preferably, X is then selected in Formula (Ia) or (la1), from -CH₂-, -C₂H₄-, -C₃H₆-, - CHCH(CH₃)₂-, -CHC(CH₃)₃-, -CH(CH(CH₃)₂)-, -CH(CH₂CH(CH₃)₂)-, or -CH(CH₂OH)-.

Alternatively, X may not be present.

According to one particuarly preferred embodiment, the present invention relates to a compound of Formula (la) or (la1), 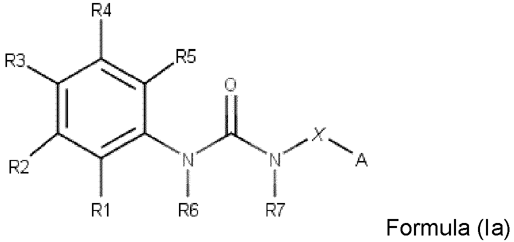 or 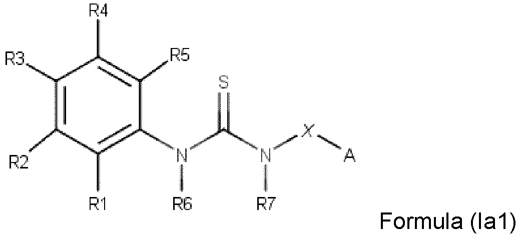 or a pharmaceutically acceceptable salt thereof, wherein A is the following aryl group: 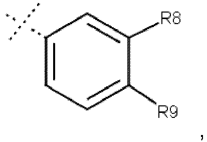
and X is preferably a C₁-C₁₀ alkyl group, e.g. is selected from -CH₂-, -C₂H₄-, -C₃H₆-, - CHCH(CH₃)₂-, -CHC(CH₃)₃-, -CH(CH(CH₃)₂)-, or -CH(CH₂CH(CH₃)₂)-, or -CH(CH₂OH)-, or X is not present; and wherein
preferably R¹, R², R³, R⁴, R⁵, R⁸ and R⁹ are each independently hydrogen, -F, -Cl, -OH, -NH₂, -S(O)₂CH₃ -(C=O)OH, -NH(C=O)NH₂, -(C=O)NH₂,-CHO,- -OCHO, -NCHO C₁-C₅ alkyl, C₁-C₅ alkoxy, C₂-C₆ alkenyl, C₃-C₇ cycloalkyl, C₃-C₇ cycloalkyl C₁-C₅ alkyl, C₂-C₁₀ cycloheteroalkyl or C₂-C₁₄ cycloheteroalkyl C₁-C₅ alkyl, C₆-C₁₄ aryl, C₆-C₁₄ aryl C₁-C₅ alkyl, C₂-C₁₄ heteroaryl, C₂-C₁₄ heteroaryl C₁-C₅ alkyl, (C=O)R¹¹, (C=O)OR¹¹, (C=O)NR¹¹R¹², (SO₂)R¹¹, C(NR¹¹)R¹², - NHC(O)R¹¹, or -NHC(O)NHR¹¹, wherein R¹¹ and R¹² are independently selected from the group consisting of C₁-C₁₀ alkyl, C₂-C₁₀ alkenyl, C₃-C₂₀ cycloalkyl, C₆-C₁₈ aryl, 5-20 membered heteroaryl, 2-20 membered heteroalkyl, ; -OCH₃, -OCF₃, -OCHF₂, -OCH₂OH; -OCH₂CH₃; - OCH(CH₃)₂; -F; -Cl; -Br; -CH₂F; -CHF₂; -CF₃; -CH₂Cl; -CHCl₂; and -CCl₃; -CN; NO₂; NH₂; -CH₃, CH(CH₃)₂; -CH₂CH₃; or
-C(O)OR₄₂, wherein R⁴² is selected from -CH₃; -CH₂CH₃, -CH₂CH₂CH₃; -CH(CH₃)₂; - CH₂CH₂CH₂CH₃; or 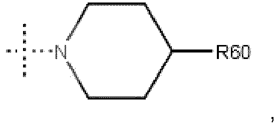 wherein R⁶⁰ is selected from hydrogen or -CH₃, or, alternatively, R¹ and R², R² and R³, R³ and R⁴, R⁴ and R⁵, or R⁸ and R⁹, preferably R¹ and R² or R² and R³ together, more preferably R² and R³ together, when taken together form a five- or six-membered cycloalkyl, five- or six-membered cycloheteroalkyl, five- or six-membered aryl, or five- or six-membered heteroaryl, preferably selected from 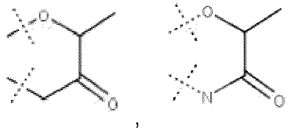 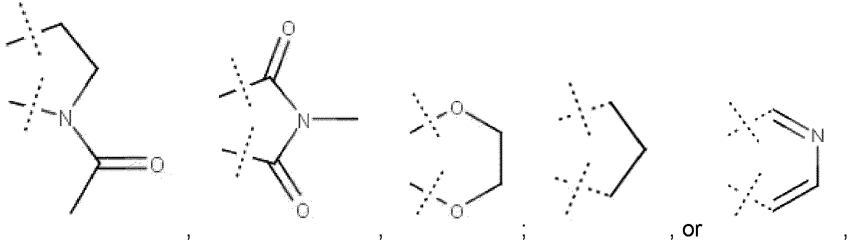 or
R⁸ and R⁹, when taken together form a optionally such a substituted five- or six-membered cycloalkyl, five- or six-membered cycloheteroalkyl, five- or six-membered aryl, or five- or six-membered heteroaryl, and
R⁶ and R⁷ are each independently hydrogen, a C₁-C₁₀ alkyl, preferably hydrogen, -CH₃, - C₂H₅, -C₃H₇, or -C₃H₉, or R⁶ and R⁷ when taken together form a five- or six-membered heterocyclic ring, preferably selected from 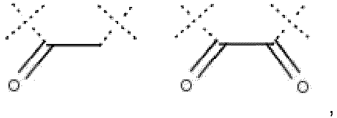 and 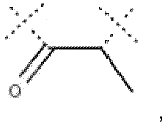 or optionally,
R⁷, N and X when taken together form a five- or six-membered heterocyclic ring, preferably selected from 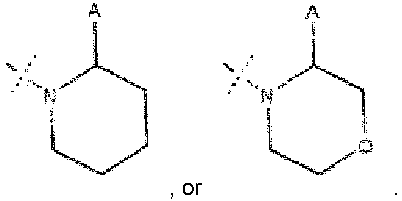
R⁸ or R⁹ may be further selected from 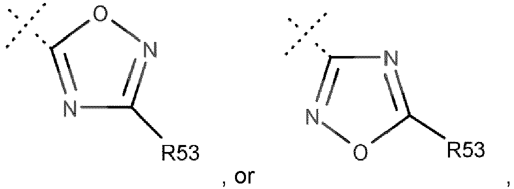 wherein R⁵³ is preferably selected from the group consisting of hydrogen, OH, -OCH₃, -OCF₃, -OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -CH₂F; -CHF₂; -CF₃; -CH₂Cl; -CHCl₂; -CCl₃;, -O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, -NH(C=O)NH₂, 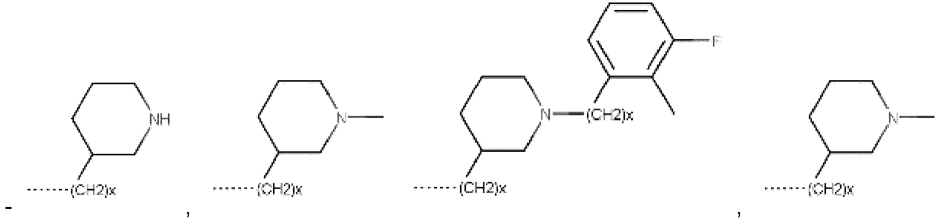 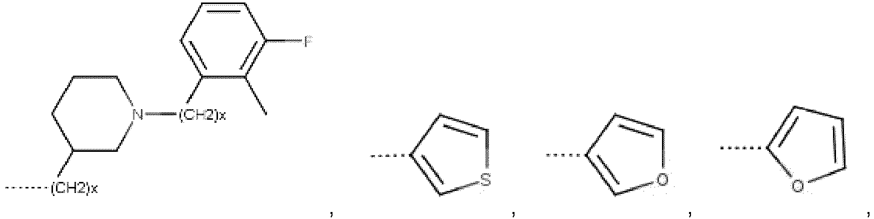 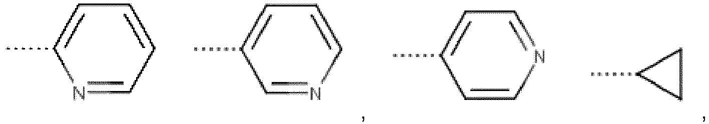 or 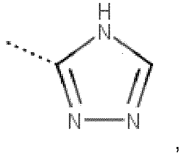 or
R⁵³ is 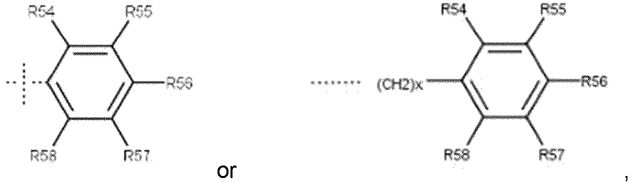 wherein R⁵⁴, R⁵⁵, R⁵⁶, R⁵⁷ or R⁵⁸ are selected each independently from hydrogen, -CH₃, -C₂H₅, -CH₂F; -CHF₂; -CF₃; -CH₂Cl; -CHCl₂; -CCl₃; OCH₃, -F, -Cl, -Br, NO₂, NH₂, and x is 0, 1 or 2;
or R⁵³ is 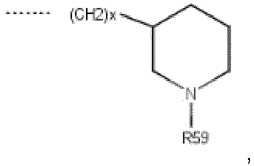 wherein R⁵⁹ is 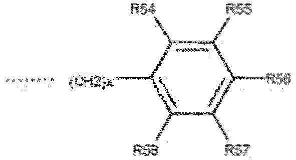 and wherein R⁵⁴, R⁵⁵, R⁵⁶, R⁵⁷ or R⁵⁸ are selected separately from the above from hydrogen, -CH₃, -C₂H₅, - CH₂F; -CHF₂; -CF₃; -CH₂Cl; -CHCl₂; -CCl₃; -OCH₃, -F, -Cl, -Br, NO₂, NH₂, and x is 0, 1 or 2; as defined above, and x is 0, 1 or 2;
and wherein preferably either all of R⁵⁴ to R⁵⁸ are hydrogen or 1, 2 or 3, preferably 1 or 2 of R⁵⁴ to R⁵⁸ are different from hydrogen and selected from substitutions presented above and remaining positions of R⁵⁴ to R⁵⁸ are selected from hydrogen;
preferably with X = 0 or 1, preferably 1, or R⁸and R⁹, when taken together form a five- or six-membered cycloalkyl, five- or six-membered cycloheteroalkyl, five- or six-membered aryl, or five- or six-membered heteroaryl.

In a compound of the invention according to Formula (la) or (la1) as defined above comprising as a moiety A the following aryl group: 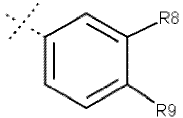 as defined above, preferably R⁸ to R⁹ are selected as follows
R⁸ is hydrogen, or pyrrolidinyl, 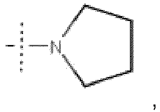 methyl, C(O)-azetidine, -F, -Cl, -CH(CH₃)₂, - CN, -OCHF₂, -NH₂, 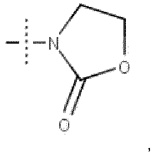 -C(CH₃)=NOH, -NO₂, -CH(CH₃)-NHC(O)CH₃, or CH₂N(CH₃)₂,
R⁹ is hydrogen,- methyl, -OCH₃, -OCF₃, -CH₂F; -CHF₂; -CF₃; -CH₂Cl; -CHCl₂; -CCl₃; -S(O)₂CH₃, -CH₂NHS(O)₂CH₃,, -C(O)₂CH₃, or -NHC(O)-R₄₁, wherein R₄₁ is methyl, -NH-CH(CH₃)₂, furan, tetrahydrofuran, cyclopropyl, -NH-cyclopropyl, cyclopentyl, -NH-tetrahydropyran, -CHF₂, or C(O)₂CH₃, or 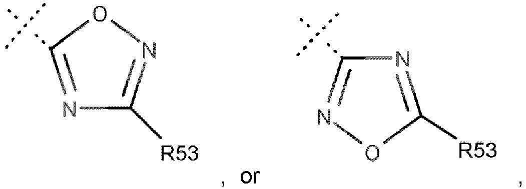 preferably with R⁵³ as defined above;
or R⁸ and R⁹ together, form a six-membered heterocyclic ring selected from

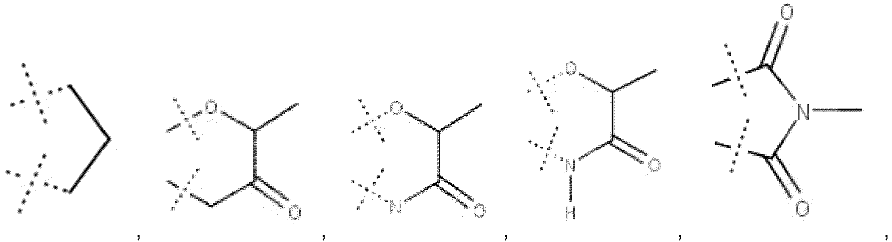

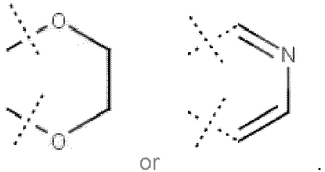

Preferably the proviso applies that R⁸ is not identical to R⁹,

Particularly preferable in a compound of the invention according to Formula (la) or (la1) as defined above comprising as a moiety A the following aryl group: 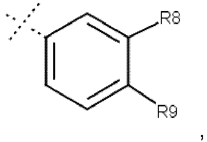 preferably R¹ to R⁵ are selected as follows:
R¹ is hydrogen, -C₁-C₂ alkoxy, -C(O)O-C₁-C₅ alkyl, -OCH₃, -C₁-C₃ alkyl, methyl, or ethyl
R² is hydrogen, methyl, -F, -Cl, -Br, -CN, -OCH₃, or -C(O)O-C₁-C₅ alkyl;
R³ is hydrogen, methyl, -CH(CH₃)₂, -CHF₂, -F, -Cl, -Br, -CN, -OCH₃, -OCF₃, NO₂, or -C(O)O-(C₁-C₅ alkyl);
R⁴ is hydrogen or -F, -Cl, -Br, or -CF₃; and/or
R⁵ is hydrogen, methyl, ethyl or -F, -Cl, -Br, or -CN, or -CH₂-phenyl.

Preferably the proviso applies that R⁸ is not identical to R⁹.

Likewise preferably, R⁸ and R⁹ are defined as indicated above.

In view of the above, in one embodiment the present invention therefore also relates to a compound of Formula (Ia) or (Ia1), wherein the compound is a compound of Formula (la2) or (la3), wherein X is not present: 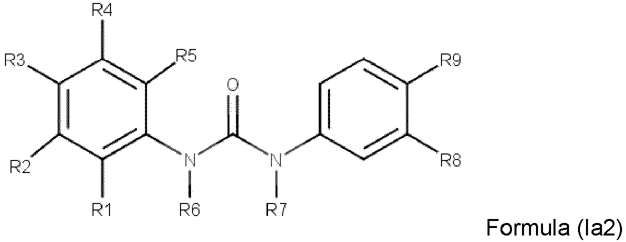 or

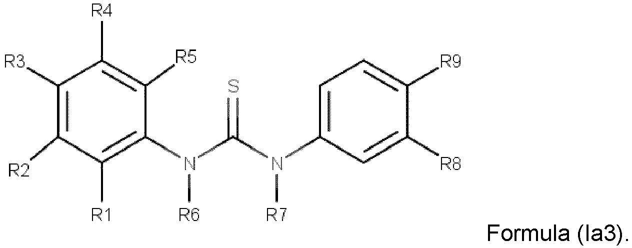

Typically, R¹, R², R³, R⁴, R⁵, R⁸ and R⁹ are preferably as defined above for Formula (la) or (la1),
In one embodiment, the present invention relates to a compound of Formula (la) or (la1), wherein the compound is a compound of Formula (la2) or (la3)
wherein R¹, R², R³, R⁴, R⁵, and R⁸ are each independently hydrogen, -F, -Cl, -Br, -NO₂,-ON=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, C₁-C₁₀ alkyl, C₁-C₁₀ alkoxy, C₂-C₁₀ alkenyl, C₃-C₂₀ cycloalkyl, C₃-C₂₀ cycloalkyl C₁-C₁₀ alkyl, 3-20 membered cycloheteroalkyl or 3-20 membered cycloheteroalkyl C₁-C₁₀ alkyl, C₆-C₁₈ aryl, C₆-C₁₈ aryl C₁-C₁₀ alkyl, 5-20 membered heteroaryl, 5-20 membered heteroaryl C₁-C₁₀ alkyl, (C=O)R¹¹, (C-O)OR¹¹, (C-O)SR¹¹, (C=O)NR¹¹R¹², (SO₂)R¹¹, (SNO)R¹¹, (P=O)OR¹¹OR¹² or C(NR¹¹)R¹², -NHC(O)R¹¹, or -NHC(O)NHR¹¹, wherein R¹¹ and R¹² are independently selected from the group consisting of C₁-C₁₀ alkyl, C₂-C₁₀ alkenyl, C₃-C₂₀ cycloalkyl, C₆-C₁₈ aryl, 5-20 membered heteroaryl, 2-20 membered heteroalkyl, -CH₂F; -CHF₂; -CF₃; -CH₂Cl; -CHCl₂; and -CCl₃; and
wherein R⁹ is selected from C₁-C₃ alkyl, five-membered heterocyclic ring, -OCH₃, -S(O)₂CH₃, -NHC(O)R¹¹, or -NHC(O)NHR¹¹, wherein R¹¹ is selected from C₂-C₅ alkyl, C₃-C₆ cycloalkyl, - CH₂F; -CHF₂; -CF₃; -CH₂Cl; -CHCl₂; -CCl₃; or five-membered heterocyclic ring.

In a preferred embodiment, R⁹ is selected from C₁-C₃ alkyl, pyrrolidine, -OCH₃, -S(O)₂CH₃, - NHC(O)R¹¹, or -NHC(O)NHR¹¹, wherein R¹¹ is selected from C₂-C₅ alkyl, C₃-C₆ cycloalkyl, - CH₂F; -CHF₂; -CF₃; -CH₂Cl; -CHCl₂; -CCl₃;; or tetrahydrofuran or furan.

In more preferred embodiment, R⁹ is selected from C₁ alkyl, C₃ alkyl, pyrrolidine, -OCH₃, - S(O)₂CH₃, -NHC(O)R¹¹, or -NHC(O)NHR¹¹, wherein R¹¹ is selected from C₅ cycloalkyl, -CHF₂, or tetrahydrofuran or furan.

In one embodiment, the present invention relates to a compound of Formula (la) or (la1), wherein the compound is a compound of Formula (la1)
wherein R¹, R², R³, R⁴, R⁵, and R⁹ are each independently hydrogen, -F, -Cl, -Br, -NO₂,-ON=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, C₁-C₁₀ alkyl, C₁-C₁₀ alkoxy, C₂-C₁₀ alkenyl, C₃-C₂₀ cycloalkyl, C₃-C₂₀ cycloalkyl C₁-C₁₀ alkyl, 3-20 membered cycloheteroalkyl or 3-20 membered cycloheteroalkyl C₁-C₁₀ alkyl, C₆-C₁₈ aryl, C₆-C₁₈ aryl C₁-C₁₀ alkyl, 5-20 membered heteroaryl, 5-20 membered heteroaryl C₁-C₁₀ alkyl, (C=O)R¹¹, (C=O)OR¹¹, (C-O)SR¹¹, (C=O)NR¹¹R¹², (SO₂)R¹¹, (SNO)R¹¹, (P=O)OR¹¹OR¹² or C(NR¹¹)R¹², -NHC(O)R¹¹, or -NHC(O)NHR¹¹,
wherein R¹¹ and R¹² are independently selected from the group consisting of C₁-C₁₀ alkyl, C₂-C₁₀ alkenyl, C₃-C₂₀ cycloalkyl, C₆-C₁₈ aryl, 5-20 membered heteroaryl, 2-20 membered heteroalkyl, -CH₂F; -CHF₂; -CF₃; -CH₂Cl; -CHCl₂; and -CCl₃; and
R⁸ is -S(O)₂CH₃, -NHC(O)R¹¹, or -NHC(O)NHR¹¹, wherein R¹¹ is selected from C₂-C₅ alkyl, C₃-C₅ cycloalkyl, -CHF₂; or five-membered heterocyclic ring.

In a preferred embodiment R⁸ is -S(O)₂CH₃, -NHC(O)R¹¹, or -NHC(O)NHR¹¹, wherein R¹¹ is selected from C₃ alkyl, C₃ cycloalkyl, -CHF₂.

In one embodiment, the present invention relates to a compound of Formula (la) or (la1), wherein the compound is a compound of Formula (la2) or (la3),
wherein R¹, R², R³, R⁴, and R⁵, are each independently hydrogen, -F, -Cl, -Br, -NO₂,-O-N=O, - N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, -NH(C=S)NH₂, -(C=O)NH₂,-CHO,-CHS, -OCHO, -NCHO, -B(OH)₂, C₁-C₁₀ alkyl, C₁-C₁₀ alkoxy, C₂-C₁₀ alkenyl, C₃-C₂₀ cycloalkyl, C₃-C₂₀ cycloalkyl C₁-C₁₀ alkyl, 3-20 membered cycloheteroalkyl or 3-20 membered cycloheteroalkyl C₁-C₁₀ alkyl, C₆-C₁₈ aryl, C₆-C₁₈ aryl C₁-C₁₀ alkyl, 5-20 membered heteroaryl, 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², - NHC(O)R¹¹, or -NHC(O)NHR¹¹, wherein R¹¹ and R¹² are independently selected from the group consisting of C₁-C₁₀ alkyl, C₂-C₁₀ alkenyl, C₃-C₂₀ cycloalkyl, C₆-C₁₈ aryl, 5-20 membered heteroaryl, 2-20 membered heteroalkyl, -CH₂F; -CHF₂; -CF₃; -CH₂Cl; -CHCl₂; and -CCl₃;; and,
wherein R⁸ and R⁹, when taken together form a heterocyclic ring is selected from:

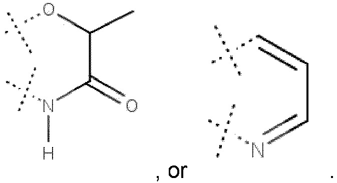

Alternatively, in one further embodiment, the present invention relates to a compound of Formula (Ia) or (Ia1), wherein the -X-A group is 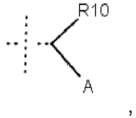 wherein R¹⁰ is hydrogen or C₁-C₃ alkyl, preferably -CH₂-, -C₂H₄-, -C₃H₆-, -CH (CH₃)₂, -CHCH(CH₃)₂-, -CHC(CH₃)₃-, - CH(CH(CH₃)₂)-, or -CH(CH₂CH(CH₃)₂)-, or -CH(CH₂OH)-,
or -(CH₂)ₙ-A, wherein n is an integer from 1-3 or in one embodiment, n is 2; in another embodiment, n is 1;
and wherein A is 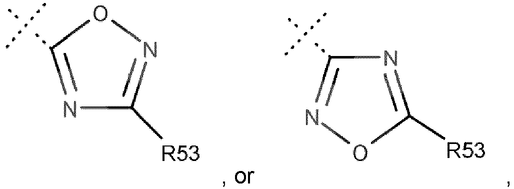 wherein R53 is as defined above.

Accordingly, the invention is also directed to a compound related to Formula (Ia) or (la1) that is further defined according to any of Formulae (Ib), (Ib1), (Ib2) or (Ib3): 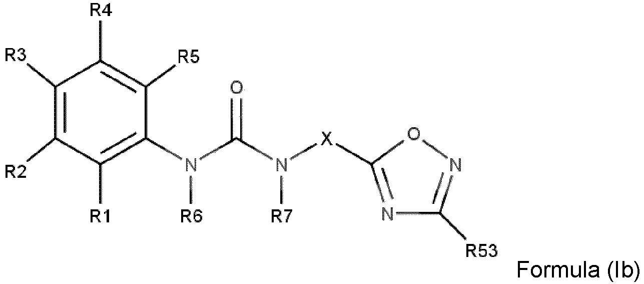 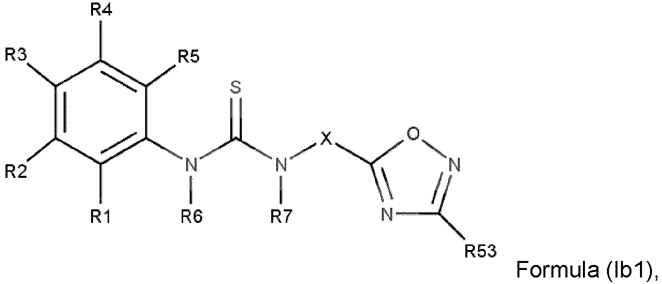 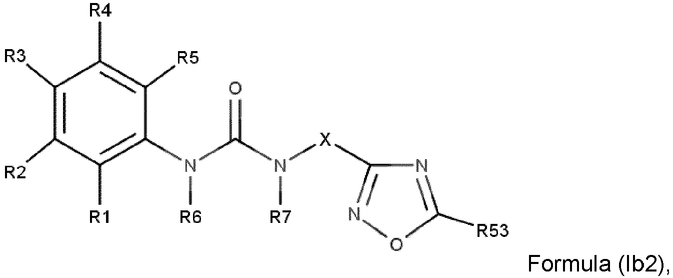 or 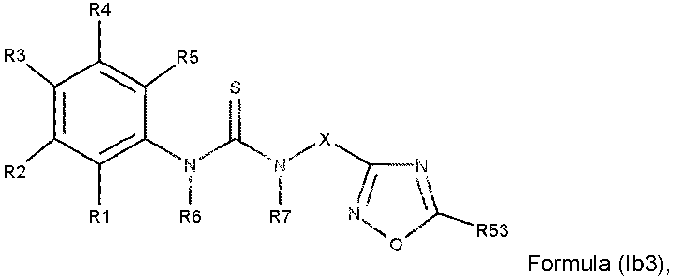
wherein X is selected from the group consisting of a C₁-C₃ alkyl,-CH (CH₃)₂ -CH₂-, -C₂H₄-, - C₃H₆-, -CHCH(CH₃)₂-, -CHC(CH₃)₃-, -CH(CH(CH₃)₂)-, or -CH(CH₂CH(CH₃)₂)-, or - CH(CH₂OH)-, or is not present,
wherein R⁵³ is hydrogen, a C₁-C₁₀ alkyl, 2-20 membered heteroalkyl, C₆-C₁₈ aryl or 5-20 membered heteroaryl, preferably a C₆-C₁₄ aryl or 5-20 membered heteroaryl. Preferably, R⁵³ is as defined above for oxadiazoles, more preferably, R53 is hydrogen, a C₁-C₁₀ alkyl, 2-20 membered heteroalkyl, C₆-C₁₈ aryl or 5-20 membered heteroaryl, preferably a C₆-C₁₄ aryl or 5-20 membered heteroaryl, more preferably wherein R⁵³ is a C₆-C₁₄ aryl or 5-10 membered heteroaryl, preferably a pyridyl or substituted C₆ aryl, more preferably 3-pyridyl or para substituted phenyl, wherein the substituent is selected from the group consisting of -F, -Cl, and -CF₃, -CH₂F; -CHF₂; -CF₃; -CH₂Cl; -CHCl₂; -CCl₃;or
R⁵³ is preferably selected from the group consisting of hydrogen, OH, -OCH₃, -OCF₃, - OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH₂-CH₂-CH(CH₃)₂; -CH₂-N(CH₃)₂; -CH(CH₃)-NHC(O)CH₃; -CH₂F; -CHF₂; -CF₃; -CH₂Cl; -CHCl₂; -CCl₃;-O-N=O, -N=O, -CH=NH, -N₃, -SH, - SO₃H, -SO₂H, -S(O)₂CH₃ -CN, -NC,-OCN, -NCO, -(C=O)OH, -(C=O)NHOH, -NH(C=O)NH₂, - NH(C=S)NH₂, -(C=O)NH₂,-CHO,-CHS, -OCHO, -NCHO, 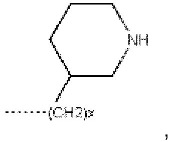 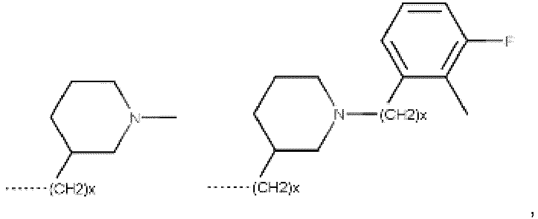 with X = 0 or 1, preferably 1; 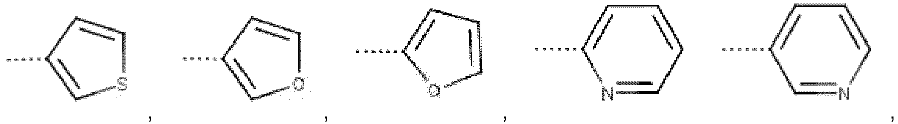 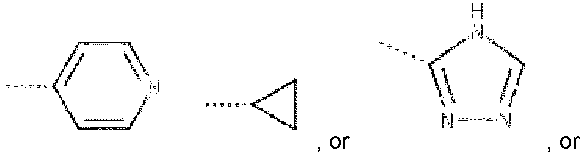
R⁵³ is 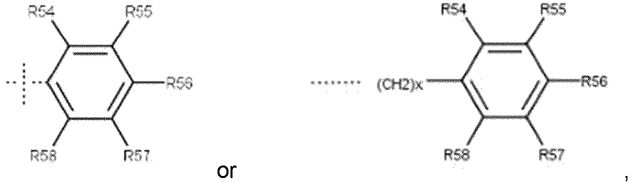 wherein R⁵⁴, R⁵⁵, R⁵⁶, R⁵⁷ or R⁵⁸ are selected each independently from hydrogen, -CH₃, -C₂H₅, -CH₂F; -CHF₂; -CF₃; -CH₂Cl; -CHCl₂; -CCl₃; -OCH₃, -F, -Cl, -Br, NH₂, NO₂, and x is 0, 1 or 2;
or R⁵³ is 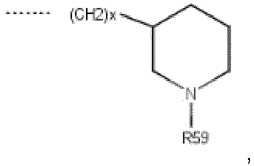 wherein R⁵⁹ is 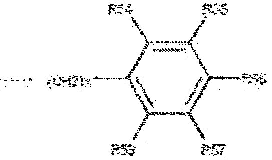 and wherein R⁵⁴, R⁵⁵, R⁵⁶, R⁵⁷ or R⁵⁸ are selected separately from the above from hydrogen, -CH₃, -C₂H₅, - CH₂F; -CHF₂; -CF₃; -CH₂Cl; -CHCl₂; -CCl₃; -OCH₃, -F, -Cl, -Br, NO₂, NH₂, and x is 0, 1 or 2; as defined above, and x is 0, 1 or 2;
and wherein preferably either all of R⁵⁴ to R⁵⁸ are hydrogen or 1, 2 or 3, preferably 1 or 2 of R⁵⁴ to R⁵⁸ are different from hydrogen and selected from substitutions presented above and remaining positions of R⁵⁴ to R⁵⁸ are selected from hydrogen.
R¹, R², R³, R⁴, and R⁵ in Formula (Ib), (Ib1), (Ib2) or (Ib3) are preferably each independently hydrogen, -F, -Cl, -Br, -NO₂,-O-N=O, -N=O, -OH, -NH₂, -CH=NH, -N₃, -SH, -SO₃H, -SO₂H, - S(O)₂CH₃ -CN, -NC,-OCN, -NCO, -SCN, -NCS, -(P=O)(OH)₂, -O(P=O)(OH)₂, -(C=O)OH, - (C=O)NHOH, -NH(C=O)NH₂, -NH(C=S)NH₂, -(C=O)NH₂,-CHO,-CHS, -OCHO, -NCHO, - B(OH)₂, C₁-C₁₀ alkyl, C₁-C₁₀ alkoxy, C₂-C₁₀ alkenyl, C₃-C₂₀ cycloalkyl, C₃-C₂₀ cycloalkyl C₁-C₁₀ alkyl, 3-20 membered cycloheteroalkyl or 3-20 membered cycloheteroalkyl C₁-C₁₀ alkyl, C₆-C₁₈ aryl, C₆-C₁₈ aryl C₁-C₁₀ alkyl, 5-20 membered heteroaryl, 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹, wherein R¹¹ and R¹² are independently selected from the group consisting of C₁-C₁₀ alkyl, C₂-C₁₀ alkenyl, C₃-C₂₀ cycloalkyl, C₆-C₁₈ aryl, 5-20 membered heteroaryl, 2-20 membered heteroalkyl, -CH₂F; -CHF₂; -CF₃; -CH₂Cl; -CHCl₂; and -CCl₃; or
R⁶ and R⁷ are each independently hydrogen, a C₁-C₁₀ alkyl or R⁶ and R⁷ when taken together form a five- or six-membered heterocyclic ring.

According to a particularly preferred embodiment in any of Formulae (Ia), (la1), (la2), (la3), (Ib), (Ib1), (Ib2) or (Ib3) or (Id) defined herein, preferably,
R¹ is hydrogen, -C₁-C₂ alkoxy, -C(O)O-C₁-C₅ alkyl, -OCH₃, -C₁-C₃ alkyl, methyl, or ethyl
R² is hydrogen, methyl, -F, -Cl, -Br, -CN, -OCH₃, or -C(O)O-C₁-C₅ alkyl;
R³ if present, is hydrogen, methyl, -CH(CH₃)₂, -CHF₂, -F, -Cl, -Br, -CN, -OCH₃, -OCF₃, NO₂, or -C(O)O-C₁-C₅ alkyl;
R⁴ is hydrogen or -F, -Cl, -Br, or -CF₃; and/or
R⁵ is hydrogen, methyl, ethyl or -F, -Cl, -Br, -CN, or -CH₂-phenyl.

Preferably, R² and R³ together, form a five- or six-membered cyclic or heterocyclic ring, preferably selected from

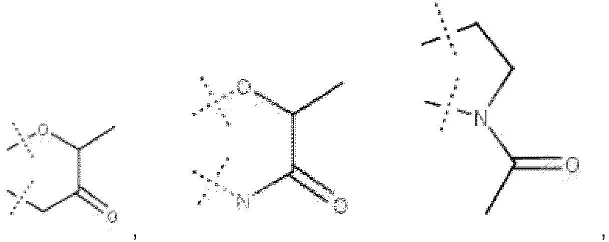

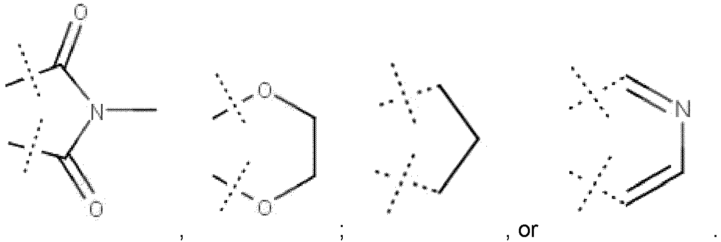

In one particular embodiment of the invention, in Formula (la) or (la1), A is a oxadiazole. In one embodiment, A is 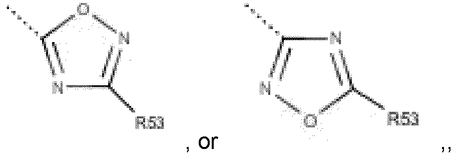
wherein R⁵³ is a C₆-C₁₄ aryl or 5-10 membered heteroaryl, preferably R⁵³ is pyridyl or substituted C₆ aryl, more preferably 3-pyridyl or para substituted phenyl, wherein the substituent is selected from the group consisting of -F, -Cl, -CH₂F; -CHF₂; -CF₃; -CH₂Cl; - CHCl₂; and -CCl₃;
In one embodiment, the present invention relates to a compound of any one of Formulae (la), (la1), (la2), (la3), (Ib), (Ib1), (lb2) or (lb3), or (Id), wherein R² and R⁵ are preferably each hydrogen,
R¹ is hydrogen or methoxy,
R³, if present, is hydrogen or methyl and
R⁴ is hydrogen or -F, wherein at least one of R¹ to R⁵ is not hydrogen, and preferably R⁵³ is as defined above.

In one embodiment, the present invention relates to a compound of Formula (la) or (la1), wherein the compound is a compound of Formula (Id): 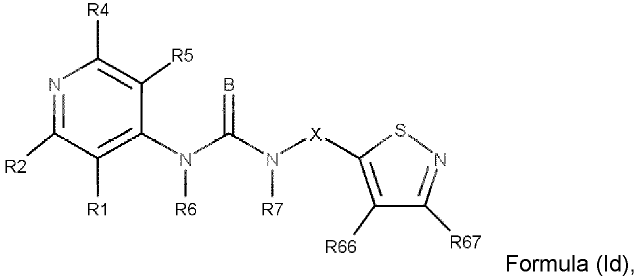 wherein preferably
B is O or S, preferably O,
X is preferably selected from C1-C5-alkyl, -CH₂-, -C₂H₄-, -C₃H₆-, -CH(CH₃)₂-, -CHCH(CH₃)₂-, -CHC(CH₃)₃-, -CH(CH(CH₃)₂)-, -CH(CH₂CH(CH₃)₂)-, or -CH(CH₂OH)-; or is not present;
R¹, R², R⁴ and R⁵ are preferably selected separately from hydrogen, -CH₃, -C₂H₅, -CH₂CH₃, - CH₂F; -CHF₂; -CF₃; -CH₂Cl; -CHCl₂; -CCl₃; -OCH₃, -F, -Cl, -Br, NO₂, NH₂, preferably wherein two or three of R¹, R², R⁴ and R⁵ are hydrogen and the remaining are not hydrogen; and
R⁵⁴ and R⁵⁵ are each preferably selected separately from hydrogen, -CH₃, -C₂H₅, -CH₂F; - CHF₂; -CF₃; -CH₂Cl; -CHCl₂; -CCl₃;-OCH₃, -F, -Cl, -Br, NO₂, NH₂, preferably wherein one of R⁵⁴ and R⁵⁵ is hydrogen and the other is one of -CH₃, -C₂H₅, -CH₂F; -CHF₂; -CF₃; -CH₂Cl; - CHCl₂; -CCl₃; -OCH₃, -F, -Cl, -Br, NO₂, NH₂.

In one further embodiment, the present invention relates to a compound of the following structure:

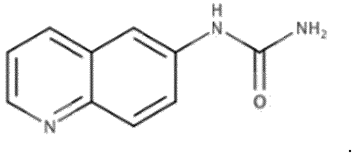

In one embodiment, the present invention relates to a compound wherein the compound is selected from the compounds delineated in Table 1.

**Table 1:**

| **No.** | **Code** | **Structure** | **IUPAC-Name** |
|---|---|---|---|
| **1** | EN22 | 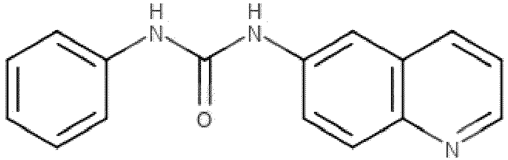 | 1-phenyl-3-(quinolin-6-yl)urea |
| **2** | EN21 | 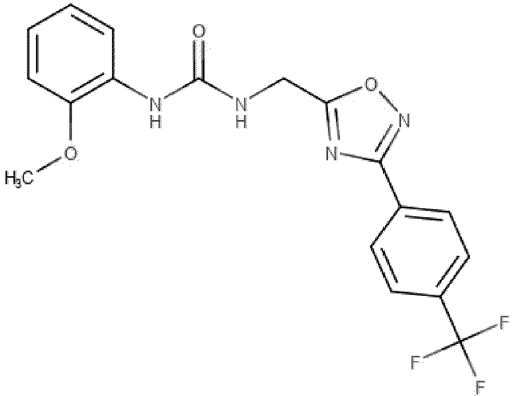 | 1-(2-methoxyphenyl )-3-((3-(4-(trifluoromethyl )phenyl)-1,2,4-oxadiazol-5-yl)methyl)urea |
| **3** | EN-A (EN23) | 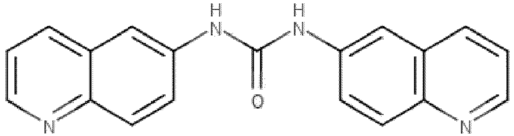 | 1,3-di(quinolin-6-yl)urea |
| **4** | AH4 | 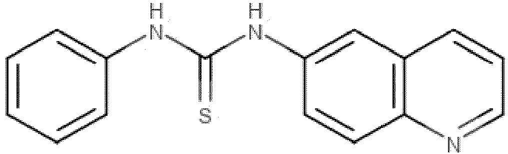 | 3-phenyl-1-(quinolin-6-yl)thiourea |
| **5** | JB010 | 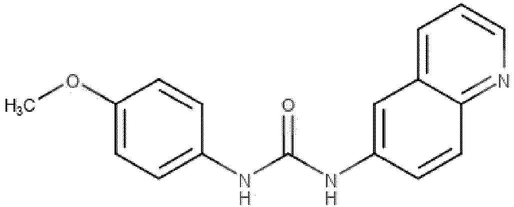 | 1-(4-methoxyphenyl )-3-(quinolin-6-yl)urea |
| **6** | BRM/BR G1 ATP Inhibitor-1 | 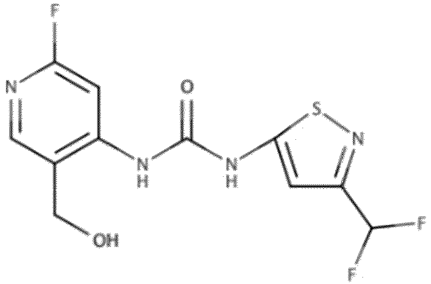 | 1-[3-(difluoromethyl )-1,2-thiazol-5-yl]-3-[2-fluoro-5-(hydroxymethy I)pyridin-4-yl]urea |
| **7** | JB011 | 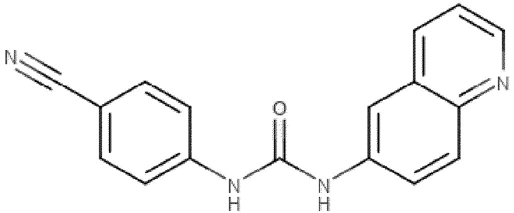 | 1-(4-cyanophenyl)-3-(quinolin-6-yl)urea |
| **8** | PM07 | 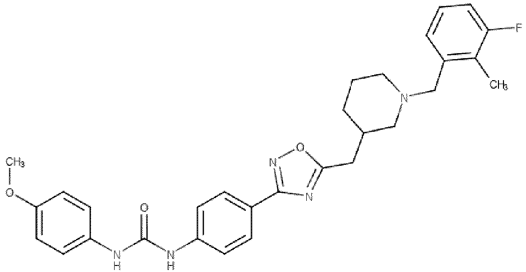 | 3-{4-[5-({1-[(3-fluoro-2-methylphenyl) methyl]piperidi n-3-yl}methyl)-1,2,4-oxadiazol-3-yl]phenyl}-1-(4-methoxyphenyl )urea |
| **9** | PM16 | 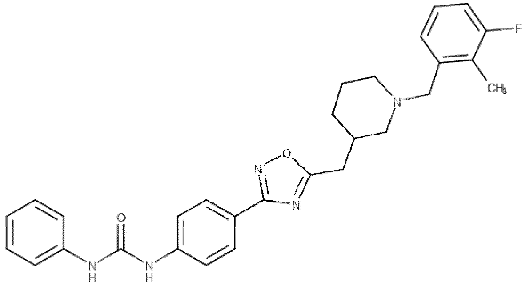 | 3-{4-[5-({1-[(3-fluoro-2-methylphenyl) methyl]piperidi n-3-yl}methyl)-1,2,4-oxadiazol-3-yl]phenyl}-1-phenylurea |
| **10** | JB014 | 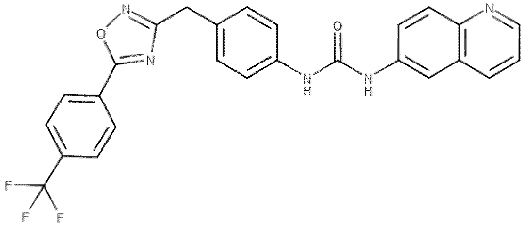 | 1-(quinolin-6-yl)-3-[4-({5-[4-(trifluoromethyl )phenyl]-1,2,4-oxadiazol-3-yl}methyl)phen yl]urea |
| **11** | AH1 | 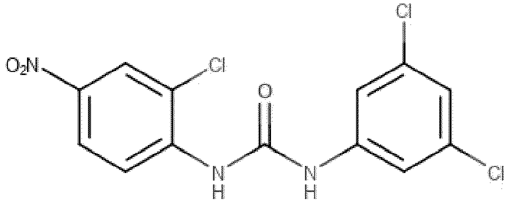 | 3-(2-chloro-4-nitrophenyl)-1-(3,5-dichlorophenyl )urea |
| **12** | AH3 | 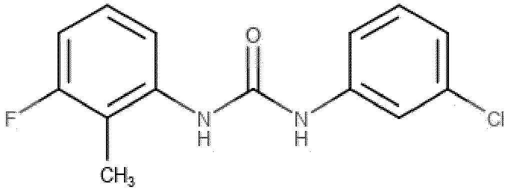 | 1-(3-chlorophenyl)-3-(3-fluoro-2-methylphenyl) urea |
| **13** | JB002 | 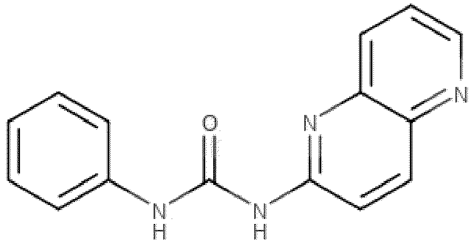 | 1-(1,5-naphthyridin-2-yl)-3-phenylurea |
| **14** | J B004 | 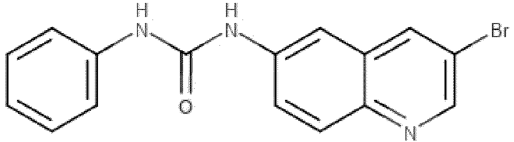 | 1-(3-bromoquinolin-6-yl)-3-phenylurea |
| **15** | EN11-16 | 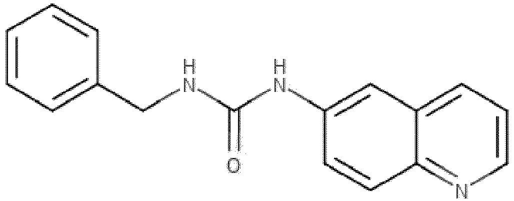 | 1-benzyl-3-(quinolin-6-yl)urea |
| **16** | EN11-13 | 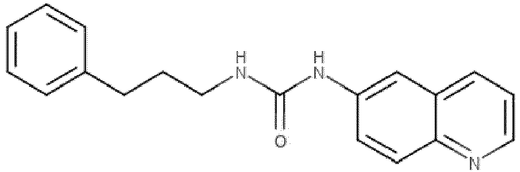 | 1-(3-phenylpropyl)-3-(quinolin-6-yl)urea |
| **17** | L01.110 | 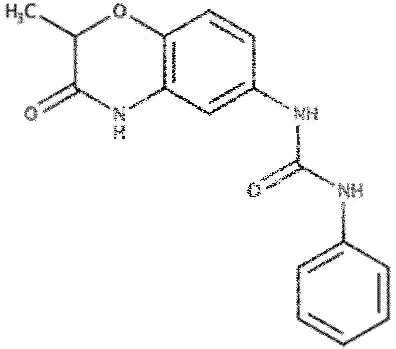 | 3-(2-methyl-3-oxo-3,4-dihydro-2H-1,4-benzoxazin-6-yl)-1-phenylurea |
| **18** | L01.041 | 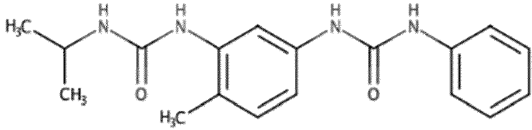 | 3-(4-methyl-3-{[(propan-2-yl)carbamoyl]a mino}phenyl)-1-phenylurea |
| **19** | L01.004 | 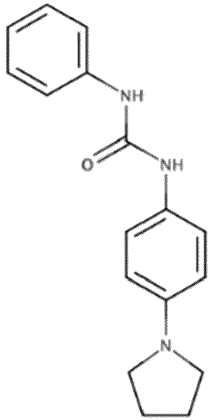 | 3-phenyl-1-[4-(pyrrolidin-1-yl)phenyl]urea |
| **20** | L01.007 | 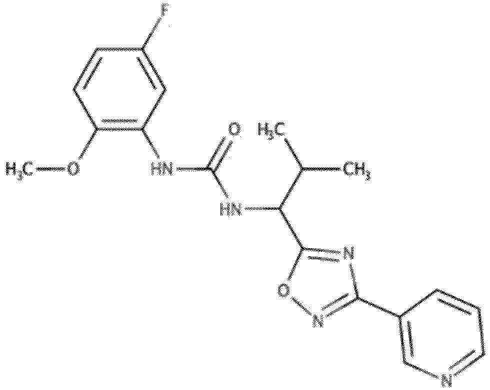 | 1-(5-fluoro-2-methoxyphenyl )-3-{2-methyl-1-[3-(pyridin-3-yl)-1,2,4-oxadiazol-5-yl]propyl}urea |
| **21** | L01.104 | 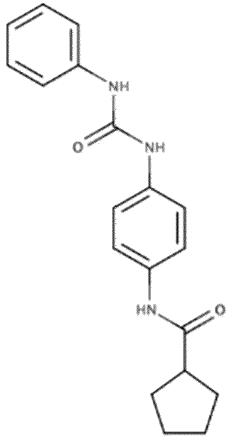 | N-{4-[(phenylcarba moyl)amino]ph enyl}cyclopent anecarboxami de |
| **22** | L01.116 | 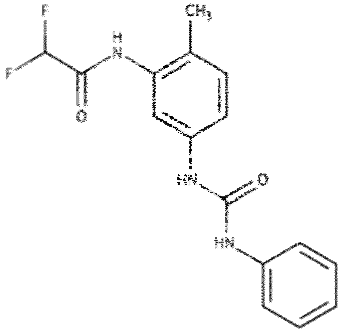 | 2,2-difluoro-N-{2-methyl-5-[(phenylcarba moyl)amino]ph enyl}acetamid e |
| **23** | L01.113 | 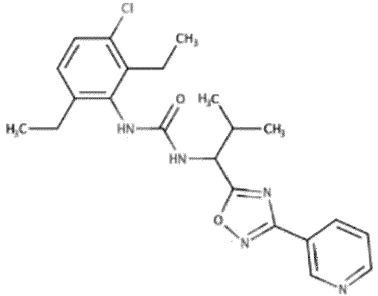 | 1-(3-chloro-2,6-diethylphenyl)-3-{2-methyl-1-[3-(pyridin-3-yl)-1,2,4-oxadiazol-5-yl]propyl}urea |
| **24** | L01.101 | 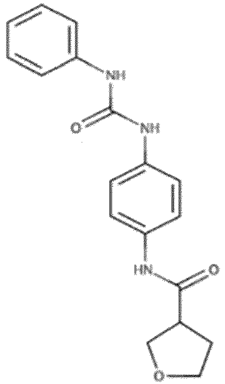 | N-{4-[(phenylcarba moyl)amino]ph enyl}oxolane-3-carboxamide |
| **25** | L01.092 | 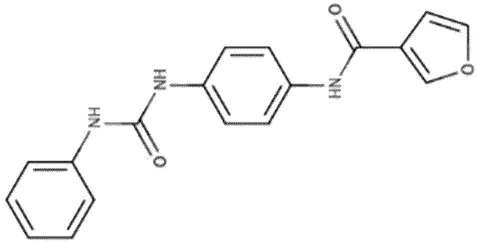 | N-{4-[(phenylcarba moyl)amino]ph enyl}furan-3-carboxamide |
| **26** | L01.124 | 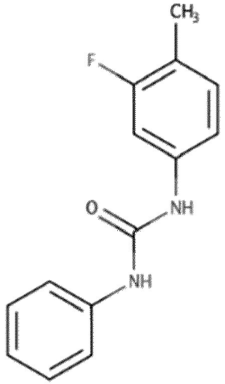 | 3-(3-fluoro-4-methylphenyl)-1-phenylurea |
| **27** | TCMDC-143175 | 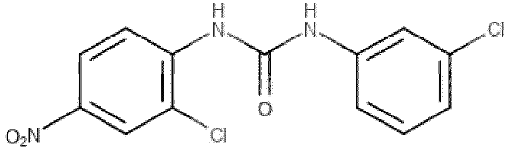 | 3-(2-chloro-4-nitrophenyl)-1-(3-chlorophenyl)u rea |
| **28** | A.C2 | 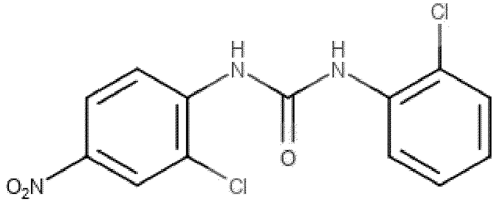 | 3-(2-chloro-4-nitrophenyl)-1-(2-chlorophenyl)u rea |
| **29** | AH2 | 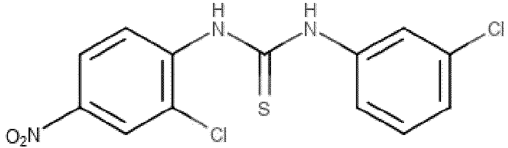 | 3-(2-chloro-4-nitrophenyl)-1-(3-chlorophenyl)t hiourea |
| **30** | EN11-07 | 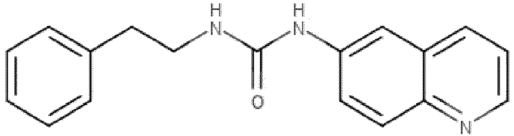 | 1-(2-phenylethyl)-3-(quinolin-6-yl)urea |
| **31** | EN11-02 | 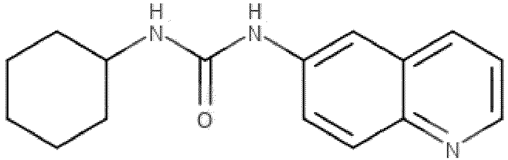 | 1-cyclohexyl-3-(quinolin-6-yl)urea |
| **32** | EN11-11 | 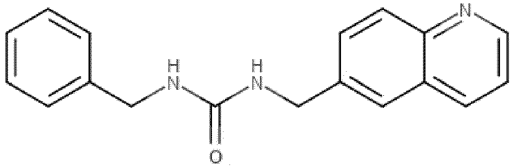 | 3-benzyl-1-[(quinolin-6-yl)methyl]urea |
| **33** | L01.016 | 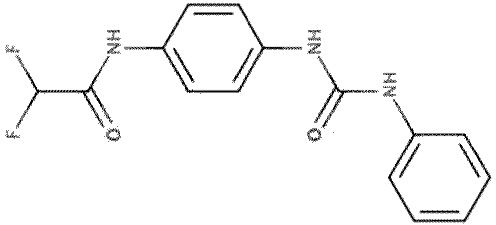 | 2,2-difluoro-N-{4-[(phenylcarba moyl)amino]ph enyl}acetamid e |
| **34** | L01.028 | 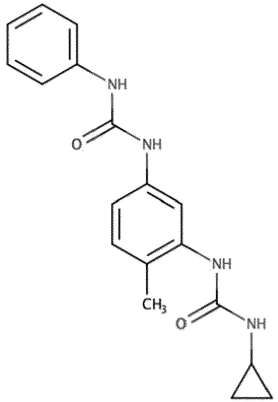 | 3-cyclopropyl-1-{2-methyl-5-[(phenylcarba moyl)amino]ph enyl}urea |
| **35** | L01.047 | 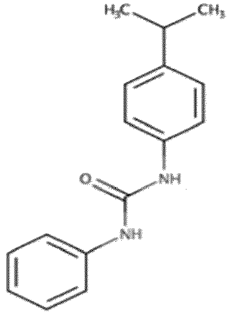 | 1-phenyl-3-[4-(propan-2-yl)phenyl]urea |
| **36** | L01.026 | 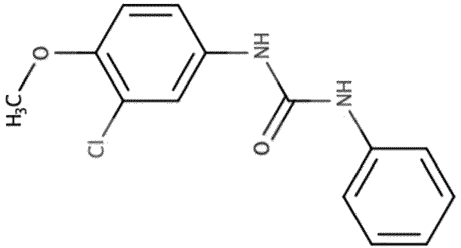 | 3-(3-chloro-4-methoxyphenyl )-1-phenylurea |
| **37** | L01.127 | 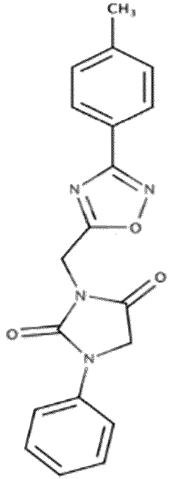 | 3-{[3-(4-methylphenyl)-1,2,4-oxadiazol-5-yl]methyl}-1-phenylimidazol idine-2,4-dione |
| **38** | L01.061 | 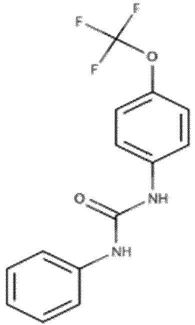 | 1-phenyl-3-[4-(trifluorometho xy)phenyl]urea |
| **39** | L01.128 | 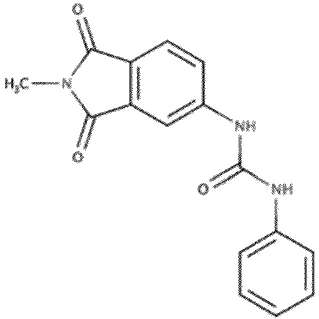 | 3-(2-methyl-1,3-dioxo-2,3-dihydro-1H-isoindol-5-yl)-1-phenylurea |
| **40** | L01.025 | 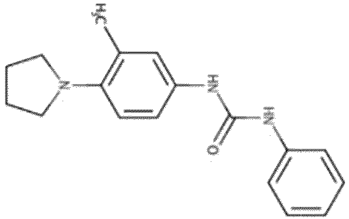 | 3-[3-methyl-4-(pyrrolidin-1-yl)phenyl]-1-phenylurea |
| **41** | L01.008 | 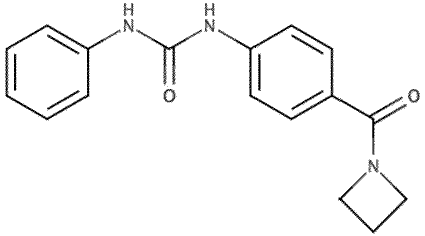 | 1-[4-(azetidine-1-carbonyl)phen yl]-3-phenylurea |
| **42** | L01.089 | 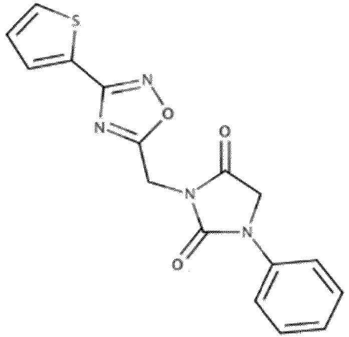 | 1-phenyl-3-{[3-(thiophen-2-yl)-1,2,4-oxadiazol-5-yl]methyl}imida zolidine-2,4-dione |
| **43** | L01.130 | 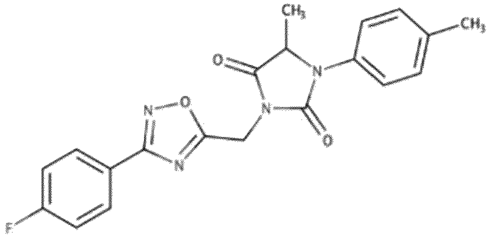 | 3-{[3-(4-fluorophenyl)-1,2,4-oxadiazol-5-yl]methyl}-5-methyl-1-(4-methylphenyl)i midazolidine-2,4-dione |
| **44** | L01.049 | 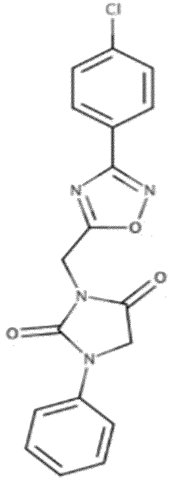 | 3-{[3-(4-chlorophenyl)-1,2,4-oxadiazol-5-yl]methyl}-1-phenylimidazol idine-2,4-dione |
| **45** | L01.066 | 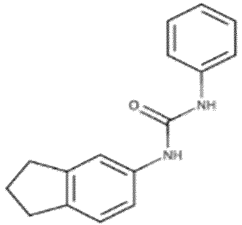 | 3-(2,3-dihydro-1H-inden-5-yl)-1-phenylurea |
| **46** | L01.043 | 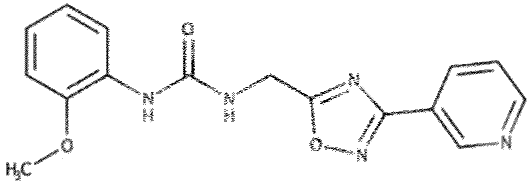 | 1-(2-methoxyphenyl )-3-{[3-(pyridin-3-yl)-1,2,4-oxadiazol-5-yl]methyl}urea |
| **47** | L01.087 | 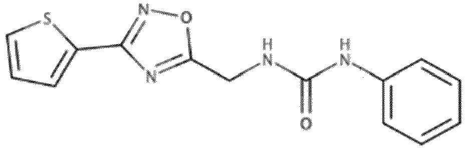 | 1-phenyl-3-{[3-(thiophen-2-yl)-1,2,4-oxadiazol-5-yl]methyl}urea |
| **48** | L01.020 | 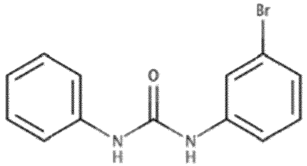 | 3-(3-bromophenyl)-1-phenylurea |
| **49** | L01.035 | 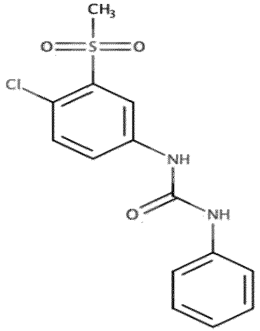 | 3-(4-chloro-3-methanesulfon ylphenyl)-1-phenylurea |
| **50** | L01.120 | 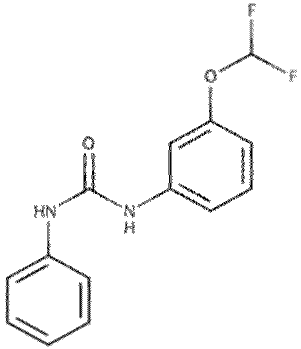 | 3-[3-(difluorometho xy)phenyl]-1-phenylurea |
| **51** | L01.033 | 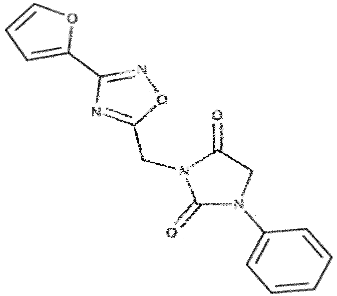 | 3-{[3-(furan-2-yl)-1,2,4-oxadiazol-5-yl]methyl}-1-phenylimidazol idine-2,4-dione |
| **52** | L01.002 | 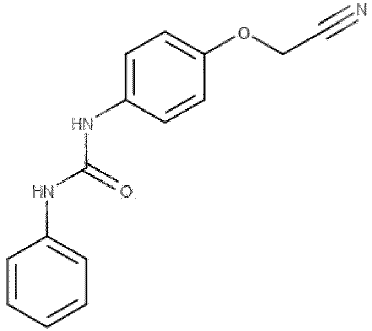 | 1-[4-(cyanomethox y)phenyl]-3-phenylurea |
| **53** | L01.027 | 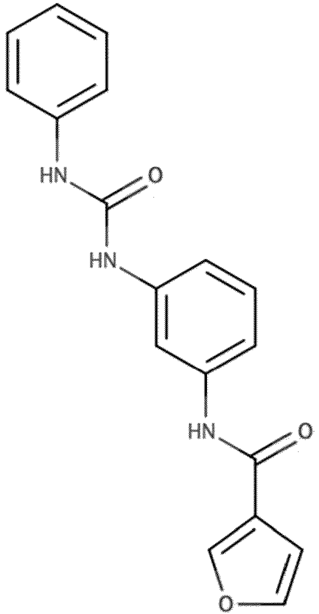 | N-{3-[(phenylcarba moyl)amino]ph enyl}furan-3-carboxamide |
| **54** | L01.003 | 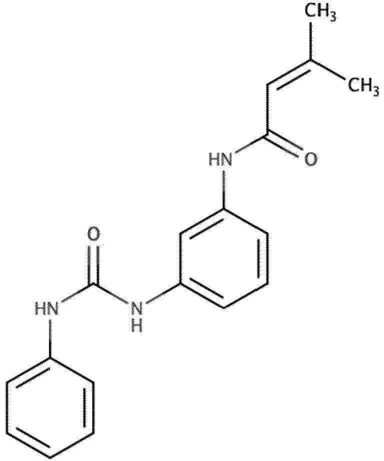 | 3-methyl-N-{3-[(phenylcarba moyl)amino]ph enyl}but-2-enamide |
| **55** | L01.038 | 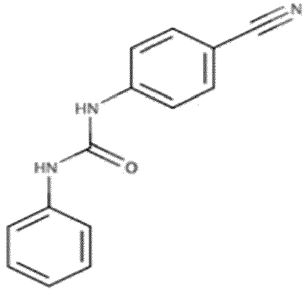 | 1-(4-cyanophenyl)-3-phenylurea |
| **56** | L01.069 | 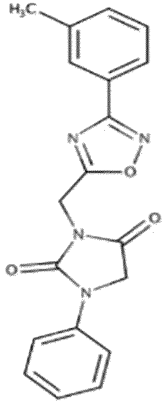 | 3-{[3-(3-methylphenyl)-1,2,4-oxadiazol-5-yl]methyl}-1-phenylimidazol idine-2,4-dione |
| **57** | L01.097 | 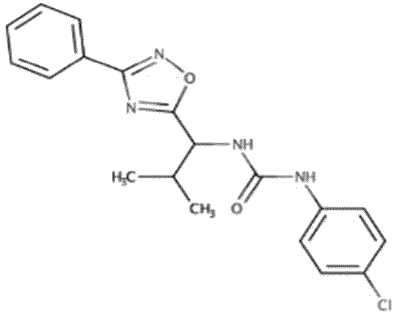 | 1-(4-chlorophenyl)-3-[2-methyl-1-(3-phenyl-1,2,4-oxadiazol-5-yl)propyl]urea |
| **58** | L01.095 | 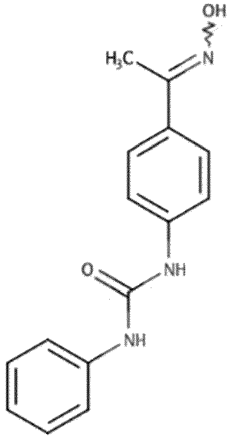 | 3-{4-[1-(hydroxyimino) ethyl]phenyl}-1-phenylurea |
| **59** | L01.013 | 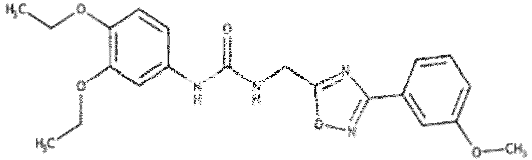 | 1-(3,4-diethoxyphenyl )-3-{[3-(3-methoxyphenyl )-1,2,4-oxadiazol-5-yl]methyl}urea |
| **60** | L01.103 | 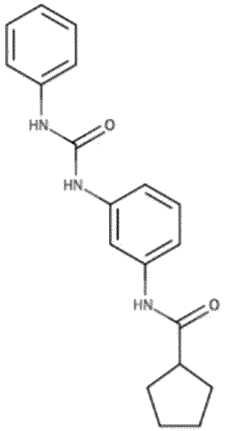 | N-{3-[(phenylcarba moyl)amino]ph enyl}cyclopent anecarboxami de |
| **61** | L01.071 | 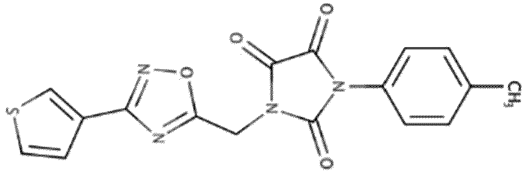 | 1-(4-methylphenyl)-3-{[3-(thiophen-3-yl)-1,2,4-oxadiazol-5-yl]methyl}imida zolidine-2,4,5-trione |
| **62** | L01.115 | 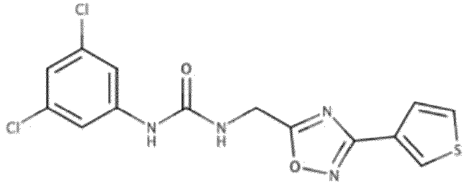 | 1-(3,5-dichlorophenyl )-3-{[3-(thiophen-3-yl)-1,2,4-oxadiazol-5-yl]methyl}urea |
| **63** | L01.045 | 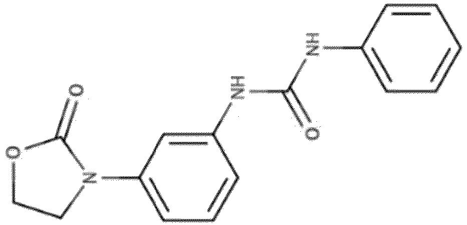 | 1-[3-(2-oxo-1,3-oxazolidin-3-yl)phenyl]-3-phenylurea |
| **64** | L01.077 | 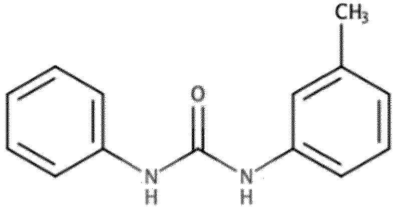 | 3-(3-methylphenyl)-1-phenylurea |
| **65** | L01.084 | 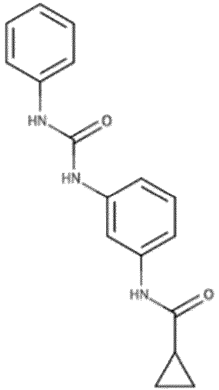 | N-{3-[(phenylcarba moyl)amino]ph enyl}cycloprop anecarboxami de |
| **66** | L01.109 | 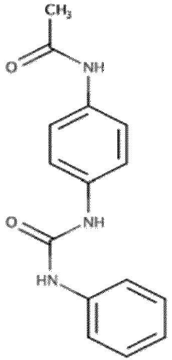 | N-{4-[(phenylcarba moyl)amino]ph enyl}acetamid e |
| **67** | L01.122 | 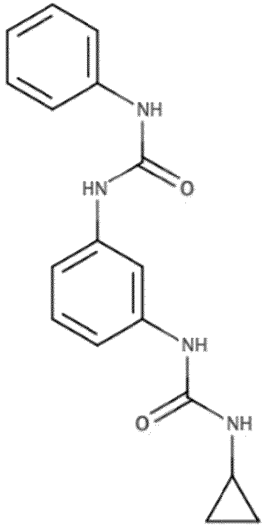 | 3-cyclopropyl-1-{3-[(phenylcarba moyl)amino]ph enyl}urea |
| **68** | L01.083 | 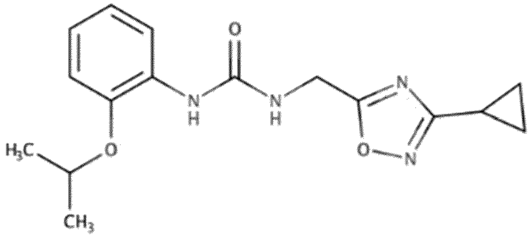 | 3-[(3-cyclopropyl-1,2,4-oxadiazol-5-yl)methyl]-1-[2-(propan-2-yloxy)phenyl]ur ea |
| **69** | L01.062 | 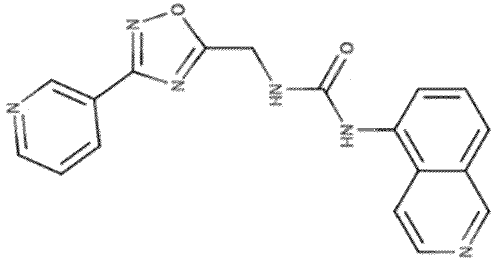 | 1-(isoquinolin-5-yl)-3-{[3-(pyridin-3-yl)-1,2,4-oxadiazol-5-yl]methyl}urea |
| **70** | L01.081 | 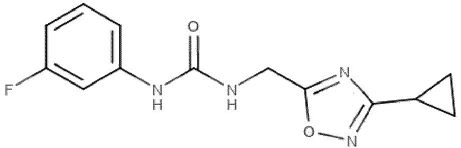 | 3-[(3-cyclopropyl-1,2,4-oxadiazol-5-yl)methyl]-1-(3-fluorophenyl)ur ea |
| **71** | L01.114 | 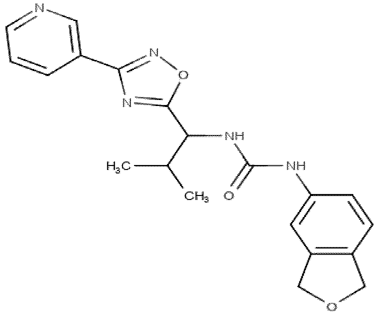 | 3-(1,3-dihydro-2-benzofuran-5-yl)-1-{2-methyl-1-[3-(pyridin-3-yl)-1,2,4-oxadiazol-5-yl]propyl}urea |
| **72** | L01.090 | 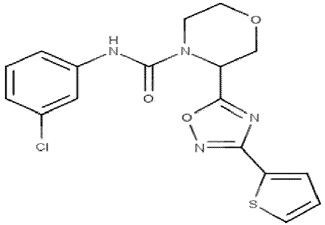 | N-(3-chlorophenyl)-3-[3-(thiophen-2-yl)-1,2,4-oxadiazol-5-yl]morpholine-4-carboxamide |
| **73** | L01.136 | 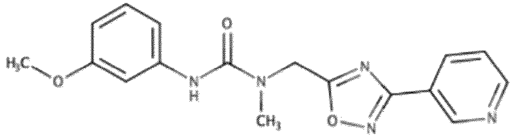 | 1-(3-methoxyphenyl )-3-methyl-3-{[3-(pyridin-3-yl)-1,2,4-oxadiazol-5-yl]methyl}urea |
| **74** | L01.023 | 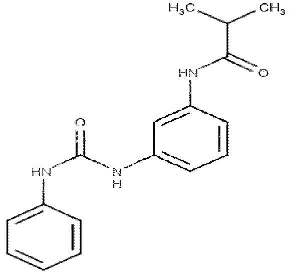 | 2-methyl-N-{3-[(phenylcarba moyl)amino]ph enyl}propanam ide |
| **75** | L01.064 | 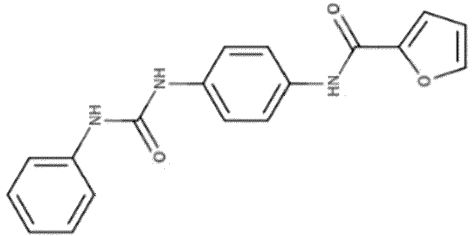 | N-{4-[(phenylcarba moyl)amino]ph enyl}furan-2-carboxamide |
| **76** | L01.093 | 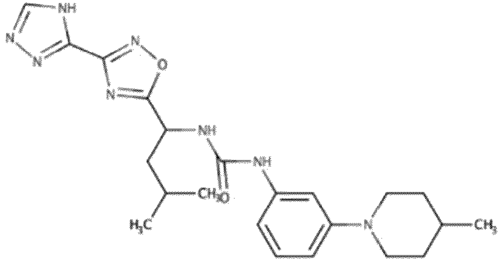 | 3-{3-methyl-1-[3-(4H-1,2,4-triazol-3-yl)-1,2,4-oxadiazol-5-yl]butyl}-1-[3-(4-methylpiperidin -1-yl)phenyl]urea |
| **77** | L01.125 | 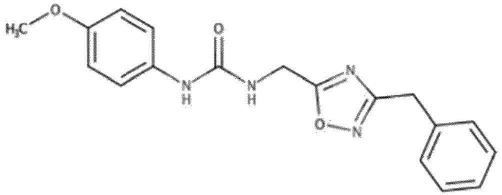 | 3-[(3-benzyl-1,2,4-oxadiazol-5-yl)methyl]-1-(4-methoxyphenyl )urea |
| **78** | L01.005 | 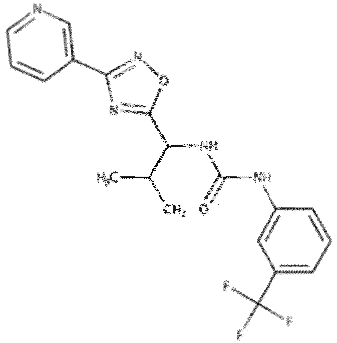 | 3-{2-methyl-1-[3-(pyridin-3-yl)-1,2,4-oxadiazol-5-yl]propyl}-1-[3-(trifluoromethyl )phenyl]urea |
| **79** | L01.135 | 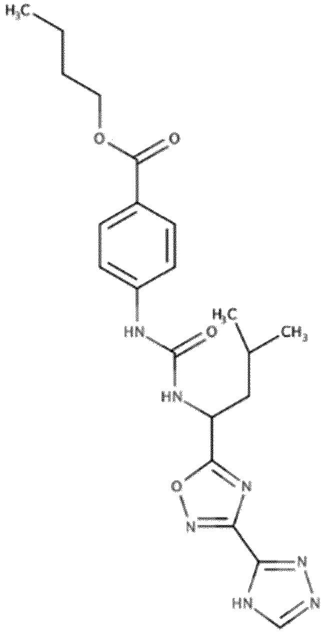 | butyl 4-[({3-methyl-1-[3-(4H-1,2,4-triazol-3-yl)-1,2,4-oxadiazol-5-yl]butyl}carba moyl)amino]be nzoate |
| **80** | L01.108 | 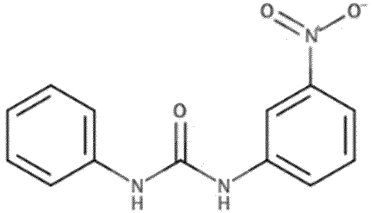 | 3-(3-nitrophenyl)-1-phenylurea |
| **81** | L01.112 | 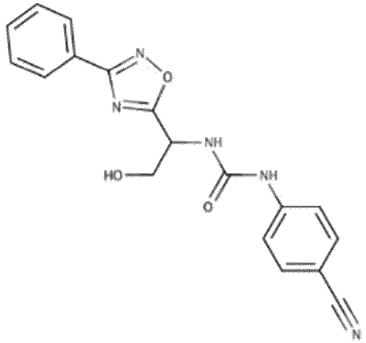 | 1-(4-cyanophenyl)-3-[2-hydroxy-1-(3-phenyl-1,2,4-oxadiazol-5-yl)ethyl]urea |
| **82** | L01.054 | 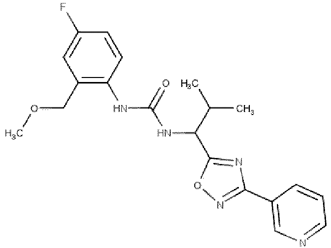 | 1-[4-fluoro-2-(methoxymeth yl)phenyl]-3-{2-methyl-1-[3-(pyridin-3-yl)-1,2,4-oxadiazol-5-yl]propyl}urea |
| **83** | L01.067 | 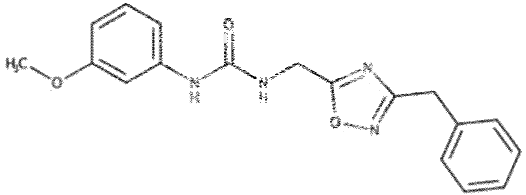 | 3-[(3-benzyl-1,2,4-oxadiazol-5-yl)methyl]-1-(3-methoxyphenyl )urea |
| **84** | L01.099 | 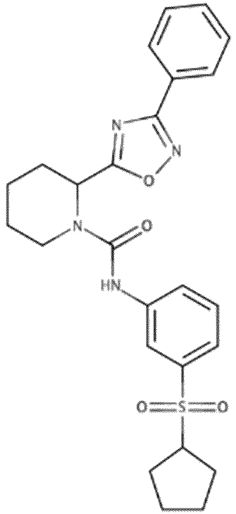 | N-[3-(cyclopentanes ulfonyl)phenyl] -2-(3-phenyl-1,2,4-oxadiazol-5-yl)piperidine-1-carboxamide |
| **85** | L01.065 | 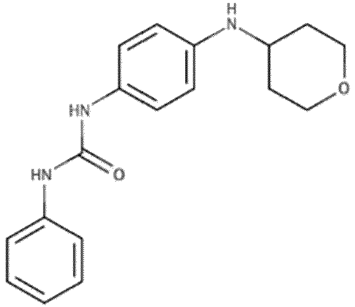 | 1-{4-[(oxan-4-yl)amino]phen yl}-3-phenylurea |
| **86** | L01.132 | 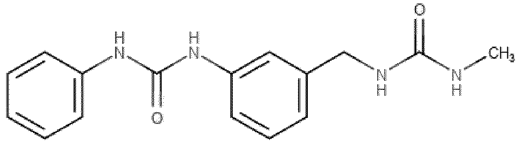 | 3-(3-{[(methylcarba moyl)amino]m ethyl}phenyl)-1-phenylurea |
| **87** | L01.121 | 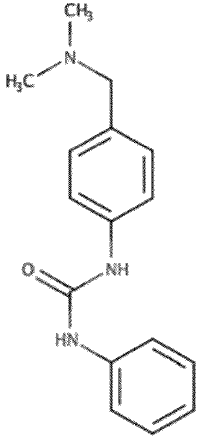 | 3-{4-[(dimethylamin o)methyl]phen yl}-1-phenylurea |
| **88** | L01.056 | 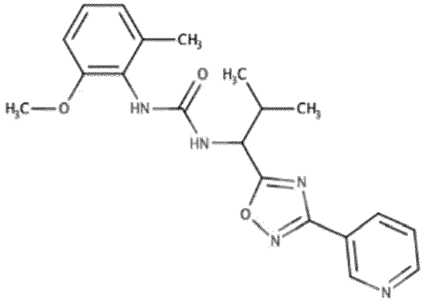 | 1-(2-methoxy-6-methylphenyl)-3-{2-methyl-1-[3-(pyridin-3-yl)-1,2,4-oxadiazol-5-yl]propyl}urea |
| **89** | L01.088 | 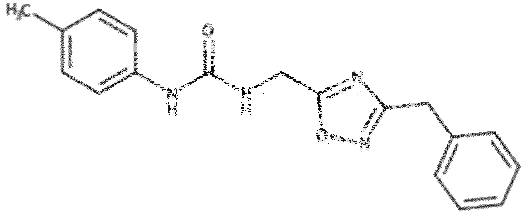 | 3-[(3-benzyl-1,2,4-oxadiazol-5-yl)methyl]-1-(4-methylphenyl) urea |
| **90** | L01.042 | 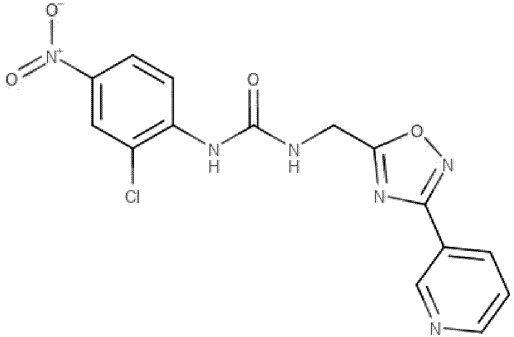 | 1-(2-chloro-4-nitrophenyl)-3-{[3-(pyridin-3-yl)-1,2,4-oxadiazol-5-yl]methyl}urea |
| **91** | L01.010 | 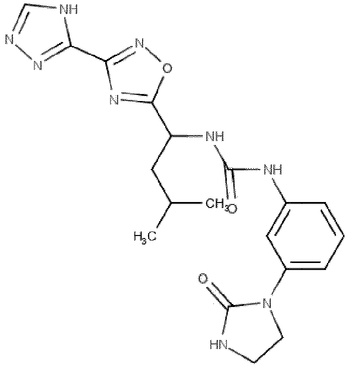 | 3-{3-methyl-1-[3-(4H-1,2,4-triazol-3-yl)-1,2,4-oxadiazol-5-yl]butyl}-1-[3-(2-oxoimidazolidi n-1-yl)phenyl]urea |

Particularly preferably, the present invention relates to a compound of Formula (I) selected from any of compounds as defined above numbered as No. 1-91, 1-75 or 1-26, more preferably from any of compounds numbered as No. 1-75 or 1-26, and most preferably from any of compounds numbered as No. 1-6 or 1-26, e.g. 1-6.

According to one further aspect, the present invention relates to a compound of Formula (II),

R⁷¹-D-CO-R⁷²

or a pharmaceutically acceceptable salt thereof, wherein:
D is a heteroaromatic bicycle selected from the group comprising 2-benzofuran, 2-benzothiophene, isoindol, indol, indolizine and purine and
R⁷¹ is a C₆-C₁₈ aryl or C₆-C₁₈ aryl C₁-C₁₀ alkyl; 5-20 membered heteroaryl or 5-20 membered heteroaryl C₁-C₁₀ alkyl; C₃-C₂₀ cycloalkyl or C₃-C₂₀ cycloalkyl C₁-C₁₀ alkyl; 3-20 membered cycloheteroalkyl or 3-20 membered cycloheteroalkyl C₁-C₁₀ alkyl, and
R⁷² is C₁-C₂₀ alkyl, C₂-C₂₀ alkenyl, 2-20 membered heteroalkyl, C₆-C₁₈ aryl or C₆-C₁₈ aryl C₁-C₁₀ alkyl; 5-20 membered heteroaryl or 5-20 membered heteroaryl C₁-C₁₀ alkyl; C₃-C₂₀ cycloalkyl or C₃-C₂₀ cycloalkyl C₁-C₁₀ alkyl; 3-20 membered cycloheteroalkyl or 3-20 membered cycloheteroalkyl C₁-C₁₀ alkyl, -OR⁷³, -NR⁷³, and wherein
R⁷³ is hydrogen, C₁-C₂₀ alkyl, C₂-C₂₀ alkenyl, 2-20 membered heteroalkyl, C₆-C₁₈ aryl or C₆-C₁₈ aryl C₁-C₁₀ alkyl; 5-20 membered heteroaryl or 5-20 membered heteroaryl C₁-C₁₀ alkyl; C₃-C₂₀ cycloalkyl or C₃-C₂₀ cycloalkyl C₁-C₁₀ alkyl; 3-20 membered cycloheteroalkyl or 3-20 membered cycloheteroalkyl C₁-C₁₀ alkyl.

In one embodiment of Formula II, D is isoindol, indol, or preferably indolizine. In one embodiment, the present invention relates to a compound of Formula (II), wherein the compound is a compound of Formula (IIa):

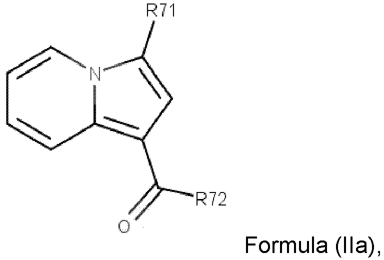

In one embodiment, the present invention relates to a compound of Formula (II) or (IIa), wherein R⁷² is -OR⁷³, -NR⁷³, wherein R⁷² is preferably -NR⁷³ and wherein
R⁷³ is hydrogen, C₁-C₂₀ alkyl, C₂-C₂₀ alkenyl, 2-20 membered heteroalkyl, C₆-C₁₈ aryl or C₆-C₁₈ aryl C₁-C₁₀ alkyl; 5-20 membered heteroaryl or 5-20 membered heteroaryl C₁-C₁₀ alkyl; C₃-C₂₀ cycloalkyl or C₃-C₂₀ cycloalkyl C₁-C₁₀ alkyl; 3-20 membered cycloheteroalkyl or 3-20 membered cycloheteroalkyl C₁-C₁₀ alkyl.

In one embodiment, the present invention relates to a compound of Formula (Ila), wherein Formula (IIa) is represented by Formula (Ilb) 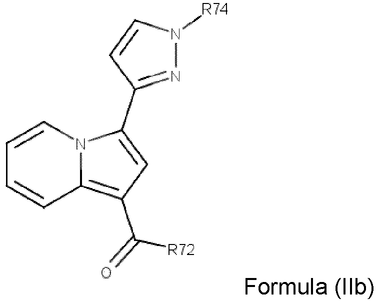
wherein R⁷² is C₁-C₂₀ alkyl, C₂-C₂₀ alkenyl, 2-20 membered heteroalkyl, C₆-C₁₈ aryl or C₆-C₁₈ aryl C₁-C₁₀ alkyl; 5-20 membered heteroaryl or 5-20 membered heteroaryl C₁-C₁₀ alkyl; C₃-C₂₀ cycloalkyl or C₃-C₂₀ cycloalkyl C₁-C₁₀ alkyl; 3-20 membered cycloheteroalkyl or 3-20 membered cycloheteroalkyl C₁-C₁₀ alkyl, -OR⁷³, -NR⁷³, and
R⁷³ is hydrogen, C₁-C₂₀ alkyl, C₂-C₂₀ alkenyl, 2-20 membered heteroalkyl, C₆-C₁₈ aryl or C₆-C₁₈ aryl C₁-C₁₀ alkyl; 5-20 membered heteroaryl or 5-20 membered heteroaryl C₁-C₁₀ alkyl; C₃-C₂₀ cycloalkyl or C₃-C₂₀ cycloalkyl C₁-C₁₀ alkyl; 3-20 membered cycloheteroalkyl or 3-20 membered cycloheteroalkyl C₁-C₁₀ alkyl and
wherein R⁷⁴ is hydrogen, C₁-C₂₀ alkyl, C₂-C₂₀ alkenyl, 2-20 membered heteroalkyl, C₆-C₁₈ aryl or C₆-C₁₈ aryl C₁-C₁₀ alkyl; 5-20 membered heteroaryl or 5-20 membered heteroaryl C₁-C₁₀ alkyl; C₃-C₂₀ cycloalkyl or C₃-C₂₀ cycloalkyl C₁-C₁₀ alkyl; 3-20 membered cycloheteroalkyl or 3-20 membered cycloheteroalkyl C₁-C₁₀ alkyl, and wherein R⁷⁴ is preferably C₃-C₂₀ cycloalkyl or C₃-C₂₀ cycloalkyl C₁-C₁₀ alkyl.

In one embodiment, the present invention relates to a compound of Formula (Ilb), wherein R⁷² is -OR⁷³, -NR⁷³, wherein R⁷² is preferably -NR⁷³ and wherein
R⁷³ is, C₂-C₂₀ alkyl, C₂-C₂₀ alkenyl, 2-20 membered heteroalkyl, C₆-C₁₈ aryl or C₆-C₁₈ aryl C₁-C₁₀ alkyl; 5-20 membered heteroaryl or 5-20 membered heteroaryl C₁-C₁₀ alkyl; C₃-C₂₀ cycloalkyl or C₃-C₂₀ cycloalkyl C₁-C₁₀ alkyl; 3-20 membered cycloheteroalkyl or 3-20 membered cycloheteroalkyl C₁-C₁₀ alkyl, and
wherein R⁷⁴ is hydrogen, C₁-C₂₀ alkyl, C₂-C₂₀ alkenyl, C₆-C₁₈ aryl or C₆-C₁₈ aryl C₁-C₁₀ alkyl; 5-20 membered heteroaryl or 5-20 membered heteroaryl C₁-C₁₀ alkyl; C₃-C₂₀ cycloalkyl or C₃-C₂₀ cycloalkyl C₁-C₁₀ alkyl; 3-20 membered cycloheteroalkyl or 3-20 membered cycloheteroalkyl C₁-C₁₀ alkyl, and wherein R⁷⁴ is preferably C₃-C₂₀ cycloalkyl or C₃-C₂₀ cycloalkyl C₁-C₁₀ alkyl.

Also described in the current disclosure is a compound of Formula (IIb), wherein the compound is

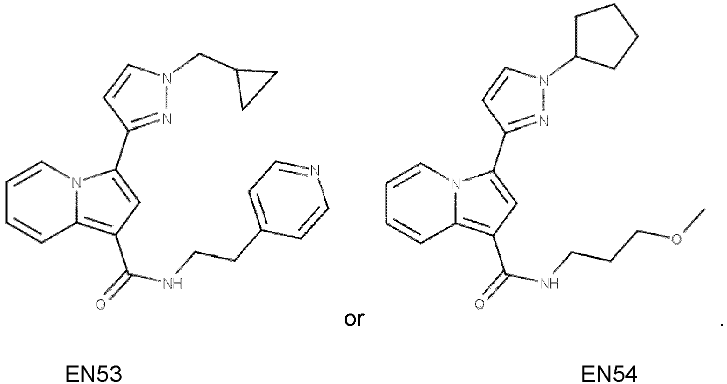

In one embodiment, the present invention relates to the herein described use of the present compounds, wherein the present compound induces the NLRP3 inflammasome activation, thereby increasing the release of IL-1β of 0.1×10⁶ BMDCs upon administering the compound in a concentration of 50 µM of the compound and incubation of 2 hours. The increase of the release of the amount of IL-1β corresponds preferably to at least a twofold increase, at least a threefold increase and an even more preferably at least a tenfold increase of the amounts of IL-1β in comparison to BMDCs that are not administered the active compound. In one embodiment, the present invention relates to the present compounds, wherein the present compound induces the NLRP3 inflammasome activation, thereby preferably increasing the release of IL-1β of 0.1×10⁶ BMDCs upon administering the compound in a concentration of 50 µM of the compound and incubation of 2 hours, wherein the increase is at least 0.1 ng ml⁻¹, 0.5 ng ml⁻¹, preferably 2 ng ml⁻¹, 10 ng ml⁻¹, 25 ng ml⁻¹, 50 ng ml⁻¹ and more preferably 4 ng ml⁻¹ or more. The increase of the release of IL-1β of 0.1×10⁶ BMDCs can be measured with the method described in Example 1.8.

### Pharmaceutical Compositions and Administration

### General

In some embodiments, a compound of the present invention (e.g., a compound that modulates e.g. agonizes) NLRP3, or a pharmaceutically acceptable salt, and/or hydrate, and/or cocrystal, and/or drug combination thereof is administered as a pharmaceutical composition that includes the compound of the invention and one or more pharmaceutically acceptable excipients, and optionally one or more additional therapeutic agents as described herein.

In some embodiments, the present compounds can be administered in combination with one or more conventional pharmaceutical excipients. Pharmaceutically acceptable excipients include, but are not limited to, ion exchangers, alumina, aluminum stearate, lecithin, self-emulsifying drug delivery systems (SEDDS) such as d-a-tocopherol polyethylene glycol 1000 succinate, surfactants used in pharmaceutical dosage forms such as Tweens, poloxamers or other similar polymeric delivery matrices, serum proteins, such as human serum albumin, buffer substances such as phosphates, tris, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such as hydroxyalkyl cyclodextrins, including 2- and 3- hydroxypropyl-P-cyclodextrins, or other solubilized derivatives can also be used to enhance delivery of compounds described herein. Dosage forms or compositions containing a compound of the invention as described herein in the range of 0.005% to 100% with the balance made up from non-toxic excipient may be prepared. The contemplated compositions may contain 0.001%-100% of a compound of the invention provided herein, in one embodiment 0.1-95%, in another embodiment 75-85%, in a further embodiment 20-80%. Actual methods of preparing such dosage forms are known, or will be apparent, to those skilled in this art; for example, see Remington: The Science and Practice of Pharmacy, 22nd Edition (Pharmaceutical Press, London, UK. 2012).

### Routes of Administration and Composition Components

In some embodiments, the present compounds described herein or a pharmaceutical composition thereof can be administered to subject in need thereof by any accepted route of administration. Acceptable routes of administration include, but are not limited to, buccal, cutaneous, endocervical, endosinusial, endotracheal, enteral, epidural, interstitial, intra-abdominal, intra-arterial, intrabronchial, intrabursal, intracerebral, intraci sternal, intracoronary, intradermal, intraductal, intraduodenal, intradural, intraepidermal, intraesophageal, intragastric, intragingival, intraileal, intralymphatic, intramedullary, intrameningeal, intramuscular, intraovarian, intraperitoneal, intraprostatic, intrapulmonary, intrasinal, intraspinal, intrasynovial, intratesticular, intrathecal, intratubular, intratumoral, intrauterine, intravascular, intravenous, nasal, nasogastric, oral, parenteral, percutaneous, peridural, rectal, respiratory (inhalation), subcutaneous, sublingual, submucosal, topical, transdermal, transmucosal, transtracheal, ureteral, urethral and vaginal. In certain embodiments, a preferred route of administration is parenteral (e.g., intratumoral). In certain embodiments, a preferred route of administration is systemic.

Compositions can be formulated for parenteral administration, e.g., formulated for injection via the intravenous, intramuscular, sub-cutaneous, or even intraperitoneal routes. Typically, such compositions can be prepared as injectables, either as liquid solutions, emulsions or suspensions; solid forms suitable for use to prepare solutions, emulsions or suspensions upon the addition of a liquid prior to injection can also be prepared; and the preparations can also be emulsified. The preparation of such formulations will be known to those of skill in the art in light of the present disclosure.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The carrier also can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof. Intratumoral injections are discussed, e.g., in Lammers, et al., "Effect of Intratumoral Injection on the Biodistribution and the Therapeutic Potential of HPMA Copolymer-Based Drug Delivery Systems" Neoplasia. 2006, 10, 788-795.

Pharmacologically acceptable excipients usable in the rectal composition as a gel, cream, enema, or rectal suppository, include, without limitation, any one or more of cocoa butter glycerides, synthetic polymers such as polyvinylpyrrolidone, PEG (like PEG ointments), glycerine, glycerinated gelatin, hydrogenated vegetable oils, poloxamers, mixtures of polyethylene glycols of various molecular weights and fatty acid esters of polyethylene glycol Vaseline, anhydrous lanolin, shark liver oil, sodium saccharinate, menthol, sweet almond oil, sorbitol, sodium benzoate, anoxid SBN, vanilla essential oil, aerosol, parabens in phenoxyethanol, sodium methyl p-oxybenzoate, sodium propyl p- oxybenzoate, diethylamine, carbomers, carbopol, methyloxybenzoate, macrogol cetostearyl ether, cocoyl caprylocaprate, isopropyl alcohol, propylene glycol, liquid paraffin, xanthan gum, carboxy-metabisulfite, sodium edetate, sodium benzoate, potassium metabi sulfite, grapefruit seed extract, methyl sulfonyl methane (MSM), lactic acid, glycine, vitamins, such as vitamin A and E and potassium acetate.

In certain embodiments, suppositories can be prepared by mixing the present compound described herein with suitable non-irritating excipients or carriers such as cocoa butter, polyethylene glycol or a suppository wax which are solid at ambient temperature but liquid at body temperature and therefore melt in the rectum and release the active compound. In other embodiments, compositions for rectal administration are in the form of an enema.

In other embodiments, the compounds described herein or a pharmaceutical composition thereof are suitable for local delivery to the digestive or GI tract by way of oral administration (e.g., solid or liquid dosage forms.).

Solid dosage forms for oral administration include capsules, tablets, pills, powders, and granules. In such solid dosage forms, the acompound of the invention is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

In one embodiment, the compositions will take the form of a unit dosage form such as a pill or tablet and thus the composition may contain, along with a compound of the invention provided herein, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In another solid dosage form, a powder, marume, solution or suspension (e.g., in propylene carbonate, vegetable oils, PEG's, poloxamer 124 or triglycerides) is encapsulated in a capsule (gelatin or cellulose base capsule). Unit dosage forms in which one or more of the present compounds provided herein or additional active agents are physically separated are also contemplated; e.g., capsules with granules (or tablets in a capsule) of each drug; two-layer tablets; two- compartment gel caps, etc. Enteric coated or delayed release oral dosage forms are also contemplated.

Other physiologically acceptable compounds include wetting agents, emulsifying agents, dispersing agents or preservatives that are particularly useful for preventing the growth or action of microorganisms. Various preservatives are well known and include, for example, phenol and ascorbic acid. In certain embodiments the excipients are sterile and generally free of undesirable matter. These compositions can be sterilized by conventional, well-known sterilization techniques. For various oral dosage form excipients such as tablets and capsules sterility is not required. The USP/NF standard is usually sufficient.

In certain embodiments, solid oral dosage forms can further include one or more components that chemically and/or structurally predispose the composition for delivery of the compound of the invention to the stomach or the lower GI; e.g., the ascending colon and/or transverse colon and/or distal colon and/or small bowel. Exemplary formulation techniques are described in, e.g., Filipski, K.J., et al., Current Topics in Medicinal Chemistry, 2013, 13, 776-802.

Examples include upper-Gl targeting techniques, e.g., Accordion Pill (Intec Pharma), floating capsules, and materials capable of adhering to mucosal walls.

Other examples include lower-Gl targeting techniques. For targeting various regions in the intestinal tract, several enteric/pH-responsive coatings and excipients are available. These materials are typically polymers that are designed to dissolve or erode at specific pH ranges, selected based upon the GI region of desired drug release. These materials also function to protect acid labile drugs from gastric fluid or limit exposure in cases where the active ingredient may be irritating to the upper Gl (e.g., hydroxypropyl methylcellulose phthalate series, Coateric (polyvinyl acetate phthalate), cellulose acetate phthalate, hydroxypropyl methylcellulose acetate succinate, Eudragit series (methacrylic acid-methyl methacrylate copolymers), and Marcoat). Other techniques include dosage forms that respond to local flora in the GI tract, Pressure-controlled colon delivery capsule, and Pulsincap. Ocular compositions can include, without limitation, one or more of any of the following: viscogens (e.g., Carboxymethylcellulose, Glycerin, Polyvinylpyrrolidone, Polyethylene glycol); Stabilizers (e.g., Pluronic (triblock copolymers), Cyclodextrins); Preservatives (e.g., Benzalkonium chloride, EDTA, SofZia (boric acid, propylene glycol, sorbitol, and zinc chloride; Alcon Laboratories, Inc.), Purite (stabilized oxychloro complex; Allergan, Inc.)). Topical compositions can include ointments and creams. Ointments are semisolid preparations that are typically based on petrolatum or other petroleum derivatives. Creams containing the selected active agent are typically viscous liquid or semisolid emulsions, often either oil-in-water or water-in-oil. Cream bases are typically water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. As with other carriers or vehicles, an ointment base should be inert, stable, nonirritating and non- sensitizing.

In any of the foregoing embodiments, pharmaceutical compositions described herein can include one or more one or more of the following: lipids, interbilayer crosslinked multilamellar vesicles, biodegradeable poly(D,L-lactic-co-glycolic acid) [PLGA]-based or poly anhydride-based nanoparticles or microparticles, and nanoporous particle-supported lipid bilayers.

### Dosages

The dosages may be varied depending on the requirement of the patient, the severity of the condition being treating and the particular compound being employed. Determination of the proper dosage for a particular situation can be determined by one skilled in the medical arts. The total daily dosage may be divided and administered in portions throughout the day or by means providing continuous delivery.

In some embodiments, the compounds described herein are administered at a dosage of from about 0.0001 mg kg⁻¹ to about 1000 mg kg⁻¹ (e.g., from about 0.001 mg kg⁻¹ to about 200 mg kg⁻¹; from about 0.01 mg kg⁻¹ to about 200 mg kg⁻¹; from about 0.01 mg kg⁻¹ to about 150 mg kg⁻¹; from about 0.01 mg kg⁻¹ to about 100 mg kg⁻¹; from about 0.01 mg kg⁻¹ to about 50 mg kg⁻¹; from about 0.01 mg kg⁻¹ to about 10 mg kg⁻¹; from about 0.01 mg kg⁻¹ to about 5 mg kg⁻¹; from about 0.01 mg kg⁻¹ to about 1 mg kg⁻¹; from about 0.01 mg kg⁻¹ to about 0.5 mg kg⁻¹; from about 0.01 mg kg⁻¹ to about 0.1 mg kg⁻¹; from about 0.1 mg kg⁻¹ to about 200 mg kg⁻¹; from about 0.1 mg kg⁻¹ to about 150 mg kg⁻¹; from about 0.1 mg kg⁻¹ to about 100 mg kg⁻¹; from about 0.1 mg kg⁻¹ to about 50 mg kg⁻¹; from about 0.1 mg kg⁻¹ to about 10 mg kg⁻¹; from about 0.1 mg kg⁻¹ to about 5 mg kg⁻¹; from about 0.1 mg kg⁻¹ to about 1 mg kg⁻¹; from about 0.1 mg kg⁻¹ to about 0.5 mg kg⁻¹).

### Regimens

The foregoing dosages can be administered on a daily basis (e.g., as a single dose or as two or more divided doses) or non-daily basis (e.g., every other day, every two days, every three days, once weekly, twice weeks, once every two weeks, once a month).

In some embodiments, the period of administration of a compound described herein is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 1 1 months, 12 months, or more. In a further embodiment, a period of during which administration is stopped is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 1 1 weeks, 12 weeks, 4 months, 5 months, 6 months, 7 months, 8 months, 9 months, 10 months, 11 months, 12 months, or more. In an embodiment, a therapeutic compound is administered to an individual for a period of time followed by a separate period of time. In another embodiment, a therapeutic compound is administered for a first period and a second period following the first period, with administration stopped during the second period, followed by a third period where administration of the therapeutic compound is started and then a fourth period following the third period where administration is stopped. In an aspect of this embodiment, the period of administration of a therapeutic compound followed by a period where administration is stopped is repeated for a determined or undetermined period of time. In a further embodiment, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

### Methods of Treatment

In some embodiments, methods for treating a subject having condition, disease or disorder in which a decrease or increase in NLRP3 activity (e.g., a decrease, e.g., repressed or impaired NLRP3 signaling) contributes to the pathology and/or symptoms and/or progression of the condition, disease or disorder (e.g., cancer) are provided.

### Indications

In any of the methods described herein, the subject can have a cancer. In some examples of any of the methods described herein, the mammal has been identified as having a cancer, or has been diagnosed as having a cancer.

Non-limiting examples of cancer include: acute myeloid leukemia, adrenocortical carcinoma, Kaposi sarcoma, lymphoma, anal cancer, appendix cancer, teratoid/rhabdoid tumor, basal cell carcinoma, bile duct cancer, bladder cancer, bone cancer, brain cancer, breast cancer, bronchial tumor, carcinoid tumor, cardiac tumor, cervical cancer, chordoma, chronic lymphocytic leukemia, chronic myeloproliferative neoplasm, colon cancer, colorectal cancer, craniopharyngioma, bile duct cancer, endometrial cancer, ependymoma, esophageal cancer, esthesioneuroblastoma, Ewing sarcoma, eye cancer, fallopian tube cancer, gallbladder cancer, gastrointestinal carcinoid tumor, gastrointestinal stromal tumor, germ cell tumor, hairy cell leukemia, head and neck cancer, heart cancer, liver cancer, hypopharngeal cancer, pancreatic cancer, kidney cancer, laryngeal cancer, chronic myelogenous leukemia, lip and oral cavity cancer, lung cancer, melanoma, Merkel cell carcinoma, mesothelioma, mouth cancer, oral cancer, osteosarcoma, ovarian cancer, penile cancer, pharyngeal cancer, prostate cancer, rectal cancer, salivary gland cancer, skin cancer, small intestine cancer, soft tissue sarcoma, testicular cancer, throat cancer, thyroid cancer, urethral cancer, uterine cancer, vaginal cancer, and vulvar cancer.

Methods for diagnosing a subject as having a cancer or identifying a mammal as having a cancer are well known in the art. For example, a medical professional (e.g., a physician, a physician's assistant, or a technician) can diagnose cancer in a mammal by observing one or more symptoms of cancer in a mammal. Non-limiting examples of symptoms of cancer include: fatigue, lump or area of thickening felt under the skin, weight change, jaundice, darkening or redness of the skin, sores that won't heal, changes to existing moles, changes in bowel or bladder habits, persistent cough or trouble breathing, difficulty swallowing, hoarseness, persistent indigestion or discomfort after eating, persistent, unexplained muscle or joint pain, persistent, unexplained fevers or night sweats, and unexplained bleeding or bruising. Methods of diagnosing a subject as having a cancer or identifying a subject as having a cancer can further include performing one or more diagnostic tests (e.g., performing one or more diagnostic tests on a biopsy or a blood sample).

In some examples of any of the methods described herein, a subject can be a subject having a cancer, a subject diagnosed as having a cancer, or a subject identified as having a cancer that has been unresponsive to a previously administered treatment for cancer. Diagnostic tests for diagnosing a subject as having a cancer or identifying a mammal as having a cancer are known in the art.

In any of the methods described herein, the subject can have an infectious disease. In some examples of any of the methods described herein, the subject has been identified as having an infectious disease, or has been diagnosed as having an infectious disease. For example, an infectious disease can be caused by a bacterium, virus, fungus, or a parasite.

Non-limiting examples of infectious disease include: Acinetoobacter infection, actinomycosis, African sleeping sickness, acquired immunodeficiency syndrome, amebiasis, anaplasmosis, anthrax, Arcanobacterium haemolyticum infection, Argentine hemorrhagic fever, ascariasis, aspergillosis, astrovirus infection, babesiosis, Bacillus cereus infection, bacterial pneumonia, bacterial vaginosis, Bacteroides infection, balantidiasis, Baylisascaris infection, BK virus infection, black piedra, Blastocystic hominis infection, blastomycosis, Bolivian hemorrhagic fever, botulism, Brazilian hemorrhagic fever, brucellosis, bubonic plaque, Burkholderi infection, Buruli ulcer, Calicivirus infection, camptobacteriosis, candidiasis, cat-scratch disease, cellulitis, Chagas disease, chancroid, chickenpox, chikungunya, chlamydia, Chlamydophila pneumoniae infection, cholera, chromoblastomycosis, clonorchiasis, Clostridium difficile infection, coccidioidomycosis, Colorado tick fever, common cold, Creutzfeldt-Jakob disease, Crimean-Congo hemorrhagic fever, crytococcosis, cryptosporidiosis, cutaneous larva migrans, cyclosporiasis, cysticercosis, cytomegalovirus infection, dengue fever, Desmodesmus infection, deintamoebiasis, diphtheria, diphyllobothriasis, dracunculiasis, ebola hemorrhagic fever, echinococcosis, ehrlichiosis, enterobiasis, Enterococcus infection, Enterovirus infection, epidemic typhus, erythema infection, exanthema subitum, fasciolopsiasis, fasciolosis, fatal familial insomnia, filariasis, food poisoning by Clostridium myonecrosis, free-living amebic infection, Fusobacterium infection, gas gangrene, geotrichosis, Gerstmann-Straussler-Scheinker syndrome, giardiasis, glanders, gnathostomiasis, gonorrhea, granuloma inguinale, Group A streptococcal infection, Group B streptococcal infection, Haemophilus influenzae infection, hand foot and mouth disease, hantavirus pulmonary syndrome, Heartland virus disease, Heliobacter pylori infection, hemolytic-uremic syndrome, hemorrhagic fever with renal syndrome, hepatitis A, hepatitis B, hepatitis C, hepatitis D, hepatitis E, herpes simplex, histoplasmosis, hookworm infection, human bocavirus infection, human ewingii ehrlichiosis, human granulocyte anaplasmosis, human metapneuomovirus infection, human monocytic ehrlichiosis, human papillomavirus infection, human parainfluenza virus infection, hymenolepiasis, Epstein- Barr virus infectious mononucleosis, influenza, isosporiasis, Kawasaki disease, keratitis, Kingella kingae infection, kuru, lassa fever, Legionnaires' disease, Pontiac fever, leishmaniasis, leprosy, leptospirosis, listeriosis, lyme disease, lymphatic filariasis, lymphocytic choriomeningitis, malaria, Marburg hemorrhagic fever, measles, Middle East respiratory syndrome, melioidosis, meningitis, meningococcal disease, metagonimiasis, microsporidiosis, molluscum contagiosum, monkeypox, mumps, murine typhus, mycoplasma pneumonia, mycetoma, myiasis, neonatal conjunctivitis, variant Creutzfeldt- Jakob disease, nocardiosis, onchocerciasis, paracoccidioidomycosis, paragonimiasis, pasteurellosis, pediculosis capitis, pediculosis corporis, pediculosis pubis, pelvic inflammatory disease, pertussis, plague, pneumonia, poliomyelitis, Prevotella infection, primary amoebic meningoencephalitis, progressive multifocal leukoencephalopathy, psittacosis, Q fever, rabies, relapsing fever, respiratory syncytial virus infection, rhinosporidiosis, rhinovirus infection, rickettsial infection, rickettsialpox, Rift Valley Fever, Rocky Mountain spotted fever, rotavirus infection, rubella, salmonellosis, severe acute respiratory syndrome, scabies, schistosomiasis, sepsis, shigellosis, shingles, smallpox, sporothrichosis, staphylococcal food poisoning, staphylococcal infection, staphylococcal infection, strongyloidiasis, subacute sclerosing panencephalitis, syphilis, taeniasis, tetanus, tinea barabe, tinea capitis, tinea corporis, tinea cruris, tinea manum, tinea nigra, tinea pedis, tinea unguium, tinea versicolor, toxocariasis, trachoma, toxoplasmosis, trichinosis, trichomoniasis, trichuriasis, tuberculosis, tularemia, typhoid fever, Ureaplasma urealyticum infection, valley fever, Venezuelan hemorrhagic fever, viral pneumonia, West Nile fever, white piedra, Yersinia psuedotuberculosis infection, yersiniosis, yellow fever, and zygomycosis.

Methods for diagnosing a subject as having an infectious disease, or identifying a subject as having an infectious disease are well known in the art. For example, a medical professional (e.g., a physician, a physician's assistant, or a technician) can diagnose infectious disease in a subject by observing one or more symptoms of infectious disease in a subject. Non-limiting examples of symptoms of infectious disease include: fever, diarrhea, fatigue, and muscle aches. Methods of diagnosing a mammal as having an infectious disease or identifying a subject as having an infectious disease can further include performing one or more diagnostic tests (e.g., performing one or more diagnostic tests on a biopsy or a blood sample). Diagnostic tests for diagnosing a subject as having an infectious disease or identifying a subject as having an infectious disease are known in the art. With regard to the combination therapy, this disclosure contemplates both monotherapy regimens as well as combination therapy regimens.

In some embodiments, the methods described herein can further include administering one or more additional therapies (e.g., one or more additional therapeutic agents and/or one or more therapeutic regimens) in combination with administration of the compounds described herein.

In certain embodiments, the methods described herein can further include administering one or more additional cancer therapies.

The one or more additional cancer therapies can include, without limitation, surgery, radiotherapy, chemotherapy, toxin therapy, immunotherapy, cryotherapy, cancer vaccines (e.g., HPV vaccine, hepatitis B vaccine, Oncophage, Provenge) and gene therapy, as well as combinations thereof. Immunotherapy, including, without limitation, adoptive cell therapy, the derivation of stem cells and/or dendritic cells, blood transfusions, lavages, and/or other treatments, including, without limitation, freezing a tumor.

In some embodiments, the one or more additional cancer therapies is chemotherapy, which can include administering one or more additional chemotherapeutic agents. In certain embodiments, the additional chemotherapeutic agent is an immunomodulatory moiety, e.g., an immune checkpoint inhibitor. In certain of these embodiments, the immune checkpoint inhibitor targets an immune checkpoint receptor selected from the group consisting of CTLA-4, PD-1, PD-L1, PD-1 - PD-L1, PD-1 - PD- L2, T cell immunoglobulin and mucin 3 (TEVI3 or HAVCR2), Galectin 9 - TEVI3, Phosphatidylserine - TIM3, lymphocyte activation gene 3 protein (LAG3), MHC class II, - LAG3, 4- 1BB-4- 1BB ligand, OX40-OX40 ligand, GITR, GITR ligand - GITR, CD27, CD70-CD27, T FRSF25, TNFRSF25-TL1 A, CD40L, CD40-CD40 ligand, HVEM- LIGHT-LTA, HVEM, HVEM - BTLA, HVEM - CD 160, HVEM - LIGHT, HVEM- BTLA-CD160, CD80, CD80 - PD-L1, PD-L2 - CD80, CD244, CD48 - CD244, CD244, ICOS, ICOS-ICOS ligand, B7-H3, B7-H4, VISTA, TMIGD2, HHLA2-TMIGD2, Butyrophilins, including BTNL2, Siglec family, TIGIT and PVR family members, K Rs, ILTs and LIRs, NKG2D and NKG2A, MICA and MICB, CD244, CD28, CD86 - CD28, CD86 - CTLA, CD80 - CD28, Phosphatidylserine, TIM3, Phosphatidylserine - TIM3, SIRPA-CD47, VEGF, Neuropilin, CD160, CD30, and CD155 (e.g., CTLA-4 or PD-1 or PD-L1) and other immunomodulatory agents, such as interleukin-2 (IL-2), indoleamine 2,3-dioxygenase (IDO), IL- 10, transforming growth factor-β (TGFp), CD39, CD73, Adenosine-CD39-CD73, and CXCR4-CXCL 12. See, e.g., Postow, M. J. Clin. Oncol. 2015, 33, 1. In certain of these embodiments, the immune checkpoint inhibitor is selected from the group consisting of: Urelumab, PF-05082566, MEDI6469, TRX518, Varlilumab, CP-870893, Pembrolizumab (PD-1), Nivolumab (PD-1), Atezolizumab (formerly MPDL3280A) (PD-L1), MEDI4736 (PD-L1), Avelumab (PD-L1), PDR001 (PD-1), BMS-986016, MGA271, Lirilumab, IPH2201, Emactuzumab, INCB024360, Galunisertib, Ulocuplumab, BKT140, Bavituximab, CC-90002, Bevacizumab, and MNRP1685A, and MGA271.

In certain embodiments, the additional chemotherapeutic agent is a STING agonist. For example, the STING agonist can include cyclic di-nucleotides, such as cAMP, cGMP, and cGAMP as well as modified cyclic di-nucleotides that include one or more of the following modification features (2'-0/3'-0 linkage, phosphorothioate linkage, adenine and/or guanine analogue, 2'-OH modification (e.g., -OCH3 or replacement, e.g., -F or N3). See, e.g., WO 2014/189805. In certain embodiments, the additional chemotherapeutic agent is an alkylating agent. Alkylating agents are so named because of their ability to alkylate many nucleophilic functional groups under conditions present in cells, including, but not limited to cancer cells. In a further embodiment, an alkylating agent includes, but is not limited to, Cisplatin, carboplatin, mechlorethamine, cyclophosphamide, chlorambucil, ifosfamide and/or oxaliplatin. In an embodiment, alkylating agents can function by impairing cell function by forming covalent bonds with the amino, carboxyl, sulfhydryl, and phosphate groups in biologically important molecules or they can work by modifying a cell's DNA. In a further embodiment an alkylating agent is a synthetic, semisynthetic or derivative.

In certain embodiments, the additional chemotherapeutic agent is an anti- metabolite. Antimetabolites masquerade as purines or pyrimidines, the building-blocks of DNA and in general, prevent these substances from becoming incorporated in to DNA during the "S" phase (of the cell cycle), stopping normal development and division. Antimetabolites can also affect RNA synthesis. In an embodiment, an antimetabolite includes, but is not limited to azathioprine and/or mercaptopurine. In a further embodiment an anti- metabolite is a synthetic, semisynthetic or derivative.

In certain embodiments, the additional chemotherapeutic agent is a plant alkaloid and/or terpenoid. These alkaloids are derived from plants and block cell division by, in general, preventing microtubule function. In an embodiment, a plant alkaloid and/or terpenoid is a vinca alkaloid, a podophyllotoxin and/or a taxane. Vinca alkaloids, in general, bind to specific sites on tubulin, inhibiting the assembly of tubulin into microtubules, generally during the M phase of the cell cycle. In an embodiment, a vinca alkaloid is derived, without limitation, from the Madagascar periwinkle, Catharanthus roseus (formerly known as Vinca rosea). In an embodiment, a vinca alkaloid includes, without limitation, Vincristine, Vinblastine, Vinorelbine and/or Vindesine. In an embodiment, a taxane includes, but is not limited, to Taxol, Paclitaxel and/or Docetaxel. In a further embodiment a plant alkaloid or terpernoid is a synthetic, semisynthetic or derivative. In a further embodiment, a podophyllotoxin is, without limitation, an etoposide and/or teniposide. In an embodiment, a taxane is, without limitation, docetaxel and/or ortataxel. In an embodiment, a cancer therapeutic is a topoisomerase. Topoisomerases are essential enzymes that maintain the topology of DNA. Inhibition of type I or type II topoisom erases interferes with both transcription and replication of DNA by upsetting proper DNA supercoiling. In a further embodiment, a topoisomerase is, without limitation, a type I topoisomerase inhibitor or a type II topoisomerase inhibitor. In an embodiment a type I topoisomerase inhibitor is, without limitation, a camptothecin. In another embodiment, a camptothecin is, without limitation, exatecan, irinotecan, lurtotecan, topotecan, BNP 1350, CKD 602, DB 67 (AR67) and/or ST 1481. In an embodiment, a type II topoisomerase inhibitor is, without limitation, epipodophyllotoxin. In a further embodiment an epipodophyllotoxin is, without limitation, an amsacrine, etoposid, etoposide phosphate and/or teniposide. In a further embodiment a topoisomerase is a synthetic, semisynthetic or derivative, including those found in nature such as, without limitation, epipodophyllotoxins, substances naturally occurring in the root of American Mayapple (Podophyllum peltatum).

In certain embodiments, the additional chemotherapeutic agent is a stilbenoid. In a further embodiment, a stilbenoid includes, but is not limited to, Resveratrol, Piceatannol, Pinosylvin, Pterostilbene, Alpha- Viniferin, Ampelopsin A, Ampelopsin E, Diptoindonesin C, Diptoindonesin F, Epsilon- Vinferin, Flexuosol A, Gnetin H, Hemsleyanol D, Hopeaphenol, Trans-Diptoindonesin B, Astringin, Piceid and Diptoindonesin A. In a further embodiment a stilbenoid is a synthetic, semisynthetic or derivative.

In certain embodiments, the additional chemotherapeutic agent is a cytotoxic antibiotic. In an embodiment, a cytotoxic antibiotic is, without limitation, an actinomycin, an anthracenedione, an anthracycline, thalidomide, dichloroacetic acid, nicotinic acid, 2- deoxyglucose and/or chlofazimine. In an embodiment, an actinomycin is, without limitation, actinomycin D, bacitracin, colistin (polymyxin E) and/or polymyxin B. In another embodiment, an antracenedione is, without limitation, mitoxantrone and/or pixantrone. In a further embodiment, an anthracycline is, without limitation, bleomycin, doxorubicin (Adriamycin), daunorubicin (daunomycin), epirubicin, idarubicin, mitomycin, plicamycin and/or valrubicin. In a further embodiment a cytotoxic antibiotic is a synthetic, semisynthetic or derivative.

In certain embodiments, the additional chemotherapeutic agent is selected from endostatin, angiogenin, angiostatin, chemokines, angioarrestin, angiostatin (plasminogen fragment), basement-membrane collagen-derived anti-angiogenic factors (tumstatin, canstatin, or arrestin), anti-angiogenic antithrombin III, signal transduction inhibitors, cartilage-derived inhibitor (CDI), CD59 complement fragment, fibronectin fragment, gro- beta, heparinases, heparin hexasaccharide fragment, human chorionic gonadotropin (hCG), interferon alpha/beta/gamma, interferon inducible protein (IP-10), interleukin-12, kringle 5 (plasminogen fragment), metalloproteinase inhibitors (TIMPs), 2-methoxyestradiol, placental ribonuclease inhibitor, plasminogen activator inhibitor, platelet factor-4 (PF4), prolactin 16 kD fragment, proliferin-related protein (PRP), various retinoids, tetrahydrocortisol-S, thrombospondin-1 (TSP-1), transforming growth factor-beta (TGF- β), vasculostatin, vasostatin (calreticulin fragment) and the like.

In certain embodiments, the additional chemotherapeutic agent is selected from abiraterone acetate, altretamine, anhydrovinblastine, auristatin, bexarotene, bicalutamide, BMS 184476, 2,3,4,5,6-pentafluoro-N-(3-fluoro-4-methoxyphenyl)benzene sulfonamide, bleomycin, N,N-dimethyl-L-valyl-L-valyl-N-methyl-L-valyl-L-proly-l-Lproline-t- butylamide, cachectin, cemadotin, chlorambucil, cyclophosphamide, 3',4'-didehydro-4'- deoxy-8'-norvin-caleukoblastine, docetaxol, doxetaxel, cyclophosphamide, carboplatin, carmustine, cisplatin, cryptophycin, cyclophosphamide, cytarabine, dacarbazine (DTIC), dactinomycin, daunorubicin, decitabine dolastatin, doxorubicin (adriamycin), etoposide, 5- fluorouracil, finasteride, flutamide, hydroxyurea and hydroxyureataxanes, ifosfamide, liarozole, lonidamine, lomustine (CCNU), MDV3100, mechlorethamine (nitrogen mustard), melphalan, mivobulin isethionate, rhizoxin, sertenef, streptozocin, mitomycin, methotrexate, taxanes, nilutamide, onapristone, paclitaxel, prednimustine, procarbazine, RPR109881, stramustine phosphate, tamoxifen, tasonermin, taxol, tretinoin, vinblastine, vincristine, vindesine sulfate, and vinflunine. In certain embodiments, the additional chemotherapeutic agent is platinum, cisplatin, carboplatin, oxaliplatin, mechlorethamine, cyclophosphamide, chlorambucil, azathioprine, mercaptopurine, vincristine, vinblastine, vinorelbine, vindesine, etoposide and teniposide, paclitaxel, docetaxel, irinotecan, topotecan, amsacrine, etoposide, etoposide phosphate, teniposide, 5-fluorouracil, leucovorin, methotrexate, gemcitabine, taxane, leucovorin, mitomycin C, tegafur-uracil, idarubicin, fludarabine, mitoxantrone, ifosfamide and doxorubicin. Additional agents include inhibitors of mTOR (mammalian target of rapamycin), including but not limited to rapamycin, everolimus, temsirolimus and deforolimus.

In still other embodiments, the additional chemotherapeutic agent can be selected from those delineated in U.S. Patent 7,927,613. In yet another embodiment, the methods can further include administering one or both of: (i) one or more anti-fungal agents (e.g., selected from the group of bifonazole, butoconazole, clotrimazole, econazole, ketoconazole, luliconazole, miconazole, omoconazole, oxiconazole, sertaconazole, sulconazole, tioconazole, albaconazole, efinaconazole, epoziconazole, fluconazole, isavuconazole, itraconazole, posaconazole, propiconazole, ravusconazole, terconazole, voriconazole, abafungin, amorolfin, butenafine, naftifine, terbinafine, anidulafungin, caspofungin, micafungin, benzoic acid, ciclopirox, flucytosine, 5-fluorocytosine, griseofulvin, haloprogin, tolnaflate, undecylenic acid, and balsam of peru) and (ii) one or more antibiotics (e.g., selected from the group of amikacin, gentamicin, kanamycin, neomycin, netilmicin, tobramycin, paromomycin, streptomycin, spectinomycin, geldanamycin, herbimycin, rifaximin, loracarbef, ertapenem, doripenem, imipenem, cilastatin, meropenem, cefadroxil, cefazolin, cefalotin, cefalothin, cefalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, dalbavancin, oritavancin, clindamycin, lincomycin, daptomycin, azithromycin, clarithromycin, dirithromycin, erythromycin, roxithromycin, troleandomycin, telithromycin, spiramycin, aztreonam, furazolidone, nitrofurantoin, linezolid, posizolid, radezolid, torezolid, amoxicillin, ampicillin, azlocillin, carbenicillin, cloxacillin, dicloxacillin, flucloxacillin, mezlocillin, methicillin, nafcillin, oxacillin, penicillin G, penicillin V, piperacillin, penicillin G, temocillin, ticarcillin, amoxicillin, calvulanate, ampicillin, subbactam, piperacillin, tazobactam, ticarcillin, clavulanate, bacitracin, colistin, polymyxin B, ciprofloxacin, enoxacin, gatifloxacin, gemifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfacetamide, sulfadiazine, silver sulfadiazine, sulfadimethoxine, sulfamethoxazole, sulfanamide, sulfasalazine, sulfisoxazole, trimethoprim-sulfamethoxazole, sulfonamideochrysoidine, demeclocycline, minocycline, oytetracycline, tetracycline, clofazimine, dapsone, dapreomycin, cycloserine, ethambutol, ethionamide, isoniazid, pyrazinamide, rifampicin, rifabutin, rifapentine, streptomycin, arsphenamine, chloramphenicol, fosfomycin, fusidic acid, metronidazole, mupirocin, platensimycin, quinupristin, dalopristin, thiamphenicol, tigecycyline, tinidazole, trimethoprim, and teixobactin).

In certain embodiments, the second therapeutic agent or regimen is administered to the subject prior to contacting with or administering the compound of the invention (e.g., about one hour prior, or about 6 hours prior, or about 12 hours prior, or about 24 hours prior, or about 48 hours prior, or about 1 week prior, or about 1 month prior).

In other embodiments, the second therapeutic agent or regimen is administered to the subject at about the same time as contacting with or administering the compound of the invention. By way of example, the second therapeutic agent or regimen and the compound of the invention are provided to the subject simultaneously in the same dosage form. As another example, the second therapeutic agent or regimen and the compound of the invention are provided to the subject concurrently in separate dosage forms.

In still other embodiments, the second therapeutic agent or regimen is administered to the subject after contacting with or administering the compound of the invention (e.g., about one hour after, or about 6 hours after, or about 12 hours after, or about 24 hours after, or about 48 hours after, or about 1 week after, or about 1 month after).

In some embodiments, the methods described herein further include the step of identifying a subject (e.g., a patient) in need of such treatment (e.g., by way of biopsy, endoscopy, or other conventional method known in the art). In certain embodiments, the NLRP3 protein can serve as a biomarker for certain types of cancer.

In some embodiments, the present compounds, methods, and compositions described herein can be administered to certain treatment-resistant patient populations (e.g., patients resistant to checkpoint inhibitors).

### Uses of the Present Compounds

The compounds of the present invention, or a salt or solvate thereof, can be used as modulators, e.g., agonists, of the NLRP3 receptor in medicaments as well as in the development of biological or diagnostic assays.

In addition, new small molecule NLRP3 activators are useful to study the biology of inflammasome and thus make an important contribution to the fundamental understanding of the activation mechanisms of the NLRP3 inflammasome and enable the targeted development of specific drugs.

The present compounds have been shown to induce typical characteristics of inflammasome activation, including the activation of caspase-1, the release of the biologically active forms of proinflammatory cytokines such as IL-1β (Figures 5A and 5B). Moreover, inflammasome-dependent lytic cell death (pyroptosis) and ASC speck formation are induced by the present compounds (Figure 6, 7, 13-16 and 18). For the induction of the lytic cell death, a concentration dependence can be observed (Figures 4A-C, 13-16 and 18). Figure 4D shows the EC50 for induction of IL-1β in BMDCs for compounds EN21 and EN22 in comparison to the imidazoquinoline NLRP3 activators imiquimod and CL097, wherein EC50 for EN21 and EN22 is surprisingly 10 times lower than for imiquimod and CL097.

Without wishing to be bound to a theory, it is assumed that the compounds EN21, EN22, BRM/BRG1 ATP Inhibitor-1, EN23, AH4, JB010 and EN54 activate the NLRP3 inflammasome, since the observed characteristics of inflammasome activation do not occur in cells isolated from NLRP3 knockout mice (Figures 7, 13-16 and 18) and, in addition, the NLRP3-specific inhibitor MCC950 completely suppresses inflammasome activation (Figure 8).

A further advantage of the present compounds is that the present compounds seem to activate NLRP3 rapidly. Especially with EN22, the inflammasome activation in BMDCs from wild-type mice without inhibitor is almost maximum after only 30 minutes. Thus, a rapid effectiveness of a medicament comprising the compounds of the invention is assumed.

It has further been shown that contrary to other small molecule NLRP3 inflammasome activators like the known activator imiquimod, the present compounds are more specific, since no simultaneous activation of Toll-like receptors (TLR7/ TLR8) occurs and thus the present compounds do not induce the release of further proinflammatory cytokines such as TNF or IL-6 (Figure 9 and 19).

In contrast to other known low molecular weight NLRP3 activators, the present compounds do not inhibit mitochondrial respiration and are therefore less susceptible to unspecific undesired effects (Figure 10).

Moreover, many known NLRP3 activators lead to massive potassium efflux from the cell via different mechanisms, which precedes inflammasome activation. Without wishing to be bound to a theory, it is assumed that the present compounds such as EN21 and EN22 activate the NLRP3 inflammasome independently of potassium efflux from the cell (Figures 11 and 17). It is assumed that reactive oxygen species (ROS) are involved in the mechanism of action of the present compounds, since the ROS scavenger ebselen can inhibit the activation of the inflammasome (Figure 11).

It has further been shown that in contrary to particulate NLRP3 activators such as uric acid crystals (mono sodium urate, MSU) or aluminum hydroxide, the present compounds are not endocyted by immune cells, followed by endolysosomal rupture to induce inflammasome activation. The endocytosis inhibitor cytochalasin D can prevent particle-induced NLRP3 activation, but cannot prevent NLRP3 activation by EN21 and EN22 (Figure 12).

Thus, the present compounds are important to the pharmaceutical industry because they can increase or rapidly induce in a selective manner the NLRP3 inflammasome.

According to the one aspect, the present invention relates to the present compounds for use as a medicament. In one embodiment, the present compounds for use as a medicament are used in a therapeutically effective amount.

The medicament can be in any physical form, such as a solid, semi-solid, plaster, solution, suspension, lotion, cream, foam, gel, paste, emulsion, or a combination thereof. Examples of the medicament include, but are not limited to, a pharmaceutical composition, an ingestible composition, a personal care composition, and a combination thereof.

According to the one aspect, the present invention relates to the present compounds for use in the prevention or treatment of a disease by activating the NLRP3 inflammasome, wherein the disease is selected from a group comprising infectious diseases or cancer.

In one embodiment, the present compounds may be used in the prevention or treatment of a bacterial infection.

In one embodiment, the present compounds may be used in the prevention or treatment of a viral infection. The viral infection may be selected from the group comprising genital warts, cutaneous warts and COVID-19.

In one embodiment, the present compounds may be used in the prevention of an infectious disease by boosting vaccination-mediated immunity against pathogens.

In one embodiment, the present compounds may be used in the prevention or treatment of a cancer. In one embodiment, the present compounds may be used in breaking the immune evasion in cancer diseases. The term "Immune evasion of cancer" or "cancer immune evasion" is the ability of cancer to persist in a host having a functioning immune system. There are a number of factors that contribute to tumor persistence despite having a normal host immune system. Immune editing is one of the key aspects why tumors evade surveillance causing the tumors to lie dormant in patients for years through "equilibrium" and "senescence" before re-emerging.

In one embodiment, the compounds of the present invention may be used as an adjuvant.

In one embodiment, the compounds of the present invention may be used to boost the immune response. The boost of the immune response by a compound of the present invention may be suitable during the prevention or treatment of cancer, an infectious disease or during a vaccination process. Preferably, the compounds of the present invention may be used as an adjuvant in the context of vaccination to boost specific immune responses by activating the NLRP3 inflammasome.

In one embodiment, the present compounds may be used in the prevention or treatment of cancer, wherein the cancer is selected from the group comprising solid tumors, skin cancer, wherein skin cancer is selected from the group comprising superficial basal cell carcinoma, genital warts and actinic keratosis, acute myeloid leukemia, adrenocortical carcinoma, Kaposi sarcoma, lymphoma, anal cancer, appendix cancer, teratoid/rhabdoid tumor, basal cell carcinoma, bile duct cancer, bladder cancer, bone cancer, brain cancer, breast cancer, bronchial tumor, carcinoid tumor, cardiac tumor, cervical cancer, chordoma, chronic lymphocytic leukemia, chronic myeloproliferative neoplasm, colon cancer, colorectal cancer, craniopharyngioma, bile duct cancer, endometrial cancer, ependymoma, esophageal cancer, esthesioneuroblastoma, Ewing sarcoma, eye cancer, fallopian tube cancer, gallbladder cancer, gastrointestinal carcinoid tumor, gastrointestinal stromal tumor, germ cell tumor, hairy cell leukemia, head and neck cancer, heart cancer, liver cancer, hypopharngeal cancer, pancreatic cancer, kidney cancer, laryngeal cancer, chronic myelogenous leukemia, lip and oral cavity cancer, lung cancer, melanoma, Merkel cell carcinoma, mesothelioma, mouth cancer, oral cancer, osteosarcoma, ovarian cancer, penile cancer, pharyngeal cancer, prostate cancer, rectal cancer, salivary gland cancer, skin cancer, small intestine cancer, soft tissue sarcoma, testicular cancer, throat cancer, thyroid cancer, urethral cancer, uterine cancer, vaginal cancer, and vulvar cancer.

In one embodiment, the present compounds may be used in the prevention or treatment of disease, wherein the compound activates the NLRP3 inflammasome and is preferably administered to a subject having a disease in which activation of the NLRP3 inflammasome contributes to the treatment of the pathology and/or symptoms of the disease and/or stops or decreases progression of the disease.

In one embodiment of the invention, a subject to be treated is a human or an animal, preferably a human.

In one embodiment of the invention, the present compounds are used in the activation of the NLRP3 inflammasome and TLR 7 and/or TLR 8 is not activated.

In one embodiment of the invention, the present compounds are administered in an amount of 0.0005 mg kg⁻¹ to 1000 mg kg⁻¹, 0.005 mg kg⁻¹ to 500 mg kg⁻¹, 0.05 mg kg⁻¹ to 100 mg kg⁻¹, 0.5 mg kg⁻¹ to 100 mg kg⁻¹, 5 mg kg⁻¹ to 75 mg kg⁻¹, preferably about 50 mg kg⁻¹ in a mouse or a human.

In one embodiment of the invention, the present compounds are administered topically, orally or systemically. In one embodiment of the invention, the present compounds are administered topically. In one embodiment of the invention, the present compounds are administered systemically.

According to the one aspect, the present invention relates to a pharmaceutical composition comprising a compound of the present invention and one or more pharmaceutically acceptable excipients.

In one embodiment of the invention, the pharmaceutical composition can be in any physical form, such as a solid, semi-solid, plaster, solution, suspension, lotion, cream, foam, gel, paste, emulsion, or a combination thereof. In one embodiment of the invention, the pharmaceutical composition is in form of a tablet, spray, ointment or gel, preferably an ointment.

In one embodiment of the present invention, the pharmaceutical composition comprises the present compound in a concentration ranging from about 0.0001 ppm to 100,000 ppm. In another embodiment of the composition, the present compound is in a concentration ranging from about 0.001 ppm to 10,000 ppm. In another embodiment of the composition, the present compound is in a concentration ranging from about 0.01 ppm to 1,000 ppm. In another embodiment of the composition, the present compound is in a concentration ranging from about 0.1 ppm to 500 ppm. In another embodiment of the composition, the present compound is in a concentration ranging from about 1 ppm to 500 ppm. In another embodiment of the composition, the present compound is in a concentration ranging from about 10 ppm to 500 ppm. In another embodiment of the composition, the present compound is in a concentration ranging from about 1 ppm to 400 ppm.

According to the one aspect, the present invention relates to the use of the compounds of the present invention for boosting immune responses or breaking immune evasion in cancer diseases in a subject.

According to the one aspect, the present invention relates to a method for modulating NLRP3 activity, the method comprising contacting NLRP3 with a compound of the present invention.

In one embodiment of the present invention, the method for modulating NLRP3 comprises increasing the activity of NLRP3.

In one embodiment of the present invention, the method for modulating NLRP3 comprises that the increase of activity of the NLRP3 inflammasome can be measured by the increase of the released amount of IL-1β of 0.25×10⁶ BMDCs, upon administering the compound in a concentration of 50µM of the compound and incubation of after 22 h, wherein the increase is at least 0.1 ng ml⁻¹, 0.5 ng ml⁻¹, 0.75 ng ml⁻¹, 1 ng ml⁻¹ 2 ng ml⁻¹, 10 ng ml⁻¹, 25 ng ml⁻¹, 50 ng ml⁻¹ and more preferably 4 ng ml⁻¹ or more, and still more preferably 6 ng ml⁻¹.

In one embodiment of the present invention, the method for modulating NLRP3 is carried out *in vitro* or *in vivo.*

According to the one aspect, the present invention relates to a method of treating cancer, comprising administering to a subject in need of such treatment an effective amount of a compound of the present invention, or a pharmaceutical composition of the present invention.

In one embodiment of the present invention, the method of treating cancer comprises that the compound is administered in combination with one or more additional cancer therapies.

In one embodiment of the present invention, the method of treating cancer comprises the one or more additional cancer therapies, which comprises surgery, radiotherapy, chemotherapy, toxin therapy, immunotherapy, cryotherapy or gene therapy, or a combination thereof.

In one embodiment of the present invention, the method of treating cancer comprises chemotherapy, which comprises administering one or more additional chemotherapeutic agents.

In one embodiment of the present invention, the method of treating cancer comprises chemotherapy, which comprises administering one or more additional chemotherapeutic agents, signal transduction inhibitors (kinase inhibitors) and immunomodulators (checkpoint inhibitors and STING agonists).

In one embodiment of the present invention, the method of treating cancer comprises chemotherapy, which comprises administering one or more additional chemotherapeutic agents, wherein the one or more additional chemotherapeutic agents is selected from an alkylating agent (e.g., cisplatin, carboplatin, mechlorethamine, cyclophosphamide, chlorambucil, ifosfamide and/or oxaliplatin); an anti-metabolite (e.g.,azathioprine and/or mercaptopurine); a terpenoid (e.g., a vinca alkaloid and/or a taxane; e.g., Vincristine, Vinblastine, Vinorelbine and/or Vindesine Taxol, Pacllitaxel and/or Docetaxel); a topoisomerase (e.g., a type I topoisomerase and/or a type 2 topoisomerase; e.g., camptothecins, such as irinotecan and/or topotecan;. amsacrine, etoposide, etoposide phosphate and/or teniposide); a cytotoxic antibiotic (e.g., actinomycin, anthracyclines, doxorubicin, daunorubicin, valrubicin, idarubicin, epirubicin, bleomycin, plicamycin and/or mitomycin); a hormone (e.g., a lutenizing hormone releasing hormone agonist; e.g., leuprolidine, goserelin, triptorelin, histrelin, bicalutamide, flutamide and/or nilutamide); an antibody (e.g., Abciximab, Adalimumab, Alemtuzumab, Atlizumab, Basiliximab, Belimumab, Bevacizumab, Bretuximab vedotin, Canakinumab, Cetuximab, Ceertolizumab pegol, Daclizumab, Denosumab, Eculizumab, Efalizumab, Gemtuzumab, Golimumab, Golimumab, Ibritumomab tiuxetan, Infliximab, Ipilimumab, Murom onab-CD3, Natalizumab, Ofatumumab, Omalizumab, Palivizumab, Panitumuab, Ranibizumab, Rituximab, Tocilizumab, Tositumomab and/or Trastuzumab); an anti- angiogenic agent; a cytokine; a thrombotic agent; a growth inhibitory agent; an antihelminthic agent; and an immune checkpoint inhibitor that targets an immune checkpoint receptor selected from the group consisting of CTLA-4, PD-1, PD-L1, PD-1 - PD-L1, PD- 1 - PD-L2, T cell immunoglobulin and mucin 3 (TFM3 or HAVCR2), Galectin 9 - TIM3, Phosphatidylserine - TIM3, lymphocyte activation gene 3 protein (LAG3), MHC class II, - LAG3, 4- 1BB-4- 1BB ligand, OX40-OX40 ligand, GITR, GITR ligand - GITR, CD27, CD70-CD27, TNFRSF25, TNFRSF25-TL1 A, CD40L, CD40-CD40 ligand, HVEM- LIGHT-LTA, HVEM, HVEM - BTLA, HVEM - CD 160, HVEM - LIGHT, HVEM- BTLA-CD160, CD80, CD80 - PD-L1, PD-L2 - CD80, CD244, CD48 - CD244, CD244, ICOS, ICOS-ICOS ligand, B7-H3, B7-H4, VISTA, TMIGD2, HHLA2-TMIGD2, Butyrophilins, including BTNL2, Siglec family, TIGIT and PVR family members, K Rs, ILTs and LIRs, NKG2D and NKG2A, MICA and MICB, CD244, CD28, CD86 - CD28, CD86 - CTLA, CD80 - CD28, Phosphatidylserine, TΓM3, Phosphatidylserine - TΓM3, SIRPA-CD47, VEGF, Neuropilin, CD160, CD30, and CD155 (e.g., CTLA-4 or PD-1 or PD-L1) and other immunomodulatory agents, such as interleukin-2 (IL-2), indoleamine, 2,3-dioxygenase (IDO), IL- 10, transforming growth factor-β (TGFp), CD39, CD73 Adenosine-CD39-CD73, and CXCR4-CXCL12.

Additional chemotherapeutic agents might be immune checkpoint inhibitors.

Preferred additional chemotherapeutic agents and/or immune checkpoint inhibitors are selected from the group consisting of Urelumab, PF-05082566, MEDI6469, TRX518, Varlilumab, CP-870893, Pembrolizumab (PD-1), Nivolumab (PD1), Atezolizumab (PD-L1), PDR001 (PD-1), BMS-986016, MGA271, Lirlumab, IPH2201, Emactuzumab, INCB024360, Galunisertib, Ulocuplumab, BKT140, Bavituximab, CC-90002, Bevacizumab, 1VINRP1685A, and MGA271.

According to the one aspect, the present invention relates to a method for analyzing the activity of NLRP3 activation.

The NLRP3 inflammasome activation may be tested in different cell types like for examples primary murine bone marrow-derived dendritic cells (BMDCs) and macrophages (BMDMs), THP-1 cells, human peripheral blood mononuclear cells (PBMCs). Cells are typically primed with 50 ng ml⁻¹ LPS for 3 hours before inflammasome activation. Markers of inflammasome activation are investigated by measuring release of the cytosolic enzyme lactate dehydrogenase (LDH) from the cells as a readout for lytic cell death and pyroptosis, detecting release of proinflammatory cytokines such as IL-1β into the cell supernatants by enzyme-linked immunosorbent assay (ELISA), looking at caspase 1 activation and cleavage of Gasdermin D and pro IL-1β via Immunoblotting and following ASC speck formation via immunofluorescence imaging.

According to one embodiment, the present invention relates to the method for analyzing the NLRP3 activation, which comprises the steps of: (i) priming cells with LPS, (ii) contacting the cells with present compounds (iii) detecting the release of IL-1β into the cell supernatant by enzyme-linked immunosorbent assay (ELISA) and analyzing the cells by immunoblotting and immunofluorescence imaging.

According to the one aspect, the present invention relates to the use of the present compounds for analyzing the activity of NLRP3 activation in vitro.

### Examples

### 1. Biological Assays

The present compounds are useful as modulators of NLRP3. In a high-throughput screen using a screening method developed by us, new, specific NLRP3 activators out of about 50.000 small molecule compounds have been identified, which differ in their structure and several properties of NLRP3 activation from all previously known and protected NLRP3 activators and are novel and unique in them.

### 1.1 In vitro preliminary data and properties of the activators

All NLRP3 activators presented herein are sufficiently soluble and cell-permeable inflammasome activators: The activators showed sufficient solubility in solvents such as DMSO and have the ability to pass the cell membrane, as only then the detection by the high-throughput screening is possible. Moreover, the present compounds showed good activity *in vitro.* This was tested in cell culture in several different *in vitro* assays classically used to investigate inflammasome activation. These include measuring inflammasome-dependent lytic cell death (pyroptosis) by detecting release of the cytosolic enzyme lactate dehydrogenase (LDH) using a commercial colorimetric assay and release of proinflammatory cytokines like IL-1β by enzyme-linked immunosorbent assay (ELISA), analyzing cleavage and activation of caspase-1 and IL-1β by immunoblotting and immunofluorescence imaging of ASC speck formation. Lytic cell death was determined via LDH release. Assay results for compounds are illustrated in the Tables below, wherein the concentration of the compounds tested had been 50 µM or as indicated in the table, and the concentration of the known activators has been tested with 5 µM of nigericin, 100 µM of each R837 and CL097. For classification of the activity of a given molecule, release of IL-1β was quantified as % of IL-1β of a respective control activator used for the experiment (highlighted in tables with bold letters). The compounds listes in Table 1 were classified into 4 groups depending on their ability to induce the NLRP3 inflammasome activation, assessed by % IL-1β release as compared to the positive control. The activity thresholds were assigned as follows:
(++++) >75% IL-1β (% positive control)
(+++) 10-75% IL-1β (% positive control)
(++) 1-10% IL-1β (% positive control)
(+) 0,8-1% IL-1β (% positive control)

### 1. In vitro data and properties of the activators

### 1.1. EN21, EN22, L01.xxx

| **No.** | **Compound** | **LDH release (% of max.)** | | | | | | **Average LDH release (% of max.)** | **Average (% positive control)** |
|---|---|---|---|---|---|---|---|---|---|
| | DMSO | 8,053 | 7,414 | 8,022 | 8,032 | 10,176 | 6,889 | 8,098 | 11,069 |
| | **Nigericin 5 µM** | **61,933** | **78,845** | **69,982** | **78,413** | **75,764** | **74,003** | **73,157** | **100,000** |
| 2 | EN21 | 36,356 | 47,704 | 43,019 | 50,628 | 60,994 | 63,141 | 50,307 | 68,766 |
| 1 | EN22 | 59,410 | 74,624 | 67,018 | 74,225 | 71,324 | 71,181 | 69,630 | 95,180 |
| | R837 100 µM | 12,734 | 15,684 | 15,495 | 20,399 | 34,109 | 57,377 | 25,966 | 35,494 |
| | CL097 100 µM | 56,511 | 69,145 | 62,611 | 76,051 | 68,018 | 76,242 | 68,096 | 93,083 |
| 17 | L01.110 | 34,845 | 34,294 | 31,850 | | | | 33,663 | 46,015 |
| 18 | L01.041 | 24,371 | 24,119 | 23,567 | | | | 24,019 | 32,832 |
| 19 | L01.004 | 15,163 | 16,970 | 18,507 | | | | 16,880 | 23,074 |
| 20 | L01.007 | 17,823 | 23,721 | 19,371 | | | | 20,305 | 27,756 |
| 21 | L01.104 | 17,190 | 17,671 | 19,727 | | | | 18,196 | 24,873 |
| 22 | L01.116 | 17,956 | 18,328 | 17,892 | | | | 18,058 | 24,685 |
| 23 | L01.113 | 37,377 | 36,390 | 34,335 | | | | 36,034 | 49,256 |
| 24 | L01.101 | 19,384 | 19,564 | 20,661 | | | | 19,870 | 27,161 |
| 25 | L01.092 | 16,396 | 14,952 | 13,605 | | | | 14,985 | 20,483 |
| 26 | L01.124 | 14,119 | 13,508 | 14,589 | | | | 14,072 | 19,235 |
| 33 | L01.016 | 11,582 | 12,903 | 13,299 | | | | 12,595 | 17,216 |
| 34 | L01.028 | 12,612 | 15,129 | 15,770 | | | | 14,503 | 19,825 |
| 35 | L01.047 | 12,065 | 12,727 | 11,451 | | | | 12,081 | 16,514 |
| 36 | L01.026 | 9,228 | 9,969 | 13,232 | | | | 10,810 | 14,776 |
| 37 | L01.127 | 10,995 | 10,459 | 9,932 | | | | 10,462 | 14,301 |
| 38 | L01.061 | 15,898 | 13,336 | 13,232 | | | | 14,155 | 19,349 |
| 39 | L01.128 | 12,143 | 11,691 | 11,469 | | | | 11,768 | 16,086 |
| 40 | L01.025 | 15,039 | 12,654 | 15,391 | | | | 14,362 | 19,631 |
| 41 | L01.008 | 10,960 | 9,763 | 7,910 | | | | 9,544 | 13,046 |
| 42 | L01.089 | 8,669 | 6,519 | 7,642 | | | | 7,610 | 10,402 |
| 43 | L01.130 | 9,387 | 8,722 | 7,450 | | | | 8,520 | 11,646 |
| 44 | L01.049 | 12,648 | 10,774 | 11,897 | | | | 11,773 | 16,093 |
| 45 | L01.066 | 15,611 | 15,078 | 12,310 | | | | 14,333 | 19,592 |
| 46 | L01.043 | 8,072 | 7,620 | 8,535 | | | | 8,076 | 11,039 |
| 47 | L01.087 | 6,788 | 7,261 | 6,936 | | | | 6,995 | 9,562 |
| 48 | L01.020 | 11,587 | 11,294 | 9,407 | | | | 10,763 | 14,712 |
| 49 | L01.035 | 8,461 | 8,303 | 7,473 | | | | 8,079 | 11,044 |
| 50 | L01.120 | 24,981 | 25,196 | 23,234 | | | | 24,470 | 33,449 |
| 51 | L01.033 | 7,999 | 9,134 | 7,247 | | | | 8,127 | 11,108 |
| 52 | L01.002 | 8,068 | 10,017 | 11,898 | | | | 9,994 | 13,662 |
| 53 | L01.027 | 5,203 | 7,373 | 8,025 | | | | 6,867 | 9,387 |
| 54 | L01.003 | 6,283 | 8,927 | 8,221 | | | | 7,810 | 10,676 |
| 55 | L01.038 | 12,838 | 11,939 | 11,078 | | | | 11,952 | 16,337 |
| 56 | L01.069 | 9,197 | 8,968 | 9,736 | | | | 9,301 | 12,713 |
| 57 | L01.097 | 39,470 | 35,627 | 37,427 | | | | 37,508 | 51,271 |
| 58 | L01.095 | 5,207 | 7,088 | 5,172 | | | | 5,822 | 7,959 |
| 59 | L01.013 | 8,752 | 7,323 | 6,537 | | | | 7,537 | 10,303 |
| 60 | L01.103 | 6,576 | 7,017 | 10,089 | | | | 7,894 | 10,791 |
| 61 | L01.071 | 9,014 | 9,438 | 7,518 | | | | 8,657 | 11,833 |
| 62 | L01.115 | 23,646 | 22,026 | 20,929 | | | | 22,200 | 30,346 |
| 63 | L01.045 | 5,976 | 5,813 | 3,780 | | | | 5,189 | 7,094 |
| 64 | L01.077 | 5,283 | 6,127 | 6,102 | | | | 5,838 | 7,980 |
| 65 | L01.084 | 6,005 | 6,209 | 5,461 | | | | 5,892 | 8,054 |
| 66 | L01.109 | 8,457 | 7,795 | 7,697 | | | | 7,983 | 10,912 |
| 67 | L01.122 | 9,165 | 8,128 | 6,967 | | | | 8,087 | 11,054 |
| 68 | L01.083 | 6,798 | 6,504 | 5,680 | | | | 6,327 | 8,649 |
| 69 | L01.062 | 13,450 | 13,307 | 13,003 | | | | 13,253 | 18,117 |
| 70 | L01.081 | 6,870 | 5,589 | 6,539 | | | | 6,332 | 8,656 |
| 71 | L01.114 | 9,462 | 9,932 | 8,701 | | | | 9,365 | 12,801 |
| 72 | L01.090 | 15,329 | 15,756 | 14,149 | | | | 15,078 | 20,610 |
| 73 | L01.136 | 6,954 | 7,428 | 6,391 | | | | 6,924 | 9,465 |
| 74 | L01.023 | 5,103 | 5,764 | 5,837 | | | | 5,568 | 7,611 |
| 75 | L01.064 | 8,962 | 8,659 | 8,624 | | | | 8,748 | 11,958 |
| 76 | L01.093 | 6,676 | 7,891 | 9,879 | | | | 8,149 | 11,139 |
| 77 | L01.125 | 7,721 | 6,471 | 6,431 | | | | 6,874 | 9,397 |
| 78 | L01.005 | 9,311 | 16,790 | 18,315 | | | | 14,805 | 20,238 |
| 79 | L01.135 | 8,895 | 7,690 | 7,743 | | | | 8,109 | 11,085 |
| 80 | L01.108 | 10,902 | 10,269 | 10,350 | | | | 10,507 | 14,362 |
| 81 | L01.112 | 7,964 | 6,971 | 7,035 | | | | 7,323 | 10,010 |
| 82 | L01.054 | 7,787 | 8,452 | 8,848 | | | | 8,362 | 11,431 |
| 83 | L01.067 | 6,481 | 6,962 | 7,048 | | | | 6,830 | 9,336 |
| 84 | L01.099 | 10,559 | 9,299 | 9,572 | | | | 9,810 | 13,410 |
| 85 | L01.065 | 8,406 | 9,014 | 9,788 | | | | 9,069 | 12,397 |
| 86 | L01.132 | 6,462 | 6,201 | 6,631 | | | | 6,431 | 8,791 |
| 87 | L01.121 | 7,801 | 9,369 | 8,962 | | | | 8,711 | 11,907 |
| 88 | L01.056 | 9,335 | 10,160 | 9,599 | | | | 9,698 | 13,256 |
| 89 | L01.088 | 5,685 | 4,709 | 5,350 | | | | 5,248 | 7,174 |
| 90 | L01.042 | 7,095 | 8,482 | 6,354 | | | | 7,310 | 9,993 |
| 91 | L01.010 | 5,639 | 6,006 | 5,521 | | | | 5,722 | 7,822 |

| **No.** | **Compound** | **IL-1β (ng/ml)** | | | | | | **Average IL-1β (ng/ml)** | **Average (% positive control)** |
|---|---|---|---|---|---|---|---|---|---|
| | DMSO | 0,042 | 0,054 | 0,055 | 0,067 | 0,057 | 0,056 | 0,055 | 0,769 |
| | **Nigericin 5 µM** | **6,834** | **6,400** | **7,872** | **6,518** | **7,890** | **7,496** | **7,168** | **100,000** |
| 2 | EN21 | 8,582 | 7,428 | 8,023 | 6,613 | 8,352 | 7,696 | 7,782 | 108,566 |
| 1 | EN22 | 8,679 | 9,014 | 9,589 | 8,363 | 10,000 | 9,819 | 9,244 | 128,957 |
| | R837 100 µM | 5,472 | 4,151 | 1,552 | 1,212 | 0,958 | 0,911 | 2,376 | 33,144 |
| | CL097 100 µM | 7,355 | 8,020 | 8,660 | 8,522 | 9,540 | 9,324 | 8,570 | 119,560 |
| 17 | L01.110 | 2,505 | 2,943 | 2,423 | | | | 2,624 | 36,599 |
| 18 | L01.041 | 1,508 | 1,847 | 1,769 | | | | 1,708 | 23,826 |
| 19 | L01.004 | 1,192 | 1,485 | 1,567 | | | | 1,415 | 19,737 |
| 20 | L01.007 | 0,859 | 1,572 | 1,101 | | | | 1,177 | 16,422 |
| 21 | L01.104 | 1,103 | 1,142 | 1,246 | | | | 1,163 | 16,230 |
| 22 | L01.116 | 1,107 | 1,192 | 1,163 | | | | 1,154 | 16,099 |
| 23 | L01.113 | 1,145 | 1,165 | 1,064 | | | | 1,125 | 15,691 |
| 24 | L01.101 | 1,160 | 1,071 | 1,041 | | | | 1,091 | 15,216 |
| 25 | L01.092 | 1,253 | 1,060 | 0,927 | | | | 1,080 | 15,066 |
| 26 | L01.124 | 0,928 | 0,902 | 1,024 | | | | 0,951 | 13,268 |
| 33 | L01.016 | 0,755 | 0,723 | 0,857 | | | | 0,778 | 10,857 |
| 34 | L01.028 | 0,686 | 0,757 | 0,705 | | | | 0,716 | 9,988 |
| 35 | L01.047 | 0,505 | 0,513 | 0,512 | | | | 0,510 | 7,117 |
| 36 | L01.026 | 0,452 | 0,473 | 0,562 | | | | 0,495 | 6,911 |
| 37 | L01.127 | 0,490 | 0,475 | 0,441 | | | | 0,469 | 6,538 |
| 38 | L01.061 | 0,508 | 0,439 | 0,420 | | | | 0,456 | 6,360 |
| 39 | L01.128 | 0,456 | 0,394 | 0,402 | | | | 0,417 | 5,819 |
| 40 | L01.025 | 0,333 | 0,280 | 0,490 | | | | 0,368 | 5,130 |
| 41 | L01.008 | 0,361 | 0,324 | 0,313 | | | | 0,332 | 4,638 |
| 42 | L01.089 | 0,313 | 0,316 | 0,344 | | | | 0,324 | 4,526 |
| 43 | L01.130 | 0,404 | 0,317 | 0,215 | | | | 0,312 | 4,350 |
| 44 | L01.049 | 0,330 | 0,251 | 0,319 | | | | 0,300 | 4,189 |
| 45 | L01.066 | 0,346 | 0,256 | 0,193 | | | | 0,265 | 3,698 |
| 46 | L01.043 | 0,282 | 0,272 | 0,242 | | | | 0,265 | 3,697 |
| 47 | L01.087 | 0,237 | 0,239 | 0,296 | | | | 0,257 | 3,589 |
| 48 | L01.020 | 0,236 | 0,231 | 0,233 | | | | 0,233 | 3,256 |
| 49 | L01.035 | 0,321 | 0,189 | 0,181 | | | | 0,230 | 3,211 |
| 50 | L01.120 | 0,207 | 0,200 | 0,196 | | | | 0,201 | 2,800 |
| 51 | L01.033 | 0,197 | 0,197 | 0,186 | | | | 0,193 | 2,698 |
| 52 | L01.002 | 0,144 | 0,249 | 0,164 | | | | 0,186 | 2,590 |
| 53 | L01.027 | 0,149 | 0,197 | 0,181 | | | | 0,176 | 2,451 |
| 54 | L01.003 | 0,167 | 0,153 | 0,175 | | | | 0,165 | 2,301 |
| 55 | L01.038 | 0,166 | 0,127 | 0,128 | | | | 0,140 | 1,956 |
| 56 | L01.069 | 0,131 | 0,138 | 0,149 | | | | 0,139 | 1,941 |
| 57 | L01.097 | 0,121 | 0,143 | 0,116 | | | | 0,126 | 1,765 |
| 58 | L01.095 | 0,137 | 0,128 | 0,102 | | | | 0,122 | 1,703 |
| 59 | L01.013 | 0,118 | 0,126 | 0,117 | | | | 0,120 | 1,677 |
| 60 | L01.103 | 0,060 | 0,049 | 0,240 | | | | 0,116 | 1,624 |
| 61 | L01.071 | 0,116 | 0,097 | 0,131 | | | | 0,115 | 1,598 |
| 62 | L01.115 | 0,122 | 0,108 | 0,111 | | | | 0,114 | 1,584 |
| 63 | L01.045 | 0,103 | 0,116 | 0,118 | | | | 0,112 | 1,568 |
| 64 | L01.077 | 0,067 | 0,165 | 0,062 | | | | 0,098 | 1,368 |
| 65 | L01.084 | 0,083 | 0,111 | 0,099 | | | | 0,098 | 1,362 |
| 66 | L01.109 | 0,120 | 0,083 | 0,070 | | | | 0,091 | 1,269 |
| 67 | L01.122 | 0,072 | 0,093 | 0,095 | | | | 0,087 | 1,214 |
| 68 | L01.083 | 0,080 | 0,077 | 0,097 | | | | 0,085 | 1,180 |
| 69 | L01.062 | 0,084 | 0,087 | 0,074 | | | | 0,082 | 1,141 |
| 70 | L01.081 | 0,075 | 0,072 | 0,088 | | | | 0,078 | 1,093 |
| 71 | L01.114 | 0,073 | 0,081 | 0,072 | | | | 0,075 | 1,049 |
| 72 | L01.090 | 0,079 | 0,077 | 0,069 | | | | 0,075 | 1,044 |
| 73 | L01.136 | 0,064 | 0,073 | 0,083 | | | | 0,073 | 1,025 |
| 74 | L01.023 | 0,065 | 0,075 | 0,080 | | | | 0,073 | 1,021 |
| 75 | L01.064 | 0,065 | 0,054 | 0,100 | | | | 0,073 | 1,014 |
| 76 | L01.093 | 0,056 | 0,050 | 0,108 | | | | 0,072 | 0,998 |
| 77 | L01.125 | 0,080 | 0,071 | 0,061 | | | | 0,071 | 0,988 |
| 78 | L01.005 | 0,053 | 0,074 | 0,084 | | | | 0,070 | 0,980 |
| 79 | L01.135 | 0,079 | 0,073 | 0,057 | | | | 0,070 | 0,975 |
| 80 | L01.108 | 0,076 | 0,061 | 0,072 | | | | 0,070 | 0,973 |
| 81 | L01.112 | 0,075 | 0,069 | 0,061 | | | | 0,068 | 0,955 |
| 82 | L01.054 | 0,067 | 0,062 | 0,074 | | | | 0,068 | 0,947 |
| 83 | L01.067 | 0,064 | 0,069 | 0,070 | | | | 0,068 | 0,942 |
| 84 | L01.099 | 0,074 | 0,067 | 0,059 | | | | 0,067 | 0,932 |
| 85 | L01.065 | 0,052 | 0,070 | 0,077 | | | | 0,067 | 0,928 |
| 86 | L01.132 | 0,062 | 0,066 | 0,069 | | | | 0,066 | 0,920 |
| 87 | L01.121 | 0,066 | 0,068 | 0,060 | | | | 0,065 | 0,906 |
| 88 | L01.056 | 0,060 | 0,073 | 0,062 | | | | 0,065 | 0,905 |
| 89 | L01.088 | 0,069 | 0,058 | 0,059 | | | | 0,062 | 0,866 |
| 90 | L01.042 | 0,060 | 0,069 | 0,048 | | | | 0,059 | 0,826 |
| 91 | L01.010 | 0,065 | 0,059 | 0,053 | | | | 0,059 | 0,822 |

### 1.2. EN23

| **No.** | **Compound** | **LDH release (% of max.)** | | | **Average LDH release (% of max.)** | **Average (% positive control)** |
|---|---|---|---|---|---|---|
| | Medium | 1,952 | 1,418 | 1,059 | 1,476 | 4,927 |
| **1** | **EN22 25 µM** | **29,575** | **29,703** | **30,593** | **29,957** | **100,000** |
| 3 | EN-A 25 µM | 33,731 | 33,093 | 32,555 | 33,126 | 110,581 |
| 3 | EN-A 50 µM | 32,141 | 32,628 | 32,392 | 32,387 | 108,112 |
| 3 | EN-A 100 µM | 34,821 | 33,136 | 31,042 | 33,000 | 110,157 |

| **No.** | **Compound** | **IL-1β (ng/ml)** | | | **Average IL-1β (ng/ml)** | **Average (% positive control)** |
|---|---|---|---|---|---|---|
| | Medium | 0,026 | 0,023 | 0,023 | 0,024 | 0,472 |
| **1** | **EN22 25 µM** | **4,821** | **5,196** | **5,062** | **5,026** | **100,000** |
| 3 | EN-A 25 µM | 5,256 | 4,576 | 4,256 | 4,696 | 93,430 |
| 3 | EN-A 50 µM | 4,819 | 5,538 | 4,722 | 5,026 | 100,007 |
| 3 | EN-A 100 µM | 5,256 | 5,328 | 4,669 | 5,084 | 101,159 |

### 1.3. BRM/BRG1 ATP Inhibitor-1

| **No.** | **Compound** | **LDH release (% of max.)** | | | **Average LDH release (% of max.)** | **Average (% positive control)** |
|---|---|---|---|---|---|---|
| | Medium | 5,371 | 6,529 | 3,826 | 5,242 | 9,764 |
| | **Nigericin 5 µM** | **50,151** | **43,167** | **67,752** | **53,690** | **100,000** |
| 6 | BRM/BRG1 ATP Inhibitor-1 5 µM | 14,128 | 16,194 | 19,482 | 16,601 | 30,921 |
| 6 | BRM/BRG1 ATP Inhibitor-1 50 µM | 38,464 | 36,463 | 31,314 | 35,414 | 65,959 |
| 6 | BRM/BRG1 ATP Inhibitor-1 100 µM | 26,966 | 18,468 | 28,907 | 24,780 | 46,154 |

| **No.** | **Compound** | **IL-1β (ng/ml)** | | | **Average IL-1β (ng/ml)** | **Average (% positive control)** |
|---|---|---|---|---|---|---|
| | Medium | 0 | 0,027929 | 0 | 0,009 | 0,29548556 |
| | **Nigericin 5 µM** | **3,007** | **3,127** | **3,318** | **3,151** | **100,000** |
| 6 | BRM/BRG1 ATP Inhibitor-1 5 µM | 3,556 | 3,899 | 3,537 | 3,664 | 116,295 |
| 6 | BRM/BRG1 ATP Inhibitor-1 50 µM | 4,561 | 6,642 | 6,737 | 5,980 | 189,7925 |
| 6 | BRM/BRG1 ATP Inhibitor-1 100 µM | 7,150 | 3,831 | 4,087 | 5,023 | 159,415 |

### 1.4. PM07, PM16, TCMDC-143175, A.C2

| **No.** | **Compound** | **LDH release (% of max.)** | | | **Average LDH release (% of max.)** | **Average (% positive control)** |
|---|---|---|---|---|---|---|
| | Medium | 7,050 | 7,244 | 6,625 | 6,973 | 9,774 |
| **1** | **EN22 18,75 µM** | **71,367** | **72,290** | **70,369** | **71,342** | **100,000** |
| 8 | PM07 18,75 µM | 50,410 | 51,715 | 50,881 | 51,002 | 71,489 |
| 8 | PM07 37,5 µM | 67,709 | 69,716 | 70,399 | 69,274 | 97,102 |
| 8 | PM07 75 µM | 85,105 | 85,806 | 86,028 | 85,647 | 120,051 |
| 9 | PM16 18,75 µM | 72,042 | 74,209 | 72,166 | 72,806 | 102,052 |
| 9 | PM16 37,5 µM | 88,994 | 89,909 | 91,152 | 90,019 | 126,179 |
| 9 | PM16 75 µM | 100,397 | 95,086 | 96,871 | 97,451 | 136,598 |
| 27 | TCMDC-143175 18,75 µM | 35,255 | 34,424 | 36,807 | 35,495 | 49,754 |
| 27 | TCMDC-143175 37,5 µM | 72,817 | 79,023 | 72,133 | 74,658 | 104,647 |
| 27 | TCMDC-143175 75 µM | 91,472 | 93,190 | 89,533 | 91,399 | 128,113 |
| 28 | A.C2 18,75 µM | 18,410 | 18,465 | 16,442 | 17,772 | 24,912 |
| 28 | A.C2 37,5 µM | 46,587 | 45,313 | 45,110 | 45,670 | 64,016 |
| 28 | A.C2 75 µM | 77,148 | 73,140 | 77,102 | 75,797 | 106,244 |

| **No.** | **Compound** | **IL-1β (ng/ml)** | | | **Average IL-1β (ng/ml)** | **Average (% positive control)** |
|---|---|---|---|---|---|---|
| | Medium | 0,040 | 0,036 | 0,054 | 0,043 | 0,684 |
| **1** | **EN22 18,75 µM** | **7,071** | **5,864** | **6,030** | **6,322** | **100,000** |
| 8 | PM07 18,75 µM | 1,117 | 1,248 | 1,641 | 1,335 | 21,124 |
| 8 | PM07 37,5 µM | 1,916 | 1,571 | 1,295 | 1,594 | 25,211 |
| 8 | PM07 75 µM | 1,202 | 1,130 | 1,320 | 1,217 | 19,256 |
| 9 | PM16 18,75 µM | 0,764 | 0,830 | 0,736 | 0,777 | 12,288 |
| 9 | PM16 37,5 µM | 0,895 | 0,795 | 0,848 | 0,846 | 13,380 |
| 9 | PM16 75 µM | 0,876 | 0,848 | 0,948 | 0,890 | 14,083 |
| 27 | TCMDC-143175 18,75 µM | 0,102 | 0,104 | 0,110 | 0,105 | 1,666 |
| 27 | TCMDC-143175 37,5 µM | 0,309 | 0,275 | 0,290 | 0,291 | 4,609 |
| 27 | TCMDC-143175 75 µM | 0,423 | 0,381 | 0,446 | 0,417 | 6,592 |
| 28 | A.C2 18,75 µM | 0,128 | 0,116 | 0,111 | 0,118 | 1,870 |
| 28 | A.C2 37,5 µM | 0,377 | 0,353 | 0,353 | 0,361 | 5,712 |
| 28 | A.C2 75 µM | 0,459 | 0,546 | 0,571 | 0,525 | 8,308 |

### 1.5. AH4, AH1, AH3, AH2

| **No.** | **Compound** | **LDH release (% of max.)** | | | **Average LDH release (% of max.)** | **Average (% positive control)** |
|---|---|---|---|---|---|---|
| | Medium | 8,589 | 9,236 | 8,871 | 8,898 | 11,586 |
| **1** | **EN22 10 µM** | **78,095** | **74,908** | **77,404** | **76,802** | **100,000** |
| 4 | AH4 25 µM | 19,952 | 17,994 | 18,179 | 18,709 | 24,359 |
| 4 | AH4 50 µM | 57,619 | 55,868 | 55,036 | 56,175 | 73,142 |
| 4 | AH4 75 µM | 67,857 | 68,689 | 68,988 | 68,511 | 89,204 |
| 11 | AH1 25 µM | 42,075 | 40,911 | 40,281 | 41,089 | 53,499 |
| 11 | AH1 50 µM | 55,721 | 59,555 | 56,124 | 57,133 | 74,390 |
| 11 | AH1 75 µM | 60,680 | 59,098 | 60,723 | 60,167 | 78,340 |
| 12 | AH3 25 µM | 12,835 | 13,259 | 12,460 | 12,851 | 16,733 |
| 12 | AH3 50 µM | 22,186 | 22,899 | 22,877 | 22,654 | 29,496 |
| 12 | AH3 75 µM | 34,626 | 33,827 | 33,778 | 34,077 | 44,370 |
| 29 | AH2 25 µM | 23,861 | 22,415 | 22,110 | 22,795 | 29,680 |
| 29 | AH2 50 µM | 22,752 | 21,681 | 21,969 | 22,134 | 28,819 |
| 29 | AH2 75 µM | 25,084 | 25,530 | 26,645 | 25,753 | 33,532 |

| **No.** | **Compound** | **IL-1β (ng/ml)** | | | **Average IL-1β (ng/ml)** | **Average (% positive control)** |
|---|---|---|---|---|---|---|
| | Medium | 0,059 | 0,053 | 0,061 | 0,058 | 0,578 |
| **1** | **EN22 10 µM** | **10,000** | **10,000** | **10,000** | **10,000** | **100,000** |
| 4 | AH4 25 µM | 2,340 | 2,250 | 1,863 | 2,151 | 21,507 |
| 4 | AH4 50 µM | 10,000 | 10,000 | 10,000 | 10,000 | 100,000 |
| 4 | AH4 75 µM | 9,988 | 10,000 | 10,000 | 9,996 | 99,960 |
| 11 | AH1 25 µM | 1,140 | 1,156 | 1,234 | 1,177 | 11,766 |
| 11 | AH1 50 µM | 1,533 | 1,609 | 1,592 | 1,578 | 15,776 |
| 11 | AH1 75 µM | 1,619 | 1,382 | 1,598 | 1,533 | 15,328 |
| 12 | AH3 25 µM | 0,540 | 0,630 | 0,593 | 0,588 | 5,875 |
| 12 | AH3 50 µM | 1,267 | 1,615 | 1,675 | 1,519 | 15,193 |
| 12 | AH3 75 µM | 2,288 | 2,351 | 2,032 | 2,224 | 22,240 |
| 29 | AH2 25 µM | 0,197 | 0,186 | 0,200 | 0,194 | 1,942 |
| 29 | AH2 25 µM | 0,280 | 0,232 | 0,243 | 0,251 | 2,514 |
| 29 | AH2 75 µM | 0,176 | 0,193 | 0,193 | 0,187 | 1,872 |

### 1.6. JB010, JB011, JB014, JB002, JB004

| **No.** | **Compound** | **LDH release (% of max.)** | | | **Average LDH release (% of max.)** | **Average (% positive control)** |
|---|---|---|---|---|---|---|
| | Medium | 10,425 | 9,244 | 8,425 | 9,365 | 17,580 |
| **1** | **EN22 20 µM** | **49,359** | **55,225** | **55,225** | **53,269** | **100,000** |
| 5 | JB010 20 µM | 24,368 | 23,784 | 23,266 | 23,806 | 44,689 |
| 5 | JB010 40 µM | 31,421 | 32,791 | 35,906 | 33,373 | 62,649 |
| 5 | JB010 80 µM | 38,624 | 41,749 | 43,361 | 41,245 | 77,427 |
| 7 | JB011 20 µM | 11,537 | 12,278 | 11,156 | 11,657 | 21,883 |
| 7 | JB011 40 µM | 12,611 | 13,286 | 13,733 | 13,210 | 24,799 |
| 7 | JB011 80 µM | 16,367 | 16,371 | 17,276 | 16,671 | 31,296 |
| 10 | JB014 20 µM | 9,465 | 7,643 | 8,614 | 8,574 | 16,095 |
| 10 | JB014 40 µM | 9,739 | 9,811 | 9,418 | 9,656 | 18,126 |
| 10 | JB014 80 µM | 25,331 | 33,166 | 30,920 | 29,806 | 55,953 |
| 13 | JB002 20 µM | 7,716 | 6,991 | 7,485 | 7,397 | 13,887 |
| 13 | JB002 40 µM | 9,376 | 9,412 | 9,594 | 9,461 | 17,760 |
| 13 | JB002 80 µM | 13,963 | 16,997 | 17,117 | 16,026 | 30,084 |
| 14 | JB004 20 µM | 30,957 | 16,589 | 15,720 | 21,089 | 39,589 |
| 14 | JB004 40 µM | 25,621 | 30,949 | 26,130 | 27,567 | 51,749 |
| 14 | JB004 80 µM | 34,302 | 36,552 | 38,206 | 36,353 | 68,244 |

| **No.** | **Compound** | **IL-1β (ng/ml)** | | | **Average IL-1β (ng/ml)** | **Average (% positive control)** |
|---|---|---|---|---|---|---|
| | Medium | 0,000 | 0,0157 | 0,0316 | 0,024 | 0,806 |
| **1** | **EN22** | **1,632** | **2,095** | **2,146** | **1,957** | **100,000** |
| 5 | JB010 20 µM | 2,200 | 3,078 | 1,934 | 2,404 | 122,814 |
| 5 | JB010 40 µM | 2,727 | 2,742 | 2,498 | 2,655 | 135,661 |
| 5 | JB010 80 µM | 2,757 | 3,613 | 5,527 | 3,966 | 202,590 |
| 7 | JB011 20 µM | 0,562 | 0,612 | 0,553 | 0,576 | 29,413 |
| 7 | JB011 40 µM | 0,538 | 0,564 | 0,711 | 0,604 | 30,877 |
| 7 | JB011 80 µM | 0,912 | 0,737 | 0,607 | 0,752 | 38,413 |
| 10 | JB014 20 µM | 0,028 | 0,035 | 0,029 | 0,031 | 1,565 |
| 10 | JB014 40 µM | 0,240 | 0,245 | 0,1944 | 0,227 | 11,579 |
| 10 | JB014 80 µM | 1,080 | 1,344 | 1,097 | 1,174 | 59,959 |
| 13 | JB002 20 µM | 0,038 | 0,050 | 0,047 | 0,045 | 2,292 |
| 13 | JB002 40 µM | 0,190 | 0,197 | 0,19017 | 0,192 | 9,827 |
| 13 | JB002 80 µM | 0,464 | 0,505 | 0,540 | 0,503 | 25,697 |
| 14 | JB004 20 µM | 0,966 | 0,765 | 1,182 | 0,971 | 49,601 |
| 14 | JB004 40 µM | 1,350 | 1,479 | 1,691 | 1,507 | 76,971 |
| 14 | JB004 80 µM | 1,684 | 1,413 | 1,876 | 1,657 | 84,675 |

### 1.7. EN11-16, EN11-13, EN11-07, EN11-02, EN11-11

| **No.** | **Compound** | **LDH release (% of max.)** | | | **Average LDH release (% of max.)** | **Average (% positive control)** |
|---|---|---|---|---|---|---|
| | Medium | 9,336 | 12,148 | 11,930 | 11,138 | 21,906 |
| 1 | EN22 25 µM | 51,852 | 49,199 | 51,489 | 50,847 | 100,000 |
| 15 | EN11-16 25 µM | 7,869 | 8,212 | 7,138 | 7,740 | 15,222 |
| 15 | EN11-16 50 µM | 8,330 | 8,767 | 7,683 | 8,260 | 16,245 |
| 15 | EN11-16 100 µM | 14,248 | 14,125 | 9,163 | 12,512 | 24,607 |
| 16 | EN11-13 25 µM | 7,549 | 7,580 | 7,769 | 7,633 | 15,011 |
| 16 | EN11-13 50 µM | 10,381 | 9,817 | 10,629 | 10,276 | 20,209 |
| 16 | EN11-13 100 µM | 19,337 | 15,515 | 13,905 | 16,252 | 31,963 |
| 30 | EN11-07 25 µM | 13,382 | 14,369 | 13,866 | 13,873 | 27,283 |
| 30 | EN11-07 50 µM | 14,413 | 15,441 | 13,545 | 14,466 | 28,451 |
| 30 | EN11-07 100 µM | 11,406 | 14,721 | 14,340 | 13,489 | 26,529 |
| 31 | EN11-02 25 µM | 10,764 | 11,389 | 9,725 | 10,626 | 20,897 |
| 31 | EN11-02 50 µM | 10,395 | 10,847 | 10,953 | 10,731 | 21,105 |
| 31 | EN11-02 100 µM | 10,119 | 10,610 | 9,486 | 10,072 | 19,808 |
| 32 | EN11-11 25 µM | 6,552 | 5,506 | 1,276 | 4,445 | 8,741 |
| 32 | EN11-11 50 µM | 6,570 | 5,610 | 0,416 | 4,199 | 8,257 |
| 32 | EN11-11 100 µM | 5,907 | 6,309 | 4,141 | 5,452 | 10,723 |

| **No.** | **Compound** | **IL-1β (ng/ml)** | | | **Average IL-1β (ng/ml)** | **Average (% positive control)** |
|---|---|---|---|---|---|---|
| | Medium | 0,013 | 0,000 | 0,011 | 0,008 | 0,536 |
| 1 | EN22 25 µM | 1,387 | 1,391 | 1,646 | 1,474 | 100,000 |
| 15 | EN11-16 25 µM | 0,010 | 0,014 | 0,000 | 0,008 | 0,555 |
| 15 | EN11-16 50 µM | 0,068 | 0,054 | 0,058 | 0,060 | 4,074 |
| 15 | EN11-16 100 µM | 0,334 | 0,324 | 0,315 | 0,325 | 22,016 |
| 16 | EN11-13 25 µM | 0,000 | 0,011 | 0,023 | 0,011 | 0,764 |
| 16 | EN11-13 50 µM | 0,067 | 0,067 | 0,067 | 0,067 | 4,560 |
| 16 | EN11-13 100 µM | 0,243 | 0,331 | 0,316 | 0,297 | 20,130 |
| 30 | EN11-07 25 µM | 0,000 | 0,000 | 0,000 | 0,000 | 0,000 |
| 30 | EN11-07 50 µM | 0,000 | 0,000 | 0,000 | 0,000 | 0,000 |
| 30 | EN11-07 100 µM | 0,059 | 0,052 | 0,049 | 0,053 | 3,621 |
| 31 | EN11-02 25 µM | 0,015 | | 0,015 | 0,015 | 1,019 |
| 31 | EN11-02 50 µM | 0,040 | 0,050 | 0,036 | 0,042 | 2,832 |
| 31 | EN11-02 100 µM | 0,053 | 0,052 | 0,046 | 0,050 | 3,424 |
| 32 | EN11-11 25 µM | 0,000 | 0,000 | 0,000 | 0,000 | 0,000 |
| 32 | EN11-11 50 µM | 0,021 | 0,022 | 0,000 | 0,014 | 0,975 |
| 32 | EN11-11 100 µM | 0,012 | 0,021 | 0,000 | 0,011 | 0,761 |

### 1.8. BMDC isolation and differentiation

Mouse bone marrow-derived dendritic cells (BMDCs) were isolated from femurs and tibias previously described (Gross, Olaf, 2012, "Measuring the inflammasome", Methods in molecular biology, Clifton, N.J., 844, pp. 199-222.). In brief, tibias and femurs were carefully removed and put to sterile 70% ethanol for 1 minute. Using a 22-G needle attached to a 20ml-syringe filled with sterile flushing medium, the bone marrow was pressed through the mesh of a 100 µM cell strainer. The cells were spun down by centrifugation at 400x g, 4°C for 5 minutes and red blood cells lysed by resuspension and incubation for 5 minutes at room temperature in RBC lysis buffer. 9 ml of flushing medium were added and cells were again spun down by centrifugation at 400x g, 4°C for 5 minutes. Cells were then resuspended in BMDC medium (RPMI, 10% fetal bovine serum, 1% penicillin-streptomycin100 U/ml, 20 ng/ml recombinant murine GM-CSF), counted and plated at a density of 0.5× 10⁶ cells ml⁻¹ in10 ml medium and incubated at 37°C, 5% CO₂ in a humidified incubator. After 2 days, 10 ml of fresh medium were added to the cells and after 4 days, the medium was fully exchanged to 20 ml of fresh medium. The cells were allowed to differentiate for 6 to 8 days before usage.

### 1.9. Harvesting of BMDCs and BMDMs

BMDCs after 6 to 8 days of differentiation were harvested by transferring floating cells to 50 ml Falcon tubes and dislodging adherent cells with HBSS containing 5 mM EDTA.

### 1.10. High-throughput screening for inflammasome activators

To screen for new small molecule inflammasome activators, a 2-step semi-automated screening strategy was devised and performed. Small molecules from a Novartis public compound library were tested and access to high-throughput screening equipment was provided via the Novartis Facilitated Access to Screening Technologies (FAST) Lab.

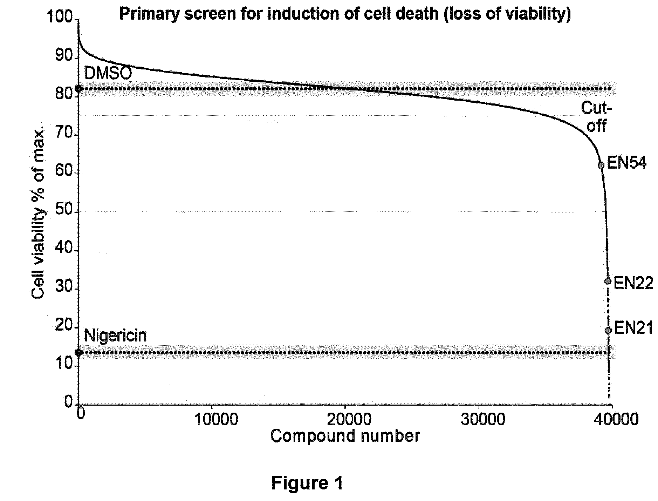

For primary screening (see **Figure 1**), 200 nl of the small molecule stock solutions (10 mM or 5 mM in DMSO) were pre-dispensed to white 384 well flat-bottom microplates with an Echo^{®} 555 Liquid Handler (Labcyte Inc.). The plates were sealed with a PlateLoc Thermal Microplate Sealer (Agilent Technologies Inc.) and stored at 4- 8°C until further use. Plates were brought to ambient temperature before usage.

BMDCs from wild-type mice after 7 days of differentiation were harvested as described above. Depending on the volume of cell suspension needed for the experiment, the cells were resuspended in an appropriate volume of BMDC and counted with a Countess^{™} II FL Automated Cell Counter (Thermo Fisher Scientific). The concentration was adjusted to 0.25x 10⁶ cells ml⁻¹ and the cells were primed with 50 ng ml⁻¹ *E. coli* K12 ultra-pure LPS. Immediately after addition of LPS, 40 µl per well cell suspension were plated to the microplates already containing the small molecules to be tested using a Multidrop^{™} Combi Reagent Dispenser (Thermo Fisher Scientific) resulting in a final small molecule concentration of 50 µM or 25 µM. The cell suspension was mixed on a regular basis to ensure a consistent plating density. 8 wells per plate were filled with nigericin at a final concentration of 5 µM, DMSO at a final concentration of 0.5% or imiquimod and CL097 as references at a final concentration of 100 µM. It was then incubated at 37°C, 5% CO₂ in a humidified incubator for about 20 hours. After the incubation, the plates were removed from the incubator and 10 µl per well CellTiter-Glo^{®} Reagent (Promega Corporation) were added with the Multidrop^{™} Combi Reagent Dispenser. After at least 15 minutes of incubation at room temperature, the luminescence was determined with an EnVision 2105 Multimode Plate Reader (PerkinElmer Inc.) equipped with an EnVision stacker for semi-automated plate loading. Luminescence signals were normalized to nigericin as a positive control for inflammasome activation and DMSO as a negative control. Small molecules were considered active and chosen for subsequent counter screening when they induced a reduction in the luminescence signal that was at least 50% (25% in some cases) of the reduction induced by 5 µM nigericin (see **Figure 1**).

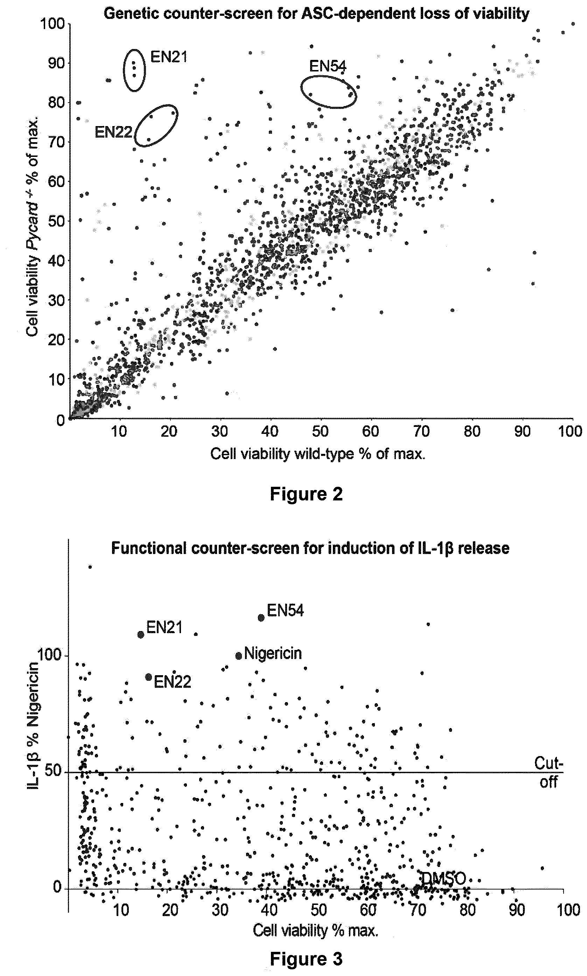

For counter screening (see **Figures 2 and 3**), two independent strategies were pursued. To test for inflammasome dependency of cell viability reduction, BMDCs from either wild-type or *Pycard*^{*-*/*-*} mice (Mariathasan, S., Newton, K., Monack, D. et al. Differential activation of the inflammasome by caspase-1 adaptors ASC and Ipaf. Nature 430, 213-218 (2004). https://doi.org/10.1038/nature02664) were used and the same steps were performed as described above for primary screening, whereas the incubation time was reduced to about 16 hours. Three independent replicates of each plate were prepared and measured and the small molecules were either tested at a final concentration of 50 µM or at a dose response of 4 or 8 concentrations per compound going down with a dilution factor of 1/ 3.16 starting from 50 µM. Small molecules, that induced a reduction in the luminescence signal in the wild-type but not in the *Pycard*^{*-*/*-*} BMDCs were considered as hits for further evaluation (**see** **Figures 2** **and** **4A-****C**).

To functionally counter-screen (see **Figure 3**) for induction of IL-1β release, BMDCs from wild-type mice were harvested, counted and the concentration adjusted to 0.25× 10⁶ cells ml⁻¹. The cells were primed with 50 ng ml⁻¹ *E. coli* K12 ultra-pure LPS directly before plating 40 µl per well cell suspension to white 384 well flat-bottom microplates and it was incubated for 3 hours at 37°C, 5% CO₂ in a humidified incubator. 200 nl or 400 nl of the small molecule stock solutions from the libraries that had before been transferred to mother plates for the Echo^{®} 555 Liquid Handler were then added to the microplates with the primed BMDCs to a final compound concentration of 50 µM. After 20 hours of incubation at 37°C, 5% CO₂, IL-β release from the cells was then determined by using HTRF^{®} technology developed by Cisbio from cell-free supernatants. HTRF (Homogeneous Time Resolved Fluorescence) is the most frequently used generic assay technology to measure analytes in a homogenous format, which is the ideal platform used for drug target studies in high-throughput screening. This technology combines fluorescence resonance energy transfer technology (FRET) with time-resolved measurement. The measurements were carried out as described in the manufacturer's manual.

### 1.11. Inflammasome stimulation

For canonical inflammasome activation BMDCs after 7- 9 days of differentiation were harvested and counted with a Neubauer Chamber (Hecht Assistant) and the concentration was set to 1 Mio cells ml⁻¹. 100 µl cell suspension per well were seeded in 96-well plates. The cells were primed with 50 ng ml⁻¹ *E. coli* K12 ultra-pure LPS for 3 hours and subsequently treated with canonical inflammasome activators or experimental compounds for 3 hours if not indicated differently (incubation time Figure 4D, 5, 7, 8, 11, 13-18: 3 h, Figure 12: 4h). Concentrations of the stimuli were as follows if not indicated differently: 5 µM nigericin, 100 µM imiquimod (also known as R837), 100 µM CL097, 100 µM R848, 1 µg ml⁻¹ poly(dA:dT), 300 µg ml⁻¹ MSU. If an inhibitor was used before stimuli treatment, the inhibitor was added after 2.5 hours of priming and it was incubated for 30 minutes before inflammasome activation. Inhibitors were used as follows: 3 µM MCC950, 45 mM KCI, 30 µM ebselen (2-Phenyl-1,2-benzoselenazol-3-one), 5 µM cytochalasin D (see **Figures 8****,** **11****,** **12** **and** **17**).

All inflammasome stimulations were performed in three technical replicates for each condition. Lytic cell death was determined via LDH release (see **Figures 8****,** **13-16** **and** **18**), cytokines quantified in cell-free supernatants via ELISA (see **Figures 4D****,** **7- 9****,** **11- 19**) and cell lysates and supernatants analyzed via immunoblotting (see **Figures 5A and 5B**) as previously described in detail (Gross, Olaf, 2012 "Measuring the inflammasome", Methods in molecular biology, Clifton, N.J., 844, pp. 199-222. Schneider, Katharina S.; Thomas, Christina J.; Groß, Olaf, 2013, "Inflammasome activation and inhibition in primary murine bone marrow-derived cells, and assays for IL-1α, IL-1β, and caspase-1", Methods in molecular biology, Clifton, N.J., 1040, pp. 117-135).

### 1.12. Toll-like receptor stimulation

To test for secretion of inflammasome independent cytokines (TNF and IL-6), BMDCs after 7-9 days of stimulation were left unprimed and then stimulated as described in Example 1.4. Cell-free supernatants were analyzed via ELISA (see **Figure 9** **and** **18**).

### 1.13. Determination of cell viability

### 1.13.1 LDH release

To assess lytic cell death, release of the stable cytosolic enzyme lactate dehydrogenase (LDH) was determined using the CytoTox 96^{®} Non-Radioactive Cytotoxicity kit (Promega Corporation) according to the manufacturer's instructions. The volumes of cell-free supernatants and reagent used were reduced to 35µl. Background signal that was determined from medium without cells was subtracted from all values and all results were depicted as % of cells that had been lysed with 0.8% Triton^{®} X-100 45 minutes before performing the assay. Absorbance at 490 nm was determined using a Tecan Infinite M200 plate reader (Tecan Life Sciences) (see **Figures 7, 8**,**13-16** **and** **18**).

### 1.13.2 Cellular ATP

Cellular ATP levels as were determined with the CellTiter-Glo^{®} Luminescent Cell Viability kit (Promega Corporation) according to the protocol provided by the manufacturer except that one fourth of the recommended amount of reagent was used. Luminescence intensities were normalized as % of the highest signals obtained, usually in the untreated control conditions.

### 1.14. Cytokine quantification

### 1.14.1. ELISA

Cytokines were quantified in cell-free supernatants by using enzyme-linked immunosorbent assay (ELISA) kits for murine IL-1β, TNF and IL-6 (Thermo Fisher Scientific). The volumes of cell-free supernatants, antibodies and reagents used were reduced to 50 µl. Each condition was measured as 3 independent technical replicates and data was depicted as mean ± SD. Absorbance at 450 nm and 570 nm was determined using a Tecan Infinite M200 plate reader (Tecan Life Sciences). Absolute values were interpolated from a standard curve that was generated with the Magellan^{™} Data Analysis Software. Depending on the expected cytokine quantity cell-free supernatants were diluted so that the detection values were within the linear range of the standard curve (see **Figures 4D****,** **7-9****, and** **11-19**).

### 1.15. Immunoblotting

Cell lysates for immunoblotting were generated by lysing the cells with 1x reducing SDS-PAGE loading buffer followed by 1 freeze-thaw cycle. Cell-free supernatants were diluted with 3x reducing SDS-PAGE loading buffer. Samples from 3 independent technical replicates were pooled and proteins were separated via SDS-PAGE (sodium dodecyl sulfate polyacrylamide gel electrophoresis) using 12% polyacrylamide gels and applying 80-120 V for 1-2.5 hours. Afterwards, proteins were transferred to nitrocellulose membranes by application of 100 V for 100 minutes. Membranes were rinsed with PBS-tween and success of protein transfer checked via Ponceau S staining. The membranes were then washed with PBS-tween again until Ponceau was completely removed and blocked with 2% skim milk powder in PBS-tween for 1 hour at room temperature. Membranes were rinsed with PBS-tween again and primary antibodies applied overnight at 4°C (diluted in PBS-tween, 2% skim milk powder, 0.05% sodium azide). Before adding HRP-conjugated secondary antibodies (diluted 1:5,000 in PBS-tween, 2% skim milk powder) for 1 hour at room temperature, membranes were washed with PBS-tween for 2 hours, replacing with fresh PBS-tween every 15 minutes. Enhanced chemiluminescence substrate was applied before imaging with a cooled charge-coupled device. Depending on the signal intensity (16 bit, 64K grayscale) it was exposed for 2-15 minutes.

### 1.16. Immunofluorescence imaging for ASC specks

BMDCs from either wild-type or ASC-citrine mice expressing a ASC fluorescent protein were seeded in chambered coverslips for cell imaging (µ-Slide, Ibidi) and primed with 50 ng ml⁻¹ LPS for 3 hours. The cells were then incubated with the inflammasome activator of interest at the respective conditions. After the treatment, the cells were washed with PBS, fixed with 4% paraformaldehyde for 10 minutes and permeabilized with 0.1% (v/v) TritonTM X-100) for 5 minutes. The cells were then stained with polyclonal anti-ASC antibody derived from rabbit (Adipogen) and FITC anti-mouse CD45 antibody (Biolegend) overnight at 4°C. The next day, the cells were washed with blocking buffer and stained with a fluorescence labelled secondary antibody specific for rabbit for 30 minutes at room temperature, washed and subsequently resuspended in Vectashield^{®} Antifade Mounting Medium with DAPI (H-1200) (Vector Laboratories) diluted 1:10 in blocking buffer for at least 10 minutes. Confocal microscopy of the immunostained cells was then performed. Immunostaining for ASC is dispensable if BMDCs from ASC-citrine mice are used.

### 1.17. Metabolic Analysis (extracellular flux analysis)

Oxygen consumption rate (OCR) and extracellular acidification rate (ECAR) were measured by extracellular flux analysis with a Seahorse XFe96 Analyzer. One day before the experiment, BMDCs were plated to XFe cell culture microplates at a density of 80,000 cells per well and a XFe sensor cartridge was rehydrated using XF Calibrant and put to 37°C in a CO₂-free incubator. On the day of the experiment, the cells were first primed with 50 ng ml⁻¹ *E. coli* K12 ultra-pure LPS for 2-3 hours. The medium was then exchanged to bicarbonate- and phenol red-free seahorse base medium supplemented with 2 mM L-glutamine, 10 mM glucose and 5 mM HEPES, pH 7.4 and the cells were incubated at 37°C in a CO₂-free incubator for 1 hour. In the meantime, 10x dilutions of compounds and inhibitors to be tested were prepared in seahorse base medium and loaded to the injection ports of the rehydrated XFe sensor cartridge. Small molecules were used as indicated and respiratory chain inhibitors (piericidin, antimycin A) and mitochondrial uncouplers (FCCP) were used as follows after an initial titration: 10 µM piericidin, 2 µM antimycin A, 1 µM FCCP.

### 1.18. Materials

- 1x PBS (GibcoTM, Thermo Fisher Scientific)
- 3x reducing SDS-PAGE loading buffer: 37.5ml 1M Tris-HCl pH 7.0 (=187.5 mM), 12g SDS (= 6% w/v), 60mg phenol red (= 0.03% w/v) (Sigma Aldrich), 69g glycerol (=30% w/v); adjust pH with 0.1N HCl to pH 6.8, 66,7 mM DTT (Carl Roth)
- 4% Paraformaldehyde solution (Sigma Aldrich)
- Blocking buffer: PBS (GibcoTM, Thermo Fisher Scientific), 5% FCS (GibcoTM, Thermo Fisher Scientific), 0.1% TritonTM X-100 (Sigma Aldrich)
- BMDC medium: RPMI (GibcoTM, Thermo Fisher Scientific), 10% FCS (GibcoTM, Thermo Fisher Scientific), 100 units ml-1 penicillin/ streptomycin (GibcoTM, Thermo Fisher Scientific), 50 ng ml-1 recombinant murine GM-CSF (Immunotools)
- CellTiter-Glo^{®} Luminescent Cell Viability Assay (Promega Corporation)
- CytoTox 96^{®} Non-Radioactive Cytotoxicity Assay (Promega Corporation)
- *E. coli* K12 ultra-pure LPS (Invivogen)
- IL-1β, TNF and IL-6 Mouse Uncoated ELISA Kit (Thermo Fisher Scientific)
- PBS-tween: 4g KH₂PO₄ (Potassium phosphate monobasic, Sigma Aldrich), 4g KCI (Potassium chloride, Sigma Aldrich), 160g NaCl (>99.8 %, Carl Roth), 28.4g Na₂HPO₄ × 2 H₂O pH 7.2 - 7.4 (di-Natriumhydrogenphosphat Dihydrat, ≥99.5 %, Carl Roth), 20 ml Tween-20 (Sigma Aldrich) in 20 I H2O
- Primary antibodies for immunoblotting and immunostaining:
   Anti-Asc, pAb (AL177) (Adipogen)
   Anti-Caspase-1 (p20) (mouse), mAb (Casper-1) (Adipogen)
   Mouse IL-1β/IL-1F2 Antibody (R&D)
- XFe flux analysis consumables and reagents (Agilent Technologies)
- Nigericin sodium salt (Sigma Aldrich)
- Imiquimod (R837) (Invivogen)
- CL097 (Invivogen)
- R848 (Invivogen)

### 1.19. Devices

- Countess^{™} II FL Automated Cell Counter (Thermo Fisher Scientific)
- Echo^{®} 555 Liquid Handler (Labcyte Inc.)
- EnVision 2105 Multimode Plate Reader (PerkinElmer Inc.)
- Humidified CO2 incubator (HeracellTM 150, Thermo Fisher Scientific)
- Intas Chemostar ECL Imager (Intas Science Imaging)
- Leica SP8 confocal microscope equipped with a 63×/1.40 and 40x/1.25 oil objectives (Leica Microsystems)
- Multidrop^{™} Combi Reagent Dispenser (Thermo Fisher Scientific)
- Neubauer chamber (Hecht Assistent)
- Seahorse XFe96 Flux Analyzer (Agilent Technologies)
- Tecan Infinite M200 plate reader (Tecan Life Sciences)

### 2. Synthesis

### 2.1. General Synthesis Procedure A

An Aniline derivative (1 eq.) was dissolved in an aprotic solvent such as dry DCM or dry THF. Isocyanatobenzene, thioisocyanatobenzene, 1-isocyanato-4-methoxybenzene or 4-isocyanato-benzonitrile (1 eq.) dissolved in an aprotic solvent such as dry DCM or dry THF was added dropwise to the reaction with a syringe over 5 min. The reaction was stirred at 25 °C for 4h or until completion of the reaction and stopped by evaporation of the solvents. The crude solid was purified by filtration with cyclohexane/DCM and/or by flash column chromatography with a cyclohexane/ethyl acetate or DCM/methanol of varying eluent composition.

### 2.2. General Synthesis Procedure B

Triphosgen (0.4 eq.) was dissolved in dry DCM or dry THF and a solution of a primary or secondary aromatic amine (1 eq.) and triethylamine (3 eq.) in dry DCM or dry THF was added dropwise with a syringe over 10 min. The reaction was stirred at 25 °C for 2h. The solvent was removed by a rotary evaporator under the hood. The crude was filtrated with cyclohexane and the filtrate collected and evaporated. The resulting solid containing the isocyanate was used without further purification in following reactions. No NMR spectra were recorded.

### 2.3. General Synthesis Procedure C

An isocyanate (1.2 eq.) or carbamic chloride (1.2 eq.) synthesized via general synthesis procedure B was dissolved in an aprotic solvent such as dry DCM or dry THF and added dropwise with a syringe over 5 min to a solution of an aniline (1 eq.) or primary amine (1 eq.) dissolved in an aprotic solvent such as dry DCM or dry THF and stirred at 25 °C for 4h or until completion of the reaction. The crude solid was purified by filtration with cyclohexane/DCM and/or by flash column chromatography on SiO2 gel with a cyclohexane/ethyl acetate or DCM/MeOH of varying eluent composition or HPLC.

### 2.4. Preparation of compound EN21

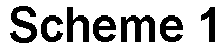

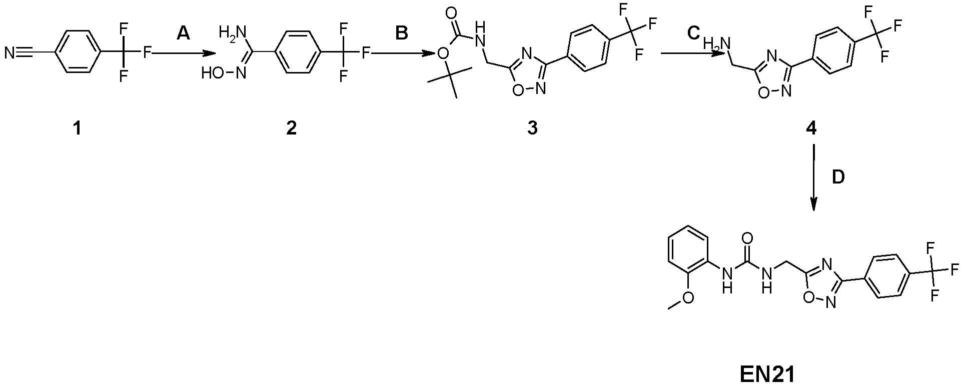

### Step A:

To a stirred suspension of 4-(trifluoromethyl)benzonitrile **(1)** (15 g, 87.7 mmol, 1 eq.) and; sodium hydrogencarbonate (8.8 g, 105.2 mmol, 1.2 eq.) in methanol (150 ml), hydroxylamine hydrochloride (7.3 g, 105.2 mmol, 1.2 eq.) was added portion wise and stirred overnight. After completion of the reaction (TLC data), the mixture was filtrated and the filtrate was carefully evaporated in vacuo at 35°C. The resulting residue was suspended in water (200 mL), stirred for 15 min and then filtrated to afford N'-hydroxy-4-(trifluoromethyl)benzimidamide (2) (16.2 g, 91 % yield) as white solid.

### Step B:

To a stirred solution of 2-((*tert*-butoxycarbonyl)amino)acetic acid (15.3 g, 87.3 mmol, 1.1 eq.) in dry 1,4-dioxane (180 mL), 1,1'-carbonyldiimidazole (CDI) (12.9 g, 79.4 mmol, 1 eq.) was added by portion at 60°C and the resulting mixture was stirred for 30 min at 60°C. N'-hydroxy-4- (trifluoromethyl)benzimidamide (2) (16.2 g, 79.4 mmol, 1 eq.) was added at once and the resulting mixture was stirred at 60°C until full consumption of 4-(trifluoromethyl)benzimidamide **(2)** was being achieved (TLC data, approximately 30 min for this scale). Then the reaction mixture was heated at 90°C for 12 h, cooled to r.t. and evaporated in vacuo. The resulting residue was diluted with 30 % aq. solution of potassium carbonate (200 mL) and ethyl acetate (200 mL), the water layer was separated and extracted with ethyl acetate (2×100 mL). The combined organic layers were dried over anhydrous sodium sulfate and evaporated in vacuo. The crude material was purified by column chromatography (chloroform : ethyl acetate, v/v= 10:1, rf = 0.8) to afford *tert*-butyl((3-(4-(trifluoromethyl)phenyl)-1,2,4-oxadiazol-5-yl)methyl)carbamae **(3)** (16.1 g, 59 % yield) as white solid.

### Step C, D :

To a stirred trifluoroacetic acid (10 mL), *tert*-butyl ((3-(4-(trifluoromethyl)phenyl)-1,2,4-oxadiazol-5-yl)methyl)carbamate **(3)** (2 g, 5.8 mmol) was added by portion at r.t. and the resulting mixture was stirred for 30 min. The solvent was removed in vacuo and the residue was diluted with dichloromethane and threated with excess of DIPEA (3.1 mL, 17.5 mmol, 3 eq.). The resulting mixture was stirred at r.t. for 5 min and then, 1-isocyanato-2-methoxybenzene (0.9 g, 5.8 mmol, 1 eq.) was added at once. The resulting mixture was stirred for additional 30 min. The resulting precipitate was filtrated and washed with dichloromethane (2×10 mL) to afford 1-(2- methoxyphenyl)-3-((3-(4-(trifluoromethyl)phenyl)-1,2,4-oxadiazol-5-yl)methyl)urea **(EN21,** also called **EN02-01)** (1.1 g, 48 % summary yield) as white solid.

**EN21:** 1H NMR (400 MHz, dmso) δ 3.86 (s, 3H), 4.72 (d, 2H), 6.83 (t, 1H), 6.91 (t, 1H), 6.99 (d, 1H), 7.67 (t, 1H), 7.94 (d, 2H), 8.02 (d, 1H), 8.22 (d, 2H), 8.28 (s, 1H).

### 2.5. - Preparation of compound EN22

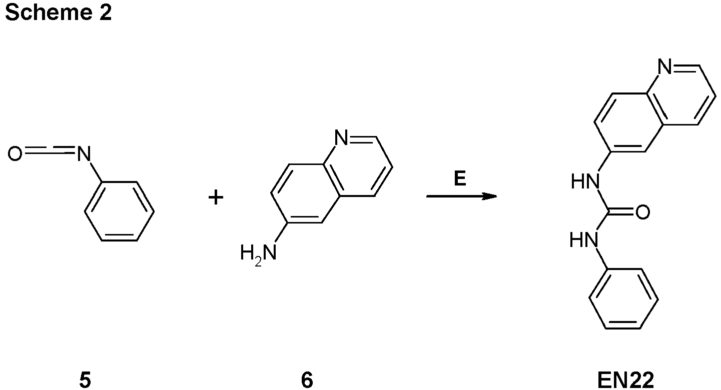

### Step E:

To a solution of amine **6** (3.29 g, 22.8 mmol) in dry dichloromethane (50 mL) was added isocyanate **5** (2.72 g, 22.8 mmol). The reaction mixture was stirred for 2 h at r.t. and the precipitated solid was collected, washed with dichloromethane and hexane, and dried in vacuum to afford 5.15 g of compound EN22 (also called EN02-02) (85 % yield).

**EN22:** 1H NMR (400 MHz, dmso+ccl4) δ 6.99 (t, 1H), 7.30 (t, 2H), 7.45 (dd, 3.9 Hz, 1H), 7.50 (d, 2H), 7.70 (d, 1H), 7.94 (d, 1H), 8.18 (s, 1H), 8.25 (d, 1H), 8.74 (d, 1H), 8.79 (s, 1H), 9.03 (s, 1H).

### 2.6. Preparation of compound EN54

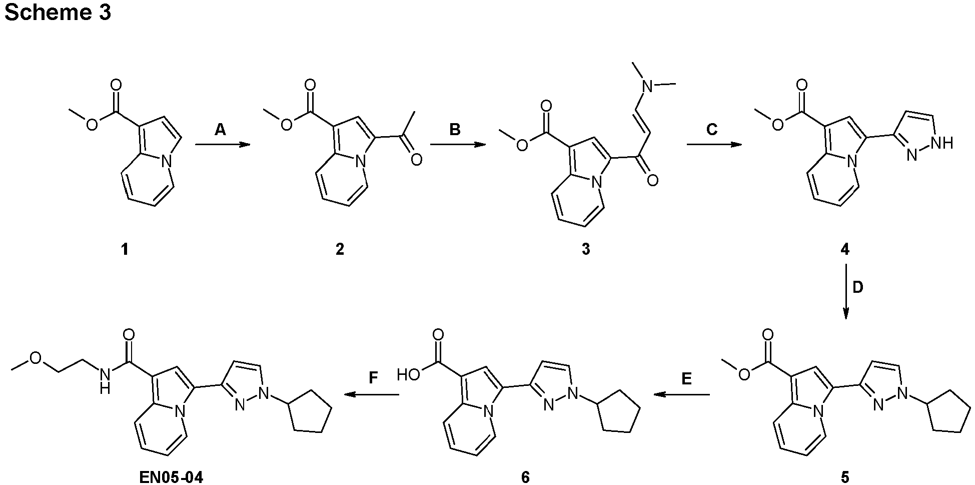

### Step A:

To a stirred solution of acetyl chloride (4.7 g, 60 mmol) in dichloromethane (60 ml) aluminum chloride (8.0 g, 60 mmol) was added. The mixture was cooled to 0 °C and methyl 1-indolizinecarboxylate **(1)** (7.0 g, 40 mmol) in dichloromethane (40 ml) was added dropwise. The resulting mixture was stirred overnight at room temperature, poured in ice water and diluted with HCl. The organic phase was separated, the water phase was extracted with dichloromethane twice, and the combined organic phase was washed with saturated aqueous sodium bicarbonate and dried over potassium sulphate. The solvent was removed under reduced pressure to give methyl 3-acetyl-1-indolizinecarboxylate **(2)** without further purification as yellow solid. Yield 7.2 g, 84%.

### Step B:

A mixture of methyl 3-acetyl-1-indolizinecarboxylate **(2)** (7.2 g, 33.6 mmol) and DMF-DMA (8.0 g, 67.2 mmol) in dimethylformamide (10 ml) was heated to 110 °C overnight. The solution was cooled to room temperature, and the formed solid product was collected by filtration and washed with hexane and dried to obtain compound **3.** Yield 7.0 g, 78%.

### Step C:

A mixture of methyl 3-[3-(dimethylamino)acryloyl]indolizine-1-carboxylate **(3)** (7.0 g, 26 mmol) and hydrazine hydrate (6.5 g, 130 mmol) in methanol (50 ml) was refluxed for 3 h. The solution was cooled to room temperature and diluted with water (100 ml). The solid product was collected by filtration and dried to obtain compound **4.** Yield 4.2 g, 67%.

### Step D:

To a stirred solution of methyl 3-(1H-pyrazol-3-yl)indolizine-1-carboxylate **(4)** (4.2 g, 17.4 mmol) in DMF (40 ml) sodium hydride (0.85 g, 20.9 mmol as suspension in mineral oil) was added. The mixture was stirred for 30 min., cooled to 0 °C and bromocyclopenthane (3.9 g, 26.1 mmol) in DMF (10 ml) was added dropwise. The resulting mixture was stirred overnight at room temperature and poured in ice water. The solid product was collected by filtration and dried to obtain compound 5. Yield 4.8 g, 89%.

### Step E:

To a stirred suspension of ethyl 3-(1-cyclopentyl-1H-pyrazol-3-yl)indolizine-1-carboxylate **(5)** (4.8 g, 15.5 mmol) in methanol (50 ml) potassium hydroxide (8.7 g, 155 mmol) in water (40 ml) was added and the resulting mixture was refluxed for 24 h. The clear solution was cooled to room temperature, diluted with water (50 ml) and diluted HCl was added. The solid product was collected by filtration and dried to obtain compound 6. Yield 4.2 g, 67%.

### Step F:

To a stirred suspension of 3-(1-cyclopentyl-1H-pyrazol-3-yl)indolizine-1-carboxylic acid **(6)** (3.2 g, 10.8 mmol) in dichloromethane (40 ml) thionyl chloride (2.6 g, 21.7 mmol) was added and the resulting mixture was stirred at room temperature to obtain of the clear dark green solution (6-8 h). The solvent and the excess of thionyl chloride were removed under reduced pressure, and the residue was dissolved in dichloromethane (40 ml) and added dropwise to a stirred solution of 2-methoxyethylamine (1.2 g, 16.3 mmol) and DIPEA (2.8 g, 21.7 mmol) in dichloromethane (40 ml) at room temperature. The resulting mixture was stirred overnight, washed with diluted HCl, saturated aqueous sodium bicarbonate and dried over potassium sulphate. The solvent was removed under reduced pressure to give 3.1 g (82%) of the title product. The analytical sample of compound **EN54** (also called **EN05-04)** was obtained by column chromatography on silica gel using ethyl acetate as an eluent.

**EN54:** 1H NMR (400 MHz, dmso) δ 1,68 (m, 2H), 1,83 (m, 2H), 2,02 (m, 2H), 2,16 (m, 2H), 3.28 (s, 3H), 3,46 (m, 4H), 4,8 (m, 1H), 6.57 (d, 1H), 6.92 (t, 1H), 7.07 (t, 1H), 7.73 (s, 1H), 7.90 (d, 1H), 8.01 (t, 1H), 8.33 (d, 1H), 9.40 (d, 1H).

The measurements of the NMR spectra were done with Bruker AVANCE DRX 500 Mhz and Bruker AVANCE III 400 Mhz.

### 2.7. Preparation of compound AH4

1-phenyl-3-(quinolin-6-yl) thiourea. To a stirred solution of quinolin-6-amine (144.18 mg, 1 mmol, 1.0 equiv.) in dichloromethane (5 mL), 1-isothiocyanatobenzene (135.18 mg, 1 mmol, 1.0 equiv.) was added drop wise. The mixture was stirred at room temperature for 5 h and concentrated. The residue was purified on silica gel column chromatography eluting with 1-10 % methanol in dichloromethane to afford the desired product as yellow solid (174.02 mg, 0.623 mmol, 62.3 %).

**AH4:** 1H NMR (400 MHz, DMSO-d6) δ 10.12 (s, 1H), 9.99 (s, 1H), 8.83 (dd, J = 4.2, 1.7 Hz, 1H), 8.31 (dd, J = 8.4, 1.7 Hz, 1H), 8.09 (d, J = 2.4 Hz, 1H), 7.98 (d, J = 9.1 Hz, 1H), 7.86 (dd, J = 9.0, 2.4 Hz, 1H), 7.61 - 7.41 (m, 3H: 2H, CH, benzyl, 1H), 7.41 - 7.27 (m, 2H), 7.20 - 7.11 (m, 1H).

### 2.8. Preparation of compound JB010

1-(4-methoxyphenyl)-3-(quinolin-6-yl)urea was synthesized according to general synthesis procedure A. The crude solid was washed and filtered with DCM/cyclohexane (50/50) to gain a white solid (303.2 mg, Yield: 90.0%); rf = 0.2 (cyclohexane/ethyl acetate 20/80).

**JB010:** 1H NMR (400 MHz, DMSO-d6) δ 8.96 (s, 1H, quinoline-NHurea), 8.74 (dd, J = 4.2, 1.7 Hz, 1H, quinoline H2), 8.62 (s, 1H, methoxyphenyl-NHUrea), 8.25 (dd, J = 8.7, 1.0 Hz, 1H, quinoline H4), 8.17 (d, J = 2.4 Hz, 1H, quinoline H5), 7.94 (d, J = 9.1 Hz, 1H, quinoline H8), 7.70 (dd, J = 9.1, 2.4 Hz, 1H, quinoline H7), 7.46 (dd, J = 8.3, 4.2 Hz, 1H, quinoline H3), 7.43 - 7.38 (m, 2H, methoxyphenyl H2,6), 6.93 - 6.87 (m, 2H, methoxyphenyl H3,5), 3.73 (s, 3H, phenyl-OCH3).

### 2.9. Preparation of compound JB011

1-(4-cyanophenyl)-3-(quinolin-6-yl)urea was synthesized according to general synthesis procedure A. The crude solid was washed and filtered with DCM/cyclohexane (50/50) to gain a white solid (267.8 mg, Yield: 90.2%); rf = 0.1 (cyclohexane/ethyl acetate 20/80).

**JB011:** 1H NMR (400 MHz, DMSO-d6) δ 9.34 (s, 1H, quinoline-NHUrea), 9.24 (s, 1H, cyanophenyl-NHUrea), 8.77 (dd, J = 4.2, 1.7 Hz, 1H, quinoline H2), 8.28 (dd, J = 8.6, 0.9 Hz, 1H, quinoline H4), 8.19 (d, J = 2.3 Hz, 1H, quinoline H5), 7.97 (d, J = 9.0 Hz, 1H, quinoline H8), 7.77 (dt, J = 8.9, 2.1 Hz, 2H, phenyl H2,6), 7.73 (dd, J = 9.1, 2.5 Hz, 1H, quinoline H7), 7.69 (dt, J = 9.0, 2.1 Hz, 2H, phenyl H3,5), 7.48 (dd, J = 8.3, 4.2 Hz, 1H, quinoline H3).

### 2.10. Preparation of compound PM07 and PM16

### Synthesis of ethyl 2-(1-(3-fluoro-2-methyl benzyl) piperidin-3-yl) acetate

The synthesis was performed according to the procedure reported in literature:
D. Monaldi, D. Rotili, J. Lancelot, M. Marek, N. Wössner, A. Lucidi, D. Tomaselli, E. Ramos-Morales, C. Romier, R.J. Pierce, A. Mai, M. Jung; J. Med. Chem. 62 (2019) 8733-8759.

### Synthesis of Tert-butyl (4-(N'-hydroxycarbamimidoyl) phenyl]carbamate

To a suspension of tert-butyl(4-cyanophenyl) carbamate (1.0 equiv.) and hydroxylamine hydrochloride (1.5 equiv.) in EtOH (25 mL), NaHCO3 (1.5 equiv) was added. The mixture was stirred under reflux for 6 h. and eventually diluted with cold water (50 mL). the aqueous phase was extracted with ethyl acetate (3 × 50 mL). the combined organic phases were dried over Na2SO4, filtrated and concentrated. The residue was then purified on silica gel column chromatography eluting with 1-10 % methanol in dichloromethane to afford the desired product as white solid (1.12 g, 4.46 mmol, 97 %).

1H NMR (400 MHz. DMSO-d6): δ 9.48 (s, 1H,), 9.45 (s, 1H), 7.55 (d, J = 8.1 Hz, 2H), 7.43 (d, J = 8.1 Hz, 2H), 5.71 (s, 2H), 1.47 (s, 9H).

### Synthesis of N-Tert-butyl (4-(5-((1-(3-fluoro-2-methylbenzyl) piperidin-3-yl) methyl) -1,2,4-oxadiazol-3-vl) phenyl)carbamate

To a solution of ethyl 2-(1-(3-fluoro-2-methyl benzyl) piperidin-3-yl) acetate (1.5 equiv.) and tert-butyl (4-(N'-hydroxycarbamimidoyl) phenyl] carbamate (1.0 equiv.) in DMSO (3.9 mL) NaOH (1.6 equiv.) was rapidly added. The mixture was stirred at room temperature for 4 h and afterwards diluted with cold water (10 mL). The aqueous phase was extracted with cyclohexane (5x 6 mL). The combined organic phases were dried over Na2SO4, filtrated and concentrated. The residue was then purified on silica gel column chromatography eluting with 1-10 % methanol in dichloromethane to afford the desired product as colourless oil (1.01 g, 2.11 mmol, 54 %).

1H NMR (400 MHz, DMSO-d6): δ 9.72 (s, 1H), 7.83 (d, J = 8.3 Hz, 2H), 7.62 (d, J = 8.3 Hz, 2H), 7.15 - 6.98 (m, 3H), 3.42 (s, 2H), 2.98 - 2.86 (m, 2H), 2.77 - 2.72 (m, 1H), 2.60 - 2.55 (m, 1H), 2.21 (s, 3H), 2.10 - 2.03 (m, 2H), 1.96 - 1.89 (m, 1H), 1.71 - 1.58 (m, 2H), 1.49 (s, 9H), 1.48 - 1.46 (m, 1H), 1.18 - 1.08 (m, 1H).

### Synthesis of 4-(5-((1-(3-fluoro-2-methylbenzyl) piperidin-3-yl) methyl)-1,2,4-oxadiazol-3-yl) aniline

To a stirred solution of N-Tert-butyl (4-(5-((1-(3-fluoro-2-methylbenzyl) piperidin-3-yl) methyl) - 1,2,4-oxadiazol-3-yl) phenyl) carbamate (1.0 equiv.) in dichloromethane (1.8 mL) trifluoroacetic acid (6 equiv.) was added at 0 °C and stirred for 2 h at 25 °C. After completion of the mixture was saturated with sodium bicarbonate solution. The aqueous phase was extracted with ethyl acetate (3x 6 mL) and the combined organic phases were dried over Na2SO4, filtrated and concentrated. The residue was then purified on silica gel column chromatography eluting with 1-10 % methanol in dichloromethane to afford the desired product as yellow solid (183.6 mg, 0.483 mmol, 69 %).

1H NMR (400 MHz, DMSO-d6): δ 7.60 (d, J = 8.1 Hz, 2H), 7.12 - 7.01 (m, 3H), 6.63 (d, J = 8.3 Hz, 2H), 5.75 (s, 2H), 3.51 - 3.39 (m, 2H), 2.97 - 2.83 (m, 2H), 2.79 - 2.71 (m, 1H), 2.67 - 2.56 (m, 1H), 2.21 (s, 3H), 2.11 - 2.02 (m, 2H), 1.95 - 1.86 (m, 1H), 1.75 - 1.55 (m, 2H), 1.50 - 1.38 (m, 1H), 1.14 - 1.08 (m, 1H).

1.0 equivalent of 4-(5-((1-(3-fluoro-2-methylbenzyl) piperidin-3-yl) methyl)-1,2,4-oxadiazol-3-yl) aniline was dissolved in DCM (1.3 mL). 1.2 equivalent of the corresponding isocyanate was added dropwise to the stirred solution. The mixture was stirred at room temperature for 4 h and concentrated.

**PM07:** 1-[4-(5-((1-(3-fluoro-2-methylbenzyl) piperidin-3-yl) methyl) -1,2,4-oxadiazol-3-yl) phenyl]-3-[(4-methoxy) phenyl] urea. The crude was purified on silica gel column chromatography eluting 5-10 % methanol in dichloromethane to afford the desired product as white solid (53.1 mg, 0.101 mmol, 39 %).

**PM07:** 1H NMR (400 MHz, DMSO-d6): δ 8.96 (s, 1H), 8.60 (s, 1H), 7.88 - 7.85 (d, J = 8.3 Hz, 2H), 7.64 - 7.61 (d, J = 8.3 Hz, 2H), 7.39 - 7.37 (d, J = 8.1 Hz, 2H), 7.17 - 6.99 (m, 3H), 6.90 - 6.86 (d, J = 8.1 Hz, 2H), 3.72 (s, 3H), 3.42 (s, 2H), 3.00 - 2.88 (m, 2H), 2.77 - 2.75 (m, 1H), 2.65 - 2.56 (m, 1H), 2.22 (s, 3H), 2.13 - 2.04 (m, 2H), 1.97 - 1.91 (m, 1H), 1.74 - 1.59 (m, 2H), 1.51 - 1.41 (m, 1H), 1.19 - 1.11 (m, 1H).

**PM16:** 1-(4-(5-((1-(3-fluoro-2-methylbenzyl) piperidin-3-yl) methyl)-1,2,4-oxadiazol-3-yl) phenyl)-3-phenylurea. The crude was purified on silica gel column chromatography eluting 1-5 % methanol in dichloromethane to afford the desired product as white solid (46.5 mg, 0.09 mmol, 36 %).

**PM16:** 1H NMR (400 MHz, DMSO-d6): δ 9.07 (s, 1H), 8.82 (s, 1H), 7.88 - 7.85 (d, J = 8.3 Hz, 1H), 7.64 - 7.61 (d, J = 8.3 Hz, 1H), 7.48 - 7.46 (dd, J = 8.3 Hz, 1.8 Hz), 7.29 (dd, J = 8.3 Hz), 7.17 - 7.10 (m, 1H), 7.08 - 6.98 (m, 3H), 3.42 (s, 2H), 3.00 - 2.87 (m, 2H), 2.76 - 2.74 (m, 1H), 2.63 - 2.56 (m, 1H), 2.22 - 2.21 (s, 3H), 2.11 - 2.05 (m, 2H), 1.98 - 1.89 (m, 1H), 1.73 - 1.58 (m, 2H), 1.52 - 1.39 (m, 1H), 1.20 - 1.07 (m, 1H).

### 2.11. Preparation of compound JB014:

1-(quinolin-6-yl)-3-((5-(4-(trifluoromethyl)phenyl)-1,2,4-oxadiazol-3-yl)methyl)urea was synthesized according to general synthesis procedure C. The crude solid was washed and filtrated with DCM/cyclohexane (50/50) and purified by flash column chromatography with DCM/MeOH (eluent composition: 100/0 → 90/10) and cyclohexane/ethyl acetate (100/0 → 0/100) to gain a white solid ( 71.6 mg, Yield: 31.9%); rf = 0.4 (DCM/MeOH 90/10).

**JB014:** 1H NMR (400 MHz, DMSO-d6) δ 9.37 (s, 1H, quinolin-NHUrea), 8.71 (dd, J = 4.2, 1.7 Hz, 1H, quinoline H2), 8.22 (d, J = 8.1 Hz, 2H, phenyl H2,6), 8.25 - 8.17 (m, 3H, phenyl H2,6 , quinoline H4), 8.13 (d, J = 2.4 Hz, 1H, quinoline H5), 7.94 (d, J = 8.2 Hz, 2H; phenyl H3,5), 7.91 (d, J = 9.1 Hz, 1H, quinoline H8), 7.68 (dd, J = 9.1, 2.4 Hz, 1H, quinoline H7), 7.43 (dd, J = 8.3, 4.2 Hz, 1H, quinoline H3), 7.12 (t, J = 5.8 Hz, 1H, methylene-NHUrea), 4.75 (d, J = 5.8 Hz, 2H, oxadiazol-CH2-Urea).

### 2.12. Preparation of compound AH1:

1-(2-chloro-4-nitrophenyl)-3-(3,5-dichlorophenyl) urea. To a stirred solution of 3, 5-dichloroanilin (162 mg, 1 mmol, 1.0 equiv.) in dichloromethane (5 mL), 2-chloro-1-isocyanato-4-nitrobenzene (200.7 mg, 1 mmol, 1.0 equiv.) was added drop wise. The mixture was stirred at room temperature for 4 h and concentrated. The residue was purified on silica gel column chromatography eluting with 30-100 % ethylacetate in cyclohexane to afford the desired product as yellow solid (184.8 mg, 0.513 mmol, 53 %).

AH1: 1H NMR (400 MHz, DMSO-d6) δ 10.04 (s, 1H), 8.91 (s, 1H), 8.51 (d, J = 9.4 Hz, 1H), 8.36 (d, J = 2.7 Hz, 1H), 8.22 (dd, J = 9.3, 2.7 Hz, 1H), 7.53 (d, J = 1.9 Hz, 2H), 7.26 (t, J = 1.8 Hz, 1H).

### 2.13. Preparation of compound AH3:

1-(3-chlorophenyl)-3-(3-fluoro-2-methylphenyl) urea. To a stirred solution of 3-chloroanilin (127.57 mg, 1 mmol, 1.0 equiv.) in dichloromethane (5 mL), 1-fluoro-3-isocyanato-2-methylbenzene (151.14 mg, 1 mmol, 1.0 equiv.) was added drop wise. The mixture was stirred at room temperature for 4.5 h and concentrated. The residue was purified on silica gel column chromatography eluting with 1-10 % methanol in dichloromethane to afford the desired product as white solid (89.8 mg, 0.322 mmol, 32 %).

AH3: 1H NMR (400 MHz, DMSO-d6) δ 9.25 (s, 1H), 8.18 (s, 1H), 7.75 (t, J = 2.1 Hz, 1H), 7.66 (d, J = 8.2 Hz, 1H), 7.32 (t, J = 8.0 Hz, 1H), 7.29 - 7.13 (m, 2H), 7.03 (ddd, J = 7.9, 2.1, 1.1 Hz, 1H), 6.89 (t, J = 8.9 Hz, 1H), 2.15 (d, J = 2.1 Hz, 3H).

### 2.14. Preparation of compound JB002:

1-(1,5-naphthyridin-2-yl)-3-phenylurea was synthesized according to general synthesis procedure A. The precipitated solid was washed and filtered with DCM/cyclohexane (50/50) to gain an orange solid (184.8 mg, Yield: 93.3%); rf = 0.32 (Cyclohexane/ethyl acetate 90/10).

**JB002:** 1H NMR (400 MHz, DMSO-d6) δ 11.67 (s, 1H, naphthyridine-NHUrea), 10.29 - 10.16 (m, 1H, phenyl-NHUrea), 8.83 (dd, J = 4.2, 1.6 Hz, 1H, H2), 8.40 - 8.33 (m, 2H, H6/8 or 7), 7.75 - 7.72 (q, J = 4.2 Hz, 1H, naphthyridine H7), 7.72-7-68 (m, 2H, phenyl H2,6), 7.67 (d, J = 9.2 Hz, 1H, H7 or 8), 7.41 - 7.33 (tt, J = 7.4, 2.0 Hz, 2H, phenyl H3,5), 7.09 (tt, J = 7.4, 1.2 Hz, 1H, phenyl H4).

### 2.15. Preparation of compound JB004:

1-(3-bromoquinolin-6-yl)-3-phenylurea was synthesized according to general synthesis procedure A. The precipitated solid was washed and filtered with DCM/cyclohexane (50/50) to gain a yellow solid (147.7 mg, Yield: 73.9%); rf = 0.1 (Cyclohexane/ethyl acetate 40/60);

**JB004:** 1H NMR (400 MHz, DMSO-d6) δ 9.15 (s, 1H, bromoquinoline-NHUrea), 8.82 (s, 1H, phenyl-NHUrea), 8.77 (d, J = 2.3 Hz, 1H, bromoquinoline H2), 8.63 (d, J = 2.4 Hz, 1H, bromoquinoline H4), 8.15 (d, J = 2.5 Hz, 1H, bromoquinoline H5), 7.96 (d, J = 9.0 Hz, 1H, bromoquinoline H8), 7.78 (dd, J = 9.1, 2.4 Hz, 1H, bromoquinoline H7), 7.51 (d, J = 7.5 Hz, 2H, phenyl H2,6), 7.32 (t, J = 8.0 Hz, 2H, phenyl H3,5), 7.01 (t, J = 7.4 Hz, 1H, phenyl H4).

### 2.16. Preparation of compound TCMDC-143175:

**TCMDC-143175:_1H** NMR (400 MHz, DMSO-d6) δ 9.93 (s, 1H), 8.87 (s, 1H), 8.56 - 8.53 (d, J = 12 Hz, 1H), 8.36 (d, J = 2.7 Hz, 1H), 8.22 - 8.20 (dd, J = 12 Hz, 2.7 Hz, 1H), 7.76 (t, J = 2.7 Hz, 1H), 7.36 (t, J = 12 Hz, 1H), 7.28 - 7.26 (dd, J = 10 Hz, 2.9 Hz 1H), 7.12 - 7.10 (dd, J = 10 Hz, 2.9 Hz 1H).

### 2.17. Preparation of compound A. C2:

A.C2: 1H NMR (400 MHz, DMSO-d6) δ 9.50 (s, 1H), 9.42 (s, 1H), 8.52 - 8.49 (d, J = 12 Hz, 1H), 8.36 (d, J = 2.7 Hz, 1H), 8.23 - 8.21 (dd, J = 12 Hz, 2.7 Hz, 1H), 8.05 - 8.03 (d, J = 12 Hz, 1H), 7.52 - 7.50 (d, J = 12 Hz, 1H), 7.35 (t, J = 12 Hz, 1H), 7.13 (t, J = 12 Hz, 1H).

### 2.18. Preparation of compound AH2:

1-(2-chloro-4-nitrophenyl)-3-(3-chlorophenyl) thiourea. To a stirred solution of 3- chloroanilin (127.57 mg, 1 mmol, 1.0 equiv.) in dichloromethane (5 mL), 2-chloro-1-isothiocyanato-4-nitrobenzene (215 mg, 1 mmol, 1.0 equiv.) was added drop wise. The mixture was stirred at room temperature for 4.5 h and concentrated. The residue was purified on silica gel column chromatography eluting with 40-100 % ethylacetate in cyclohexane to afford the desired product as yellow solid (132.8 mg, 0.388 mmol, 39 %).

AH2: 1H NMR (400 MHz, DMSO-d6) δ 10.50 (s, 1H), 9.93 (s, 1H), 8.39 (s, 1H), 8.21 (d, J = 9.0 Hz, 1H), 8.12 (d, J = 9.1 Hz, 1H), 7.81 (s, 1H), 7.47 (d, J = 8.1 Hz, 1H), 7.41 (t, J = 8.1 Hz, 1H), 7.25 (d, J = 7.8 Hz, 1H).

## Claims

1. A compound for use in the prevention or treatment of a disease by activating NLRP3 inflammasome, wherein the disease is selected from a group comprising infectious diseases or cancer, and
(I) wherein the compound is a compound of Formula (I) 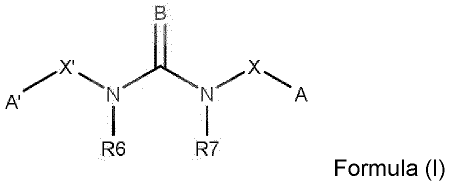 or a pharmaceutically acceceptable salt thereof, wherein:
B is selected from O or S, preferably O;
X and X' are each independently present or not, wherein X and X' are each independently a C₁-C₅ alkyl group, or -CH(CH₂OH)-; and
R⁶ and R⁷ are each independently hydrogen, or a C₁-C₃ alkyl, preferably -H, -CH₃, - C₂H₅, -C₃H₇, or -C₃H₉, or optionally
R⁶ and R⁷ when taken together form a five-membered heterocyclic ring selected from 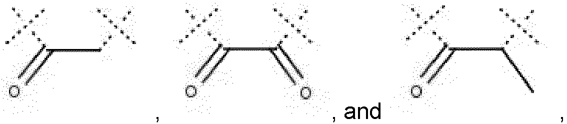 or optionally,
R⁷, N and X when taken together form an six-membered heterocyclic ring selected from 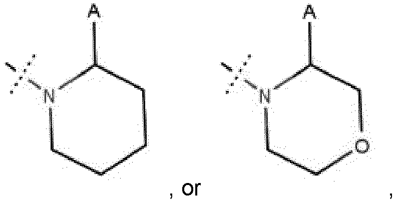 andwherein
A and A' are different from each other and each independently selected from any of one of the following groups:
a) a benzene group of the following structure: 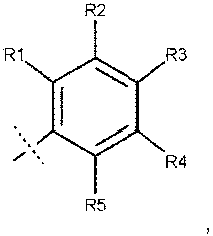
wherein R¹-R⁵ are each independently selected from the group consisting of: hydrogen; -OH, -OCH₃, -OCF₃, -OCHF₂, -OCH₂OH; -OCH₂CH₃; -OCH(CH₃)₂; -F; -Cl; - Br; -CN; NO₂; NH₂; C₁-C₅ alkyl, C₁-C₅ alkoxy, -CH₃, -CH₂F; -CHF₂; -CF₃; -CH₂Cl; - CHCl₂; -CCl₃; CH(CH₃)₂; -CH₂CH₃; -CH-CH₂-CH(CH₃)₂; -CH₂-N(CH₃)₂; -CH(CH₃)-NHC(O)CH₃; - -NH-CH(CH₃)₂, -NH-cyclopropyl, -CH₃, CH₂CH₃, CH₂CH(CH₃)₂; -C(O)-C₂H₅; -C(O)-CH₃; -C(O)-aziridine; -C(O)-azetidine; -C(O)-pyrrolidine; -C(CH₃)=NOH; - S(O)₂-cyclopentyl; -S(O)₂-CH₃; -S(O)₂-cyclopentyl; pyrrolidinyl, 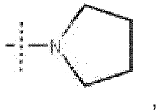 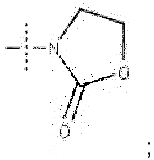
NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-CH(CH₃)₂, -NH-cyclopropyl, C₁-C₅ alkyl, -CH₂F; -CHF₂; -CF₃; -CH₂Cl; -CHCl₂; -CCl₃;-CH₃, CH₂CH₃, CH₂CH(CH₃)₂;
-C(O)OR⁴², wherein R⁴² is selected from C₁-C₅ alkyl, including -CH₃; -CH₂CH₃, - CH₂CH₂CH₃; -CH(CH₃)₂; -CH₂CH₂CH₂CH₃; -CH₂NHS(O)₂CH₃;
-NHR⁴³, wherein R⁴³ is selected from 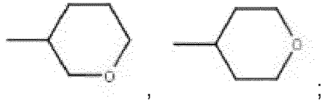 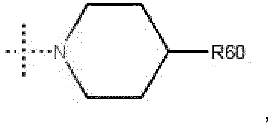 wherein R⁶⁰ is selected from hydrogen or -CH₃; 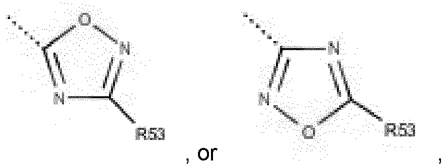
wherein
R⁵³ is preferably selected from the group consisting of hydrogen, OH, -OCH₃, -OCF₃, - OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH-CH₂-CH(CH₃)₂; -CH₂-N(CH₃)₂; -CH(CH₃)-NHC(O)CH₃; -CH₂F; -CHF₂; -CF₃; -CH₂Cl; -CHCl₂; -CCl₃; -O-N=O, -N=O, -CH=NH, -N₃, -SH, -SO₃H, -SO₂H, -S(O)₂CH₃ -CN, -NC,-OCN, -NCO, - (C=O)OH, -(C=O)NHOH, -NH(C=O)NH₂, -NH(C=S)NH₂, -(C=O)NH₂,-CHO,-CHS, - OCHO, -NCHO, 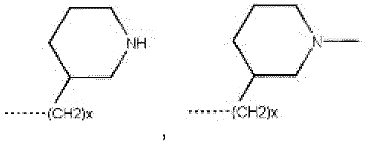 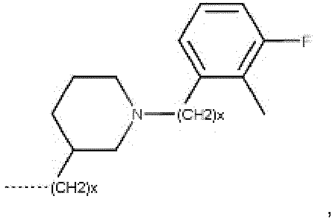 with X = 0 or 1, preferably 1; 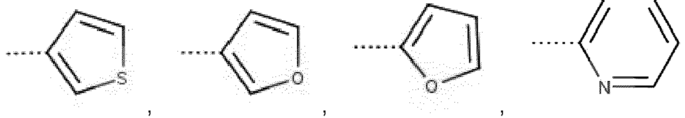 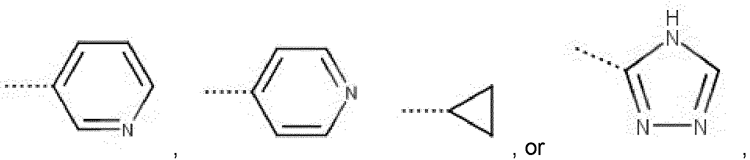 or
R⁵³ is 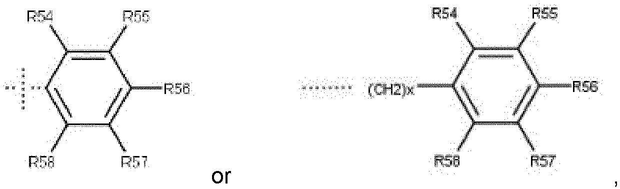
wherein R⁵⁴ R⁵⁵, R⁵⁶, R⁵⁷ or R⁵⁸ are selected each independently from hydrogen, -CH₃, -C₂H₅, -CH₂F; - CHF₂; -CF₃; -CH₂Cl; -CHCl₂; -CCl₃; -OCH₃, -F, -Cl, -Br, NH₂, NO₂, and x is 0, 1 or 2;
or R⁵³ is 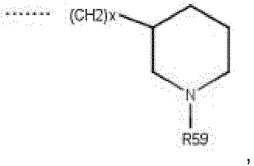
wherein R⁵⁹ is 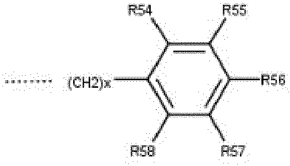
and wherein R⁵⁴, R⁵⁵, R⁵⁶, R⁵⁷ or R⁵⁸ are selected separately from the above from hydrogen, -CH₃, -C₂H₅, -CH₂F; -CHF₂; -CF₃; -CH₂Cl; -CHCl₂; -CCl₃;-OCH₃, -F, -Cl, -Br, NO₂, NH₂, and x is 0, 1 or 2; as defined above;
and
optionally, wherein R¹ and R², R² and R³, R³ and R⁴, or R⁴ and R⁵ together, preferably R² and R³ together, form a five- or six-membered cyclic or heterocyclic ring, selected from 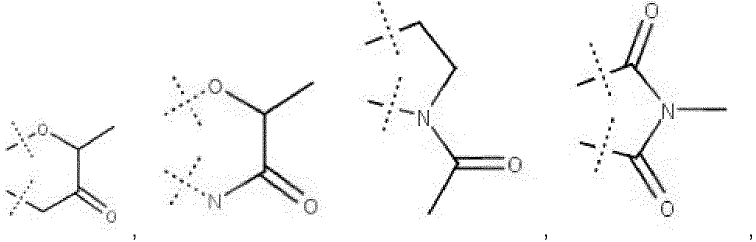 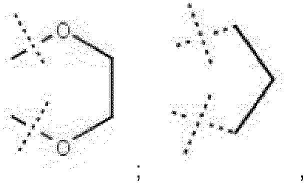
wherein preferably either all of R¹ to R⁵ are hydrogen or 1, 2 or 3, preferably 1 or 2 of R¹ to R⁵ are different from hydrogen and selected from substitutions defined above under a) for R¹ to R⁵, and remaining substituents of R¹ to R⁵ are hydrogen;
b) a pyridine group selected from one of the following structures : 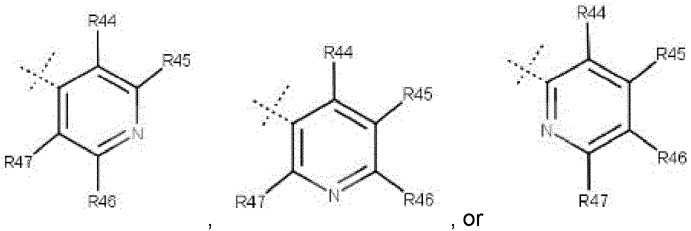 wherein R⁴⁴-R⁴⁷ are each independently selected from the group consisting of:
hydrogen, -OH, -OCH₃, CH₃, F, CI, Br, -CH₂F; -CHF₂; -CF₃; -CH₂Cl; -CHCl₂; -CCl₃;-OCF₃, -OCHF₂, -OCH₂OH; -OCH₂CH₃; -CN; NO₂; NH₂; -CH₃, CH(CH₃)₂; -CH₂CH₃; - CH₂OH; -CH-CH₂-CH(CH₃)₂; -CH₂-N(CH₃)₂; -CH(CH₃)-NHC(O)CH₃; 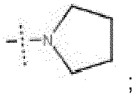
-NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-cyclopropyl, -CH₂F; - CHF₂; -CF₃; -CH₂Cl; -CHCl₂; -CCl₃; -CH₃, CH₂CH₃, CH₂CH(CH₃)₂; -C(O)-aziridine; - C(O)-azetidine; -C(O)-pyrrolidine;
-C(O)OR⁴², wherein R⁴² is selected from C₁-C₅ alkyl, including -CH₃; -CH₂CH₃, - CH₂CH₂CH₃; -CH(CH₃)₂; -CH₂CH₂CH₂CH₃; -CH₂NHS(O)₂CH₃; -C(CH₃)=NOH; -S(O)₂-cyclopentyl; -S(O)₂-CH₃; -S(O)₂-cyclopentyl; pyrrolidinyl, 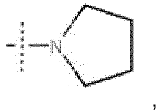 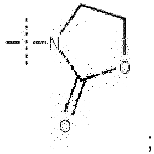 or
-NHR⁴³, wherein is selected from 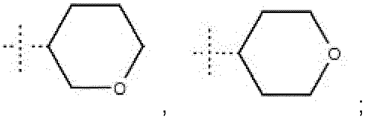
wherein preferably either all of R⁴⁴ to R⁴⁷ are hydrogen or 1, 2 or 3, preferably 1 or 2 of R⁴⁵ to R⁴⁷ are different from hydrogen and selected from substitutions defined above under b) for R⁴⁴ to R⁴⁷ and remaining substituents of R⁴⁴ to R⁴⁷ are hydrogen;
c) a quinoline or an isoquinoline group selected from one of the following structures: 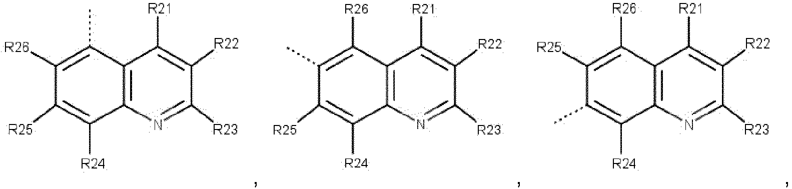 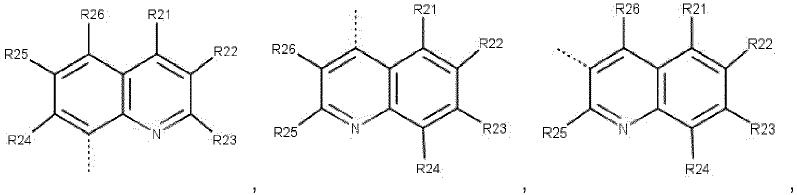 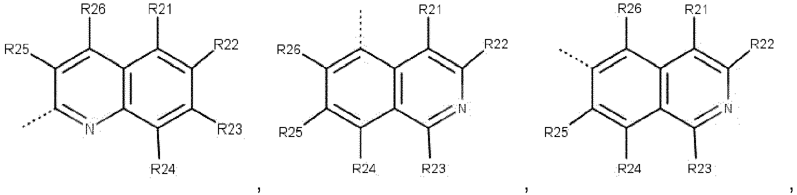 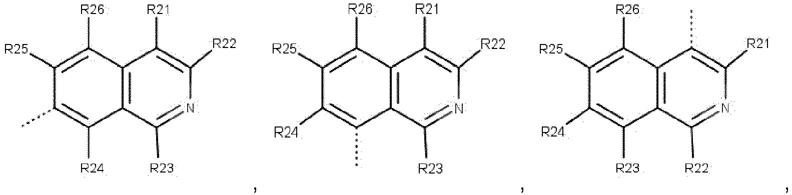 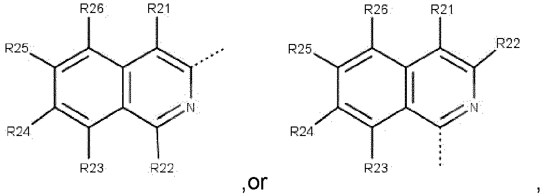 wherein
R²¹ to R²⁶ are each independently selected from the group consisting of:
hydrogen, OH, -OCH₃, -OCF₃, -OCHF₂, -OCH₂OH; -OCH₂CH₃; -F; -Cl; -Br; -CH₂F; - CHF₂; -CF₃; -CH₂Cl; -CHCl₂; -CCl₃; -CN; NO₂; NH₂; C₁-C₅ alkyl, C₁-C₅ alkoxy, C₂-C₅ alkenyl, preferably -CH₃, CH(CH₃)₂; -CH₂CH₃; -CH-CH₂-CH(CH₃)₂; -CH₂-N(CH₃)₂; - CH(CH₃)-NHC(O)CH₃; -O-N=O, -N=O, -CH=NH, -N₃, -SH, -SO₃H, -SO₂H, -S(O)₂CH₃ - CN, -NC,-OCN, -NCO, -SCN, -NCS, -(P=O)(OH)₂, -O(P=O)(OH)₂, -(C=O)OH, - (C=O)NHOH, -NH(C=O)NH₂, -NH(C=S)NH₂, -(C=O)NH₂,-CHO,-CHS, -OCHO, - NCHO, -C(O)-aziridine; -C(O)-azetidine; -C(O)-pyrrolidine; -C(O)OR⁴², wherein R⁴² is selected from C1-C5 alkyl, including -CH₃; -CH₂CH₃, -CH₂CH₂CH₃; -CH(CH₃)₂; - CH₂CH₂CH₂CH₃; -CH₂NHS(O)₂CH₃; -C(CH₃)=NOH; -S(O)₂-cyclopentyl; -S(O)₂-CH₃; - S(O)₂-cyclopentyl; pyrrolidinyl, 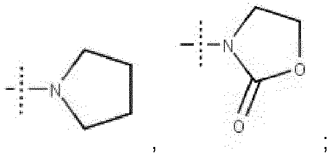
-NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-cyclopropyl, -CH₂F; - CHF₂; -CF₃; -CH₂Cl; -CHCl₂; -CCl₃; -CH₃, CH₂CH₃, CH₂CH(CH₃)₂;
-NHR⁴³, wherein is selected from 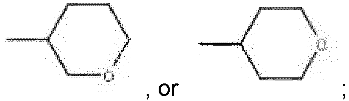
or more preferably wherein R²¹ to R²⁶ are each hydrogen, OH, -OCH₃, -OCF₃, - OCHF₂, -OCH₂OH; -OCH₂CH₃; -F; -Cl; -Br; -CH₂F; -CHF₂; -CF₃; -CH₂Cl; -CHCl₂; - CCl₃;-CN; NO₂; NH₂; C₁-C₅ alkyl, C₁-C₅ alkoxy, C₂-C₅ alkenyl, preferably -CH₃, CH(CH₃)₂; -CH₂CH₃; -CH-CH₂-CH(CH₃)₂; -CH₂-N(CH₃)₂; -CH(CH₃)-NHC(O)CH₃;
or even more preferably wherein R²¹ to R²⁶ are each hydrogen, or -F; Cl, Br,
wherein preferably either all of R²¹ to R²⁶ are hydrogen or 1, 2 or 3, preferably 1 or 2 of R²¹ to R²⁶ are different from hydrogen and selected from substitutions defined above under c) for R²¹ to R²⁶ and remaining substituents of R²¹ to R²⁶ are hydrogen;
d) a 1,5 naphthyridine group, a 1,6 naphthyridine group or a 1,8 naphthyridine group of any of the following structures: 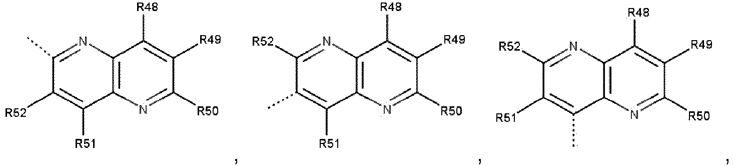 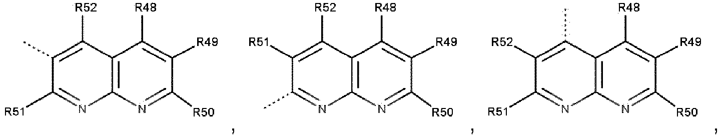 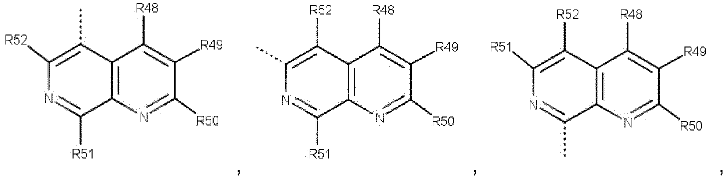 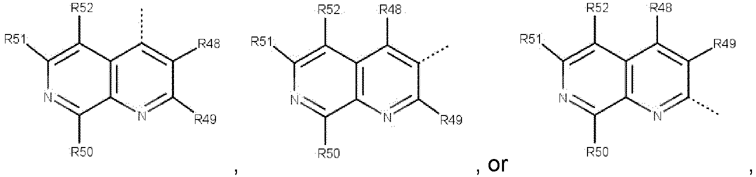 wherein
R⁴⁸ to R⁵² are each independently selected from the group consisting of:
hydrogen, OH, -OCH₃, -OCF₃, -OCHF₂, -OCH₂OH; -OCH₂CH₃; -F; -Cl; -Br; -CH₂F; - CHF₂; -CF₃; -CH₂Cl; -CHCl₂; -CCl₃;-CN; NO₂; NH₂; C₁-C₅ alkyl, C₁-C₅ alkoxy, C₂-C₅ alkenyl, preferably -CH₃, CH(CH₃)₂; -CH₂CH₃; -CH-CH₂-CH(CH₃)₂; -CH₂-N(CH₃)₂; - CH(CH₃)-NHC(O)CH₃; -O-N=O, -N=O, -CH=NH, -N₃, -SH, -SO₃H, -SO₂H, -S(O)₂CH₃ - CN, -NC,-OCN, -NCO, -SCN, -NCS, -(P=O)(OH)₂, -O(P=O)(OH)₂, -(C=O)OH, - (C=O)NHOH, -NH(C=O)NH₂, -NH(C=S)NH₂, -(C=O)NH₂,-CHO,-CHS, -OCHO, - NCHO,
-NHC(O)-R⁴¹, wherein R⁴¹ is selected from C₁-C₁₀ cycloalkyl, C₁-C₁₀ cycloheteroalkyl, furan, tetrahydrofuran, cyclopentyl, cyclobutyl, cyclopropyl, -NH-cyclopropyl, -CH₂F; - CHF₂; -CF₃; -CH₂Cl; -CHCl₂; -CCl₃; -CH₃, CH₂CH₃, CH₂CH(CH₃)₂; -C(O)-aziridine; - C(O)-azetidine; -C(O)-pyrrolidine;
-C(O)OR⁴², wherein R⁴² is selected from C₁-C₅ alkyl, including -CH₃; -CH₂CH₃, - CH₂CH₂CH₃; -CH(CH₃)₂; -CH₂CH₂CH₂CH₃; -CH₂NHS(O)₂CH₃; -C(CH₃)=NOH; -S(O)₂-cyclopentyl; -S(O)₂-CH₃; -S(O)₂-cyclopentyl; pyrrolidinyl, 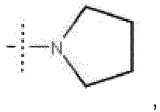 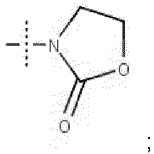 -NHR₄₃, wherein R₄₃ is selected from 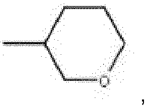 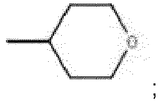
or more preferably wherein R⁴⁸ to R⁵² are each hydrogen, OH, -OCH₃, -OCF₃, - OCHF₂, -OCH₂OH; -OCH₂CH₃; -F; -Cl; -Br; -CH₂F; -CHF₂; -CF₃; -CH₂Cl; -CHCl₂; - CCl₃;-CN; NO₂; NH₂; C₁-C₅ alkyl, C₁-C₅ alkoxy, C₂-C₅ alkenyl, preferably -CH₃, CH(CH₃)₂; -CH₂CH₃; -CH-CH₂-CH(CH₃)₂; -CH₂-N(CH₃)₂; -CH(CH₃)-NHC(O)CH₃; or even more preferably wherein R⁴⁸ to R⁵² are each hydrogen, or -F; Cl, Br,
wherein preferably either all of R⁴⁸ to R⁵² are hydrogen or 1, 2 or 3, preferably 1 or 2 of R⁴⁸ to R⁵² are different from hydrogen and selected from substitutions defined or presented herein above under d) for R⁴⁸ to R⁵² and remaining substituents of R⁴⁸ to R⁵² are hydrogen;
e) an oxadiazole group, selected from one of the following structures: 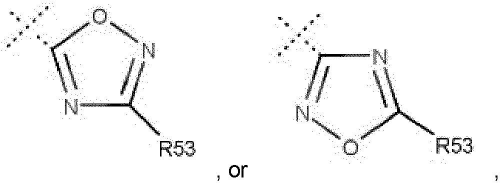 wherein
R⁵³ is preferably selected from the group consisting of:
hydrogen, OH, -OCH₃, -OCF₃, -OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; - CH-CH₂-CH(CH₃)₂; -CH₂-N(CH₃)₂; -CH(CH₃)-NHC(O)CH₃; -CH₂F; -CHF₂; -CF₃; - CH₂Cl; -CHCl₂; -CCl₃;-O-N=O, -N=O, -CH=NH, -N₃, -SH, -SO₃H, -SO₂H, -S(O)₂CH₃ - CN, -NC,-OCN, -NCO, -(C=O)OH, -(C=O)NHOH, -NH(C=O)NH₂, -NH(C=S)NH₂, - (C=O)NH₂,-CHO,-CHS, -OCHO, -NCHO, 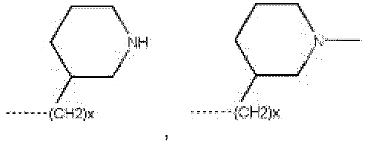 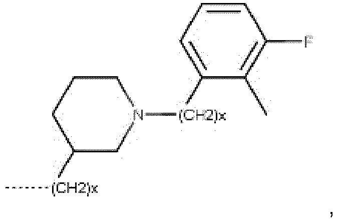 with X = 0 or 1, preferably 1; 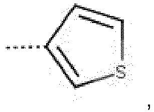 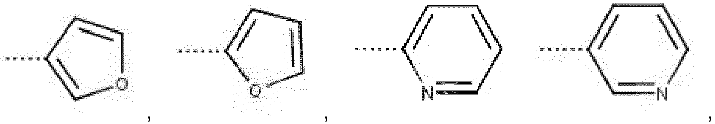 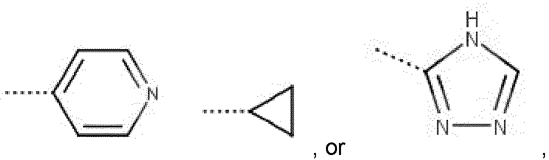 or
R⁵³ is 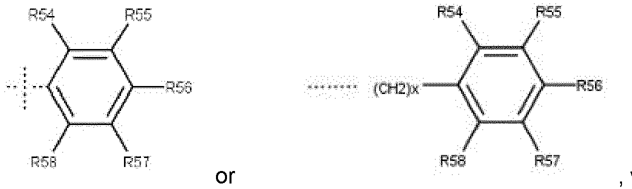
wherein R⁵⁴, R⁵⁵, R⁵⁶, R⁵⁷ or R⁵⁸ are selected each independently from hydrogen, -CH₃, -C₂H₅, - CH₂F; -CHF₂; -CF₃; -CH₂Cl; -CHCl₂; -CCl₃; -OCH₃, -F, -Cl, -Br, NH₂, NO₂, and x is 0, 1 or 2;
or R53 is 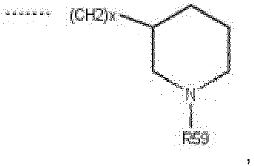 wherein R⁵⁹ is 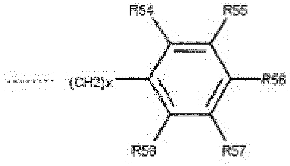 and wherein R⁵⁴, R⁵⁵, R⁵⁶, R⁵⁷ or R⁵⁸ are selected separately from the above from hydrogen, -CH₃, -C₂H₅, -CH₂F; -CHF₂; -CF₃; -CH₂Cl; -CHCl₂; -CCl₃; -OCH₃, -F, -Cl, -Br, NO₂, NH₂, and x is 0, 1 or 2; as defined above, and x is 0, 1 or 2;
and wherein preferably either all of R⁵⁴ to R⁵⁸ are hydrogen or 1, 2 or 3, preferably 1 or 2 of R⁵⁴ to R⁵⁸ are different from hydrogen and selected from substitutions defined above under e) for R⁵⁴ to R⁵⁸ and remaining positions of R⁵⁴ to R⁵⁸ are hydrogen;
f) a thiadiazole group, selected from one of the following structures: 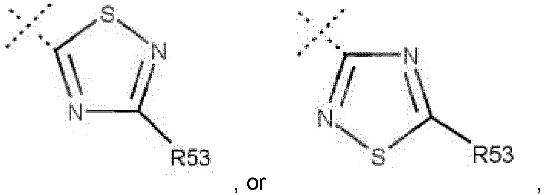 wherein R⁵³ is preferably as defined above
g) an isothiazole group, selected from one of the following structures: 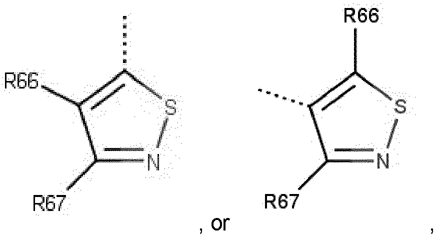 wherein R⁶⁶ and R⁶⁷ are each selected independently from hydrogen, -CH₃, -C₂H₅, -CH₂F; -CHF₂; -CF₃; -CH₂Cl; -CHCl₂; - CCl₃;-F, -Cl, -Br, preferably wherein either of R⁶⁶ or R⁶⁷ is hydrogen and the other residue is selected from -CH₃, -C₂H₅, -CF₃, -CHF₂, -F, -Cl, -Br; or
h) a thiophene group, selected from one of the following structures: 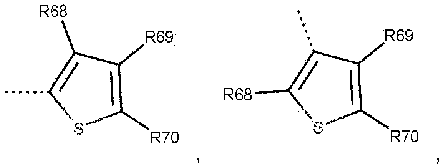 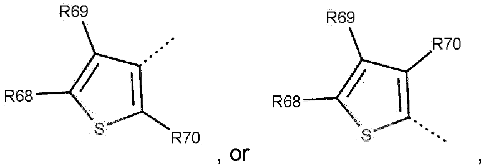 wherein R⁶⁸, R⁶⁹ and R⁷⁰ are each selected independently from hydrogen, -CH₃, - C₂H₅, -CH₂F; -CHF₂; -CF₃; -CH₂Cl; -CHCl₂; -CCl₃;-F, -Cl, -Br, preferably wherein either of R⁶⁸, R⁶⁹ or R⁷⁰ is hydrogen and the other residue is selected from -CH₃, -C₂H₅, - CF₃, -CHF₂, -F, -Cl, -Br,
or
(II) wherein the compound is a compound of Formula (Ic), a compound of Formula (Ic1), a compound of Formula (Ic2) a compound of Formula (Ic3), or a compound of Formula (Ic4), or a pharmaceutically acceptable salt thereof: 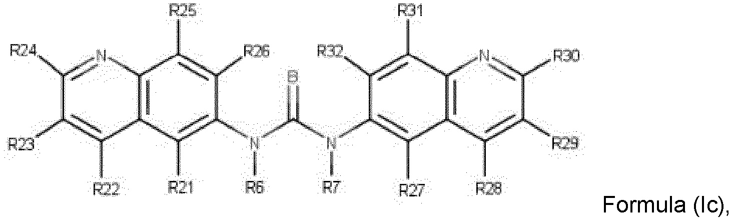 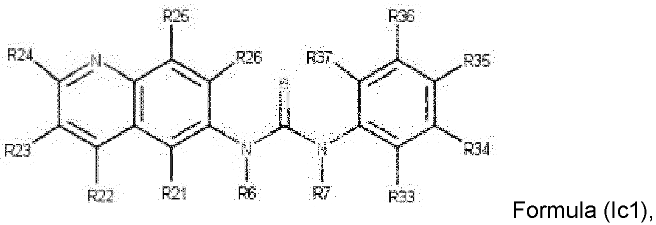 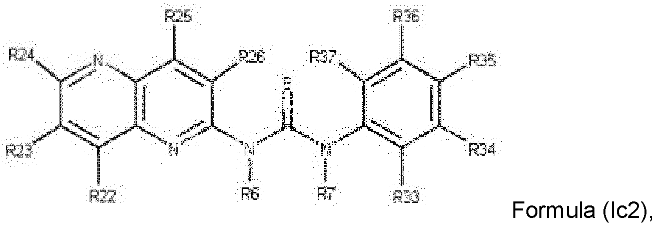 or 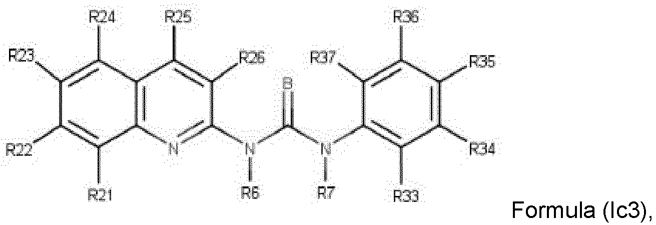 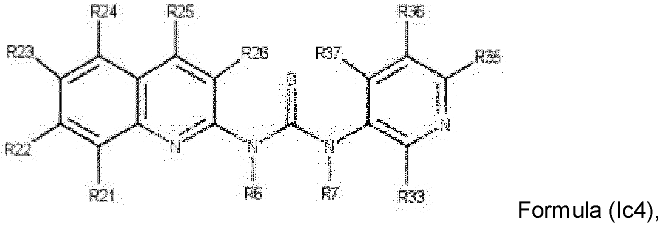 wherein
B = O or S, preferably O;
R⁶ and R⁷ are independently each hydrogen, C₁₋₃ alkyl, preferably hydrogen, CH₃, more preferably, either R⁶ is hydrogen and R⁷ is CH₃, or R6 and R7 are both hydrogen;
R²¹ to R³⁷ are each independently selected from the group consisting of hydrogen, OH, -OCH₃, -OCF₃, -OCHF₂, -OCH₂OH; -OCH₂CH₃; -CH₂F; -CHF₂; -CF₃; -CH₂Cl; - CHCl₂; -CCl₃;-F; -Cl; -Br; -CN; NO₂; NH₂; -CH₃, CH(CH₃)₂; -CH₂CH₃; -CH-CH₂-CH(CH₃)₂; -CH₂-N(CH₃)₂; -CH(CH₃)-NHC(O)CH₃; -O-N=O, -N=O, -OH, -NH₂, - CH=NH, -N₃, -SH, -SO₃H, -SO₂H, -S(O)₂CH₃ -CN, -NC,-OCN, -NCO, -SCN, -NCS, - (P=O)(OH)₂, -O(P=O)(OH)₂, -(C=O)OH, -(C=O)NHOH, -NH(C=O)NH₂, -NH(C=S)NH₂, -(C=O)NH₂,-CHO,-CHS, -OCHO, -NCHO, -B(OH)₂ (C=O)R¹¹, (C=O)OR¹¹, (C=O)SR¹¹, (C=O)NR¹¹R¹², (SO₂)R¹¹, or C(NR¹¹)R¹², -NHC(O)R¹¹, and -NHC(O)NHR¹¹,
wherein R¹¹ and R¹² are independently selected from the group consisting of C₁-C₅ alkyl, preferably -CH₃, CH(CH₃)₂; -CH₂CH₃; -CH-CH₂-CH(CH₃)₂; -CH₂-N(CH₃)₂; - CH(CH₃)-NHC(O)CH₃; I, -CH₂F; -CHF₂; -CF₃; -CH₂Cl; -CHCl₂; -CCl₃;-CHCl₂, and - CCl₃; or
preferably wherein R²¹ to R³⁷ are each independently selected from the group consisting of hydrogen, -CH₂(SO₂)CH₃, -CH₂(SO₂)CH₂CH₃, -CH₂CH₂(SO₂)CH₂CH₃; - (SO₂)C₁-C₅ alkyl, preferably -(SO₂)CH₃, -(SO₂)CH₂CH₃, -CH₂F; -CHF₂; -CF₃; -CH₂Cl; - CHCl₂; -CCl₃;-F, -Cl, and -Br; or
wherein preferably either all of R²¹ to R³⁷ are hydrogen or 1, 2 or 3, preferably 1 or 2 of R²¹ to R³⁷ are different from hydrogen and selected from substitutions presented above and remaining positions of R²¹ to R³⁷ are hydrogen; or
more preferably wherein R²¹ to R³⁷ are each hydrogen.

2. The compound for use according to claim 1 , wherein the compound is a compound of Formula (Ic5), a compound of Formula (Ic6), a compound of Formula (Ic7), a compound of Formula (Ic8), a compound of Formula (Ic9) or a compound of Formula (Ic10), a compound of Formula (Ic11), a compound of Formula (Ic12), or a compound of Formula (Ic13), or a pharmaceutically acceptable salt thereof: 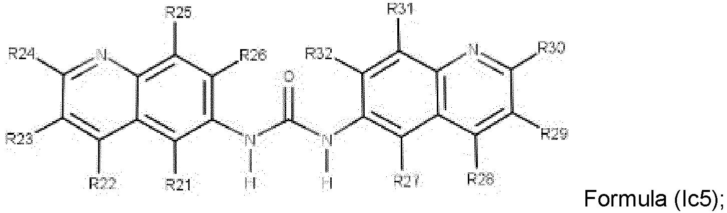 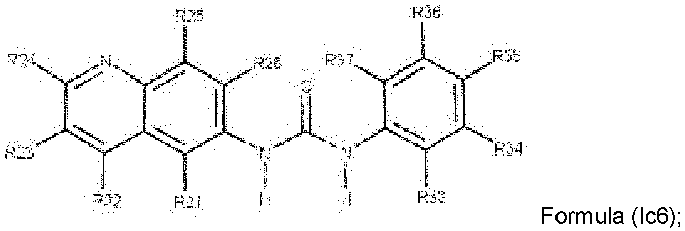 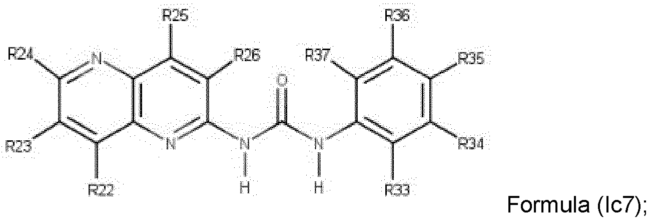 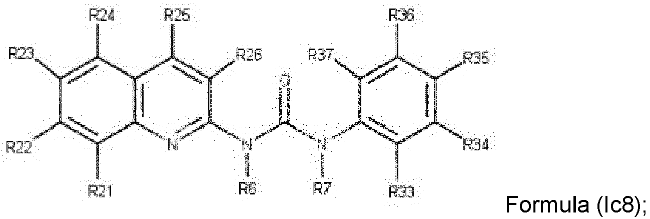 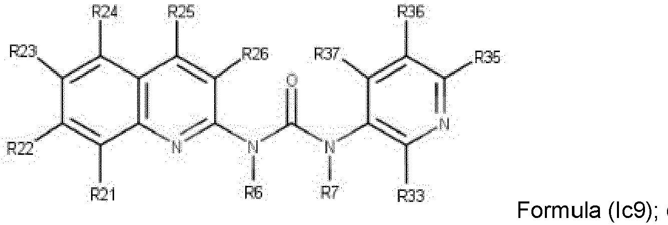 or 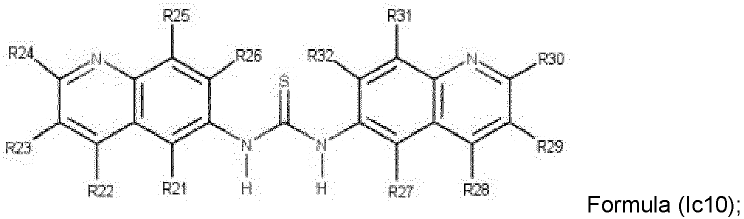 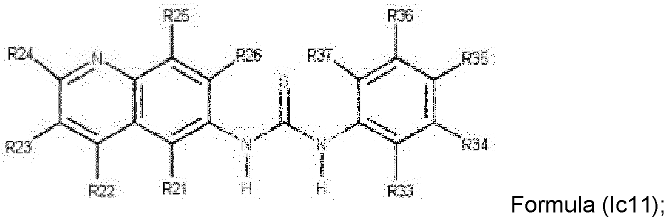 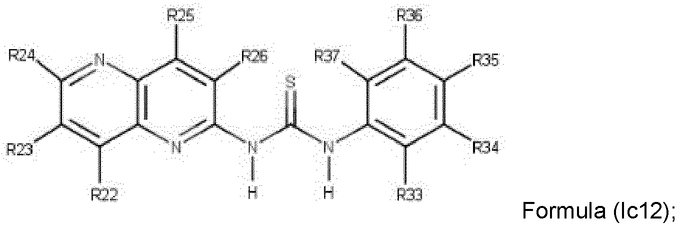 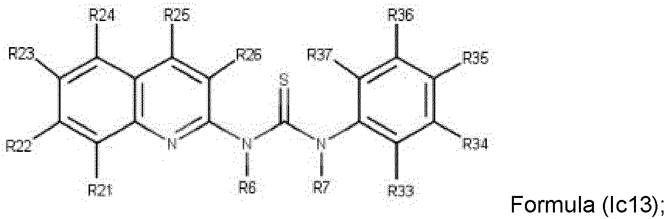 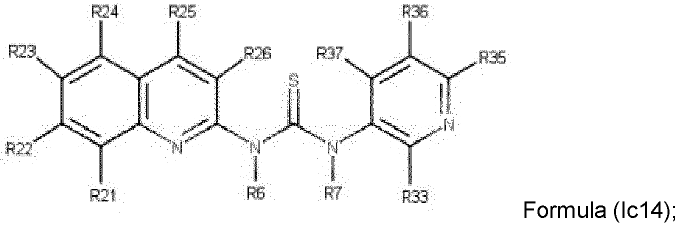
wherein R²¹ to R³⁷ are each independently selected from the group consisting of hydrogen, OH, -OCH₃, -OCF₃, -OCHF₂, -OCH₂OH; -OCH₂CH₃; -CH₂F; -CHF₂; -CF₃; -CH₂Cl; -CHCl₂; -CCl₃; -F; -Cl; -Br; -CN; NO₂; NH₂; -CH₃, CH(CH₃)₂; -CH₂CH₃; or - CH₂(SO₂)CH₃, -CH₂(SO₂)CH₂CH₃, -CH₂CH₂(SO₂)CH₂CH₃; -(SO₂)C₁-C₅ alkyl, preferably -(SO₂)CH₃, -(SO₂)CH₂CH₃, or
wherein preferably either all of R²¹ to R³⁷ are hydrogen or 1, 2 or 3, preferably 1 or 2 of R²¹ to R³⁷ are different from hydrogen and selected from substitutions presented above and remaining positions of R²¹ to R³⁷ are selected from hydrogen; or
more preferably wherein R²¹ to R³⁷ are each hydrogen.

3. The compound for use according to any of claims 1 or 2, wherein the compound is a compound of one of Formulae (la) or (la1) 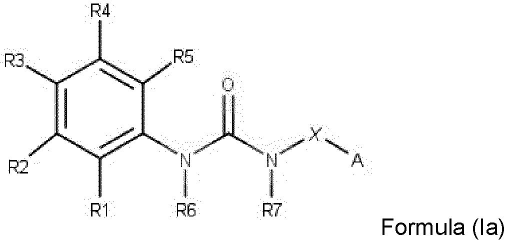 or 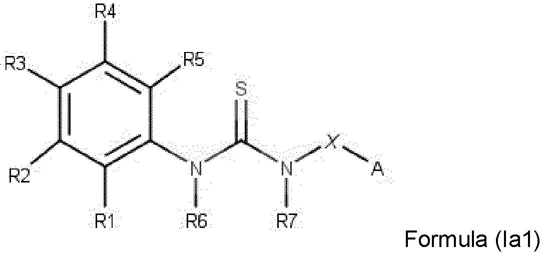 or a pharmaceutically acceceptable salt thereof, wherein:
A and A' are different to each other,
X is either present or not, and wherein X, if present, is selected from -CH₂-, -C₂H₄-, - C₃H₆-, -C(CH₃)₂-, -CHCH(CH₃)₂-, -CHC(CH₃)₃-, -CH(CH(CH₃)₂)-, - CH(CH₂CH(CH₃)₂)-, or -CH(CH₂OH)-; and
R⁶ and R⁷ are each independently hydrogen, -CH₃, -C₂H₅, -C₃H₇, or -C₃H₉, or optionally,
R⁶ and R⁷ when taken together form a five-membered heterocyclic ring, selected from 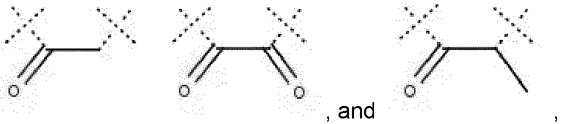 or optionally,
R⁷, N and X when taken together form a six-membered heterocyclic ring, selected from 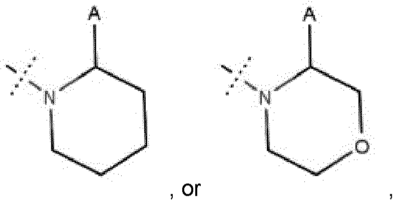
wherein R¹-R⁵ are each independently selected from the group consisting of hydrogen; -OH, -OCH₃, -OCF₃, -OCHF₂, -OCH₂OH; -OCH₂CH₃; -OCH(CH₃)₂; -F; -Cl; -Br; -CN; NO₂; NH₂; -CH₃, -CH₂F; -CHF₂; -CF₃; -CH₂Cl; -CHCl₂; -CCl₃; CH(CH₃)₂; - CH₂CH₃;
-C(O)OR₄₂, wherein R⁴² is selected from -CH₃; -CH₂CH₃, -CH₂CH₂CH₃; -CH(CH₃)₂; - CH₂CH₂CH₂CH₃; 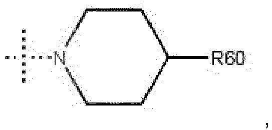 wherein R⁶⁰ is selected from hydrogen or -CH₃;
optionally, wherein R¹ and R², R² and R³, R³ and R⁴, or R⁴ and R⁵ together, preferably R¹ and R² or R² and R³ together, form a five- or six-membered cyclic or heterocyclic ring, selected from 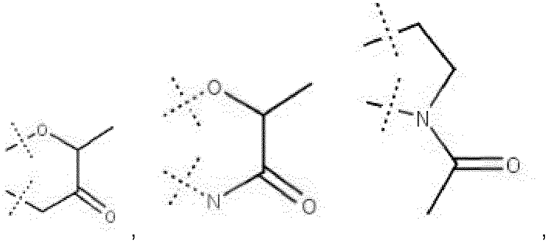 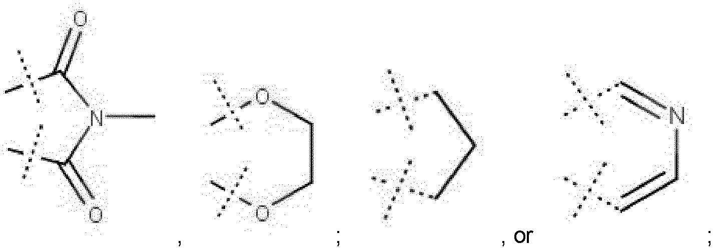
and wherein A is selected from one of the following substituents a) to e):
a) a benzene group of the following structure: 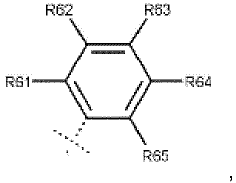 wherein R⁶¹-R⁶⁵ are each independently selected from the group consisting of:
hydrogen; -OH, -OCH₃, -OCF₃, -OCHF₂, -OCH₂OH; -OCH₂CH₃; -OCH(CH₃)₂; -F; -Cl; - Br; -CN; NO₂; NH₂; -CH₃, -CH₂F; -CHF₂; -CF₃; -CH₂Cl; -CHCl₂; -CCl₃;CH(CH₃)₂ - CH₂CH₃; -CH-CH₂-CH(CH₃)₂; -CH₂-N(CH₃)₂; -CH(CH₃)-NHC(O)CH₃; -C(O)-C₂H₅; - C(O)-CH₃; -C(O)-aziridine; -C(O)-azetidine; -C(O)-pyrrolidine; -CH₂NHS(O)₂CH₃; - C(CH₃)=NOH; -S(O)₂-cyclopentyl; -S(O)₂-CH₃; -S(O)₂-cyclopentyl; pyrrolidinyl, 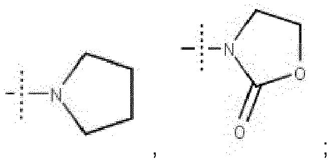
-NHC(O)-R₄₁, wherein R₄₁ is selected from furan, tetrahydrofuran, cyclopentyl, cyclobutyl, cyclopropyl, -NH-CH(CH₃)₂, -NH-cyclopropyl, -CH₂F; -CHF₂; -CF₃; -CH₂Cl; -CHCl₂; -CCl₃;-CH₃, CH₂CH₃, CH₂CH(CH₃)₂;
-C(O)OR₄₂, wherein R⁴² is selected from C₁-C₅ alkyl, including -CH₃; -CH₂CH₃, - CH₂CH₂CH₃; -CH(CH₃)₂; -CH₂CH₂CH₂CH₃;
-NHR₄₃, wherein R₄₃ is selected from 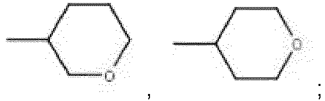 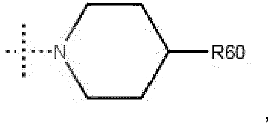 wherein R⁶⁰ is selected from hydrogen or -CH₃; 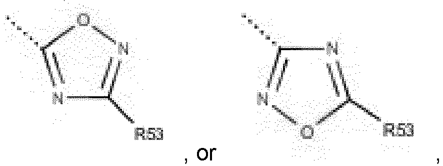
wherein R⁵³ is preferably selected from the group consisting of hydrogen, OH, -OCH₃, -OCF₃, -OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH-CH₂-CH(CH₃)₂; -CH₂-N(CH₃)₂; -CH(CH₃)-NHC(O)CH₃; - CH₂F; -CHF₂; -CF₃; -CH₂Cl; -CHCl₂; -CCl₃; -O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, -NH(C=O)NH₂, - 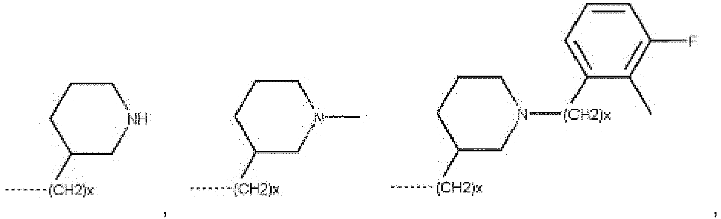 with X = 0 or 1, preferably 1; , 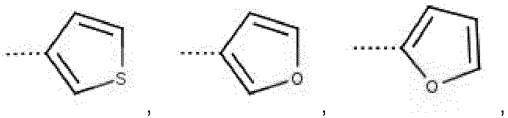 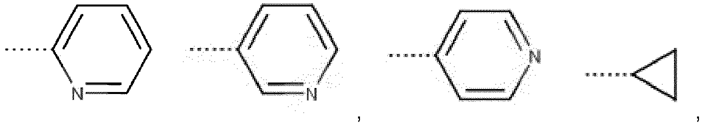 or 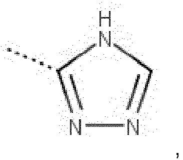
or R⁵³ is 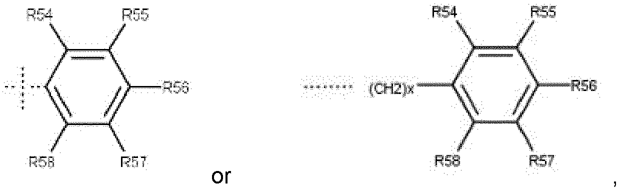
wherein R^{54,} R⁵⁵, R⁵⁶, R⁵⁷ or R⁵⁸ are selected each independently from hydrogen, -CH₃, -C₂H₅, - CH₂F; -CHF₂; -CF₃; -CH₂Cl; -CHCl₂; -CCl₃; -OCH₃, -F, -Cl, -Br, NO₂, NH₂, and x is 0, 1 or 2;
or R⁵³ is 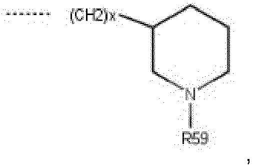
wherein R⁵⁹ is 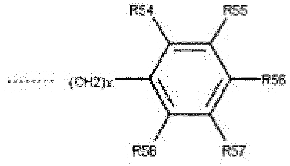
and wherein R^{54,} R⁵⁵, R⁵⁶, R⁵⁷ or R⁵⁸ are selected separately from the above from hydrogen, -CH₃, -C₂H₅, -CH₂F; -CHF₂; -CF₃; -CH₂Cl; -CHCl₂; -CCl₃;-OCH₃, -F, -Cl, -Br, NO₂, NH₂, and x is 0, 1 or 2; as defined above, and x is 0, 1 or 2;
and
optionally, wherein R⁶¹ and R⁶², R⁶² and R⁶³, R⁶³ and R⁶⁴, or R⁶⁴ and R⁶⁵ together, preferably R⁶² and R⁶³ together, form a five- or six-membered cyclic or heterocyclic ring, selected from 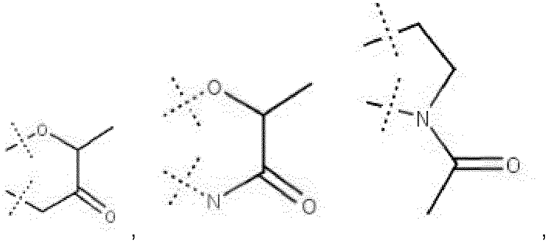 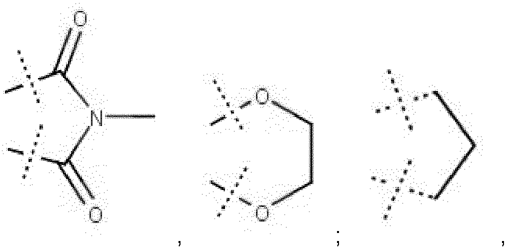 and
wherein preferably X is not present, and
wherein preferably either all of R¹ to R⁵ are hydrogen or 1, 2 or 3, preferably 1 or 2 of R¹ to R⁵ are different from hydrogen and selected from substitutions defined above for R¹ to R⁵ and remaining substituents of R¹ to R⁵ are hydrogen;
b) an oxadiazole group of one of the following structure: 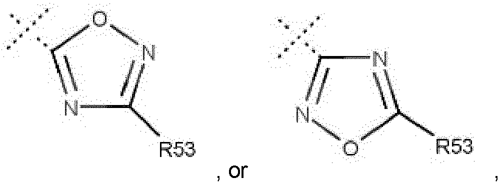 wherein
R⁵³ is preferably selected from the group consisting of hydrogen, OH, -OCH₃, -OCF₃, - OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH-CH₂-CH(CH₃)₂; -CH₂-N(CH₃)₂; -CH(CH₃)-NHC(O)CH₃; -CH₂F; -CHF₂; -CF₃; -CH₂Cl; -CHCl₂; -CCl₃;-O-N=O, -N=O, -CH=NH, -N₃, -SH, -SO₃H, -SO₂H, -S(O)₂CH₃ -CN, -NC,-OCN, -NCO, - (C=O)OH, -(C=O)NHOH, -NH(C=O)NH₂, -NH(C=S)NH₂, -(C=O)NH₂,-CHO,-CHS, - OCHO, -NCHO, - 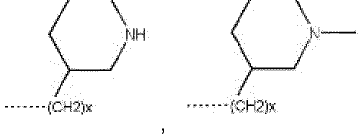 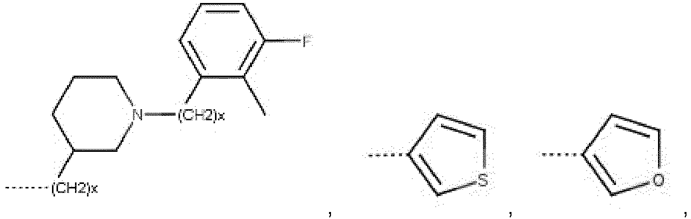 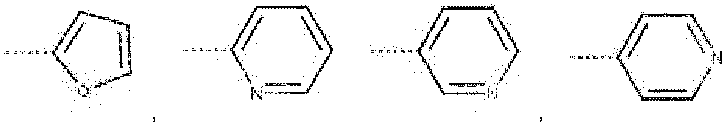 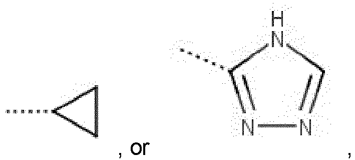 or
R⁵³ is 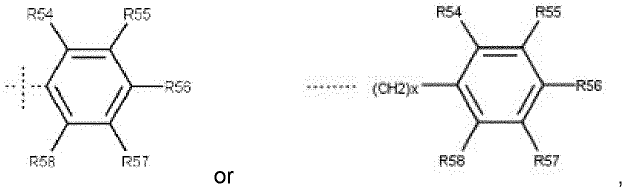 wherein R^{54,} R⁵⁵, R⁵⁶, R⁵⁷ or R⁵⁸ are selected each independently from hydrogen, -CH₃, -C₂H₅, - CH₂F; -CHF₂; -CF₃; -CH₂Cl; -CHCl₂; -CCl₃; -OCH₃, -F, -Cl, -Br, NO₂, NH₂, and x is 0, 1 or 2;
or R⁵³ is 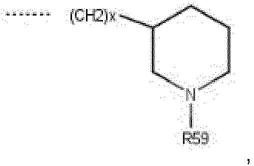 wherein R⁵⁹ is 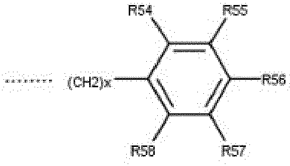 and wherein R⁵⁴, R⁵⁵, R⁵⁶, R⁵⁷ or R⁵⁸ are selected separately from the above from hydrogen, -CH₃, -C₂H₅, -CH₂F; -CHF₂; -CF₃; -CH₂Cl; -CHCl₂; -CCl₃;-OCH₃, -F, -Cl, -Br, NO₂, NH₂, and x is 0, 1 or 2; as defined above, and x is 0, 1 or 2; and
wherein preferably X is is selected from -CH₂-, -C₂H₄-, -C₃H₆-, -C(CH₃)₂-, - CHCH(CH₃)₂-, -CHC(CH₃)₃-, -CH(CH(CH₃)₂)-, -CH(CH₂CH(CH₃)₂)-, or -CH(CH₂OH)-;
and wherein preferably either all of R⁵⁴ to R⁵⁸ are hydrogen or 1, 2 or 3, preferably 1 or 2 of R⁵⁴ to R⁵⁸ are different from hydrogen and selected from substitutions presented above and remaining positions of R⁵⁴ to R⁵⁸ are selected from hydrogen;
c) a thiadiazole group of one of the following structure: 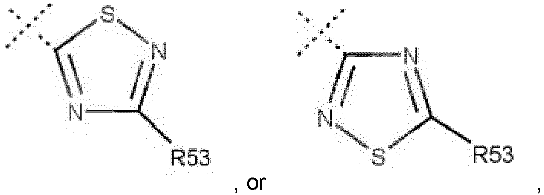
wherein R⁵³ is preferably as defined herein above, and
preferably X is selected from -CH₂-, -C₂H₄-, -C₃H₆-, -C(CH₃)₂-, -CHCH(CH₃)₂-, - CHC(CH₃)₃-, -CH(CH(CH₃)₂)-, -CH(CH₂CH(CH₃)₂)-, or -CH(CH₂OH)-;
d) an isothiazole group of the following structure: 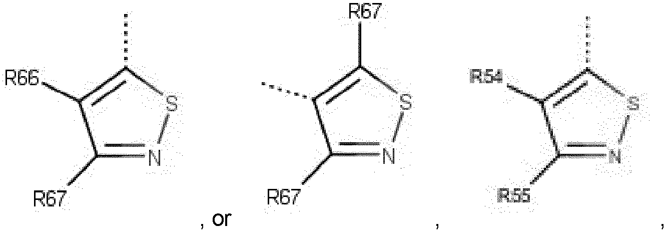 wherein R⁶⁶ and R⁶⁷ are each selected independently from hydrogen, -CH₃, -C₂H₅, -CH₂F; -CHF₂; -CF₃; -CH₂Cl; -CHCl₂; -CCl₃;-F, -Cl, -Br;
and X is preferably is selected from -CH₂-, -C₂H₄-, -C₃H₆-, -C(CH₃)₂-,-CHCH(CH₃)₂-, - CHC(CH₃)₃-, -CH(CH(CH₃)₂)-, -CH(CH₂CH(CH₃)₂)-, or -CH(CH₂OH)-; or
e) a thiophene group, preferably 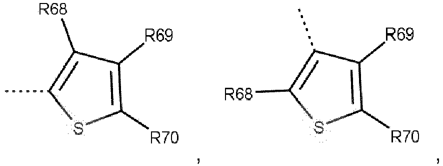 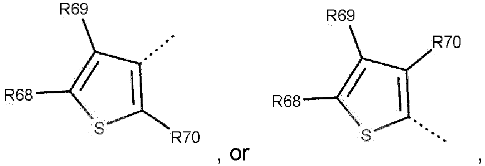 wherein R⁶⁸, R⁶⁹ and R⁷⁰ are each selected independently from hydrogen, -CH₃, -C₂H₅, -CH₂F; -CHF₂; -CF₃; -CH₂Cl; - CHCl₂; -CCl₃;-F, -Cl, -Br, preferably wherein either of R⁶⁸, R⁶⁹ or R⁷⁰ is hydrogen and the other residue is selected from -CH₃, -C₂H₅, -CF₃, -CHF₂, -F, -Cl, -Br, and preferably
X is -CH₂-, -C₂H₄-, -C₃H₆-, -C(CH₃)₂-, -CHCH(CH₃)₂-, -CHC(CH₃)₃-, -CH(CH(CH₃)₂)-, - CH(CH₂CH(CH₃)₂)-, or -CH(CH₂OH)-.

4. The compound for use according to claim 3, wherein R¹, R², R³, R⁴ and R⁵ are each independently hydrogen, -OCH₃, -OCF₃, -OCH₂CH₃, -OCH(CH₃)₂, - -CH₃, -C₂H₅, -Cl, -Br, -CN, -CH₂F; -CHF₂; -CF₃; -CH₂Cl; -CHCl₂; -CCl₃;NO₂, or 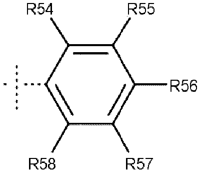 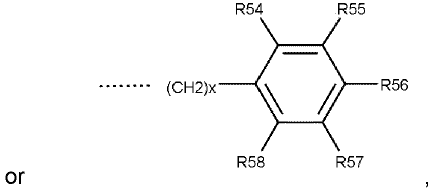 wherein R⁵⁴, R⁵⁵, R⁵⁶, R⁵⁷ or R⁵⁸ are selected each independently from hydrogen,
wherein preferably X is not present, and
wherein preferably either all of R¹ to R⁵ are hydrogen or 1, 2 or 3, preferably 1 or 2 of R¹ to R⁵ are different from hydrogen and selected from substitutions presented herein in claim 8 and remaining positions of R¹ to R⁵ are selected from hydrogen.

5. The compound for use according to any of claims 3 to 4, wherein R⁶ and R⁷ are each independently hydrogen, or R⁶ and R⁷ when taken together form a heterocyclic ring selected from:

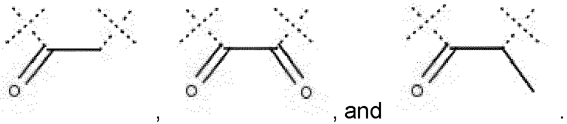

6. The compound for use according to any of claims 3 to 4, wherein R⁷, N and X when taken together form an six-membered heterocyclic ring, selected from 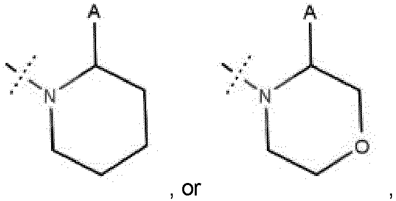 wherein A is as defined in claim 3.

7. The compound for use according to claims 3 to 6, wherein R¹, R², R³, R⁴ and R⁵ are each hydrogen and/or wherein A is 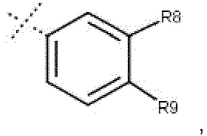 and R⁸ and R⁹ are identical or different to each other, preferably different from each other, and R⁸ and R⁹ are each independently selected from hydrogen; -OH, -OCH₃, -OCF₃, -OCHF₂, - OCH₂OH; -OCH₂CH₃; -OCH(CH₃)₂; -F; -Cl; -Br; -CN; NO₂; NH₂; -CH₃, -CH₂F; -CHF₂; -CF₃; -CH₂Cl; -CHCl₂; -CCl₃;CH(CH₃)₂; -CH₂CH₃; -CH-CH₂-CH(CH₃)₂; -CH₂-N(CH₃)₂; -CH(CH₃)-NHC(O)CH₃; - -NH-CH(CH₃)₂, -NH-cyclopropyl, -CHF₂, -CF₃, -CH₃, CH₂CH₃, CH₂CH(CH₃)₂; -C(O)-C₂H₅; -C(O)-CH₃; -C(O)-aziridine; -C(O)-azetidine; - C(O)-pyrrolidine; -C(CH₃)=NOH; -CH₂NHS(O)₂CH₃; -S(O)₂-cyclopentyl; -S(O)₂-CH₃;
-S(O)₂-cyclopentyl; pyrrolidinyl, 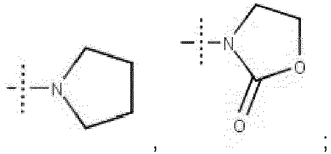
NHC(O)-R⁴¹, wherein R⁴¹ is selected from furan, tetrahydrofuran, cyclopentyl, cyclobutyl, cyclopropyl, -NH-CH(CH₃)₂, -NH-cyclopropyl, C₁-C₅ alkyl, -CH₂F; -CHF₂; - CF₃; -CH₂Cl; -CHCl₂; -CCl₃;-CH₃, CH₂CH₃, CH₂CH(CH₃)₂;
-C(O)OR⁴², wherein R⁴² is selected from -CH₃; -CH₂CH₃, -CH₂CH₂CH₃; -CH(CH₃)₂; - CH₂CH₂CH₂CH₃;
-NHR₄₃, wherein R⁴³ is selected from 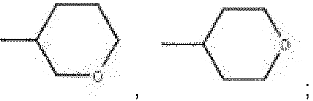 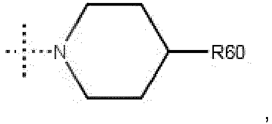 wherein R⁶⁰ is selected from hydrogen or -CH₃; 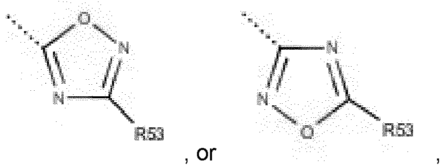
wherein
R⁵³ is selected from the group consisting of hydrogen, OH, -OCH₃, -OCF₃, -OCHF₂, - OCH₂OH; -OCH₂CH₃; -F; -Cl; -Br; -CN; NO₂; NH₂; -CH₃, CH(CH₃)₂; -CH₂CH₃; -CH-CH₂-CH(CH₃)₂; -CH₂-N(CH₃)₂; -CH(CH₃)-NHC(O)CH₃; -CH₂F; -CHF₂; -CF₃; -CH₂Cl; - CHCl₂; -CCl₃;-O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, -NH(C=O)NH₂, -NH(C=S)NH₂, -(C=O)NH₂,-CHO,-CHS, -OCHO, -NCHO, 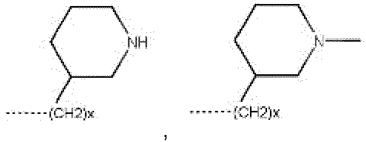 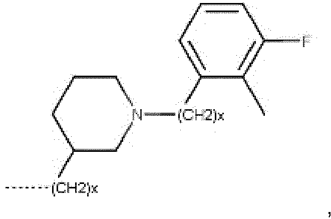 with X = 0 or 1, preferably 1; 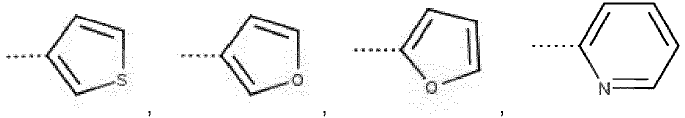 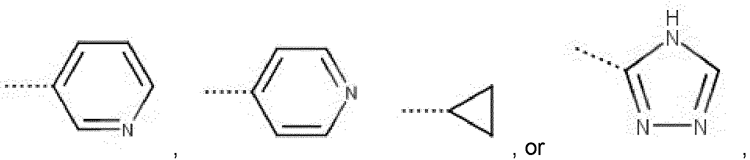 or
R⁵³ is 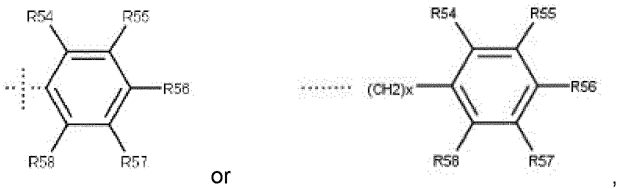 wherein R⁵⁴ R⁵⁵, R⁵⁶, R⁵⁷ or R⁵⁸ are selected each independently from hydrogen, -CH₃, -C₂H₅, -CH₂F; - CHF₂; -CF₃; -CH₂Cl; -CHCl₂; -CCl₃; -OCH₃, -F, -Cl, -Br, NH₂, NO₂, and x is 0, 1 or 2;
or R⁵³ is 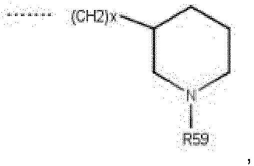 wherein R⁵⁹ is 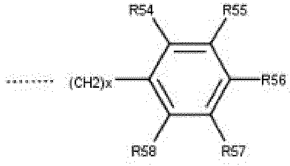 and
wherein R⁵⁴, R⁵⁵, R⁵⁶, R⁵⁷ or R⁵⁸ are selected separately from the above from hydrogen, -CH₃, -C₂H₅, -CH₂F; -CHF₂; -CF₃; -CH₂Cl; -CHCl₂; -CCl₃;-OCH₃, -F, -Cl, - Br, NO₂, NH₂, and x is 0, 1 or 2; as defined above; or optionally
R⁸ and R⁹, when taken together form an five- or six-membered cycloalkyl, five- or six-membered cycloheteroalkyl or five or six-membered aryl, five- or six-membered heteroaryl, preferably the heterocyclic ring is selected from:

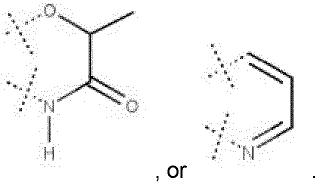

8. The compound for use according to claim 7, with the proviso that R⁸ or R⁹ are not identical to each other.

9. The compound for use according to any of claims 3 to 8, wherein R¹ to R⁵ are selected as follows:
R¹ is hydrogen, -C₁-C₂ alkoxy, -C(O)O-C₁-C₅ alkyl, -OCH₃, -C₁-C₃ alkyl, methyl, or ethyl;
R² is hydrogen, methyl, -F, -Cl, -Br, -CN, -OCH₃, or -C(O)O-C₁-C₅ alkyl;
R³ is hydrogen, methyl, -CH(CH₃)₂, -CHF₂, -F, -Cl, -Br, -CN, -OCH₃, -OCF₃, NO₂, -or C(O)O-C₁-C₅ alkyl,
R⁴ is hydrogen or -F, -Cl, -Br, or -CF₃; and/or
R⁵ is hydrogen, methyl, ethyl or -F, -Cl, -Br, or -CN, -CH₂-phenyl.

10. The compound for use according to any of claims 3 to 6, wherein the compound is a compound according to any of Formulae (Ib), (Ib1), (Ib2) or (Ib3): 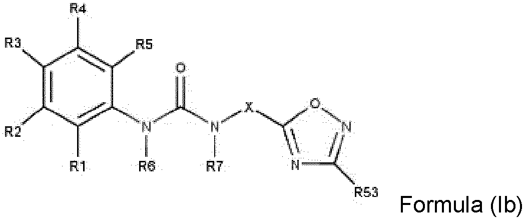 or 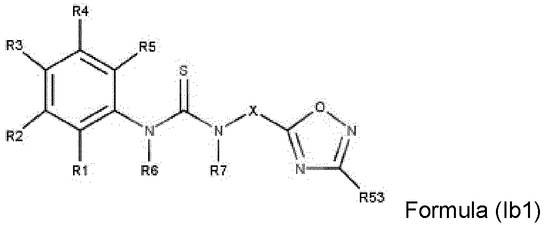 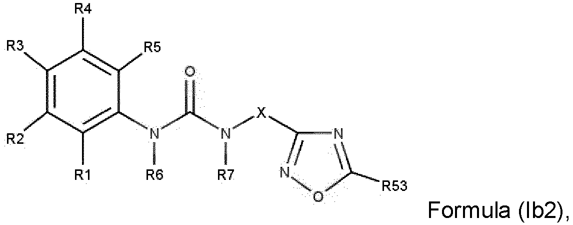 or 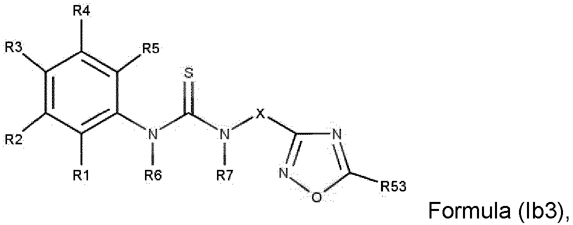
wherein X is selected from the group consisting of a C₁-C₃ alkyl,-CH(CH₃)₂ -CH₂-, - C₂H₄-, -C₃H₆-, -CHCH(CH₃)₂-, -CHC(CH₃)₃-, -CH(CH(CH₃)₂)-, or -CH(CH₂CH(CH₃)₂)-, or -CH(CH₂OH)-, or is not present, and
wherein R⁵³ is selected from the group consisting of hydrogen, OH, -OCH₃, -OCF₃, - OCHF₂, -OCH₂OH; -OCH₂CH₃; -F; -Cl; -Br; -CN; NO₂; NH₂; C₁-C₅ alkyl, C₁-C₅ alkoxy, C₂-C₅ alkenyl, preferably -CH₃, CH(CH₃)₂; -CH₂CH₃; -CH-CH₂-CH(CH₃)₂; - CH₂-N(CH₃)₂; -CH(CH₃)-NHC(O)CH₃; -CH₂F; -CHF₂; -CF₃; -CH₂Cl; -CHCl₂; -CCl₃;-ON=O, -N=O, -CH=NH, -N₃, -SH, -SO₃H, -SO₂H, -S(O)₂CH₃ -CN, -NC,-OCN, -NCO, - (C=O)OH, -(C=O)NHOH, -NH(C=O)NH₂, -NH(C=S)NH₂, -(C=O)NH₂,-CHO,-CHS, - OCHO, -NCHO, 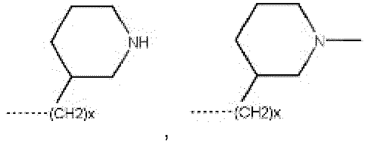 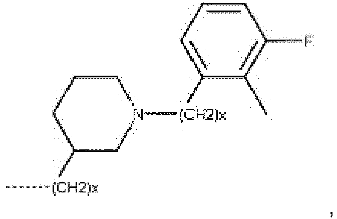 with X = 0 or 1, preferably 1; 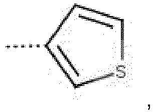 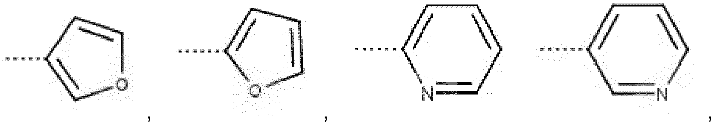 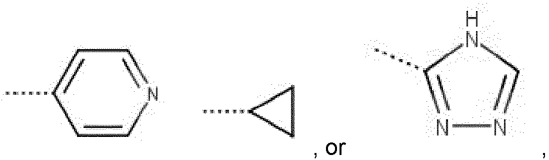 or
R⁵³ is 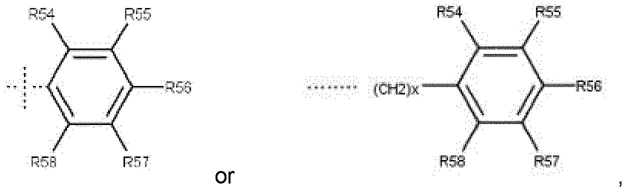 wherein R^{54,} R⁵⁵, R⁵⁶, R⁵⁷ or R⁵⁸ are selected each independently from hydrogen, -CH₃, -C₂H₅, - CH₂F; -CHF₂; -CF₃; -CH₂Cl; -CHCl₂; -CCl₃; -OCH₃, -F, -Cl, -Br, NH₂, NO₂, and x is 0, 1 or 2;
or R⁵³ is 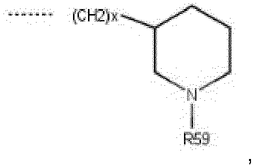 wherein R⁵⁹ is 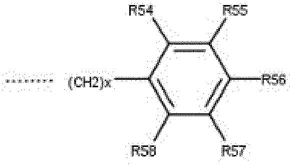 and
wherein R^{54,} R⁵⁵, R⁵⁶, R⁵⁷ or R⁵⁸ are selected separately from the above from hydrogen, -CH₃, -C₂H₅, -CH₂F; -CHF₂; -CF₃; -CH₂Cl; -CHCl₂; -CCl₃;-OCH₃, -F, -Cl, - Br, NO₂, NH₂, and x is 0, 1 or 2; as defined above, and x is 0, 1 or 2;
and wherein preferably either all of R⁵⁴ to R⁵⁸ are hydrogen or 1, 2 or 3, preferably 1 or 2 of R⁵⁴ to R⁵⁸ are different from hydrogen and selected from substitutions presented above and remaining positions of R⁵⁴ to R⁵⁸ are selected from hydrogen.

11. The compound for use according to claim 1 , wherein the compound is a compound of Formula (Id): 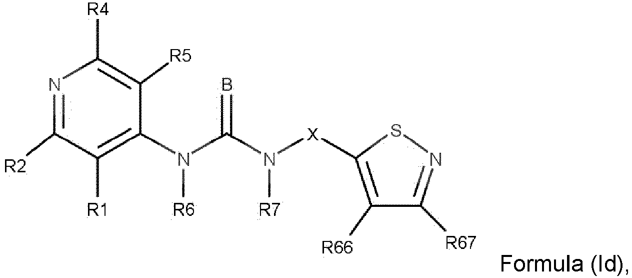 wherein preferably
B is O or S, preferably O,
X is selected from C₁-C₅-alkyl, -CH₂-, -C₂H₄-, -C₃H₆-, -C(CH₃)₂-, -CHCH(CH₃)₂-, - CHC(CH₃)₃-, -CH(CH(CH₃)₂)-, -CH(CH₂CH(CH₃)₂)-, or -CH(CH₂OH)-;
R¹, R², R⁴ and R⁵ are selected separately from hydrogen, -CH₃, -C₂H₅, -CH₂CH₃-CH₂F; -CHF₂; -CF₃; -CH₂Cl; -CHCl₂; -CCl₃; -OCH₃, -F, -Cl, -Br, NO₂, NH₂, preferably wherein two or three of R¹, R², R⁴ and R⁵ are hydrogen and the remaining are not hydrogen; and
R⁵⁴ and R⁵⁵ are each selected separately from hydrogen, -CH₃, -C₂H₅, -CH₂F; - CHF₂; -CF₃; -CH₂Cl; -CHCl₂; -CCl₃;-OCH₃, -F, -Cl, -Br, NO₂, NH₂, preferably wherein one of R⁵⁴ and R⁵⁵ is hydrogen and the other is one of -CH₃, -C₂H₅, -CH₂F; -CHF₂; - CF₃; -CH₂Cl; -CHCl₂; -CCl₃;-OCH₃, -F, -Cl, -Br, NO₂, NH₂.

12. The compound for use according to any of claims 3 to 6 and 10 to 11, wherein the compound is a compound according to any of Formulae (Ib), (Ib1), (Ib2), (Ib3) or (Id) and R¹ to R⁵ are selected as follows:
R' is hydrogen, -C₁-C₂ alkoxy, -C(O)O-C₁-C₅ alkyl, -OCH₃, -C₁-C₃ alkyl, methyl, or ethyl
R² is hydrogen, methyl, -F, -Cl, -Br, -CN, -OCH₃, or -C(O)O-C₁-C₅ alkyl;
R³ , if present, is hydrogen, methyl, -CH(CH₃)₂, -CHF₂, -F, -Cl, -Br, -CN, -OCH₃, - OCF₃, NO₂, or -C(O)O-(C₁-C₅ alkyl);
R⁴ is hydrogen or -F, -Cl, -Br, or -CF₃; and/or
R⁵ is hydrogen, methyl, ethyl or -F, -Cl, -Br, -CN, or -CH₂-phenyl.

13. The compound for use according to any of claims 1 to 12, wherein the compound is selected from the compounds delineated in Table 1.
**Table 1:**
| **No.** | **Code** | **Structure** | **IUPAC-Name** |
|---|---|---|---|
| **1** | EN22 | 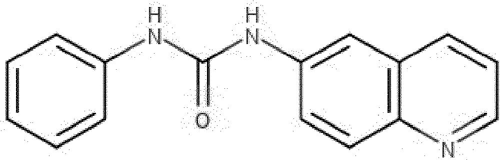 | 1-phenyl-3-(quinolin-6-yl)urea |
| **2** | EN21 | 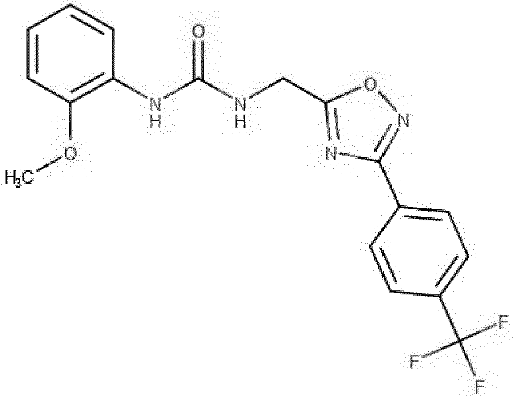 | 1-(2-methoxyphenyl)-3-((3-(4-(trifluoromethyl)p henyl)-1,2,4-oxadiazol-5-yl)methyl)urea |
| **3** | EN-A (EN23) | 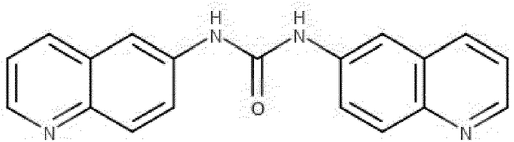 | 1,3-di(quinolin-6-yl)urea |
| **4** | AH4 | 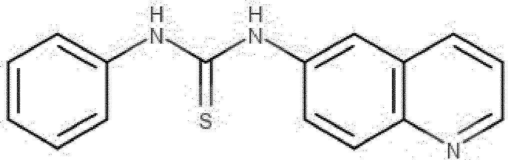 | 3-phenyl-1-(quinolin-6-yl)thiourea |
| **5** | JB010 | 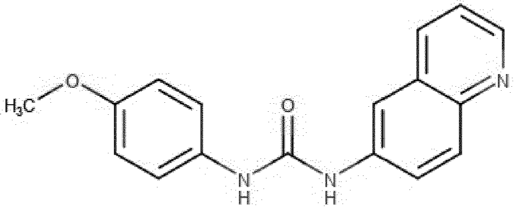 | 1-(4-methoxyphenyl)-3-(quinolin-6-yl)urea |
| **6** | BRM/BR G1 ATP Inhibitor-1 | 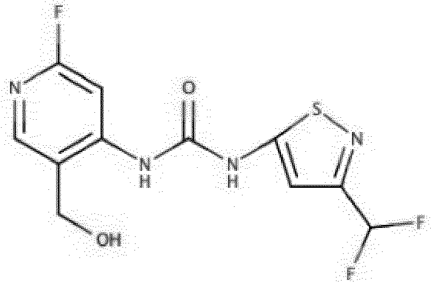 | 1-[3-(difluoromethyl)-1,2-thiazol-5-yl]-3-[2-fluoro-5-(hydroxymethyl)p yridin-4-yl]urea |
| **7** | JB011 | 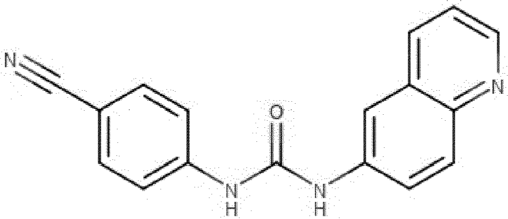 | 1-(4-cyanophenyl)-3-(quinolin-6-yl)urea |
| **8** | PM07 | 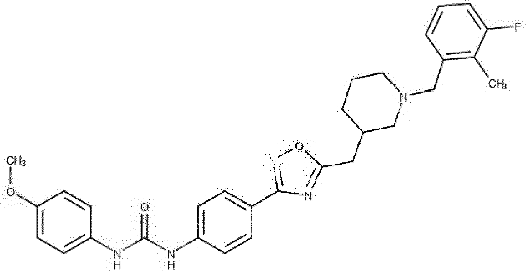 | 3-{4-[5-({1-[(3-fluoro-2-methylphenyl)me thyl]piperidin-3-yl}methyl)-1,2,4-oxadiazol-3-yl]phenyl}-1-(4-methoxyphenyl)u rea |
| **9** | PM16 | 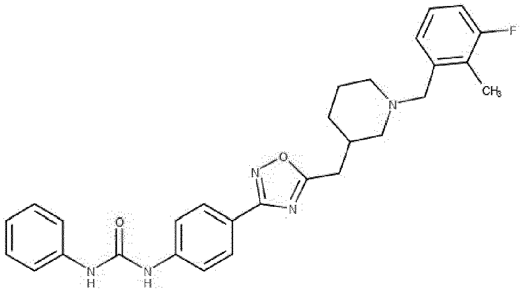 | 3-{4-[5-({1-[(3-fluoro-2-methylphenyl)me thyl]piperidin-3-yl}methyl)-1,2,4-oxadiazol-3-yl]phenyl}-1-phenylurea |
| **10** | JB014 | 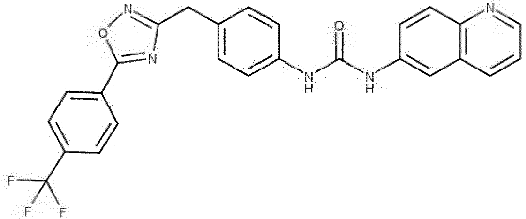 | 1-(quinolin-6-yl)-3-[4-({5-[4-(trifluoromethyl)p henyl]-1,2,4-oxadiazol-3-yl}methyl)phenyl] urea |
| **11** | AH1 | 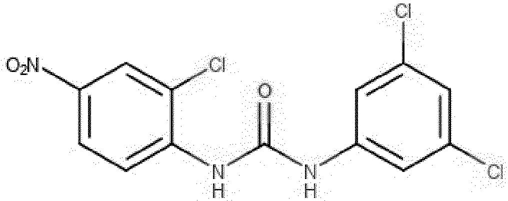 | 3-(2-chloro-4-nitrophenyl)-1-(3,5-dichlorophenyl)ur ea |
| **12** | AH3 | 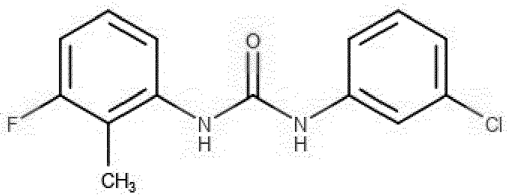 | 1-(3-chlorophenyl)-3-(3-fluoro-2-methylphenyl)ure a |
| **13** | JB002 | 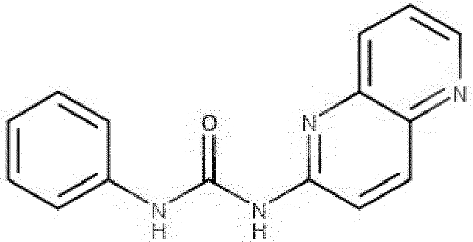 | 1-(1,5-naphthyridin-2-yl)-3-phenylurea |
| **14** | JB004 | 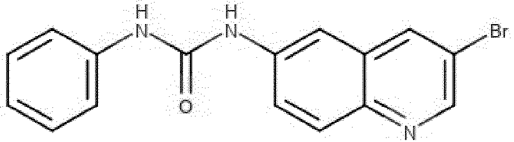 | 1-(3-bromoquinolin-6-yl)-3-phenylurea |
| **15** | EN11-16 | 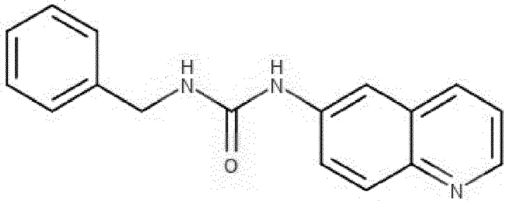 | 1-benzyl-3-(quinolin-6-yl)urea |
| **16** | EN11-13 | 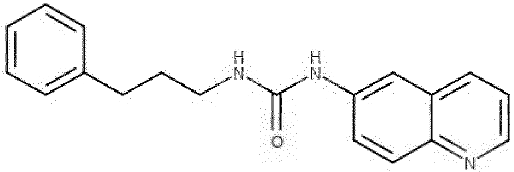 | 1-(3-phenylpropyl)-3-(quinolin-6-yl)urea |
| **17** | L01.110 | 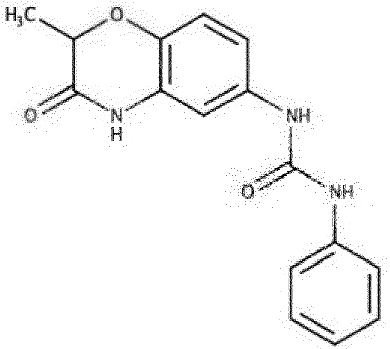 | 3-(2-methyl-3-oxo-3,4-dihydro-2H-1,4-benzoxazin-6-yl)-1-phenylurea |
| **18** | L01.041 | 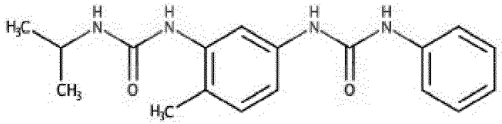 | 3-(4-methyl-3-{[(propan-2-yl)carbamoyl]ami no}phenyl)-1-phenylurea |
| **19** | L01.004 | 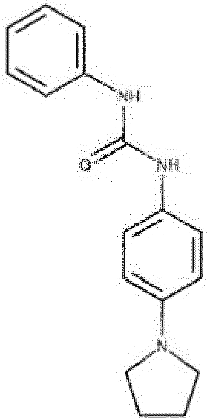 | 3-phenyl-1-[4-(pyrrolidin-1-yl)phenyl]urea |
| **20** | L01.007 | 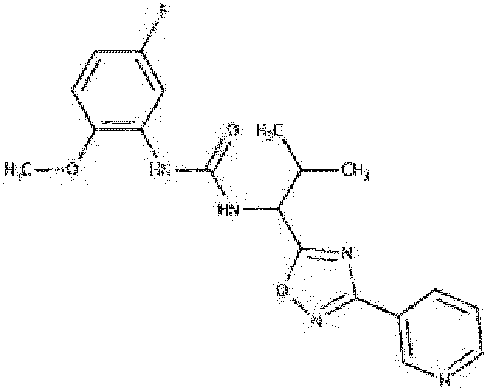 | 1-(5-fluoro-2-methoxyphenyl)-3-{2-methyl-1-[3-(pyridin-3-yl)-1,2,4-oxadiazol-5-yl]propyl}urea |
| **21** | L01.104 | 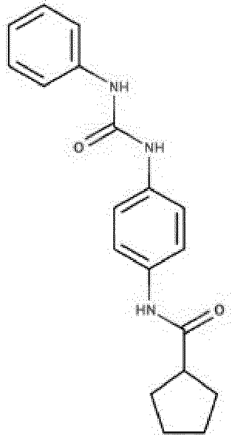 | N-{4-[(phenylcarbamo yl)amino]phenyl} cyclopentanecar boxamide |
| **22** | L01.116 | 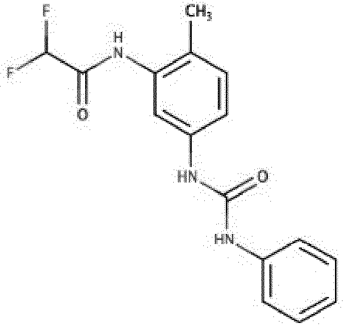 | 2,2-difluoro-N-{2-methyl-5-[(phenylcarbamo yl)amino]phenyl} acetamide |
| **23** | L01.113 | 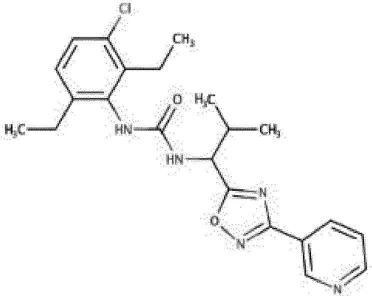 | 1-(3-chloro-2,6-diethylphenyl)-3-{2-methyl-1-[3-(pyridin-3-yl)-1,2,4-oxadiazol-5-yl]propyl}urea |
| **24** | L01.101 | 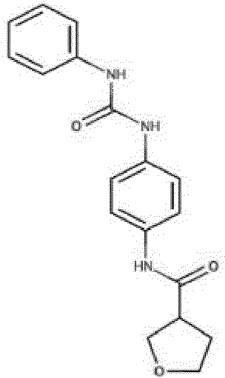 | N-{4-[(phenylcarbamo yl)amino]phenyl} oxolane-3-carboxamide |
| **25** | L01.092 | 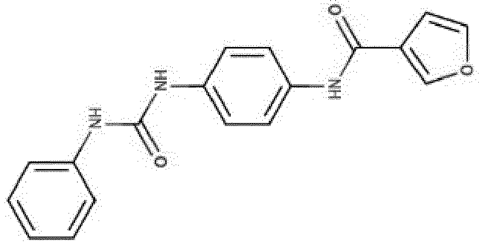 | N-{4-[(phenylcarbamo yl)amino]phenyl}f uran-3-carboxamide |
| **26** | L01.124 | 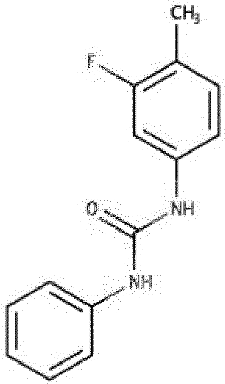 | 3-(3-fluoro-4-methylphenyl)-1-phenylurea |
| **27** | TCMDC-143175 | 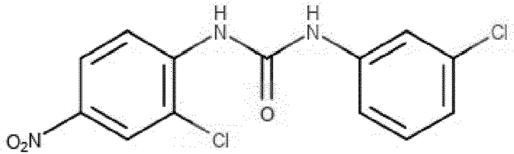 | 3-(2-chloro-4-nitrophenyl)-1-(3-chlorophenyl)ure a |
| **28** | A.C2 | 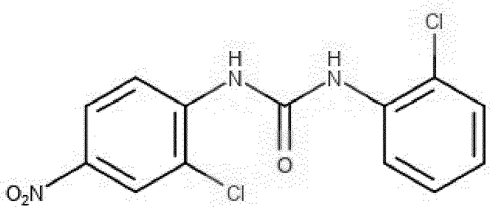 | 3-(2-chloro-4-nitrophenyl)-1-(2-chlorophenyl)ure a |
| **29** | AH2 | 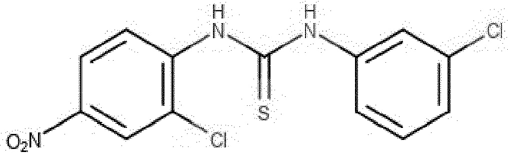 | 3-(2-chloro-4-nitrophenyl)-1-(3-chlorophenyl)thio urea |
| **30** | EN11-07 | 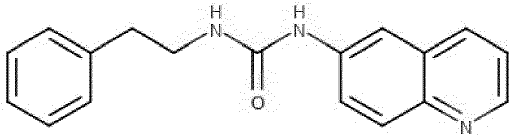 | 1-(2-phenylethyl)-3-(quinolin-6-yl)urea |
| **31** | EN11-02 | 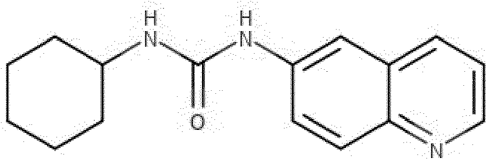 | 1-cyclohexyl-3-(quinolin-6-yl)urea |
| **32** | EN11-11 | 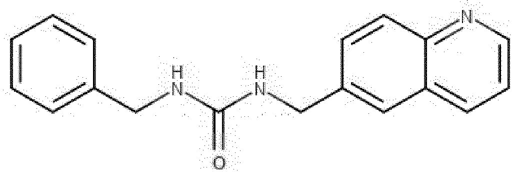 | 3-benzyl-1-[(quinolin-6-yl)methyl]urea |
| **33** | L01.016 | 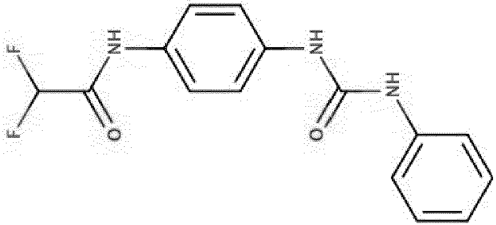 | 2,2-difluoro-N-{4-[(phenylcarbamo yl)amino]phenyl} acetamide |
| **34** | L01.028 | 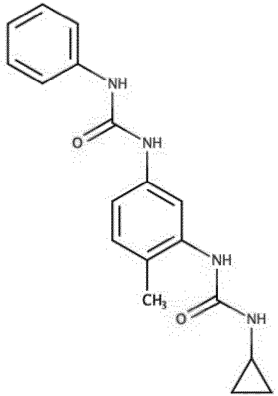 | 3-cyclopropyl-1-{2-methyl-5-[(phenylcarbamo yl)amino]phenyl} urea |
| **35** | L01.047 | 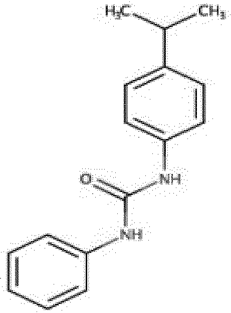 | 1-phenyl-3-[4-(propan-2-yl)phenyl]urea |
| **36** | L01.026 | 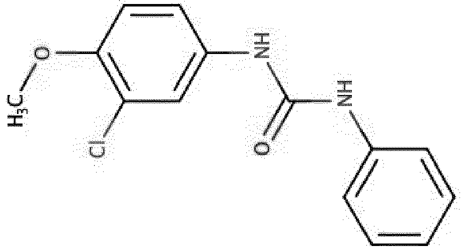 | 3-(3-chloro-4-methoxyphenyl)-1-phenylurea |
| **37** | L01.127 | 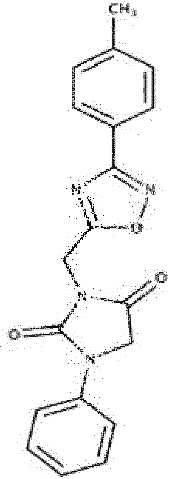 | 3-{[3-(4-methylphenyl)-1,2,4-oxadiazol-5-yl]methyl}-1-phenylimidazolidi ne-2,4-dione |
| **38** | L01.061 | 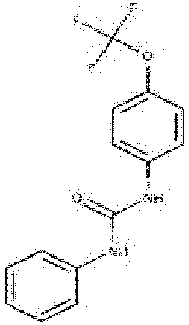 | 1-phenyl-3-[4-(trifluoromethoxy )phenyl]urea |
| **39** | L01.128 | 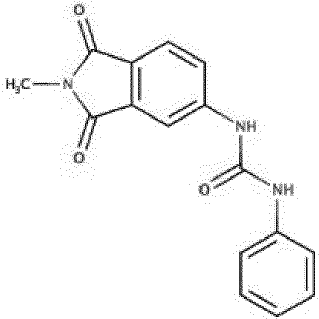 | 3-(2-methyl-1,3-dioxo-2,3-dihydro-1H-isoindol-5-yl)-1-phenylurea |
| **40** | L01.025 | 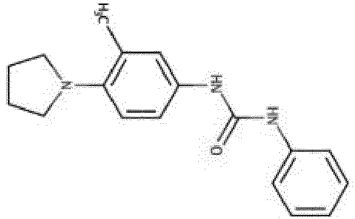 | 3-[3-methyl-4-(pyrrolidin-1-yl)phenyl]-1-phenylurea |
| **41** | L01.008 | 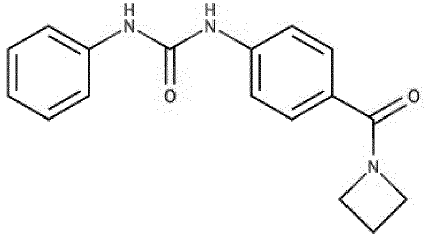 | 1-[4-(azetidine-1-carbonyl)phenyl]-3-phenylurea |
| **42** | L01.089 | 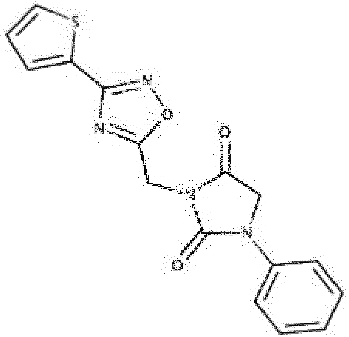 | 1-phenyl-3-{[3-(thiophen-2-yl)-1,2,4-oxadiazol-5-yl]methyl}imidaz olidine-2,4-dione |
| **43** | L01.130 | 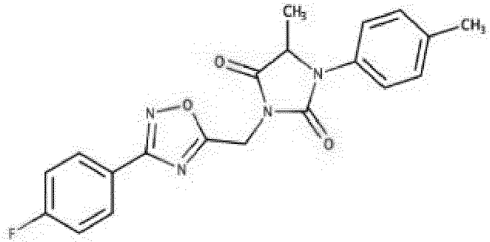 | 3-{[3-(4-fluorophenyl)-1,2,4-oxadiazol-5-yl]methyl}-5-methyl-1-(4-methylphenyl)imi dazolidine-2,4-dione |
| **44** | L01.049 | 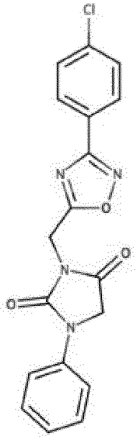 | 3-{[3-(4-chlorophenyl)-1,2,4-oxadiazol-5-yl]methyl}-1-phenylimidazolidi ne-2,4-dione |
| **45** | L01.066 | 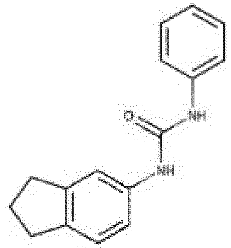 | 3-(2,3-dihydro-1H-inden-5-yl)-1-phenylurea |
| **46** | L01.043 | 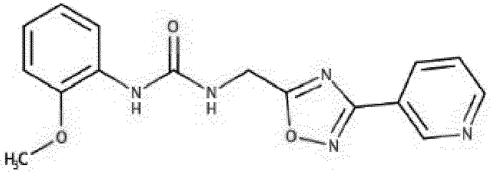 | 1-(2-methoxyphenyl)-3-{[3-(pyridin-3-yl)-1,2,4-oxadiazol-5-yl]methyl}urea |
| **47** | L01.087 | 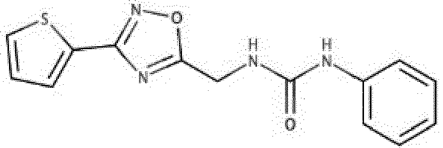 | 1-phenyl-3-{[3-(thiophen-2-yl)-1,2,4-oxadiazol-5-yl]methyl}urea |
| **48** | L01.020 | 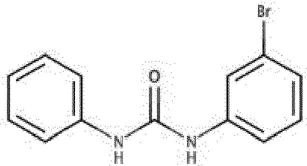 | 3-(3-bromophenyl)-1-phenylurea |
| **49** | L01.035 | 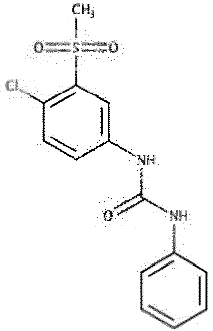 | 3-(4-chloro-3-methanesulfonyl phenyl)-1-phenylurea |
| **50** | L01.120 | 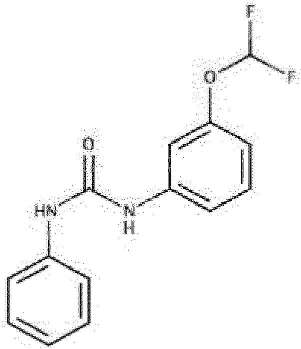 | 3-[3-(difluoromethoxy )phenyl]-1-phenylurea |
| **51** | L01.033 | 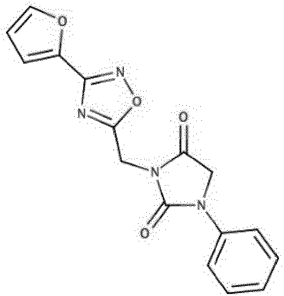 | 3-{[3-(furan-2-yl)-1,2,4-oxadiazol-5-yl]methyl}-1-phenylimidazolidi ne-2,4-dione |
| **52** | L01.002 | 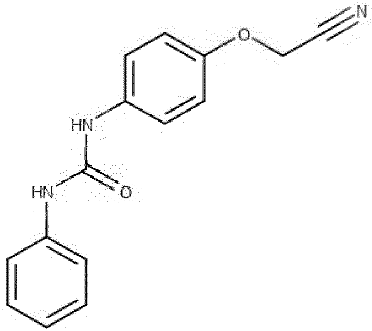 | 1-[4-(cyanomethoxy) p henyl]-3-phenylurea |
| **53** | L01.027 | 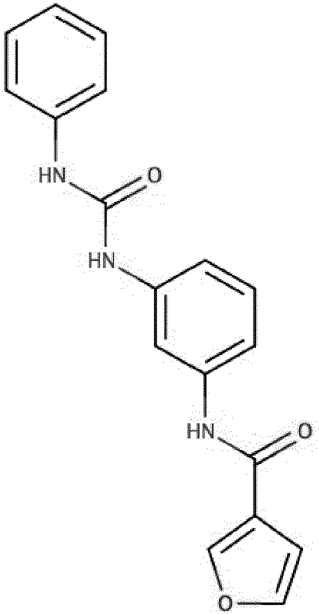 | N-{3-[(phenylcarbamo yl)amino]phenyl}f uran-3-carboxamide |
| **54** | L01.003 | 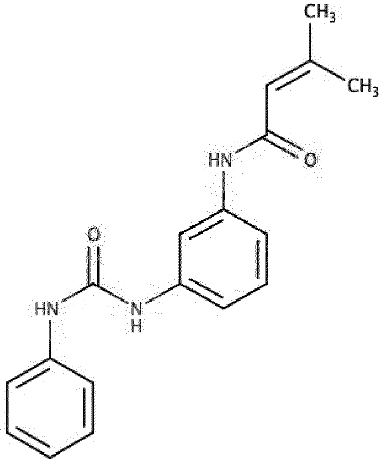 | 3-methyl-N-{3-[(phenylcarbamo yl)amino]phenyl} but-2-enamide |
| **55** | L01.038 | 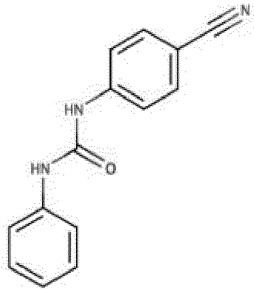 | 1-(4-cyanophenyl)-3-phenylurea |
| | | | |
| **56** | L01.069 | 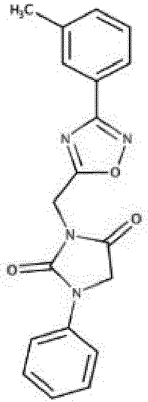 | 3-{[3-(3-methylphenyl)-1,2,4-oxadiazol-5-yl]methyl}-1-phenylimidazolidi ne-2,4-dione |
| **57** | L01.097 | 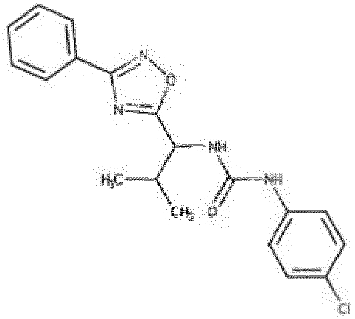 | 1-(4-chlorophenyl)-3-[2-methyl-1-(3-phenyl-1,2,4-oxadiazol-5-yl)propyl]urea |
| **58** | L01.095 | 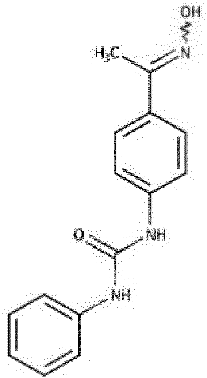 | 3-{4-[1-(hydroxyimino)et hyl]phenyl}-1-phenylurea |
| **59** | L01.013 | 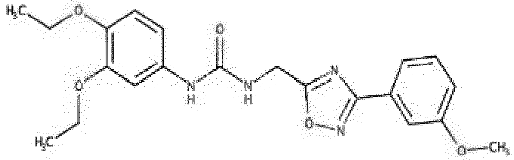 | 1-(3,4-diethoxyphenyl)-3-{[3-(3-methoxyphenyl)-1,2,4-oxadiazol-5-yl]methyl}urea |
| **60** | L01.103 | 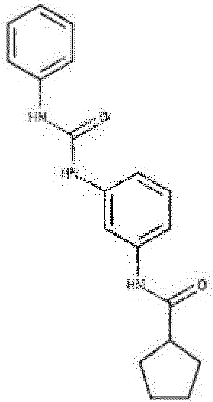 | N-{3-[(phenylcarbamo yl)amino]phenyl} cyclopentanecar boxamide |
| **61** | L01.071 | 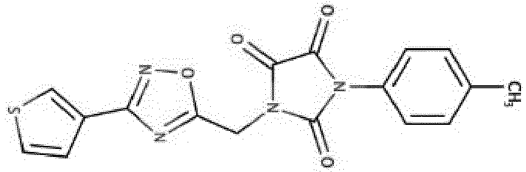 | 1-(4-methylphenyl)-3-{[3-(thiophen-3-yl)-1,2,4-oxadiazol-5-yl]methyl}imidaz olidine-2,4,5-trione |
| **62** | L01.115 | 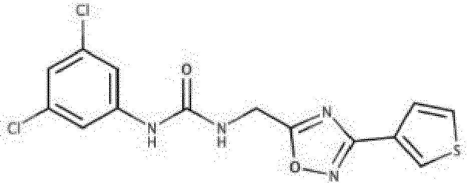 | 1-(3,5-dichlorophenyl)-3-{[3-(thiophen-3-yl)-1,2,4-oxadiazol-5-yl]methyl}urea |
| **63** | L01.045 | 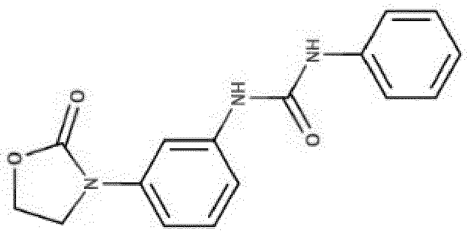 | 1-[3-(2-oxo-1,3-oxazolidin-3-yl)phenyl]-3-phenylurea |
| **64** | L01.077 | 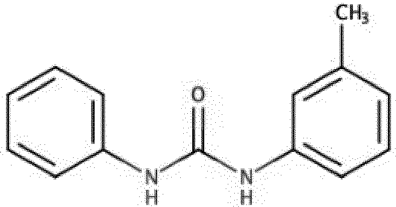 | 3-(3-methylphenyl)-1-phenylurea |
| **65** | L01.084 | 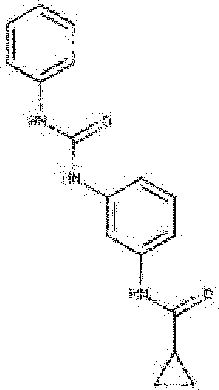 | N-{3-[(phenylcarbamo yl)amino]phenyl} cyclopropanecar boxamide |
| **66** | L01.109 | 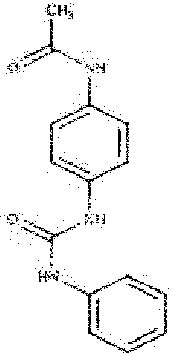 | N-{4-[(phenylcarbamo yl)amino]phenyl} acetamide |
| **67** | L01.122 | 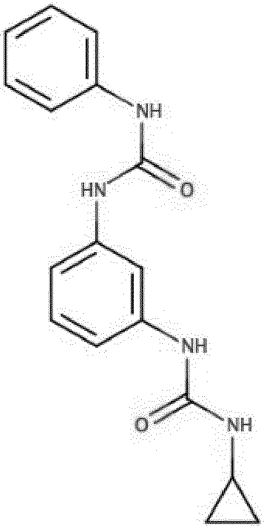 | 3-cyclopropyl-1-{3-[(phenylcarbamo yl)amino]phenyl} urea |
| **68** | L01.083 | 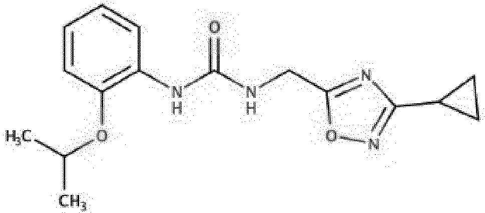 | 3-[(3-cyclopropyl-1,2,4-oxadiazol-5-yl)methyl]-1-[2-(propan-2-yloxy)phenyl]ure a |
| **69** | L01.062 | 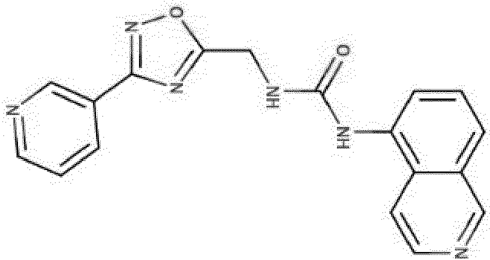 | 1-(isoquinolin-5-yl)-3-{[3-(pyridin-3-yl)-1,2,4-oxadiazol-5-yl]methyl}urea |
| **70** | L01.081 | 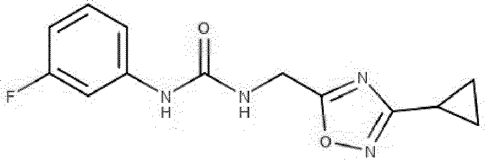 | 3-[(3-cyclopropyl-1,2,4-oxadiazol-5-yl)methyl]-1-(3-fluorophenyl)ure a |
| **71** | L01.114 | 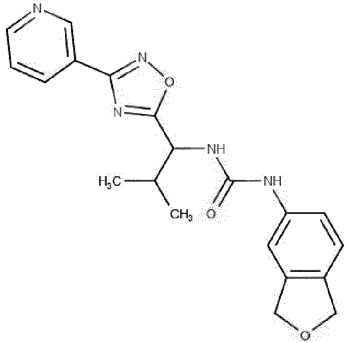 | 3-(1,3-dihydro-2-benzofuran-5-yl)-1-{2-methyl-1-[3-(pyridin-3-yl)-1,2,4-oxadiazol-5-yl]propyl}urea |
| **72** | L01.090 | 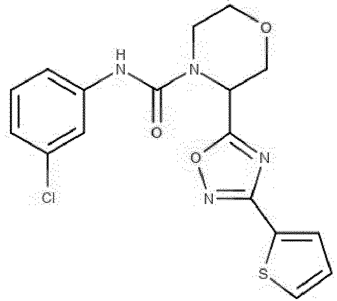 | N-(3-chlorophenyl)-3-[3-(thiophen-2-yl)-1,2,4-oxadiazol-5-yl]morpholine-4-carboxamide |
| **73** | L01.136 | 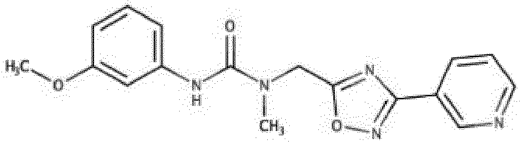 | 1-(3-methoxyphenyl)-3-methyl-3-{[3-(pyridin-3-yl)-1,2,4-oxadiazol-5-yl]methyl}urea |
| **74** | L01.023 | 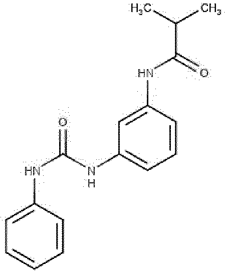 | 2-methyl-N-{3-[(phenylcarbamo yl)amino]phenyl} propanamide |
| **75** | L01.064 | 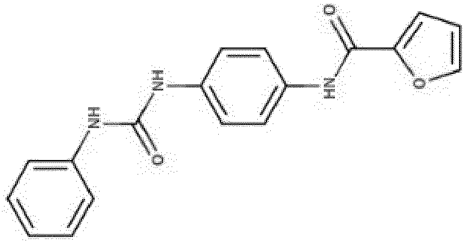 | N-{4-[(phenylcarbamo yl)amino]phenyl}f uran-2-carboxamide |
| **76** | L01.093 | 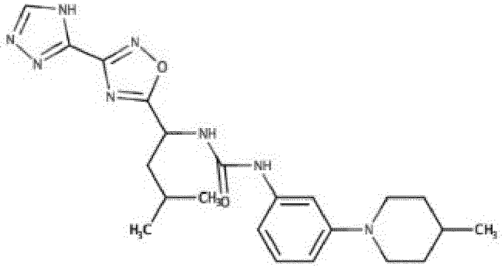 | 3-{3-methyl-1-[3-(4H-1,2,4-triazol-3-yl)-1,2,4-oxadiazol-5-yl]butyl}-1-[3-(4-methylpiperidin-1-yl)phenyl]urea |
| **77** | L01.125 | 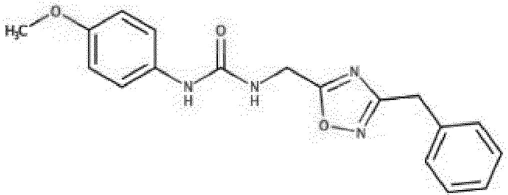 | 3-[(3-benzyl-1,2,4-oxadiazol-5-yl)methyl]-1-(4-methoxyphenyl)u rea |
| **78** | L01.005 | 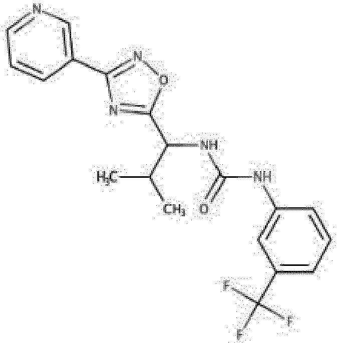 | 3-{2-methyl-1-[3-(pyridin-3-yl)-1,2,4-oxadiazol-5-yl]propyl}-1-[3-(trifluoromethyl)p henyl]urea |
| **79** | L01.135 | 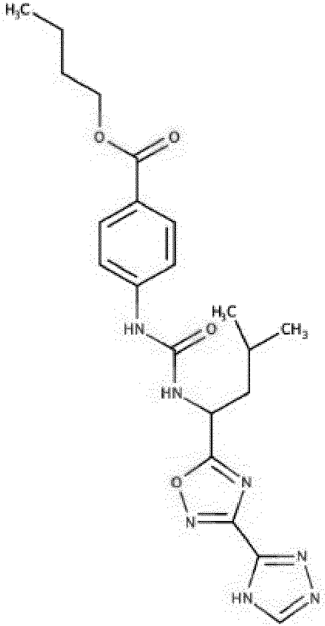 | butyl 4-[({3-methyl-1-[3-(4H-1,2,4-triazol-3-yl)-1,2,4-oxadiazol-5-yl]butyl}carbamo yl)amino]benzoat e |
| **80** | L01.108 | 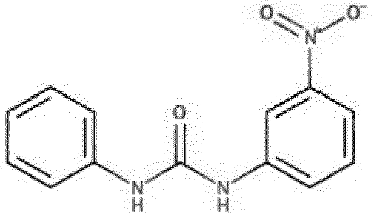 | 3-(3-nitrophenyl)-1-phenylurea |
| **81** | L01.112 | 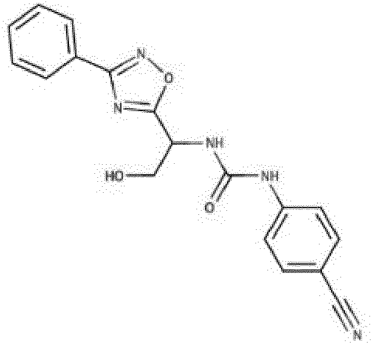 | 1-(4-cyanophenyl)-3-[2-hydroxy-1-(3-phenyl-1,2,4-oxadiazol-5-yl)ethyl]urea |
| **82** | L01.054 | 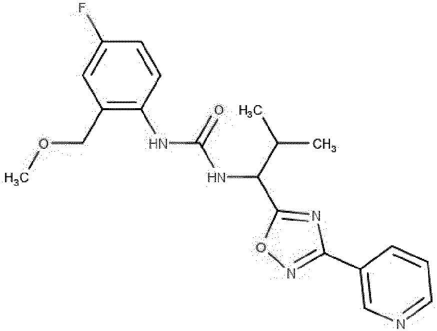 | 1-[4-fluoro-2-(methoxymethyl) phenyl]-3-{2-methyl-1-[3-(pyridin-3-yl)-1,2,4-oxadiazol-5-yl]propyl}urea |
| **83** | L01.067 | 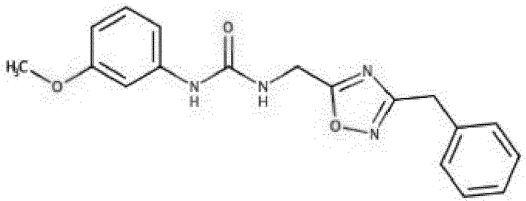 | 3-[(3-benzyl-1,2,4-oxadiazol-5-yl)methyl]-1-(3-methoxyphenyl)u rea |
| **84** | L01.099 | 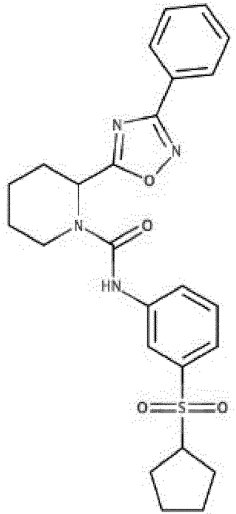 | N-[3-(cyclopentanesul fonyl)phenyl]-2-(3-phenyl-1,2,4-oxadiazol-5-yl)piperidine-1-carboxamide |
| **85** | L01.065 | 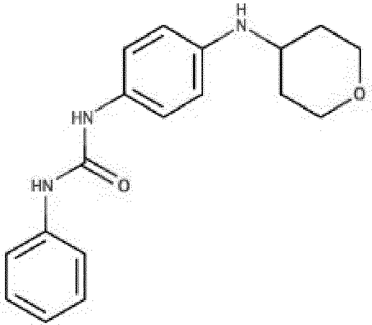 | 1-{4-[(oxan-4-yl)amino]phenyl}-3-phenylurea |
| **86** | L01.132 | 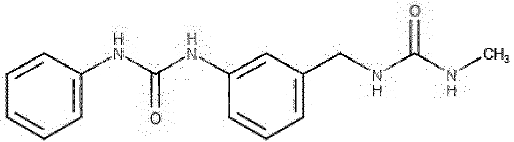 | 3-(3-{[(methylcarbam oyl)amino]methyl }phenyl)-1-phenylurea |
| **87** | L01.121 | 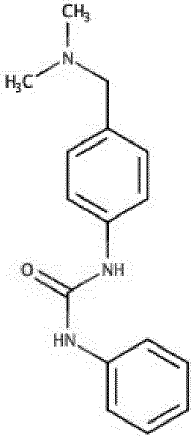 | 3-{4-[(dimethylamino) methyl]phenyl}-1-phenylurea |
| **88** | L01.056 | 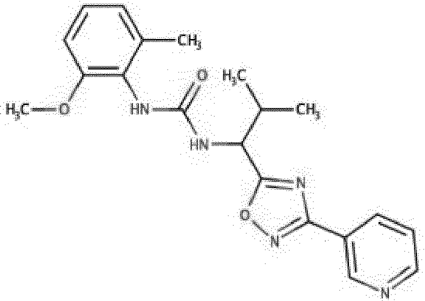 | 1-(2-methoxy-6-methylphenyl)-3-{2-methyl-1-[3-(pyridin-3-yl)-1,2,4-oxadiazol-5-yl]propyl}urea |
| **89** | L01.088 | 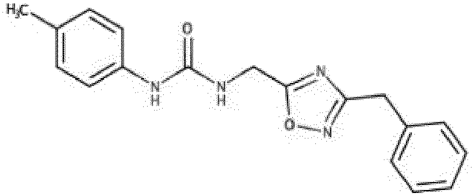 | 3-[(3-benzyl-1,2,4-oxadiazol-5-yl)methyl]-1-(4-methylphenyl)ure a |
| **90** | L01.042 | 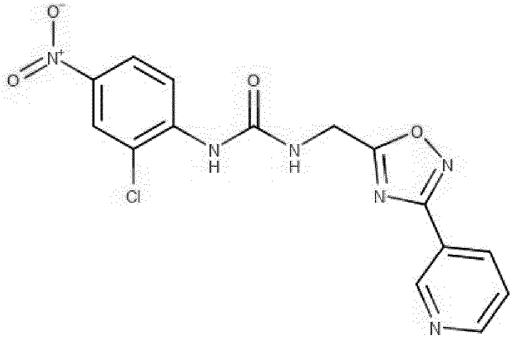 | 1-(2-chloro-4-nitrophenyl)-3-{[3-(pyridin-3-yl)-1,2,4-oxadiazol-5-yl]methyl}urea |
| **91** | L01.010 | 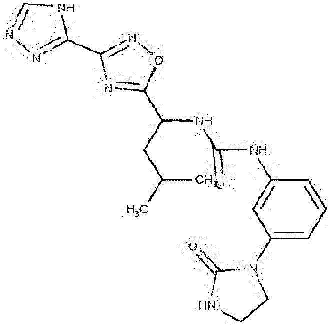 | 3-{3-methyl-1-[3-(4H-1,2,4-triazol-3-yl)-1,2,4-oxadiazol-5-yl]butyl}-1-[3-(2-oxoimidazolidin-1-yl)phenyl]urea |

14. A compound of any of claims 1 to 13 for use in the prevention or treatment of a disease by activating NLRP3 inflammasome, wherein the disease is selected from a group comprising infectious diseases or cancer, or for use for boosting immune responses or breaking immune evasion in cancer diseases in a subject, or for use as an adjuvant in the context of vaccination to boost specific immune responses by activating NLRP3 inflammasome.

15. Use of the compounds as defined in claim 13 for analyzing the activity of NLRP3 activation *in vitro.*

## Patentansprüche

1. Verbindung zur Verwendung bei der Vorbeugung oder Behandlung einer Krankheit durch Aktivierung des NLRP3-Inflammasoms, wobei die Krankheit aus einer Gruppe ausgewählt ist, die Infektionskrankheiten oder Krebs umfasst, und
(I) wobei die Verbindung eine Verbindung der Formel (I) 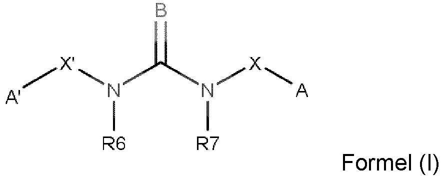 oder ein pharmazeutisch annehmbares Salz davon ist, wobei:
B ausgewählt ist aus O oder S, vorzugsweise O;
X und X' jeweils unabhängig voneinander vorhanden sind oder nicht, wobei X und X' jeweils unabhängig voneinander eine C₁-C₅-Alkylgruppe oder -CH(CH₂OH)- sind; und
R⁶ und R⁷ jeweils unabhängig voneinander Wasserstoff oder ein C₁-C₃-Alkyl sind, vorzugsweise -H, -CH₃, -C₂H₅, -C₃H₇ oder -C₃H₉, oder optional
R⁶ und R⁷ zusammen einen fünfgliedrigen heterocyclischen Ring bilden, ausgewählt aus 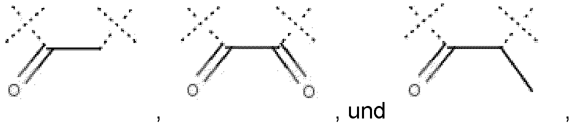 oder optional,
R⁷, N und X zusammen einen sechsgliedrigen heterocyclischen Ring bilden, ausgewählt aus 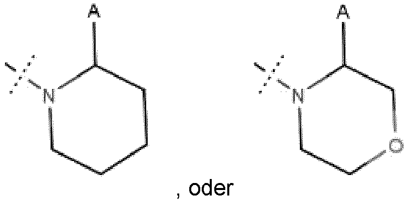
und wobei
A und A' voneinander verschieden und jeweils unabhängig voneinander aus einer der folgenden Gruppen ausgewählt sind:
a) eine Benzolgruppe mit der folgenden Struktur: 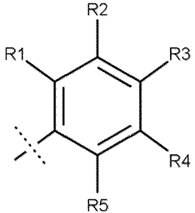 worin R¹-R⁵ jeweils unabhängig voneinander ausgewählt sind aus der Gruppe bestehend aus:
Wasserstoff; -OH, -OCH₃, -OCF₃, -OCHF₂, -OCH₂OH; -OCH₂CH₃; -OCH(CH₃)₂; -F; - CI; -Br; -CN; NO₂; NH₂; C₁-C₅-Alkyl, C₁-C₅-Alkoxy, -CH₃, -CH₂F; -CHF₂; -CF₃; -CH₂Cl; -CHCl₂; -CCl₃; CH(CH₃)₂; -CH₂CH₃; -CH-CH₂-CH(CH₃)₂; -CH₂-N(CH₃)₂; -CH(CH₃)-NHC(O)CH₃; - -NH-CH(CH₃)₂, -NH-Cyclopropyl, -CH₃, CH₂CH₃, CH₂CH(CH₃)₂; -C(O)-C₂H₅; -C(O)-CH₃; -C(O)-Aziridin; -C(O)-Azetidin; -C(O)-Pyrrolidin; -C(CH₃)=NOH; - S(O)₂-Cyclopentyl; -S(O)₂-CH₃; -S(O)₂-Cyclopentyl; Pyrrolidinyl, 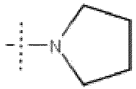 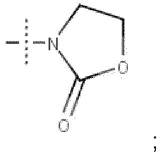
NHC(O)-R⁴¹, wobei R⁴¹ ausgewählt ist aus C₁-C₁₀ Cycloalkyl, C₁-C₁₀ Cycloheteroalkyl, Furan, Tetrahydrofuran, Cyclopentyl, Cyclobutyl, Cyclopropyl, -NH-CH(CH₃)₂, -NH-Cyclopropyl, C₁-C₅ Alkyl, -CH₂F; -CHF₂; -CF₃; -CH₂Cl; -CHCl₂; -CCl₃;-CH₃, CH₂CH₃, CH₂CH(CH₃)₂;
-C(O)OR⁴², wobei R⁴² ausgewählt ist aus C₁-C₅-Alkyl, einschließlich -CH₃; -CH₂CH₃, - CH₂CH₂CH₃; -CH(CH₃)₂; -CH₂CH₂CH₂CH₃; -CH₂NHS(O)₂CH₃;
-NHR₄₃, wobei R⁴³ ausgewählt ist aus 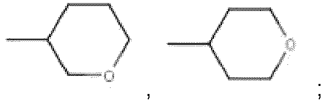 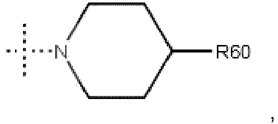 wobei R⁶⁰ ausgewählt ist aus Wasserstoff oder -CH₃; 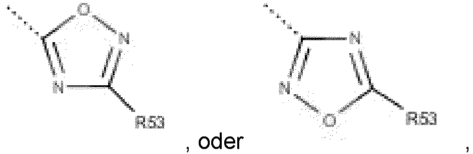 wobei
R⁵³ vorzugsweise ausgewählt ist aus der Gruppe bestehend aus Wasserstoff, OH, - OCH₃, -OCF₃, -OCHF₂, -OCH₂OH; -OCH₂CH₃; -F; -Cl; -Br; -CN; NO₂; NH₂; C₁-C₅-Alkyl, C₁-C₅-Alkoxy, C₂-C₅-Alkenyl, vorzugsweise -CH₃, CH(CH₃)₂; -CH₂CH₃; -CH-CH₂-CH(CH₃)₂; -CH₂-N(CH₃)₂; -CH(CH₃)-NHC(O)CH₃; -CH₂F; -CHF₂; -CF₃; -CH₂Cl; - CHCl₂; -CCl₃; -O-N=O, -N=O, -CH=NH, -N₃, -SH, -SO₃H, -SO₂H, -S(O)₂CH₃-CN, - NC,-OCN, -NCO, -(C=O)OH, -(C=O)NHOH, -NH(C=O)NH₂, -NH(C=S)NH₂, - (C=O)NH₂,-CHO,-CHS,
-OCHO, -NCHO, 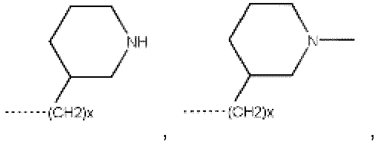 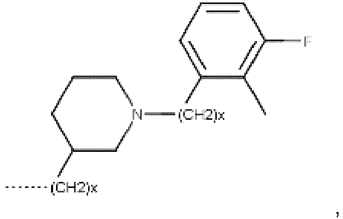 mit X = 0 oder 1, vorzugsweise 1; 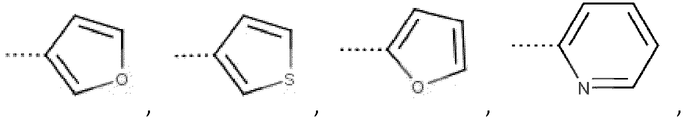 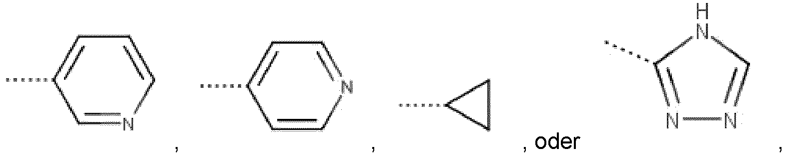 oder
R⁵³ ist 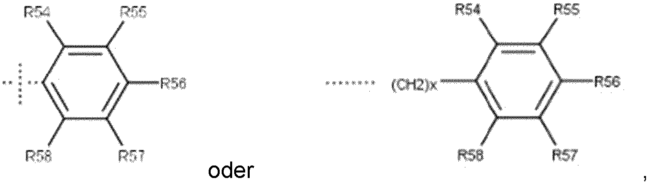
wobei R⁵⁴, R⁵⁵, R⁵⁶, R⁵⁷ oder R⁵⁸ jeweils unabhängig voneinander ausgewählt sind aus Wasserstoff, -CH₃, -C₂H₅, -CH₂F; -CHF₂; -CF₃; -CH₂Cl; -CHCl₂; -CCl₃; -OCH₃, -F, -Cl, -Br, NH₂, NO₂, und x 0, 1 oder 2 ist;
oder R⁵³ ist 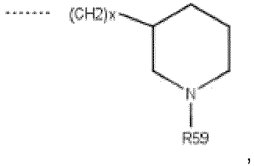 worin R⁵⁹ 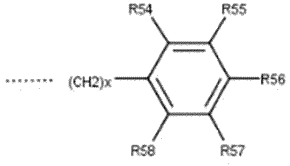 ist und worin
R⁵⁴, R⁵⁵, R⁵⁶, R⁵⁷ oder R⁵⁸ unabhängig von den obigen ausgewählt sind aus Wasserstoff, -CH₃, -C₂H₅, -CH₂F; -CHF₂; -CF₃; -CH₂Cl; -CHCl₂; -CCl₃; -OCH₃, -F, -Cl, -Br, NO₂, NH₂, und x 0, 1 oder 2 ist; wie oben definiert;
und
wobei R¹ und R², R² und R³, R³ und R⁴ oder R⁴ und R⁵ zusammen, vorzugsweise R² und R³ zusammen, einen fünf- oder sechsgliedrigen zyklischen oder heterozyklischen Ring bilden, ausgewählt aus 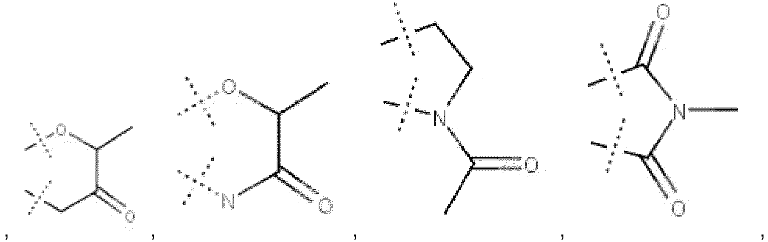 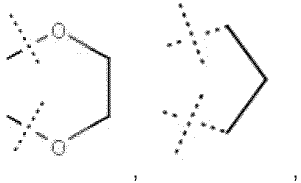
worin vorzugsweise entweder alle R¹ bis R⁵ Wasserstoff sind,
oder 1, 2 oder 3, vorzugsweise 1 oder 2 von R¹ bis R⁵ nicht Wasserstoff sind und aus den oben unter a) für R¹ bis R⁵ definierten Substitutionen ausgewählt sind, und die restlichen Substituenten von R¹ bis R⁵ Wasserstoff sind;
b) eine Pyridin-Gruppe, ausgewählt aus einer der folgenden Strukturen: 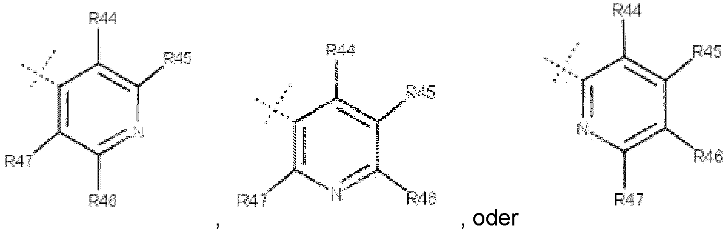 worin R⁴⁴-R⁴⁷ jeweils unabhängig voneinander ausgewählt sind aus der Gruppe bestehend aus:
Wasserstoff, -OH, -OCH₃, CH₃, F, Cl, Br, -CH₂F; -CHF₂; -CF₃; -CH₂Cl; -CHCl₂; -CCl₃ - OCF₃, -OCHF₂, -OCH₂OH; -OCH₂CH₃; -CN; NO₂; NH₂; -CH₃, CH(CH₃)₂; -CH₂CH₃; - CH₂OH; -CH-CH₂-CH(CH₃)₂; -CH₂-N(CH₃)₂; -CH(CH₃)-NHC(O)CH₃; 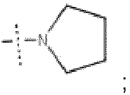 -NHC(O)-R⁴¹, wobei R⁴¹ ausgewählt ist aus C₁-C₁₀ Cycloalkyl, C₁-C₁₀ Cycloheteroalkyl, Furan, Tetrahydrofuran, Cyclopentyl, Cyclobutyl, Cyclopropyl, -NH-Cyclopropyl, - CH₂F; -CHF₂; -CF₃; -CH₂Cl; -CHCl₂; -CCl₃; -CH₃, CH₂CH₃, CH₂CH(CH₃)₂; -C(O)-Aziridin; -C(O)-Azetidin; -C(O)-Pyrrolidin;
-C(O)OR⁴², wobei R⁴² ausgewählt ist aus C₁-C₅-Alkyl, einschließlich -CH₃; -CH₂CH₃, - CH₂CH₂CH₃; -CH(CH₃)₂; -CH₂CH₂CH₂CH₃; -CH₂NHS(O)₂CH₃; -C(CH₃)=NOH; -S(O)₂-Cyclopentyl; -S(O)₂-CH₃; -S(O)₂-Cyclopentyl; Pyrrolidinyl, 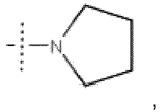 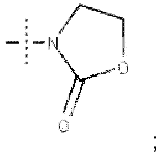 oder
-NHR⁴³, wobei R⁴³ ausgewählt ist aus 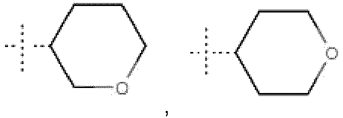 worin vorzugsweise entweder alle R⁴⁴ bis R⁴⁷ Wasserstoff sind,
oder 1, 2 oder 3, vorzugsweise 1 oder 2 von R⁴⁵ bis R⁴⁷ nicht Wasserstoff sind und aus den oben unter b) für R⁴⁴ bis R⁴⁷ definierten Substitutionen ausgewählt sind, und die restlichen Substituenten von R⁴⁴ bis R⁴⁷ Wasserstoff sind;
c) eine Chinolingruppe oder eine Isochinolingruppe, ausgewählt aus einer der folgenden Strukturen: 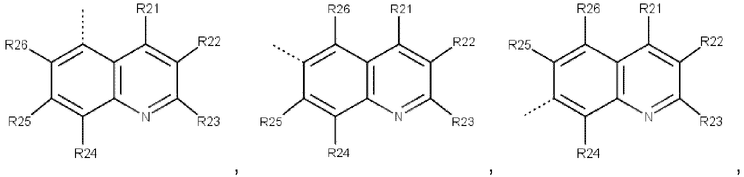 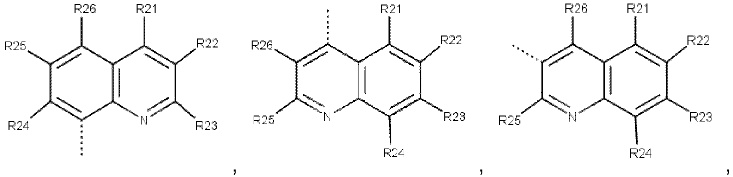 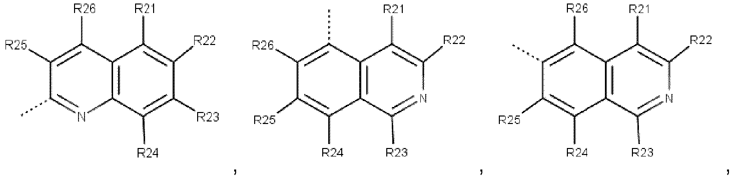 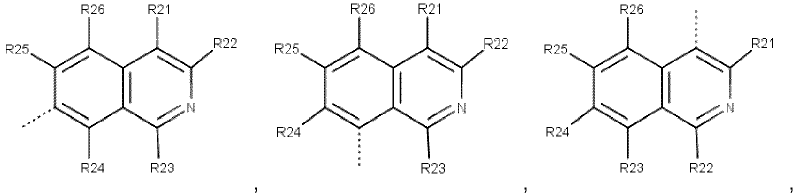 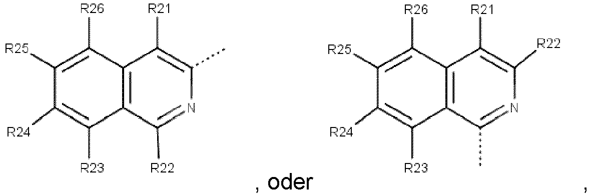 wobei
R²¹ bis R²⁶ jeweils unabhängig voneinander ausgewählt sind aus der Gruppe bestehend aus
Wasserstoff, OH, -OCH₃, -OCF₃, -OCHF₂, -OCH₂OH; -OCH₂CH₃; -F; -Cl; -Br; -CH₂F; - CHF₂; -CF₃; -CH₂Cl; -CHCl₂; -CCl₃; -CN; NO₂; NH₂; C₁-C₅-Alkyl, C₁-C₅-Alkoxy, C₂-C₅-Alkenyl, vorzugsweise -CH₃, CH(CH₃)₂; -CH₂CH₃; -CH-CH₂-CH(CH₃)₂; -CH₂-N(CH₃)₂; -CH(CH₃)-NHC(O)CH₃; -O-N=O, -N=O, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, - (C=O)NHOH, -NH(C=O)NH₂, -NH(C=S)NH₂, -(C=O)NH₂,-CHO,-CHS, -OCHO, - NCHO, -C(O)-Aziridin; -C(O)-Azetidin; -C(O)-Pyrrolidin; -C(O)OR₄₂, wobei R⁴² ausgewählt ist aus C1-C5-Alkyl, einschließlich -CH₃; -CH₂CH₃, -CH₂CH₂CH₃; - CH(CH₃)₂; -CH₂CH₂CH₂CH₃; -CH₂NHS(O)₂CH₃; -C(CH₃)=NOH; -S(O)₂-cyclopentyl; - S(O)₂-CH₃; -S(O)₂-cyclopentyl; Pyrrolidinyl, 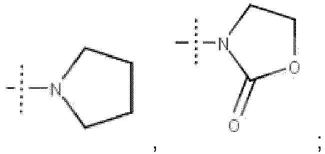
-NHC(O)-R⁴¹, wobei R⁴¹ ausgewählt ist aus C₁-C₁₀ Cycloalkyl, C₁-C₁₀ Cycloheteroalkyl, Furan, Tetrahydrofuran, Cyclopentyl, Cyclobutyl, Cyclopropyl, -NH-Cyclopropyl, - CH₂F; - CHF₂; -CF₃; -CH₂Cl; -CHCl₂; -CCl₃; -CH₃, CH₂CH₃, CH₂CH(CH₃)₂;
-NHR⁴³, wobei R⁴³ ausgewählt ist aus 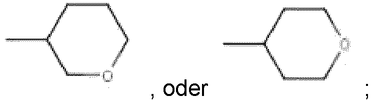
oder, noch bevorzugter, wobei R²¹ bis R²⁶ jeweils Wasserstoff, OH, -OCH₃, -OCF₃, - OCHF₂, -OCH₂OH; -OCH₂CH₃; -F; -Cl; -Br; -CH₂F; -CHF₂; -CF₃; -CH₂Cl; -CHCl₂; - CCl₃;-CN; NO₂; NH₂; C₁-C₅-Alkyl, C₁-C₅-Alkoxy, C₂-C₅-Alkenyl, vorzugsweise -CH₃, CH(CH₃)₂; -CH₂CH₃; -CH-CH₂-CH(CH₃)₂; -CH₂-N(CH₃)₂; -CH(CH₃)-NHC(O)CH₃ sind;
oder noch mehr bevorzugt, wobei R²¹ bis R²⁶ jeweils Wasserstoff oder -F; Cl, Br sind,
wobei vorzugsweise entweder alle R²¹ bis R²⁶ Wasserstoff sind,
oder 1, 2 oder 3, vorzugsweise 1 oder 2 von R²¹ bis R²⁶ nicht Wasserstoff sind und aus den oben unter c) für R²¹ bis R²⁶ definierten Substitutionen ausgewählt sind, und die restlichen Substituenten von R²¹ bis R²⁶ Wasserstoff sind;
d) eine 1,5-Naphthyridin-Gruppe, eine 1,6-Naphthyridin-Gruppe oder eine 1,8-Naphthyridin-Gruppe mit einer der folgenden Strukturen 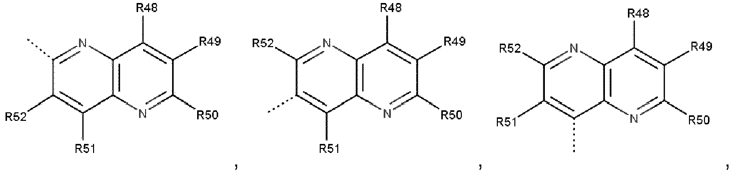 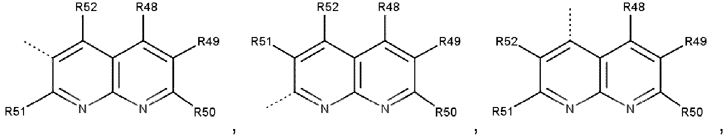 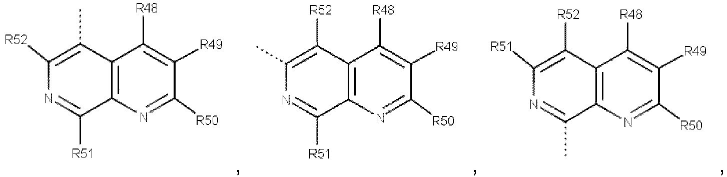 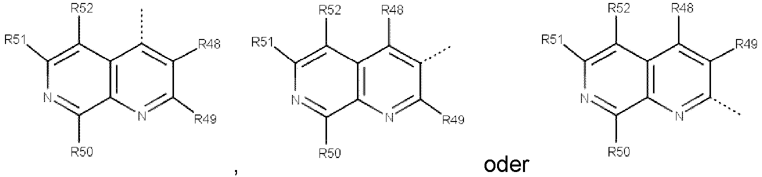 , wobei R⁴⁸ bis R⁵² sind jeweils unabhängig voneinander ausgewählt sind aus der Gruppe bestehend aus:
Wasserstoff, OH, -OCH₃, -OCF₃, -OCHF₂, -OCH₂OH; -OCH₂CH₃; -F; -Cl; -Br; -CH₂F; - CHF₂; -CF₃; -CH₂Cl; -CHCl₂; -CCl₃;-CN; NO₂; NH₂; C₁-C₅Alkyl, C₁-C₅Alkoxy, C₂-C₅ Alkenyl, vorzugsweise -CH₃, CH(CH₃)₂; -CH₂CH₃; -CH-CH₂-CH(CH₃)₂; -CH₂-N(CH₃)₂; -CH(CH₃)-NHC(O)CH₃; -O-N=O, -N=O, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, - (C=O)NHOH, -NH(C=O)NH₂, -NH(C=S)NH₂, -(C=O)NH₂,-CHO,-CHS, -OCHO, - NCHO,
-NHC(O)-R⁴¹, worin R⁴¹ ausgewählt ist aus C₁-C₄₀ Cycloalkyl, C₁-C₁₀ Cycloheteroalkyl, Furan, Tetrahydrofuran, Cyclopentyl, Cyclobutyl, Cyclopropyl, -NH-Cyclopropyl, - CH₂F; -CHF₂; -CF₃; -CH₂Cl; -CHCl₂; -CCl₃; -CH₃, CH₂CH₃, CH₂CH(CH₃)₂; -C(O)-Aziridin; -C(O)-Azetidin; -C(O)-Pyrrolidin;
-C(O)OR⁴², wobei R⁴² ausgewählt ist aus C₁-C₅-Alkyl, einschließlich -CH₃; -CH₂CH₃, - CH₂CH₂CH₃; -CH(CH₃)₂; -CH₂CH₂CH₂CH₃; -CH₂NHS(O)₂CH₃; -C(CH₃)=NOH; -S(O)₂-Cyclopentyl; -S(O)₂-CH₃; -S(O)₂-Cyclopentyl; Pyrrolidinyl, 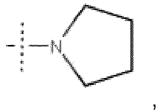 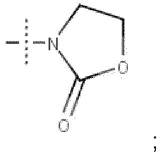 -NHR₄₃, wobei R₄₃ ausgewählt ist aus , 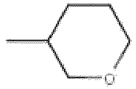 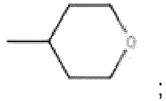
oder noch bevorzugter, worin R⁴⁸ bis R⁵² jeweils Wasserstoff, OH, -OCH₃, -OCF₃, - OCHF₂, -OCH₂OH; -OCH₂CH₃; -F; -Cl; -Br; -CH₂F; -CHF₂; -CF₃; -CH₂Cl; -CHCl₂; - CCl₃;-CN; NO₂; NH₂; C₁-C₅-Alkyl, C₁-C₅-Alkoxy, C₂-C₅-Alkenyl, vorzugsweise -CH₃, CH(CH₃)₂; -CH₂CH₃; -CH-CH₂-CH(CH₃)₂; -CH₂-N(CH₃)₂; -CH(CH₃)-NHC(O)CH₃;
oder noch mehr bevorzugt, wobei R⁴⁸ bis R⁵² jeweils Wasserstoff oder -F; Cl, Br sind,
worin vorzugsweise entweder alle R⁴⁸ bis R⁵² Wasserstoff sind,
oder 1, 2 oder 3, vorzugsweise 1 oder 2 von R⁴⁸ bis R⁵² nicht Wasserstoff sind und aus Substitutionen ausgewählt sind, die hier oben unter d) für R⁴⁸ bis R⁵² definiert oder dargestellt sind, und die restlichen Substituenten von R⁴⁸ bis R⁵² Wasserstoff sind;
e) eine Oxadiazolgruppe, ausgewählt aus einer der folgenden Strukturen: 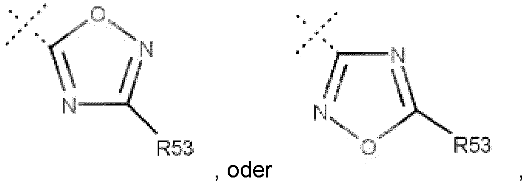 wobei
R⁵³ ist vorzugsweise ausgewählt aus der Gruppe bestehend aus:
Wasserstoff, OH, -OCH₃, -OCF₃, -OCHF₂, -OCH₂OH; -OCH₂CH₃; -F; -Cl; -Br; -CN; NO₂; NH₂; C₁-C₅-Alkyl, C₁-C₅-Alkoxy, C₂-C₅-Alkenyl, vorzugsweise -CH₃, CH(CH₃)₂; - CH₂CH₃; -CH-CH₂-CH(CH₃)₂; -CH₂-N(CH₃)₂; -CH(CH₃)-NHC(O)CH₃; -CH₂F; -CHF₂; - CF₃; -CH₂Cl; -CHCl₂; -CCl₃;-O-N=O, -N=O, -CH=NH, -N₃, -SH, -SO₃H, -SO₂H, - S(O)₂CH₃-CN, -NC,-OCN, -NCO, -(C=O)OH, -(C=O)NHOH, -NH(C=O)NH₂, -NH(C=S)NH₂,
-(C=O)NH₂,-CHO,-CHS, -OCHO, -NCHO, 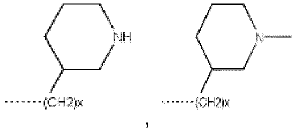 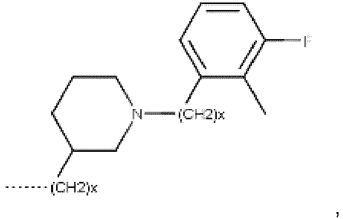 mit X = 0 oder 1, vorzugsweise 1; 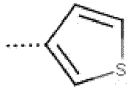 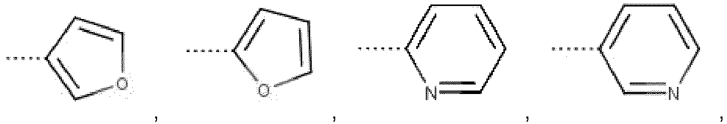 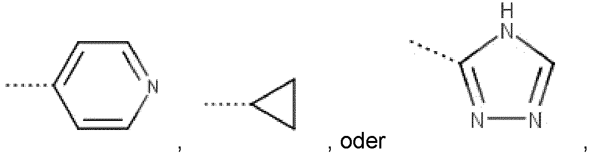 oder
R⁵³ ist 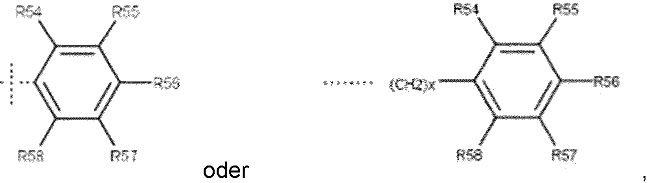 wobei R⁵⁴, R⁵⁵, R⁵⁵, R⁵⁷ oder R⁵⁸ jeweils unabhängig voneinander ausgewählt sind aus Wasserstoff, -CH₃, -C₂H₅, -CH₂F; -CHF₂; -CF₃; -CH₂Cl; -CHCl₂; -CCl₃; -OCH₃, -F, -Cl, -Br, NH₂, NO₂, und x 0, 1 oder 2 ist;
oder R⁵³ ist 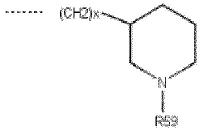 ist, worin R⁵⁹ 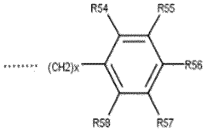 **ist und worin** R⁵⁴, R⁵⁵, R⁵⁶, R⁵⁷ oder R⁵⁸ ausgewählt sind aus Wasserstoff, -CH₃, -C₂H₅, -CH₂F; -CHF₂; -CF₃; - CH₂Cl; -CHCl₂; -CCl₃; -OCH₃, -F, -Cl, -Br, NO₂, NH₂, und x 0, 1 oder 2 ist; wie oben definiert, und x 0, 1 oder 2 ist
und worin vorzugsweise entweder alle R⁵⁴ bis R⁵⁸ Wasserstoff sind,
oder 1, 2 oder 3, vorzugsweise 1 oder 2 von R⁵⁴ bis R⁵⁸ nicht Wasserstoff sind und aus den oben unter e) für R⁵⁴ bis R⁵⁸ definierten Substitutionen ausgewählt sind, und die restlichen Positionen von R⁵⁴ bis R⁵⁸ Wasserstoff sind;
f) eine Thiadiazolgruppe, ausgewählt aus einer der folgenden Strukturen: 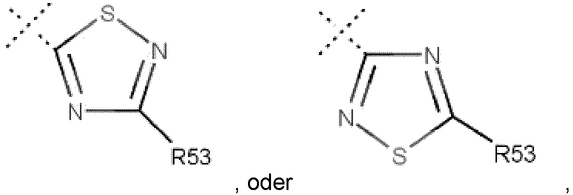 worin R⁵³ vorzugsweise wie oben definiert ist
g) eine Isothiazolgruppe, ausgewählt aus einer der folgenden Strukturen: 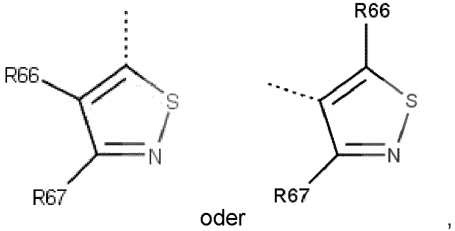 wobei R⁶⁶ und R⁶⁷ jeweils unabhängig voneinander ausgewählt sind aus Wasserstoff, -CH₃, -C₂H₅, -CH₂F; -CHF₂; -CF₃; - CH₂Cl; -CHCl₂; -CCl₃;-F, -Cl, -Br, wobei vorzugsweise einer der Reste R⁶⁶ oder R⁶⁷ Wasserstoff ist und der andere Rest ausgewählt ist aus -CH₃, -C₂H₅, -CF₃, -CHF₂, -F, -Cl, -Br; oder
h) eine Thiophengruppe, ausgewählt aus einer der folgenden Strukturen: 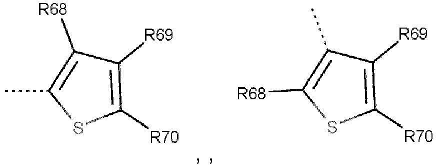 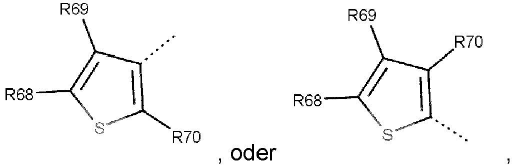 worin R⁶⁸, R⁶⁹ und R⁷⁰ jeweils unabhängig voneinander ausgewählt sind aus Wasserstoff, -CH₃, -C₂H₅, -CH₂F; -CHF₂; -CF₃; -CH₂Cl; -CHCl₂; -CCl₃;-F, -Cl, -Br, vorzugsweise wobei entweder R⁶⁸, R⁶⁹ oder R⁷⁰ Wasserstoff ist und der andere Rest ausgewählt ist aus -CH₃, -C₂H₅, -CF₃, -CHF₂, -F, -Cl, -Br,
oder
(II) wobei die Verbindung eine Verbindung der Formel (Ic), eine Verbindung der Formel (Ic1), eine Verbindung der Formel (Ic2), eine Verbindung der Formel (Ic3) oder eine Verbindung der Formel (Ic4) oder ein pharmazeutisch akzeptables Salz davon ist: 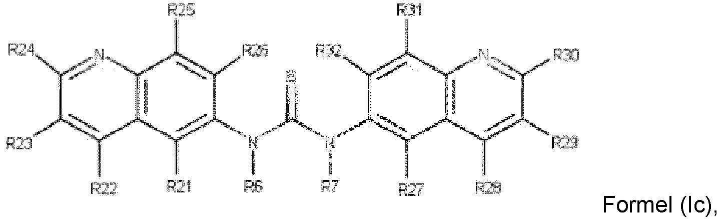 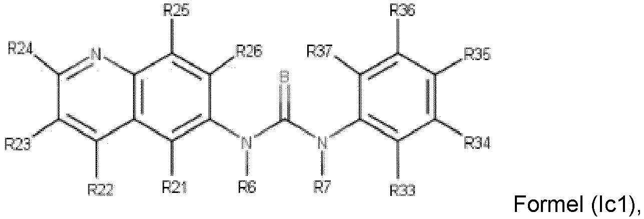 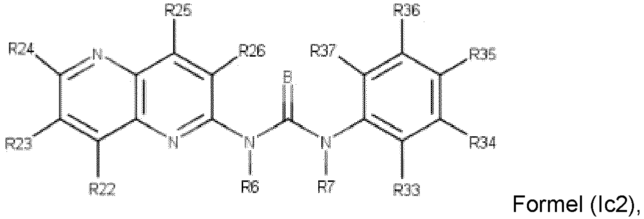 oder 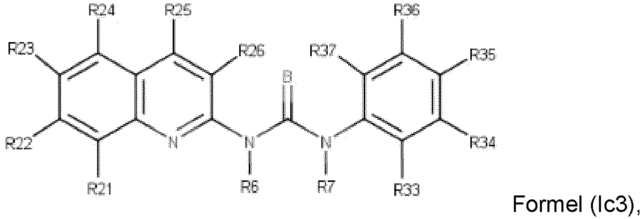 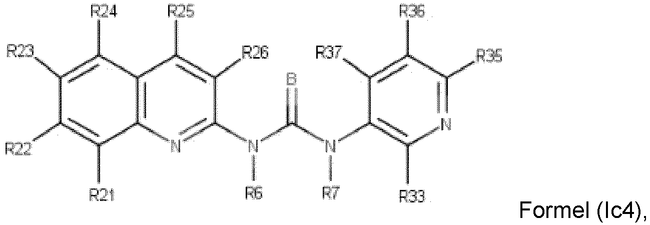 wobei
B = O oder S, vorzugsweise O;
R⁶ und R⁷ unabhängig voneinander jeweils Wasserstoff oder C₁₋₃-Alkyl sind, vorzugsweise Wasserstoff, CH₃, noch bevorzugter entweder R⁶ Wasserstoff und R⁷ CH₃, oder R6 und R7 beide Wasserstoff sind;
R²¹ bis R³⁷ jeweils unabhängig voneinander ausgewählt sind aus der Gruppe bestehend aus Wasserstoff, OH, -OCH₃, -OCF₃, -OCHF₂, -OCH₂OH; -OCH₂CH₃; - CH₂F; -CHF₂; -CF₃; -CH₂Cl; -CHCl₂; -CCl₃;-F; -Cl; -Br; -CN; NO₂; NH₂; -CH₃, CH(CH₃)₂; -CH₂CH₃; -CH-CH₂-CH(CH₃)₂; -CH₂-N(CH₃)₂; -CH(CH₃)-NHC(O)CH₃; -O-N=O, -N=O, -OH, -NH₂, -CH=NH, -N₃, -SH, -SO₃H, -SO₂H, -S(O)₂CH₃-CN, -NC,-OCN, -NCO, -SCN, -NCS, -(P=O)(OH)₂, -O(P=O)(OH)₂, -(C=O)OH, -(C=O)NHOH, - NH(C=O)NH₂, -NH(C=S)NH₂, -(C=O)NH₂, -CHO, -CHS, -OCHO, -NCHO, -B(OH)₍₂₎, (C=O)R¹¹, (C=O)OR¹¹, (C=O)SR¹¹, (C=O)NR¹¹R¹², (SO₂)R¹¹ oder C(NR¹¹)R¹²,-NHC(O)R¹¹ und -NHC(O)NHR¹¹,
wobei R¹¹ und R¹² unabhängig voneinander ausgewählt sind aus der Gruppe bestehend aus C₁-C₅-Alkyl, vorzugsweise -CH₃, CH(CH₃)₂; -CH₂CH₃; -CH-CH₂-CH(CH₃)₂; -CH₂-N(CH₃)₂; -CH(CH₃)-NHC(O)CH₃; I, -CH₂F; -CHF₂; -CF₃; -CH₂Cl; - CHCl₂; -CCl₃; -CHCl₂, und -CCl₃; oder
vorzugsweise worin R²¹ bis R³⁷ jeweils unabhängig voneinander ausgewählt sind aus der Gruppe bestehend aus Wasserstoff, -CH₂(SO₂)CH₃, -CH₂(SO₂)CH₂CH₃, - CH₂CH₂(SO₂)CH₂CH₃; -(SO₂)C₁-C₅-Alkyl, vorzugsweise -(SO₂)CH₃, -(SO₂)CH₂CH₃, - CH₂F; -CHF₂; -CF₃; -CH₂Cl; -CHCl₂; -CCl₃; -F, -Cl, und -Br; oder
worin vorzugsweise entweder alle R²¹ bis R³⁷ Wasserstoff sind,
oder 1, 2 oder 3, vorzugsweise 1 oder 2 von R²¹ bis R³⁷ nicht Wasserstoff sind und aus den oben dargestellten Substitutionen ausgewählt sind, und die restlichen Positionen von R²¹ bis R³⁷ Wasserstoff sind; oder
bevorzugter, wobei R²¹ bis R³⁷ jeweils Wasserstoff sind.

2. Verbindung zur Verwendung nach Anspruch 1, wobei die Verbindung eine Verbindung der Formel (Ic5), eine Verbindung der Formel (Ic6), eine Verbindung der Formel (Ic7), eine Verbindung der Formel (Ic8), eine Verbindung der Formel (Ic9) oder eine Verbindung der Formel (Ic10), eine Verbindung der Formel (Ic11), eine Verbindung der Formel (Ic12) oder eine Verbindung der Formel (Ic13) oder ein pharmazeutisch annehmbares Salz davon ist: 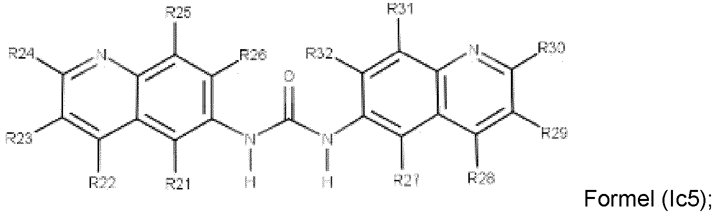 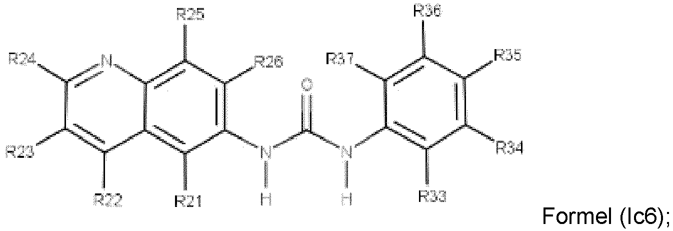 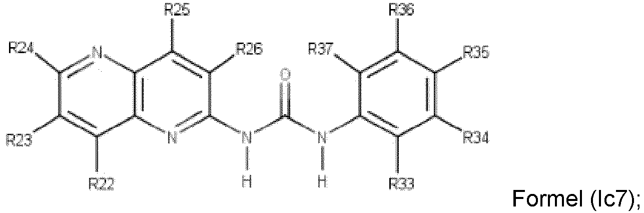 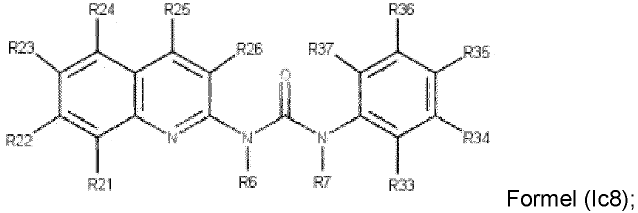 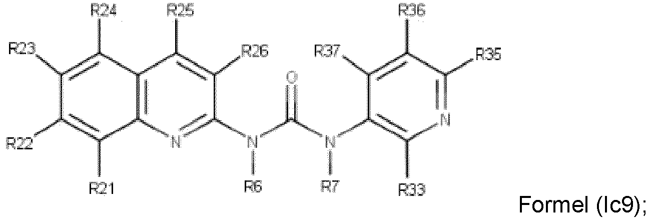 oder 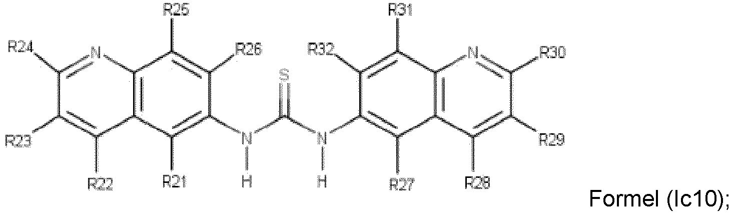 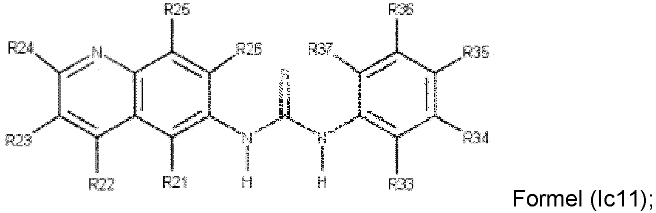 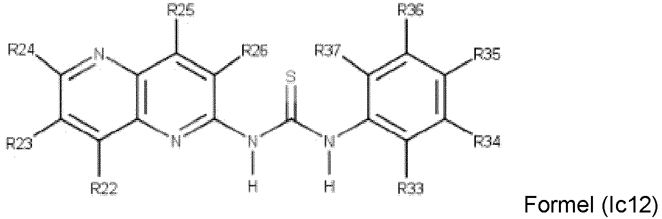 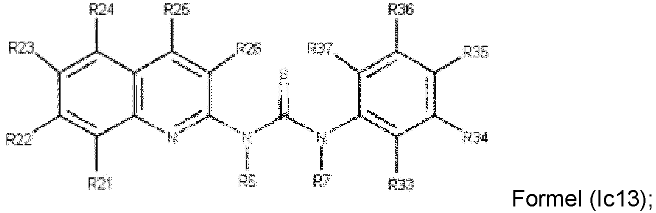 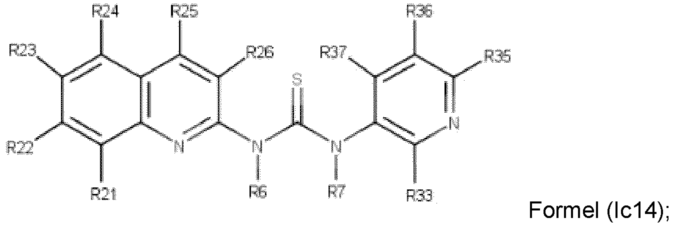
worin R²¹ bis R³⁷ jeweils unabhängig voneinander ausgewählt sind aus der Gruppe bestehend aus Wasserstoff, OH, -OCH₃, -OCF₃, -OCHF₂, -OCH₂OH; -OCH₂CH₃; - CH₂F; -CHF₂; -CF₃; -CH₂Cl; -CHCl₂; -CCl₃; -F; -Cl; -Br; -CN; NO₂; NH₂; -CH₃, CH(CH₃)₂; -CH₂CH₃; oder -CH₂(SO₂)CH₃, -CH₂(SO₂)CH₂CH₃, -CH₂CH₂(SO₂)CH₂CH₃; -(SO₂)C₁-C₅Alkyl, vorzugsweise -(SO₂)CH₃, -(SO₂)CH₂CH₃, oder
worin vorzugsweise entweder alle R²¹ bis R³⁷ Wasserstoff sind,
oder 1, 2 oder 3, vorzugsweise 1 oder 2 von R²¹ bis R³⁷ nicht Wasserstoff sind und aus den oben dargestellten Substitutionen ausgewählt sind, und die restlichen Positionen von R²¹ bis R³⁷ aus Wasserstoff ausgewählt sind; oder
bevorzugter, wobei R²¹ bis R³⁷ jeweils Wasserstoff sind.

3. Verbindung zur Verwendung nach einem der Ansprüche 1 oder 2, wobei die Verbindung eine Verbindung mit einer der Formeln (la) oder (la1) ist 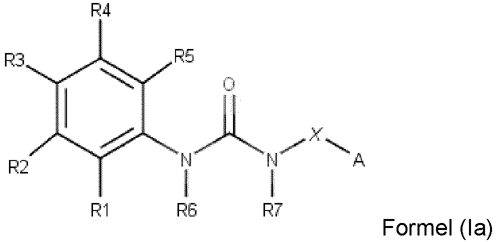 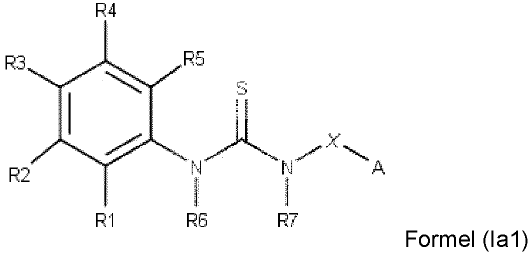 oder ein pharmazeutisch annehmbares Salz davon, wobei:
A und A' voneinander verschieden sind,
X entweder vorhanden ist oder nicht, und wobei X, falls vorhanden, ausgewählt ist aus -CH₂-, -C₂H₄-, -C₃H₆-, -C(CH₃)₂-, -CHCH(CH₃)₂-, -CHC(CH₃)₃-, -CH(CH(CH₃)₂)-, -CH(CH₂CH(CH₃)₂)-, oder -CH(CH₂OH)-; und
R⁶ und R⁷ jeweils unabhängig voneinander Wasserstoff, -CH₃, -C₂H₅, -C₃H₇ oder -C₃H₉ sind, oder optional,
R⁶ und R⁷ zusammen einen fünfgliedrigen heterocyclischen Ring bilden, ausgewählt aus 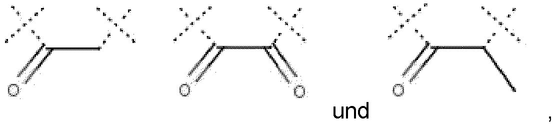 oder optional,
R⁷, N und X zusammen einen sechsgliedrigen heterocyclischen Ring bilden, ausgewählt aus 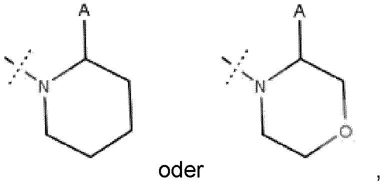
wobei R¹-R⁵ jeweils unabhängig voneinander ausgewählt sind aus der Gruppe bestehend aus Wasserstoff; -OH, -OCH₃, -OCF₃, -OCHF₂, -OCH₂OH; -OCH₂CH₃; - OCH(CH₃₎₍₂₎; -F; -Cl; -Br; -CN; NO₂; NH₂; -CH₃, -CH₂F; -CHF₂; -CF₃; -CH₂Cl; -CHCl₂; -CCl₃; CH(CH₃)₂; -CH₂CH₃;
-C(O)OR⁴², wobei R⁴² ausgewählt ist aus -CH₃; -CH₂CH₃, -CH₂CH₂CH₃; -CH(CH₃)₂; - CH₂CH₂CH₍₂₎CH₃; 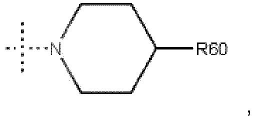 wobei R⁶⁰ aus Wasserstoff oder -CH₃ ausgewählt ist;
wobei R¹ und R², R² und R³, R³ und R⁴ oder R⁴ und R⁵ zusammen, vorzugsweise R¹ und R² oder R² und R³ zusammen, einen fünf- oder sechsgliedrigen zyklischen oder heterozyklischen Ring bilden
ausgewählt aus 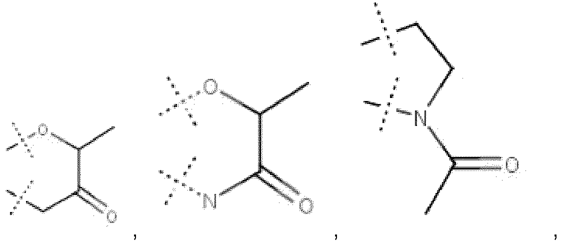 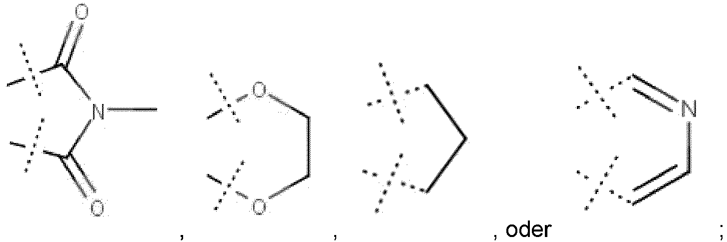
und worin A aus einem der folgenden Substituenten a) bis e) ausgewählt ist:
a) eine Benzolgruppe mit der folgenden Struktur: 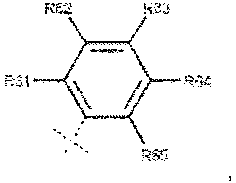 worin R⁶¹-R⁶⁶ jeweils unabhängig voneinander ausgewählt sind aus der Gruppe bestehend aus:
Wasserstoff; -OH, -OCH₃, -OCF₃, -OCHF₂, -OCH₂OH; -OCH₂CH₃; -OCH(CH₃)₂; -F; - CI; -Br; -CN; NO₂; NH₂; -CH₃, -CH₂F; -CHF₂; -CF₃; -CH₂Cl; -CHCl₂; -CCl₃;CH(CH₃)₂; - CH₂CH₃; -CH-CH₂-CH(CH₃)₂; -CH₂-N(CH₃₎₍₂₎; -CH(CH₃)-NHC(O)CH₃; -C(O)-C₂H₅; - C(O)-CH₃; -C(O)-Aziridin; -C(O)-Azetidin; -C(O)-Pyrrolidin; -CH₂NHS(O)₂CH₃; - C(CH₃)=NOH; -S(O)₂-Cyclopentyl; -S(O)₂-CH₃; -S(O)₂-Cyclopentyl; Pyrrolidinyl, , ; 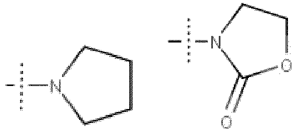
-NHC(O)-R₄₁, wobei R₄₁ ausgewählt ist aus Furan, Tetrahydrofuran, Cyclopentyl, Cyclobutyl, Cyclopropyl, -NH-CH(CH₃)₂, -NH-Cyclopropyl, -CH₂F; -CHF₂; -CF₃; - CH₂Cl; -CHCl₂; -CCl₃;-CH₃, CH₂CH₃, CH₂CH(CH₃)₂;
-C(O)OR₄₂, wobei R₄₂ ausgewählt ist aus C₁-C₅-Alkyl, einschließlich -CH₃; -CH₂CH₃, - CH₂CH₂CH₃; -CH(CH₃)₂; -CH₂CH₂CH₂CH₃;
-NHR₄₃, wobei R₄₃ ausgewählt ist aus 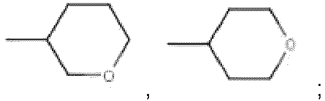 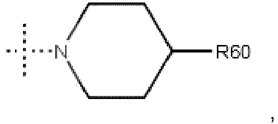 wobei R⁶⁰ ausgewählt ist aus Wasserstoff oder -CH₃; 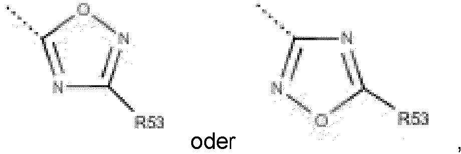 wobei R⁵³ vorzugsweise ausgewählt ist aus der Gruppe bestehend aus Wasserstoff, OH, -OCH₃, -OCF₃, -OCHF₂, -OCH₂OH; -OCH₂CH₃; -F; -Cl; -Br; -CN; NO₂; NH₂; C₁-C₅-Alkyl, C₁-C₅-Alkoxy, C₂-C₅-Alkenyl, vorzugsweise -CH₃, CH(CH₃)₂; -CH₂CH₃; -CH-CH₂-CH(CH₃)₂; -CH₂-N(CH₃₎₍₂₎; -CH(CH₃)-NHC(O)CH₃; -CH₂F; -CHF₂; -CF₃; -CH₂Cl; -CHCl₂; -CCl₃; -O-N=O, -N=O, - CH=NH, -N₃, -SH, -SO₃H, -SO₂H, -S(O)₂CH₃-CN, -NC, -OCN, -NCO, -(C=O)OH, - (C=O)NHOH, -NH(C=O)NH₂, 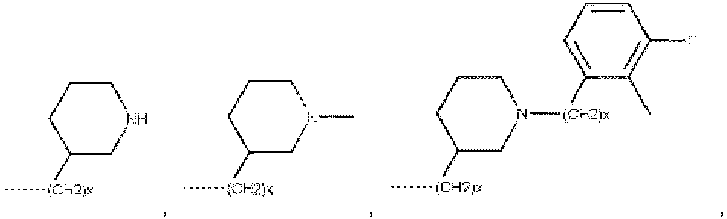 mit X = 0 oder 1, vorzugsweise 1; 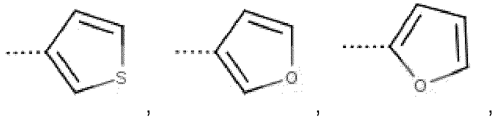 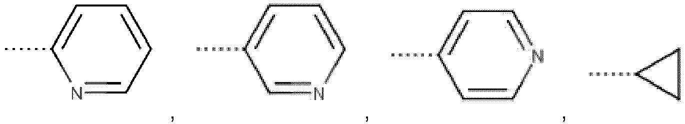 oder 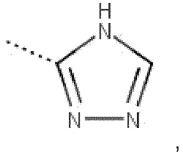
oder R⁵³ ist 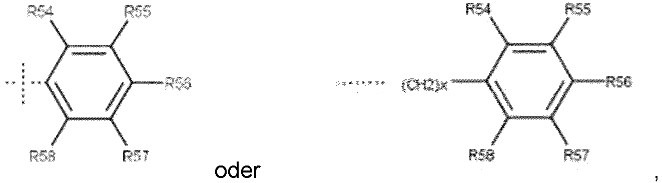 wobei R⁵⁴, R⁵⁵, R⁵⁶, R⁵⁷ oder R⁵⁸ jeweils unabhängig voneinander ausgewählt sind aus Wasserstoff, -CH₃, -C₂H₅, -CH₂F; -CHF₂; -CF₃; -CH₂Cl; -CHCl₂; -CCl₃; -OCH₃, -F, -Cl, -Br, NO₂, NH₂, und x 0, 1 oder 2 ist;
oder R⁵³ 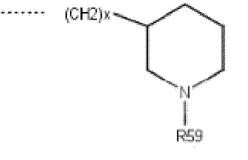 ist, wobei R⁵⁹ 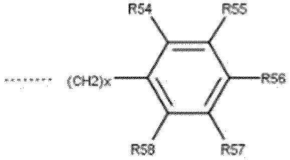 ist und wobei R⁵⁴, R⁵⁵, R⁵⁶, R⁵⁷ oder R⁵⁸ unabhängig von den oben genannten ausgewählt sind aus Wasserstoff, -CH₃, -C₂H₅, -CH₂F; -CHF₂; -CF₃; -CH₂Cl; -CHCl₂; -CCl₃; - OCH₃, -F, -Cl, -Br, NO₂, NH₂, und x 0, 1 oder 2 ist; wie oben definiert, und x 0, 1 oder 2 ist;
und
wobei R⁶¹ und R⁶², R⁶² und R⁶³, R⁶³ und R⁶⁴ oder R⁶⁴ und R⁶⁵ zusammen, vorzugsweise R⁶² und R⁶³ zusammen, einen fünf- oder sechsgliedrigen zyklischen oder heterozyklischen Ring
bilden, ausgewählt aus 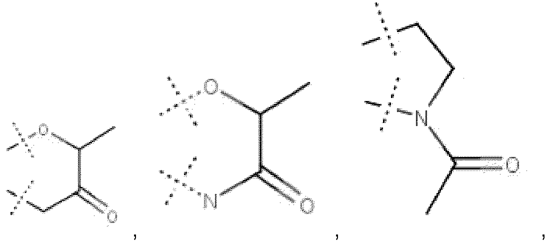 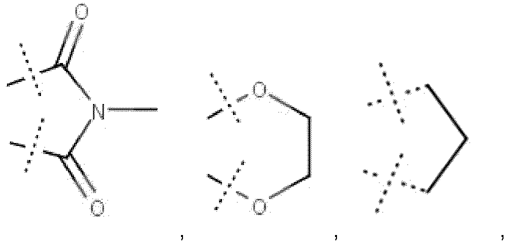 und
wobei X vorzugsweise nicht vorhanden ist, und
worin vorzugsweise entweder alle R¹ bis R⁵ Wasserstoff sind, oder 1, 2 oder 3, vorzugsweise 1 oder 2 von R¹ bis R⁵ nicht Wasserstoff sind und aus den oben für R¹ bis R⁵ definierten Substitutionen ausgewählt sind, und die restlichen Substituenten von R¹ bis R⁵ Wasserstoff sind;
b) eine Oxadiazolgruppe mit einer der folgenden Strukturen: 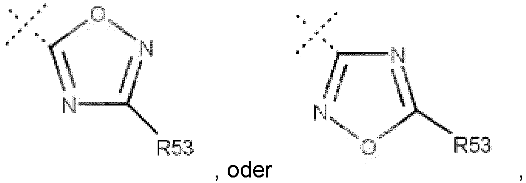 wobei
R⁵³ ist vorzugsweise ausgewählt aus der Gruppe bestehend aus Wasserstoff, OH, - OCH₃, -OCF₃, -OCHF₂, -OCH₂OH; -OCH₂CH₃; -F; -Cl; -Br; -CN; NO₂; NH₂; C₁-C₅-Alkyl, C₁-C₅-Alkoxy, C₂-C₅-Alkenyl, vorzugsweise -CH₃, CH(CH₃)₂; -CH₂CH₃; -CH-CH₂-CH(CH₃)₂; -CH₂-N(CH₃₎₍₂₎; -CH(CH₃)-NHC(O)CH₃; -CH₂F; -CHF₂; -CF₃; -CH₂Cl; - CHCl₂; -CCl₃;-O-N=O, -N=O, -CH=NH, -N₃, -SH, -SO₃H, -SO₂H, -S(O)₂CH₃-CN, -NC,-OCN, -NCO, -(C=O)OH, -(C=O)NHOH, -NH(C=O)NH₂, -NH(C=S)NH₂, -(C=O)NH₂,-CHO,-CHS, - OCHO, -NCHO, - 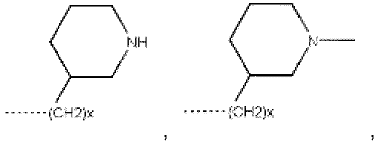 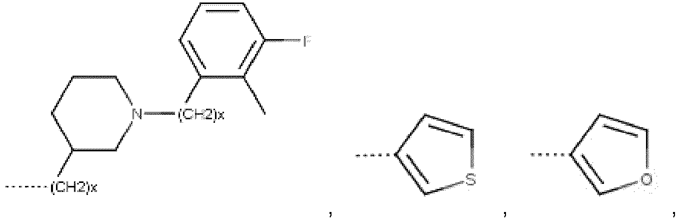 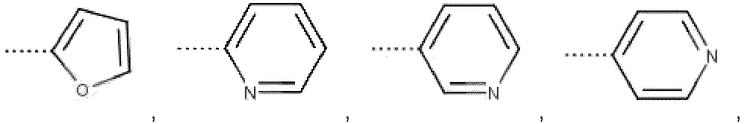 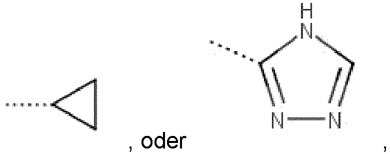 oder
R⁵³ ist 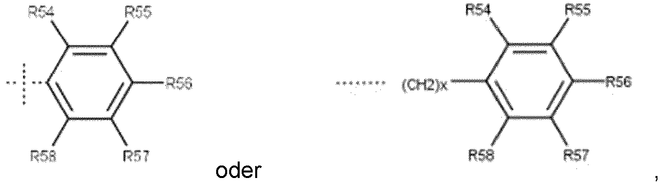 wobei R⁵⁴, R⁵⁵, R⁵⁶, R⁵⁷ oder R⁵⁸ jeweils unabhängig voneinander ausgewählt sind aus Wasserstoff, -CH₃, -C₂H₅, -CH₂F; -CHF₂; -CF₃; -CH₂Cl; -CHCl₂; -CCl₃; -OCH₃, -F, -Cl, -Br, NO₂, NH₂, und x 0, 1 oder 2 ist;
oder R⁵³ ist 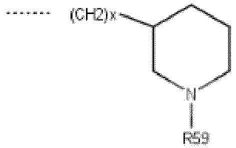 worin R⁵⁹ 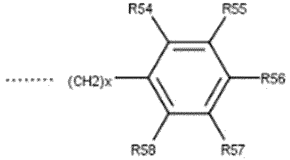 ist und worin R⁵⁴, R⁵⁵, R⁵⁶, R⁵⁷ oder R⁵⁸ unabhängig von den oben genannten aus Wasserstoff, -CH₃, - C₂H₅, -CH₂F; -CHF₂; -CF₃; -CH₂Cl; -CHCl₂; -CCl₃; -OCH₃, -F, -Cl, -Br, NO₂, NH₂, und x 0, 1 oder 2 ist; wie oben definiert, und x 0, 1 oder 2 ist; und
wobei X vorzugsweise ausgewählt ist aus -CH₂-, -C₂H₄-, -C₃H₆-, -C(CH₃)₂-, - CHCH(CH(₃))₂-, -CHC(CH₃)₃-, -CH(CH(CH₃)₂)-, -CH(CH₂CH(CH₃)₂)- oder - CH(CH₂OH)-;
und wobei vorzugsweise entweder alle R⁵⁴ bis R⁵⁸ Wasserstoff sind, oder 1, 2 oder 3, vorzugsweise 1 oder 2 von R⁵⁴ bis R⁵⁸ nicht Wasserstoff sind und aus den oben dargestellten Substitutionen ausgewählt sind, und die verbleibenden Positionen von R⁵⁴ bis R⁵⁸ aus Wasserstoff ausgewählt sind;
c) eine Thiadiazolgruppe mit einer der folgenden Strukturen: 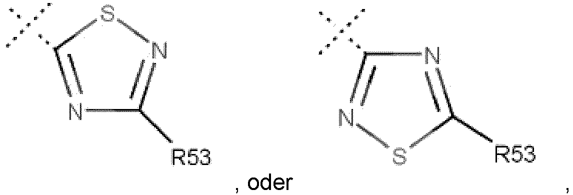
worin R⁵³ vorzugsweise wie oben definiert ist, und
vorzugsweise ist X ausgewählt aus -CH₂-, -C₂H₄-, -C₃H₆-, -C(CH₃)₂-, -CHCH(CH(₃₎)₂-, -CHC(CH₃)₃-, -CH(CH(CH₃)₂)-, -CH(CH₂CH(CH₃)₂)-, oder -CH(CH₂OH)-;
d) eine Isothiazolgruppe mit der folgenden Struktur: 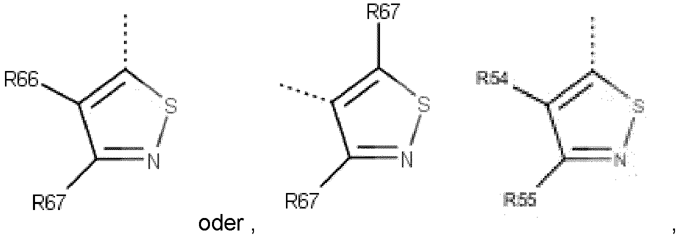 wobei R⁶⁶ und R⁶⁷ jeweils unabhängig voneinander ausgewählt sind aus Wasserstoff, -CH₃, -C₂H₅, - CH₂F; -CHF₂; -CF₃; -CH₂Cl; -CHCl₂; -CCl₃;-F, -Cl, -Br
und X vorzugsweise ausgewählt ist aus -CH₂-, -C₂H₄-, -C₃H₆-, -C(CH₃)₂-, - CHCH(CH(₃₎)₂-, -CHC(CH₃)₃-, -CH(CH(CH₃)₂)-, -CH(CH₂CH(CH₃)₂)-, oder - CH(CH₂OH)-; oder
e) eine Thiophengruppe, vorzugsweise 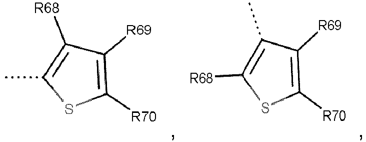 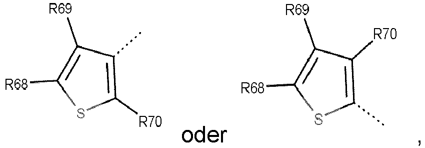 wobei R⁶⁸, R⁶⁹ und R⁷⁰ jeweils unabhängig voneinander ausgewählt sind aus Wasserstoff, -CH₃, -C₂H₅, -CH₂F; -CHF₂; -CF₃;-CH₂Cl; -CHCl₂; -CCl₃;-F, -Cl, -Br, vorzugsweise wobei einer der Reste R⁶⁸, R⁶⁹ oder R⁷⁰ Wasserstoff ist und der andere Rest ausgewählt ist aus -CH₃, -C₂H₅, -CF₃, -CHF₂, -F, -Cl, -Br, und vorzugsweise
X -CH₂-, -C₂H₄-, -C₃H₆-, -C(CH₃)₂-, -CHCH(CH₃)₂-, -CHC(CH₃)₃-, -CH(CH(CH₃)₂)-, - CH(CH₂CH(CH₃)₂)-, oder -CH(CH₂OH)- ist.

4. Verbindung zur Verwendung nach Anspruch 3, wobei R¹, R², R³, R⁴ und R⁵ jeweils unabhängig voneinander Wasserstoff, -OCH₃, -OCF₃, -OCH₂CH₃, -OCH(CH₃)₂, - - CH₃, -C₂H₅, -Cl,
-Br, -CN, -CH₂F; -CHF₂; -CF₃; -CH₂Cl; -CHCl₂; -CCl₃;NO₂, oder 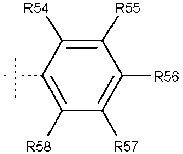 oder 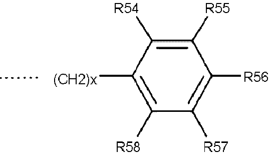 **ist, wobei** R⁵⁴, R⁵⁵, R⁵⁶, R⁵⁷ oder R⁵⁸ jeweils unabhängig voneinander aus Wasserstoff ausgewählt sind,
wobei X vorzugsweise nicht vorhanden ist, und
wobei vorzugsweise entweder alle R¹ bis R⁵ Wasserstoff sind, oder 1, 2 oder 3, vorzugsweise 1 oder 2 von R¹ bis R⁵ nicht Wasserstoff sind und aus den hier in Anspruch 8 dargestellten Substitutionen ausgewählt sind, und die verbleibenden Positionen von R¹ bis R⁵ aus Wasserstoff ausgewählt sind.

5. Verbindung zur Verwendung nach einem der Ansprüche 3 bis 4, wobei R⁶ und R⁷ jeweils unabhängig voneinander Wasserstoff sind, oder R⁶ und R⁷ zusammengenommen einen heterocyclischen Ring bilden, der ausgewählt ist aus:

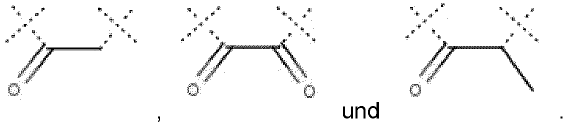

6. Verbindung zur Verwendung nach einem der Ansprüche 3 bis 4, wobei R⁷, N und X zusammengenommen einen sechsgliedrigen heterocyclischen Ring bilden,
ausgewählt aus 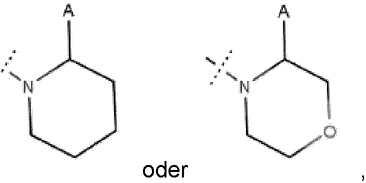 wobei A wie in Anspruch 3 definiert ist.

7. Verbindung zur Verwendung nach einem der Ansprüche 3 bis 6, worin R¹, R², R³, R⁴ und R⁵ jeweils Wasserstoff sind und/oder worin A 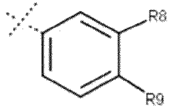 ist und R⁸ und R⁹ identisch oder voneinander verschieden sind, vorzugsweise voneinander verschieden, und R⁸ und R⁹ jeweils unabhängig voneinander ausgewählt sind aus Wasserstoff; -OH, -OCH₃, -OCF₃, -OCHF₂, -OCH₂OH; -OCH₂CH₃; -OCH(CH₃)₂; -F; - CI; -Br; -CN; NO₂; NH₂; -CH₃, -CH₂F; -CHF₂; -CF₃; -CH₂Cl; -CHCl₂; -CCl₃;CH(CH₃)₂; - CH₂CH₃; -CH-CH₂-CH(CH₃)₂; -CH₂-N(CH₃)₂; -CH(CH₃)-NHC(O)CH₃; - -NH-CH(CH₃)₂, -NH-Cyclopropyl, -CHF₂, -CF₃, -CH₃, CH₂CH₃, CH₂CH(CH₃)₂; -C(O)-C₂H₅; -C(O)-CH₃; -C(O)-Aziridin; -C(O)-Azetidin; -C(O)-Pyrrolidin; -C(CH₃)=NOH; -CH₂NHS(O)₂CH₃; - S(O)₂-Cyclopentyl; -S(O)₂-CH₃
-S(O)₂-cyclopentyl; Pyrrolidinyl 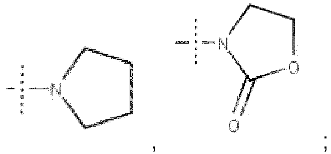
NHC(O)-R⁴¹, wobei R⁴¹ ausgewählt ist aus Furan, Tetrahydrofuran, Cyclopentyl, Cyclobutyl, Cyclopropyl, -NH-CH(CH₃)₂, -NH-Cyclopropyl, C₁-C₅Alkyl, -CH₂F; -CHF₂; -CF₃; -CH₂Cl; -CHCl₂; -CCl₃;-CH₃, CH₂CH₃, CH₂CH(CH₃)₂;
-C(O)OR⁴², wobei R⁴² ausgewählt ist aus -CH₃; -CH₂CH₃, -CH₂CH₂CH₃; -CH(CH₃)₂; - CH₂CH₂CH₂CH₃;
-NHR⁴³, wobei R⁴³ ausgewählt ist aus 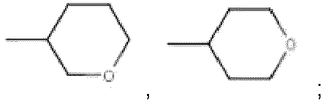 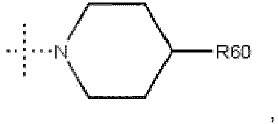 wobei R⁶⁰ ausgewählt ist aus Wasserstoff oder -CH₃; 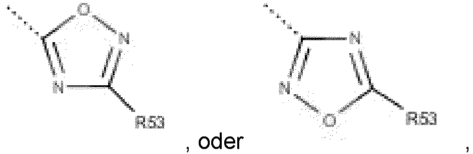
wobei
R⁵³ ist ausgewählt aus der Gruppe bestehend aus Wasserstoff, OH, -OCH₃, -OCF₃, - OCHF₂, -OCH₂OH; -OCH₂CH₃; -F; -Cl; -Br; -CN; NO₂; NH₂; -CH₃, CH(CH₃)₂; -CH₂CH₃; -CH-CH₂-CH(CH₃)₂; -CH₂-N(CH₃)₂; -CH(CH₃)-NHC(O)CH₃; -CH₂F; -CHF₂; -CF₃; - CH₂Cl; -CHCl₂; -CCl₃;-O-N=O, -N=O, -CH=NH, -N₃, -SH, -SO₃H, -SO₂H, -S(O)₂CH₃-CN, -NC,-OCN, -NCO, -(C=O)OH, -(C=O)NHOH, -NH(C=O)NH₂, -NH(C=S)NH₂, - (C=O)NH₂,-CHO,-CHS, -OCHO, -NCHO, 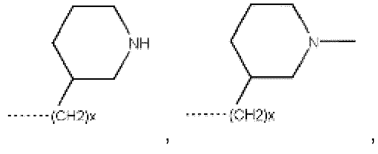 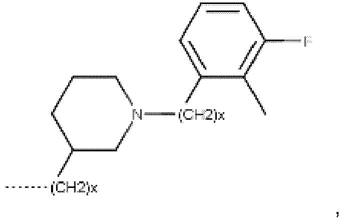 mit X = 0 oder 1, vorzugsweise 1; 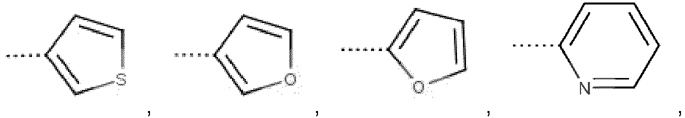 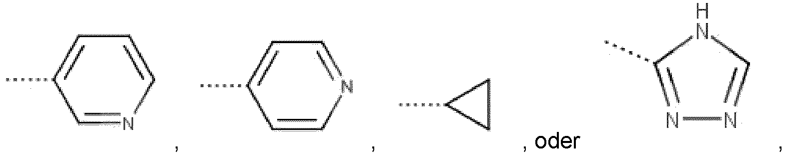 oder
R⁵³ ist 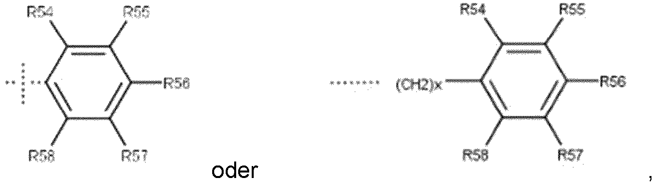 wobei R⁵⁴, R⁵⁵, R⁵⁶, R⁵⁷ oder R⁵⁸ jeweils unabhängig voneinander ausgewählt sind aus Wasserstoff, -CH₃, -C₂H₅, -CH₂F; -CHF₂; -CF₃; -CH₂Cl; -CHCl₂; -CCl₃; -OCH₃, -F, -Cl, -Br, NH₂, NO₂, und x 0, 1 oder 2 ist;
oder R⁵³ ist 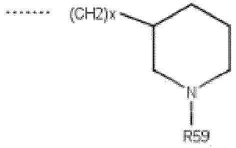 worin R⁵⁹ 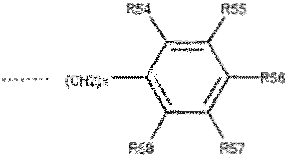 ist und worin R⁵⁴, R⁵⁵, R⁵⁶, R⁵⁷ oder R⁵⁸ unabhängig von den oben genannten ausgewählt sind aus Wasserstoff, -CH₃, -C₂H₅, -CH₂F; -CHF₂; -CF₃; -CH₂Cl; -CHCl₂; -CCl₃; -OCH₃, -F, - Cl, -Br, NO₂, NH₂, und x 0, 1 oder 2 ist; wie oben definiert; oder optional
R⁸ und R⁹ zusammen einen fünf- oder sechsgliedrigen Cycloalkyl-, fünf- oder sechsgliedrigen Cycloheteroalkyl- oder fünf- oder sechsgliedrigen Aryl-, fünf- oder sechsgliedrigen Heteroarylring bilden, wobei der heterocyclische Ring vorzugsweise ausgewählt ist aus:

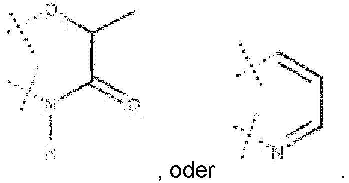

8. Verbindung zur Verwendung nach Anspruch 7, mit der Maßgabe, dass R⁸ oder R⁹ nicht identisch miteinander sind.

9. Verbindung zur Verwendung nach einem der Ansprüche 3 bis 8, wobei R¹ bis R⁵ wie folgt ausgewählt sind:
R¹ ist Wasserstoff, -C₁-C₂ Alkoxy, -C(O)O-C₁-C₅ Alkyl, -OCH₃, -C₁-C₃ Alkyl, Methyl oder Ethyl;
R² ist Wasserstoff, Methyl, -F, -Cl, -Br, -CN, -OCH₃ oder -C(O)O-C₁-C₅-Alkyl;
R³ ist Wasserstoff, Methyl, -CH(CH₃)₂, -CHF₂, -F, -Cl, -Br, -CN, -OCH₃, -OCF₃, NO₂ oder C(O)O-C₁-C₅-Alkyl,
R⁴ ist Wasserstoff oder -F, -Cl, -Br oder -CF₃; und/oder
R⁵ ist Wasserstoff, Methyl, Ethyl oder -F, -Cl, -Br oder -CN, -CH₂-Phenyl.

10. Verbindung zur Verwendung nach einem der Ansprüche 3 bis 6, wobei die Verbindung eine Verbindung nach einer der Formeln (Ib), (Ib1), (Ib2) oder (Ib3) ist: 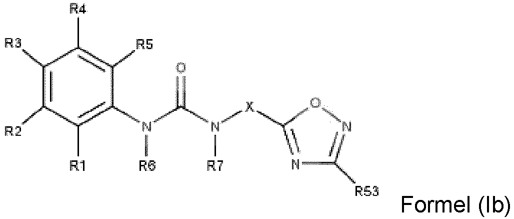 oder 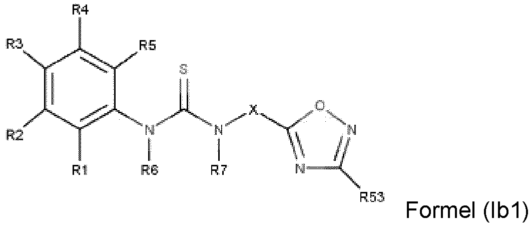 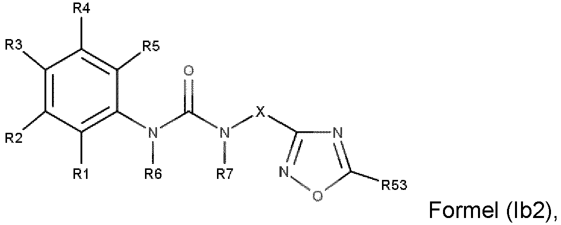 oder 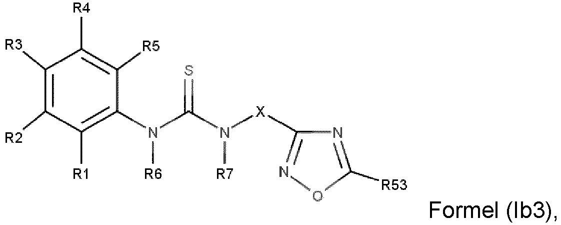
worin X ausgewählt ist aus der Gruppe bestehend aus einem C₁-C₃-Alkyl,-CH(CH₃)₂ -CH₂-, -C₂H₄-, -C₃H₆-, -CHCH(CH₃)₂-, -CHC(CH₃)₃-, -CH(CH(CH(₃))₂)-, oder - CH(CH₂CH(CH₃)(2))-, oder -CH(CH₂OH)-, oder nicht vorhanden ist, und
worin R⁵³ ausgewählt ist aus der Gruppe bestehend aus Wasserstoff, OH, -OCH₃, - OCF₃, -OCHF₂, -OCH₂OH; -OCH₂CH₃; -F; -Cl; -Br; -CN; NO₂; NH₂; C₁-C₅-Alkyl, C₁-C₅-Alkoxy, C₂-C₅-Alkenyl, vorzugsweise -CH₃, CH(CH₃)₂; -CH₂CH₃; -CH-CH₂-CH(CH₃)₂; -CH₂-N(CH₃)₂; -CH(CH₃)-NHC(O)CH₃; -CH₂F; -CHF₂; -CF₃; -CH₂Cl; - CHCI₂; -CCl₃;-O-N=O, -N=O, -CH=NH, -N₃, -SH, -SO₃H, -SO₂H, -S(O)₂CH₃-CN, - NC,-OCN, -NCO, -(C=O)OH, -(C=O)NHOH, -NH(C=O)NH₂, -NH(C=S)NH₂, - (C=O)NH₂,-CHO,-CHS, -OCHO, -NCHO, 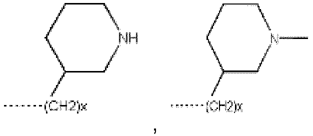 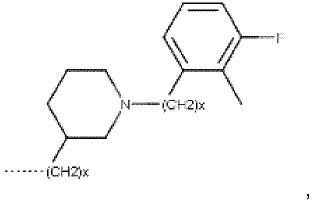 mit X = 0 oder 1, vorzugsweise 1, 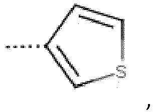 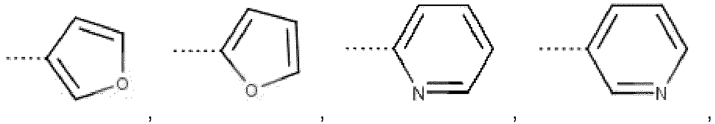 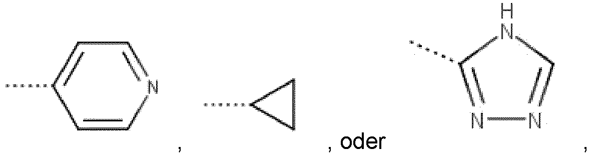 oder
R⁵³ ist 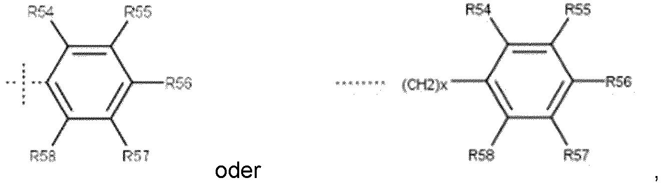 wobei R⁵⁴, R⁵⁵, R⁵⁶, R⁵⁷ oder R⁵⁸ jeweils unabhängig voneinander ausgewählt sind aus Wasserstoff, -CH₃, -C₂H₅, -CH₂F; -CHF₂; -CF₃; -CH₂Cl; -CHCl₂; -CCl₃; -OCH₃, -F, - Cl, -Br, NH₂, NO₂, und x 0, 1 oder 2 ist;
oder R⁵³ ist 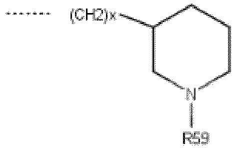 worin R⁵⁹ 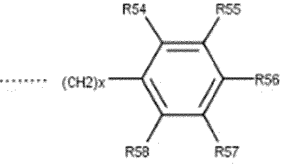 ist und worin R⁵⁴, R⁵⁵, R⁵⁶, R⁵⁷ oder R⁵⁸ unabhängig von den oben genannten ausgewählt sind aus Wasserstoff, -CH₃, -C₂H₅, -CH₂F; -CHF₂; -CF₃; -CH₂Cl; -CHCl₂; -CCl₃; -OCH₃, -F, - Cl, -Br, NO₂, NH₂, und x 0, 1 oder 2 ist; wie oben definiert, und x 0, 1 oder 2 ist;
und wobei vorzugsweise entweder alle R⁵⁴ bis R⁵⁸ Wasserstoff sind, oder 1, 2 oder 3, vorzugsweise 1 oder 2 von R⁵⁴ bis R⁵⁸ nicht Wasserstoff sind und aus den oben dargestellten Substitutionen ausgewählt sind, und die verbleibenden Positionen von R⁵⁴ bis R⁵⁸ aus Wasserstoff ausgewählt sind.

11. Verbindung zur Verwendung nach Anspruch 1, wobei die Verbindung eine Verbindung der Formel (Id) ist: 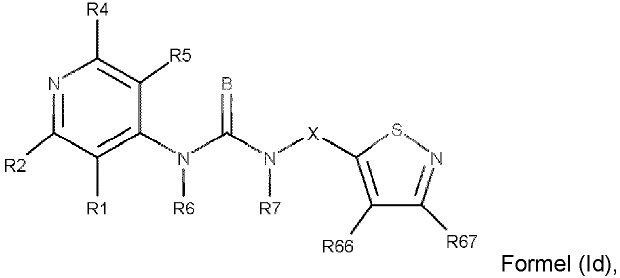 wobei vorzugsweise
B O oder S ist, vorzugsweise O,
X ausgewählt ist aus C₁-C₅-Alkyl, -CH₂-, -C₂H₄-, -C₃H₆-, -C(CH₃)₂-, -CHCH(CH₍₃₎₎₂₎-, -CHC(CH₃)₃-, -CH(CH(CH₃₎₂₎-, -CH(CH₂CH(CH₃)₂)-, oder -CH(CH₂OH)-;
R¹, R², R⁴ und R⁵ unabhängig voneinander ausgewählt sind aus Wasserstoff, -CH₃, - C₂H₅, -CH₂CH₃-CH₂F; -CHF₂; -CF₃; -CH₂Cl; -CHCl₂; -CCl₃; -OCH₃, -F, -Cl, -Br, NO₂, NH₂, wobei vorzugsweise zwei oder drei von R¹, R², R⁴ und R⁵ Wasserstoff sind und die übrigen nicht Wasserstoff sind; und
R⁵⁴ und R⁵⁵ unabhängig voneinander ausgewählt sind aus Wasserstoff, -CH₃, -C₂H₅, -CH₂F; -CHF₂; -CF₃; -CH₂Cl; -CHCl₂; -CCl₃;-OCH₃, -F, -Cl, -Br, NO₂, NH₂, vorzugsweise worin eines von R⁵⁴ und R⁵⁵ Wasserstoff ist und das andere eines von -CH₃, -C₂H₅, -CH₂F; -CHF₂; -CF₃; -CH₂Cl; -CHCl₂; -CCl₃; -OCH₃, -F, -Cl, -Br, NO₂, NH₂ ist.

12. Verbindung zur Verwendung nach einem der Ansprüche 3 bis 6 und 10 bis 11, wobei die Verbindung eine Verbindung nach einer der Formeln (Ib), (Ib1), (Ib2), (Ib3) oder (Id) ist und R¹ bis R⁵ wie folgt ausgewählt sind:
R¹ Wasserstoff, -C₁-C₂ Alkoxy, -C(O)O-C₁-C₅ Alkyl, -OCH₃, -C₁-C₃ Alkyl, Methyl oder Ethyl ist
R² t Wasserstoff, Methyl, -F, -Cl, -Br, -CN, -OCH₃ oder -C(O)O-C₁-C₅-Alkyl ist;
R³, falls vorhanden, Wasserstoff, Methyl, -CH(CH₃)₂, -CHF₂, -F, -Cl, -Br, -CN, -OCH₃, -OCF₃, NO₂ oder -C(O)O-(C₁-C₅-Alkyl) ist;
R⁴ Wasserstoff oder -F, -Cl, -Br oder -CF₃ ist; und/oder
R⁵ Wasserstoff, Methyl, Ethyl oder -F, -Cl, -Br, -CN oder -CH₂-Phenyl ist.

13. Verbindung zur Verwendung nach einem der Ansprüche 1 bis 12, wobei die Verbindung aus den in Tabelle 1 beschriebenen Verbindungen ausgewählt ist.
**Tabelle 1:**
| **NO** | **Code** | **Struktur** | **IUPAC-Name** |
|---|---|---|---|
| **1** | DE22 | 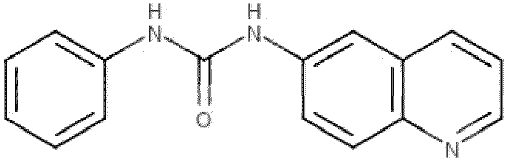 | 1-phenyl-3-(quinolin-6-yl)urea |
| **2** | DE21 | 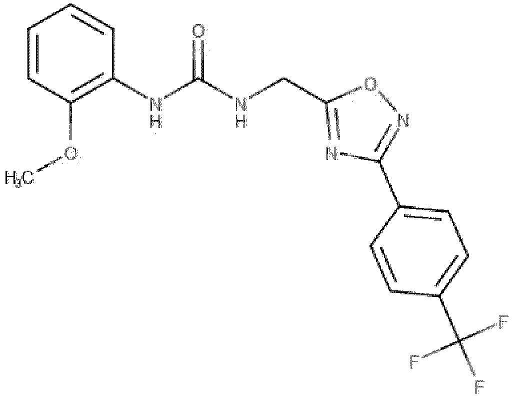 | 1-(2-methoxyphenyl)-3-((3-(4-(trifluoromethyl)p henyl)-1,2,4-oxadiazol-5-yl)methyl)urea |
| **3** | EN-A (EN23) | 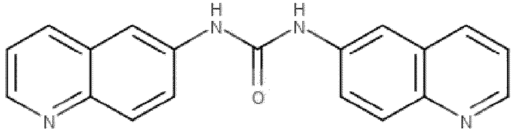 | 1,3-di(quinolin-6-yl)urea |
| **4** | AH4 | 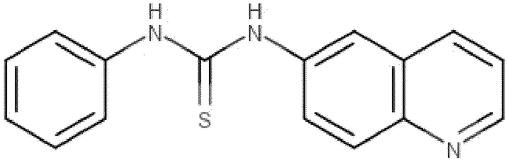 | 3-phenyl-1-(quinolin-6-yl)thiourea |
| **5** | JB010 | 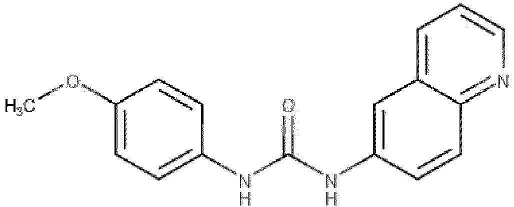 | 1-(4-methoxyphenyl)-3-(quinolin-6-yl)urea |
| **6** | BRM/BR G1 ATP-Inhibitor-1 | 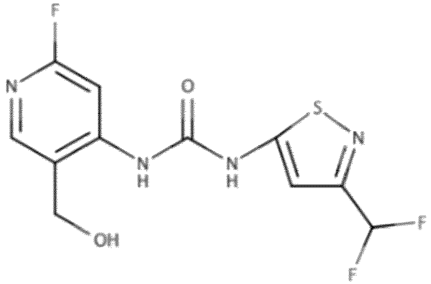 | 1-[3-(difluoromethyl)-1,2-thiazol-5-yl]-3-[2-fluoro-5-(hydroxymethyl)p yridin-4-yl]urea |
| **7** | J B011 | 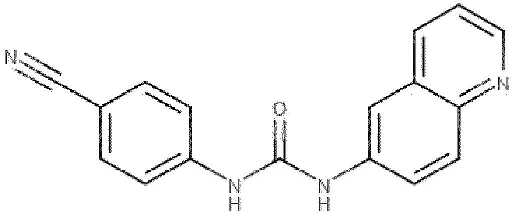 | 1-(4-cyanophenyl)-3-(quinolin-6-yl)urea |
| **8** | PM07 | 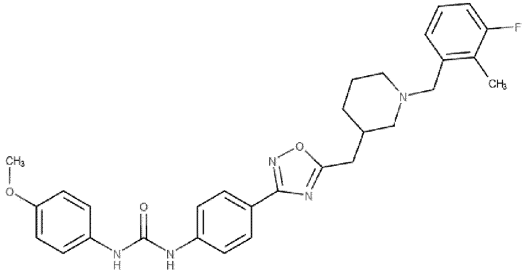 | 3-{4-[5-({1-[(3-fluoro-2-methylphenyl)me thyl]piperidin-3-yl}methyl)-1,2,4-oxadiazol-3-yl]phenyl}-1-(4-methoxyphenyl)u rea |
| **9** | PM16 | 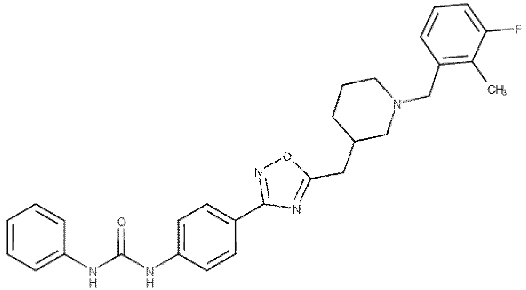 | 3-{4-[5-({1-[(3-fluoro-2-methylphenyl)me thyl]piperidin-3-yl}methyl)-1,2,4-oxadiazol-3-yl]phenyl}-1-phenylurea |
| **10** | JB014 | 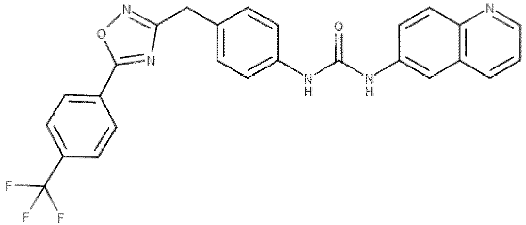 | 1-(quinolin-6-yl)-3-[4-({5-[4-(trifluoromethyl)p henyl]-1,2,4-oxadiazol-3-yl}methyl)phenyl] urea |
| **11** | AH1 | 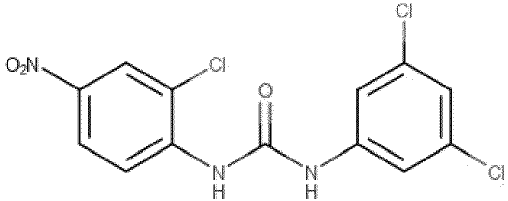 | 3-(2-chloro-4-nitrophenyl)-1-(3,5-dichlorophenyl)ur ea |
| **12** | AH3 | 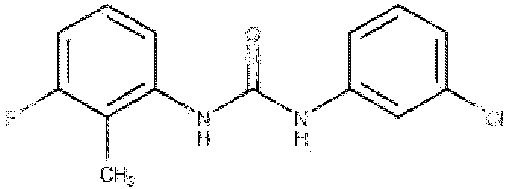 | 1-(3-chlorophenyl)-3-(3-fluoro-2-methylphenyl)ure a |
| **13** | JB002 | 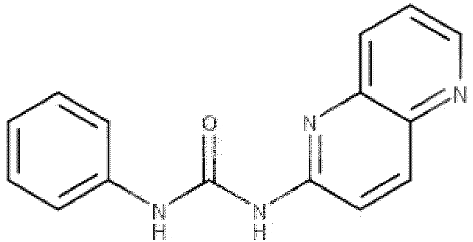 | 1-(1,5-naphthyridin-2-yl)-3-phenylurea |
| **14** | JB004 | 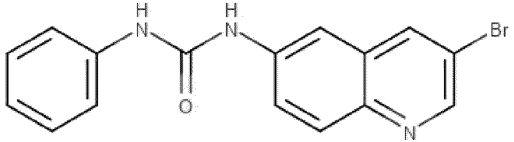 | 1-(3-bromoquinolin-6-yl)-3-phenylurea |
| **15** | DE11-16 | 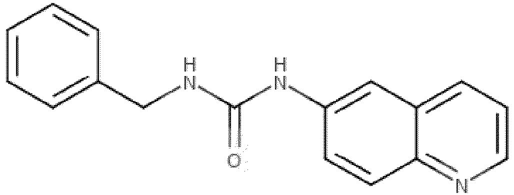 | 1-benzyl-3-(quinolin-6-yl)urea |
| **16** | DE11-13 | 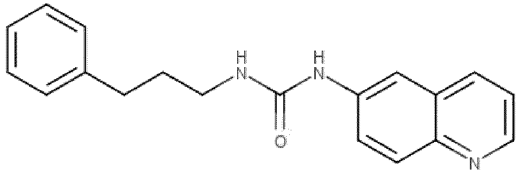 | 1-(3-phenylpropyl)-3-(quinolin-6-yl)urea |
| **17** | L01.110 | 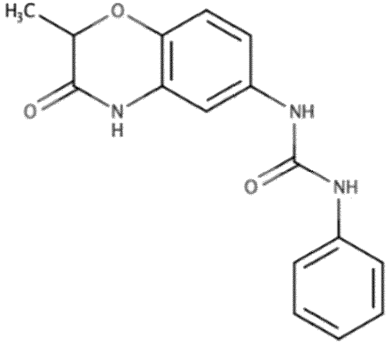 | 3-(2-methyl-3-oxo-3,4-dihydro-2H-1,4-benzoxazin-6-yl)-1-phenylurea |
| **18** | L01.041 | 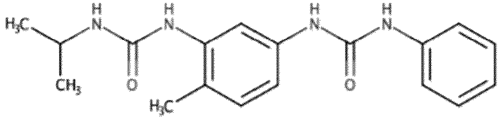 | 3-(4-methyl-3-{[(propan-2-yl)carbamoyl]ami no}phenyl)-1-phenylurea |
| **19** | L01.004 | 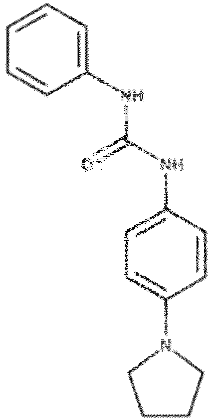 | 3-phenyl-1-[4-(pyrrolidin-1-yl)phenyl]urea |
| **20** | L01.007 | 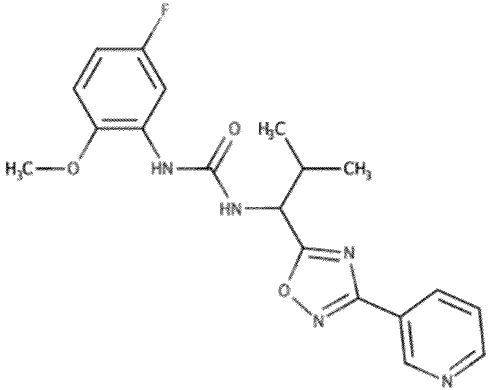 | 1-(5-fluoro-2-methoxyphenyl)-3-{2-methyl-1-[3-(pyridin-3-yl)-1,2,4-oxadiazol-5-yl]propyl}urea |
| **21** | L01.104 | 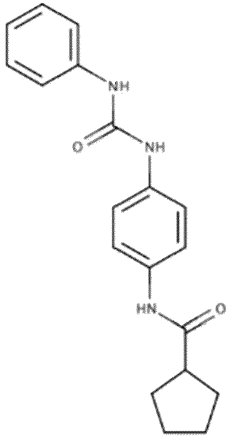 | N-{4-[(phenylcarbamo yl)amino]phenyl} cyclopentanecar boxamide |
| **22** | L01.116 | 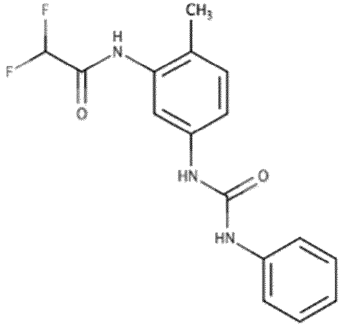 | 2,2-difluoro-N-{2-methyl-5-[(phenylcarbamo yl)amino]phenyl} acetamide |
| **23** | L01.113 | 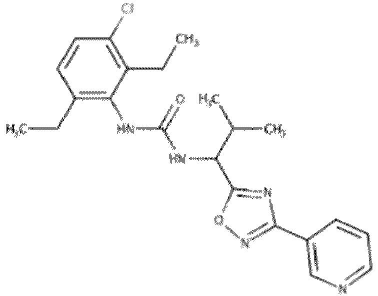 | 1-(3-chloro-2,6-diethylphenyl)-3-{2-methyl-1-[3-(pyridin-3-yl)-1,2,4-oxadiazol-5-yl]propyl}urea |
| **24** | L01.101 | 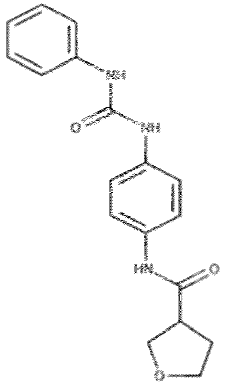 | N-{4-[(phenylcarbamo yl)amino]phenyl} oxolane-3-carboxamide |
| **25** | L01.092 | 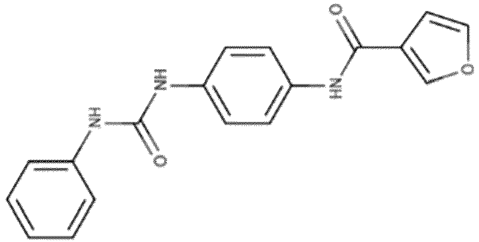 | N-{4-[(phenylcarbamo yl)amino]phenyl}f uran-3-carboxamide |
| **26** | L01.124 | 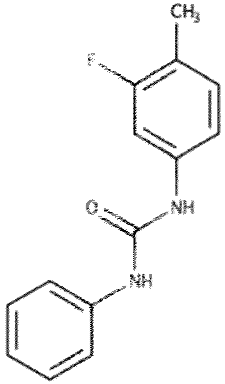 | 3-(3-fluoro-4-methylphenyl)-1-phenylurea |
| **27** | TCMDC-143175 | 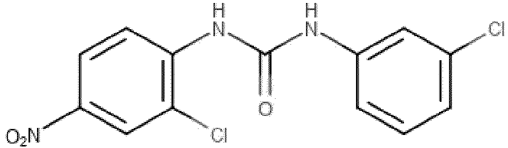 | 3-(2-chloro-4-nitrophenyl)-1-(3-chlorophenyl)ure a |
| **28** | A.C2 | 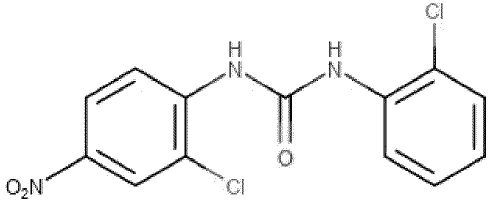 | 3-(2-chloro-4-nitrophenyl)-1-(2-chlorophenyl)ure a |
| **29** | AH2 | 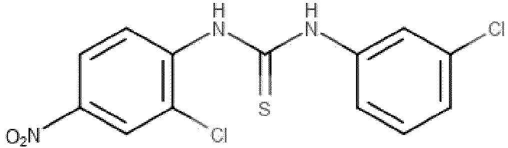 | 3-(2-chloro-4-nitrophenyl)-1-(3-chlorophenyl)thio urea |
| **30** | EN11-07 | 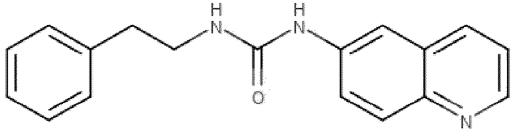 | 1-(2-phenylethyl)-3-(quinolin-6-yl)urea |
| **31** | EN11-02 | 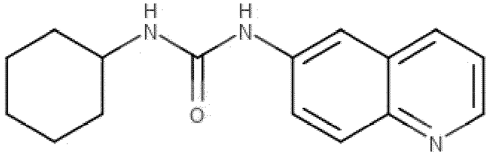 | 1-cyclohexyl-3-(quinolin-6-yl)urea |
| **32** | EN11-11 | 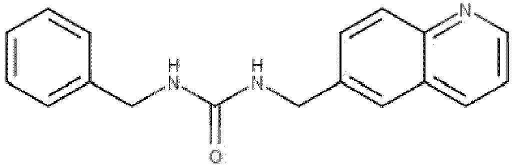 | 3-benzyl-1-[(quinolin-6-yl)methyl]urea |
| **33** | L01.016 | 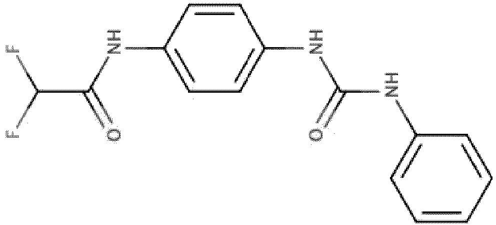 | 2,2-difluoro-N-{4-[(phenylcarbamo yl)amino]phenyl} acetamide |
| **34** | L01.028 | 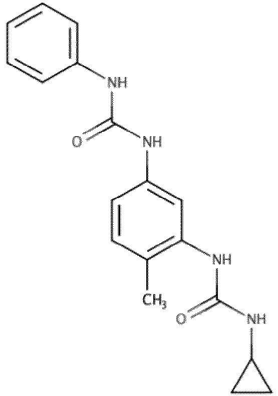 | 3-cyclopropyl-1-{2-methyl-5-[(phenylcarbamo yl)amino]phenyl} urea |
| **35** | L01.047 | 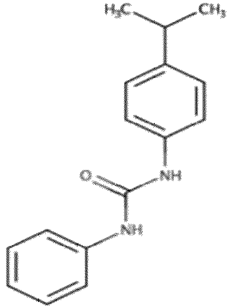 | 1-phenyl-3-[4-(propan-2-yl)phenyl]urea |
| **36** | L01.026 | 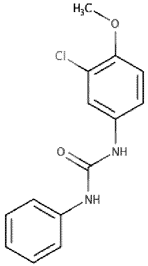 | 3-(3-chloro-4-methoxyphenyl)-1-phenylurea |
| **37** | L01.127 | 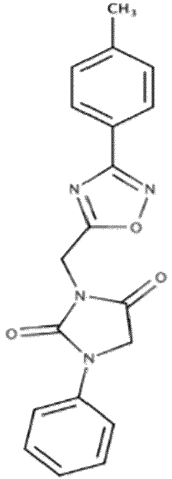 | 3-{[3-(4-methylphenyl)-1,2,4-oxadiazol-5-yl]methyl}-1-phenylimidazolidi ne-2,4-dione |
| **38** | L01.061 | 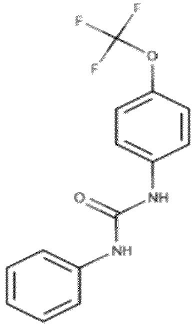 | 1-phenyl-3-[4-(trifluoromethoxy )phenyl]urea |
| **39** | L01.128 | 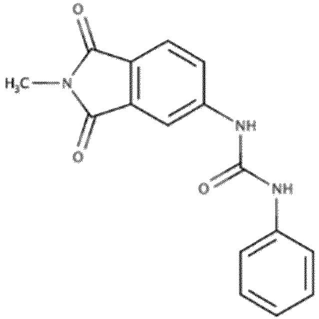 | 3-(2-methyl-1,3-dioxo-2,3-dihydro-1H-isoindol-5-yl)-1-phenylurea |
| **40** | L01.025 | 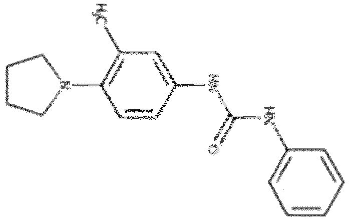 | 3-[3-methyl-4-(pyrrolidin-1-yl)phenyl]-1-phenylurea |
| **41** | L01.008 | 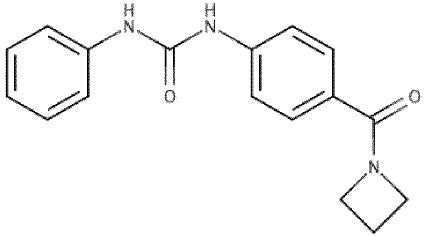 | 1-[4-(azetidine-1-carbonyl)phenyl]-3-phenylurea |
| **42** | L01.089 | 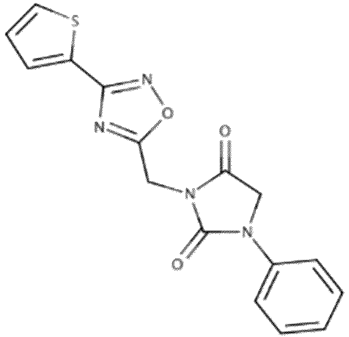 | 1-phenyl-3-{[3-(thiophen-2-yl)-1,2,4-oxadiazol-5-yl]methyl}imidaz olidine-2,4-dione |
| **43** | L01.130 | 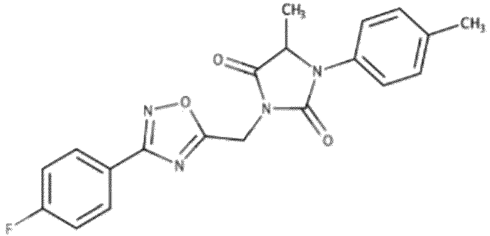 | 3-{[3-(4-fluorophenyl)-1,2,4-oxadiazol-5-yl]methyl}-5-methyl-1-(4-methylphenyl)imi dazolidine-2,4-dione |
| **44** | L01.049 | 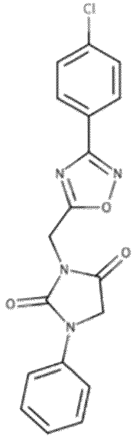 | 3-{[3-(4-chlorophenyl)-1,2,4-oxadiazol-5-yl]methyl}-1-phenylimidazolidi ne-2,4-dione |
| **45** | L01.066 | 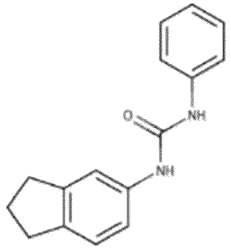 | 3-(2,3-dihydro-1H-inden-5-yl)-1-phenylurea |
| **46** | L01.043 | 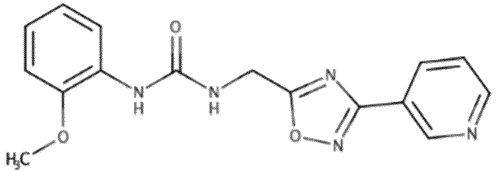 | 1-(2-methoxyphenyl)-3-{[3-(pyridin-3-yl)-1,2,4-oxadiazol-5-yl]methyl}urea |
| **47** | L01.087 | 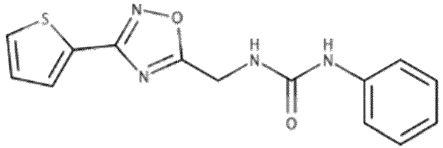 | 1-phenyl-3-{[3-(thiophen-2-yl)-1,2,4-oxadiazol-5-yl]methyl}urea |
| **48** | L01.020 | 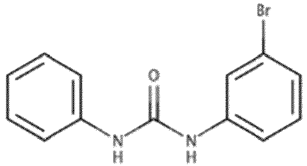 | 3-(3-bromophenyl)-1-phenylurea |
| **49** | L01.035 | 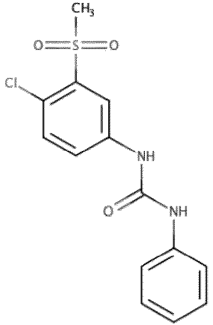 | 3-(4-chloro-3-methanesulfonyl phenyl)-1-phenylurea |
| **50** | L01.120 | 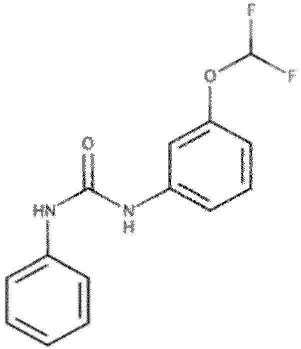 | 3-[3-(difluoromethoxy )phenyl]-1-phenylurea |
| **51** | L01.033 | 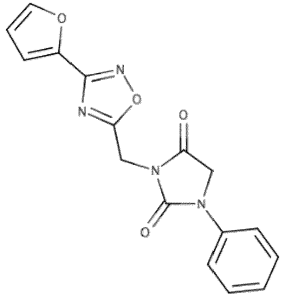 | 3-{[3-(furan-2-yl)-1,2,4-oxadiazol-5-yl]methyl}-1-phenylimidazolidi ne-2,4-dione |
| **52** | L01.002 | 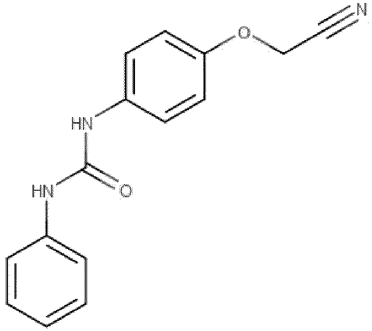 | 1-[4-(cyanomethoxy)p henyl]-3-phenylurea |
| **53** | L01.027 | 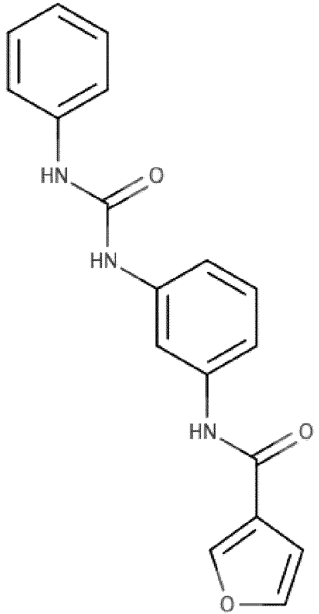 | N-{3-[(phenylcarbamo yl)amino]phenyl}f uran-3-carboxamide |
| **54** | L01.003 | 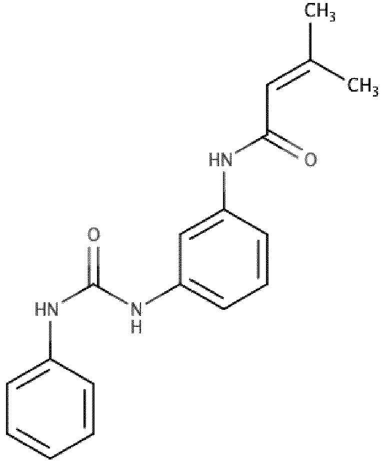 | 3-methyl-N-{3-[(phenylcarbamo yl)amino]phenyl} but-2-enamide |
| **55** | L01.038 | 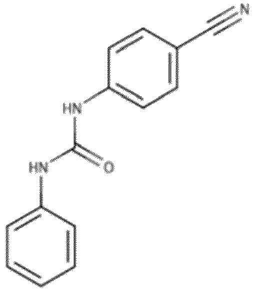 | 1-(4-cyanophenyl)-3-phenylurea |
| **56** | L01.069 | 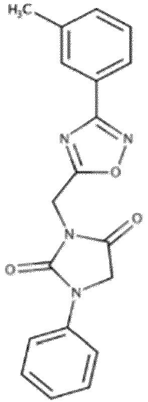 | 3-{[3-(3-methylphenyl)-1,2,4-oxadiazol-5-yl]methyl}-1-phenylimidazolidi ne-2,4-dione |
| **57** | L01.097 | 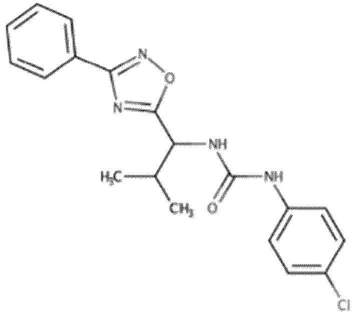 | 1-(4-chlorophenyl)-3-[2-methyl-1-(3-phenyl-1,2,4-oxadiazol-5-yl)propyl]urea |
| **58** | L01.095 | 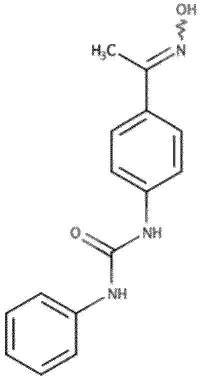 | 3-{4-[1-(hydroxyimino)et hyl]phenyl}-1-phenylurea |
| **59** | L01.013 | 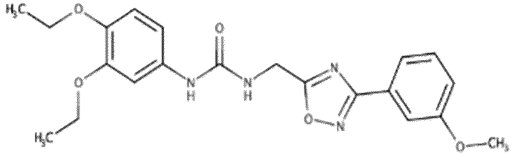 | 1-(3,4-diethoxyphenyl)-3-{[3-(3-methoxyphenyl)-1,2,4-oxadiazol-5-yl]methyl}urea |
| **60** | L01.103 | 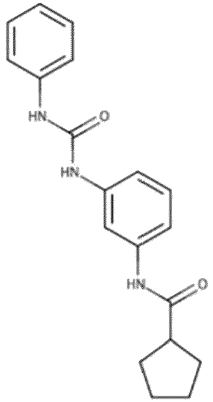 | N-{3-[(phenylcarbamo yl)amino]phenyl} cyclopentanecar boxamide |
| **61** | L01.071 | 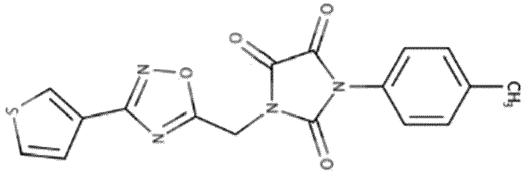 | 1-(4-methylphenyl)-3-{[3-(thiophen-3-yl)-1,2,4-oxadiazol-5-yl]methyl}imidaz olidine-2,4,5-trione |
| **62** | L01.115 | 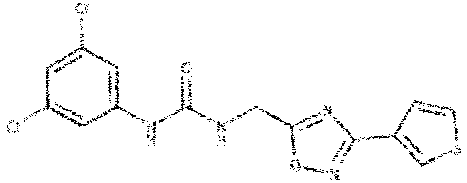 | 1-(3,5-dichlorophenyl)-3-{[3-(thiophen-3-yl)-1,2,4-oxadiazol-5-yl]methyl}urea |
| **63** | L01.045 | 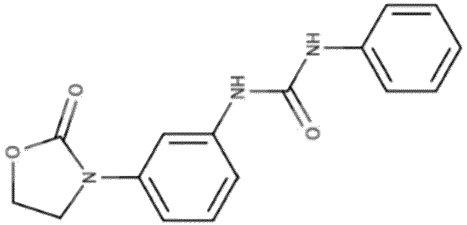 | 1-[3-(2-oxo-1,3-oxazolidin-3-yl)phenyl]-3-phenylurea |
| **64** | L01.077 | 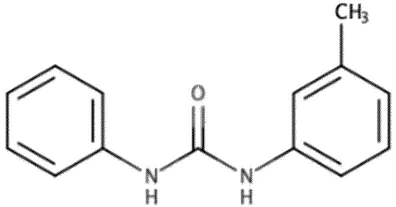 | 3-(3-methylphenyl)-1-phenylurea |
| **65** | L01.084 | 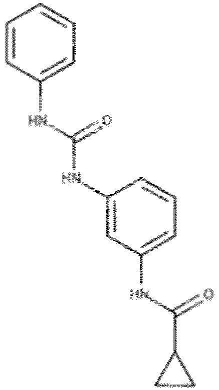 | N-{3-[(phenylcarbamo yl)amino]phenyl} cyclopropanecar boxamide |
| **66** | L01.109 | 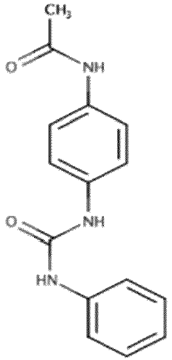 | N-{4-[(phenylcarbamo yl)amino]phenyl} acetamide |
| **67** | L01.122 | 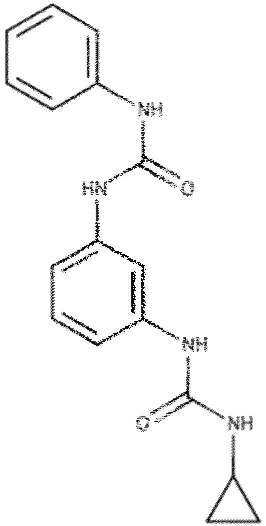 | 3-cyclopropyl-1-{3-[(phenylcarbamo yl)amino]phenyl} urea |
| **68** | L01.083 | 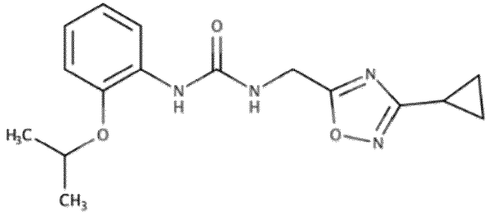 | 3-[(3-cyclopropyl-1,2,4-oxadiazol-5-yl)methyl]-1-[2-(propan-2-yloxy)phenyl]ure a |
| **69** | L01.062 | 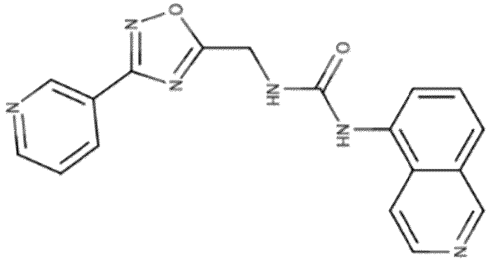 | 1-(isoquinolin-5-yl)-3-{[3-(pyridin-3-yl)-1,2,4-oxadiazol-5-yl]methyl}urea |
| **70** | L01.081 | 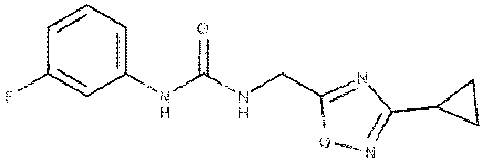 | 3-[(3-cyclopropyl-1,2,4-oxadiazol-5-yl)methyl]-1-(3-fluorophenyl)ure a |
| **71** | L01.114 | 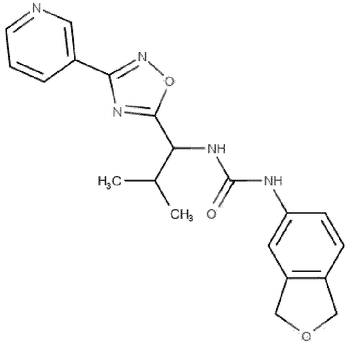 | 3-(1,3-dihydro-2-benzofuran-5-yl)-1-{2-methyl-1-[3-(pyridin-3-yl)-1,2,4-oxadiazol-5-yl]propyl}urea |
| **72** | L01.090 | 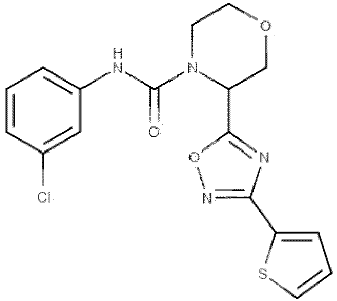 | N-(3-chlorophenyl)-3-[3-(thiophen-2-yl)-1,2,4-oxadiazol-5-yl]morpholine-4-carboxamide |
| **73** | L01.136 | 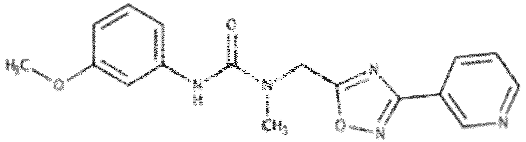 | 1-(3-methoxyphenyl)-3-methyl-3-{[3-(pyridin-3-yl)-1,2,4-oxadiazol-5-yl]methyl}urea |
| **74** | L01.023 | 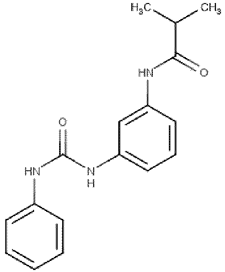 | 2-methyl-N-{3-[(phenylcarbamo yl)amino]phenyl} propanamide |
| **75** | L01.064 | 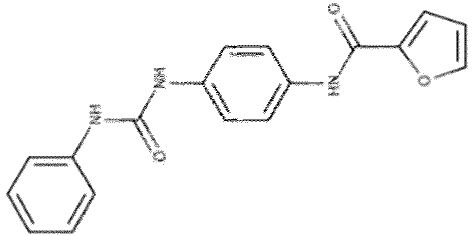 | N-{4-[(phenylcarbamo yl)amino]phenyl}f uran-2-carboxamide |
| **76** | L01.093 | 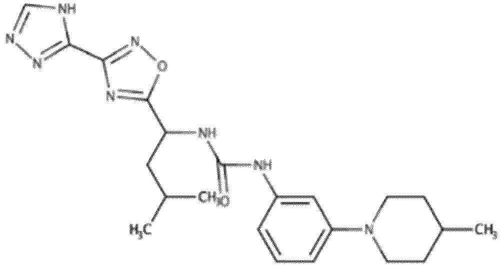 | 3-{3-methyl-1-[3-(4H-1,2,4-triazol-3-yl)-1,2,4-oxadiazol-5-yl]butyl}-1-[3-(4-methylpiperidin-1-yl)phenyl]urea |
| **77** | L01.125 | 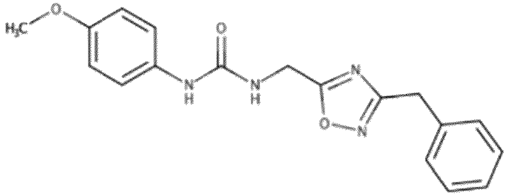 | 3-[(3-benzyl-1,2,4-oxadiazol-5-yl)methyl]-1-(4-methoxyphenyl)u rea |
| **78** | L01.005 | 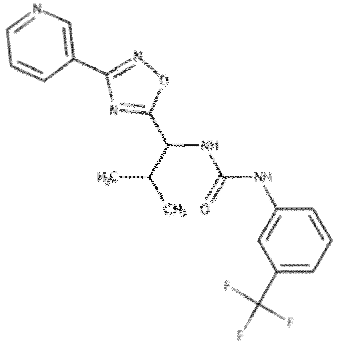 | 3-{2-methyl-1-[3-(pyridin-3-yl)-1,2,4-oxadiazol-5-yl]propyl}-1-[3-(trifluoromethyl)p henyl]urea |
| **79** | L01.135 | 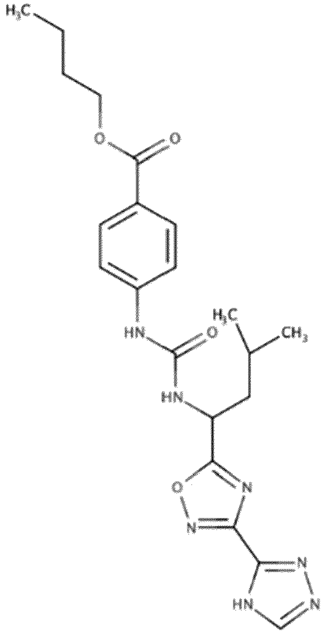 | butyl 4-[({3-methyl-1-[3-(4H-1,2,4-triazol-3-yl)-1,2,4-oxadiazol-5-yl]butyl}carbamo yl)amino]benzoat e |
| **80** | L01.108 | 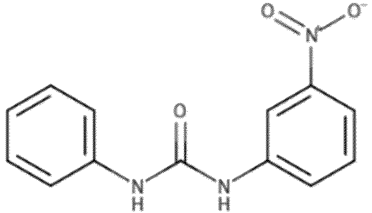 | 3-(3-nitrophenyl)-1-phenylurea |
| **81** | L01.112 | 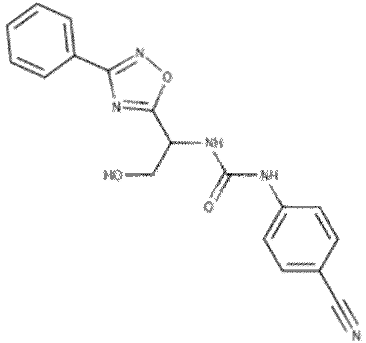 | 1-(4-cyanophenyl)-3-[2-hydroxy-1-(3-phenyl-1,2,4-oxadiazol-5-yl)ethyl]urea |
| **82** | L01.054 | 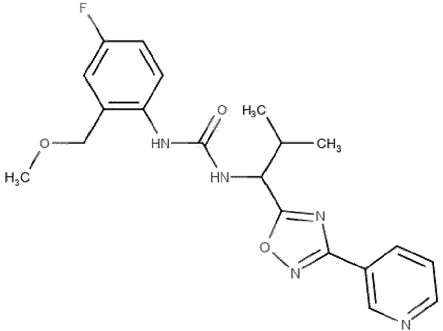 | 1-[4-fluoro-2-(methoxymethyl) phenyl]-3-{2-methyl-1-[3-(pyridin-3-yl)-1,2,4-oxadiazol-5-yl]propyl}urea |
| **83** | L01.067 | 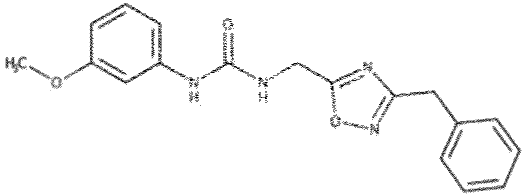 | 3-[(3-benzyl-1,2,4-oxadiazol-5-yl)methyl]-1-(3-methoxyphenyl)u rea |
| **84** | L01.099 | 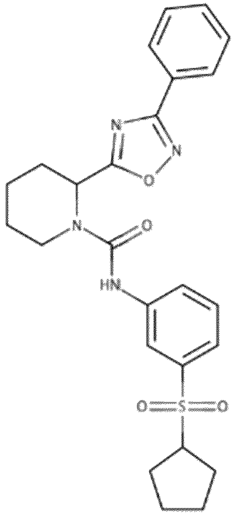 | N-[3-(cyclopentanesul fonyl)phenyl]-2-(3-phenyl-1,2,4-oxadiazol-5-yl)piperidine-1-carboxamide |
| **85** | L01.065 | 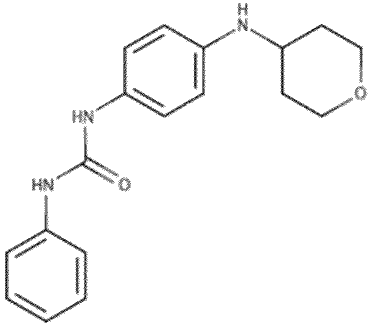 | 1-{4-[(oxan-4-yl)amino]phenyl}-3-phenylurea |
| **86** | L01.132 | 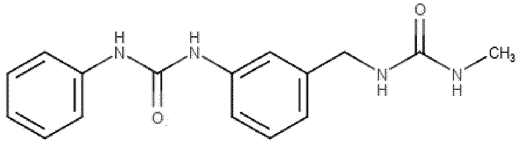 | 3-(3-{[(methylcarbam oyl)amino]methyl }phenyl)-1-phenylurea |
| **87** | L01.121 | 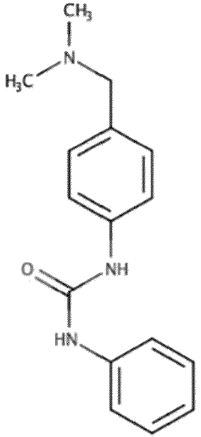 | 3-{4-[(dimethylamino) methyl]phenyl}-1-phenylurea |
| **88** | L01.056 | 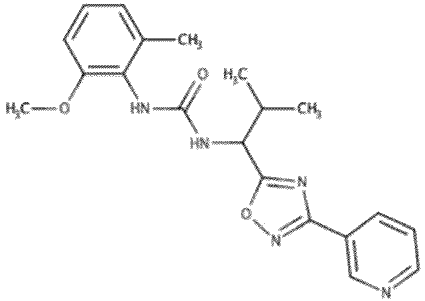 | 1-(2-methoxy-6-methylphenyl)-3-{2-methyl-1-[3-(pyridin-3-yl)-1,2,4-oxadiazol-5-yl]propyl}urea |
| **89** | L01.088 | 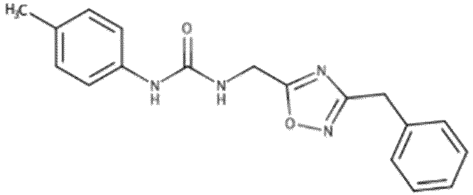 | 3-[(3-benzyl-1,2,4-oxadiazol-5-yl)methyl]-1-(4-methylphenyl)ure a |
| **90** | L01.042 | 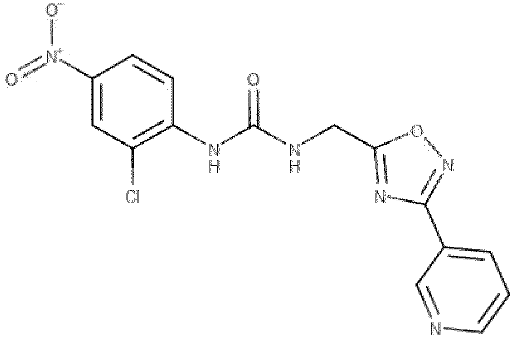 | 1-(2-chloro-4-nitrophenyl)-3-{[3-(pyridin-3-yl)-1,2,4-oxadiazol-5-yl]methyl}urea |
| **91** | L01.010 | 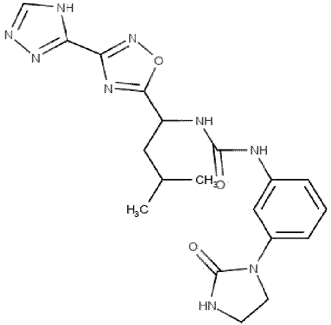 | 3-{3-methyl-1-[3-(4H-1,2,4-triazol-3-yl)-1,2,4-oxadiazol-5-yl]butyl}-1-[3-(2-oxoimidazolidin-1-yl)phenyl]urea |

14. Verbindung nach einem der Ansprüche 1 bis 13 zur Verwendung bei der Vorbeugung oder Behandlung einer Krankheit durch Aktivierung des NLRP3-Inflammasoms, wobei die Krankheit aus einer Gruppe ausgewählt ist, die Infektionskrankheiten oder Krebs umfasst, oder zur Verwendung zur Verstärkung von Immunantworten oder zum Durchbrechen der Immunevasion bei Krebskrankheiten in einem Subjekt, oder zur Verwendung als Adjuvans im Zusammenhang mit einer Impfung, um spezifische Immunantworten durch Aktivierung des NLRP3-Inflammasoms zu verstärken.

15. Verwendung der in Anspruch 13 definierten Verbindungen zur Analyse der Aktivität der NLRP3-Aktivierung *in vitro.*

## Revendications

1. Composé utilisé pour la prévention ou le traitement d'une maladie par l'activation de l'inflammasome NLRP3, la maladie étant choisie dans un groupe comprenant les maladies infectieuses ou le cancer, et
(I) dans lequel le composé est un composé de formule (I 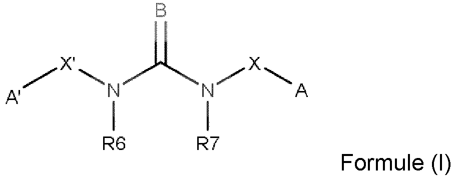 ou un de ses sels pharmaceutiquement acceptables, dans lequel:
B est choisi parmi O ou S, de préférence O;
X et X' sont indépendamment présents ou non, X et X' étant indépendamment un groupe alkyle C₁-C₅ ou -CH(CH₂OH)-; et
R⁶ et R⁷ sont chacun indépendamment un hydrogène ou un alkyle en C₁-C₃, de préférence -H, -CH₃, -C₂H₅, -C₃H₇, ou -C₃H₉, ou éventuellement
R⁶ et R⁷ forment ensemble un cycle hétérocyclique à cinq chaînons choisi parmi , 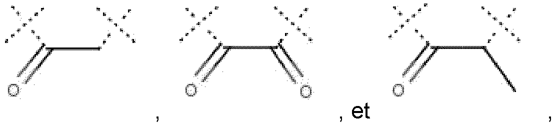 ou éventuellement,
R⁷, N et X forment ensemble un anneau hétérocyclique à six chaînons choisi parmi 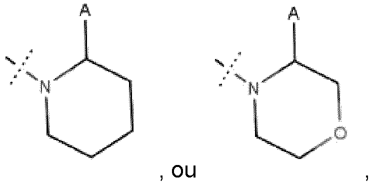
et
A et A' sont différents l'un de l'autre et chacun indépendamment choisi dans l'un des groupes suivants:
un groupe benzène de la structure suivante: 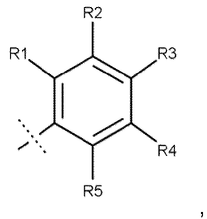
dans lequel les R¹-R⁵ sont chacun indépendamment choisi dans le groupe consistant en:
hydrogène; -OH, -OCH₃, -OCF₃, -OCHF₂, -OCH₂OH; -OCH₂CH₃; -OCH(CH₃)₂; -F; -Cl; -Br; -CN; NO_{2;} NH₂; alkyle en C₁-C₅, alcoxy en C₁-C₅, -CH₃, -CH₂F; -CHF₂; -CF₃; - CH₂Cl; -CHCl₂; -CCl₃; CH(CH₃)₂; -CH₂CH₃; -CH-CH₂-CH(CH₃)₂; -CH₂-N(CH₃)₂; - CH(CH₃)-NHC(O)CH₃; - -NH-CH(CH₃)₂, -NH-cyclopropyl, -CH₃, CH₂CH₃, CH₂CH(CH₃)₂; -C(O)-C₂H₅; -C(O)-CH₃; -C(O)-aziridine;-C(O)-azétidine; -C(O)-pyrrolidine; -C(CH₃)=NOH;
-S(O)₂-cyclopentyle; -S(O)₂-CH₃; -S(O)₂-cyclopentyle; pyrrolidinyle, 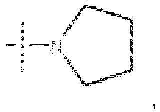 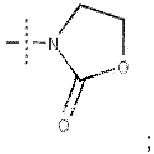 NHC(O)-R⁽⁴¹), dans lequel R⁴¹ est choisi parmi C₁-C₁₀ cycloalkyle, C₁-C₁₀ cyclohétéroalkyle, furane, tétrahydrofurane, cyclopentyle, cyclobutyle, cyclopropyle, -NH-CH(CH₃)₂, -NH-cyclopropyle, C₁-C₅ alkyle, -CH₂F; -CHF₂; -CF₃; - CH₂Cl; -CHCl₂; -CCl₃;-CH₃, CH₂CH₃, CH₂CH(CH₃)₂;
-C(O)OR⁽⁴²), dans lequel R⁴² est choisi parmi les alkyles en C₁-C₅, y compris -CH₃; -CH₂CH₃, -CH₂CH₂CH₃; -CH(CH₃)₂; -CH₂CH₂CH₂CH₃; -CH₂NHS(O)₂CH₃;
-NHR⁴³, dans lequel R⁴³ est choisi parmi , 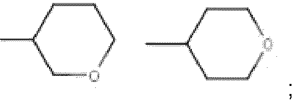 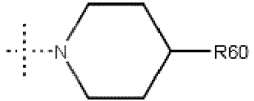 dans lequel R⁶⁰ est choisi parmi l'hydrogène ou -CH₃; 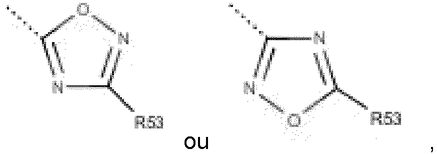 dans lequel
R⁵³ est de préférence choisi dans le groupe constitué par l'hydrogène, OH, -OCH₃, - OCF₃, -OCHF₂, -OCH₂OH, -OCH₂CH₃, -F, -Cl, -Br, -CN, NO_{2, NH2}, alkyle en C₁-C₅, alcoxy en C1-C5, alcényle en C2-C5; NH₂; alkyle en C₁-C₅, alcoxy en C₁-C₅, alcényle en C₂-C₅, de préférence -CH₃, CH(CH₃)₂; -CH₂CH₃; -CH-CH₂-CH(CH₃)₂; -CH₂-N(CH₃)₂; -CH(CH₃)-NHC(O)CH₃; -CH₂F; -CHF₂; -CF₃; -CH₂Cl; -CHCl₂; -CCl₃; -O-N=O, -N=O, -CH=NH, -N₃, -SH, -SO₃H, -SO₂H, -S(O)₂CH₃ -CN, -NC,-OCN, -NCO, - (C=O)OH, -(C=O)NHOH, -NH(C=O)NH₂, -NH(C=S)NH₂, -(C=O)NH₂,-CHO,-CHS, - OCHO, -NCHO, 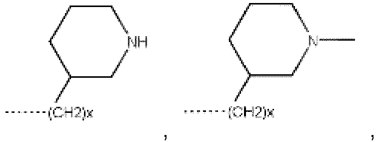 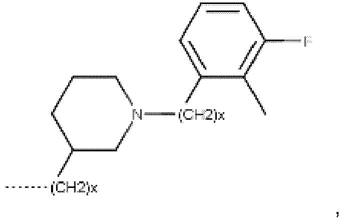 avec X = 0 ou 1, de préférence 1; 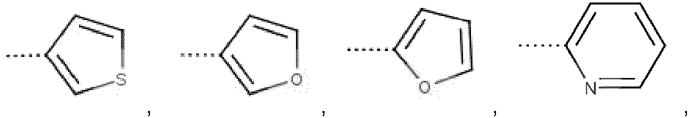 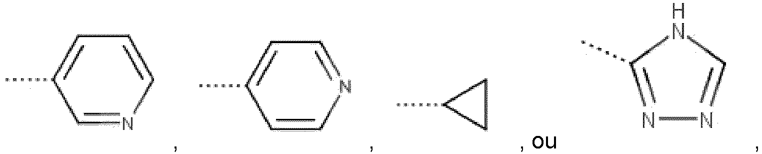 ou
R⁵³ est 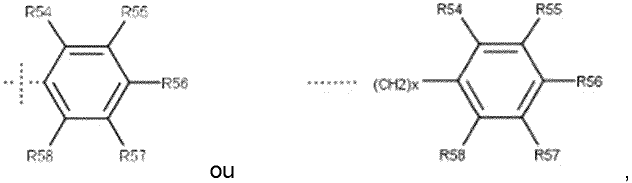 dans lequel R⁵⁴, R⁵⁵, R⁵⁶, R⁵⁷ ou R⁵⁸ sont choisis chacun indépendamment parmi hydrogène, - CH₃, -C₂H₅, -CH₂F; -CHF₂; -CF₃; -CH₂Cl; -CHCl₂; -CCl₃; -OCH₃, -F, -Cl, -Br, NH₂, NO₂, et x est égal à 0, 1 ou 2;
ou R⁵³ est 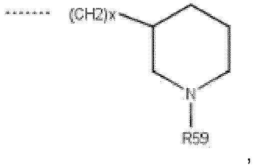 dans lequel R⁵⁹ est 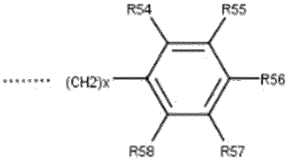 et dans lequel R⁵⁴, R⁵⁵, R⁵⁶, R⁵⁷ ou R⁵⁸ sont choisis séparément parmi l'hydrogène, -CH₃, - C₂H₅, -CH₂F; -CHF₂; -CF₃; -CH₂Cl; -CHCl₂; -CCl₃;-OCH₃, -F, -Cl, -Br, NO₂, NH₂, et x est égal à 0, 1 ou 2; comme défini ci-dessus;
et
éventuellement, dans lequel R¹ et R², R² et R³, R³ et R⁴, ou R⁴ et R⁵ ensemble, de préférence R² et R³ ensemble, forment un anneau cyclique ou hétérocyclique à cinq ou six chaînons
choisi parmi 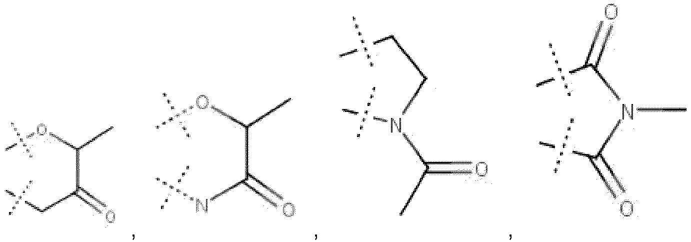 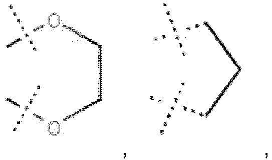
dans lequel, de préférence, tous les R¹ à R⁵ sont de l'hydrogène ou 1, 2 ou 3, de préférence 1 ou 2 des R¹ à R⁵ sont différents de l'hydrogène et choisis parmi les substitutions définies ci-dessus sous a) pour les R¹ à R⁵, et les substituants restants des R¹ à R⁵ sont de l'hydrogène;
b) un groupe pyridine choisi parmi les structures suivantes: 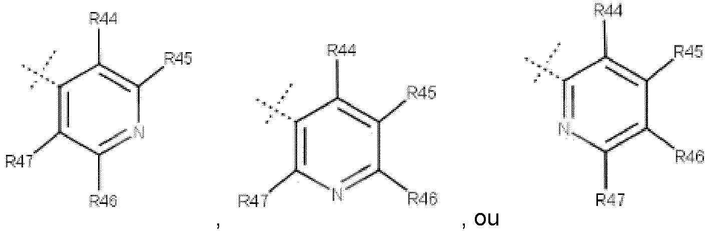 dans lequel les R⁴⁴-R⁴⁷ sont chacun indépendamment choisi dans le groupe consistant en:
hydrogène, -OH, -OCH₃, CH₃, F, Cl, Br, -CH₂F; -CHF₂; -CF₃; -CH₂Cl; -CHCl₂; -CCl₃;-OCF₃, -OCHF₂, -OCH₂OH; -OCH₂CH₃; -CN; NO_{2;} NH₂; -CH₃, CH(CH₃)₂; -CH₂CH₃; - CH₂OH; -CH-CH₂-CH₃₂; -CH₂-N(CH₃)₂; -CH(CH₃)-NHC(O)CH₃; 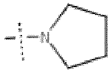
-NHC(O)-R⁽⁴¹), dans lequel R⁴¹ est choisi parmi C₁-C₁₀ cycloalkyle, C₁-C₁₀ cyclohétéroalkyle, furane, tétrahydrofurane, cyclopentyle, cyclobutyle, cyclopropyle, - NH-cyclopropyle, -CH₂F; -CHF₂; -CF₃; -CH₂Cl; -CHCl₂; -CCl₃; -CH₃, CH₂CH₃, CH₂CH(CH₃)₂; -C(O)-aziridine; -C(O)-azétidine; -C(O)-pyrrolidine;
-C(O)OR⁴², dans lequel R⁴² est choisi parmi les alkyles en C₁-C₅, y compris -CH₃; - CH₂CH₃, -CH₂CH₂CH₃; -CH(CH₃)₂; -CH₂CH₂CH₂CH₃; -CH₂NHS(O)₂CH₃;-C(CH₃)=NOH; -S(O)₂-cyclopentyle; -S(O)₂-CH₃; -S(O)₍₂₎-cyclopentyle; pyrrolidinyle, 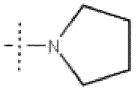 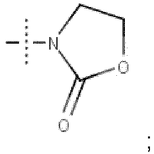 ou
-NHR⁴³, dans lequel R⁴³ est choisi parmi 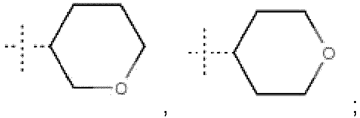 dans lequel, de préférence, tous les R⁴⁴ à R⁴⁷ sont de l'hydrogène ou 1, 2 ou 3, de préférence 1 ou 2 des R⁴⁵ à R⁴⁷ sont différents de l'hydrogène et choisis parmi les substitutions définies ci-dessus au point b) pour les R⁴⁴ à R⁴⁷ et les substituants restants des R⁴⁴ à R⁴⁷ sont de l'hydrogène;
c) un groupe quinoléine ou isoquinoléine choisi parmi les structures suivantes: 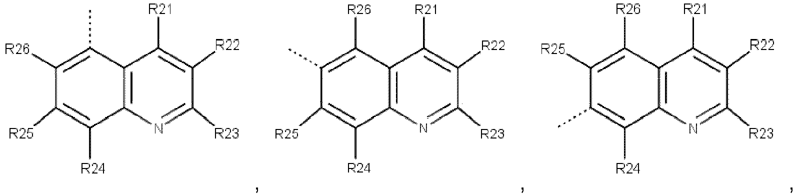 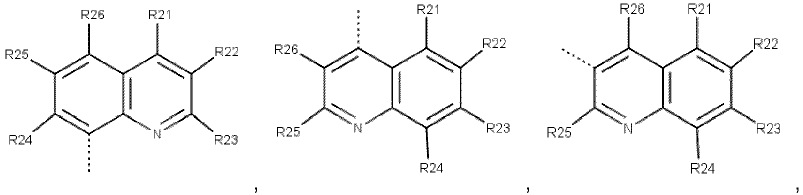 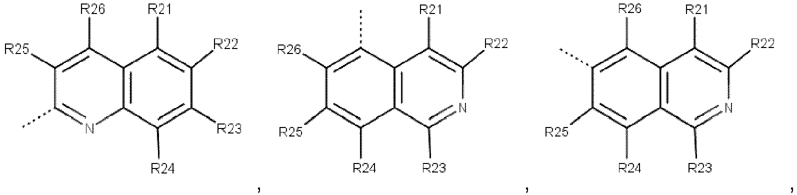 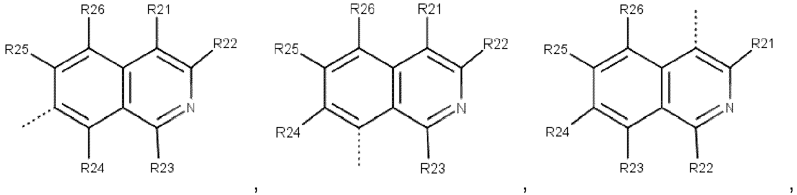 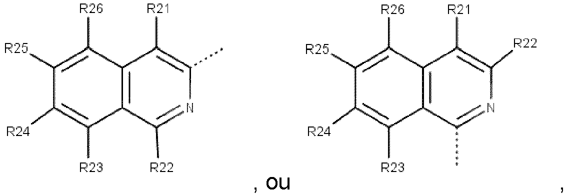
R²¹ à R²⁶ sont chacun indépendamment choisi dans le groupe consistant en hydrogène, OH, -OCH₃, -OCF₃, -OCHF₂, -OCH₂OH; -OCH₂CH₃; -F; -Cl; -Br; -CH₂F; - CHF₂; -CF₃; -CH₂Cl; -CHCl₂; -CCl₃; -CN; NO_{2;} NH₂; alkyle en C1-C5, alcoxy en C1-C5, alcényle en C₂-C₅, de préférence -CH₃, CH(CH₃)₂; -CH₂CH₃; -CH-CH₂-CH(CH₃)₂; - CH₂-N(CH₃)₂; -CH(CH(₃))-NHC(O)CH_{3;} -O-N=O, -N=O, -CH=NH, -N₃, -SH, -SO₃H, - SO₂H, -S(O)₂CH₃-CN, -NC,-OCN, -NCO, -SCN, -NCS, -(P=O)(OH)₂, -O(P=O)(OH)₂, - (C=O)OH, -(C=O)NHOH, -NH(C=O)NH₂, -NH(C=S)NH₂, -(C=O)NH₂,-CHO,-CHS, - OCHO, -NCHO, -C(O)-aziridine; -C(O)-aziridine; -C(O)-azétidine; -C(O)-pyrrolidine; - C(O)OR₄₂, dans lequel R⁴² est choisi parmi les alkyles en C1-C5, y compris -CH₃; - CH₂CH₃, -CH₂CH₂CH₃; -CH(CH₃)₂; -CH₂CH₂CH₂CH₃; -CH₂NHS(O)₂CH₃; - C(CH₃)=NOH; -S(O)₂-cyclopentyle; -S(O)₂-CH_{3;}
-S(O)(₂)-cyclopentyle; pyrrolidinyle, 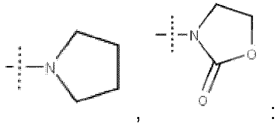
-NHC(O)-R⁽⁴¹), dans lequel R⁴¹ est choisi parmi C₁-C₁₀ cycloalkyle, C₁-C₁₀ cyclohétéroalkyle, furane, tétrahydrofurane, cyclopentyle, cyclobutyle, cyclopropyle, - NH-cyclopropyle, -CH₂F; - CHF₂; -CF₃; -CH₂Cl; -CHCl₂; -CCl₃; -CH₃, CH₂CH₃, CH₂CH(CH₃)₂;
-NHR⁴³, dans lequel R⁴³ est choisi parmi 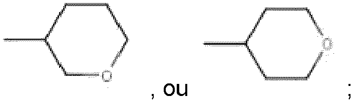
ou plus préférentiellement dans lequel R²¹ à R²⁶ sont chacun un hydrogène, OH, - OCH₃, -OCF₃, -OCHF₂, -OCH₂OH; -OCH₂CH₃; -F; -Cl; -Br; -CH₂F; -CHF₂; -CF₃; - CH₂Cl; -CHCl₂; -CCl_{3;}-CN; NO_{2;} NH₂; alkyle en C1-C5, alcoxy en C1-C5, alcényle en C₂-C₅, de préférence -CH₃, CH(CH₃)₂; -CH₂CH₃; -CH-CH₂-CH(CH₃)₂; -CH₂-N(CH₃)₂; - CH(CH₃)-NHC(O)CH₃;
ou, de préférence encore, dans lequel les R²¹ à R²⁶ sont chacun un hydrogène, ou -F; Cl, Br,
dans lequel, de préférence, tous les R²¹ à R²⁶ sont de l'hydrogène ou 1, 2 ou 3, de préférence 1 ou 2 des R²¹ à R²⁶ sont différents de l'hydrogène et choisis parmi les substitutions définies ci-dessus au point c) pour les R²¹ à R²⁶ et les substituants restants des R²¹ à R²⁶ sont de l'hydrogène;
d) un groupe 1,5 naphthyridine, un groupe 1,6 naphthyridine ou un groupe 1,8 naphthyridine présentant l'une des structures suivantes 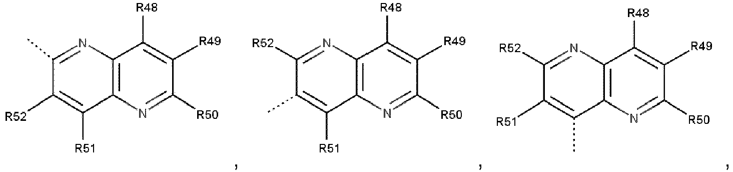 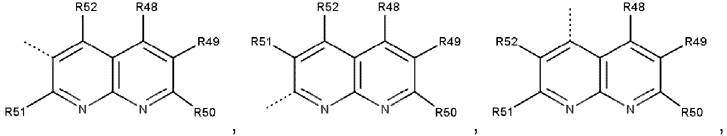 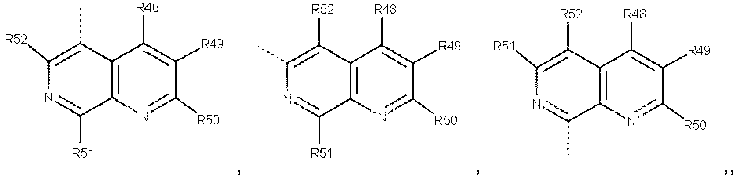 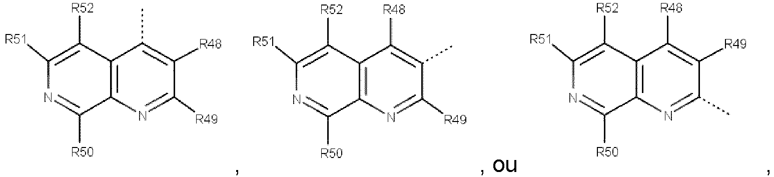 dans lequel R⁴⁸ à R⁵² sont chacun indépendamment choisi dans le groupe consistant en:
hydrogène, OH, -OCH₃, -OCF₃, -OCHF₂, -OCH₂OH; -OCH₂CH₃; -F; -Cl; -Br; -CH₂F; - CHF₂; -CF₃; -CH₂Cl; -CHCl₂; -CCl₃;-CN; NO₂; NH₂; alkyle en C1-C5, alcoxy en C1-C5, alcényle en C₂-C₅, de préférence -CH₃, CH(CH₃)₂; -CH₂CH₃; -CH-CH₂-CH(CH₃)₂; - CH₂-N(CH₃)₂; -CH(CH₃)-NHC(O)CH₃; -O-N=O, -N=O, -CH=NH, -N₃, -SH, -SO₃H, - SO₂H, -S(O)₂CH₃ -CN, -NC,-OCN, -NCO, -SCN, -NCS, -(P=O)(OH)₂, -O(P=O)(OH)₂, - (C=O)OH, -(C=O)NHOH, -NH(C=O)NH₂, -NH(C=S)NH₂, -(C=O)NH₂,-CHO,-CHS, - OCHO, -NCHO,
-NHC(O)-R⁴¹, dans lequel R⁴¹ est choisi parmi C₁-C₁₀ cycloalkyle, C₁-C₁₀ cyclohétéroalkyle, furane, tétrahydrofurane, cyclopentyle, cyclobutyle, cyclopropyle, - NH-cyclopropyle, -CH₂F; -CHF₂; -CF₃; -CH₂Cl; -CHCl₂; -CCl₃; -CH₃, CH₂CH₃, CH₂CH(CH₃)₂; -C(O)-aziridine; -C(O)-azétidine; -C(O)-pyrrolidine;
-C(O)OR⁽⁴²), dans lequel R⁴² est choisi parmi les alkyles en C₁-C₅, y compris -CH₃; - CH₂CH₃, -CH₂CH₂CH₃; -CH(CH₃)₂; -CH₂CH₂CH₂CH₃; -CH₂NHS(O)₂CH₃;-C(CH₃)=NOH; -S(O)₂-cyclopentyle; -S(O)₂-CH₃; -S(O)₂-cyclopentyle; pyrrolidinyle, 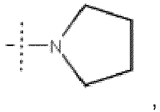 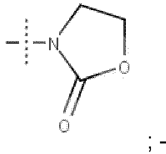 NHR₄₃, dans lequel R₄₃ est choisi parmi 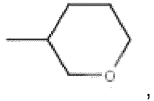 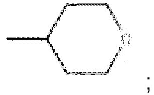
ou plus préférentiellement dans lequel R⁴⁸ à R⁵² sont chacun un hydrogène, OH, - OCH₃, -OCF₃, -OCHF₂, -OCH₂OH; -OCH₂CH₃; -F; -Cl; -Br; -CH₂F; -CHF₂; -CF₃; - CH₂Cl; -CHCl₂; -CCl₃;-CN; NO_{2;} NH₂; alkyle en C1-C5, alcoxy en C1-C5, alcényle en C₂-C₅, de préférence -CH₃, CH(CH₃)₂; -CH₂CH₃; -CH-CH₂-CH(CH₃)₂; -CH₂-N(CH₃)₂; - CH(CH₃)-NHC(O)CH₃; ou, de préférence encore, dans lequel les R⁴⁸ à R⁵² sont chacun un hydrogène, ou -F; Cl, Br,
dans lequel, de préférence, tous les R⁴⁸ à R⁵² sont de l'hydrogène ou 1, 2 ou 3, de préférence 1 ou 2 des R⁴⁸ à R⁵² sont différents de l'hydrogène et choisis parmi les substitutions définies ou présentées ci-dessus sous d) pour les R⁴⁸ à R⁵² et les substituants restants des R⁴⁸ à R⁵² sont de l'hydrogène;
e) un groupe oxadiazole, choisi parmi les structures suivantes: 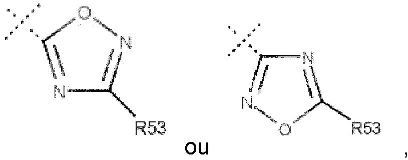 dans lequel R⁵³ est de préférence choisi dans le groupe constitué par:
hydrogène, OH, -OCH₃, -OCF₃, -OCHF₂, -OCH₂OH; -OCH₂CH₃; -F; -Cl; -Br; -CN; NO_{2;} NH₂; alkyle en C1-C5, alcoxy en C1-C5, alcényle en C₂-C₅, de préférence -CH₃, CH(CH₃)₂; -CH₂CH₃; -CH-CH₂-CH(CH₃)₂; -CH₂-N(CH₃)₂; -CH(CH₃)-NHC(O)CH₃; - CH₂F; -CHF₂; -CF₃; -CH₂Cl; -CHCl₂; -CCl₃;-O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, -NH(C=O)NH₂, - NH(C=S)NH₂, -(C=O)NH₂,
-CHO,-CHS, -OCHO, -NCHO, 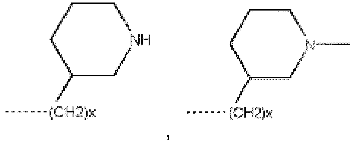 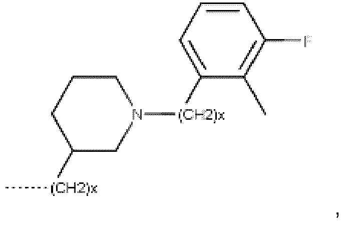 avec X = 0 ou 1, de préférence 1; 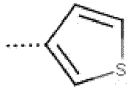 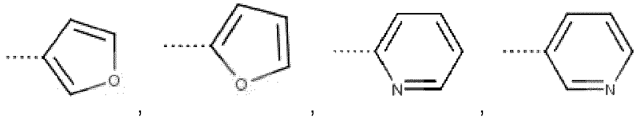 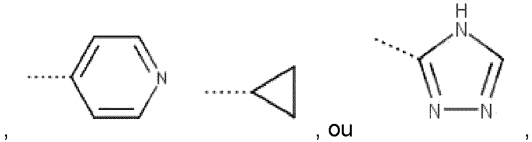 ou
R⁵³ est 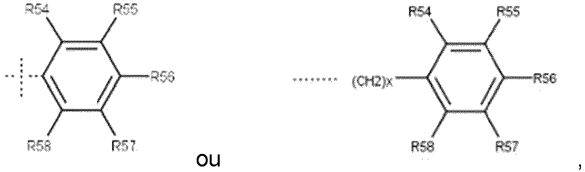 dans lequel R⁵⁴, R⁵⁵, R⁵⁶, R⁵⁷ ou R⁵⁸ sont choisis chacun indépendamment parmi hydrogène, -CH₃, - C₂H₅, -CH₂F; -CHF₂; -CF₃; -CH₂Cl; -CHCl₂; -CCl₃; -OCH₃, -F, -Cl, -Br, NH₂, NO₂, et x est égal à 0, 1 ou 2;
ou R⁵³ est 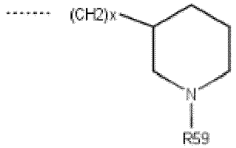 dans lequel R⁵⁹ est 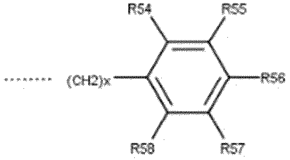 et dans lequel R⁵⁴, R⁵⁵, R⁵⁶, R⁵⁷ ou R⁵⁸ sont choisis séparément parmi l'hydrogène, -CH₃, - C₂H₅, -CH₂F; -CHF₂; -CF₃; -CH₂Cl; -CHCl₂; -CCl₃; -OCH₃, -F, -Cl, -Br, NO₂, NH₂, et x est égal à 0, 1 ou 2; comme défini ci-dessus, et x est égal à 0, 1 ou 2
et dans lequel, de préférence, tous les R⁵⁴ à R⁵⁸ sont de l'hydrogène ou 1, 2 ou 3, de préférence 1 ou 2 des R⁵⁴ à R⁵⁸ sont différents de l'hydrogène et choisis parmi les substitutions définies ci-dessus au point e) pour les R⁵⁴ à R⁵⁸ et les positions restantes des R⁵⁴ à R⁵⁸ sont de l'hydrogène;
f) un groupe thiadiazole, choisi parmi les structures suivantes: 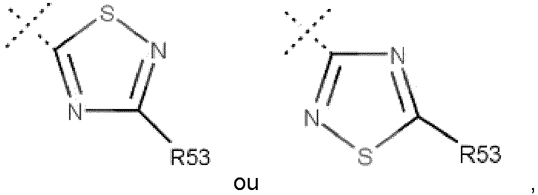 dans lequel R⁵³ est de préférence tel que défini ci-dessus
g) un groupe isothiazole, choisi parmi les structures suivantes: 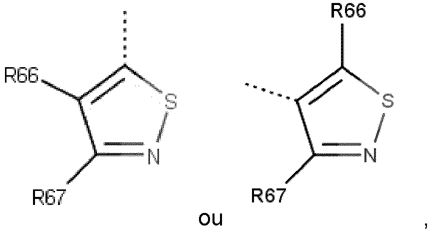 dans lequel R⁶⁶ et R⁶⁷ sont chacun choisis indépendamment parmi l'hydrogène, -CH₃, -C₂H₅, -CH₂F; -CHF₂; -CF₃; -CH₂Cl; -CHCl₂; -CCl₃;-F, -Cl, -Br, de préférence dans lequel R⁶⁶ ou R⁶⁷ est un hydrogène et l'autre résidu est choisi parmi -CH₃, -C₂H₅, -CF₃, -CHF₂, -F, -Cl, -Br; ou
h) un groupe thiophène, choisi structures suivantes: 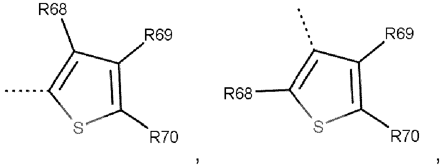 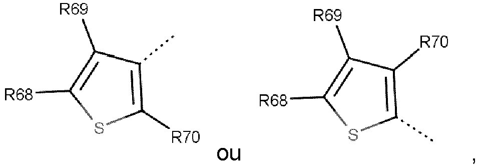 dans lequel R⁶⁸, R⁶⁹ et R⁷⁰ sont chacun choisi indépendamment parmi l'hydrogène, - CH₃, -C₂H₅, -CH₂F; -CHF₂; -CF₃; -CH₂Cl; -CHCl₂; -CCl₃;-F, -Cl, -Br, de préférence dans lequel de R⁶⁸, R⁶⁹ ou R⁷⁰ est un hydrogène et l'autre résidu est choisi parmi -CH₃, - C₂H₅, -CF₃, -CHF₂, -F, -Cl, -Br,
ou
(II) dans lequel le composé est un composé de formule (Ic), un composé de formule (lc1), un composé de formule (lc2), un composé de formule (lc3), ou un composé de formule (Ic4), ou un sel pharmaceutiquement acceptable de celui-ci: 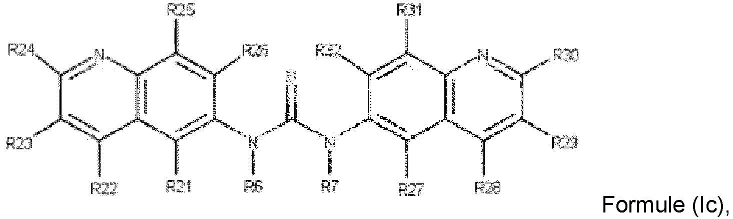 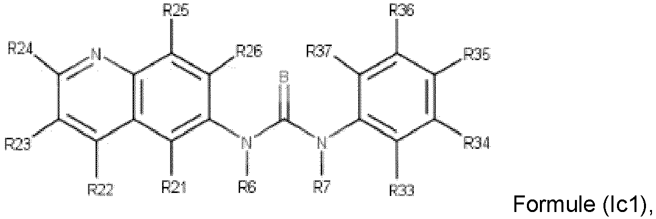 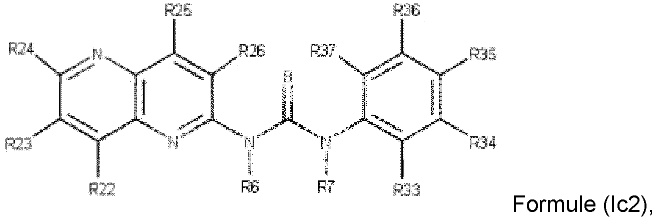 ou 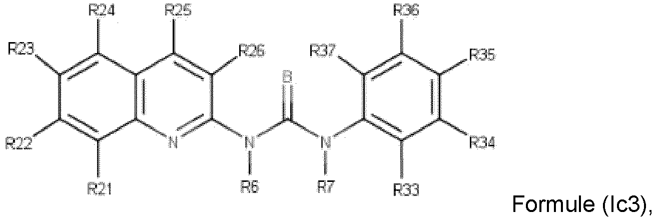 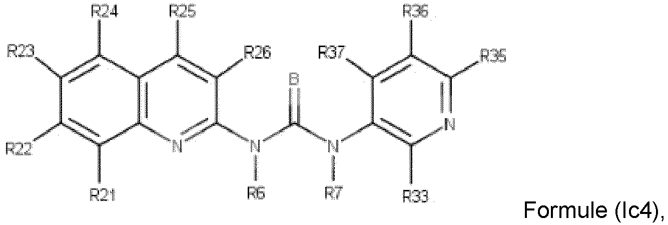 dans lequel
B = O ou S, de préférence O;
R⁶ et R⁷ sont indépendamment chacun un hydrogène, un alkyle en C₁₋₃, de préférence un hydrogène, CH₃, plus préférentiellement, soit R⁶ est un hydrogène et R⁷ est CH₃, soit R6 et R7 sont tous deux un hydrogène;
R²¹ à R³⁷ sont chacun indépendamment choisi dans le groupe constitué par l'hydrogène, OH, -OCH₃, -OCF₃, -OCHF₂, -OCH₂OH; -OCH₂CH₃; -CH₂F; -CHF₂; -CF₃; -CH₂Cl; -CHCl₂; -CCl₃;-F; -Cl; -Br; -CN; NO₂; NH₂; -CH₃, CH(CH₃)₂; -CH₂CH₃; -CH-CH₂-CH(CH₃)₂; -CH₂-N(CH₃)₂; -CH(CH₃)-NHC(O)CH₃; -O-N=O, -N=O, -OH, -NH₂, - CH=NH, -N₃, -SH, -SO₃H, -SO₂H, -S(O)₂CH₃ -CN, -NC,-OCN, -NCO, -SCN, -NCS, - (P=O)(OH)₂, -O(P=O)(OH)₂, -(C=O)OH, -(C=O)NHOH, -NH(C=O)NH₂, -NH(C=S)NH₂, -(C=O)NH₂,-CHO,-CHS, -OCHO, -NCHO, -B(OH)₂ (C=O)R¹¹, (C=O)OR¹¹, (C=O)SR¹¹, (C=O)NR¹¹R¹², (SO₂)R¹¹, or C(NR¹¹)R¹², -NHC(O)R¹¹, et -NHC(O)NHR¹¹,
dans lequel R¹¹ et R¹² sont indépendamment choisis dans le groupe constitué de C₁-C₅ alkyl, de préférence -CH₃, CH(CH₃)₂; -CH₂CH₃; -CH-CH₂-CH(CH₃)₂; -CH₂-N(CH₃)₂; -CH(CH₃)-NHC(O)CH₃; I, -CH₂F; -CHF₂; -CF₃; -CH₂Cl; -CHCl₂; -CCl₃; -CHCl₂, and - CCl₃; ou
de préférence dans lequel R²¹ à R³⁷ sont chacun indépendamment choisi dans le groupe constitué par l'hydrogène, CH₂(SO₂)CH₃, -CH₂(SO₂)CH₂CH₃, - CH₂CH₂(SO₂)CH₂CH₃; -(SO₂)C₁-C₅ alkyl, de préférence -(SO₂)CH₃, -(SO₂)CH₂CH₃, - CH₂F; -CHF₂; -CF₃; -CH₂Cl; -CHCl₂; -CCl₃;-F, -Cl, et -Br; ou
dans lequel, de préférence, tous les R²¹ à R³⁷ sont de l'hydrogène ou 1, 2 ou 3, de préférence 1 ou 2 des R²¹ à R³⁷ sont différents de l'hydrogène et choisis parmi les substitutions présentées ci-dessus, et les positions restantes des R²¹ à R³⁷ sont de l'hydrogène; ou
de préférence, dans lequel R²¹ à R³⁷ sont chacun de l'hydrogène.

2. Composé à utiliser selon la revendication 1, dans lequel le composé est un composé de formule (Ic5), un composé de formule (lc6), un composé de formule (lc7), un composé de formule (lc8), un composé de formule (lc9) ou un composé de formule (lc10), un composé de formule (lc11), un composé de formule (lc12), ou un composé de formule (lc13), ou un sel pharmaceutiquement acceptable de ceux-ci: 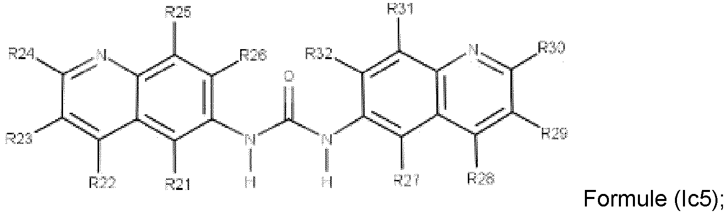 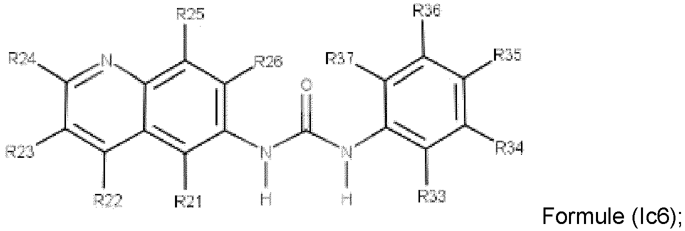 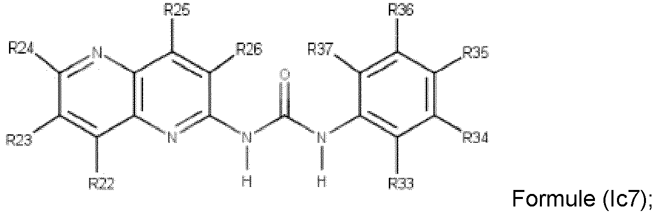 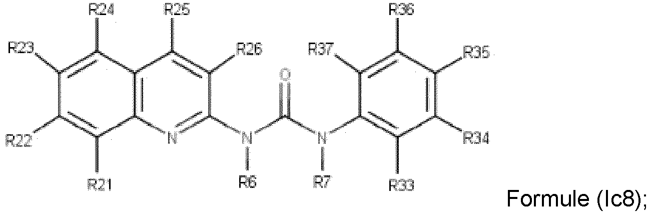 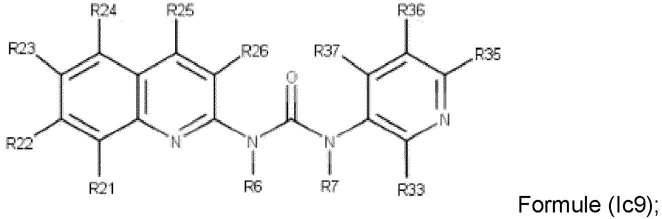 ou 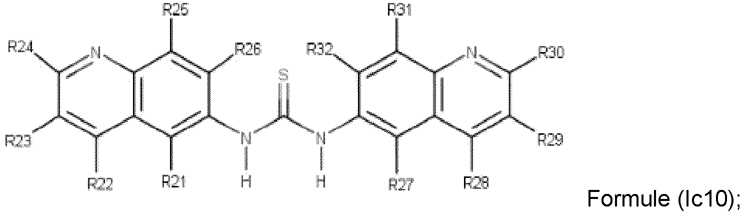 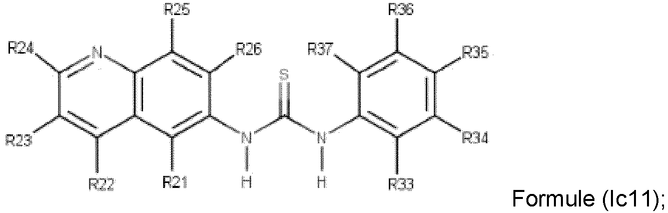 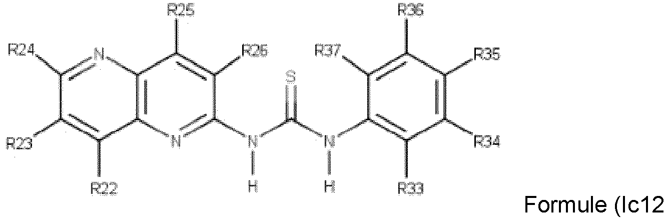 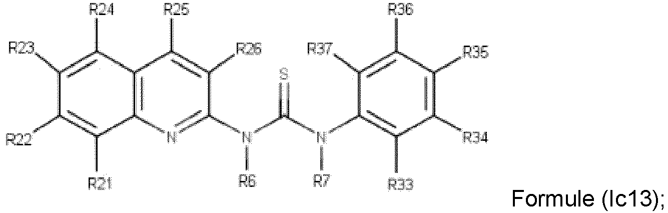 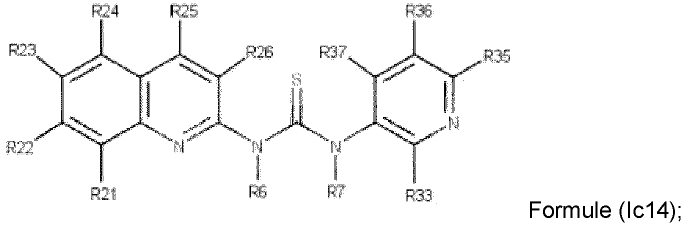
dans lequel R²¹ à R³⁷ sont chacun indépendamment choisi dans le groupe consistant en hydrogène, OH, -OCH₃, -OCF₃, -OCHF₂, -OCH₂OH; -OCH₂CH₃; -CH₂F; -CHF₂; - CF₃; -CH₂Cl; -CHCl₂; -CCl₃; -F; -CI; -Br; -CN; NO₂; NH₂; -CH₃, CH(CH₃)₂; -CH₂CH₃; ou -CH₂(SO₂)CH₃, -CH₂(SO₂)CH₂CH₃, -CH₂CH₂(SO₂)CH₂CH₃; -(SO₂)alkyle en C₁-C₅, de préférence -(SO₂)CH₃, -(SO₂)CH₂CH₃, ou
dans lequel, de préférence, tous les R²¹ à R⁽³⁷) sont de l'hydrogène ou 1, 2 ou 3, de préférence 1 ou 2 des R²¹ à R³⁷ sont différents de l'hydrogène et choisis parmi les substitutions présentées ci-dessus et les positions restantes des R²¹ à R³⁷ sont choisies parmi l'hydrogène; ou
de préférence, dans lequel les R²¹ à R³⁷ sont chacun de l'hydrogène.

3. Composé à utiliser selon l'une des revendications 1 ou 2, dans lequel le composé est un composé de l'une des formules (Ia) ou (Ia1). 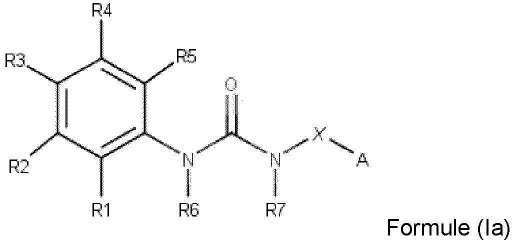 ou 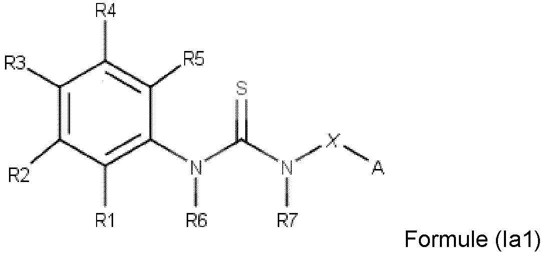 ou un de ses sels pharmaceutiquement acceptables, dans lequel:
A et A' sont différents l'un de l'autre,
X est présent ou non, et dans lequel X, s'il est présent, est choisi parmi -CH₂-, - C₂H₄-, -C₃H₆-, -C(CH₃)₂-, -CHCH(CH₃)₂-, -CHC(CH₃)₃-, -CH(CH(CH₃)₂)-, - CH(CH₂CH(CH₃)₂)-, ou -CH(CH₂OH)-; et
R⁶ et R⁷ sont chacun indépendamment hydrogène, -CH₃, -C₂H₅, -C₃H₇, ou -C₃H₉, ou facultativement,
R⁶ et R⁷ forment ensemble un anneau hétérocyclique à cinq chaînons, choisi parmi 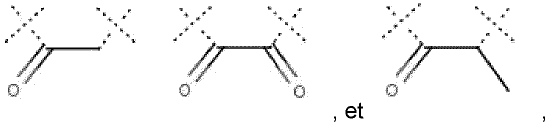 ou facultativement,
R⁷, N et X forment ensemble un anneau hétérocyclique à six chaînons, choisi parmi 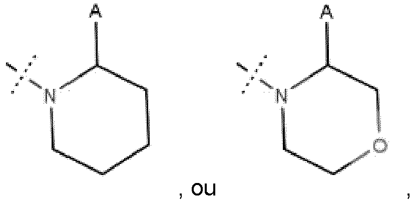
dans lequel R¹-R⁵ sont chacun indépendamment choisis dans le groupe constitué par l'hydrogène; -OH, -OCH₃, -OCF₃, -OCHF₂, -OCH₂OH; -OCH₂CH₃; -OCH(CH₃)_{2;} - F; -Cl; -Br; -CN; NO₂; NH₂; -CH₃, -CH₂F; -CHF₂; -CF₃; -CH₂Cl; -CHCl₂; -CCl₃; CH(CH₃)₂; -CH₂CH₃;
-C(O)OR₄₂, dans lequel R⁴² est choisi parmi -CH₃; -CH₂CH₃, -CH₂CH₂CH₃; - CH(CH₃)₂; -CH₂CH₂CH₂CH₃; 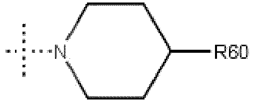 dans lequel R⁶⁰ est choisi parmi l'hydrogène ou -CH₃;
éventuellement, dans lequel R¹ et R², R⁽²) et R³, R³ et R⁴, ou R⁴ et R⁵ ensemble, de préférence R¹ et R² ou R² et R³ ensemble, forment un anneau cyclique ou hétérocyclique à cinq ou six chaînons,
choisi parmi 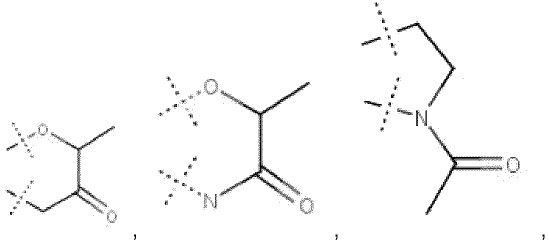 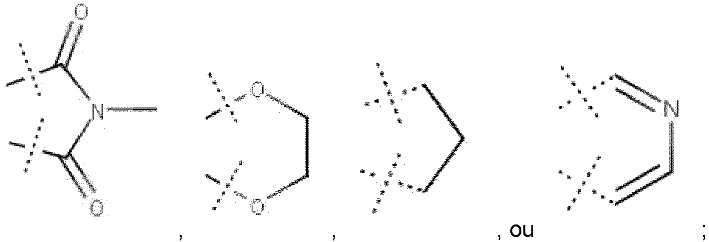
et dans lequel A est choisi parmi les substituants suivants a) à e):
a) un groupe benzène de la structure suivante: 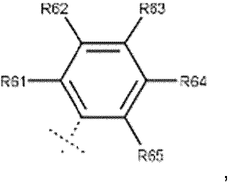 dans lequel les R⁶¹-R⁶⁶ sont chacun indépendamment choisi dans le groupe consistant en:
hydrogène; -OH, -OCH₃, -OCF₃, -OCHF₂, -OCH₂OH; -OCH₂CH₃; -OCH(CH₃)₂; -F; -Cl; -Br; -CN; NO₂; NH₂; -CH₃, -CH₂F; -CHF₂; -CF₃; -CH₂Cl; -CHCl₂; -CCl₃;CH(CH₃)₂ - CH₂CH₃; -CH-CH₂-CH(CH₃)₂; -CH₂-N(CH₃)₂; -CH(CH₃)-NHC(O)CH₃; -C(O)-C₂H₅; - C(O)-CH₃; -C(O)-aziridine; -C(O)-azétidine; -C(O)-pyrrolidine; -CH₂NHS(O)₂CH₃; - C(CH₃)=NOH; -S(O)₂-cyclopentyle; -S(O)₂-CH₃; -S(O)(₂)-cyclopentyle; pyrrolidinyle, 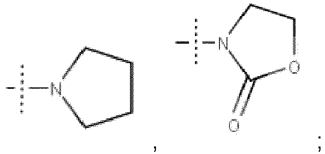
-NHC(O)-R₄₁, dans lequel R₄₁ est choisi parmi le furane, le tétrahydrofurane, le cyclopentyle,
cyclobutyle, le cyclopropyle, -NH-CH(CH₃)₂, -NH-cyclopropyle, -CH₂F; -CHF₂; -CF₃; - CH₂Cl; -CHCl₂; -CCl₃; -CH₃, CH₂CH₃, CH₂CH(CH₃)₂;
-C(O)OR₄₂, dans lequel R₄₂ est choisi parmi les alkyles en C₁-C₅, y compris -CH₃; - CH₂CH₃, -CH₂CH₂CH₃; -CH(CH₃)₂; -CH₂CH₂CH₂CH₃;
-NHR₄₃, dans lequel R₄₃ est choisi parmi 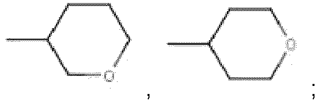 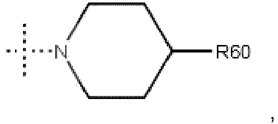 dans lequel R⁶⁰ est choisi parmi l'hydrogène ou -CH₃; 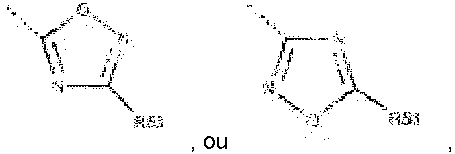 dans lequel R⁵³ est de préférence choisi dans le groupe constitué par l'hydrogène, OH, -OCH₃, -OCF₃, -OCHF₂, - OCH₂OH; -OCH₂CH₃; -F; -Cl; -Br; -CN; NO₂; NH₂; alkyle en C1-C5, alcoxy en C₁-C₅, alcényle en C₂-C₅, de préférence -CH₃, CH(CH₃)₂; -CH₂CH₃; -CH-CH₂-CH(CH₃)₂; - CH₂-N(CH₃)₂; -CH(CH₃)-NHC(O)CH₃; -CH₂F; -CHF₂; -CF₃; -CH₂Cl; -CHCl₂; -CCl₃; -O-N=O, -N=O, -CH=NH, -N₃, -SH, -SO₃H, -SO₂H, -S(O)₂CH₃ -CN, -NC,-OCN, -NCO, - (C=O)OH, -(C=O)NHOH, -NH(C=O)NH₂ 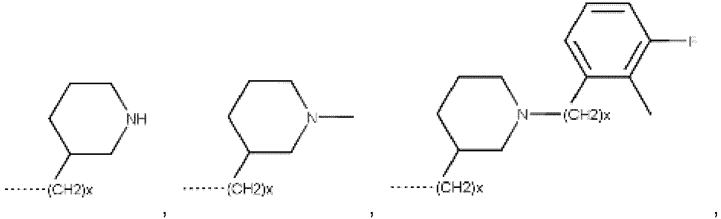 avec X = 0 ou 1, de préférence 1; 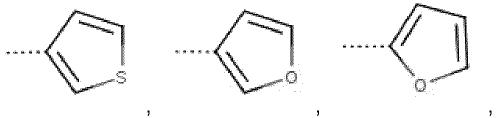 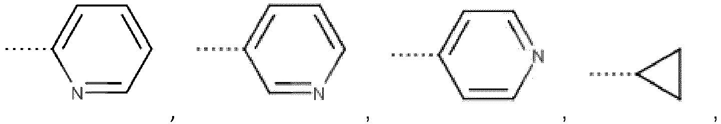 ou 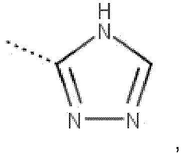
ou R⁵³ est 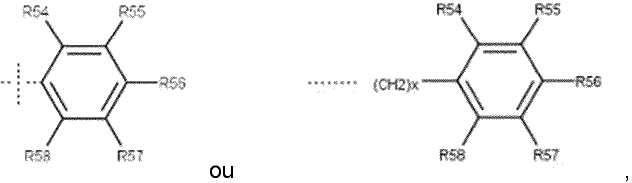 dans lequel R⁵⁴, R⁵⁵, R⁵⁶, R ⁵⁷ou R⁵⁸ sont choisis chacun indépendamment parmi hydrogène, - CH₃, -C₂H₅, -CH₂F; -CHF₂; -CF₃; -CH₂Cl; -CHCl₂; -CCl₃; -OCH₃, -F, -Cl, -Br, NO₂, NH₂, et x est égal à 0, 1 ou 2;
ou R⁵³ est 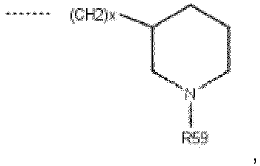 dans lequel R⁵⁹ est 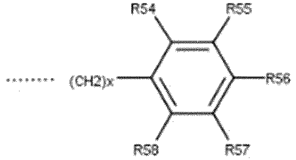 et dans lequel R⁵⁴, R⁵⁵, R⁵⁶, R⁵⁷ ou R⁵⁸ sont choisis séparément parmi les éléments ci-dessus parmi l'hydrogène, -CH₃, -C₂H₅, -CH₂F; - CHF₂; -CF₃; -CH₂Cl; -CHCl₂; -CCl₃;-OCH₃, -F, -Cl, -Br, NO₂, NH₂, et x est égal à 0, 1 ou 2; tel que défini ci-dessus, et x est égal à 0, 1 ou 2;
et
éventuellement, dans lequel R⁶¹ et R⁽⁶⁾⁽²⁾, R⁶⁽²⁾ et R⁽⁶⁾⁽³⁾, R⁶³ et R⁽⁶⁾⁽⁴⁾, ou R⁶⁴ et R⁽⁶⁾⁽⁵⁾ ensemble, de préférence R⁶² et R⁶³ ensemble, forment un anneau hétérocyclique à cinq ou six chaînons
choisi parmi 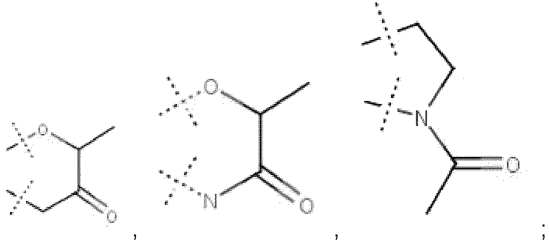 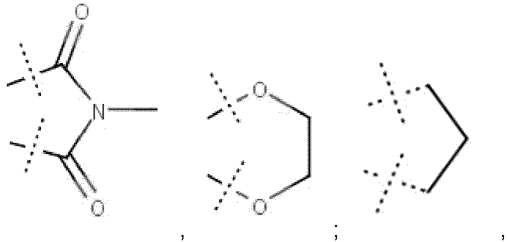 et
dans lequel, de préférence, X n'est pas présent, et
dans lequel, de préférence, tous les R¹ à R⁵ sont de l'hydrogène ou 1, 2 ou 3, de préférence 1 ou 2 des R¹ à R⁵ sont différents de l'hydrogène et choisis parmi les substitutions définies ci-dessus pour les R¹ à R⁵ et les substituants restants des R¹ à R⁵ sont de l'hydrogène;
b) un groupe oxadiazole de l'une des structures suivantes: 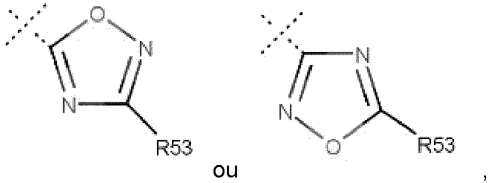 dans lequel
R⁵³ est de préférence choisi dans le groupe constitué par l'hydrogène, OH, -OCH₃, - OCF₃, -OCHF₂, -OCH₂OH; -OCH₂CH₃; -F; -Cl; -Br; -CN; NO_{2;} NH₂; alkyle en C1-C5, alcoxy en C1-C5, alcényle en C₂-C₅, de préférence -CH₃, CH(CH₃)₂; -CH₂CH₃; -CH-CH₂-CH(CH₃)₂; -CH₂-N(CH₃)₂; -CH(CH₃)-NHC(O)CH₃; -CH₂F; -CHF₂; -CF₃; -CH₂Cl; - CHCl₂; -CCl₃;-O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, -NH(C=O)NH₂, -NH(C=S)NH₂, -(C=O)NH₂,-CHO,-CHS,
- OCHO, -NCHO, - 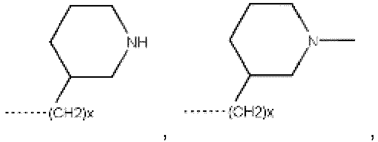 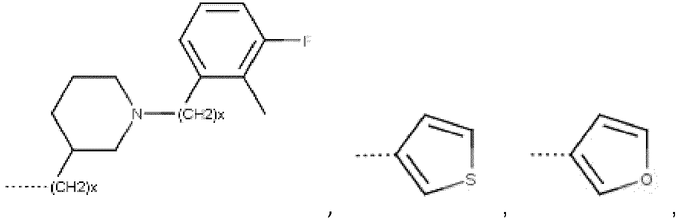 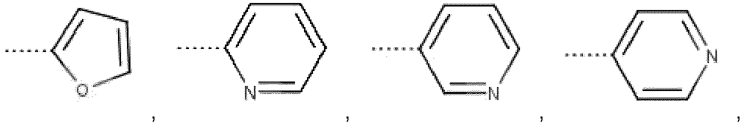 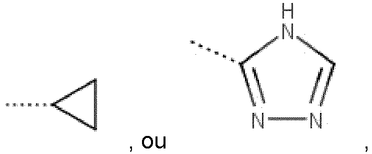 ou
R⁵³ est 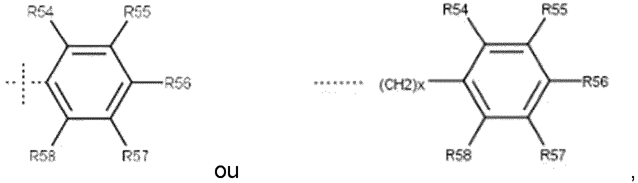 dans lequel R⁵⁴, R⁵⁵, R⁵⁶, R ⁵⁷ou R⁵⁸ sont choisis chacun indépendamment parmi hydrogène, - CH₃, -C₂H₅, -CH₂F; -CHF₂; -CF₃; -CH₂Cl; -CHCl₂; -CCl₃; -OCH₃, -F, -Cl, -Br, NO₂, NH₂, et x est égal à 0, 1 ou 2;
ou R⁵³ est 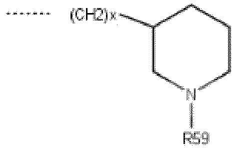 dans lequel R⁵⁹ est 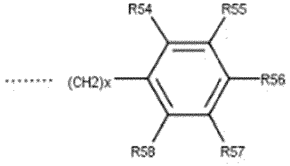 et dans lequel R⁵⁴, R⁵⁵, R⁵⁶, R⁵⁷ ou R⁵⁸ sont choisis séparément parmi les éléments ci-dessus parmi l'hydrogène, -CH₃, -C₂H₅, -CH₂F; -CHF₂; -CF₃; -CH₂Cl; -CHCl₂; -CCl₃;-OCH₃, -F, -Cl, -Br, NO₂, NH₂, et x est 0, 1 ou 2; tel que défini ci-dessus, et x est 0, 1 ou 2; et
dans lequel, de préférence, X est choisi parmi -CH₂-, -C₂H₄-, -C₃H₆-, -C(CH₃)₂-, - CHCH(CH₃)₂-, -CHC(CH₃)₃-, -CH(CH(CH₃)₂)-, -CH(CH₂CH(CH₃)₂)-, ou -CH(CH₂OH)-;
et dans lequel, de préférence, tous les R⁵⁴ à R⁵⁸ sont de l'hydrogène ou 1, 2 ou 3, de préférence 1 ou 2 des R⁵⁴ à R⁵⁸ sont différents de l'hydrogène et choisis parmi les substitutions présentées ci-dessus et les positions restantes des R⁵⁴ à R⁵⁸ sont choisies parmi l'hydrogène;
c) un groupe thiadiazole de l'une des structures suivantes: 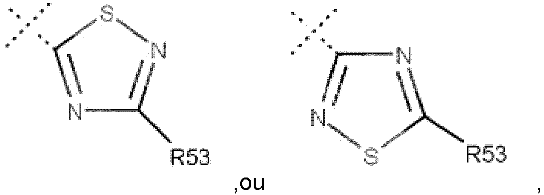
dans lequel R⁵³ est de préférence tel que défini ci-dessus, et
de préférence, X est choisi parmi -CH₂-, -C₂H₄-, -C₃H₆-, -C(CH₃)₂-, -CHCH(CH₃)₂-, - CHC(CH₃)₃-, -CH(CH(CH₃)₂)-, -CH(CH₂CH(CH₃)₂)-, ou -CH(CH₂OH)-;
d) un groupe isothiazole de la structure suivante: 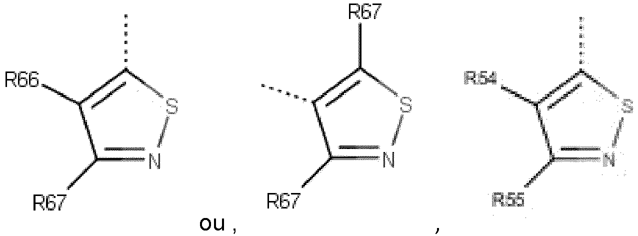 das lequel R⁶⁶ et R⁶⁷ sont chacun choisi indépendamment parmi l'hydrogène, -CH₃, -C₂H₅, -CH₂F; - CHF₂; -CF₃; -CH₂Cl; -CHCl₂; -CCl₃;-F, -Cl, -Br
et X est de préférence choisi parmi -CH₂-, -C₂H₄-, -C₃H₆-, -C(CH₃)₂-,-CHCH(CH₃)₂-, - CHC(CH₃)₃-, -CH(CH(CH₃)₂)-, -CH(CH₂CH(CH₃)₂)-, ou -CH(CH₂OH)-; ou
e) un groupe thiophène, de préférence 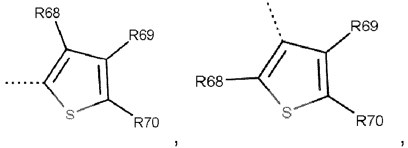 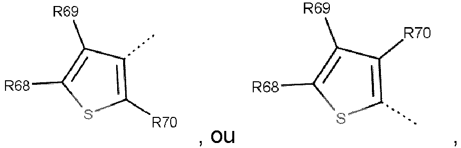 dans lequel R⁶⁸, R⁶⁹ et R⁷⁰ sont chacun choisi indépendamment parmi l'hydrogène, -CH₃, -C₂H₅, -CH₂F; -CHF₂; -CF₃; -CH₂Cl;-CHCl₂; -CCl₃;-F, -Cl, -Br, de préférence dans lequel l'un des R⁶⁸, R⁶⁹ ou R⁷⁰ est un hydrogène et l'autre résidu est choisi parmi -CH₃, -C₂H₅, -CF₃, -CHF₂, -F, -Cl, -Br, et de préférence
X est -CH₂-, -C₂H₄-, -C₃H₆-, -C(CH₃)₂-, -CHCH(CH₃)₂-, -CHC(CH₃)₃-, -CH(CH(CH₃)₂)-, -CH(CH₂CH(CH₃)₂)-, ou -CH(CH₂OH)-.

4. Composé utilisé selon la revendication 3, dans lequel R¹, R², R³, R⁴ et R⁵ sont chacun indépendamment hydrogène, -OCH₃, -OCF₃, -OCH₂CH₃, -OCH(CH₃)₂, - -CH₃, -C₂H₅, - CI, -Br, -CN, -CH₂F; -CHF₂; -CF₃; -CH₂Cl; -CHCl₂; -CCl₃; NO₂, ou 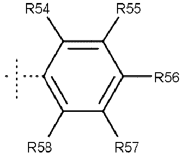 ou 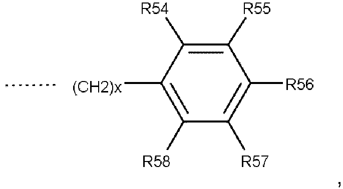 dans lequel R⁵⁴, R⁵⁵, R⁵⁶, R⁵⁷ ou R⁵⁸ sont choisis chacun indépendamment parmi l'hydrogène,
dans lequel, de préférence, X n'est pas présent, et
dans lequel, de préférence, tous les R¹ à R⁵ sont de l'hydrogène ou 1, 2 ou 3, de préférence 1 ou 2 des R¹ à R⁵ sont différents de l'hydrogène et choisis parmi les substitutions présentées dans la revendication 8 et les positions restantes des R¹ à R⁵ sont choisies parmi l'hydrogène.

5. Composé utilisable selon l'une des revendications 3 à 4, dans lequel R⁶ et R⁷ sont chacun indépendamment un hydrogène, ou R⁶ et R⁷ forment ensemble un anneau hétérocyclique choisi parmi:

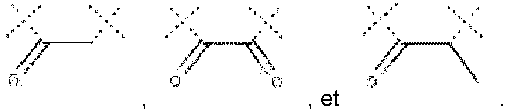

6. Composé utilisable selon l'une des revendications 3 à 4, dans lequel R⁷, N et X forment ensemble un anneau hétérocyclique à six chaînons, choisi parmi 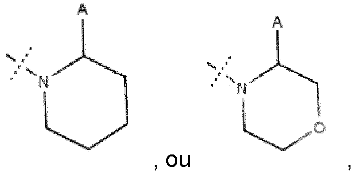 dans lequel A est tel que défini dans la revendication 3.

7. Composé utilisable selon les revendications 3 à 6, dans lequel R¹, R², R³, R⁴ et R⁵ sont chacun un hydrogène et/ou dans lequel A est 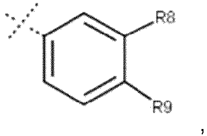 et R⁸ et R⁹ sont identiques ou différents l'un de l'autre, de préférence différents l'un de l'autre, et R⁸ et R⁹ sont chacun indépendamment choisi parmi l'hydrogène; -OH, -OCH₃, -OCF₃, -OCHF₂, -OCH₂OH; -OCH₂CH₃; -OCH(CH₃)₂; -F; -Cl; -Br; -CN; NO₂; NH₂; -CH₃, - CH₂F; -CHF₂; -CF₃; -CH₂Cl; -CHCl₂; -CCl₃;CH(CH₃)₂; -CH₂CH₃; -CH-CH₂-CH(CH₃)₂; - CH₂-N(CH₃)₂; -CH(CH₃)-NHC(O)CH₃; - -NH-CH(CH₃)₂, -NH-cyclopropyle, -CHF₂, - CF₃, -CH₃, CH₂CH₃, CH₂CH(CH₃)₂; -C(O)-C₂H₅; -C(O)-CH₃; -C(O)-aziridine; -C(O)-azétidine; -C(O)-pyrrolidine; -C(CH₃)=NOH; -CH₂NHS(O)₂CH₃; -S(O)₂-cyclopentyle; - S(O)₂-CH₃; -S(O)₂-cyclopentyle; pyrrolidinyle, , 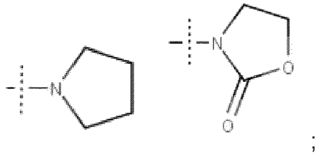
NHC(O)-R(41), dans lequel R⁴¹ est choisi parmi le furane, le tétrahydrofurane, le cyclopentyle,
cyclobutyle, le cyclopropyle, -NH-CH(CH₃)₂, -NH-cyclopropyl, C₁-C₅ alkyl, -CH₂F; - CHF₂; -CF₃; -CH₂Cl; -CHCl₂; -CCl₃;-CH₃, CH₂CH₃, CH₂CH(CH₃)₂;
-C(O)OR⁴², dans lequel R⁴² est choisi parmi -CH₃; -CH₂CH₃, -CH₂CH₂CH₃; -CH(CH₃)₂; -CH₂CH₂CH₂CH₃;
-NHR⁴³, dans lequel R⁴³ est choisi parmi 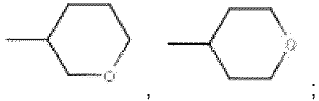 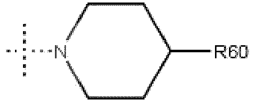 dans lequel R⁶⁰ est choisi parmi l'hydrogène ou -CH₃; 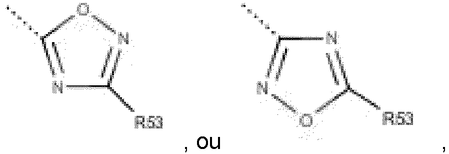 dans lequel
R⁵³ est choisi dans le groupe constitué par l'hydrogène, OH, -OCH₃, -OCF₃, -OCHF₂, - OCH₂OH; -OCH₂CH₃; -F; -Cl; -Br; -CN; NO₂; NH₂; -CH₃, CH(CH₃)₂; -CH₂CH₃; -CH-CH₂-CH(CH₃)₂; -CH₂-N(CH₃)₂; -CH(CH₃)-NHC(O)CH₃; -CH₂F; -CHF₂; -CF₃; -CH₂Cl; - CHCl₂; -CCl₃;-O-N=O, -N=O, -CH=NH, -N₃, -SH, -SO₃H, -SO₂H, -S(O)₂CH₃ -CN, -NC,-OCN, -NCO, -(C=O)OH, -(C=O)NHOH, -NH(C=O)NH₂, -NH(C=S)NH₂, -(C=O)NH₂,-CHO,-CHS, -OCHO, -NCHO, , 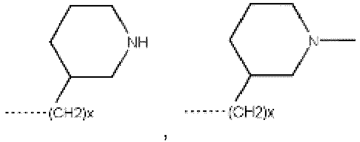 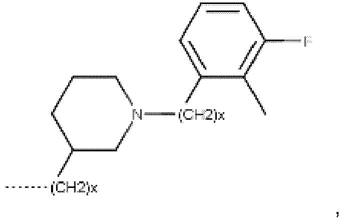 avec X = 0 ou 1, de préférence 1; 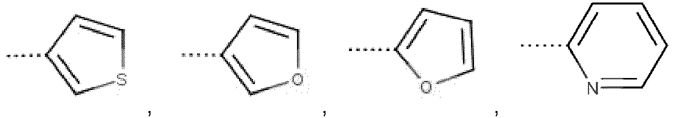 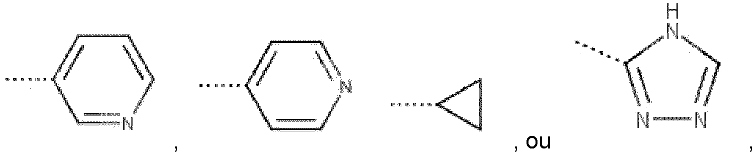 ou
R⁵³ est 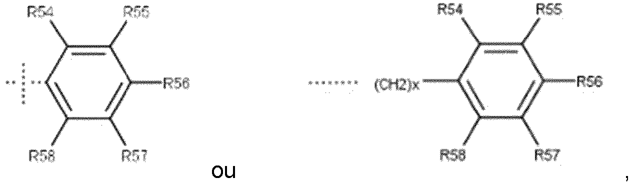 dans lequel R⁵⁴, R⁵⁵, R⁵⁶, R ⁵⁷ou R⁵⁸ sont choisis chacun indépendamment parmi hydrogène, - CH₃, -C₂H₅, -CH₂F; -CHF₂; -CF₃; -CH₂Cl; -CHCl₂; -CCl₃; -OCH₃, -F, -Cl, -Br, NH₂, NO₂, et x est égal à 0, 1 ou 2;
ou R⁵³ est 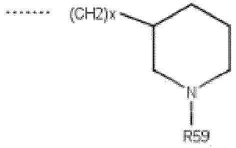 dans lequel R⁵⁹ est 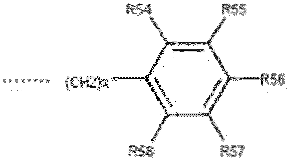 et dans lequel R⁵⁴, R⁵⁵, R⁵⁶, R⁵⁷ ou R⁵⁸ sont choisis séparément parmi l'hydrogène, -CH₃, - C₂H₅, -CH₂F; -CHF₂; -CF₃; -CH₂Cl; -CHCl₂; -CCl₃;-OCH₃, -F, -Cl, -Br, NO₂, NH₂, et x est égal à 0, 1 ou 2; comme défini ci-dessus; ou éventuellement
R⁸ et R⁹ forment ensemble un cycloalkyle à cinq ou six chaînons, un cyclohétéroalkyle à cinq ou six chaînons, un aryle à cinq ou six chaînons, un hétéroaryle à cinq ou six chaînons; de préférence, l'anneau hétérocyclique est choisi parmi les suivants:

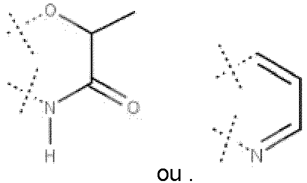

8. Composé utilisable selon la revendication 7, à condition que R⁸ ou R⁹ ne soient pas identiques l'un à l'autre.

9. Composé utilisable selon l'une des revendications 3 à 8, dans lequel les R¹ à R⁵ sont choisis comme suit:
R¹ est l'hydrogène, -C₁-C₂ alcoxy, -C(O)O-C₁-C₅ alkyl, -OCH₃, -C₁-C₃ alkyl, méthyl ou éthyl;
R² est l'hydrogène, le méthyle, -F, -Cl, -Br, -CN, -OCH₃, ou -C(O)O-C₁-C₅ alkyle;
R³ est l'hydrogène, le méthyle, -CH(CH₃)₂, -CHF₂, -F, -Cl, -Br, -CN, -OCH₃, -OCF₃, NO₂, -ou C(O)O-C₁-C₅ alkyle,
R⁴ est un hydrogène ou -F, -Cl, -Br, ou -CF₃; et/ou
R⁵ est l'hydrogène, le méthyle, l'éthyle ou -F, -Cl, -Br, ou -CN, -CH₂-phényle.

10. Composé à utiliser selon l'une des revendications 3 à 6, dans lequel le composé est un composé selon l'une des formules (Ib), (Ib1), (lb2) ou (lb3): 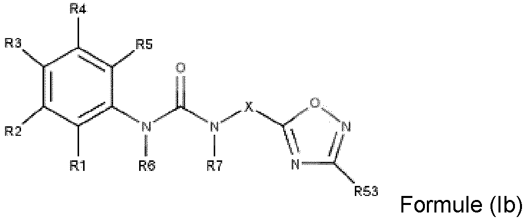 ou 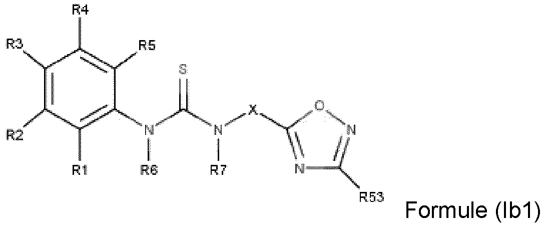 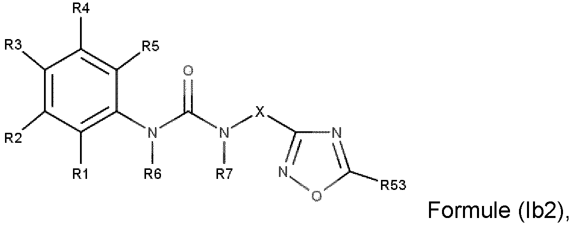 ou 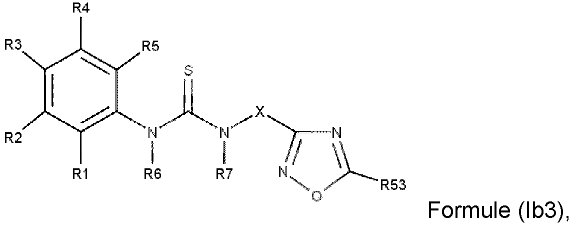
dans lequel X est choisi dans le groupe constitué d'un alkyle en C₁-C₃, -CH(CH₃)₂ - CH₂-, -C₂H₄-, -C₃H₆-, -CHCH(CH₃)₂-, -CHC(CH₃)₃-, -CH(CH(CH₃)₂)-, ou - CH(CH₂CH(CH₃)₂)-, ou -CH(CH₂OH)-, ou n'est pas présent, et
dans lequel R⁵³ est choisi dans le groupe constitué par l'hydrogène, OH, -OCH₃, - OCF₃, -OCHF₂, -OCH₂OH; -OCH₂CH₃; -F; -Cl; -Br; -CN; NO₂; NH₂; alkyle en C1-C5, alcoxy en C1-C5, alcényle en C2-C5; NH₂; alkyle en C1-C5, alcoxy en C1-C5, alcényle en C₂-C₅, de préférence -CH₃, CH(CH₃)₂; -CH₂CH₃; -CH-CH₂-CH(CH₃)₂; - CH₂-N(CH₃)₂; -CH(CH₃)-NHC(O)CH₃; -CH₂F; -CHF₂; -CF₃; -CH₂Cl; -CHCl₂; -CCl₃;-ON=O, -N=O, -CH=NH, -N₃, -SH, -SO₃H, -SO₂H, -S(O)₂CH₃ -CN, -NC,-OCN, -NCO, - (C=O)OH, -(C=O)NHOH, -NH(C=O)NH₂, -NH(C=S)NH₂, -(C=O)NH₂,-CHO,-CHS, - OCHO, -NCHO, 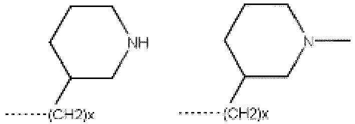 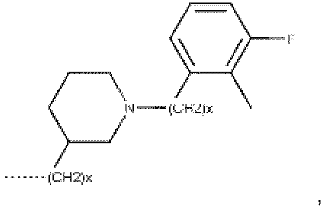 avec X = 0 ou 1, de préférence 1; 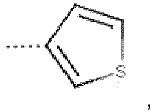 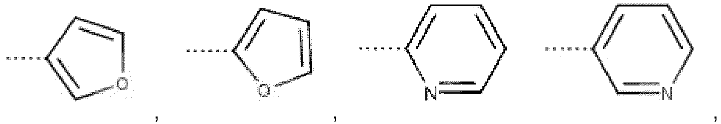 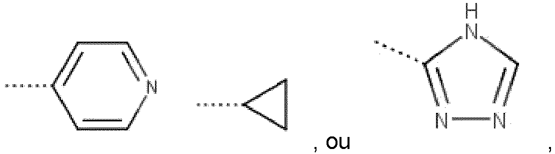 ou
R⁵³ est 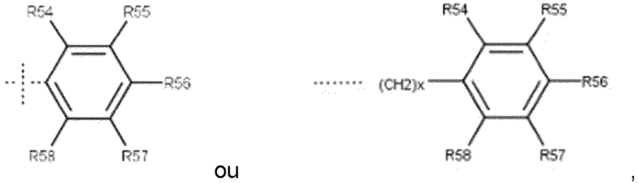 dans lequel R⁵⁴, R⁵⁵, R⁵⁶, R⁵⁷ ou R⁵⁸ sont choisis chacun indépendamment parmi hydrogène, - CH₃, -C₂H₅, -CH₂F; -CHF₂; -CF₃; -CH₂Cl; -CHCl₂; -CCl₃; -OCH₃, -F, -Cl, -Br, NH₂, NO₂, et x est égal à 0, 1 ou 2;
ou R⁵³ est 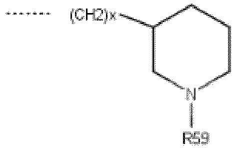 dans lequel R⁵⁹ est 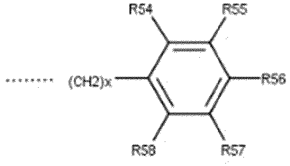 et dans lequel R⁵⁴, R⁵⁵, R⁵⁶, R⁵⁷ ou R⁵⁸ sont choisis séparément parmi l'hydrogène, -CH₃, - C₂H₅, -CH₂F; -CHF₂; -CF₃; -CH₂Cl; -CHCl₂; -CCl₃;-OCH₃, -F, -Cl, -Br, NO₂, NH₂, et x est égal à 0, 1 ou 2; tel que défini ci-dessus, et x est égal à 0, 1 ou 2;
et dans lequel, de préférence, tous les R⁵⁴ à R⁵⁸ sont de l'hydrogène ou 1, 2 ou 3, de préférence 1 ou 2 des R⁵⁴ à R⁵⁸ sont différents de l'hydrogène et choisis parmi les substitutions présentées ci-dessus et les positions restantes des R⁵⁴ à R⁵⁸ sont choisies parmi l'hydrogène.

11. Composé à utiliser selon la revendication 1, dans lequel le composé est un composé de formule (Id): 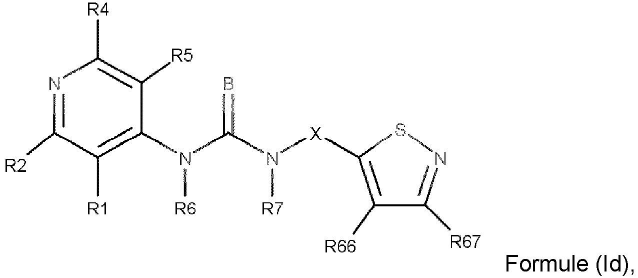 dans lequel, de préférence
B est O ou S, de préférence O,
X est choisi parmi C₁-C₅-alkyl, -CH₂-, -C₂H₄-, -C₃H₆-, -C(CH₃)₂-, -CHCH(CH₃)₂-, - CHC(CH₃)₃-, -CH(CH(CH₃)₂)-, -CH(CH₂CH(CH₃)₂)-, ou -CH(CH₂OH)-;
R¹, R², R⁴ et R⁵ sont choisis séparément parmi l'hydrogène, -CH₃, -C₂H₅, -CH₂CH₃-CH₂F; -CHF₂; -CF₃; -CH₂Cl; -CHCl₂; -CCl₃; -OCH₃, -F, -Cl, -Br, NO₂, NH₂, de préférence dans lequel deux ou trois des R¹, R², R⁴ et R⁵ sont de l'hydrogène et les autres ne sont pas de l'hydrogène; et
R⁵⁴ et R⁵⁵ sont chacun choisi séparément parmi l'hydrogène, -CH₃, -C₂H₅, -CH₂F; - CHF₂; -CF₃; -CH₂Cl; -CHCl₂; -CCl₃;-OCH₃, -F, -Cl, -Br, NO₂, NH₂, de préférence dans lequel l'un des R⁵⁴ et R⁵⁵ est un hydrogène et l'autre est l'un des éléments suivants: - CH₃, -C₂H₅, -CH₂F; -CHF₂; -CF₃; -CH₂Cl; -CHCl₂; -CCl₃;-OCH₃, -F, -Cl, -Br, NO₂, NH₂.

12. Composé utilisable selon l'une des revendications 3 à 6 et 10 à 11, dans lequel le composé est un composé selon l'une des formules (Ib), (Ib1), (Ib2), (Ib3) ou (Id) et R¹ à R⁵ sont choisis comme suit:
R¹ est un hydrogène, -C₁-C₂ alcoxy, -C(O)O-C₁-C₅ alkyle, -OCH₃, -C₁-C₃ alkyle, méthyle ou éthyle
R² est l'hydrogène, le méthyle, -F, -Cl, -Br, -CN, -OCH₃, ou -C(O)O-C₁-C₅ alkyle;
R³ , le cas échéant, est l'hydrogène, le méthyle, -CH(CH₃)₂, -CHF₂, -F, -Cl, -Br, -CN, - OCH₃, -OCF₃, NO₂, ou -C(O)O-(C₁-C₅ alkyl);
R⁴ est un hydrogène ou -F, -Cl, -Br, ou -CF₃; et/ou
R⁵ est l'hydrogène, le méthyle, l'éthyle ou -F, -Cl, -Br, -CN, ou -CH₂-phényle.

13. Composé à utiliser selon l'une des revendications 1 à 12, dans lequel le composé est choisi parmi les composés décrits dans le tableau 1.
**Tableau 1:**
| **No n.** | **Code** | **Structure** | **Nom IUPAC** |
|---|---|---|---|
| **1** | EN22 | 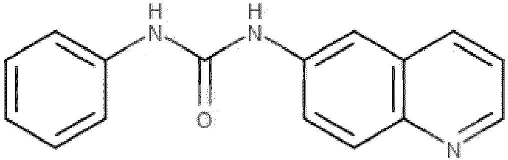 | 1-phenyl-3-(quinolin-6-yl)urea |
| **2** | EN21 | 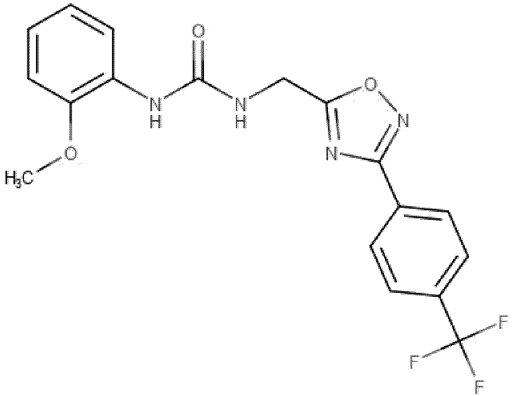 | 1-(2-methoxyphenyl)-3-((3-(4-(trifluoromethyl)p henyl)-1,2,4-oxadiazol-5-yl)methyl)urea |
| **3** | EN-A (EN23) | 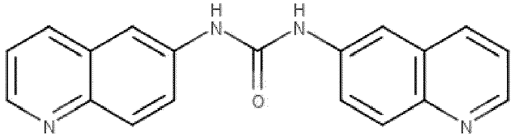 | 1,3-di(quinolin-6-yl)urea |
| **4** | AH4 | 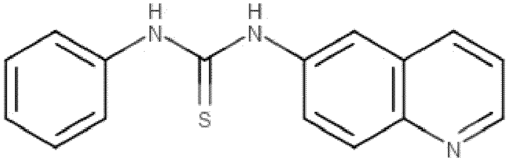 | 3-phenyl-1-(quinolin-6-yl)thiourea |
| **5** | JB010 | 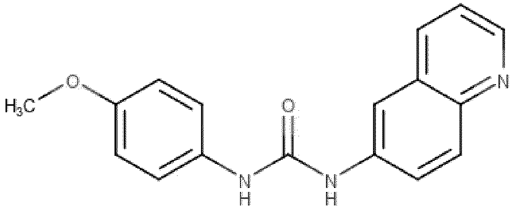 | 1-(4-methoxyphenyl)-3-(quinolin-6-yl)urea |
| **6** | BRM/BR G1 Inhibiteur de l'ATP-1 | 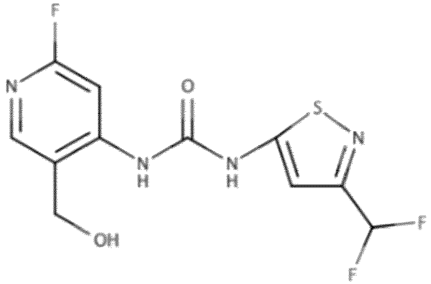 | 1-[3-(difluoromethyl)-1,2-thiazol-5-yl]-3-[2-fluoro-5-(hydroxymethyl)p yridin-4-yl]urea |
| **7** | JB011 | 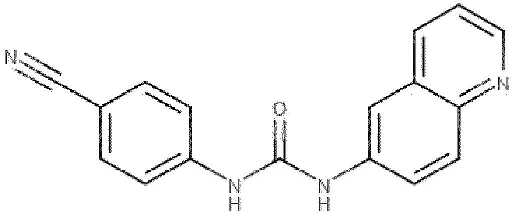 | 1-(4-cyanophenyl)-3-(quinolin-6-yl)urea |
| **8** | PM07 | 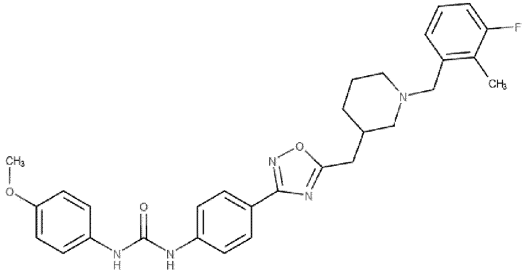 | 3-{4-[5-({1-[(3-fluoro-2-methylphenyl)me thyl]piperidin-3-yl}methyl)-1,2,4-oxadiazol-3-yl]phenyl}-1-(4-methoxyphenyl)u rea |
| **9** | PM16 | 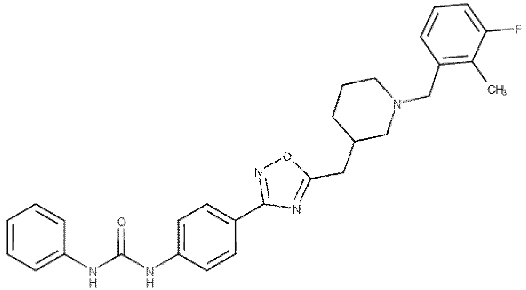 | 3-{4-[5-({1-[(3-fluoro-2-methylphenyl)me thyl]piperidin-3-yl}methyl)-1,2,4-oxadiazol-3-yl]phenyl}-1-phenylurea |
| **10** | JB014 | 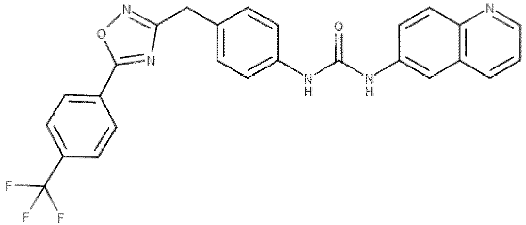 | 1-(quinolin-6-yl)-3-[4-({5-[4-(trifluoromethyl)p henyl]-1,2,4-oxadiazol-3-yl}methyl)phenyl] urea |
| **11** | AH1 | 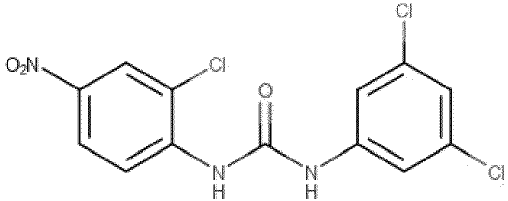 | 3-(2-chloro-4-nitrophenyl)-1-(3,5-dichlorophenyl)ur ea |
| **12** | AH3 | 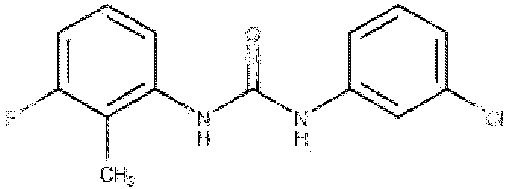 | 1-(3-chlorophenyl)-3-(3-fluoro-2-methylphenyl)ure a |
| **13** | JB002 | 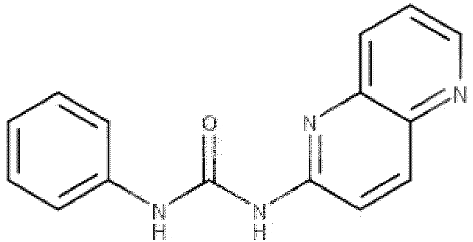 | 1-(1,5-naphthyridin-2-yl)-3-phenylurea |
| **14** | JB004 | 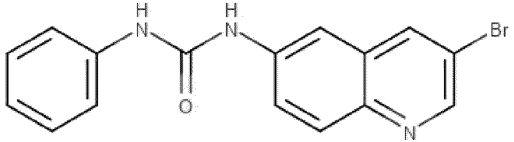 | 1-(3-bromoquinolin-6-yl)-3-phenylurea |
| **15** | EN11-16 | 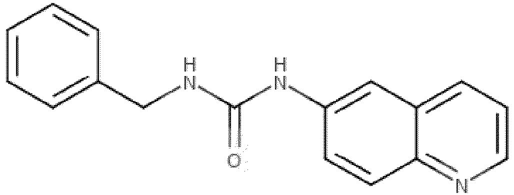 | 1-benzyl-3-(quinolin-6-yl)urea |
| **16** | EN11-13 | 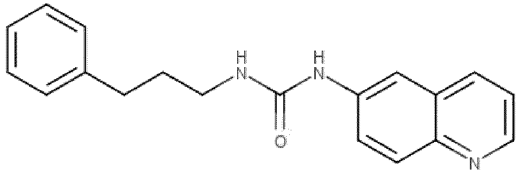 | 1-(3-phenylpropyl)-3-(quinolin-6-yl)urea |
| **17** | L01.110 | 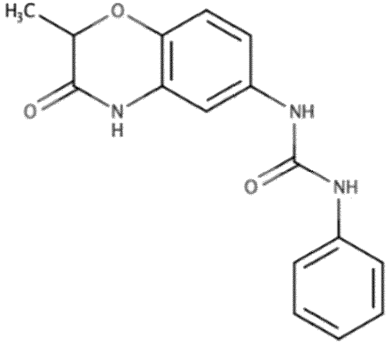 | 3-(2-methyl-3-oxo-3,4-dihydro-2H-1,4-benzoxazin-6-yl)-1-phenylurea |
| **18** | L01.041 | 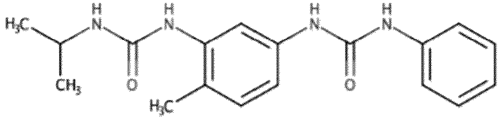 | 3-(4-methyl-3-{[(propan-2-yl)carbamoyl]ami no}phenyl)-1-phenylurea |
| **19** | L01.004 | 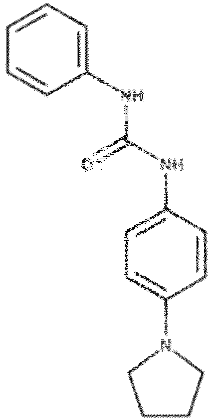 | 3-phenyl-1-[4-(pyrrolidin-1-yl)phenyl]urea |
| **20** | L01.007 | 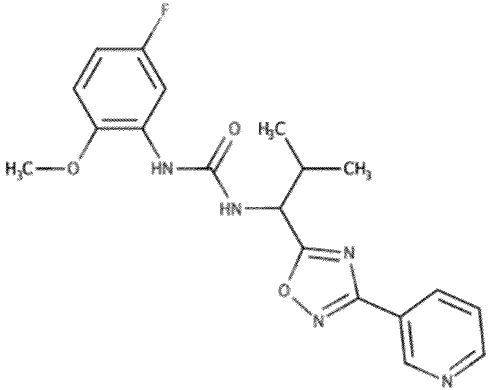 | 1-(5-fluoro-2-methoxyphenyl)-3-{2-methyl-1-[3-(pyridin-3-yl)-1,2,4-oxadiazol-5-yl]propyl}urea |
| **21** | L01.104 | 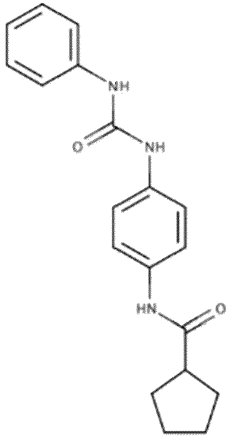 | N-{4-[(phenylcarbamo yl)amino]phenyl} cyclopentanecar boxamide |
| **22** | L01.116 | 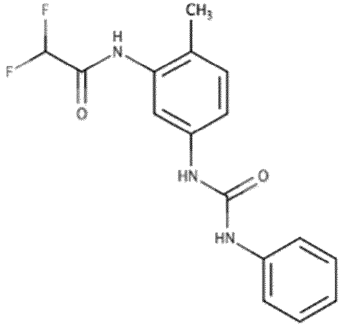 | 2,2-difluoro-N-{2-methyl-5-[(phenylcarbamo yl)amino]phenyl} acetamide |
| **23** | L01.113 | 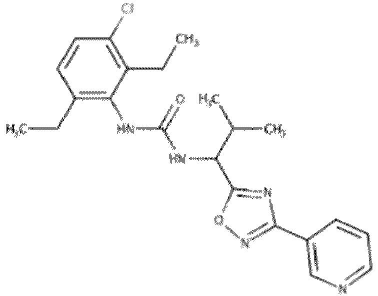 | 1-(3-chloro-2,6-diethylphenyl)-3-{2-methyl-1-[3-(pyridin-3-yl)-1,2,4-oxadiazol-5-yl]propyl}urea |
| **24** | L01.101 | 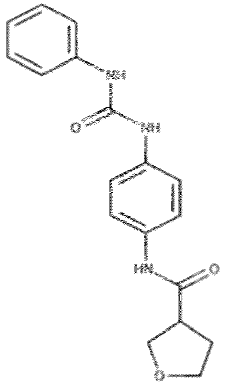 | N-{4-[(phenylcarbamo yl)amino]phenyl} oxolane-3-carboxamide |
| **25** | L01.092 | 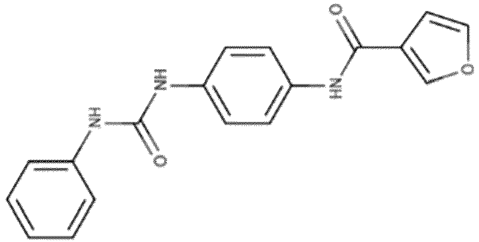 | N-{4-[(phenylcarbamo yl)amino]phenyl}f uran-3-carboxamide |
| **26** | L01.124 | 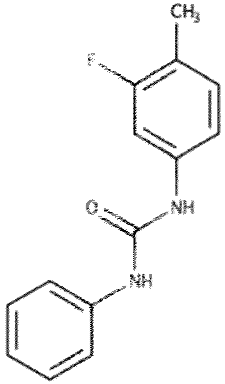 | 3-(3-fluoro-4-methylphenyl)-1-phenylurea |
| **27** | TCMDC-143175 | 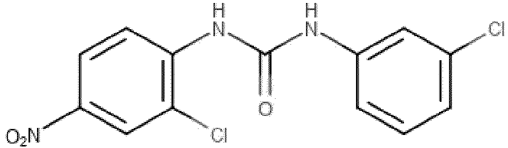 | 3-(2-chloro-4-nitrophenyl)-1-(3-chlorophenyl)ure a |
| **28** | A.C2 | 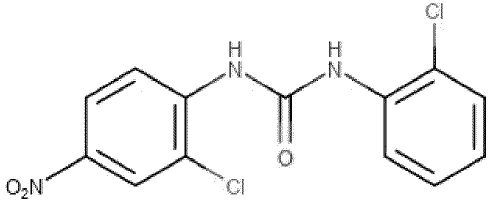 | 3-(2-chloro-4-nitrophenyl)-1-(2-chlorophenyl)ure a |
| **29** | AH2 | 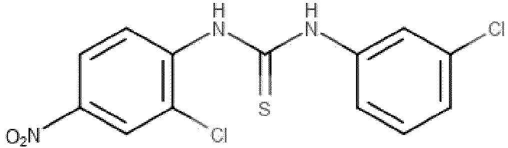 | 3-(2-chloro-4-nitrophenyl)-1-(3-chlorophenyl)thio urea |
| **30** | EN11-07 | 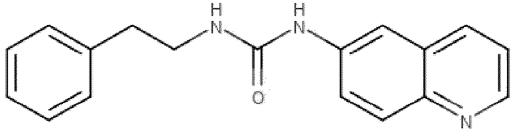 | 1-(2-phenylethyl)-3-(quinolin-6-yl)urea |
| **31** | EN11-02 | 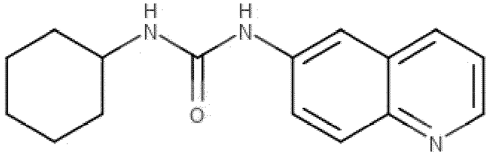 | 1-cyclohexyl-3-(quinolin-6-yl)urea |
| **32** | EN11-11 | 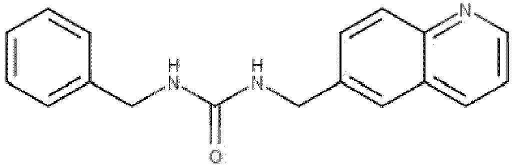 | 3-benzyl-1-[(quinolin-6-yl)methyl]urea |
| **33** | L01.016 | 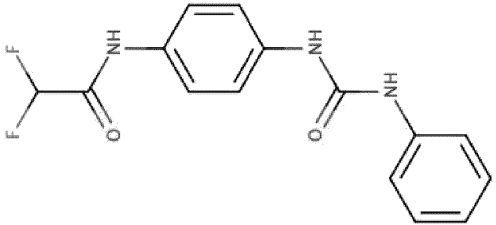 | 2,2-difluoro-N-{4-[(phenylcarbamo yl)amino]phenyl} acetamide |
| **34** | L01.028 | 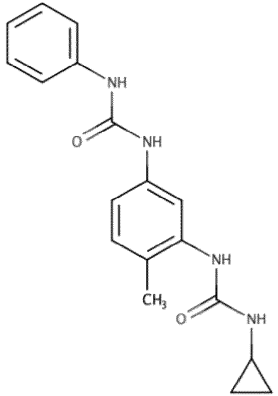 | 3-cyclopropyl-1-{2-methyl-5-[(phenylcarbamo yl)amino]phenyl} urea |
| **35** | L01.047 | 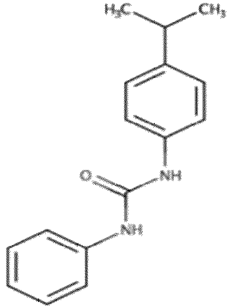 | 1-phenyl-3-[4-(propan-2-yl)phenyl]urea |
| **36** | L01.026 | 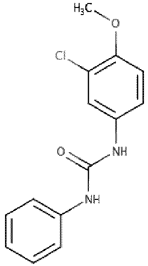 | 3-(3-chloro-4-methoxyphenyl)-1-phenylurea |
| 37 | L01.127 | 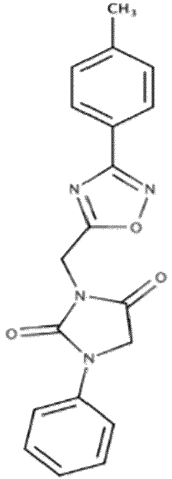 | 3-{[3-(4-methylphenyl)-1,2,4-oxadiazol-5-yl]methyl}-1-phenylimidazolidi ne-2,4-dione |
| **38** | L01.061 | 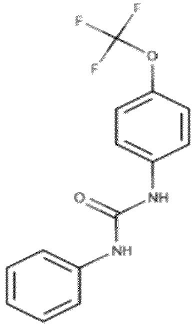 | 1-phenyl-3-[4-(trifluoromethoxy )phenyl]urea |
| **39** | L01.128 | 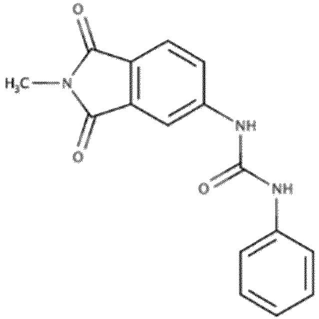 | 3-(2-methyl-1,3-dioxo-2,3-dihydro-1H-isoindol-5-yl)-1-phenylurea |
| **40** | L01.025 | 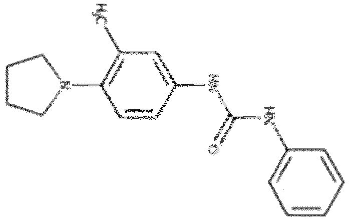 | 3-[3-methyl-4-(pyrrolidin-1-yl)phenyl]-1-phenylurea |
| **41** | L01.008 | 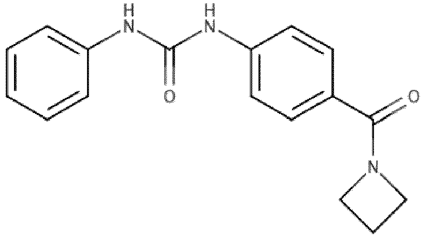 | 1-[4-(azetidine-1-carbonyl)phenyl]-3-phenylurea |
| **42** | L01.089 | 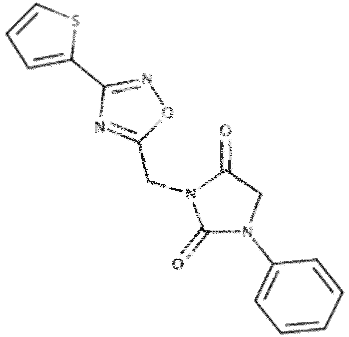 | 1-phenyl-3-{[3-(thiophen-2-yl)-1,2,4-oxadiazol-5-yl]methyl}imidaz olidine-2,4-dione |
| **43** | L01.130 | 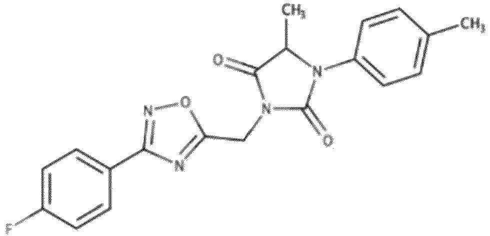 | 3-{[3-(4-fluorophenyl)-1,2,4-oxadiazol-5-yl]methyl}-5-methyl-1-(4-methylphenyl)imi dazolidine-2,4-dione |
| **44** | L01.049 | 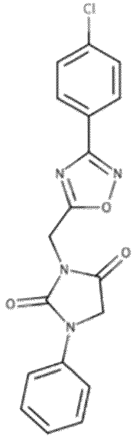 | 3-{[3-(4-chlorophenyl)-1,2,4-oxadiazol-5-yl]methyl}-1-phenylimidazolidi ne-2,4-dione |
| **45** | L01.066 | 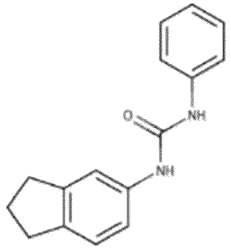 | 3-(2,3-dihydro-1H-inden-5-yl)-1-phenylurea |
| **46** | L01.043 | 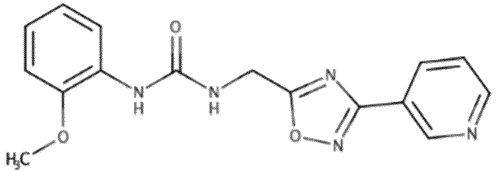 | 1-(2-methoxyphenyl)-3-{[3-(pyridin-3-yl)-1,2,4-oxadiazol-5-yl]methyl}urea |
| **47** | L01.087 | 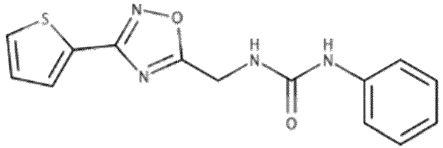 | 1-phenyl-3-{[3-(thiophen-2-yl)-1,2,4-oxadiazol-5-yl]methyl}urea |
| **48** | L01.020 | 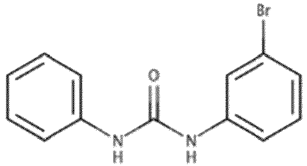 | 3-(3-bromophenyl)-1-phenylurea |
| **49** | L01.035 | 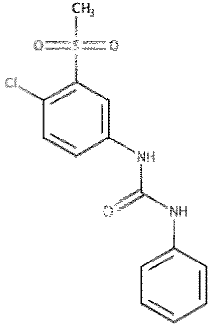 | 3-(4-chloro-3-methanesulfonyl phenyl)-1-phenylurea |
| **50** | L01.120 | 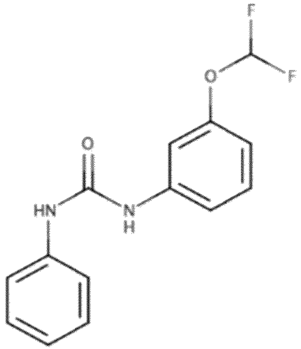 | 3-[3-(difluoromethoxy )phenyl]-1-phenylurea |
| **51** | L01.033 | 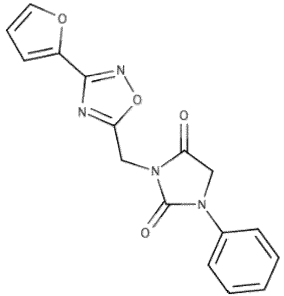 | 3-{[3-(furan-2-yl)-1,2,4-oxadiazol-5-yl]methyl}-1-phenylimidazolidi ne-2,4-dione |
| **52** | L01.002 | 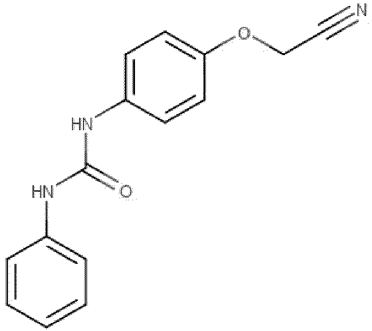 | 1-[4-(cyanomethoxy)p henyl]-3-phenylurea |
| **53** | L01.027 | 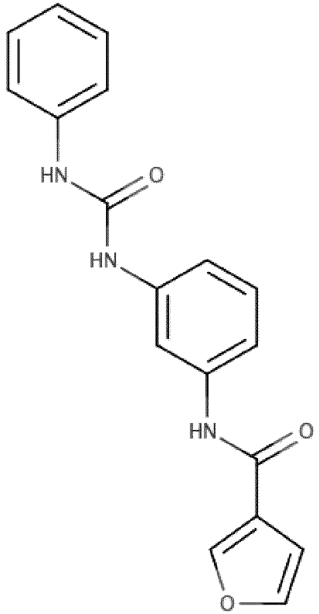 | N-{3-[(phenylcarbamo yl)amino]phenyl}f uran-3-carboxamide |
| **54** | L01.003 | 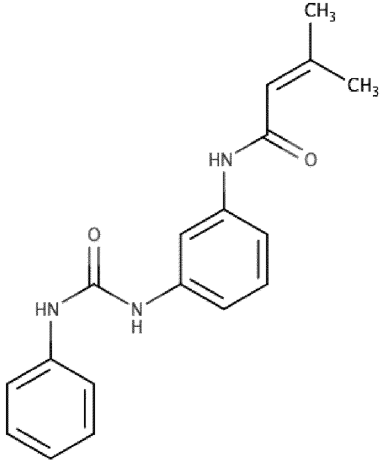 | 3-methyl-N-{3-[(phenylcarbamo yl)amino]phenyl} but-2-enamide |
| **55** | L01.038 | 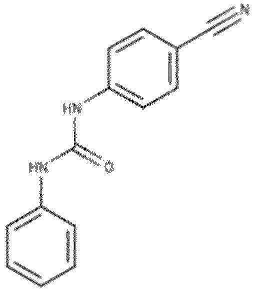 | 1-(4-cyanophenyl)-3-phenylurea |
| | | | |
| **56** | L01.069 | 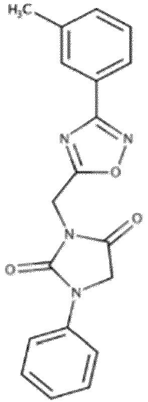 | 3-{[3-(3-methylphenyl)-1,2,4-oxadiazol-5-yl]methyl}-1-phenylimidazolidi ne-2,4-dione |
| **57** | L01.097 | 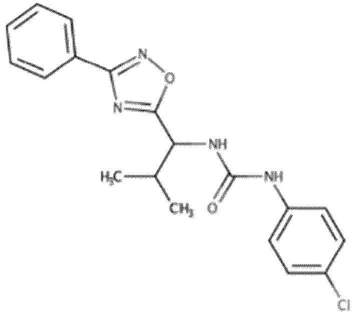 | 1-(4-chlorophenyl)-3-[2-methyl-1-(3-phenyl-1,2,4-oxadiazol-5-yl)propyl]urea |
| **58** | L01.095 | 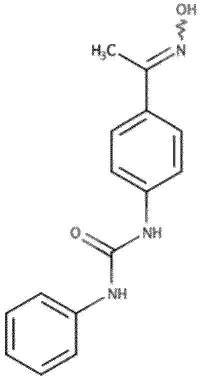 | 3-{4-[1-(hydroxyimino)et hyl]phenyl}-1-phenylurea |
| **59** | L01.013 | 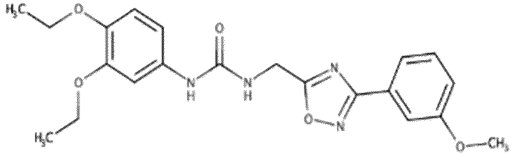 | 1-(3,4-diethoxyphenyl)-3-{[3-(3-methoxyphenyl)-1,2,4-oxadiazol-5-yl]methyl}urea |
| **60** | L01.103 | 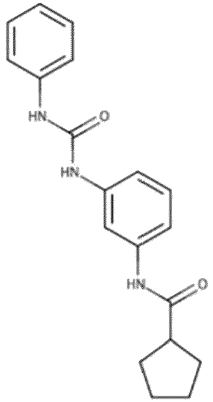 | N-{3-[(phenylcarbamo yl)amino]phenyl} cyclopentanecar boxamide |
| **61** | L01.071 | 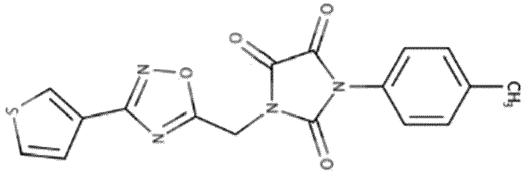 | 1-(4-methylphenyl)-3-{[3-(thiophen-3-yl)-1,2,4-oxadiazol-5-yl]methyl}imidaz olidine-2,4,5-trione |
| **62** | L01.115 | 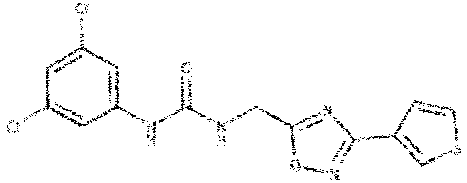 | 1-(3,5-dichlorophenyl)-3-{[3-(thiophen-3-yl)-1,2,4-oxadiazol-5-yl]methyl}urea |
| **63** | L01.045 | 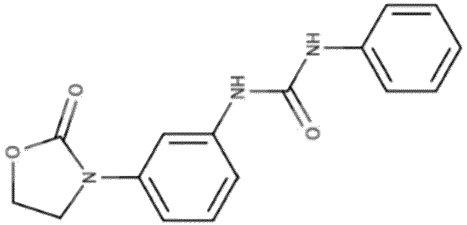 | 1-[3-(2-oxo-1,3-oxazolidin-3-yl)phenyl]-3-phenylurea |
| **64** | L01.077 | 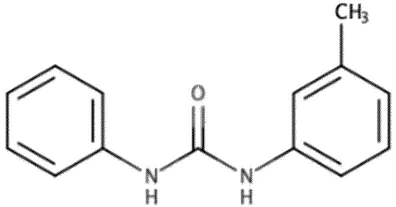 | 3-(3-methylphenyl)-1-phenylurea |
| **65** | L01.084 | 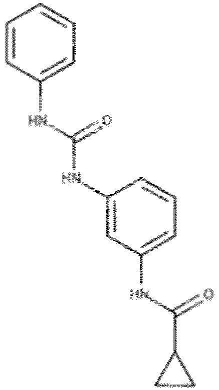 | N-{3-[(phenylcarbamo yl)amino]phenyl} cyclopropanecar boxamide |
| **66** | L01.109 | 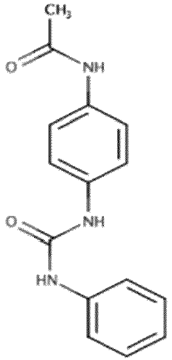 | N-{4-[(phenylcarbamo yl)amino]phenyl} acetamide |
| **67** | L01.122 | 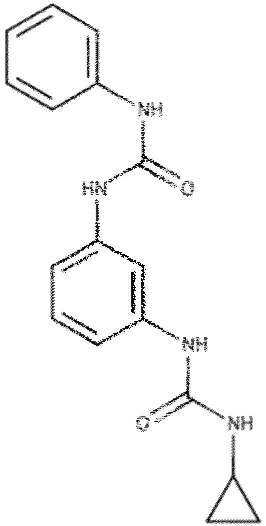 | 3-cyclopropyl-1-{3-[(phenylcarbamo yl)amino]phenyl} urea |
| **68** | L01.083 | 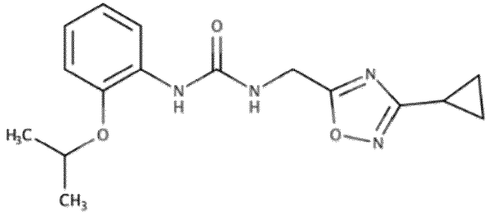 | 3-[(3-cyclopropyl-1,2,4-oxadiazol-5-yl)methyl]-1-[2-(propan-2-yloxy)phenyl]ure a |
| **69** | L01.062 | 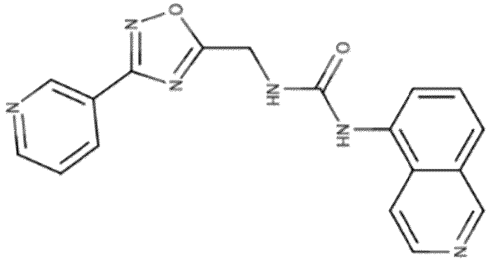 | 1-(isoquinolin-5-yl)-3-{[3-(pyridin-3-yl)-1,2,4-oxadiazol-5-yl]methyl}urea |
| **70** | L01.081 | 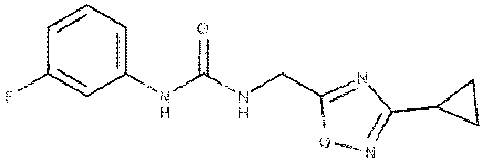 | 3-[(3-cyclopropyl-1,2,4-oxadiazol-5-yl)methyl]-1-(3-fluorophenyl)ure a |
| **71** | L01.114 | 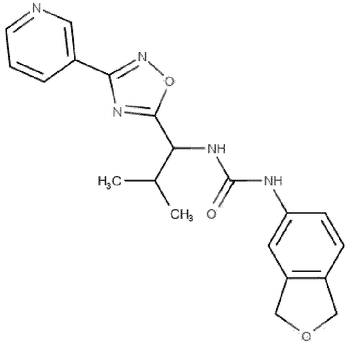 | 3-(1,3-dihydro-2-benzofuran-5-yl)-1-{2-methyl-1-[3-(pyridin-3-yl)-1,2,4-oxadiazol-5-yl]propyl}urea |
| **72** | L01.090 | 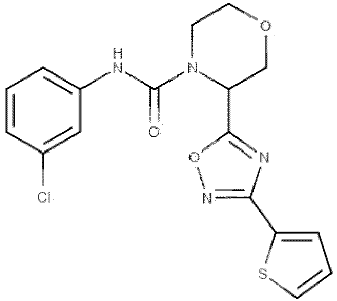 | N-(3-chlorophenyl)-3-[3-(thiophen-2-yl)-1,2,4-oxadiazol-5-yl]morpholine-4-carboxamide |
| **73** | L01.136 | 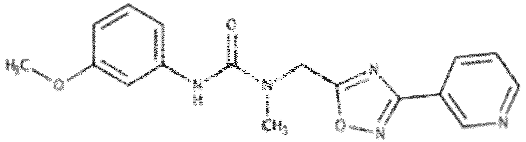 | 1-(3-methoxyphenyl)-3-methyl-3-{[3-(pyridin-3-yl)-1,2,4-oxadiazol-5-yl]methyl}urea |
| **74** | L01.023 | 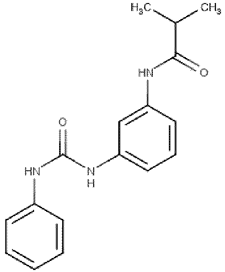 | 2-methyl-N-{3-[(phenylcarbamo yl)amino]phenyl} propanamide |
| **75** | L01.064 | 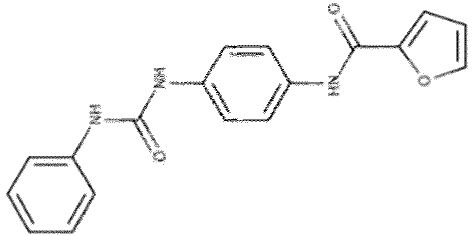 | N-{4-[(phenylcarbamo yl)amino]phenyl}f uran-2-carboxamide |
| **76** | L01.093 | 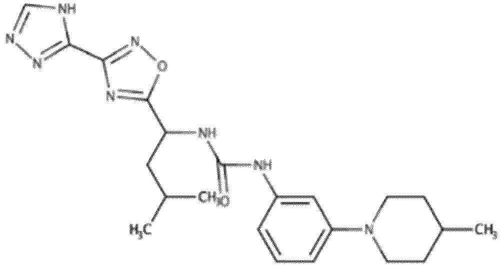 | 3-{3-methyl-1-[3-(4H-1,2,4-triazol-3-yl)-1,2,4-oxadiazol-5-yl]butyl}-1-[3-(4-methylpiperidin-1-yl)phenyl]urea |
| **77** | L01.125 | 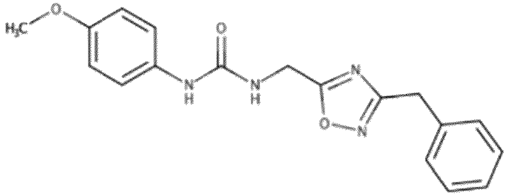 | 3-[(3-benzyl-1,2,4-oxadiazol-5-yl)methyl]-1-(4-methoxyphenyl)u rea |
| **78** | L01.005 | 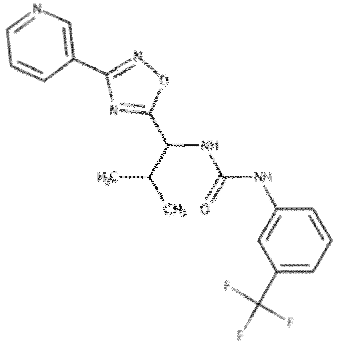 | 3-{2-methyl-1-[3-(pyridin-3-yl)-1,2,4-oxadiazol-5-yl]propyl}-1-[3-(trifluoromethyl)p henyl]urea |
| **79** | L01.135 | 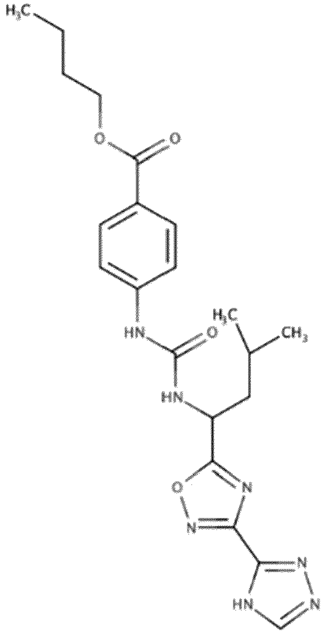 | butyl 4-[({3-methyl-1-[3-(4H-1,2,4-triazol-3-yl)-1,2,4-oxadiazol-5-yl]butyl}carbamo yl)amino]benzoat e |
| **80** | L01.108 | 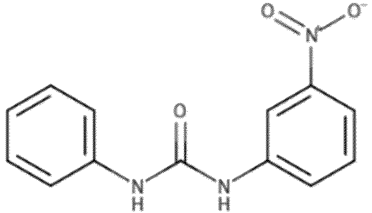 | 3-(3-nitrophenyl)-1-phenylurea |
| **81** | L01.112 | 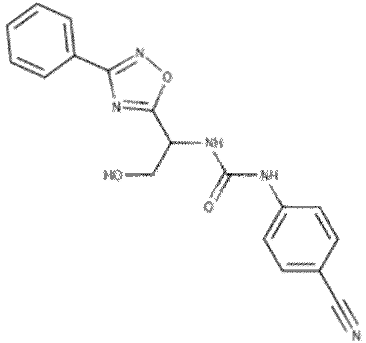 | 1-(4-cyanophenyl)-3-[2-hydroxy-1-(3-phenyl-1,2,4-oxadiazol-5-yl)ethyl]urea |
| **82** | L01.054 | 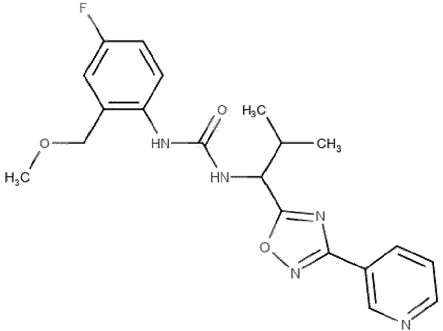 | 1-[4-fluoro-2-(methoxymethyl) phenyl]-3-{2-methyl-1-[3-(pyridin-3-yl)-1,2,4-oxadiazol-5-yl]propyl}urea |
| **83** | L01.067 | 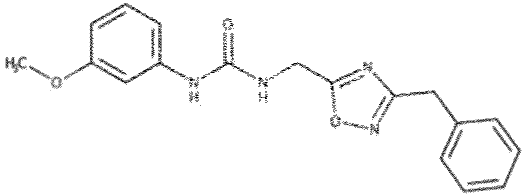 | 3-[(3-benzyl-1,2,4-oxadiazol-5-yl)methyl]-1-(3-methoxyphenyl)u rea |
| **84** | L01.099 | 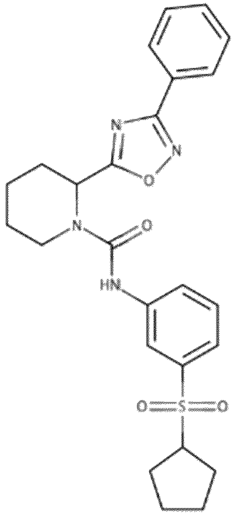 | N-[3-(cyclopentanesul fonyl)phenyl]-2-(3-phenyl-1,2,4-oxadiazol-5-yl)piperidine-1-carboxamide |
| **85** | L01.065 | 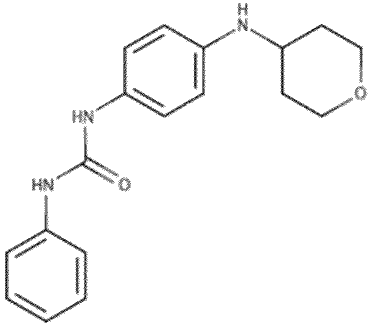 | 1-{4-[(oxan-4-yl)amino]phenyl}-3-phenylurea |
| **86** | L01.132 | 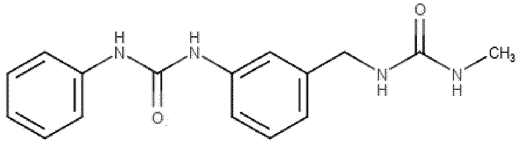 | 3-(3-{[(methylcarbam oyl)amino]methyl }phenyl)-1-phenylurea |
| **87** | L01.121 | 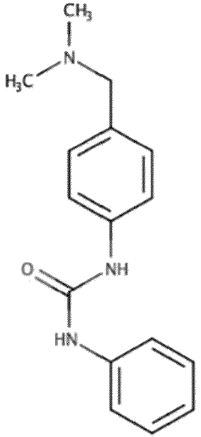 | 3-{4-[(dimethylamino) methyl]phenyl}-1-phenylurea |
| **88** | L01.056 | 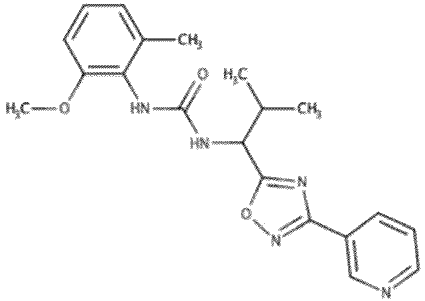 | 1-(2-methoxy-6-methylphenyl)-3-{2-methyl-1-[3-(pyridin-3-yl)-1,2,4-oxadiazol-5-yl]propyl}urea |
| **89** | L01.088 | 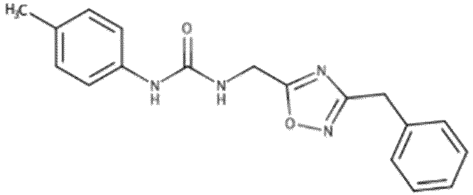 | 3-[(3-benzyl-1,2,4-oxadiazol-5-yl)methyl]-1-(4-methylphenyl)ure a |
| **90** | L01.042 | 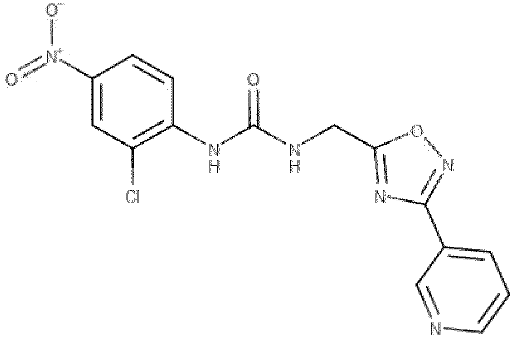 | 1-(2-chloro-4-nitrophenyl)-3-{[3-(pyridin-3-yl)-1,2,4-oxadiazol-5-yl]methyl}urea |
| **91** | L01.010 | 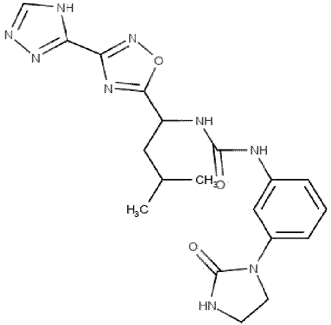 | 3-{3-methyl-1-[3-(4H-1,2,4-triazol-3-yl)-1,2,4-oxadiazol-5-yl]butyl}-1-[3-(2-oxoimidazolidin-1-yl)phenyl]urea |

14. Composé de l'une des revendications 1 à 13 pour la prévention ou le traitement d'une maladie par l'activation de l'inflammasome NLRP3, la maladie étant choisie dans un groupe comprenant les maladies infectieuses ou le cancer, ou pour stimuler les réponses immunitaires ou briser l'évasion immunitaire dans les maladies cancéreuses chez un sujet, ou pour une utilisation comme adjuvant dans le contexte de la vaccination pour stimuler les réponses immunitaires spécifiques par l'activation de l'inflammasome NLRP3.

15. Utilisation des composés tels que définis dans la revendication 13 pour analyser l'activité d'activation de NLRP3 *in vitro.*
